# EUROPEAN PATENT SPECIFICATION

(11) **EP 2 867 376 B1**
(45) Date of publication and mention of the grant of the patent: **06.09.2017**
(21) Application number: 13810495.5
(22) Date of filing: 28.06.2013
(51) Int. Cl.: C12Q 1/68, C12N 15/11

(54) **TARGETED RNA-SEQ METHODS AND MATERIALS FOR THE DIAGNOSIS OF PROSTATE CANCER**
GEZIELTE RNS-SEQ-VERFAHREN UND MATERIALIEN ZUR DIAGNOSE VON PROSTATAKREBS
MÉTHODES CIBLÉES CONNUES SOUS LE NOM D'ARN-SEQ ET MATÉRIAUX POUR LE DIAGNOSTIC DU CANCER DE LA PROSTATE

(30) Priority: 28.06.2012 US 201261665849 P; 21.08.2012 US 201261691743 P; 04.10.2012 US 201261709517 P
(43) Date of publication of application: 06.05.2015
(73) Proprietor: Caldera Health Limited, Ellerslie, Auckland 1542 (NZ)
(72) Inventor: WATSON, James Douglas, Auckland 1071 (NZ); ELTON, Clare, Auckland 1151 (NZ); MUSGRAVE, David Rex, Hamilton 3210 (NZ)
(74) Representative: Docherty, Robert Charles
(86) International application number: PCT/NZ2013/000114
(87) International publication number: WO 2014/003580

(56) References cited:
- WO-A1-2010/115154
- WO-A1-2012/040784
- WO-A2-2005/003387
- US-A1- 2008 233 563
- US-B1- 7 566 532
- LEVIN JOSHUA Z ET AL: "Targeted next-generation sequencing of a cancer transcriptome enhances detection of sequence variants and novel fusion transcripts", GENOME BIOLOGY, BIOMED CENTRAL LTD., LONDON, GB, vol. 10, no. 10, 16 October 2009 (2009-10-16), page R115, XP021065359, ISSN: 1465-6906, DOI: 10.1186/GB-2009-10-10-R115
- JAKUBOWSKA, A. ET AL.: 'Detection of germline mutations in the BRCA1 gene by RNA-based sequencing.' HUMAN MUTATION vol. 18, 2001, pages 149 - 156, XP055182806
- PRENSNER, J.R. ET AL.: 'Transcriptome sequencing across a prostate cancer cohort identifies PCAT 1, an unannotated lincRNA implicated in disease progression' NATURE BIOTECHNOLOGY vol. 29, no. 8, August 2011, pages 742 - 749, XP055106101
- MAHER, C.A. ET AL.: 'Chimeric transcript discovery by paired-end transcriptome sequencing.' PNAS vol. 106, no. 30, 2009, pages 12353 - 12358, XP002606464
- MARTENS-UZUNOVA, E.S. ET AL.: 'Diagnostic and prognostic signatures from the small non-coding RNA transcriptome in prostate cancer.' ONCOGENE vol. 31, no. 8, 23 February 2012, pages 978 - 991, XP055182808
- LEVIN, J.Z. ET AL.: 'Targeted next-generation sequencing of a cancer transcriptome enhances detection of sequence variants and novel fusion transcripts' GENOME BIOLOGY vol. 10, no. R115, 2009, XP021065359 & YASSOUR, M. ET AL.: 'Ab initio construction of a eukaryotic transcriptome by massively parallel mRNA sequencing.' PNAS vol. 106, no. 9, 2009, pages 3264 - 3269, XP055182810

### SEQUENCE LISTING

<110> Watson, James D
   Elton, Clare
   Musgrave, David Rex
<120> Methods and Materials for the Diagnosis
   of Prostate Cancer
<130> 24700.1009PCT
<150> 61/665,849
   <151> 2012-06-28
<150> 61/691,743
   <151> 2012-08-21
<150> 61/709,517
   <151> 2012-10-04
<160> 326
<170> FastSEQ for windows version 4.0
<210> 1
   <211> 1449
   <212> DNA
   <213> Homo sapiens
<400> 1
   ctggatagaa cagctcaagc cttgccactt cgggcttctc actgcagctg ggcttggact      60
   tcggagtttt gccattgcca gtgggacgtc tgagactttc tccttcaagt acttggcaga    120
   tcactctctt agcagggtct gcgcttcgca gccgggatga agctggtttc cgtcgccctg    180
   atgtacctgg gttcgctcgc cttcctaggc gctgacaccg ctcggttgga tgtcgcgtcg    240
   gagtttcgaa agaagtggaa taagtgggct ctgagtcgtg ggaagaggga actgcggatg    300
   tccagcagct accccaccgg gctcgctgac gtgaaggccg ggcctgccca gacccttatt    360
   cggccccagg acatgaaggg tgcctctcga agccccgaag acagcagtcc ggatgccgcc    420
   cgcatccgag tcaagcgcta ccgccagagc atgaacaact tccagggcct ccggagcttt    480
   ggctgccgct tcgggacgtg cacggtgcag aagctggcac accagatcta ccagttcaca    540
   gataaggaca aggacaacgt cgcccccagg agcaagatca gcccccaggg ctacggccgc    600
   cggcgccggc gctccctgcc cgaggccggc ccgggtcgga ctctggtgtc ttctaagcca    660
   caagcacacg gggctccagc ccccccgagt ggaagtgctc cccactttct ttaggattta    720
   ggcgcccatg gtacaaggaa tagtcgcgca agcatcccgc tggtgcctcc cgggacgaag    780
   gacttcccga gcggtgtggg gaccgggctc tgacagccct gcggagaccc tgagtccggg    840
   aggcaccgtc cggcggcgag ctctggcttt gcaagggccc ctccttctgg gggcttcgct    900
   tccttagcct tgctcaggtg caagtgcccc agggggcggg gtgcagaaga atccgagtgt    960
   ttgccaggct taaggagagg agaaactgag aaatgaatgc tgagaccccc ggagcagggg   1020
   tctgagccac agccgtgctc gcccacaaac tgatttctca cggcgtgtca ccccaccagg   1080
   gcgcaagcct cactattact tgaactttcc aaaacctaaa gaggaaaagt gcaatgcgtg   1140
   ttgtacatac agaggtaact atcaatattt aagtttgttg ctgtcaagat tttttttgta   1200
   acttcaaata tagagatatt tttgtacgtt atatattgta ttaagggcat tttaaaagca   1260
   attatattgt cctcccctat tttaagacgt gaatgtctca gcgaggtgta aagttgttcg   1320
   ccgcgtggaa tgtgagtgtg tttgtgtgca tgaaagagaa agactgatta cctcctgtgt   1380
   ggaagaagga aacaccgagt ctctgtataa tctatttaca taaaatgggt gatatgcgaa   1440
   cagcaaacc                                                           1449
<210> 2
   <211> 996
   <212> DNA
   <213> Homo sapiens
<400> 2
   aatcacttgg ggaaaggaag gttcgtttct gagttagcaa caagtaaatg cagcactagt     60
   gggtgggatt gaggtatgcc ctggtgcata aatagagact cagctgtgct ggcacactca    120
   gaagcttgga ccgcatccta gccgccgact cacacaaggc aggtgggtga ggaaatccag    180
   agttgccatg gagaaaattc cagtgtcagc attcttgctc cttgtggccc tctcctacac    240
   tctggccaga gataccacag tcaaacctgg agccaaaaag gacacaaagg actctcgacc    300
   caaactgccc cagaccctct ccagaggttg gggtgaccaa ctcatctgga ctcagacata    360
   tgaagaagct ctatataaat ccaagacaag caacaaaccc ttgatgatta ttcatcactt    420
   ggatgagtgc ccacacagtc aagctttaaa gaaagtgttt gctgaaaata aagaaatcca    480
   gaaattggca gagcagtttg tcctcctcaa tctggtttat gaaacaactg acaaacacct    540
   ttctcctgat ggccagtatg tccccaggat tatgtttgtt gacccatctc tgacagttag    600
   agccgatatc actggaagat attcaaatcg tctctatgct tacgaacctg cagatacagc    660
   tctgttgctt gacaacatga agaaagctct caagttgctg aagactgaat tgtaaagaaa    720
   aaaaatctcc aagcccttct gtctgtcagg ccttgagact tgaaaccaga agaagtgtga    780
   gaagactggc tagtgtggaa gcatagtgaa cacactgatt aggttatggt ttaatgttac    840
   aacaactatt ttttaagaaa aacaagtttt agaaatttgg tttcaagtgt acatgtgtga    900
   aaacaatatt gtatactacc atagtgagcc atgattttct aaaaaaaaaa ataaatgttt    960
   tgggggtgtt ctgttttctc caaaaaaaaa aaaaaa                             996
<210> 3
   <211> 1251
   <212> DNA
   <213> Homo sapiens
<400> 3
   gcccattgtt tttgtaatct ctgaggagaa gcagcagcaa acatttgcta gtcagacaag 60
   tgacagggaa tggattccaa acaccagtgt gtaaagctaa atgatggcca cttcatgcct 120
   gtattgggat ttggcaccta tgcacctcca gaggttccga gaagtaaagc tttggaggtc 180
   acaaaattag caatagaagc tgggttccgc catatagatt ctgctcattt atacaataat 240
   gaggagcagg ttggactggc catccgaagc aagattgcag atggcagtgt gaagagagaa 300
   gacatattct acacttcaaa gctttggtcc acttttcatc gaccagagtt ggtccgacca 360
   gccttggaaa actcactgaa gaaagctcaa ttggactatg ttgacctcta tcttattcat 420
   tctccaatgt ctctaaagcc aggtgaggaa ctttcaccaa cagatgaaaa tggaaaagta 480
   atatttgaca tagtggatct ctgtaccacc tgggaggcca tggagaagtg taaggatgca 540
   ggattggcca agtccattgg ggtgtcaaac ttcaaccgca ggcagctgga gatgatcctc 600
   aacaagccag gactcaagta caagcctgtc tgcaaccagg tagaatgtca tccgtatttc 660
   aaccggagta aattgctaga tttctgcaag tcgaaagata ttgttctggt tgcctatagt 720
   gctctgggat ctcaacgaga caaacgatgg gtggacccga actccccggt gctcttggag 780
   gacccagtcc tttgtgcctt ggcaaaaaag cacaagcgaa ccccagccct gattgccctg 840
   cgctaccagc tgcagcgtgg ggttgtggtc ctggccaaga gctacaatga gcagcgcatc 900
   agacagaacg tgcaggtttt tgagttccag ttgactgcag aggacatgaa agccatagat 960
   ggcctagaca gaaatctcca ctattttaac agtgatagtt ttgctagcca ccctaattat 1020
   ccatattcag atgaatatta acatggaggg ctttgcctga tgtctaccag aagccctgtg 1080
   tgtggatggt gacgcagagg acgtctctat gccggtgact ggacatatca cctctactta 1140
   aatccgtcct gtttagcgac ttcagtcaac tacagctgag tccataggcc agaaagacaa 1200
   taaattttta tcattttgaa ataaaaaaaa aaaaaaaaaa aaaaaaaaaa a 1251
<210> 4
   <211> 464
   <212> DNA
   <213> Homo sapiens
<400> 4
   ctccccagcc tgataaaggt cctgcgggca ggacaggacc tcccaaccaa gccctccagc 60
   aaggattcag agtgcccctc cggcctcgcc atgaggctct tcctgtcgct cccggtcctg 120
   gtggtggttc tgtcgatcgt cttggaaggc ccagccccag cccaggggac cccagacgtc 180
   tccagtgcct tggataagct gaaggagttt ggaaacacac tggaggacaa ggctcgggaa 240
   ctcatcagcc gcatcaaaca gagtgaactt tctgccaaga tgcgggagtg gttttcagag 300
   acatttcaga aagtgaagga gaaactcaag attgactcat gaggacctga agggtgacat 360
   cccaggaggg gcctctgaaa tttcccacac cccagcgcct gtgctgagga ctccctccat 420
   gtggccccag gtgccaccaa taaaaatcct acagaaaatt caaa 464
<210> 5
   <211> 1223
   <212> DNA
   <213> Homo sapiens
<400> 5
   gggatccttg agtcctactc agccccagcg gaggtgaagg acgtccttcc ccaggagccg 60
   actggccaat cacaggcagg aagatgaagg ttctgtgggc tgcgttgctg gtcacattcc 120
   tggcaggatg ccaggccaag gtggagcaag cggtggagac agagccggag cccgagctgc 180
   gccagcagac cgagtggcag agcggccagc gctgggaact ggcactgggt cgcttttggg 240
   attacctgcg ctgggtgcag acactgtctg agcaggtgca ggaggagctg ctcagctccc 300
   aggtcaccca ggaactgagg gcgctgatgg acgagaccat gaaggagttg aaggcctaca     360
   aatcggaact ggaggaacaa ctgaccccgg tggcggagga gacgcgggca cggctgtcca     420
   aggagctgca ggcggcgcag gcccggctgg gcgcggacat ggaggacgtg tgcggccgcc     480
   tggtgcagta ccgcggcgag gtgcaggcca tgctcggcca gagcaccgag gagctgcggg     540
   tgcgcctcgc ctcccacctg cgcaagctgc gtaagcggct cctccgcgat gccgatgacc     600
   tgcagaagcg cctggcagtg taccaggccg gggcccgcga gggcgccgag cgcggcctca     660
   gcgccatccg cgagcgcctg gggcccctgg tggaacaggg ccgcgtgcgg gccgccactg     720
   tgggctccct ggccggccag ccgctacagg agcgggccca ggcctggggc gagcggctgc     780
   gcgcgcggat ggaggagatg ggcagccgga cccgcgaccg cctggacgag gtgaaggagc     840
   aggtggcgga ggtgcgcgcc aagctggagg agcaggccca gcagatacgc ctgcaggccg     900
   aggccttcca ggcccgcctc aagagctggt tcgagcccct ggtggaagac atgcagcgcc     960
   agtgggccgg gctggtggag aaggtgcagg ctgccgtggg caccagcgcc gcccctgtgc    1020
   ccagcgacaa tcactgaacg ccgaagcctg cagccatgcg accccacgcc accccgtgcc    1080
   tcctgcctcc gcgcagcctg cagcgggaga ccctgtcccc gccccagccg tcctcctggg    1140
   gtggacccta gtttaataaa gattcaccaa gtttcacgca aaaaaaaaaa aaaaaaaaaa    1200
   aaaaaaaaaa aaaaaaaaaa aaa                                            1223
<210> 6
   <211> 10661
   <212> DNA
   <213> Homo sapiens
<400> 6
   cgagatcccg gggagccagc ttgctgggag agcgggacgg tccggagcaa gcccagaggc      60
   agaggaggcg acagagggaa aaagggccga gctagccgct ccagtgctgt acaggagccg     120
   aagggacgca ccacgccagc cccagcccgg ctccagcgac agccaacgcc tcttgcagcg     180
   cggcggcttc gaagccgccg cccggagctg ccctttcctc ttcggtgaag tttttaaaag     240
   ctgctaaaga ctcggaggaa gcaaggaaag tgcctggtag gactgacggc tgcctttgtc     300
   ctcctcctct ccaccccgcc tccccccacc ctgccttccc cccctccccc gtcttctctc     360
   ccgcagctgc ctcagtcggc tactctcagc caacccccct caccaccctt ctccccaccc     420
   gcccccccgc ccccgtcggc ccagcgctgc cagcccgagt ttgcagagag gtaactccct     480
   ttggctgcga gcgggcgagc tagctgcaca ttgcaaagaa ggctcttagg agccaggcga     540
   ctggggagcg gcttcagcac tgcagccacg acccgcctgg ttaggctgca cgcggagaga     600
   accctctgtt ttcccccact ctctctccac ctcctcctgc cttccccacc ccgagtgcgg     660
   agccagagat caaaagatga aaaggcagtc aggtcttcag tagccaaaaa acaaaacaaa     720
   caaaaacaaa aaagccgaaa taaaagaaaa agataataac tcagttctta tttgcaccta     780
   cttcagtgga cactgaattt ggaaggtgga ggattttgtt tttttctttt aagatctggg     840
   catcttttga atctaccctt caagtattaa gagacagact gtgagcctag cagggcagat     900
   cttgtccacc gtgtgtcttc ttctgcacga gactttgagg ctgtcagagc gctttttgcg     960
   tggttgctcc cgcaagtttc cttctctgga gcttcccgca ggtgggcagc tagctgcagc    1020
   gactaccgca tcatcacagc ctgttgaact cttctgagca agagaagggg aggcggggta    1080
   agggaagtag gtggaagatt cagccaagct caaggatgga agtgcagtta gggctgggaa    1140
   gggtctaccc tcggccgccg tccaagacct accgaggagc tttccagaat ctgttccaga    1200
   gcgtgcgcga agtgatccag aacccgggcc ccaggcaccc agaggccgcg agcgcagcac    1260
   ctcccggcgc cagtttgctg ctgctgcagc agcagcagca gcagcagcag cagcagcagc    1320
   agcagcagca gcagcagcag cagcagcagc agcaagagac tagccccagg cagcagcagc    1380
   agcagcaggg tgaggatggt tctccccaag cccatcgtag aggccccaca ggctacctgg    1440
   tcctggatga ggaacagcaa ccttcacagc cgcagtcggc cctggagtgc caccccgaga    1500
   gaggttgcgt cccagagcct ggagccgccg tggccgccag caaggggctg ccgcagcagc    1560
   tgccagcacc tccggacgag gatgactcag ctgccccatc cacgttgtcc ctgctgggcc    1620
   ccactttccc cggcttaagc agctgctccg ctgaccttaa agacatcctg agcgaggcca    1680
   gcaccatgca actccttcag caacagcagc aggaagcagt atccgaaggc agcagcagcg    1740
   ggagagcgag ggaggcctcg gggctcccca cttcctccaa ggacaattac ttagggggca     1800
   cttcgaccat ttctgacaac gccaaggagt tgtgtaaggc agtgtcggtg tccatgggcc    1860
   tgggtgtgga ggcgttggag catctgagtc caggggaaca gcttcggggg gattgcatgt    1920
   acgccccact tttgggagtt ccacccgctg tgcgtcccac tccttgtgcc ccattggccg    1980
   aatgcaaagg ttctctgcta gacgacagcg caggcaagag cactgaagat actgctgagt    2040
   attccccttt caagggaggt tacaccaaag ggctagaagg cgagagccta ggctgctctg    2100
   gcagcgctgc agcagggagc tccgggacac ttgaactgcc gtctaccctg tctctctaca    2160
   agtccggagc actggacgag gcagctgcgt accagagtcg cgactactac aactttccac    2220
   tggctctggc cggaccgccg ccccctccgc cgcctcccca tccccacgct cgcatcaagc    2280
   tggagaaccc gctggactac ggcagcgcct gggcggctgc ggcggcgcag tgccgctatg    2340
   gggacctggc gagcctgcat ggcgcgggtg cagcgggacc cggttctggg tcaccctcag    2400
   ccgccgcttc ctcatcctgg cacactctct tcacagccga agaaggccag ttgtatggac    2460
   cgtgtggtgg tggtgggggt ggtggcggcg gcggcggcgg cggcggcggc ggcggcggcg    2520
   gcggcggcgg cggcgaggcg ggagctgtag ccccctacgg ctacactcgg ccccctcagg    2580
   ggctggcggg ccaggaaagc gacttcaccg cacctgatgt gtggtaccct ggcggcatgg    2640
   tgagcagagt gccctatccc agtcccactt gtgtcaaaag cgaaatgggc ccctggatgg    2700
   atagctactc cggaccttac ggggacatgc gtttggagac tgccagggac catgttttgc 2760
   ccattgacta ttactttcca ccccagaaga cctgcctgat ctgtggagat gaagcttctg 2820
   ggtgtcacta tggagctctc acatgtggaa gctgcaaggt cttcttcaaa agagccgctg 2880
   aagggaaaca gaagtacctg tgcgccagca gaaatgattg cactattgat aaattccgaa 2940
   ggaaaaattg tccatcttgt cgtcttcgga aatgttatga agcagggatg actctgggag 3000
   cccggaagct gaagaaactt ggtaatctga aactacagga ggaaggagag gcttccagca 3060
   ccaccagccc cactgaggag acaacccaga agctgacagt gtcacacatt gaaggctatg 3120
   aatgtcagcc catctttctg aatgtcctgg aagccattga gccaggtgta gtgtgtgctg 3180
   gacacgacaa caaccagccc gactcctttg cagccttgct ctctagcctc aatgaactgg 3240
   gagagagaca gcttgtacac gtggtcaagt gggccaaggc cttgcctggc ttccgcaact 3300
   tacacgtgga cgaccagatg gctgtcattc agtactcctg gatggggctc atggtgtttg 3360
   ccatgggctg gcgatccttc accaatgtca actccaggat gctctacttc gcccctgatc 3420
   tggttttcaa tgagtaccgc atgcacaagt cccggatgta cagccagtgt gtccgaatga 3480
   ggcacctctc tcaagagttt ggatggctcc aaatcacccc ccaggaattc ctgtgcatga 3540
   aagcactgct actcttcagc attattccag tggatgggct gaaaaatcaa aaattctttg 3600
   atgaacttcg aatgaactac atcaaggaac tcgatcgtat cattgcatgc aaaagaaaaa 3660
   atcccacatc ctgctcaaga cgcttctacc agctcaccaa gctcctggac tccgtgcagc 3720
   ctattgcgag agagctgcat cagttcactt ttgacctgct aatcaagtca cacatggtga 3780
   gcgtggactt tccggaaatg atggcagaga tcatctctgt gcaagtgccc aagatccttt 3840
   ctgggaaagt caagcccatc tatttccaca cccagtgaag cattggaaac cctatttccc 3900
   caccccagct catgccccct ttcagatgtc ttctgcctgt tataactctg cactactcct 3960
   ctgcagtgcc ttggggaatt tcctctattg atgtacagtc tgtcatgaac atgttcctga 4020
   attctatttg ctgggctttt tttttctctt tctctccttt ctttttcttc ttccctccct 4080
   atctaaccct cccatggcac cttcagactt tgcttcccat tgtggctcct atctgtgttt 4140
   tgaatggtgt tgtatgcctt taaatctgtg atgatcctca tatggcccag tgtcaagttg 4200
   tgcttgttta cagcactact ctgtgccagc cacacaaacg tttacttatc ttatgccacg 4260
   ggaagtttag agagctaaga ttatctgggg aaatcaaaac aaaaacaagc aaacaaaaaa 4320
   aaaaagcaaa aacaaaacaa aaaataagcc aaaaaacctt gctagtgttt tttcctcaaa 4380
   aataaataaa taaataaata aatacgtaca tacatacaca catacataca aacatataga 4440
   aatccccaaa gaggccaata gtgacgagaa ggtgaaaatt gcaggcccat ggggagttac 4500
   tgattttttc atctcctccc tccacgggag actttatttt ctgccaatgg ctattgccat 4560
   tagagggcag agtgacccca gagctgagtt gggcaggggg gtggacagag aggagaggac 4620
   aaggagggca atggagcatc agtacctgcc cacagccttg gtccctgggg gctagactgc 4680
   tcaactgtgg agcaattcat tatactgaaa atgtgcttgt tgttgaaaat ttgtctgcat 4740
   gttaatgcct cacccccaaa cccttttctc tctcactctc tgcctccaac ttcagattga 4800
   ctttcaatag tttttctaag acctttgaac tgaatgttct cttcagccaa aacttggcga 4860
   cttccacaga aaagtctgac cactgagaag aaggagagca gagatttaac cctttgtaag 4920
   gccccatttg gatccaggtc tgctttctca tgtgtgagtc agggaggagc tggagccaga 4980
   ggagaagaaa atgatagctt ggctgttctc ctgcttagga cactgactga atagttaaac 5040
   tctcactgcc actacctttt ccccaccttt aaaagacctg aatgaagttt tctgccaaac 5100
   tccgtgaagc cacaagcacc ttatgtcctc ccttcagtgt tttgtgggcc tgaatttcat 5160
   cacactgcat ttcagccatg gtcatcaagc ctgtttgctt cttttgggca tgttcacaga 5220
   ttctctgtta agagccccca ccaccaagaa ggttagcagg ccaacagctc tgacatctat 5280
   ctgtagatgc cagtagtcac aaagatttct taccaactct cagatcgctg gagcccttag 5340
   acaaactgga aagaaggcat caaagggatc aggcaagctg ggcgtcttgc ccttgtcccc 5400
   cagagatgat accctcccag caagtggaga agttctcact tccttcttta gagcagctaa 5460
   aggggctacc cagatcaggg ttgaagagaa aactcaatta ccagggtggg aagaatgaag 5520
   gcactagaac cagaaaccct gcaaatgctc ttcttgtcac ccagcatatc cacctgcaga 5580
   agtcatgaga agagagaagg aacaaagagg agactctgac tactgaatta aaatcttcag 5640
   cggcaaagcc taaagccaga tggacaccat ctggtgagtt tactcatcat cctcctctgc 5700
   tgctgattct gggctctgac attgcccata ctcactcaga ttccccacct ttgttgctgc 5760
   ctcttagtca gagggaggcc aaaccattga gactttctac agaaccatgg cttctttcgg 5820
   aaaggtctgg ttggtgtggc tccaatactt tgccacccat gaactcaggg tgtgccctgg 5880
   gacactggtt ttatatagtc ttttggcaca cctgtgttct gttgacttcg ttcttcaagc 5940
   ccaagtgcaa gggaaaatgt ccacctactt tctcatcttg gcctctgcct ccttacttag 6000
   ctcttaatct catctgttga actcaagaaa tcaagggcca gtcatcaagc tgcccatttt 6060
   aattgattca ctctgtttgt tgagaggata gtttctgagt gacatgatat gatccacaag 6120
   ggtttccttc cctgatttct gcattgatat taatagccaa acgaacttca aaacagcttt 6180
   aaataacaag ggagagggga acctaagatg agtaatatgc caatccaaga ctgctggaga 6240
   aaactaaagc tgacaggttc cctttttggg gtgggataga catgttctgg ttttctttat 6300
   tattacacaa tctggctcat gtacaggatc acttttagct gttttaaaca gaaaaaaata 6360
   tccaccactc ttttcagtta cactaggtta cattttaata ggtcctttac atctgttttg 6420
   gaatgatttt catcttttgt gatacacaga ttgaattata tcattttcat atctctcctt 6480
   gtaaatacta gaagctctcc tttacatttc tctatcaaat ttttcatctt tatgggtttc 6540
   ccaattgtga ctcttgtctt catgaatata tgtttttcat ttgcaaaagc caaaaatcag 6600
   tgaaacagca gtgtaattaa aagcaacaac tggattactc caaatttcca aatgacaaaa 6660
   ctagggaaaa atagcctaca caagccttta ggcctactct ttctgtgctt gggtttgagt 6720
   gaacaaagga gattttagct tggctctgtt ctcccatgga tgaaaggagg aggatttttt 6780
   ttttcttttg gccattgatg ttctagccaa tgtaattgac agaagtctca ttttgcatgc 6840
   gctctgctct acaaacagag ttggtatggt tggtatactg tactcacctg tgagggactg 6900
   gccactcaga cccacttagc tggtgagcta gaagatgagg atcactcact ggaaaagtca 6960
   caaggaccat ctccaaacaa gttggcagtg ctcgatgtgg acgaagagtg aggaagagaa 7020
   aaagaaggag caccagggag aaggctccgt ctgtgctggg cagcagacag ctgccaggat 7080
   cacgaactct gtagtcaaag aaaagagtcg tgtggcagtt tcagctctcg ttcattgggc 7140
   agctcgccta ggcccagcct ctgagctgac atgggagttg ttggattctt tgtttcatag 7200
   ctttttctat gccataggca atattgttgt tcttggaaag tttattattt ttttaactcc 7260
   cttactctga gaaagggata ttttgaagga ctgtcatata tctttgaaaa aagaaaatct 7320
   gtaatacata tatttttatg tatgttcact ggcactaaaa aatatagaga gcttcattct 7380
   gtcctttggg tagttgctga ggtaattgtc caggttgaaa aataatgtgc tgatgctaga 7440
   gtccctctct gtccatactc tacttctaaa tacatatagg catacatagc aagttttatt 7500
   tgacttgtac tttaagagaa aatatgtcca ccatccacat gatgcacaaa tgagctaaca 7560
   ttgagcttca agtagcttct aagtgtttgt ttcattaggc acagcacaga tgtggccttt 7620
   ccccccttct ctcccttgat atctggcagg gcataaaggc ccaggccact tcctctgccc 7680
   cttcccagcc ctgcaccaaa gctgcatttc aggagactct ctccagacag cccagtaact 7740
   acccgagcat ggcccctgca tagccctgga aaaataagag gctgactgtc tacgaattat 7800
   cttgtgccag ttgcccaggt gagagggcac tgggccaagg gagtggtttt catgtttgac 7860
   ccactacaag gggtcatggg aatcaggaat gccaaagcac cagatcaaat ccaaaactta 7920
   aagtcaaaat aagccattca gcatgttcag tttcttggaa aaggaagttt ctacccctga 7980
   tgcctttgta ggcagatctg ttctcaccat taatcttttt gaaaatcttt taaagcagtt 8040
   tttaaaaaga gagatgaaag catcacatta tataaccaaa gattacattg tacctgctaa 8100
   gataccaaaa ttcataaggg caggggggga gcaagcatta gtgcctcttt gataagctgt 8160
   ccaaagacag actaaaggac tctgctggtg actgacttat aagagctttg tgggtttttt 8220
   tttccctaat aatatacatg tttagaagaa ttgaaaataa tttcgggaaa atgggattat 8280
   gggtccttca ctaagtgatt ttataagcag aactggcttt ccttttctct agtagttgct 8340
   gagcaaattg ttgaagctcc atcattgcat ggttggaaat ggagctgttc ttagccactg 8400
   tgtttgctag tgcccatgtt agcttatctg aagatgtgaa acccttgctg ataagggagc 8460
   atttaaagta ctagattttg cactagaggg acagcaggca gaaatcctta tttctgccca 8520
   ctttggatgg cacaaaaagt tatctgcagt tgaaggcaga aagttgaaat acattgtaaa 8580
   tgaatatttg tatccatgtt tcaaaattga aatatatata tatatatata tatatatata 8640
   tatatatata tagtgtgtgt gtgtgttctg atagctttaa ctttctctgc atctttatat 8700
   ttggttccag atcacacctg atgccatgta cttgtgagag aggatgcagt tttgttttgg 8760
   aagctctctc agaacaaaca agacacctgg attgatcagt taactaaaag ttttctcccc 8820
   tattgggttt gacccacagg tcctgtgaag gagcagaggg ataaaaagag tagaggacat 8880
   gatacattgt actttactag ttcaagacag atgaatgtgg aaagcataaa aactcaatgg 8940
   aactgactga gatttaccac agggaaggcc caaacttggg gccaaaagcc tacccaagtg 9000
   attgaccagt ggccccctaa tgggacctga gctgttggaa gaagagaact gttccttggt 9060
   cttcaccatc cttgtgagag aagggcagtt tcctgcattg gaacctggag caagcgctct 9120
   atctttcaca caaattccct cacctgagat tgaggtgctc ttgttactgg gtgtctgtgt 9180
   gctgtaattc tggttttgga tatgttctgt aaagattttg acaaatgaaa atgtgttttt 9240
   ctctgttaaa acttgtcaga gtactagaag ttgtatctct gtaggtgcag gtccatttct 9300
   gcccacaggt agggtgtttt tctttgatta agagattgac acttctgttg cctaggacct 9360
   cccaactcaa ccatttctag gtgaaggcag aaaaatccac attagttact cctcttcaga 9420
   catttcagct gagataacaa atcttttgga attttttcac ccatagaaag agtggtagat 9480
   atttgaattt agcaggtgga gtttcatagt aaaaacagct tttgactcag ctttgattta 9540
   tcctcatttg atttggccag aaagtaggta atatgcattg attggcttct gattccaatt 9600
   cagtatagca aggtgctagg ttttttcctt tccccacctg tctcttagcc tggggaatta 9660
   aatgagaagc cttagaatgg gtggcccttg tgacctgaaa cacttcccac ataagctact 9720
   taacaagatt gtcatggagc tgcagattcc attgcccacc aaagactaga acacacacat 9780
   atccatacac caaaggaaag acaattctga aatgctgttt ctctggtggt tccctctctg 9840
   gctgctgcct cacagtatgg gaacctgtac tctgcagagg tgacaggcca gatttgcatt 9900
   atctcacaac cttagccctt ggtgctaact gtcctacagt gaagtgcctg gggggttgtc 9960
   ctatcccata agccacttgg atgctgacag cagccaccat cagaatgacc cacgcaaaaa 10020
   aaagaaaaaa aaaattaaaa agtcccctca caacccagtg acacctttct gctttcctct 10080
   agactggaac attgattagg gagtgcctca gacatgacat tcttgtgctg tccttggaat 10140
   taatctggca gcaggaggga gcagactatg taaacagaga taaaaattaa ttttcaatat 10200
   tgaaggaaaa aagaaataag aagagagaga gaaagaaagc atcacacaaa gattttctta 10260
   aaagaaacaa ttttgcttga aatctcttta gatggggctc atttctcacg gtggcacttg 10320
   gcctccactg ggcagcagga ccagctccaa gcgctagtgt tctgttctct ttttgtaatc 10380
   ttggaatctt ttgttgctct aaatacaatt aaaaatggca gaaacttgtt tgttggacta 10440
   catgtgtgac tttgggtctg tctctgcctc tgctttcaga aatgtcatcc attgtgtaaa 10500
   atattggctt actggtctgc cagctaaaac ttggccacat cccctgttat ggctgcagga 10560
   tcgagttatt gttaacaaag agacccaaga aaagctgcta atgtcctctt atcattgttg 10620
   ttaatttgtt aaaacataaa gaaatctaaa atttcaaaaa a 10661
<210> 7
   <211> 929
   <212> DNA
   <213> Homo sapiens
<400> 7
   cggttgtggt cgctatatat aaggtgggga ggccgccggc ccgttcggtt ccgggcgtta      60
   ccatcgtccg tgcgcaccgc ccggcgtcca ggtgagtctc ccatctgcag agacgcggac     120
   gcgccggccc gcagttggcc tgcggagcgc ggtggacggt ttggcgccca ccaggcgatc     180
   aatactttgg atttttaatt tctagatttg gcaattcttc gctgaagtca tcatgagctt     240
   tttccaactc ctgatgaaaa ggaaggaact cattcccttg gtggtgttca tgactgtggc     300
   ggcgggtgga gcctcatctt tcgctgtgta ttctctttgg aaaaccgatg tgatccttga     360
   tcgaaaaaaa aatccagaac cttgggaaac tgtggaccct actgtacctc aaaagcttat     420
   aacaatcaac caacaatgga aacccattga agagttgcaa aatgtccaaa gggtgaccaa     480
   atgacgagcc ctcgcctctt tcttctgaag agtactctat aaatctagtg gaaacatttc     540
   tgcacaaact agattctgga caccagtgtg cggaaatgct tctgctacat ttttagggtt     600
   tgtctacatt ttttgggctc tggataagga attaaaggag tgcagcaata actgcactgt     660
   ctaaaagttt gtgcttattt tcttgtaaat ttgaatattg catattgaaa tttttgttta     720
   tgatctatga atgtttttct taaaatttac aaagctttgt aaattagatt ttctttaata     780
   aaatgccatt tgtgcaagat ttctcaaaga ttaggtatat atttaaatgg aagagaaaat     840
   atttttatgg gagaaaaata catttgaacc atgaaatttc atcttttaaa taacatccag     900
   tacagatttc tgtgtaaaaa aaaaaaaaa                                       929
<210> 8
   <211> 2231
   <212> DNA
   <213> Homo sapiens
<400> 8
   aaaacaggaa ataggtgttt catatatacg gctctaacct tctctctctg caccttcctt 60
   ctgtcaatag atgaaacaaa tacttcatcc tgctctggaa accactgatc cctgttccac 120
   cggttttgtt ttcccagcaa tgacattatt cccagtgctg ttgttcctgg ttgctgggct 180
   gcttccatct tttccagcaa atgaagataa ggatcccgct tttactgctt tgttaaccac 240
   ccaaacacaa gtgcaaaggg agattgtgaa taagcacaat gaactgagga gagcagtatc 300
   tccccctgcc agaaacatgc tgaagatgga atggaacaaa gaggctgcag caaatgccca 360
   aaagtgggca aaccagtgca attacagaca cagtaaccca aaggatcgaa tgacaagtct 420
   aaaatgtggt gagaatctct acatgtcaag tgcctccagc tcatggtcac aagcaatcca 480
   aagctggttt gatgagtaca atgattttga ctttggtgta gggccaaaga ctcccaacgc 540
   agtggttgga cattatacac aggttgtttg gtactcttca tacctcgttg gatgtggaaa 600
   tgcctactgt cccaatcaaa aagttctaaa atactactat gtttgccaat attgtcctgc 660
   tggtaattgg gctaatagac tatatgtccc ttatgaacaa ggagcacctt gtgccagttg 720
   cccagataac tgtgacgatg gactatgcac caatggttgc aagtacgaag atctctatag 780
   taactgtaaa agtttgaagc tcacattaac ctgtaaacat cagttggtca gggacagttg 840
   caaggcctcc tgcaattgtt caaacagcat ttattaaata cgcattacac accgagtagg 900
   gctatgtaga gaggagtcag attatctact tagatttggc atctacttag atttaacata 960
   tactagctga gaaattgtag gcatgtttga tacacatttg atttcaaatg tttttcttct 1020
   ggatctgctt tttattttac aaaaatattt ttcatacaaa tggttaaaaa gaaacaaaat 1080
   ctataacaac aactttggat ttttatatat aaactttgtg atttaaattt actgaattta 1140
   attagggtga aaattttgaa agttgtattc tcatatgact aagttcacta aaaccctgga 1200
   ttgaaagtga aaattatgtt cctagaacaa aatgtacaaa aagaacaata taattttcac 1260
   atgaaccctt ggctgtagtt gcctttccta gctccactct aaggctaagc atcttcaaag 1320
   acgttttccc atatgctgtc ttaattcttt tcactcattc acccttcttc ccaatcatct 1380
   ggctggcatc ctcacaattg agttgaagct gttcctccta aaacaatcct gacttttatt 1440
   ttgccaaaat caatacaatc ctttgaattt tttatctgca taaattttac agtagaatat 1500
   gatcaaacct tcatttttaa acctctcttc tctttgacaa aacttcctta aaaaagaata 1560
   caagataata taggtaaata ccctccactc aaggaggtag aactcagtcc tctcccttgt 1620
   gagtcttcac taaaatcagt gactcacttc caaagagtgg agtatggaaa gggaaacata 1680
   gtaactttac aggggagaaa aatgacaaat gacgtcttca ccaagtgatc aaaattaacg 1740
   tcaccagtga taagtcattc agatttgttc tagataatct ttctaaaaat tcataatccc 1800
   aatctaatta tgagctaaaa catccagcaa actcaagttg aaggacattc tacaaaatat 1860
   ccctggggta ttttagagta ttcctcaaaa ctgtaaaaat catggaaaat aagggaatcc 1920
   tgagaaacaa tcacagacca catgagacta aggagacatg tgagccaaat gcaatgtgct 1980
   tcttggatca gatcctggaa cagaaaaaga tcagtaatga aaaaactgat gaagtctgaa 2040
   tagaatctgg agtattttta acagtagtgt tgatttctta atcttgataa atatagcagg 2100
   gtaatgtaag atgataacgt tagagaaact gaaactgggt gagggctatc taggaattct 2160
   ctgtactatc ttaccaaatt ttcggtaagt ctaagaaagc aatgcaaaat aaaaagtgtc 2220
   ttgaaaaaaa a 2231
<210> 9
   <211> 1975
   <212> DNA
   <213> Homo sapiens
<400> 9
   aactgtcact gtggagagga gagagagagg acagagagca agtcactccc ggctgccttt      60
   ttcacctctg acagagccca gacaccatga acgcaagtga attccgaagg agagggaagg     120
   agatggtgga ttacgtggcc aactacatgg aaggcattga gggacgccag gtctaccctg     180
   acgtggagcc cgggtacctg cggccgctga tccctgccgc tgcccctcag gagccagaca     240
   cgtttgagga catcatcaac gacgttgaga agataatcat gcctggggtg acgcactggc     300
   acagccccta cttcttcgcc tacttcccca ctgccagctc gtacccggcc atgcttgcgg     360
   acatgctgtg cggggccatt ggctgcatcg gcttctcctg ggcggcaagc ccagcatgca     420
   cagagctgga gactgtgatg atggactggc tcgggaagat gctggaacta ccaaaggcat     480
   ttttgaatga gaaagctgga gaagggggag gagtgatcca gggaagtgcc agtgaagcca     540
   ccctggtggc cctgctggcc gctcggacca aagtgatcca tcggctgcag gcagcgtccc     600
   cagagctcac acaggccgct atcatggaga agctggtggc ttactcatcc gatcaggcac     660
   actcctcagt ggaaagagct gggttaattg gtggagtgaa attaaaagcc atcccctcag     720
   atggcaactt cgccatgcgt gcgtctgccc tgcaggaagc cctggagaga gacaaagcgg     780
   ctggcctgat tcctttcttt atggttgcca ccctggggac cacaacatgc tgctcctttg     840
   acaatctctt agaagtcggt cctatctgca acaaggaaga catatggctg cacgttgatg     900
   cagcctacgc aggcagtgca ttcatctgcc ctgagttccg gcaccttctg aatggagtgg     960
   agtttgcaga ttcattcaac tttaatcccc acaaatggct attggtgaat tttgactgtt    1020
   ctgccatgtg ggtgaaaaag agaacagact taacgggagc ctttagactg gaccccactt    1080
   acctgaagca cagccatcag gattcagggc ttatcactga ctaccggcat tggcagatac    1140
   cactgggcag aagatttcgc tctttgaaaa tgtggtttgt atttaggatg tatggagtca    1200
   aaggactgca ggcttatatc cgcaagcatg tccagctgtc ccatgagttt gagtcactgg    1260
   tgcgccagga tccccgcttt gaaatctgtg tggaagtcat tctggggctt gtctgctttc    1320
   ggctaaaggg ttccaacaaa gtgaatgaag ctcttctgca aagaataaac agtgccaaaa    1380
   aaatccactt ggttccatgt cacctcaggg acaagtttgt cctgcgcttt gccatctgtt    1440
   ctcgcacggt ggaatctgcc catgtgcagc gggcctggga acacatcaaa gagctggcgg    1500
   ccgacgtgct gcgagcagag agggagtagg agtgaagcca gctgcaggaa tcaaaaattg    1560
   aagagagata tatctgaaaa ctggaataag aagcaaataa atatcatcct gccttcatgg    1620
   aactcagctg tctgtggctt cccatgtctt tctccaaagt tatccagagg gttgtgattt    1680
   tgtctgctta gtatctcatc aacaaagaaa tattatttgc taattaaaaa gttaatcttc    1740
   atggccatag cttttattca ttagctgtga tttttgttga ttaaaacatt atagattttc    1800
   atgttcttgc agtcatcaga agtggtagga aagcctcact gatatatttt ccagggcaat    1860
   caatgttcac gcaacttgaa attatatctg tggtcttcaa attgtctttt gtcatgtggc    1920
   taaatgccta ataaacaatt caagtgaaat actaaaaaaa aaaaaaaaaa aaaaa         1975
<210> 10
   <211> 6114
   <212> DNA
   <213> Homo sapiens
<400> 10
   gtcatgcata cattagagcc tctgatgagt ttgtctttgg ggactctggg gctgtactac      60
   ccagtgtcat cacaaattac caggagaaat tgcttccagc tcacaatcac atctgctttt     120
   ggcaagaact aatgcaccaa gacttcaagt tctaagcctc tgttcagatt ttaattgcaa     180
   ttgatcaggt ttatattatt gtacctccag agacctccta gagccagaac ccggctggct     240
   tgctgtttcc tttagagcag cgcatatcat tatttggtgt tctggtggag gacttttctg     300
   atggcagaaa ttagtttctc tgggttcatc aggacgggat gcttcaagat ttaagtgcaa     360
   gtgtcttctt tccaccttgt tcacaacaca gaacgttagc tcaggtacct gacaatgatg     420
   agcagtttgt accagactat caggctgaaa gtttggcttt tcatggcctg ccactgaaaa     480
   tcaagaaaga accccacagt ccatgttcag aaatcagctc tgcctgcagt caagaacagc     540
   cctttaaatt cagctatgga gaaaagtgcc tgtacaatgt cagatttcgc cgccagcttt     600
   ctgaaccctg taactccttt cctcctttgc cgacgatgcc aagggaagga cgtcctatgt     660
   accaacgcca gatgtctgag ccaaacatcc ccttcccacc acaaggcttt aagcaggagt     720
   accacgaccc agtgtatgaa cacaacacca tggttggcag tgcggccagc caaagctttc     780
   cccctcctct gatgattaaa caggaaccca gagattttgc atatgactca gaagtgccta     840
   gctgccactc catttatatg aggcaagaag gcttcctggc tcatcccagc agaacagaag     900
   gctgtatgtt tgaaaagggc cccaggcagt tttatgatga cacctgtgtt gtcccagaaa     960
   aattcgatgg agacatcaaa caagagccag gaatgtatcg ggaaggaccc acataccaac    1020
   ggcgaggatc acttcagctc tggcagtttt tggtagctct tctggatgac ccttcaaatt    1080
   ctcattttat tgcctggact ggtcgaggca tggaatttaa actgattgag cctgaagagg    1140
   tggcccgacg ttggggcatt cagaaaaaca ggccagctat gaactatgat aaacttagcc    1200
   gttcactccg ctattactat gagaaaggaa ttatgcaaaa ggtggctgga gagagatatg    1260
   tctacaagtt tgtgtgtgat ccagaagccc ttttctccat ggcctttcca gataatcagc    1320
   gtccactgct gaagacagac atggaacgtc acatcaacga ggaggacaca gtgcctcttt 1380
   ctcactttga tgagagcatg gcctacatgc cggaaggggg ctgctgcaac ccccacccct 1440
   acaacgaagg ctacgtgtat taacacaagt gacagtcaag cagggcgttt ttgcgctttt 1500
   ccttttttct gcaagataca gagaattgct gaatctttgt tttatttctg ttgtttgtat 1560
   tttattttta aataataata cacaaaaagg ggcttttcct gttgcattat tctatggtct 1620
   gccatggact gtgcacttta tttgagggtg ggtgggagta atctaaacat ttattctgtg 1680
   taacaggaag ctaatgggtg aatgggcaga gggatttggg gattactttt tacttaggct 1740
   tgggatgggg tcctacaagt tttgagtatg atgaaactat atcatgtctg tttgatttca 1800
   taacaacata agataatgtt tattttatcg gggtatctat ggtacagtta atttcacgtt 1860
   gtgtaaatat ccacttggag actatttgcc ttgggcattt tcccctgtca tttatgagtc 1920
   tctgcaggtg tacaaaaaaa ccccaatcta ctgtaaatgg cagtttaatt gttagaaatg 1980
   actgtttttg caccacttgt aaaaaggtat ttagcgattg catttgctgt ttgttgtttt 2040
   attttgcttt atatatgact tgcagaggat aaccataaaa tgggtaattc tctctgaagt 2100
   tgaataatca ccatgactgt aaatgagggg cacaattttg gactctggcg ccaaactgag 2160
   tcataggcca gtagcattac gtgtatctgg tgccaccttg ctgtttagat acaaatcata 2220
   ccgtctttta aatattttga agcccatttc agttaaataa tgacatgtca tggtcctttg 2280
   gaatcttcat ttaaatgtta aatctggaat caaaatgaag caaaaaatat ctgtctcctt 2340
   ttcactttct tcagtacata aatacattat ttaatcaata agaattaact gtactaaatc 2400
   atgtattatg ctgttctagt tacagcaaac actctttaag aaaaatatcc aatacactaa 2460
   ataggtacta tagtaatttt tagacatggt acccattgat atgcatttaa accttttact 2520
   gctgtgttat gttgataaca tatataaata ttagataatg ctaatgcttc tgctgctgtc 2580
   ttttctgtaa tattctcttt catgctgaat ttactatgac catttataag cagtgcagtt 2640
   aactacagat agcatttcag gacaaaatag atgactcaaa ccatttattg cttaaaaaat 2700
   agcttacgcc atgctatgct ataagcagct tttatgcaca ttgacaaatg aagagtaagc 2760
   ttcagcttgc taaaggaaac tgtggaacct tttgtaactt ttggtgatat ggaaaattat 2820
   ttacaaaccg tcaaagaata tgaggaagtt gctgtatgac atagtgctgg cactgatatt 2880
   atccatcatc tctttttgga cacttctgta aatgtgattg gattgtttga aagaagattt 2940
   aaagtttcaa agttttttgt tctgtttttg ctttgcattt ggagaaaata ttgaaagcag 3000
   ggtatgttgt ttcattcacc ttgaaaaaac catgagtaaa tggggatata gaatctctga 3060
   atagctcgct aaaagattca agcaagggac atgaattttg ttccatctat caataatatc 3120
   cagaagaaca acttttttaa agagtctata gcaaaaagca aaaaaaaaaa aaaattctaa 3180
   acacaaagtc aaaataaacc tattgtaaaa gcatttcgtg atgagcatga aaaagattgt 3240
   ttaaagatga tccccccagc tacccatttt ccaaaactac acagatcaca gctcatttct 3300
   ctaagtggag cagttatcaa gaaacccaaa caccaaaatt gctactcttc acatttaatc 3360
   ctacaaaaag tactccaatt tcaaaatatg tatgtaacct gcgatttcaa tgattgttgt 3420
   tcatatacat catgtattat tttggcccat tttgggccta aaaaagaaaa ctatgcctta 3480
   aaaatcagaa ccttttctcc ccactatgct tatgtggcca tctacagcac ttagaataaa 3540
   aacagatgtt aaaatattca gtgaaagttt tattggaaaa aggaattgag atatataatt 3600
   gagatttggt gaaattgaag gagaaaattt aagtgagtct ttaaaatata ttctgaatga 3660
   aaactgtatt gaggattcat ttttgttcct tttttttctt tttctctttt ctcctttttc 3720
   ttctttttaa tagtctagtt ttagtcagtc agtgaggaag aattgggcca tgctaacgtt 3780
   atcacaagag aacaatggca gaaatggtat tagttatata atatttaagg acaaactata 3840
   tgttttgctg ttttaacgta gtgactcact gaactaaata cataattgac caacattaag 3900
   tgtatttcca atacagaagg gttgaaaata ttacattata aactcttttg aaaaatgtat 3960
   ctaaaatttt ttaagttctg ttttgattcc actttttggt tgagttttta tgtttttgtt 4020
   ttcaggtaga ttaataaatc tggcagctga tttctgcaag attcttgtgt tttgaatttc 4080
   tcattgaatt ggctactcaa acatagaaat catttgttaa tgatgtaatg tcttctctca 4140
   gcttttatct tcactgctgt ttgctgtctc ttgatgatga catgttaata cccaatagat 4200
   taattgcaac aaacacttat actcaaataa ctaagtaaaa ataatttttc ttgttatgtc 4260
   catgaaaagt gcttcagaat aaaaatccac aagactgaca gtgcagaaca tttttctcaa 4320
   atcatgggcg gatcttggag gtctagtttc ccgtagatgc tgtaaccaat taccacaact 4380
   tcagtaattt acacaaattt atcttatagt tctggaggca gaagttcaaa agaagcctta 4440
   agagactaaa accaagatgt ccttaggtct ggttccttct ggaggctcca ggggagattc 4500
   ttccagcttt cacttctaga gtctgctgac attccttggc tcctggctac atcacttcaa 4560
   tctctgcttc catggtcaca tactcttcta ctatagtcaa atttccttcc tgcctcttat 4620
   aaggatgctt gtgattacat ttaggggatg ctcagataat ccaggacaat ctctccatct 4680
   caagatcctt aacttaatga cgtgtgccaa gtccctttgg ctagataatt attcataggt 4740
   cccagggatt aggacatgga tgtaaggggt gagggcaggg ctgttattca gaacaccgca 4800
   cggaggagga agactgtgta gcaaagactc taattgattt actcaggaac agtggagttc 4860
   tgctgaggga tctaggattt gaaagtacta gagtttgctt ttatttacca ctgagatatt 4920
   ttccccttat tctgcataaa taattttgaa aactttctat attaaatttc aactattcca 4980
   ctaaaatgtc tggtaatcac atcaagcctt tagattattc aaatccttcc ccagccccca 5040
   ggaaaacact aagtcatgaa acagaaaaac agaaggtatg ataataatag taataacagt 5100
   taaatcagtg gtctaatcca gattttattt tttaatacat ttcttttggt gttaatatgg 5160
   gttactatgt gatcttatca tttgctagtg attattactt attaggtaag aacaatgtgt 5220
   aaaatatgtc tattactcaa aagaacaatt gcaaaatgag tcaacttatc tttatataac 5280
   caggaaagaa atatattgcc agaagctaca gaattttgcc agatgatagg gatttctaaa 5340
   atgagccact ttgtctatca tgcagccttt tcagagcttg taatgagaaa acattacaga 5400
   ggagaaggtc atttggatgt ttgttacttg gaatcctaga aaacaaaaac taaaatttaa 5460
   aaataagaag tgagtaagct attttccatt tgcgatttgg tatggagaag agaggaaata 5520
   gaattattaa aaaaatacaa attgggtaaa agtgatggtg gaaaaaatat aaagaaggca 5580
   aatgtacata ttaagcaatt ctactaagaa ttggaaaaat caagtttcaa aaagatggta 5640
   atagttgggc atgatactag aaaatttcac ccagtttatt cagagctcaa ctagtacttt 5700
   taggacttct ttttttatat acatgagact cactttgaca tacttaaaaa aaaaacagtt 5760
   tatggaaagt acagtttaag aggagaattt gattagacta agtggatatc tttatagaaa 5820
   tattaatgat ttcagaattt tcagttacaa gtgtatatac cgtggctatt gtttatggat 5880
   tcatatgtaa ggtagggtct tttttgcata tagactccag tattagttac tttcattcta 5940
   aaattatatt tatgcttcta tggggaagaa aatttttaat tcacttggtt gtattaaaat 6000
   tatacttacg gtttgagaaa acatgctatg aaaatcatga ttatagcaaa ttaaatatgc 6060
   tcaaaattta aatctaaaat aaaagcccag aaactgaaaa aaaaaaaaaa aaaa 6114
<210> 11
   <211> 2298
   <212> DNA
   <213> Homo sapiens
<400> 11
   cacgttctta tgtaaccgag cccgggtaaa gcagggctgc agaaagcaga aacggcgagc 60
   ccggctcctg ggagcaggtc tcggcccccg cttggggccc cggccgtgcg gccggaggga 120
   gcggccggat ggagcggagg atgaaagccg gatacttgga ccagcaagtg ccctacacct 180
   tcagcagcaa atcgcccgga aatgggagct tgcgcgaagc gctgatcggc ccgctgggga 240
   agctcatgga cccgggctcc ctgccgcccc tcgactctga agatctcttc caggatctaa 300
   gtcacttcca ggagacgtgg ctcgctgaag ctcaggtacc agacagtgat gagcagtttg 360
   ttcctgattt ccattcagaa aacctagctt tccacagccc caccaccagg atcaagaagg 420
   agccccagag tccccgcaca gacccggccc tgtcctgcag caggaagccg ccactcccct 480
   accaccatgg cgagcagtgc ctttactcca gtgcctatga cccccccaga caaatcgcca 540
   tcaagtcccc tgcccctggt gcccttggac agtcgcccct acagcccttt ccccgggcag 600
   agcaacggaa tttcctgaga tcctctggca cctcccagcc ccaccctggc catgggtacc 660
   tcggggaaca tagctccgtc ttccagcagc ccctggacat ttgccactcc ttcacatctc 720
   agggaggggg ccgggaaccc ctcccagccc cctaccaaca ccagctgtcg gagccctgcc 780
   caccctatcc ccagcagagc tttaagcaag aataccatga tcccctgtat gaacaggcgg 840
   gccagccagc cgtggaccag ggtggggtca atgggcacag gtacccaggg gcgggggtgg 900
   tgatcaaaca ggaacagacg gacttcgcct acgactcaga tgtcaccggg tgcgcatcaa 960
   tgtacctcca cacagagggc ttctctgggc cctctccagg tgacggggcc atgggctatg 1020
   gctatgagaa acctctgcga ccattcccag atgatgtctg cgttgtccct gagaaatttg 1080
   aaggagacat caagcaggaa ggggtcggtg catttcgaga ggggccgccc taccagcgcc 1140
   ggggtgccct gcagctgtgg caatttctgg tggccttgct ggatgaccca acaaatgccc 1200
   atttcattgc ctggacgggc cggggaatgg agttcaagct cattgagcct gaggaggtcg 1260
   ccaggctctg gggcatccag aagaaccggc cagccatgaa ttacgacaag ctgagccgct 1320
   cgctccgata ctattatgag aaaggcatca tgcagaaggt ggctggtgag cgttacgtgt 1380
   acaagtttgt gtgtgagccc gaggccctct tctctttggc cttcccggac aatcagcgtc 1440
   cagctctcaa ggctgagttt gaccggcctg tcagtgagga ggacacagtc cctttgtccc 1500
   acttggatga gagccccgcc tacctcccag agctggctgg ccccgcccag ccatttggcc 1560
   ccaagggtgg ctactcttac tagcccccag cggctgttcc ccctgccgca ggtgggtgct 1620
   gccctgtgta catataaatg aatctggtgt tggggaaacc ttcatctgaa acccacagat 1680
   gtctctgggg cagatcccca ctgtcctacc agttgcccta gcccagactc tgagctgctc 1740
   accggagtca ttgggaagga aaagtggaga aatggcaagt ctagagtctc agaaactccc 1800
   ctgggggttt cacctgggcc ctggaggaat tcagctcagc ttcttcctag gtccaagccc 1860
   cccacacctt ttccccaacc acagagaaca agagtttgtt ctgttctggg ggacagagaa 1920
   ggcgcttccc aacttcatac tggcaggagg gtgaggaggt tcactgagct ccccagatct 1980
   cccactgcgg ggagacagaa gcctggactc tgccccacgc tgtggccctg gagggtcccg 2040
   gtttgtcagt tcttggtgct ctgtgttccc agaggcaggc ggaggttgaa gaaaggaacc 2100
   tgggatgagg ggtgctgggt ataagcagag agggatgggt tcctgctcca aggacccctt 2160
   tgcctttctt ctgccctttc ctaggcccag gcctgggttt gtacttccac ctccaccaca 2220
   tctgccagac cttaataaag gcccccactt ctcccattaa aaaaaaaaaa aaaaaaaaaa 2280
   aaaaaaaaaa aaaaaaaa 2298
<210> 12
   <211> 9179
   <212> DNA
   <213> Homo sapiens
<400> 12
   gcaagaactg caggggagga ggacgctgcc acccacagcc tctagagctc attgcagctg 60
   ggacagcccg gagtgtggtt agcagctcgg caagcgctgc ccaggtcctg gggtggtggc 120
   agccagcggg agcaggaaag gaagcatgtt cccaggctgc ccacgcctct gggtcctggt 180
   ggtcttgggc accagctggg taggctgggg gagccaaggg acagaagcgg cacagctaag 240
   gcagttctac gtggctgctc agggcatcag ttggagctac cgacctgagc ccacaaactc 300
   aagtttgaat ctttctgtaa cttcctttaa gaaaattgtc tacagagagt atgaaccata 360
   ttttaagaaa gaaaaaccac aatctaccat ttcaggactt cttgggccta ctttatatgc 420
   tgaagtcgga gacatcataa aagttcactt taaaaataag gcagataagc ccttgagcat 480
   ccatcctcaa ggaattaggt acagtaaatt atcagaaggt gcttcttacc ttgaccacac 540
   attccctgcg gagaagatgg acgacgctgt ggctccaggc cgagaataca cctatgaatg 600
   gagtatcagt gaggacagtg gacccaccca tgatgaccct ccatgcctca cacacatcta 660
   ttactcccat gaaaatctga tcgaggattt caactcgggg ctgattgggc ccctgcttat 720
   ctgtaaaaaa gggaccctaa ctgagggtgg gacacagaag acgtttgaca agcaaatcgt 780
   gctactattt gctgtgtttg atgaaagcaa gagctggagc cagtcatcat ccctaatgta 840
   cacagtcaat ggatatgtga atgggacaat gccagatata acagtttgtg cccatgacca 900
   catcagctgg catctgctgg gaatgagctc ggggccagaa ttattctcca ttcatttcaa 960
   cggccaggtc ctggagcaga accatcataa ggtctcagcc atcacccttg tcagtgctac 1020
   atccactacc gcaaatatga ctgtgggccc agagggaaag tggatcatat cttctctcac 1080
   cccaaaacat ttgcaagctg ggatgcaggc ttacattgac attaaaaact gcccaaagaa 1140
   aaccaggaat cttaagaaaa taactcgtga gcagaggcgg cacatgaaga ggtgggaata 1200
   cttcattgct gcagaggaag tcatttggga ctatgcacct gtaataccag cgaatatgga 1260
   caaaaaatac aggtctcagc atttggataa tttctcaaac caaattggaa aacattataa 1320
   gaaagttatg tacacacagt acgaagatga gtccttcacc aaacatacag tgaatcccaa 1380
   tatgaaagaa gatgggattt tgggtcctat tatcagagcc caggtcagag acacactcaa 1440
   aatcgtgttc aaaaatatgg ccagccgccc ctatagcatt taccctcatg gagtgacctt 1500
   ctcgccttat gaagatgaag tcaactcttc tttcacctca ggcaggaaca acaccatgat 1560
   cagagcagtt caaccagggg aaacctatac ttataagtgg aacatcttag agtttgatga 1620
   acccacagaa aatgatgccc agtgcttaac aagaccatac tacagtgacg tggacatcat 1680
   gagagacatc gcctctgggc taataggact acttctaatc tgtaagagca gatccctgga 1740
   caggcgagga atacagaggg cagcagacat cgaacagcag gctgtgtttg ctgtgtttga 1800
   tgagaacaaa agctggtacc ttgaggacaa catcaacaag ttttgtgaaa atcctgatga 1860
   ggtgaaacgt gatgacccca agttttatga atcaaacatc atgagcacta tcaatggcta 1920
   tgtgcctgag agcataacta ctcttggatt ctgctttgat gacactgtcc agtggcactt 1980
   ctgtagtgtg gggacccaga atgaaatttt gaccatccac ttcactgggc actcattcat 2040
   ctatggaaag aggcatgagg acaccttgac cctcttcccc atgcgtggag aatctgtgac 2100
   ggtcacaatg gataatgttg gaacttggat gttaacttcc atgaattcta gtccaagaag 2160
   caaaaagctg aggctgaaat tcagggatgt taaatgtatc ccagatgatg atgaagactc 2220
   atatgagatt tttgaacctc cagaatctac agtcatggct acacggaaaa tgcatgatcg 2280
   tttagaacct gaagatgaag agagtgatgc tgactatgat taccagaaca gactggctgc 2340
   agcattagga atcaggtcat tccgaaactc atcattgaat caggaagaag aagagttcaa 2400
   tcttactgcc ctagctctgg agaatggcac tgaattcgtt tcttcaaaca cagatataat 2460
   tgttggttca aattattctt ccccaagtaa tattagtaag ttcactgtca ataaccttgc 2520
   agaacctcag aaagcccctt ctcaccaaca agccaccaca gctggttccc cactgagaca 2580
   cctcattggc aagaactcag ttctcaattc ttccacagca gagcattcca gcccatattc 2640
   tgaagaccct atagaggatc ctctacagcc agatgtcaca gggatacgtc tactttcact 2700
   tggtgctgga gaattcaaaa gtcaagaaca tgctaagcat aagggaccca aggtagaaag 2760
   agatcaagca gcaaagcaca ggttctcctg gatgaaatta ctagcacata aagttgggag 2820
   acacctaagc caagacactg gttctccttc cggaatgagg ccctgggagg accttcctag 2880
   ccaagacact ggttctcctt ccagaatgag gccctggaag gaccctccta gtgatctgtt 2940
   actcttaaaa caaagtaact catctaagat tttggttggg agatggcatt tggcttctga 3000
   gaaaggtagc tatgaaataa tccaagatac tgatgaagac acagctgtta acaattggct 3060
   gatcagcccc cagaatgcct cacgtgcttg gggagaaagc acccctcttg ccaacaagcc 3120
   tggaaagcag agtggccacc caaagtttcc tagagttaga cataaatctc tacaagtaag 3180
   acaggatgga ggaaagagta gactgaagaa aagccagttt ctcattaaga cacgaaaaaa 3240
   gaaaaaagag aagcacacac accatgctcc tttatctccg aggacctttc accctctaag 3300
   aagtgaagcc tacaacacat tttcagaaag aagacttaag cattcgttgg tgcttcataa 3360
   atccaatgaa acatctcttc ccacagacct caatcagaca ttgccctcta tggattttgg 3420
   ctggatagcc tcacttcctg accataatca gaattcctca aatgacactg gtcaggcaag 3480
   ctgtcctcca ggtctttatc agacagtgcc cccagaggaa cactatcaaa cattccccat 3540
   tcaagaccct gatcaaatgc actctacttc agaccccagt cacagatcct cttctccaga 3600
   gctcagtgaa atgcttgagt atgaccgaag tcacaagtcc ttccccacag atataagtca 3660
   aatgtcccct tcctcagaac atgaagtctg gcagacagtc atctctccag acctcagcca 3720
   ggtgaccctc tctccagaac tcagccagac aaacctctct ccagacctca gccacacgac 3780
   tctctctcca gaactcattc agagaaacct ttccccagcc ctcggtcaga tgcccatttc 3840
   tccagacctc agccatacaa ccctttctcc agacctcagc catacaaccc tttctttaga 3900
   cctcagccag acaaacctct ctccagaact cagtcagaca aacctttctc cagccctcgg 3960
   tcagatgccc ctttctccag acctcagcca tacaacccct tctctagact tcagccagac 4020
   aaacctctct ccagaactca gccatatgac tctctctcca gaactcagtc agacaaacct 4080
   ttccccagcc ctcggtcaga tgcccatttc tccagacctc agccatacaa ccctttctct 4140
   agacttcagc cagacaaacc tctctccaga actcagtcaa acaaaccttt ccccagccct    4200
   cggtcagatg cccctttctc cagaccccag ccatacaacc ctttctctag acctcagcca    4260
   gacaaacctc tctccagaac tcagtcagac aaacctttcc ccagacctca gtgagatgcc    4320
   cctctttgca gatctcagtc aaattcccct taccccagac ctcgaccaga tgacactttc    4380
   tccagacctt ggtgagacag atctttcccc aaactttggt cagatgtccc tttccccaga    4440
   cctcagccag gtgactctct ctccagacat cagtgacacc acccttctcc cggatctcag    4500
   ccagatatca cctcctccag accttgatca gatattctac ccttctgaat ctagtcagtc    4560
   attgcttctt caagaattta atgagtcttt tccttatcca gaccttggtc agatgccatc    4620
   tccttcatct cctactctca atgatacttt tctatcaaag gaatttaatc cactggttat    4680
   agtgggcctc agtaaagatg gtacagatta cattgagatc attccaaagg aagaggtcca    4740
   gagcagtgaa gatgactatg ctgaaattga ttatgtgccc tatgatgacc cctacaaaac    4800
   tgatgttagg acaaacatca actcctccag agatcctgac aacattgcag catggtacct    4860
   ccgcagcaac aatggaaaca gaagaaatta ttacattgct gctgaagaaa tatcctggga    4920
   ttattcagaa tttgtacaaa gggaaacaga tattgaagac tctgatgata ttccagaaga    4980
   taccacatat aagaaagtag tttttcgaaa gtacctcgac agcactttta ccaaacgtga    5040
   tcctcgaggg gagtatgaag agcatctcgg aattcttggt cctattatca gagctgaagt    5100
   ggatgatgtt atccaagttc gttttaaaaa tttagcatcc agaccgtatt ctctacatgc    5160
   ccatggactt tcctatgaaa aatcatcaga gggaaagact tatgaagatg actctcctga    5220
   atggtttaag gaagataatg ctgttcagcc aaatagcagt tatacctacg tatggcatgc    5280
   cactgagcga tcagggccag aaagtcctgg ctctgcctgt cgggcttggg cctactactc    5340
   agctgtgaac ccagaaaaag atattcactc aggcttgata ggtcccctcc taatctgcca    5400
   aaaaggaata ctacataagg acagcaacat gcctatggac atgagagaat ttgtcttact    5460
   atttatgacc tttgatgaaa agaagagctg gtactatgaa aagaagtccc gaagttcttg    5520
   gagactcaca tcctcagaaa tgaaaaaatc ccatgagttt cacgccatta atgggatgat    5580
   ctacagcttg cctggcctga aaatgtatga gcaagagtgg gtgaggttac acctgctgaa    5640
   cataggcggc tcccaagaca ttcacgtggt tcactttcac ggccagacct tgctggaaaa    5700
   tggcaataaa cagcaccagt taggggtctg gccccttctg cctggttcat ttaaaactct    5760
   tgaaatgaag gcatcaaaac ctggctggtg gctcctaaac acagaggttg gagaaaacca    5820
   gagagcaggg atgcaaacgc catttcttat catggacaga gactgtagga tgccaatggg    5880
   actaagcact ggtatcatat ctgattcaca gatcaaggct tcagagtttc tgggttactg    5940
   ggagcccaga ttagcaagat taaacaatgg tggatcttat aatgcttgga gtgtagaaaa    6000
   acttgcagca gaatttgcct ctaaaccttg gatccaggtg gacatgcaaa aggaagtcat    6060
   aatcacaggg atccagaccc aaggtgccaa acactacctg aagtcctgct ataccacaga    6120
   gttctatgta gcttacagtt ccaaccagat caactggcag atcttcaaag ggaacagcac    6180
   aaggaatgtg atgtatttta atggcaattc agatgcctct acaataaaag agaatcagtt    6240
   tgacccacct attgtggcta gatatattag gatctctcca actcgagcct ataacagacc 6300
   tacccttcga ttggaactgc aaggttgtga ggtaaatgga tgttccacac ccctgggtat 6360
   ggaaaatgga aagatagaaa acaagcaaat cacagcttct tcgtttaaga aatcttggtg    6420
   gggagattac tgggaaccct tccgtgcccg tctgaatgcc cagggacgtg tgaatgcctg    6480
   gcaagccaag gcaaacaaca ataagcagtg gctagaaatt gatctactca agatcaagaa    6540
   gataacggca attataacac agggctgcaa gtctctgtcc tctgaaatgt atgtaaagag    6600
   ctataccatc cactacagtg agcagggagt ggaatggaaa ccatacaggc tgaaatcctc    6660
   catggtggac aagatttttg aaggaaatac taataccaaa ggacatgtga agaacttttt    6720
   caacccccca atcatttcca ggtttatccg tgtcattcct aaaacatgga atcaaagtat    6780
   tgcacttcgc ctggaactct ttggctgtga tatttactag aattgaacat tcaaaaaccc    6840
   ctggaagaga ctctttaaga cctcaaacca tttagaatgg gcaatgtatt ttacgctgtg    6900
   ttaaatgtta acagttttcc actatttctc tttcttttct attagtgaat aaaattttat    6960
   acaagaagct tttataatgt aactccttgc taccagtaag taagataatg gctattactt    7020
   ctgcattaat ttgaatacag gtaggaaaat atcaagaacc aacaagaaaa gggcttatct    7080
   ttcttaatga ttgaaaatgc tatgaagtaa tatttatgta gttaaaatgc ttcattataa    7140
   ctcttttaaa tcctttacac actagtaaaa cagatattac tttaaataat aattgataga    7200
   cctggataac tttcacaaac acatgatttt ttaatggttt ttcttgagtg aagagaaaaa    7260
   caatattatc aaatgaaata agtacttaaa atatcctgtc tttcccatat aacaatgatt    7320
   tttctgactt tccatgagta aaaaaacagc caagcatctt tccagtagcc ccattgaaat    7380
   tgtgaatccg tcctggtctc cctaaggact gcacacattg atattcaagg ttggtggtca    7440
   ttagatatgg aacagaactg aaataaccat ggtagaactg aatgtgtaat gttggcttta    7500
   ttctagctgg tactacatgg cacacagttt caaaacataa tttcacctac tggaaagctc    7560
   agacctgtaa aacagagcat gggaactgct ggtctaaatg cagttgttcc tgctcaaaga    7620
   gacctctggc caaactggca agcagttaaa gttttctttc agggccttcc tctctatggc    7680
   ctcaacttcc tcctctctct tcttccagca acttcccctt tcatcattcc tttccctggg    7740
   gacttggcat tcagtgatcc tgtagatatt gcacaactgg ggaaccttta gacatcctta    7800
   aaatcacatg agatagacag tcatttgggg tgtctgaaat aaaccacccc aaaacttagt    7860
   gttaaaagag caaccaaaaa aaatttatgt gagattatgg atttgttact tagcttgatt    7920
   taatcatcct gtaacgtgta catatatcaa aatgttatgt ataccataaa tatataaaat    7980
   tttatcaacg aaattcataa caatctctca gaccacagag aaatcaaatt agaactgagg    8040
   actaagaaac tcactcgaaa ccacacaact acatggaaac tgaacaacct gctcctgaat    8100
   gactactggg taaataatga aattaaggca gaaataaata agttccttaa aaccaatgag    8160
   aacaaagaga caacatacca gaatctctag gagacagggc tttgcttttg ctgcattcta    8220
   ttcgttgtga acacaaatta caggccagtc tcgattcagt gtagaaggga actgcataag     8280
   gaccacatac caggaggcat aattcactgg gagcatcttt agaaactacc agagttacct     8340
   gttgcccata ccagtggggt aagccctatg aatgtatatg agagtttcaa acatccacaa     8400
   aacattggct ttctaatatt cgtattccca ctattccttt cttttcatga ttcatgtcat     8460
   tgtcccatca acatttctaa gatttccatt ccgttaagag caaaagagaa tgttggaagg     8520
   tgggggaaaa catttctttg ttttctacag ggccagcttc ttggatgtgt gtgatctgtt     8580
   cagttgcaaa gggtcacatg ctcagaagga ccgcatgcta aatttaatgc tttgcagtta     8640
   ccctcttgaa atcctttatt ttttaagaag gaattcgaca tttccatttt tcaatgagcc     8700
   ccacaaatta cgcagctagt cctgggcttc tctactctga aattgggcag gatctctctt     8760
   gatctagaat ttactaaggc ataatagggg caagaaaatc ttatgaaata atggggggta     8820
   gggaagagat gggaatggag catgagatcc agcttcgtta ttctctactt gagaaaaata     8880
   aggccccaaa gattaaacaa cttgcccaag gatattgctt gttagtgtca gaactgaaac     8940
   cagaaaccaa atgatcatat ccctagactt ttagtctgct ttctcttcca taaaatgaaa     9000
   cttataatgt ttctaatcca ttgctcagac aggtagacat gaatattaat tgataatgac     9060
   tattaattga tctggaaaat acttgtttgg ggatcaataa tatgtttggg ctattatcta     9120
   atgctgtgta gaaatattaa aacccctgtt attttgaaat aaaaaagata cccactttt      9179
<210> 13
   <211> 1665
   <212> DNA
   <213> Homo sapiens
<400> 13
   cttctggtaa ggaggccccg tgatcagctc cagccatttg cagtcctggc tatcccagga       60
   gcttacataa agggacaatt ggagcctgag aggtgacagt gctgacacta caaggctcgg      120
   agctccgggc actcagacat catgagttgg tccttgcacc cccggaattt aattctctac      180
   ttctatgctc ttttatttct ctcttcaaca tgtgtagcat atgttgctac cagagacaac      240
   tgctgcatct tagatgaaag attcggtagt tattgtccaa ctacctgtgg cattgcagat      300
   ttcctgtcta cttatcaaac caaagtagac aaggatctac agtctttgga agacatctta      360
   catcaagttg aaaacaaaac atcagaagtc aaacagctga taaaagcaat ccaactcact      420
   tataatcctg atgaatcatc aaaaccaaat atgatagacg ctgctacttt gaagtccagg      480
   aaaatgttag aagaaattat gaaatatgaa gcatcgattt taacacatga ctcaagtatt      540
   cgatatttgc aggaaatata taattcaaat aatcaaaaga ttgttaacct gaaagagaag      600
   gtagcccagc ttgaagcaca gtgccaggaa ccttgcaaag acacggtgca aatccatgat      660
   atcactggga aagattgtca agacattgcc aataagggag ctaaacagag cgggctttac      720
   tttattaaac ctctgaaagc taaccagcaa ttcttagtct actgtgaaat cgatgggtct      780
   ggaaatggat ggactgtgtt tcagaagaga cttgatggca gtgtagattt caagaaaaac      840
   tggattcaat ataaagaagg atttggacat ctgtctccta ctggcacaac agaattttgg      900
   ctgggaaatg agaagattca tttgataagc acacagtctg ccatcccata tgcattaaga      960
   gtggaactgg aagactggaa tggcagaacc agtactgcag actatgccat gttcaaggtg     1020
   ggacctgaag ctgacaagta ccgcctaaca tatgcctact tcgctggtgg ggatgctgga     1080
   gatgcctttg atggctttga ttttggcgat gatcctagtg acaagttttt cacatcccat     1140
   aatggcatgc agttcagtac ctgggacaat gacaatgata agtttgaagg caactgtgct     1200
   gaacaggatg gatctggttg gtggatgaac aagtgtcacg ctggccatct caatggagtt     1260
   tattaccaag gtggcactta ctcaaaagca tctactccta atggttatga taatggcatt     1320
   atttgggcca cttggaaaac ccggtggtat tccatgaaga aaaccactat gaagataatc     1380
   ccattcaaca gactcacaat tggagaagga cagcaacacc acctgggggg agccaaacag     1440
   gctggagacg tttaaaagac cgtttcaaaa gagatttact tttttaaagg actttatctg     1500
   aacagagaga tataatattt ttcctattgg acaatggact tgcaaagctt cacttcattt     1560
   taagagcaaa agaccccatg ttgaaaactc cataacagtt ttatgctgat gataatttat     1620
   ctacatgcat ttcaataaac cttttgtttc ctaagactag aaaaa                     1665
<210> 14
   <211> 2071
   <212> DNA
   <213> Homo sapiens
<400> 14
   agcggtctcc cgcccgcggc gccatcgcgc cattcctagt taaggcggca cagggccgag      60
   gcgtagtgtg ggtgactcct ccgttccttg ggtcccgtcg tctgtgatac tgcagcgcag     120
   ccatggcaga accgcagccc ccgtccggcg gcctcacgga cgaggccgcc ctcagttgct     180
   gctccgacgc ggaccccagt accaaggatt ttctattgca gcagaccatg ctacgagtga     240
   aggatcctaa gaagtcactg gatttttata ctagagttct tggaatgacg ctaatccaaa     300
   aatgtgattt tcccattatg aagttttcac tctacttctt ggcttatgag gataaaaatg     360
   acatccctaa agaaaaagat gaaaaaatag cctgggcgct ctccagaaaa gctacacttg     420
   agctgacaca caattggggc actgaagatg atgagaccca gagttaccac aatggcaatt     480
   cagaccctcg aggattcggt catattggaa ttgctgttcc tgatgtatac agtgcttgta     540
   aaaggtttga agaactggga gtcaaatttg tgaagaaacc tgatgatggt aaaatgaaag     600
   gcctggcatt tattcaagat cctgatggct actggattga aattttgaat cctaacaaaa     660
   tggcaacctt aatgtagtgc tgtgagaatt ctcctttgag atttcagaag aaaggaaaca     720
   atgtgattca agatatttac ataccagaag catctaggac tgatggatca ctgtcccgat     780
   tcaaattatt cttcagtcca tttccccttc ctatttcagc tgttcctttt cacctaactg     840
   ttcagtcatt ctggttttca agcagtgctt tatctcatgt ccttgaatat agttgtgtaa     900
   ctttattttt taggtaataa ttagaacagt tcccttcaga ggctgcattt gccttcttct     960
   gccacctaaa tattacttcc cttcaaatct gcctttgaat catcattttt aaaaaaaaat    1020
   taacatgttt ttgttgtagt tatcttctgg ggtttcaatt cctcagaaac aacttttttc   1080
   acaacggaaa ggaaagaaca ctagtgttct ttcagtaaag tacaaagtgt ttattttaca    1140
   aaagagtagg tactcttgag agcaattcaa atcatgctga caaggatact gatagaaaaa    1200
   gtgatttctt cttattataa agtacattta aagttcaagg actaacctta tttatttggg    1260
   aaaggggagg aggaaggaaa tgatatggta cccagacact gggctaggct gcaactttat    1320
   ctcatttaat actcccagct gtcatgtgag aaagaaagca ggctaggcat gtgaaatcac    1380
   tttcatggat tattaatgga tttaagaggg catcaatcag ctcaactcaa gatttcataa    1440
   tcatttttag tatttagatt gtgcctcaaa gttgtagtac ctcacaatac ctccactggt    1500
   ttcctgttgt aaaaaccttc agtgagtttg accattgtgc tcttggctct tgggctggag    1560
   taccgtggtg agggagtaaa cactagaagt ctttagtaca aaactgctct agggacacct    1620
   ggtgattcct acacaagtga tgtttatatt tctcataaag agtcttccct atcccaaggt    1680
   cttcatgatg ccagtagcca tatatgataa attatgttca gtgataactt agttatcaga    1740
   aatcagctca gtggtcttcc ccgccatgat tcacatttga tgagttttta aaaatcaaag    1800
   tgattttgaa aatctctaat ggctcagaaa ataaaaacat ccagtttgtg gatgactata    1860
   tttagatttc tctagactct agtggaagac ctttggaaag gccatgccaa ccgtgcttgt    1920
   actgctagaa gcactttatg tttccttttt gggtgaaatg gatttatgtg agtgctttaa    1980
   acaaatagca atacttatag actgaaataa aatgaaactt caaataagac tatgtttaat    2040
   ttgtaaaaaa aaaaaaaaaa aaaaaaaaaa a                                   2071
<210> 15
   <211> 4337
   <212> DNA
   <213> Homo sapiens
<400> 15
   cgtcatgtta gggtgaagca gaggacctca gtgctgaaca tgctaaggag gttggacaaa      60
   atcaggttca gaggtcacaa gagagatgac ttcctcgatc tagcggagtc tccaaatgcc     120
   tcggacaccg aatgcagcga cgaaatcccc ctgaaggtac cgcggacctc gccccgggac     180
   agcgaggagc tgagggaccc tgctggtcca gggaccctca tcatggccac aggagtccag     240
   gactttaacc ggacagagtt tgatcgactg aatgagatca aaggtcacct ggaaattgcc     300
   ttattggaaa aacatttctt acaggaggag ctccggaagc tgcgagaaga aaccaacgcg     360
   gagatgctgc ggcaggagct ggaccgcgag cggcagcggc ggatggagct ggagcagaag     420
   gtgcaggagg tgctgaaggc cagaaccgag gagcagatgg ctcagcagcc cccaaaaggg     480
   caggcccagg ccagcaatgg agcagagcgc cggagccagg ggctgtcctc gcgcctgcag     540
   aagtggttct acgagcggtt cggggagtac gtggaggact tccggttcca gcccgaggag     600
   aacactgtgg agacagagga acccctgagc gcccgcaggt taactgaaaa tatgagacgg     660
   ctcaagcgcg gtgccaagcc ggtcactaac tttgtgaaga acctctctgc cttatccgac     720
   tggtactccg tctacacgtc tgccattgcc ttcaccgtgt acatgaatgc cgtgtggcat     780
   ggctgggcca tcccattgtt cttatttcta gcaattctga ggttatccct caattacctc     840
   atcgccaggg ggtggcggat acagtggagc atcgtgcccg aagtgtctga gcccgtggaa     900
   cctccaaagg aagacctgac tgtgtctgag aagttccagc tggtgctgga cgtcgcccag     960
   aaagcccaga accttttcgg gaagatggct gacatcctgg agaagatcaa gaacttgttc    1020
   atgtgggtcc agccggagat cacacagaag ctgtatgtgg cgctctgggc tgccttcctg    1080
   gcctcctgct tcttccccta ccgcctggtg gggcttgccg tgggactcta tgctggtatc    1140
   aagttcttcc tcattgattt catctttaaa cgctgcccga ggctgcgcgc caagtacgac    1200
   acgccctata tcatctggag gagtctcccc accgacccgc agctcaagga gcgctccagc    1260
   gccgcagtct cacgcaggct gcagacgacc tcgtcacgga gctacgtacc cagcgcaccg    1320
   gccggcctgg gtaaagagga ggacgccggt cgcttccaca gcaccaagaa gggcaatttc    1380
   cacgagatct tcaatctgac agaaaacgag cgtccgctgg cggtgtgcga gaatggctgg    1440
   cgctgctgcc tcatcaacag ggaccggaag atgcccacgg actacatcag gaacggggtg    1500
   ctctacgtca cggagaatta cttgtgcttc gaaagctcca aatctgggtc ctcaaagagg    1560
   aacaaagtca tcaagctagt ggacatcacg gacatccaga agtacaaggt cctgtctgtc    1620
   ctcccaggct caggcatggg gattgccgtg tcgacgccat ccacccagaa accgctcgtg    1680
   tttggtgcca tggtgcacag ggatgaggcc ttcgagacca ttctcagcca gtacatcaag    1740
   atcacctcag cggcagcgtc tggcggggac agctagtatt gacttgccca ggacgttgct    1800
   ggaattttct ttttcttttt ctttttcttt tttttttttt acgatttggt agtggaaaca    1860
   attggacatc ctcatgagct tttgcaataa ttctcctgga cctgtggttc tattgtgttg    1920
   acctctgcgt tttatcgacc aagaaggggc cagggctcac agggacgggg gtgcccctct    1980
   cccacagggc acgtcaggtg cctctgaggg ccacccgcag actgggggag ggggcagagg    2040
   ccctcggggg cccgtggaga agacacacag gacccctggc cctgcccttc tccgttccag 2100
   cctggacaga gaaacctctc cagccacccc aagaggttct cgcaaccttg tgtcccgctc 2160
   tccagaggcc agaagctcgt ccaccaccaa agccatagct gaagagtgcg gggcccttcc 2220
   tcctggggac agaaagatgt cgtcaaggag ggacatgggg gcctttcacc aaccaccgag 2280
   aaacgggcct ggcggccctc cttcctctta catgagaccc tcctgtggca tttgcccttg 2340
   gtgccgggct ggggccgggc gcagtgaccc tgcctgcgct ccacactcgc tccacgggaa 2400
   cagagagggt gagaagggcc cacccctcgc ctgccctcag tgtctttggt ggcaccttcc 2460
   ttgctggcct ccagggcgct cagcaccgcg tctgtaaggg cctgcctgct gctctcggcc 2520
   tgacacgccg gccaggaggt ctgtagctgg ggaccagtaa gggcacagga tggtgcaggt 2580
   aaaagcacat ctttctcaca ctttgctctt tggaaggccc aggagaacat ccgcgaaggc 2640
   tgttggaggt gctccgagca ctgtggcatg tctggcacat ggcccccagg ctgcggttgc 2700
   ctgggttggt tgggggagga agtggggagg agtgttccgg gaccatggtg gcccaggctg 2760
   cagccgcctt tgggccatcc gagaggctct ggcagcccct gtgctttagg gagcaaccgt 2820
   gagccgagcc cagaggcctg ggcctgcact gcctgcagcc gacatgcgac agcgttccct 2880
   cccccgcgtg cctagccggt gccggtccgg gcacagaccc ccccagcccc cgccctgccc 2940
   cagggaagcc tgggcttccc gggaacaagg tggcatttgt ggagggagcg cccgcaggcc 3000
   tggtctgctg gggccgcctg cgctgggctg aagggaggga aaggcggctt gggcctcctg 3060
   gaaggaggtg gccaccccgc gggcctgcgt gtctgctggg gcggatcccg cagctccctc 3120
   agcttgtcct gagtcccttg ggtgtcgttg agattgttgt tttttgaaga aacagaagat 3180
   tctatttttt acagcgagca agctggtttt cttatttttg tatccttttt cagatgtaat 3240
   ttttatcttt gctccgatcc tcatttgctg gtgtgggtga gggatccggc ggcatgggct 3300
   ggtttcaccc ccttcacgag gggccgcaga gtcacacgct ggtgccgggg gtgctttggg 3360
   gggagctgcg ccgatcacca gattaagcac atgtcctatc ccaggcggtg gagcggagcc 3420
   cccgtggctc tggactgcgc ggacgttggc gtcaggatga ccacacggcg gcctttcccg 3480
   aatggggaca gaacccgctc tgagccgtgg gtctggctcc tgtaggggac tggctctctt 3540
   ggtgcaccag gggaggggga catatcccag tgaaccccac cttggcgcct gaggcaacac 3600
   agggtgggca ctgacccacc cccaggggcg gctgcagagg cagtgcccgc agacaatggc 3660
   cacacctctc tccccagggc ccggcagtgc ccaaggatgg gtccggggcc tcggggccaa 3720
   tgagcgcctc ttcctaggtg ctgggattca gtccccaaac acagcgggag gggtccctgg 3780
   ggcagatggg gctttaccag cgtcgggtgg tttagttcga gtcccttttg tggagaaagg 3840
   gagatgaaaa ctgaccacgt gccaggtgtg gccgaagccc ccagggaggg ccacattcgg 3900
   ggagcggggg gtcgggggag ggccaccgac tggctctgct gccagcacag gcccctccct 3960
   ggaagtcctc gggagcggag cgcggatcgg cacgggctct gggctccccg tggagagaag 4020
   ctgtagtttt taccaaattg tgtacatctg ggcagatgtt taatttctgt gactaatcac 4080
   tgaactagac gaatgttaaa ttttttatgt ctgaagcctg agtctatttt ggatctgtaa 4140
   ataatcattg ccagtgtgac ttttgttcaa caaaaggatt gtactgtatt aagaaccgat 4200
   gaaaaaaatt ctcctgtaac atttttttaa gaaaactttg tttgtttaaa gaaaaagtat 4260
   tgtataaatt ataattttta tttaaataaa cctaaaatgc tttgtgctaa ggctcaaaaa 4320
   aaaaaaaaaa aaaaaaa 4337
<210> 16
   <211> 7771
   <212> DNA
   <213> Homo sapiens
<400> 16
   cagagaggaa gtagcgagag aagagagaaa atgaagtcgg cgctggggga gcctgcagga 60
   gggtggccaa cagtggagga aggtggattt ggcttctttt ccgcaccccg ggcgtgaaag 120
   ccctctccaa cgcgacccca ggaaataagt gggtctcgcc tgggcagaaa aggaaaagaa 180
   tccaggcgag agcgcgtcgc tcctctgtca ctgctgcccc cgaggaactc cggctgcttc 240
   tcatcccggc cgcctcgcgg ggccggacgc agtgcccgag gcgccctgca gatggggcgg 300
   gcagggaacg ggcgctccag ctgcgggtga caggcgccgg cccgcccgcc tgcctgctca 360
   gcgcagtgac cgggcgggca gaggatgcca ggcggaggga cctgggagcg ggatctgaga 420
   ctgccggagg cgcgctacgc tccaacttgc atggcctaga gaccgctcca gctcctggga 480
   ccgcttcacc gagtggagtg aagctgcgcg cgggacctgg aggcggagac ctcaggcagc 540
   ggctgcagag gggcgagccg ggcgcaggag ggggcgcgct ttctccctgc gggtctcagt 600
   aatgaggaga ctgagtttgt ggtggctgct gagcagggtc tgtctgctgt tgccgccgcc 660
   ctgcgcactg gtgctggccg gggtgcccag ctcctcctcg cacccgcagc cctgccagat 720
   cctcaagcgc atcgggcacg cggtgagggt gggcgcggtg cacttgcagc cctggaccac 780
   cgccccccgc gcggccagcc gcgctccgga cgacagccga gcaggagccc agagggatga 840
   gccggagcca gggactaggc ggtccccggc gccctcgccg ggcgcacgct ggttggggag 900
   caccctgcat ggccgggggc cgccgggctc ccgtaagccc ggggagggcg ccagggcgga 960
   ggccctgtgg ccacgggacg ccctcctatt tgccgtggac aacctgaacc gcgtggaagg 1020
   gctgctaccc tacaacctgt ctttggaagt agtgatggcc atcgaggcag gcctgggcga 1080
   tctgccactt ttgcccttct cctcccctag ttcgccatgg agcagtgacc ctttctcctt 1140
   cctgcaaagt gtgtgccata ccgtggtggt gcaaggggtg tcggcgctgc tcgccttccc 1200
   ccagagccag ggcgaaatga tggagctcga cttggtcagc ttagtcctgc acattccagt 1260
   gatcagcatc gtgcgccacg agtttccacg ggagagtcag aatccccttc acctacaact 1320
   gagtttagaa aattcattaa gttctgatgc tgatgtcact gtctcaatcc tgaccatgaa    1380
   caactggtac aattttagct tgttgctgtg ccaggaagac tggaacatca ccgacttcct    1440
   cctccttacc cagaataatt ccaagttcca ccttggttct atcatcaaca tcaccgctaa    1500
   cctcccctcc acccaggacc tcttgagctt cctacagatc cagcttgaga gtattaagaa    1560
   cagcacaccc acagtggtga tgtttggctg cgacatggaa agtatccggc ggattttcga    1620
   aattacaacc cagtttgggg tcatgccccc tgaacttcgt tgggtgctgg gagattccca    1680
   gaatgtggag gaactgagga cagagggtct gcccttaggg ctcattgctc atggaaaaac    1740
   aacacagtct gtctttgagc actacgtaca agatgctatg gagctggtcg caagagctgt    1800
   agccacagcc accatgatcc aaccagaact tgctctcatt cccagcacga tgaactgcat    1860
   ggaggtggaa actacaaatc tcacttcagg acaatattta tcaaggtttc tagccaatac    1920
   cactttcaga ggcctcagtg gttccatcag agtaaaaggt tccaccatcg tcagctcaga    1980
   aaacaacttt ttcatctgga atcttcaaca tgaccccatg ggaaagccaa tgtggacccg    2040
   cttgggcagc tggcaggggg gaaagattgt catggactat ggaatatggc cagagcaggc    2100
   ccagagacac aaaacccact tccaacatcc aagtaagcta cacttgagag tggttaccct    2160
   gattgagcat ccttttgtct tcacaaggga ggtagatgat gaaggcttgt gccctgctgg    2220
   ccaactctgt ctagacccca tgactaatga ctcttccaca ttggacagcc tttttagcag    2280
   cctccatagc agtaatgata cagtgcccat taaattcaag aagtgctgct atggatattg    2340
   cattgatctg ctggaaaaga tagcagaaga catgaacttt gacttcgacc tctatattgt    2400
   aggggatgga aagtatggag catggaaaaa tgggcactgg actgggctag tgggtgatct    2460
   cctgagaggg actgcccaca tggcagtcac ttcctttagc atcaatactg cacggagcca    2520
   ggtgatagat ttcaccagcc ctttcttctc caccagcttg ggcatcttag tgaggacccg    2580
   agatacagca gctcccattg gagccttcat gtggccactc cactggacaa tgtggctggg    2640
   gatttttgtg gctctgcaca tcactgccgt cttcctcact ctgtatgaat ggaagagtcc    2700
   atttggtttg actcccaagg ggcgaaatag aagtaaagtc ttctcctttt cttcagcctt    2760
   gaacatctgt tatgccctct tgtttggcag aacagtggcc atcaaacctc caaaatgttg    2820
   gactggaagg tttctaatga acctttgggc cattttctgt atgttttgcc tttccacata    2880
   cacggcaaac ttggctgctg tcatggtagg tgagaagatc tatgaagagc tttctggaat    2940
   acatgacccc aagttacatc atccttccca aggattccgc tttggaactg tccgagaaag    3000
   cagtgctgaa gattatgtga gacaaagttt cccagagatg catgaatata tgagaaggta    3060
   caatgttcca gccacccctg atggagtgga gtatctgaag aatgatccag agaaactaga    3120
   cgccttcatc atggacaaag cccttctgga ttatgaagtg tcaatagatg ctgactgcaa    3180
   acttctcact gtggggaagc catttgccat agaaggatac ggcattggcc tcccacccaa    3240
   ctctccattg accgccaaca tatccgagct aatcagtcaa tacaagtcac atgggtttat    3300
   ggatatgctc catgacaagt ggtacagggt ggttccctgt ggcaagagaa gttttgctgt    3360
   cacggagact ttgcaaatgg gcatcaaaca cttctctggg ctctttgtgc tgctgtgcat    3420
   tggatttggt ctgtccattt tgaccaccat tggtgagcac atagtataca ggctgctgct    3480
   accacgaatc aaaaacaaat ccaagctgca atactggctc cacaccagcc agagattaca    3540
   cagagcaata aatacatcat ttatagagga aaagcagcag catttcaaga ccaaacgtgt    3600
   ggaaaagagg tctaatgtgg gaccccgtca gcttaccgta tggaatactt ccaatctgag    3660
   tcatgacaac cgacggaaat acatctttag tgatgaggaa ggacaaaacc agctgggcat    3720
   ccggatccac caggacatcc ccctccctcc aaggagaaga gagctccctg ccttgcggac    3780
   caccaatggg aaagcagact ccctaaatgt atctcggaac tcagtgatgc aggaactctc    3840
   agagctcgag aagcagattc aggtgatccg tcaggagctg cagctggctg tgagcaggaa    3900
   aacggagctg gaggagtatc aaaggacaag tcggacttgt gagtcctagg tgaccacact    3960
   gcttcccttt ctcagttcct gaccttcctc tgagcccttg agacactttg taatgctctt    4020
   ttgtaactat cgacaaaggt gtggggaagc tgaggtctag gtcttcttaa aggtcaagtc    4080
   tgctctccct cgcctaaagt gcagcagcag ctcctctcaa gctcactctc taggtctcca    4140
   gggtaggagt gtttttctag caagaatctt agtcaggagt aagctctgtg cgagagatct    4200
   gtgaataacc agataacccc agctgccgtt aaccttttca ccaggtgcca cagtaatatt    4260
   tctggttttt agccctttct ctgcactacc aacaagagat aaaattgtta ctcacactta    4320
   tgtcttactg ggttgctggt tttcatcgta acacagaacg aggttatcta gggttgtagc    4380
   ttttgataca actccccgat ctagatttat tcctacattc tgaatgggga gcaggtaaga    4440
   gcagagcacc tcccactggg ggtggggtat ttaaaaatta actcattagt atcataaacg    4500
   tcaaggattg attggaccag gcaagagcca tgtttttgag aaggttctgg atctctgact    4560
   ccatcctgac tgtttagtaa gagcatgctt acaccctact gtgaaaaggg gaggggatgt    4620
   ggtaagcgga aacagaagac aggcagcaga ggcattaaaa atgcatacca tgctttcaga    4680
   acaaaagctc tgggccagaa aggcaatttg gctaaaaaat gaataagact acttctaatg    4740
   taactaagca tctccactat ggtgtgtgcc ttttataaag gaaaagagag aaaaaggcaa    4800
   agcaaggttg tggccttagg ttggacctgg aatatccctt attgcctata atggaatatg    4860
   tgacactgtg ggtgaaatgt tctacacacc acacactagg ccattttcag atcagcagtc    4920
   acccatcgct tagcatagaa atcccaaaac ctccagcccg ggaacactat aagcttcgac    4980
   cattcaggaa tctgccctgc actttgcata tctgtataga aaatcaagtc aatcccccat    5040
   cctcacaccc actcatctct gaggagctat gaactggttt tggtccctct aatgatcctc    5100
   cagcctcatc taatgccccc caaagactga tacaagtaac ctcccctctg cttaggtgtc    5160
   actttctcag catatcaagt ttaggcagca agggaaagga atatgggtca gttctcaaat    5220
   gtcaatgtag ataagagtca tctagtagag aactcatcag agtgcggatt gccaagaccc    5280
   ttctccagag attatggggt tgggggtgga ggtctagagg tgagctcaga aacctactgt    5340
   taaccaacac ccccaagtga ctgacacagg tggtctaaaa attacttttc tagaaacacc    5400
   attctggaag tttggctgcc cacaggcagg aggagaagca tgaagagaaa acctgtttga 5460
   gaagttttgt tttgttttgt tttgcttttt aataatttta gcacacatct gctgactctc 5520
   cttcaacatc ctcaccccca cccctgggca ccatttagga caagacttcc ttatttatca 5580
   attacttgat ttatcttctc aggactcatt gttccacccc caaccaattt gaatgcctac 5640
   aataagttca ggagctgtgc caagcacttt cctcttttac agctggagat cactggaaag 5700
   gtgtctcagt cacaaaactt ctccctctac tactggatga aatgtctgca tttccaccaa 5760
   aatctaccca gtcacccagg gaataacaac ttaagctgta gttagataac acctagtgat 5820
   taattggctg agaaaaccct ggagtggagg gaggctcaga gatactgata tggatgtggg 5880
   agggctctaa agttagaggt caccaactcc acagatgaaa cagttcaata atgaggaaac 5940
   aggtgagccc tgaaaacaca aaaggacagt tctgtgttga aacaccccat cccctcacgt 6000
   tctcacccca ggcccagaag taggttgcaa ctgcctttgg aagattttgc cccttagcca 6060
   tccccaccca cttgtaccag ctaagaatgc tggagactct gccaccatgc tctgcgtgcc 6120
   cctgaacctc tgtgcagccc ggaaggctga tgtacaggtg tacctcaatc cacattacag 6180
   ccatgctcct aatgtacatg gacatttttg taactcagct catattctga ctgtatttga 6240
   gaagctggct gtttaaggga acccagaagt gaattctttt gtaaagtaaa gcaccctttt 6300
   gtaatgcaat taattatccc ttaatgtatc tgttttgtaa gtctgcattt ttgtatatcg 6360
   gatttacctt aagcttctct agtgaggcat tctgagcagt ggtgatcaca tgccagatcg 6420
   ccctgcctat ccacaaagta gatgaccaat gcacgctcct caaacatctt tggaggaact 6480
   acctggccaa aacactggcc aggatgcagc aagcagcagc aggggctgac agcaggctta 6540
   ctgccatcaa cattgcttga aatgcctcta tgttctgaat aaagaaaaac cataattgct 6600
   tgtggtgaaa cgaagcagtc ttcatgttaa gtagcaatgg ttatttttat tggtagtaac 6660
   tgaacagtgt tttgcaattt gtgaaacagt gtattgtgtt ttgtaaaatg atgtcatgaa 6720
   atggtgggtc cttggaaacc tcctttccgt tcagctctgc ctctgttctt tcaactcctt 6780
   tgaggctcaa aaaaaacaca aagatcagaa gccttcagat agagggtggt attctggtaa 6840
   agaagaaaga gataagggac gctaccttgc ttttctggca caggaagcac atgataaagc 6900
   atgctcagat gagctggaac agatatagct acctggttcg tgtaaataag aataatcaag 6960
   gccccagagt gtgtatgctt ccaggtggag gagaaagggg aatctcccaa aatttaaaaa 7020
   caaattggaa gaataaccag gacagccaag tgaagcagcc acagggaccc aagcagtcga 7080
   ggtctttaat gtgcctggag atgactctct gctattcatg aatcttgcta ttgcacaaac 7140
   cctatcaaga gctgctgctt cccttccagc cagaaaagtg gtaagcggag caagtgccaa 7200
   gcagaacaga ccttatcatc tgggtaacag acttctcagt gttggtgctg tgtctgttag 7260
   agccttagag caagttaagc acttccttgg tgtgggtaaa gaataaaggg gaaagaaact 7320
   actttagagc ctctttttct cccaactcat atttttgata ggaaaaacag aaaacccatc 7380
   cagttcttca gaaattgctt tctaggcatt aatactactt tactatctat actgtttagt 7440
   tattcctttc tttacccacc taaactatcc atctaatcca ggattccctc actctttttt 7500
   tttagttact aatcatttta tgaaaataat gtatttataa gtattttctt aaggtttgtg 7560
   aagagtattt gcattgtgtc ttcattttaa tgtgtttgca atcgctccgc tccaggaaga 7620
   acggaaatgc tgtcttgtga gcatgaagtg aacgggctgt tttgctccag ccacttttct 7680
   tgtacaacca catggatgga ttagatgtcc tcaggtcttt tccatcttca gtttctatga 7740
   ctgtggaata aatgttcaga tagaaacttc a                                7771
<210> 17
   <211> 4082
   <212> DNA
   <213> Homo sapiens
<400> 17
   gcgcgcgccg gcctgggcag gcgagcgggc gcgctcccgc cccctctccc ctccccgcgc      60
   gcccgagcgc gcctccgccc ttgcccgccc cctgacgctg cctcagctcc tcagtgcaca     120
   gtgctgcctc gtctgagggg acaggaggat caccctcttc gtcgcttcgg ccagtgtgtc     180
   gggctgggcc ctgacaagcc acctgaggag aggctcggag ccgggcccgg accccggcga     240
   ttgccgcccg cttctctcta gtctcacgag gggtttcccg cctcgcaccc ccacctctgg     300
   acttgccttt ccttctcttc tccgcgtgtg gagggagcca gcgcttaggc cggagcgagc     360
   ctgggggccg cccgccgtga agacatcgcg gggaccgatt caccatggag ggcgccggcg     420
   gcgcgaacga caagaaaaag ataagttctg aacgtcgaaa agaaaagtct cgagatgcag     480
   ccagatctcg gcgaagtaaa gaatctgaag ttttttatga gcttgctcat cagttgccac     540
   ttccacataa tgtgagttcg catcttgata aggcctctgt gatgaggctt accatcagct     600
   atttgcgtgt gaggaaactt ctggatgctg gtgatttgga tattgaagat gacatgaaag     660
   cacagatgaa ttgcttttat ttgaaagcct tggatggttt tgttatggtt ctcacagatg     720
   atggtgacat gatttacatt tctgataatg tgaacaaata catgggatta actcagtttg     780
   aactaactgg acacagtgtg tttgatttta ctcatccatg tgaccatgag gaaatgagag     840
   aaatgcttac acacagaaat ggccttgtga aaaagggtaa agaacaaaac acacagcgaa     900
   gcttttttct cagaatgaag tgtaccctaa ctagccgagg aagaactatg aacataaagt     960
   ctgcaacatg gaaggtattg cactgcacag gccacattca cgtatatgat accaacagta    1020
   accaacctca gtgtgggtat aagaaaccac ctatgacctg cttggtgctg atttgtgaac    1080
   ccattcctca cccatcaaat attgaaattc ctttagatag caagactttc ctcagtcgac    1140
   acagcctgga tatgaaattt tcttattgtg atgaaagaat taccgaattg atgggatatg     1200
   agccagaaga acttttaggc cgctcaattt atgaatatta tcatgctttg gactctgatc     1260
   atctgaccaa aactcatcat gatatgttta ctaaaggaca agtcaccaca ggacagtaca     1320
   ggatgcttgc caaaagaggt ggatatgtct gggttgaaac tcaagcaact gtcatatata     1380
   acaccaagaa ttctcaacca cagtgcattg tatgtgtgaa ttacgttgtg agtggtatta     1440
   ttcagcacga cttgattttc tcccttcaac aaacagaatg tgtccttaaa ccggttgaat     1500
   cttcagatat gaaaatgact cagctattca ccaaagttga atcagaagat acaagtagcc     1560
   tctttgacaa acttaagaag gaacctgatg ctttaacttt gctggcccca gccgctggag     1620
   acacaatcat atctttagat tttggcagca acgacacaga aactgatgac cagcaacttg     1680
   aggaagtacc attatataat gatgtaatgc tcccctcacc caacgaaaaa ttacagaata     1740
   taaatttggc aatgtctcca ttacccaccg ctgaaacgcc aaagccactt cgaagtagtg     1800
   ctgaccctgc actcaatcaa gaagttgcat taaaattaga accaaatcca gagtcactgg     1860
   aactttcttt taccatgccc cagattcagg atcagacacc tagtccttcc gatggaagca     1920
   ctagacaaag ttcacctgag cctaatagtc ccagtgaata ttgtttttat gtggatagtg     1980
   atatggtcaa tgaattcaag ttggaattgg tagaaaaact ttttgctgaa gacacagaag      2040
   caaagaaccc attttctact caggacacag atttagactt ggagatgtta gctccctata     2100
   tcccaatgga tgatgacttc cagttacgtt ccttcgatca gttgtcacca ttagaaagca     2160
   gttccgcaag ccctgaaagc gcaagtcctc aaagcacagt tacagtattc cagcagactc     2220
   aaatacaaga acctactgct aatgccacca ctaccactgc caccactgat gaattaaaaa     2280
   cagtgacaaa agaccgtatg gaagacatta aaatattgat tgcatctcca tctcctaccc     2340
   acatacataa agaaactact agtgccacat catcaccata tagagatact caaagtcgga     2400
   cagcctcacc aaacagagca ggaaaaggag tcatagaaca gacagaaaaa tctcatccaa     2460
   gaagccctaa cgtgttatct gtcgctttga gtcaaagaac tacagttcct gaggaagaac     2520
   taaatccaaa gatactagct ttgcagaatg ctcagagaaa gcgaaaaatg gaacatgatg     2580
   gttcactttt tcaagcagta ggaattggaa cattattaca gcagccagac gatcatgcag     2640
   ctactacatc actttcttgg aaacgtgtaa aaggatgcaa atctagtgaa cagaatggaa     2700
   tggagcaaaa gacaattatt ttaataccct ctgatttagc atgtagactg ctggggcaat     2760
   caatggatga aagtggatta ccacagctga ccagttatga ttgtgaagtt aatgctccta     2820
   tacaaggcag cagaaaccta ctgcagggtg aagaattact cagagctttg gatcaagtta     2880
   actgagcttt ttcttaattt cattcctttt tttggacact ggtggctcat tacctaaagc     2940
   agtctattta tattttctac atctaatttt agaagcctgg ctacaatact gcacaaactt     3000
   ggttagttca attttgatcc cctttctact taatttacat taatgctctt ttttagtatg     3060
   ttctttaatg ctggatcaca gacagctcat tttctcagtt ttttggtatt taaaccattg      3120
   cattgcagta gcatcatttt aaaaaatgca cctttttatt tatttatttt tggctaggga    3180
   gtttatccct ttttcgaatt atttttaaga agatgccaat ataatttttg taagaaggca      3240
   gtaacctttc atcatgatca taggcagttg aaaaattttt acaccttttt tttcacattt     3300
   tacataaata ataatgcttt gccagcagta cgtggtagcc acaattgcac aatatatttt     3360
   cttaaaaaat accagcagtt actcatggaa tatattctgc gtttataaaa ctagttttta     3420
   agaagaaatt ttttttggcc tatgaaattg ttaaacctgg aacatgacat tgttaatcat     3480
   ataataatga ttcttaaatg ctgtatggtt tattatttaa atgggtaaag ccatttacat     3540
   aatatagaaa gatatgcata tatctagaag gtatgtggca tttatttgga taaaattctc     3600
   aattcagaga aatcatctga tgtttctata gtcactttgc cagctcaaaa gaaaacaata     3660
   ccctatgtag ttgtggaagt ttatgctaat attgtgtaac tgatattaaa cctaaatgtt     3720
   ctgcctaccc tgttggtata aagatatttt gagcagactg taaacaagaa aaaaaaaatc     3780
   atgcattctt agcaaaattg cctagtatgt taatttgctc aaaatacaat gtttgatttt     3840
   atgcactttg tcgctattaa catccttttt ttcatgtaga tttcaataat tgagtaattt     3900
   tagaagcatt attttaggaa tatatagttg tcacagtaaa tatcttgttt tttctatgta     3960
   cattgtacaa atttttcatt ccttttgctc tttgtggttg gatctaacac taactgtatt     4020
   gttttgttac atcaaataaa catcttctgt ggaccaggca aaaaaaaaaa aaaaaaaaaa     4080
   aa                                                                    4082
<210> 18
   <211> 15164
   <212> DNA
   <213> Homo sapiens
<400> 18
   cgctccgccc gcccggccgc cccgagcccc gagccccgag ccccccgcgc cgggcccggg     60
   cggcagcggc ggcggcggcg gcggcggcgc gcccggcccc ctcccccggc gccggccacg    120
   ggaggcggtg atgcgggcgc gggcggcctc ggctgcgccg agagcggaga cacaggctca    180
   agatggcaga ttccgactga ggctgggggg gccgagctcg cgcgccgctt tcccgtcccc    240
   gttgccatga accgcggaca ccccggcccc gatggccccc gtgtacgaag gtatggcctc    300
   acatgtgcaa gttttctccc ctcacaccct tcaatcaagt gccttctgta gtgtgaagaa    360
   actgaaaata gagccgagtt ccaactggga catgactggg tacggctccc acagcaaagt    420
   gtatagccag agcaagaaca tccccctgtc gcagccagcc accacaaccg tcagcacctc    480
   cttgccggtc ccaaacccaa gcctacctta cgagcagacc atcgtcttcc caggaagcac    540
   cgggcacatc gtggtcacct cagcaagcag cacttctgtc accgggcaag tcctcggcgg    600
   accacacaac ctaatgcgtc gaagcactgt gagcctcctt gatacctacc aaaaatgtgg    660
   actcaagcgt aagagcgagg agatcgagaa cacaagcagc gtgcagatca tcgaggagca    720
   tccacccatg attcagaata atgcaagcgg ggccactgtc gccactgcca ccacgtctac    780
   tgccacctcc aaaaacagcg gctccaacag cgagggcgac tatcagctgg tgcagcatga    840
   ggtgctgtgc tccatgacca acacctacga ggtcttagag ttcttgggcc gagggacgtt    900
   tgggcaagtg gtcaagtgct ggaaacgggg caccaatgag atcgtagcca tcaagatcct    960
   gaagaaccac ccatcctatg cccgacaagg tcagattgaa gtgagcatcc tggcccggtt   1020
   gagcacggag agtgccgatg actataactt cgtccgggcc tacgaatgct tccagcacaa   1080
   gaaccacacg tgcttggtct tcgagatgtt ggagcagaac ctctatgact ttctgaagca   1140
   aaacaagttt agccccttgc ccctcaaata cattcgccca gttctccagc aggtagccac   1200
   agccctgatg aaactcaaaa gcctaggtct tatccacgct gacctcaaac cagaaaacat   1260
   catgctggtg gatccatcta gacaaccata cagagtcaag gtcatcgact ttggttcagc   1320
   cagccacgtc tccaaggctg tgtgctccac ctacttgcag tccagatatt acagggcccc   1380
   tgagatcatc cttggtttac cattttgtga ggcaattgac atgtggtccc tgggctgtgt   1440
   tattgcagaa ttgttcctgg gttggccgtt atatccagga gcttcggagt atgatcagat   1500
   tcggtatatt tcacaaacac agggtttgcc tgctgaatat ttattaagcg ccgggacaaa   1560
   gacaactagg tttttcaacc gtgacacgga ctcaccatat cctttgtgga gactgaagac   1620
   accagatgac catgaagcag agacagggat taagtcaaaa gaagcaagaa agtacatttt   1680
   caactgttta gatgatatgg cccaggtgaa catgacgaca gatttggaag ggagcgacat   1740
   gttggtagaa aaggctgacc ggcgggagtt cattgacctg ttgaagaaga tgctgaccat   1800
   tgatgctgac aagagaatca ctccaatcga aaccctgaac catccctttg tcaccatgac   1860
   acacttactc gattttcccc acagcacaca cgtcaaatca tgtttccaga acatggagat   1920
   ctgcaagcgt cgggtgaata tgtatgacac ggtgaaccag agcaaaaccc ctttcatcac   1980
   gcacgtggcc cccagcacgt ccaccaacct gaccatgacc tttaacaacc agctgaccac   2040
   tgtccacaac cagccctcag cggcatccat ggctgcagtg gcccagcgga gcatgcccct   2100
   gcagacagga acagcccaga tttgtgcccg gcctgacccg ttccagcaag ctctcatcgt   2160
   gtgtcccccc ggcttccaag gcttgcaggc ctctccctct aagcacgctg gctactcggt   2220
   gcgaatggaa aatgcagttc ccatcgtcac tcaagcccca ggagctcagc ctcttcagat   2280
   ccaaccaggt ctgcttgccc agcaggcttg gccaagtggg acccagcaga tcctgcttcc   2340
   cccagcatgg cagcaactga ctggagtggc cacccacaca tcagtgcagc atgccaccgt   2400
   gattcccgag accatggcag gcacccagca gctggcggac tggagaaata cgcatgctca   2460
   cggaagccat tataatccca tcatgcagca gcctgcacta ttgaccggtc atgtgaccct   2520
   tccagcagca cagcccttaa atgtgggtgt ggcccacgtg atgcggcagc agccaaccag   2580
   caccacctcc tcccggaaga gtaagcagca ccagtcatct gtgagaaatg tctccacctg   2640
   tgaggtgtcc tcctctcagg ccatcagctc cccacagcga tccaagcgtg tcaaggagaa   2700
   cacacctccc cgctgtgcca tggtgcacag tagcccggcc tgcagcacct cggtcacctg   2760
   tgggtggggc gacgtggcct ccagcaccac ccgggaacgg cagcggcaga caattgtcat   2820
   tcccgacact cccagcccca cggtcagcgt catcaccatc agcagtgaca cggacgagga   2880
   ggaggaacag aaacacgccc ccaccagcac tgtctccaag caaagaaaaa acgtcatcag   2940
   ctgtgtcaca gtccacgact ccccctactc cgactcctcc agcaacacca gcccctactc   3000
   cgtgcagcag cgtgctgggc acaacaatgc caatgccttt gacaccaagg ggagcctgga   3060
   gaatcactgc acggggaacc cccgaaccat catcgtgcca cccctgaaaa cccaggccag   3120
   cgaagtattg gtggagtgtg atagcctggt gccagtcaac accagtcacc actcgtcctc   3180
   ctacaagtcc aagtcctcca gcaacgtgac ctccaccagc ggtcactctt cagggagctc   3240
   atctggagcc atcacctacc ggcagcagcg gccgggcccc cacttccagc agcagcagcc   3300
   actcaatctc agccaggctc agcagcacat caccacggac cgcactggga gccaccgaag   3360
   gcagcaggcc tacatcactc ccaccatggc ccaggctccg tactccttcc cgcacaacag   3420
   ccccagccac ggcactgtgc acccgcatct ggctgcagcc gctgccgctg cccacctccc   3480
   cacccagccc cacctctaca cctacactgc gccggcggcc ctgggctcca ccggcaccgt   3540
   ggcccacctg gtggcctcgc aaggctctgc gcgccacacc gtgcagcaca ctgcctaccc   3600
   agccagcatc gtccaccagg tccccgtgag catgggcccc cgggtcctgc cctcgcccac   3660
   catccacccg agtcagtatc cagcccaatt tgcccaccag acctacatca gcgcctcgcc   3720
   agcctccacc gtctacactg gatacccact gagccccgcc aaggtcaacc agtaccctta    3780
   catataaaca ctggagggga gggagggagg gagggaggga gagaatggcc cgagggagga   3840
   gggagagaag gagggaggcg ctcctgggac cgtgggcgct ggccttttat actgaagatg   3900
   ccgcacacaa acaatgcaaa cggggcaggg gcgggggggg gggggggggc agagggcagg   3960
   gggacgggtc gggacaccag tgaaacttga accgggaagt gggaggacgt agagcagaga   4020
   agagaacatt tttaaaagga agggattaaa gagggtggga aatctatggt ttttatttta   4080
   aaaaagaaaa aggaaaaaaa aaaagtcaat aacaaaaaac ccagctcaag aacccattct   4140
   acgccaaact ggaaaggaga agagagcaac aggaagattc cagaaacggg gggccccagt   4200
   ttttgaagaa ctttatgaac ttttcaaaga ttattttcat atggcagcaa gtgatacgga   4260
   agactgctgt cagggacacc tgatatggaa atcaaataga tttttaatta attgaacata   4320
   agatttaggg atttttccag aactcgaaag ggtcaacagc cctccagaat gtcgggctgc   4380
   agcctgagga ggctgatgtt tggagctggt gtgggattgg cgaagcccag tccgggctcc   4440
   ctagtcagga aagacggggg acggccaggc tgctggaagg cccccggggg cgcggggcga   4500
   gttttctttt tctgagcact ctggataaat ccctaagcaa cgttgtttct caaatgtcat   4560
   taataatgtg tgttgcaaac tttaggtttt tttcttttct gaaaatgtat tttctctttg   4620
   aatccacccc tagtcgcgta gcgtagggct agcggtcgtc acagacaccc tagtagaatg    4680
   tagcactcag cacccttgtc tcctaccttg tgttcaactc caatgatacc aatagaatat    4740
   tcctcaatgt aattgcacaa aaaaaagcga tataacatag gcatgtaacc aatgtggcgg 4800
   tgcaggtgtg cgggtgagcg agcacgtgtg ggtgtgcacg cgccgccctc cccgcgtggc 4860
   cctcggcgcc gccaccctag ctggcgcagt cttgacactg catccttccc tcctagtgcc 4920
   ttaccgagcg acagacgcgg cgtgagggtt tacttccact ggtactccaa gaaactgagg 4980
   ctagtcagac acaatctcag ctcttctgtt gtgctgttgt aacagtttac gctggccttt 5040
   tttttttctt tttctttttc tttttaaaat gttaatgccc gttgtctttc ctgggctgtt 5100
   tgctagcgga aggatgccag ggaagccagc aggagctagg agagagtccg tggatctcga 5160
   aagaaatatg ggagacagat gcccggcggg tgcgtctgga gatggggacg gcgggagttg 5220
   agttgtggca gtagttgagt tgtaatttgt gggcggaggc ccagagagac tccccaccct 5280
   tcacccctgc cccactctgt ccccagttcc gccatttgtg aggccagagg tttccggact 5340
   gttggcctcg ccaggcagcc gtctcccgcc ccaggcggca tcccccagtc cctcccgcct 5400
   ccacgagagc ctggagctct cagcctcgcc cggggctcca ctctctcctc cggctccctg 5460
   ggctgttttg ctctaacgat cttgccagat ccctccctct gtagacaacc accaacctct 5520
   gtttgctgtt gaattctctc ctcacattac ccaggtctgc tcaagacatg attttggttt 5580
   tggtttctga gggttctagt gggcagaagg ttggagggac acttatgagg gtggccgggg 5640
   gtctgacgct gcactttgga aaaactcaca cagttgaatt tccaaagaaa tctgcccttt 5700
   gccctctttg cacctttgat acattctgga agttttctca ggctttggac acttctgggg 5760
   atggaggtgt ggagaagtgg ggagttccct ctcttcatag taaataactc tgaaatatgt 5820
   gaatgtgaat ggcaggagaa tctggccaag gatggggccg aaaagggtgg ttctaattgt 5880
   ttgcttctga tgttgagtct ttagctgacc ccacaggcag gtttccaagg tgcaaagaga 5940
   tctttcccga gtcagcggcc ccatcctcat cctccctccc tttacttcct cactgtgcag 6000
   tctccctcaa ggatctactg tgaaaggtgt gtttgtagtg atatccaacc taactcagta 6060
   acgaagtcgt tacttagctc ttagctgtga aataactctg gaaacttccc caccccaacc 6120
   ataaattctt acttataaag aaacaggtcc ccaaactgga aacagcttag tccaggcctc 6180
   agcgagaagg aaggacacca tgactgctcc atgctgggca cagccgggca gtcttgccaa 6240
   gtgcctgctg gaggctgtgc cggcaagagg cctgcagcaa ggagattccc ttccctcggg 6300
   ccattatcaa tactgtcttt atctggaggt ggggaagcgc agccctctga gacagcagga 6360
   caatggtcag ttcagagagg gtgagggcag caaacgcttc agaggacaca gaagccagag 6420
   gacccccccc cgccccacag ctgggtcagc ctggaaaatc catctattag ggactttttg 6480
   gcagccagat ggcagcaata gcccattagg tctcatcccg agttccaagt cttggctgca 6540
   aatgagcctc agttcgcctt actggagagc acccccagat tcctgggcac agttcatttc 6600
   cagccctttc tagatctgat cttttagggg gaaagacagc ttaaaatgtt cttttcattt 6660
   taaagaaaat tattctgtct gcttaagttg gaggctactt actctttcac ctgacatttt 6720
   ctttcctttt attcttccag atcaggaatg aaatttccat gctgctcata aagataatat 6780
   tattgtacta attattttta ttaccattgt aattatgatc attatgttga tattttagtc 6840
   agggttttaa atgcacattt attccaagta tctttgtgtt ttctctttaa tatttaaact 6900
   tattctctct gtgagtatat aagtagactg gagggacatc cagatgtcca gttttgtcag 6960
   gcaaaaaaaa aaaggaaaga cttaggaagt aggaaaattg tttctgtcat ctctatccca 7020
   acaagagacg tcaagaaaga tccaccacag aacaaaagtt taaagaagaa tcaaagcctt 7080
   gattgggctt ctgacaacat ggtcaccatc aaggttgtca ttttctagat cccagaggcc 7140
   tgggatgcga cgtcaggtgg catctcatgg gctcggggaa tgtcgagtca ctgactgtcc 7200
   agcccttagc cagcttctct cccacatcct cagagctctc ctgtgcttct gaaatctgtt 7260
   aactaaatct ttggcttgcc tctggtattt aagcaagaaa attccctccc agaggtgacc 7320
   ccatccgctt ccccacaatc catccttttg ccatcggcgc acctggggcg tggcttaggt 7380
   tcttcaatgc agggacattt gccccctccc agaagctgct gggcacagtg aggtggcgta 7440
   agagtgactg gcaggtggta ccttccccag gaaatttcac cacaccaccc agttcctcag 7500
   cctgccccct ccccctgtga tgcatgcccc cagcacccaa ttctagccag ctggaagtgg 7560
   gtggagggac agcaggaggc cagagaaacc ctgaacaaag ctgggcggct gctcaggcat 7620
   cacaggctgc accccctctg aaagcatccc cactgggctc cggccacatc ttcagtgcac 7680
   tgtgctgtgt gcgctgggtg ctcacacgct gtccccagac ccacaaagtg ctaggcccca 7740
   gttgaagaaa ggggtgaaat agccagcttc accgaaggga agggaaggga agtattgggc 7800
   gatgccagcc ccacagacgc tcagcaaaca ttagtgcaca ttctcctagt cctcacccaa 7860
   tggcctcctc tacccccatg catggagctg ccacatcaga agccccaaga gaagctccct 7920
   gcaggagagg ccagctccct ggatgcccaa ttgcatacct ggccgaatct gccattgagt 7980
   caccttagca aataggctgc tgtcactagg accaagctct caagcagagg gatgccaacc 8040
   tagtccttac ttagcccacg aatcatctag agcatcctct agtcttttgt gggctccctc 8100
   cttcccattt gaagagacat tgttcagagg aagaggggaa gatttgaaat gtcaggtcac 8160
   ggaggagtgt ttaactggag cctggtgaac cgcagggcaa tttgcttctg ctcactgggt 8220
   tctgactggc ccgtctggac gtgggccccc atgtctctgt gcttagggcc tcttcatgat 8280
   gttttggatg tttccaaggg aagtgggtga gcagatcaag gggtgggaga gtcgaggctt 8340
   gatgccagtt aatactgtga agtggagcgt gcggtcagtg gaattcagag gaaaaagaag 8400
   ggttggagca aagcggcatt catctcctgg actgttagcc tttctagtct tcctggtggc 8460
   tgaggtgttc acgggctggg ggagccagct gacctttgtc ctcttcaacc tagaagactc 8520
   agcccgccca gacaccaacg tgtgagacgg atggacatca ggaagggaag gggagattag 8580
   cccaactgct gacagaacga tttcccttgg ttggaccttg ggaatggcaa acactcatat 8640
   tggaacaagc ttggggtgga agatttaggc cgtgtgagca tgtgtgagtg agtggaacaa 8700
   actttcttgg aaactggagg gaggagatga ggaggcttcg ggaagtatta ctgatggctc 8760
   atggttgaga gagcgacgtg gggacccagc tcgccccagc ttttgtccca ggttctcttt 8820
   gtctgatgct gagggcaggg tggggtgtgg gaccaccact cttgttggcc tgtcaagtag 8880
   accctaggac agaaaatgga aagaaggaaa tggctcggtg ctctcaacta gcagagagaa 8940
   ttgaggagag gtaagggttc cttctgcagg ccagcctggg actccacagc gccagcagga 9000
   gtgacttggc cacaagacat tccagcccca gggactttgc aggcttcatt ccctgtctgt 9060
   gtcttttcct tctggtgtgt tttacagact tctgatgggg aagcttcaaa cttgagcagg 9120
   ccagagatgt ccttaccaaa ttggaaagga aggtgaaact gttcctttct ttagccaaag 9180
   aacccttctc aaagacgcct ccagaaatgg acaaaatggc cttcccttcg ttcctttcca 9240
   ggcaataatg acatcattag tgatgcaatt ctatttgtct ttctctttcc tctctgtcct 9300
   tttttttaaa aaaaaaaaat gcatttattt caaaactgtg ctattctttt aagaggagtg 9360
   gaggtgaccc cttcgatgct gctgctatcg ggagacaagg tgccatacca atacgtgggc 9420
   ttgactaatc ccaggccacc atgggagaga gcaaagcagg gctgccagga gttcagttgc 9480
   atcaagggcg tagagcacgc gggggctggg ctcggaatag cagtactttt ccactttgat 9540
   gccttagaac tctcacttct catctccaca gaccagactc agtaaaatct caggccacta 9600
   gagaatggaa ggcggtgaaa caggatttaa atgcaaaaaa aacctattgg aggcttttgg 9660
   caccgtggct cactagaggg acccagcata gtagaggttt ctcttgttgc agcttctgaa 9720
   aagttcaaaa aagaactcca ggccgttctt ccctcaaacc cagtgagagt ttgcagagaa 9780
   gtgccccctg cagggctccc gtcccagaac accagcacca gagagggtct tcccgatgcc 9840
   ccccgctgga cttgcccaag cctctgggag cccctcatct cagatccctg tgttgaacat 9900
   gacactgact gtcccttatt tgttaaaatt tgcaatatct ctcaagtaaa taatagccaa 9960
   catttgttga atgctttcat gactcccggg ctaaggcctt tatgagcgtt gtctcaaggg 10020
   gccccaacag ccatcccaca gggaggggga taacagcccc catttataga taagggagct 10080
   gaccggatgc tctgagaagt ggcaggtgtt gaaggaagga taaagcagtg atgggccaga 10140
   atccccaagg ttcccttttt tgttcatcag gcccttcctg agatgtgatt tttaatcttt 10200
   taactttttt taattaatag caatgcgtgg cctcatattt ctatgaacca tttagtgata 10260
   ctcccgcttc ctgcatgcca cacactgtgc tgggaattgc tcatgggttg tcctgtttca 10320
   tcttctccgt agccctgtga gatcggcaat attagtcccc ctacagccaa ggaaactgcc 10380
   cagagccaca caactcttga ggggcgagga gggcttgaac ctgagtctgc ccagctccag 10440
   aactgagctt gcagccatta gccacagctg tctcctgcat gtctgagcaa agaaaggcct 10500
   ttacacagca tcaccctgtg ccatcccatg caccgtggga ctcagctaaa ggactgtgca 10560
   aagagggggc tcctgagttg gatttaggca aaaggggcag aattcgtttg atttttagag 10620
   aaaatctctg gagagtttct tttgattcat agaattcctt ttagatttct ttccagcata 10680
   ccaactagct ttagtagtgc tgctacaacc agctcttata agtaagagtg aaaaagtatt 10740
   cttttcttct ttaaaaaata agtttttctt gcttatagtt aattctagaa aggcaatact 10800
   aaaggtatat atttttttca aaatgctatt ttttactgca cttgataatt atcctgacag 10860
   ctctgatctc tgtaatagat tcactcttca gctctgggca gaaccagagg cagggttcac 10920
   accaaatttg taaataccat atgtgggtct ggtgtccagg aacttttttc tttctgttaa 10980
   aaaaaagaaa aaaaaaagaa aaaaaaaaag aagtagaggt ggaagaaaga caagacttag 11040
   aggaacaaaa gaatgttttc ttttgagata ctcttctcaa agaaatagca acaattgtat 11100
   aaacaggaaa accagccagc tttcatgata aaaggaaggc gtgtctcttg ccctggtatg 11160
   agattaacag aaatacagat gcatttttat tttgattgaa agatggtgag aatgtagaaa 11220
   tgcttaggac tagattttta attttttaaa ataactatta tcatttatta tgaaatattt 11280
   gttcagttgt tttgagtggg tttcttgttc cttttttcat ttaaaacctt ctttgttgac 11340
   tggctccagg cttgtttgcc taaattctta ggtagtttac acaagttcta gaatctttta 11400
   gaactttaac tccattggaa gcaaacctaa ctaatcggag tttgagatcc tggttggttt 11460
   caataggtat tctggaattc tggcagaaca cctaaagatt tttttttttt ttagaaggtt 11520
   ttagatacat tatcttacac aaactgtgac ctaatggcaa taattacctc aaatgtgggc 11580
   attcatcctg gttttagcct tttttgaaat catgtagcca gcttgatctt ggaatttaaa 11640
   gactatgaat tctctgtggg ctgaaaataa tgattacttc atacccccgg tcatcgttgc 11700
   ttaagtgaat tctgaaaata gctcatcttt acaacaaaaa ttaaaccaag gaagagatta 11760
   ttctttgtgt gttgtactca aatgcgatgt tcaaatgcac atgttaagta tatatgtttt 11820
   tagctactgt aaaatgctgt tagccttcta agctatcaaa acagtcacat tttaaatgag 11880
   taaactaaac aattgactgt ggatacttaa gcatatttct ggctacgttt tatagttaaa 11940
   gtgttttata gtttacattt agactggtac tttttaaaga aaagttctgt ttataactga 12000
   catccgcaaa ccccagtgaa tgcctcttag ttggaggttg tgtctccccc aaggcaagtg 12060
   tgttgtccca gactcttctg tagtccagca tgcgcacttc cctctggatt attactttcc 12120
   acgtgaactc aagagaacat gaaaggcaat ccagatggag ggaaaaggtg tgagtccgca 12180
   gcccgggcca gatgcgaagg tctcatgcgt gtcgtctaaa cacttgtctt caaggccttc 12240
   tctctgacat cttggaagag tcattgagaa cagataacct ggttcattga tttttgtctt 12300
   gatttgaata tttaacttat taatagatcc actgatttcc aggcaccagg cagtagaaga 12360
   gactgggatt caggtgacca tgaaggcaca gctgctactt ctgggccggg ggtgatattt 12420
   tgatcagcgt tttgtagggg aggaccatat acccctattc ccatggtcgc tggctgggtt 12480
   ttccatatat ctgctgtcat ttattcgttt tccccttaaa agcaaaatca atgtaaaagg 12540
   ctatgtttac gttttactca ttgtccagct tagactcaaa gtctagttcg gtgggagggg 12600
   gaccttagca tcctctcaga gatggtcagg gctgagcagg aggaggcaga gacagagggg 12660
   cagctcagcc tggtccattg agacccactc taaacagaca tcatatttgg aacaagaaga 12720
   tgcttcgaga caggaatggg ccccactgtc atgcagaaac agactggggg aatggcagtt 12780
   tccctgagtc ttggtttctt ttatgttttc tcttgtgcca ccaccaaact gcagaggacc 12840
   tgctgtgacc taaagggcat tcctttagca gataagacct tgaaaactgc aaaacacctg 12900
   ggaccaggga gcttttaaaa aatacaaaaa aataccacat ttgctttttc cctgtgaact   12960
   gtattgacag cgtgttctta ggacagtctt ttggtggaaa tgttactgta aaatagtttt   13020
   catctcaccc ctcctaatca tactcccact ttcctgtttg tgtggtggtg ttgctgtttt   13080
   ttcctttaca tgaattaacc aaatgaattt tgtgtcattg tttttggggc ttatattttt   13140
   aaaacataga aattgccttt tgttcatttg aaaagtaagt atgttgtatc tgaaaaaggg   13200
   ctctgcctct gctctccctc gcttccttgt aaccaatctc caaacgaatc tctcctggca   13260
   ccgccccctt ccttatatag ggtcactgtc cccggggcca cctctgcctc caccctgctg   13320
   tcaccactgc cctgggccaa ggcacccagg actcccagaa agcgcgagag ccagcaagaa   13380
   ggccccactc agccttgaga ctggtggtca cacctccctg tcagagtcgc ctgctgggct   13440
   gaaggggcaa tggattgtca ttgttgaaat tgtttggctc aggttataag gaggaacttg   13500
   ggaagtagaa agtgacttga ccatgtgcat ccttggtagc ttcctgtaac taacaaatgg   13560
   aacagagagc acacccccgc cccgccccac cccaagcaga tgttcccgtc agcgctgccc   13620
   tgagtcagtc ggtcccccgt ttctgctctc ctcccttttg tgttcctgct cacttcaagc   13680
   ttcttccatg gactttccag ggcacagtca tctctagccc ccaaatcatc tcttcatcct   13740
   ctgtgtgtgc atttttttaa ccaaatgaaa tagacaagaa agtcatactt tggggcagca   13800
   gaatttctaa tttagtagaa tcactgtata gagatagatg ttgatatata tgtttgtgta   13860
   tatatatcaa accaaattgg ataggagaag tatagcttta cagatgagga gaaggagctc   13920
   tttagaggtc ggagtcaaga ctggtgtctt ggacgtgcat gggctgtgtc ccaggccact   13980
   ccgcacacat ggggctgagg cgtggcgccg ggcccttgtc atccacctca ccacggcaga   14040
   gccagcaggc cctgtagggt gctgctgctg tctcactggg tcccagcttc aagcgcatca   14100
   gtgggtgacg ggggcaacaa atcagagtga ctggaagttt ccatcccgtt ttgctttgac   14160
   cacgtgtact gagctgcagc ctctgatact ctgtcacgtt tccaaaaatg gtatccatta   14220
   ggatagaaag agaatggatc tgcagaaatg tttacctttc aactgctcat gaattcagga   14280
   cactggatag aaagactcac tccccaaaat gagaacaggg aagaggagac ccggcgacac   14340
   taagtcacca ggtccaagga acgtggctcc ctccccaggg tcatctcacc tagatctttc   14400
   tctcccaggt catctcagct caatctcaat aaccctatga aagccctggt ctgttgtgtt   14460
   ccttcaccgt acggtttctg taataaaaag tgttaatcca tgttaatctg tgtgaaaatt   14520
   attgcgtgca acagtatttt ctcgtgtacc tctttttcct atgtgaattg tccctctttt   14580
   ttatttataa atgtctactt ttgttttttt aaagacaaac caatgtgttg tagacctata   14640
   tgtaacctat tccttagtct catattatag gtatgttata agaatggata ttttacttgg   14700
   ctttagaatg ttttacaaga aaactaattc ttaactgatc aagtccttgc tactaaaatg   14760
   cttgtgtttt tcatcatgac gtcgtgtgct tctaaattaa tcattttcgt tgtagaaaaa   14820
   tggagtgaat ttatattagt cttggaaact aataatagca ttgtaaattt atgagatgat   14880
   tttaacagaa aaaatataga agaatatagt tattttaatt gtaatattac taactgtagg   14940
   gtgagaaaaa gggggggggg tcccattgtg gtgaactatg ttatagcttg ttactcatag   15000
   tttctttttg atcatttttt cggtctccga ggtgaaatga cttattaatt aaaatttgta   15060
   aactcacata tgcatattgt atatgtgtag aaatgtaatc acactttgtc ttggaattac   15120
   attaaactgt ttgaaatcac tgtaaaaaaa aaaaaaaaaa aaaa                    15164
<210> 19
   <211> 2300
   <212> DNA
   <213> Homo sapiens
<400> 19
   ttattgtggt ttgtccgttc cgagcgctcc gcagaacagt cctccctgta agagcctaac     60
   cattgccagg gaaacctgcc ctgggcgctc ccttcattag cagtattttt tttaaattaa    120
   tctgattaat aattattttt cccccattta attttttttc ctcccaggtg gagttgccga    180
   agctgggggc agctggggag ggtggggatg ggaggggaga gacagaagtt gagggcatct     240
   ctctcttcct tcccgaccct ctggccccca aggggcagga ggaatgcagg agcaggagtt    300
   gagcttggga gctgcagatg cctccgcccc tcctctctcc caggctcttc ctcctgcccc    360
   cttcttgcaa ctctccttaa ttttgtttgg cttttggatg attataatta tttttatttt    420
   tgaatttata taaagtatat gtgtgtgtgt gtggagctga gacaggctcg gcagcggcac    480
   agaatgaggg aagacgagaa agagagtggg agagagagag gcagagaggg agagagggag    540
   agtgacagca gcgctcgcgg gggctcaacc cccagacctc cagaaatgac gtcagaatca    600
   tttgcatccc gctgcctcta cctgcctggt ccagctggga ccctgcctcg ccggccgcat    660
   ggccagaggg ttggaaatta atgatcatga gctcgtattt gatggactct aactacatcg    720
   atccgaaatt tcctccatgc gaagaatatt cgcaaaatag ctacatccct gaacacagtc    780
   cggaatatta cggccggacc agggaatcgg gattccagca tcaccaccag gagctgtacc    840
   caccaccgcc tccgcgccct agctaccctg agcgccagta tagctgcacc agtctccagg    900
   ggcccggcaa ttcgcgaggc cacgggccgg cccaggcggg ccaccaccac cccgagaaat    960
   cacagtcgct ctgcgagccg gcgcctctct caggcgcctc cgcctccccg tccccagccc   1020
   cgccagcctg cagccagcca gcccccgacc atccctccag cgccgccagc aagcaaccca   1080
   tagtctaccc atggatgaaa aaaattcacg ttagcacggt gaaccccaat tataacggag   1140
   gggaacccaa gcgctcgagg acagcctata cccggcagca agtcctggaa ttagagaaag   1200
   agtttcatta caaccgctac ctgacccgaa ggagaaggat cgagatcgcc cactcgctgt   1260
   gcctctctga gaggcagatc aaaatctggt tccaaaaccg tcgcatgaaa tggaagaagg   1320
   accaccgact ccccaacacc aaagtcaggt cagcaccccc ggccggcgct gcgcccagca  1380
   ccctttcggc agctaccccg ggtacttctg aagaccactc ccagagcgcc acgccgccgg   1440
   agcagcaacg ggcagaggac attaccaggt tataaaacat aactcacacc cctgccccca   1500
   ccccatgccc ccaccctccc ctcacacaca aattgactct tatttataga atttaatata   1560
   tatatatata tatatatata taggttcttt tctctcttcc tctcaccttg tcccttgtca   1620
   gttccaaaca gacaaaacag ataaacaaac aagccccctg ccctcctctc cctcccactg  1680
   ttaaggaccc ttttaagcat gtgatgttgt cttagcatgg tacctgctgg gtgttttttt   1740
   ttaaaaggcc attttggggg gttatttatt ttttaagaaa aaaagctgca aaaattatat   1800
   attgcaaggt gtgatggtct ggcttgggtg aatttcaggg gaaatgagga aaagaaaaaa   1860
   ggaaagaaat tttaaagcca attctcatcc ttctcctcct cctccttccc cccctctttc   1920
   cttaggcctt ttgcattgaa aatgcaccag gggaggttag tgagggggaa gtcattttaa   1980
   ggagaacaaa gctatgaagt tcttttgtat tattgttggg ggggggtgtg ggaggagagg   2040
   gggcgaagac agcagacaaa gctaaatgca tctggagagc ctctcagagc tgttcagttt   2100
   gaggagccaa aagaaaatca aaatgaactt tcagttcaga gaggcagtct ataggtagaa   2160
   tctctcccca cccctatcgt ggttattgtg tttttggact gaatttactt gattattgta   2220
   aaacttgcaa taaagaattt tagtgtcgat gtgaaatgcc ccgtgatcaa taataaacca   2280
   gtggatgtga attagtttta                                               2300
<210> 20
   <211> 1602
   <212> DNA
   <213> Homo sapiens
<400> 20
   ggcttaggct gagccgtggc cgccacagcc catcgtaatg ccgcatggtg cttggcactc 60
   cagagagcca ataggaatga aagaattcat ttgaatcggc caatgccggc gggttagggg 120
   gcgggggttg aaaaccctat aaaggcgtcg atcggccgga caggcggcag cggcggctcc 180
   tgcagcggtg gtcggctgtt gggtgtggag tttcccagcg cccctcgggt ccgacccttt 240
   gagcgttctg ctccggcgcc agcctacctc gctcctcggc gccatgacca caaccaccac 300
   cttcaaggga gtcgacccca acagcaggaa tagctcccga gttttgcggc ctccaggtgg 360
   tggatccaat ttttcattag gttttgatga accaacagaa caacctgtga ggaagaacaa 420
   aatggcctct aatatctttg ggacacctga agaaaatcaa gcttcttggg ccaagtcagc 480
   aggtgccaag tctagtggtg gcagggaaga cttggagtca tctggactgc agagaaggaa 540
   ctcctctgaa gcaagctccg gagacttctt agatctgaag ggagaaggtg atattcatga 600
   aaatgtggac acagacttgc caggcagcct ggggcagagt gaagagaagc ccgtgcctgc 660
   tgcgcctgtg cccagcccgg tggccccggc cccagtgcca tccagaagaa atccccctgg 720
   cggcaagtcc agcctcgtct tgggttagct ctgactgtcc tgaacgctgt cgttctgtct 780
   gtttcctcca tgcttgtgaa ctgcacaact tgagcctgac tgtacatctc ttggatttgt 840
   ttcattaaaa agaagcactt tatgtactgc tgtctttttt ttttttcttt tgaagaacag 900
   gtttctctct gtccttgact cttgggtctg tgggccatgg catgagtgtt ttctagtagt 960
   agattggagg gaaagctttg tgacacttag tactgtgttt ttaagaagaa ataatttggt 1020
   tccagatgtg ttagaggatc ttttgtactg aggtttttaa cactttactt gggtttacca 1080
   agcctcaact ggacagacca taaacagtcc acaggcaccg ttcctgccag gccccaaccc 1140
   acagggagtc tctccgcaga gccttcttgg tgttgcccta acttgccagt ggcctttgct 1200
   cagagcctcc tcctgtgaca tgtgaacaat gaagaggcct gcgcctcctg ccttgccgcc 1260
   tgcaaagcaa agaaactgcc ttttattttt taaccttaaa aagtagccag atagtaacaa 1320
   gactggctgg ctgatgagca aagcctttgc tctcacgcag aggaaggctt ggatgtacaa 1380
   tgaaactgcc tggaactaaa agcagtgaag caagggaggc aatcacactg aagcgggtct 1440
   tcctccagga acggggtccc acaggcgtgt tgttttaaat aacctgatgc tgtgtgcatg 1500
   atgctggtgc ttgaccatga aaggaaagtc tcatccttaa aatgtgttgt acttcacaat 1560
   cctggactgt tgcttcaagt aaacaatatc cacattttga aa 1602
<210> 21
   <211> 3044
   <212> DNA
   <213> Homo sapiens
<400> 21
   gctttattgt ttgcttgttt tgttccggag tcggggccgg gagggagtgc aggaggaggg 60
   atccaagctt ccaagcctct gctccgctct ccttctatcc agttggtctt tagggcactg 120
   aaggaaactc ttcttcagaa ataacctttt aacttttctt ctgtcagctg cctgccaatc 180
   acggagccag aggctgaggg gaggctttga gccggtctgc gagtccggaa ggcaaagatc 240
   gcgaagcttg gcgctccaga acgctcaggg ggcaggtgac acagtcgtgg gttccccggc 300
   gggcgctggc ttgacagttt cctccccgcc cactggcagg ggagcgcccc gccgggctgc 360
   acgcgcgcgc gcgcaggggg gcataaaagc cgcggccgcg cggagacgcg gagctcgccc 420
   accgcccgcc ccagcagtgg ctgcaccatg cacgtgaacg gcaaagtggc gctggtgacc 480
   ggcgcggctc agggcatagg cagagccttt gcagaggcgc tgctgcttaa gggcgccaag 540
   gtagcgctgg tggattggaa tcttgaagca ggtgtacagt gtaaagctgc cctggatgag     600
   cagtttgaac ctcagaagac tctgttcatc cagtgcgatg tggctgacca gcaacaactg     660
   agagacactt ttagaaaagt tgtagaccac tttggaagac tggacatttt ggtcaataat     720
   gctggagtga ataatgagaa aaactgggaa aaaactctgc aaattaattt ggtttctgtt     780
   atcagtggaa cctatcttgg tttggattac atgagtaagc aaaatggagg tgaaggcggc     840
   atcattatca atatgtcatc tttagcagga ctcatgcccg ttgcacagca gccggtttat     900
   tgtgcttcaa agcatggcat agttggattc acacgctcag cagcgttggc tgctaatctt     960
   atgaacagtg gtgtgagact gaatgccatt tgtccaggct ttgttaacac agccatcctt    1020
   gaatcaattg aaaaagaaga aaacatggga caatatatag aatataagga tcatatcaag    1080
   gatatgatta aatactatgg aattttggac ccaccattga ttgccaatgg attgataaca    1140
   ctcattgaag atgatgcttt aaatggtgct attatgaaga tcacaacttc taagggaatt    1200
   cattttcaag actatgatac aactccattt caagcaaaaa cccaatgaac agcttatgtg    1260
   ttagccatag ctgaaaataa gcacaaatag cttatattca gatcctatct tcatttgaat    1320
   atagctttta aatgaaatgt tacagtttga agttttcctt catgcacttg gtgataaacg    1380
   ttttctaaat ttttagttaa gtatatggat aaaaagttat gaactattaa aaatgtgatg    1440
   tggaccaaag gctaggttgt aatcttgata gtctaaaaaa tgatcaaaac aaatgatttt    1500
   caaggaatat tcaatattct gcctttcaga aagtgtattt atatctgtgc ttcataaata    1560
   ttaatgttct tcagaacatc attttaaagg agatacttga attgttattt aaatcaaacc    1620
   agatgtaaaa cactcacata caagttcata ctttaaaaga ggaaagctac ttaacaatga    1680
   caaatatttc acaataataa tttttactta tataccatct ttcaactgaa catttcagtt    1740
   cttccaagag cttcttagag tagtatattt tgggggcagt caaggaataa actacagtgt    1800
   aaacatatcc cagatgaaaa ctgctgtatg gaaaaatgac agaaagtaac tgattgacac    1860
   tgttgattca cagttcagcc tcctatctgg gaaagacatt tctttcctct gctcacttta    1920
   agaactttta ccgactccaa aaatctcagg aattaaactt ttaacagtta cagcaataaa    1980
   gaatagttag tactccaaaa atattatatt taagatgctc aacaagaaaa aaatgcaaat    2040
   gtaatatttt tttcaaatta cttctttatt gacttgtcca aatttcaaaa gtgcctaccc    2100
   ttcaataaaa cttttttatt ctgatctcca taaattactt agtcttctat gtatagctat    2160
   caaggaaata aaaccaattt tgccacagcc acaactgtaa atgtttttgt acccatgctg    2220
   aaactcataa caacacagac ataaaaatag ctgtgaggtt ttgctttttt tgttgtcagc    2280
   tatcttaaga atcattaaat acacctgctt tgggtaaaac tctttgcaag cagtaattaa    2340
   cactagtaac agtgaaagca caagatttcc aaatcagtcg ttttctcaaa aaaatatcgt    2400
   ataagtgact catcctgtct gctaactcca gacctcccag cttgaagcca aatctttcca    2460
   tgtgagattg atatggattt cctagaagta ctggaatgtt gtcatatctt gccctatttt    2520
   aattctgcta tagaaaacaa ttgccttcac ttttaaggag taatttgaat attaataact    2580
   ctggtctaga ttttcatata atgtattaaa gacaaagtag tgaacatcaa tgaacatctg    2640
   atagagataa actgtaatca ggcataagct tgtttgtatg ttctggcagt gactaatcag    2700
   taaatgatgt cggtttgccc agtatcactt atcttctgta tttttcctct gtcgtgtaaa    2760
   tagtataacc ttttcattta tggacaattt tttggactag tagccttcaa tatacattct    2820
   gctttgaatt aattttttca aatcaataaa ttatgtagac atttaaaatc aaatatcaag    2880
   tagaattgaa aaatgtgagt tacataagtt aaaaacttac tttaaatctt accttctata    2940
   ggtagctcta aataaattca tatggttata tggcatctct ggtgtatact gattgagaaa    3000
   ataattaaac tgaagttagg ggaggggaaa aaaaaaaaaa aaaa                     3044
<210> 22
   <211> 2389
   <212> DNA
   <213> Homo sapiens
<400> 22
   tcgagcccgc tttccaggga ccctacctga gggcccacag gtgaggcagc ctggcctagc      60
   aggccccacg ccaccgcctc tgcctccagg ccgcccgctg ctgcggggcc accatgctcc     120
   tgcccaggcc tggagactga cccgaccccg gcactacctc gaggctccgc ccccacctgc      180
   tggaccccag ggtaaggaca agggccccca gactcacagt tccagccctg aggacagggg     240
   ttccctcatc cccccaccca gcctaatgcc cacctcctaa tagaggggtt cctggggacc     300
   tgaagagggg gcactatgac gtccccccaa gcacctaggt gttctgtcct gctcttcctt      360
   cagactcagc cgttggaccc cagtcctttc ctccccagac ccaggagttc cagccctcag     420
   gcccctcctc cctcatacta gggagtcctg gcccccaaat tcctcctttc ccaagactta     480
   tgatttcagg tcctcagctg tctcctccct caaaccggga tcctcagtcc cctgctccac     540
   caggctcagg catgggggtc cccatccctg caaatccagg cgtccccccg ctgctggtca     600
   gacactgacc ccatccttga acccagccca atctgcgtcc gtgatcacgg cgtgctctgg     660
   ccaaggccca gtccctacag cctgcctgga tggacgcctg ggactggggg cgccaggact     720
   gggctgggct gggctccccc aggccctgcc tccccgtcca tctcctcaca ggtcccaccc     780
   tggcccagga ggtcagccag ggaatcatta acaagaggca gtgacatggc gcagaaggag     840
   ggtggccgga ctgtgccatg ctgctccaga cccaaggtgg cagctctcac tgcggggacc     900
   ctgctacttc tgacagccat cggggcggca tcctgggcca ttgtggctgt tctcctcagg     960
   agtgaccagg agccgctgta cccagtgcag gtcagctctg cggacgctcg gctcatggtc    1020
   tttgacaaga cggaagggac gtggcggctg ctgtgctcct cgcgctccaa cgccagggta    1080
   gccggactca gctgcgagga gatgggcttc ctcagggcac tgacccactc cgagctggac    1140
   gtgcgaacgg cgggcgccaa tggcacgtcg ggcttcttct gtgtggacga ggggaggctg    1200
   ccccacaccc agaggctgct ggaggtcatc tccgtgtgtg attgccccag aggccgtttc    1260
   ttggccgcca tctgccaaga ctgtggccgc aggaagctgc ccgtggaccg catcgtggga    1320
   ggccgggaca ccagcttggg ccggtggccg tggcaagtca gccttcgcta tgatggagca    1380
   cacctctgtg ggggatccct gctctccggg gactgggtgc tgacagccgc ccactgcttc   1440
   ccggagcgga accgggtcct gtcccgatgg cgagtgtttg ccggtgccgt ggcccaggcc    1500
   tctccccacg gtctgcagct gggggtgcag gctgtggtct accacggggg ctatcttccc    1560
   tttcgggacc ccaacagcga ggagaacagc aacgatattg ccctggtcca cctctccagt    1620
   cccctgcccc tcacagaata catccagcct gtgtgcctcc cagctgccgg ccaggccctg    1680
   gtggatggca agatctgtac cgtgacgggc tggggcaaca cgcagtacta tggccaacag    1740
   gccggggtac tccaggaggc tcgagtcccc ataatcagca atgatgtctg caatggcgct    1800
   gacttctatg gaaaccagat caagcccaag atgttctgtg ctggctaccc cgagggtggc    1860
   attgatgcct gccagggcga cagcggtggt ccctttgtgt gtgaggacag catctctcgg    1920
   acgccacgtt ggcggctgtg tggcattgtg agttggggca ctggctgtgc cctggcccag    1980
   aagccaggcg tctacaccaa agtcagtgac ttccgggagt ggatcttcca ggccataaag    2040
   actcactccg aagccagcgg catggtgacc cagctctgac cggtggcttc tcgctgcgca    2100
   gcctccaggg cccgaggtga tcccggtggt gggatccacg ctgggcctag gatgggacgt    2160
   ttttcttctt gggcccggtc cacaggtcca aggacaccct ccctccaggg tcctctcttc    2220
   cacagtggcg ggcccactca gccccgagac cacccaacct caccctcctg acccccatgt    2280
   aaatattgtt ctgctgtctg ggactcctgt ctaggtgccc ctgatgacgg gatgctcttt    2340
   aaataataaa gatggttttg attaaaaaaa aaaaaaaaaa aaaaaaaaa                2389
<210> 23
   <211> 914
   <212> DNA
   <213> Homo sapiens
<400> 23
   gcatggggag gggcggccct caaacgggtc attgccatta atagagacct caaacaccgc       60
   ctgctaaaaa tacccgactg gaggagcata aaagcgcagc cgagcccagc gccccgcact      120
   tttctgagca gacgtccaga gcagagtcag ccagcatgac cgagcgccgc gtccccttct      180
   cgctcctgcg gggccccagc tgggacccct tccgcgactg gtacccgcat agccgcctct      240
   tcgaccaggc cttcgggctg ccccggctgc cggaggagtg gtcgcagtgg ttaggcggca      300
   gcagctggcc aggctacgtg cgccccctgc cccccgccgc catcgagagc cccgcagtgg      360
   ccgcgcccgc ctacagccgc gcgctcagcc ggcaactcag cagcggggtc tcggagatcc      420
   ggcacactgc ggaccgctgg cgcgtgtccc tggatgtcaa ccacttcgcc ccggacgagc      480
   tgacggtcaa gaccaaggat ggcgtggtgg agatcaccgg caagcacgag gagcggcagg      540
   acgagcatgg ctacatctcc cggtgcttca cgcggaaata cacgctgccc cccggtgtgg      600
   accccaccca agtttcctcc tccctgtccc ctgagggcac actgaccgtg gaggccccca      660
   tgcccaagct agccacgcag tccaacgaga tcaccatccc agtcaccttc gagtcgcggg      720
   cccagcttgg gggcccagaa gctgcaaaat ccgatgagac tgccgccaag taaagcctta      780
   gcccggatgc ccacccctgc tgccgccact ggctgtgcct cccccgccac ctgtgtgttc      840
   ttttgataca tttatcttct gtttttctca aataaagttc aaagcaacca cctgtcaaaa      900
   aaaaaaaaaa aaaa                                                        914
<210> 24
   <211> 1660
   <212> DNA
   <213> Homo sapiens
<400> 24
   ggtgcactag caaaacaaac ttattttgaa cactcagctc ctagcgtgcg gcgctgccaa       60
   tcattaacct cctggtgcaa gtggcgcggc ctgtgccctt tataaggtgc gcgctgtgtc      120
   cagcgagcat cggccaccgc catcccatcc agcgagcatc tgccgccgcg ccgccgccac      180
   cctcccagag agcactggcc accgctccac catcacttgc ccagagtttg ggccaccgcc      240
   cgccgccacc agcccagaga gcatcggccc ctgtctgctg ctcgcgcctg gagatgtcag      300
   aggtccccgt tgctcgcgtc tggctggtac tgctcctgct gactgtccag gtcggcgtga      360
   cagccggcgc tccgtggcag tgcgcgccct gctccgccga gaagctcgcg ctctgcccgc      420
   cggtgtccgc ctcgtgctcg gaggtcaccc ggtccgccgg ctgcggctgt tgcccgatgt      480
   gcgccctgcc tctgggcgcc gcgtgcggcg tggcgactgc acgctgcgcc cggggactca      540
   gttgccgcgc gctgccgggg gagcagcaac ctctgcacgc cctcacccgc ggccaaggcg      600
   cctgcgtgca ggagtctgac gcctccgctc cccatgctgc agaggcaggg agccctgaaa      660
   gcccagagag cacggagata actgaggagg agctcctgga taatttccat ctgatggccc      720
   cttctgaaga ggatcattcc atcctttggg acgccatcag tacctatgat ggctcgaagg      780
   ctctccatgt caccaacatc aaaaaatgga aggagccctg ccgaatagaa ctctacagag      840
   tcgtagagag tttagccaag gcacaggaga catcaggaga agaaatttcc aaattttacc      900
   tgccaaactg caacaagaat ggattttatc acagcagaca gtgtgagaca tccatggatg      960
   gagaggcggg actctgctgg tgcgtctacc cttggaatgg gaagaggatc cctgggtctc     1020
   cagagatcag gggagacccc aactgccaga tatattttaa tgtacaaaac tgaaaccaga     1080
   tgaaataatg ttctgtcacg tgaaatattt aagtatatag tatatttata ctctagaaca     1140
   tgcacattta tatatatatg tatatgtata tatatatagt aactactttt tatactccat     1200
   acataacttg atatagaaag ctgtttattt attcactgta agtttatttt ttctacacag     1260
   taaaaacttg tactatgtta ataacttgtc ctatgtcaat ttgtatatca tgaaacactt     1320
   ctcatcatat tgtatgtaag taattgcatt tctgctcttc caaagctcct gcgtctgttt     1380
   ttaaagagca tggaaaaata ctgcctagaa aatgcaaaat gaaataagag agagtagttt     1440
   ttcagctagt ttgaaggagg acggttaact tgtatattcc accattcaca tttgatgtac     1500
   atgtgtaggg aaagttaaaa gtgttgatta cataatcaaa gctacctgtg gtgatgttgc     1560
   cacctgttaa aatgtacact ggatatgttg ttaaacacgt gtctataatg gaaacattta     1620
   caataaatat tctgcatgga aatactgtta aaaaaaaaaa                            1660
<210> 25
   <211> 1464
   <212> DNA
   <213> Homo sapiens
<400> 25
   agccccaagc ttaccacctg cacccggaga gctgtgtcac catgtgggtc ccggttgtct      60
   tcctcaccct gtccgtgacg tggattggtg ctgcacccct catcctgtct cggattgtgg     120
   gaggctggga gtgcgagaag cattcccaac cctggcaggt gcttgtggcc tctcgtggca     180
   gggcagtctg cggcggtgtt ctggtgcacc cccagtgggt cctcacagct gcccactgca     240
   tcaggaacaa aagcgtgatc ttgctgggtc ggcacagcct gtttcatcct gaagacacag     300
   gccaggtatt tcaggtcagc cacagcttcc cacacccgct ctacgatatg agcctcctga     360
   agaatcgatt cctcaggcca ggtgatgact ccagccacga cctcatgctg ctccgcctgt     420
   cagagcctgc cgagctcacg gatgctgtga aggtcatgga cctgcccacc caggagccag     480
   cactggggac cacctgctac gcctcaggct ggggcagcat tgaaccagag gagttcttga     540
   ccccaaagaa acttcagtgt gtggacctcc atgttatttc caatgacgtg tgtgcgcaag     600
   ttcaccctca gaaggtgacc aagttcatgc tgtgtgctgg acgctggaca gggggcaaaa     660
   gcacctgctc gggtgattct gggggcccac ttgtctgtaa tggtgtgctt caaggtatca     720
   cgtcatgggg cagtgaacca tgtgccctgc ccgaaaggcc ttccctgtac accaaggtgg     780
   tgcattaccg gaagtggatc aaggacacca tcgtggccaa cccctgagca cccctatcaa     840
   ccccctattg tagtaaactt ggaaccttgg aaatgaccag gccaagactc aagcctcccc     900
   agttctactg acctttgtcc ttaggtgtga ggtccagggt tgctaggaaa agaaatcagc     960
   agacacaggt gtagaccaga gtgtttctta aatggtgtaa ttttgtcctc tctgtgtcct    1020
   ggggaatact ggccatgcct ggagacatat cactcaattt ctctgaggac acagatagga    1080
   tggggtgtct gtgttatttg tggggtacag agatgaaaga ggggtgggat ccacactgag    1140
   agagtggaga gtgacatgtg ctggacactg tccatgaagc actgagcaga agctggaggc    1200
   acaacgcacc agacactcac agcaaggatg gagctgaaaa cataacccac tctgtcctgg    1260
   aggcactggg aagcctagag aaggctgtga gccaaggagg gagggtcttc ctttggcatg    1320
   ggatggggat gaagtaagga gagggactgg accccctgga agctgattca ctatgggggg    1380
   aggtgtattg aagtcctcca gacaaccctc agatttgatg atttcctagt agaactcaca    1440
   gaaataaaga gctgttatac tgtg                                           1464
<210> 26
   <211> 2855
   <212> DNA
   <213> Homo sapiens
<400> 26
   agccccaaac tcaccacctg gccgtggaca cctgtgtcag catgtgggac ctggttctct      60
   ccatcgcctt gtctgtgggg tgcactggtg ccgtgcccct catccagtct cggattgtgg     120
   gaggctggga gtgtgagaag cattcccaac cctggcaggt ggctgtgtac agtcatggat     180
   gggcacactg tgggggtgtc ctggtgcacc cccagtgggt gctcacagct gcccattgcc     240
   taaagaagaa tagccaggtc tggctgggtc ggcacaacct gtttgagcct gaagacacag     300
   gccagagggt ccctgtcagc cacagcttcc cacacccgct ctacaatatg agccttctga     360
   agcatcaaag ccttagacca gatgaagact ccagccatga cctcatgctg ctccgcctgt     420
   cagagcctgc caagatcaca gatgttgtga aggtcctggg cctgcccacc caggagccag     480
   cactggggac cacctgctac gcctcaggct ggggcagcat cgaaccagag gagttcttgc     540
   gccccaggag tcttcagtgt gtgagcctcc atctcctgtc caatgacatg tgtgctagag     600
   cttactctga gaaggtgaca gagttcatgt tgtgtgctgg gctctggaca ggtggtaaag     660
   acacttgtgg gggtgattct gggggtccac ttgtctgtaa tggtgtgctt caaggtatca     720
   catcatgggg ccctgagcca tgtgccctgc ctgaaaagcc tgctgtgtac accaaggtgg     780
   tgcattaccg gaagtggatc aaggacacca tcgcagccaa cccctgagtg cccctgtccc     840
   acccctacct ctagtaaatt taagtccacc tcacgttctg gcatcacttg gcctttctgg     900
   atgctggaca cctgaagctt ggaactcacc tggccgaagc tcgagcctcc tgagtcctac     960
   tgacctgtgc tttctggtgt ggagtccagg gctgctagga aaaggaatgg gcagacacag    1020
   gtgtatgcca atgtttctga aatgggtata atttcgtcct ctccttcgga acactggctg    1080
   tctctgaaga cttctcgctc agtttcagtg aggacacaca caaagacgtg ggtgaccatg     1140
   ttgtttgtgg ggtgcagaga tgggaggggt ggggcccacc ctggaagagt ggacagtgac     1200
   acaaggtgga cactctctac agatcactga ggataagctg gagccacaat gcatgaggca     1260
   cacacacagc aaggatgacg ctgtaaacat agcccacgct gtcctggggg cactgggaag     1320
   cctagataag gccgtgagca gaaagaaggg gaggatcctc ctatgttgtt gaaggaggga     1380
   ctagggggag aaactgaaag ctgattaatt acaggaggtt tgttcaggtc ccccaaacca     1440
   ccgtcagatt tgatgatttc ctagcaggac ttacagaaat aaagagctat catgctgtgg     1500
   tttattatgg tttgttacat tgataggata catactgaaa tcagcaaaca aaacagatgt     1560
   atagattaga gtgtggagaa aacagaggaa aacttgcagt tacgaagact ggcaacttgg     1620
   ctttactaag ttttcagact ggcaggaagt caaacctatt aggctgagga ccttgtggag     1680
   tgtagctgat ccagctgata gaggaactag ccaggtgggg gcctttccct ttggatgggg     1740
   ggcatatctg acagttattc tctccaagtg gagacttacg gacagcatat aattctccct     1800
   gcaaggatgt atgataatat gtacaaagta attccaactg aggaagctca cctgatcctt     1860
   agtgtccagg gtttttactg ggggtctgta ggacgagtat ggagtacttg aataattgac     1920
   ctgaagtcct cagacctgag gttccctaga gttcaaacag atacagcatg gtccagagtc     1980
   ccagatgtac aaaaacaggg attcatcaca aatcccatct ttagcatgaa gggtctggca     2040
   tggcccaagg ccccaagtat atcaaggcac ttgggcagaa catgccaagg aatcaaatgt     2100
   catctcccag gagttattca agggtgagcc ctttacttgg gatgtacagg ctttgagcag     2160
   tgcagggctg ctgagtcaac cttttattgt acaggggatg agggaaaggg agaggatgag     2220
   gaagcccccc tggggatttg gtttggtctt gtgatcaggt ggtctatggg gctatcccta     2280
   caaagaagaa tccagaaata ggggcacatt gaggaatgat actgagccca aagagcattc     2340
   aatcattgtt ttatttgcct tcttttcaca ccattggtga gggagggatt accaccctgg     2400
   ggttatgaag atggttgaac accccacaca tagcaccgga gatatgagat caacagtttc     2460
   ttagccatag agattcacag cccagagcag gaggacgctg cacaccatgc aggatgacat     2520
   gggggatgcg ctcgggattg gtgtgaagaa gcaaggactg ttagaggcag gctttatagt     2580
   aacaagacgg tggggcaaac tctgatttcc gtgggggaat gtcatggtct tgctttacta     2640
   agttttgaga ctggcaggta gtgaaactca ttaggctgag aaccttgtgg aatgcagctg     2700
   acccagctga tagaggaagt agccaggtgg gagcctttcc cagtgggtgt gggacatatc     2760
   tggcaagatt ttgtggcact cctggttaca gatactgggg cagcaaataa aactgaatct     2820
   tgttttcaga ccttaaaaaa aaaaaaaaaa aaaaa                                 2855
<210> 27
   <211> 9657
   <212> DNA
   <213> Homo sapiens
<400> 27
<210> 28
   <211> 3201
   <212> DNA
   <213> Homo sapiens
<400> 28
   gaaggaggga cggccggtcc cgtcagtcag gcagcgggag ccgccgggag cggatggcgg      60
   cggccgtagc ggctccactc gccgccgggg gtgaggaggc ggcagccacg acctccgtgc     120
   ccgggtctcc aggtctgccg gggagacgca gtgcagagcg ggccctagag gaggccgtgg     180
   ccaccgggac cctgaacctg tctaaccggc gcttgaagca cttcccccgg ggcgcggccc     240
   gtagctacga cctgtcagac atcacccagg ctgacctgtc ccggaaccgg tttcccgagg     300
   tgcccgaggc ggcgtgccag ctggtgtccc tggagggcct gagcctctac cacaattgcc     360
   tgagatgcct gaacccagcc ttggggaatc tcacagccct cacctacctc aacctcagcc     420
   gaaaccagct gtcgctgctg ccaccctaca tctgccagct gcccctgagg gtcctcatcg     480
   tcagcaacaa caagctggga gccctgcccc ctgacatcgg caccctggga agcctgcgac     540
   agcttgacgt gagcagcaac gagctccaat ccctgccctc ggaactgtgt ggcctctctt     600
   ccctgcggga cctcaatgtc cggaggaacc agctcagtac gctgcccgaa gagctggggg     660
   acctccctct ggtccgcctg gatttctcct gtaaccgcgt ctcccgaatc ccagtctcct     720
   tctgccgcct gaggcacctg caggtcattc tgctggacag caaccctctg cagagtccac     780
   ctgcccaggt ctgcctgaag gggaaacttc acatcttcaa gtatttgtcc acagaggccg     840
   ggcagcgtgg gtcggccctg ggggacctgg ccccttctcg gcccccgagt ttcagtccct     900
   gccctgcaga ggatctattt ccgggacatc ggtacgatgg tgggctggac tcaggcttcc     960
   acagcgttga tagtggcagc aagaggtggt ctggaaatga gtcaacagat gaattttcag    1020
   agctgtcatt ccggatctca gagctggccc gggagccccg gggacccaga gaacgcaagg    1080
   aggatggctc agcggacgga gaccctgtgc agattgactt catcgacagc catgtccccg    1140
   gggaggatga agagcgaggc actgtggagg agcagcgacc acccgaatta agccctgggg    1200
   caggggacag ggagagggca ccaagcagca ggcgggagga gccggcaggg gaggagcggc    1260
   ggcgcccgga caccttgcag ctgtggcagg agcgggaacg gcggcagcag cagcagagcg    1320
   gggcgtgggg ggccccgagg aaggatagcc tcttgaagcc agggctcagg gctgttgtgg    1380
   gaggggccgc cgccgtgtcc actcaagcca tgcacaacgg ctcgcctaag tccagtgcct    1440
   cccaagcagg ggctgcagcg gggcagggag cccccgcccc tgcccctgcc tcccaagagc    1500
   cccttcccat agctggacca gcgacagcac ctgctccacg gccacttggc tccattcaga    1560
   gaccaaacag cttcctcttc cgttcctcct ctcagagtgg ctcaggccct tcctcaccag    1620
   actctgtcct gagacctcgg cggtaccccc aggttccaga tgagaaggac ttaatgactc    1680
   agctgcgcca ggtccttgag tcccggctgc agcggcccct gcctgaggac ctggccgagg    1740
   ctctggccag tggggtcatc ctgtgccagc tggccaacca gctacggccg cgctccgtgc    1800
   ccttcatcca tgtgccctcc cctgctgtgc caaaactcag tgccctcaag gctcggaaga    1860
   atgtggagag ttttctagaa gcctgtcgaa aaatgggggt gcctgaggct gacctgtgct    1920
   cgccctcgga tctcctccag ggcactgccc gggggctgcg gaccgcgctg gaggccgtga    1980
   agcgggtggg gggcaaggcc ctaccgcccc tctggccccc ctctggtctg ggcggcttcg    2040
   tcgtcttcta cgtggtcctc atgctgctgc tctatgtcac ctacactcgg ctcctgggtt    2100
   cctaggcccc aaaatcggcc ctccctcacc cctttccctt cctctctatt tataaggtcc    2160
   ctgctccacc cgaccccacc tgcggtgcct tcagccccaa ccaaagacac tagtgcaccc    2220
   ccttcacaga cactgacctc agaggcccca ctctggtgcc cccagaccct gggcccccag    2280
   cctctggcct ccctccagta gccccacgag tccccacctc tcagtgctga cggtgccttc    2340
   atgtccccgc cggccctgcc cctgccctct gtaccccgtg aggggtggca ggagctggag    2400
   tctccccctt cctcctgtgc cctccccttc cccccccaac agctgctatg ggggggctaa    2460
   attatctcta ttttgtagag aggatctata tttgtagggg ttcggggccc aggccgggtc    2520
   cctatctctg tgtataaact gtacagaccg tggccgccct gcctgtgtgt gtgtgtgtgc    2580
   gcgcgcgcgc gcgtctgctc cgcgtgttgg tggctgtggc catggctctg tgcccaccag    2640
   catctccctc ctgagatgcc ggcctctcat gctcccggag cgtccgccaa ccccccgtgt     2700
   cacctccctt ctgttatcgc tgacagcttt cttgcgtctc atttgtcgcc gagccccgag    2760
   cgcacggtga tgctcgggtc tgcccccgac cccctgccac aggccggaag ccgcaggggg    2820
   caccgtgggg aagctaaccc ggccccttcc cccaggagtc actgtgccag ccccaccaca    2880
   tcctggaaga ggagggggcc ccgggaaggg gcctccccta catcgctgct gtcgtccacg    2940
   ccctgctgga ccggccttag gggtgaaggt gggggcacag ggcccacccc gcccgggacc    3000
   ccgggagcag aagacgcgcc tgggctccgc gctctcagag aagcacgtgg tgagggtggc    3060
   ctgggcctgg gcacccttgg cctgccggcc tggctgcctc tggggtcgag ggtctgggtg    3120
   gaaggaccgc gggtgtcgca ggtgtcgtgt cagccgcaat aaacagaagc cagaagccct    3180
   ctgggtggcc ctcaggtgta a                                              3201
<210> 29
   <211> 1853
   <212> DNA
   <213> Homo sapiens
<400> 29
   cgctccacct ctcaagcagc cagcgcctgc ctgaatctgt tctgccccct ccccacccat      60
   ttcaccacca ccatgacacc gggcacccag tctcctttct tcctgctgct gctcctcaca     120
   gtgcttacag ctaccacagc ccctaaaccc gcaacagttg ttacgggttc tggtcatgca     180
   agctctaccc caggtggaga aaaggagact tcggctaccc agagaagttc agtgcccagc     240
   tctactgaga agaatgctgt gagtatgacc agcagcgtac tctccagcca cagccccggt     300
   tcaggctcct ccaccactca gggacaggat gtcactctgg ccccggccac ggaaccagct     360
   tcaggttcag ctgccacctg gggacaggat gtcacctcgg tcccagtcac caggccagcc     420
   ctgggctcca ccaccccgcc agcccacgat gtcacctcag ccccggacaa caagccagcc     480
   ccgggctcca ccgccccccc agcccacggt gtcacctcgg ccccggacac caggccggcc     540
   ccgggctcca ccgccccccc agcccatggt gtcacctcgg ccccggacaa caggcccgcc     600
   ttgggctcca ccgcccctcc agtccacaat gtcacctcgg cctcaggctc tgcatcaggc     660
   tcagcttcta ctctggtgca caacggcacc tctgccaggg ctaccacaac cccagccagc     720
   aagagcactc cattctcaat tcccagccac cactctgata ctcctaccac ccttgccagc     780
   catagcacca agactgatgc cagtagcact caccatagca cggtacctcc tctcacctcc     840
   tccaatcaca gcacttctcc ccagttgtct actggggtct ctttcttttt cctgtctttt     900
   cacatttcaa acctccagtt taattcctct ctggaagatc ccagcaccga ctactaccaa     960
   gagctgcaga gagacatttc tgaaatgttt ttgcagattt ataaacaagg gggttttctg    1020
   ggcctctcca atattaagtt caggccagga tctgtggtgg tacaattgac tctggccttc    1080
   cgagaaggta ccatcaatgt ccacgacgtg gagacacagt tcaatcagta taaaacggaa    1140
   gcagcctctc gatataacct gacgatctca gacgtcagcg tgagtgatgt gccatttcct    1200
   ttctctgccc agtctggggc tggggtgcca ggctggggca tcgcgctgct ggtgctggtc    1260
   tgtgttctgg ttgcgctggc cattgtctat ctcattgcct tggctgtctg tcagtgccgc    1320
   cgaaagaact acgggcagct ggacatcttt ccagcccggg atacctacca tcctatgagc    1380
   gagtacccca cctaccacac ccatgggcgc tatgtgcccc ctagcagtac cgatcgtagc    1440
   ccctatgaga aggtttctgc aggtaatggt ggcagcagcc tctcttacac aaacccagca    1500
   gtggcagcca cttctgccaa cttgtagggg cacgtcgccc gctgagctga gtggccagcc    1560
   agtgccattc cactccactc aggttcttca gggccagagc ccctgcaccc tgtttgggct    1620
   ggtgagctgg gagttcaggt gggctgctca cagcctcctt cagaggcccc accaatttct    1680
   cggacacttc tcagtgtgtg gaagctcatg tgggcccctg agggctcatg cctgggaagt    1740
   gttgtggtgg gggctcccag gaggactggc ccagagagcc ctgagatagc ggggatcctg    1800
   aactggactg aataaaacgt ggtctcccac tgcgccaaaa aaaaaaaaaa aaa           1853
<210> 30
   <211> 5403
   <212> DNA
   <213> Homo sapiens
<400> 30
   tccgggttac tgagcgctcg gggccttttc aaatcgggat ccgttaccgc ttccccggca     60
   gccgccattg tcgcgctcgg agcccctcag ctcaggcggc cgaggcggag gcagcggcgg    120
   cgggatggcg gacgccaaca aggccgaggt gcccggggcc actggtggcg acagcccgca    180
   cctgcagccc gcagagccgc cgggcgagcc gcggcgagag ccgcaccccg cggaggcgga    240
   gaagcagcag ccgcagcaca gcagcagctc caatggcgtt aaaatggaga atgatgaatc    300
   agcaaaagaa gagaaatctg acttaaagga aaaatctaca ggaagtaaga aggccaatag    360
   atttcatcct tattcaaaag acaagaattc gggcactgga gaaaagaagg gtccaaatcg    420
   taacagagtt ttcattagca acatcccata tgacatgaaa tggcaagcta ttaaagatct    480
   aatgagagag aaagttggtg aggttacata cgtggagctc tttaaggatg cggaaggaaa    540
   atcaaggggt tgtggtgtgg ttgaattcaa agatgaagaa tttgtaaaga aagccctaga    600
   aactatgaac aaatatgatc ttagtggaag accccttaat attaaagagg atcctgatgg    660
   agaaaatgct cgtagggcat tgcagcgaac aggaggatca tttccaggag gacacgtccc    720
   tgatatggga tcagggttga tgaatttacc accttccata ctcaataatc caaacattcc    780
   tcctgaagtc atcagtaatt tgcaggccgg tagacttggt tccacaattt ttgttgccaa    840
   tcttgacttc aaagttggtt ggaagaagct aaaggaagtg ttcagcatag ctggaactgt    900
   gaagcgggca gatattaaag aagacaaaga tggcaagagc agaggaatgg gcactgtcac    960
   ttttgagcaa gcaattgaag cagttcaagc aatttctatg ttcaatgggc agtttttatt   1020
   tgatagacct atgcatgtga aaatggatga caagtctgtt cctcatgaag agtaccgttc   1080
   acatgatggt aaaacaccac aattaccacg tggtcttgga ggcattggga tgggacttgg   1140
   tccgggtgga cagcctatta gtgccagcca gttgaacata ggtggagtaa tgggaaattt   1200
   aggtccaggt ggtatgggaa tggatggtcc aggttttgga ggaatgaata gaattggagg   1260
   aggaataggg tttggtggtc tggaagcaat gaatagcatg ggaggatttg gaggagttgg   1320
   ccgaatggga gagctgtacc gtggtgcgat gactagtagc atggagcgag attttggacg   1380
   tggtgatatt ggaataaatc gaggctttgg agattccttt ggtagacttg gcagtgcaat   1440
   gattggaggg tttgcaggaa gaataggatc ttctaacatg ggtccagtag gatctggaat   1500
   aagtggtgga atgggtagca tgaacagtgt gactggagga atggggatgg gactggaccg   1560
   gatgagttcc agctttgata gaatgggacc aggtatagga gctatactgg aaaggagcat   1620
   cgatatggat cgaggatttt tatcgggtcc aatgggaagc ggaatgagag agagaatagg   1680
   ctccaaaggc aaccagatat ttgtcagaaa tctacctttt gacttgactt ggcagaaact   1740
   aaaagagaaa ttcagtcagt gtggtcatgt aatgtttgca gaaataaaaa tggagaatgg   1800
   aaagtcaaaa ggctgtggaa cagtcagatt tgactcccca gaatcagctg aaaaagcctg   1860
   cagaataatg aatggcataa aaatcagtgg cagagaaatt gatgttcgct tggatcgtaa   1920
   tgcataattt caagccatgg ttggaacatt cctacatctg ttttgctgaa tctcctagta   1980
   aaagtcattt ttttaaagta atattgtatg cttacaaaag ctgtaaaaat gaacttttaa   2040
   aactcccacc agcttttaac agtataatgt taaaaatata ctgtaatttt tgttaatctc 2100
   aagtttgggt ttttaaagac agcaagtctg gtcattcagt ttaaatgaat ggttatactg 2160
   tttttaatga aataagccat tttcttgttg ttttcagtac tacatagttg gatttgtttg 2220
   ttctagtttc tccagttttt gtcaacttat tgtatggtaa aacatagatt ttttcccccc 2280
   aaacttctgt tttataatat gtaatttttc atgaaaagaa agggctcaga aaaattagga 2340
   tgtgattttg gttggtttta aattactgta agttttagat tctaaggttc aagattttta 2400
   aaatttgatt taaatgaaga aatggatttt tctctctgcc cctccctgcc attcatattt 2460
   tctgcataac actattaata atatcaacct ccacagcccc ttattttatt atttccaata 2520
   attccaagtt catatagaac tgataatgta gcaagcccca agtatgataa taggcagact 2580
   attcccaact ttctgtctag tttccagcca ttgaagtgaa ctgctaaaaa aagaaaaata 2640
   attgaaatgt tgagagagat ggttatgtaa gttagtcctc tgctgtttac ttctgcagga 2700
   gctgatccat ttataaatgc agttttaata aaccatggaa taccaaggca caacatatca 2760
   aacacattgg atcccacgat gttagacata gccatatctc ctttccctac aaaagaaaag 2820
   catactgtta aaatgtgctt accaatactg tgttttatta ataatcttca taagaaaaga 2880
   aacactggat acttttttgt tgttgattcg ttttgaaaaa ttgttgttag agcaaaagtc 2940
   ttactgaatt tgtattttta aatttttctt gggttagtat ttaaagtctt aacatttatt 3000
   taataattat atttattaaa ccatttttca ataaggtata tagcttattt gttgcttttc 3060
   attgtaattt aacatggtta atggttaatt actatttaac acacatttca aatgaatatt 3120
   atttggggga ttagattgag tgaaattaac ctgctattaa atagtaaact tttcctctgg 3180
   agtcactttt ttcccccttc aaagtatgtt actgaggaag taaacttttt tttttttttt      3240
   tggtttttgt tttttgagac acagtctcgc tctgttgccc aggctgctgg agtgccgtgg 3300
   cgcaatctcg gctcactgca acctccgcct cctggattca aacaattctc ctgcctcagc 3360
   ctcctgagta gctgggatta caggcacatg ccaccacgcc cggctaattt ttgtattttt 3420
   agtagagact gggtttcacc atgttggtca ggctggtctc aaactcctga cctcgtgatc 3480
   cacccgcctc ggcctcccaa aatcctggga ttacaggcgc gagccaccac acccggctgg 3540
   aagtaaacat ttttaaagct acttttactc attctagcct tgtagaatga ccattgcagc 3600
   ttgagggacc tagttcttac cttttcttgc aaccaacaca cttgcaattg tgtctggtat 3660
   gcttgttcct gctgctaata aagtaaggcc cattactgta tcgggaattt ctagtgtttc 3720
   ccctgtaata aacagatatt tcaagttaca aatcttaaag attcactaac catcctttgc 3780
   agttattttg gatatttcct tcgtgaacaa aaataaaata ggcacattta gaattcagag 3840
   ccaatatgtg cttgcttatt agttttttag ctagcaacat atttgaatca ggctggtaat 3900
   tcgggtaacc caggtagcac agatttttaa tgacatatct aaagatacgt aacagctaaa 3960
   attctgccag tgagaaattt tcctgtttga tattcttaca aaagatgttt atgtccacca 4020
   ttatctcatc agggctgtgc tgaatatttg ataatgagac tgatcattcc gctttttctt 4080
   tcttaaaaat attagtcaga gttaagcaaa ttaattatag ctatctttaa gctataaatg 4140
   tgttaacatg tatatatacc atttattatg ttctacttta gtgatatacc ttaatttagt 4200
   gggctttggc agggcggggg agggggaacg ttcattaatc tctgaggaaa acaaaacctg 4260
   ttttctactt gagtctaaca tatggtccca atttattaat acttctgtta aatttgatgt 4320
   caggtcaaca tttttcagaa atgtatttat tctcagaaac agaaccagag agaagttaaa 4380
   caaaaggtta tgtaactgtt cctttaatgt tgtaattgaa aacttggttt agcgtctttt 4440
   ttttctttct cttttttttt cttaaaatgc caactaaaat aattagaaag tagcttattt 4500
   attgcatgct tatacattga tattggaatt ggaattggtt gttaatttct gttactggct 4560
   ttgctagaat tcatatgtgc ataaataaca ctaatattta tcatcttggg ggctgttctg 4620
   tgatctatta catgattttt gctctctttt tatttttcta gtatcaaatg tgttctggct 4680
   gagtgaattt ggatagatat aatatgcaag ctaattggac tttttcattt ttctcattaa 4740
   aaaaaggaag aagtagtcca ctttttgcta gcatactatt ctgagactta cattaatttg 4800
   ggaaaagtca agggtcaaca aatttttgtg tctgtgaggg gccagatagt aattatttag 4860
   gctttgcagg ctgtgtgatt tctgttgcag ttattcagct ttgccaatgt agtgtgaaaa 4920
   caatcataga taatatgtaa aaccttacta ataaaaacaa gctgggttat gttggcacac 4980
   aggcagtagt ttgttcacct ctgctgtaag ttaatttggg ttaacctttg taggagaaat 5040
   tacagattat ttttgtcttt gttcatagtt tttttgttta gggactgtag tggattatgg 5100
   ccaagcagca gtagctagtt aagtcttaat catgaaggta gtagaaacct agaataaaat 5160
   tattatgccc ttttcactca tttcataata tgtttgtatt gccgtgttga aaagccaaat 5220
   aaaatacaat ccagttaagc agtgagcacc cattgttttc ccaaaaatag agctccttgc 5280
   tttgtttttg tttttttgtt cttttttttt tttattatgg aatggaattc aatataaaga 5340
   gtttattgtg gaccccatgg aatgtgatta aattattagc ataataaaaa gacatcctat 5400
   gaa                                                                  5403
<210> 31
   <211> 4959
   <212> DNA
   <213> Homo sapiens
<400> 31
   agtgggagac gtgcgctgag aggcgggggc tgcgctcggc ggaacagcag ccctcgggcg      60
   gagagcgggg ccggggtccg agagcaggtg atgccaagag ctgagcggga ctcgtgagcg     120
   cgcggttcag cacctaccag ggcgtcccgt aaaaaacctc gccttcgcct gtctctggga     180
   accatagcgc cgcaggtgcc gcctgtcctc gccttcctgc tgcaatcgcc ccaccatgga     240
   ctccccgatc cagatcttcc gcggggagcc gggccctacc tgcgccccga gcgcctgcct     300
   gccccccaac agcagcgcct ggtttcccgg ctgggccgag cccgacagca acggcagcgc     360
   cggctcggag gacgcgcagc tggagcccgc gcacatctcc ccggccatcc cggtcatcat     420
   cacggcggtc tactccgtag tgttcgtcgt gggcttggtg ggcaactcgc tggtcatgtt     480
   cgtgatcatc cgatacacaa agatgaagac agcaaccaac atttacatat ttaacctggc     540
   tttggcagat gctttagtta ctacaaccat gccctttcag agtacggtct acttgatgaa     600
   ttcctggcct tttggggatg tgctgtgcaa gatagtaatt tccattgatt actacaacat     660
   gttcaccagc atcttcacct tgaccatgat gagcgtggac cgctacattg ccgtgtgcca     720
   ccccgtgaag gctttggact tccgcacacc cttgaaggca aagatcatca atatctgcat     780
   ctggctgctg tcgtcatctg ttggcatctc tgcaatagtc cttggaggca ccaaagtcag     840
   ggaagacgtc gatgtcattg agtgctcctt gcagttccca gatgatgact actcctggtg     900
   ggacctcttc atgaagatct gcgtcttcat ctttgccttc gtgatccctg tcctcatcat     960
   catcgtctgc tacaccctga tgatcctgcg tctcaagagc gtccggctcc tttctggctc    1020
   ccgagagaaa gatcgcaacc tgcgtaggat caccagactg gtcctggtgg tggtggcagt    1080
   cttcgtcgtc tgctggactc ccattcacat attcatcctg gtggaggctc tggggagcac    1140
   ctcccacagc acagctgctc tctccagcta ttacttctgc atcgccttag gctataccaa    1200
   cagtagcctg aatcccattc tctacgcctt tcttgatgaa aacttcaagc ggtgtttccg    1260
   ggacttctgc tttccactga agatgaggat ggagcggcag agcactagca gagtccgaaa    1320
   tacagttcag gatcctgctt acctgaggga catcgatggg atgaataaac cagtatgact    1380
   agtcgtggag atgtcttcgt acagttcttc gggaagagag gagttcaatg atctaggttt    1440
   aactcagatc actactgcag tctgacatga aaagatagaa tttaaaataa acttttagag    1500
   atctcatggt ctgaagtcat cagatgcaga ccacgttcgt gatcagtgga aacatcaagg    1560
   acaaaaggtc cacgcaacac atgcacctct tctctagggc acagaagcga ggcagagcct    1620
   cagttcctct gatgaacaag ggaacaggtc ttttccttct ggtttgctag gtaaggttca    1680
   gcacccatct gctgtggcct tcctatgaaa cgtagtttca aaagctcggt ttaagaaaaa    1740
   aggaaaagaa aaaacattgc tttcagagct caatgggcat ccaatccaga tgtttcacaa    1800
   tgacagcaac aacctccaac ggaatgggaa ctatttccag ggttcatgcc cagctcactg    1860
   tatgctgtgt ggacatgcat ttcatgtggc tgtgtggtaa gaattacagc ttacatatgg    1920
   cttggaccca catctgagga atctgatgtt cacttatacc agaacattat cttgctattt    1980
   atgaaattat ttaaacgttc aaaagattgt ttttaacatg gtttaatttc ccaaaaacta    2040
   cagttttttt tcttagcatg ctattcaggt aaacagtctt ataataaagc atgtcccatt    2100
   gtcaagaaac ataaagtggt gtgaatacca ctgaaaatat atatatagta tcttctgtaa    2160
   ataatagtac ctgtgtgaat aaggaatagg cttgcctccc agccaggcaa tttcctgagg    2220
   gcacactaat gatatcccct gagttgctaa gttgatgctg agacattttg ctgggaatta    2280
   gtcatggcat gatctctttc agactccctg aataccattt agtcccgtaa cagtgctcac    2340
   aactcatgtg ctaatgaatc acaaaggctt taactagctc ccaggttgta gccttcgcag    2400
   gatctagttt atttgccaca tctctttatg aacatatagc gattcgcaga tctctctatt    2460
   cacggagagg aaggtgtttt gcttctgtag atctcaaggt actattttgt ggctctcagc    2520
   aggaagtaga attgttccta aatgtgtgct gaatgaggac atgatgtccc tcctggtgcc    2580
   aggacacatc ctgcatggca ttctgtgaag ggcatacctg ctgaggataa tgccaggagc    2640
   agcacattta gggtaatttt gctaaaactt ccagatgaca tataaattcc ctctttttcc    2700
   ccctgaaact taccatttca gaggtaatgg ctttttctgt aatttgcctg agaagaaaaa    2760
   agggataatt tagaaataac actccaagcc tatatattac tagggctgca tcctctggaa    2820
   tttaatagaa aaattgaata tatagcccta tgtaactaca gacatcatta caactgttaa    2880
   gttctgttca ccatgcaagt tctcggtggt gtatgtactc tcaccttttg gaaacaaaac    2940
   cttgcattga aggttttaca catggcttca gaatgttact ctgaccatgt ttgcctctac    3000
   tccagattat catggcacct aacacagatg acatatggca gaatggaagt gatttctcct    3060
   aatgaaaatg aagtgctctt ttgagtattt cttgttttat tgagattcct atctgccctt    3120
   gtatttttat aactgaatgt aaataggaat tgcttaaata ctggtttaca ctaatcccat    3180
   agttaaaaat cttggctatg gtacaggaag gatatctata tagaggatag ttccagaaac    3240
   aagccctggt gaaataggaa atataaaaac ttctttatgg aacaggaaaa cccaagagac    3300
   tttctcgtca gattcctttc cagtgcaaag tataacagag ataggattat gagatgaaat    3360
   cacagtagtg tgtttccttt aattttttct gtcttctttt tttttttttt tttttttgag    3420
   acggagtctc gctctgttgc ccaggctgga gtgcggtagc acagtcttgg ctcactgcaa    3480
   cctccacctc ctgggttcaa gcgattctcc tgcctctgcc tcctaagtag ctgggattac    3540
   aggcgtgcac cgccaagccc ggctaatttt tgtattttta gtagagacgg ggtttcacca    3600
   tgttatgcag gctggtctca aactcctgac ctcagaatat tcacctgcct cggcctccca    3660
   aagtgctggg attacagatg tgaatggcca cgcctggcct gtttccttta agttagcact    3720
   tcacgtgctc ttacagcgtt aattgccatt attggtttgc ctgattttcc tgcatctcct    3780
   catatgtaag tctgcagccc aaggagagaa tagtagctgt atgtgcccac aaggggtgtc    3840
   caagcttctt gattcttgtt aatatcgact ccccaacatt gaaaaaggag tgaagaaggg    3900
   aatatcattt tagggtactg ctttaaagga atgataaagt aaacaacgta gcagggaaaa    3960
   taccacttgc acaaatgtat ttctttgtca aagtgtgtac acgctttctg tttgtcctga    4020
   tgtcctgctg tgaagcaggg accacattgc catttattca ttgttccagg caatgcattt    4080
   gtccaaaccc ctctggctct ctgatatctc tcatttcttg atgttcctat tctctgacct    4140
   caacaaggag gacactgtct gttgtgtctc tttgagacta gaactccatg tgtttaacct    4200
   tcagatccat tctctacatg aaccctattc tttccaccct ggtaagaagg tctggaagac    4260
   aggtcctaac gaggggtcag agaaggcaca gagaaatgaa ccagccatgc aggggcagca    4320
   ctgaggagga aaccccccac tctaactggg aggagagtgg aaacactctt agtatctcag    4380
   agtagttacc aatctaagag catccttcga gtttaagtca aaactctagg cttaatacca    4440
   aaaataatac atcatttgga aaaatagctt ggagaaaaaa aatcaataaa gagtcacata    4500
   aaatcttaag aaaatggttc tattctgtga tatattacta agaaatacag ttcttaaacg    4560
   tgtataacta ttgtcagaca atttataggt gtttcatcta gtcctgggat gaaatacgat    4620
   gactcatgca aattcaggga gcacactggc tgactttaaa ttggaacatt gtaaagtgaa    4680
   agctgtgaaa ggttgtgtgt cctatcaatg agatttattt tctaaccaag agctgttcgg    4740
   atgattcagt ataaatacga aaatcacatc caaaaacggt ataacccagc ttccttaagg    4800
   caattttctt ctctgaaaca agaatatact catatgttct ttactataat gtatatgatt    4860
   tttatcttgt actttaaaag atgataatta tgcattgtat atacgattgt gtgccttgca    4920
   ataaaatcaa aactgtacct gctgaaaatc acaacagtc                           4959
<210> 32
   <211> 2190
   <212> DNA
   <213> Homo sapiens
<400> 32
   agtcgcgacc ttttgtcttc tcttacagcg cacccgttgg tgcgcgggaa taggtgtgca      60
   tgccccggcc tgggcctttt tctgttgacc cacggcatca ccttagcaag ggtgttgtcc     120
   ttttcagtcc attccctgaa gcgcagaacc ggaggccttg tgagaacctg gctttttgtc     180
   cagtcctgtc ctcagaactc aaggaggcat cacgggggga gtcatttacc tccctggtct     240
   caggtgtctc tcacagtaca ctcctgatca tgtagttgga cctggagcag acattgatcc     300
   cactcaaata acctttcccg gatgcatttg tgtcaaaact ccctgcctcc ctggcacttg     360
   ctcctgtctc cgccatggag agaactatga tgataactca tgccttagag atataggatc     420
   tggaggaaag tatgcagagc ctgtttttga atgcaatgtc ctgtgccgat gcagtgacca     480
   ctgcagaaac agagtggtcc agaaaggtct acagttccac ttccaagtgt tcaagacgca     540
   taaaaaaggc tggggacttc gtaccttgga atttataccg aaaggaaggt ttgtctgtga     600
   atatgctggt gaggttttag gattctctga agttcagaga agaattcact tacaaacaaa     660
   atccgactcc aattacatta tagccatcag ggaacatgtt tataatgggc aggtaatgga     720
   aacatttgtt gaccctactt atataggaaa tattggaaga ttccttaatc attcttgtga     780
   gccaaacctt ttgatgattc ctgtccgaat tgactcaatg gtacctaagt tggcactttt     840
   tgcagccaaa gatattgtgc cagaagaaga actctcttat gattattcag gaagatatct     900
   taatctaaca gtcagtgaag acaaagaaag gctagatcat gggaaactaa ggaaaccttg     960
   ttactgtggt gccaaatcat gtactgcttt cctgcctttt gacagttctc tgtactgccc    1020
   cgtagaaaag tcgaacatca gttgtggaaa tgagaaggaa cccagcatgt gtggctcagc    1080
   cccttctgtg ttcccctcct gcaagcgatt gacccttgag actatgaaaa tgatgttaga    1140
   caaaaagcaa attcgagcaa ttttcttatt cgagttcaaa atgggtcgta aagcagcaga    1200
   aacaactcgc aacatcaaca atgcatttgg cccaggaact gctaacgaac gtacagtgca    1260
   gtggtggttc aagaagtttt gcaaaggaga tgagagcctt gaagatgagg agcgtagtgg    1320
   ccggccatca gaagttgaca acgaccagtt gagagcaatc atcgaagctg atccccttac    1380
   aactacacga gaagttgctg aagaactcaa tgtcaaccat tctacggtcg ttcgacattt    1440
   gaagcaaatt ggaaaggtga aaaagctcga taagtgggtg cctcatgagc tgactgaaaa    1500
   tcaaaaaaat cgtcgttttg aagtgtcatc ttctcttatt ctacgcaacc acaacgaacc    1560
   atttctcgat cggattgtga cgtgtgatga aaagtggatt ttatatgaca accggcgacg    1620
   atcagctcag tggttggatc aagaagaagc tccaaagcac ttcccaaagc caatcttgca    1680
   cccaaaaaag gtcatggtca ctatttggtg gtctgctgct ggtctgatcc actacagctt    1740
   tctgaatccc ggtgaaacca ttacatctga gaagtatgct caggaaatcg atgagatgaa    1800
   ccaaaaactg caacgcctgc agctggcatt ggtcaacaga aagggcccaa ttcttctcca    1860
   cgacaatgcc cgaccgcatg ttgcacaacc cacacttcaa aagttgaatg aattgggcta    1920
   tgaagttttg cctcatccac cgtattcacc tgacctcttg ccaaccaact accacgtctt    1980
   taagcatctc aacaactttt tgcagggaaa acgcttccac aaccagcagg atgcagaaaa    2040
   tgctttccaa gagttcgtcg aatcccaaag cacggatttt tacgctacag gaataaacca    2100
   acttatttct cgttggcaaa aatgtgttga ttgtaatggt tcctattttg attaataaaa    2160
   atgcgttgag cctaaaaaaa aaaaaaaaaa                                     2190
<210> 33
   <211> 2296
   <212> DNA
   <213> Homo sapiens
<400> 33
   gggacccagg gactgggagg ccggttgggg ctgggctcag gggccgagac ctagctgggc      60
   ttggggcggg gccgagacgg agcgaggggt ccagggtgtg ggaaacgggg aggggtttga     120
   ggaggggatc ggaatgtggc tcaagttcgg gaggcgttac ctgcggaggg tttgaggcag     180
   gcccaagagc gagcccatgg tcttccgacg cggggccagg ggcgggcccc aggatccgga     240
   gcttcgtgcg gggccgagtc caggtttggg gcccgggagg cggggccagt tagggcgact     300
   gtccctggga tcgtcgggtc aggccttggg ctaacgtagg cactctcgca gttcctccgc     360
   cttcaggaag gtcttttcag caggggcctt acgggtgcat gcttcggtcc tggaggcctt     420
   atcctagcct cctctccatc agcgccaccc gtctggggcc cgaaaggagg gagctttccc     480
   tctgtccccc agcctttgga ctgtcaccaa acaagccatt cgttcatcaa atacttttta     540
   agcgcctacc atgtgcctga caagggagat gtaacggtga gaaaaactag gtgtggtcca     600
   ggcccttcag gggctcaggt gctcgtggaa gaagtggaca ttgaagtact tatcacacaa     660
   atgaggataa aagtacgata gcgatatcta ccacgaagct gttgttcccc accagaacca     720
   aatgagcgca agatctgaca aagaaaaaaa aaggttcatc ttttattcct ccaaacactt     780
   tcatttaaat caagaggatg ggatgtggtt attgctgtgt ttttagacag aatcaacggt     840
   ttctgggtct gagatgttgc atacaccttc tcagtccctg tatcctgaga tggagtcacc     900
   tgagaatcca cagcaagtcc taaccaggga tgggtctggg tgattaagga aggttggctt     960
   cagaactggg ccaggggcac tgctttgctt ttgctgtttt gatcagctct ctgcctgaag    1020
   gagacaagaa aaaccaacgg gaacagctgg ggaaactgag ttacagagct tgtctgcact    1080
   gagtcatcag gagcaaatgc tagatcagat agggtctctc tctctgccac ccaggccaga    1140
   gtgcagttga cgcagggcag gggagccccg aagtggagca tagtgtgtcc ggaactggtg    1200
   ggttcttggt ctcactgact tcaagaaaga agccgcggac cctcgcgaaa ctcctggagg    1260
   gcagaggcat gcctgccatc ttcatcattg cattaccacc acccaacact atcgggggac    1320
   ctgccctgat aatcagtcta caggtgtatc cagcagctcc agagagacag cgaccagcga    1380
   gaaggggcca tgatgatgga ggtggttttg tcaaaacgaa aatggggata tgtagggaaa    1440
   agaaagagag atcagactgt tactgtgtct acatagaaag ggaagacata agagactcca    1500
   ttttgaaaaa gacctgtact ttaaacaatt gctttgctga gatgttgtta atctgtagct    1560
   ttgccccagc cactttgccc caaccacttt gacccaatct ggagctcata aaaacatgtg    1620
   ttgtatgaaa tcaaggttta aggcatgtag ggctgtgcag gacgtgcctt gttaaccaaa    1680
   tgtttgcaag cagtatactt ggtaaaagtc atcaccattc tctcatctca ataaaccagg    1740
   ggcacaatgc actgtggaaa gccgcaggga cctctgccct tgaaagctgg gtattgtcca    1800
   aagttcctcc ccatgtgata gtctgaaata tggcctcgtg ggatgagaaa gacctgacgg    1860
   tcccccagcg cgacacccat aaaagatctg tgctgaggtg gattagtcaa agaggaaaga    1920
   cttgcagttg agatagagga aggccactgt ctcctgactg cccctgggaa ctgaatgtct    1980
   cggtataaaa cacgattgta catttgttca gttctgagat gggagaaaaa ccgccctatg    2040
   gtgggaggcg agacatgttt acagcaatgc tgccttgtta tcctttactc cactgagatg    2100
   tctgggtgga gagaaacata aatctggctt acatgcacgt ccagtcatag taccttccct    2160
   tgaacttcat tatgacatgg attctattgc tcacgtttgt tgctgacctt ctccttatta    2220
   tcaccctgcc ctcctactac attccttttt gctgaaataa tgaagataat aatcaataaa    2280
   aactgaggga attcag                                                    2296
<210> 34
   <211> 894
   <212> DNA
   <213> Homo sapiens
<400> 34
   gcccgtcttc gtgtctcctc cctccctcgc cttcctcctt cctagctcct ctcctccagg      60
   gccagactga gcccaggttg atttcaggcg gacaccaata gactccacag cagctccagg     120
   agcccagaca ccggcggcca gaagcaaggc taggagctgc tgcagccatg tcggccctca     180
   gcctcctcat tctgggcctg ctcacggcag tgccacctgc cagctgtcag caaggcctgg     240
   ggaaccttca gccctggatg cagggcctta tcgcggtggc cgtgttcctg gtcctcgttg     300
   caatcgcctt tgcagtcaac cacttctggt gccaggagga gccggagcct gcacacatga     360
   tcctgaccgt cggaaacaag gcagatggag tcctggtggg aacagatgga aggtactctt     420
   cgatggcggc cagtttcagg tccagtgagc atgagaatgc ctatgagaat gtgcccgagg     480
   aggaaggcaa ggtccgcagc accccgatgt aaccttctct gtggctccaa ccccaagact     540
   cccaggcaca tgggatggat gtccagtgct accacccaag ccccctcctt ctttgtgtgg     600
   aatctgcaat agtgggctga ctccctccag ccccatgccg gccctacccg cccttgaagt     660
   atagccagcc aaggttggag ctcagaccgt gtctaggttg gggctcggct gtggccctgg     720
   ggtctcctgc tcagctcaga agagccttct ggagaggaca gtcagctgag cacctcccat     780
   cctgctcaca cgtccttccc cataactatg gaaatggccc taatttctgt gaaataaaga     840
   ctttttgtat ttctggggct gaggctcagc aacagcccct caggcttcca gtga           894
<210> 35
   <211> 2034
   <212> DNA
   <213> Homo sapiens
<400> 35
<210> 36
   <211> 3401
   <212> DNA
   <213> Homo sapiens
<400> 36
<210> 37
   <211> 2657
   <212> DNA
   <213> Homo sapiens
<400> 37
   gagtcaggcg cgcgcgggca gggtccccat tgcctgctgc gcacccggac gtgcggctcc      60
   cctcggcctc ctcgccatgg acgcggacga ctcccgggcc cccaagggct ccttgcggaa     120
   gttcctggag cacctctccg gggccggcaa ggccatcggc gtgctgacca gcggcgggga     180
   tgctcaaggt atgaacgctg ccgtccgtgc cgtggtgcgc atgggtatct acgtgggggc     240
   caaggtgtac ttcatctacg agggctacca gggcatggtg gacggaggct caaacatcgc     300
   agaggccgac tgggagagtg tctccagcat cctgcaagtg ggcgggacga tcattggcag     360
   tgcgcggtgc caggccttcc gcacgcggga aggccgcctg aaggctgctt gcaacctgct     420
   gcagcgcggc atcaccaacc tgtgtgtgat cggcggggac gggagcctca ccggggccaa     480
   cctcttccgg aaggagtgga gtgggctgct ggaggagctg gccaggaacg gccagatcga     540
   taaggaggcc gtgcagaagt acgcctacct caacgtggtg ggcatggtgg gctccatcga     600
   caatgatttc tgcggcaccg acatgaccat cggcacggac tccgccctgc acaggatcat     660
   cgaggtcgtc gacgccatca tgaccacggc ccagagccac cagaggacct tcgttctgga     720
   ggtgatggga cgacactgtg ggtacctggc cctggtgagt gccttggcct gcggtgcgga     780
   ctgggtgttc cttccagaat ctccaccaga ggaaggctgg gaggagcaga tgtgtgtcaa     840
   actctcggag aaccgtgccc ggaaaaaaag gctgaatatt attattgtgg ctgaaggagc     900
   aattgatacc caaaataaac ccatcacctc tgagaaaatc aaagagcttg tcgtcacgca     960
   gctgggctat gacacacgtg tgaccatcct cgggcacgtg cagagaggag ggaccccttc    1020
   ggcattcgac aggatcttgg ccagccgcat gggagtggag gcagtcatcg ccttgctaga    1080
   ggccaccccg gacaccccag cttgcgtcgt gtcactgaac gggaaccacg ccgtgcgcct    1140
   gccgctgatg gagtgcgtgc agatgactca ggatgtgcag aaggcgatgg acgagaggag    1200
   atttcaagat gcggttcgac tccgagggag gagctttgcg ggcaacctga acacctacaa    1260
   gcgacttgcc atcaagctgc cggatgatca gatcccaaag accaattgca acgtagctgt    1320
   catcaacgtg ggggcacccg cggctgggat gaacgcagcc gtacgctcag ctgtgcgcgt    1380
   gggcattgcc gacggccaca ggatgctcgc catctatgat ggctttgacg gcttcgccaa    1440
   gggccagatc aaagaaatcg gctggacaga tgtcgggggc tggaccggcc aaggaggctc    1500
   cattcttggg acaaaacgcg ttctcccggg gaagtacttg gaagagatcg ccacacagat    1560
   gcgcacgcac agcatcaacg cgctgctgat catcggtgga ttcgaggcct acctgggact    1620
   cctggagctg tcagccgccc gggagaagca cgaggagttc tgtgtcccca tggtcatggt    1680
   tcccgctact gtgtccaaca atgtgccggg ttccgatttc agcatcgggg cagacaccgc    1740
   cctgaacact atcaccgaca cctgcgaccg catcaagcag tccgccagcg gaaccaagcg    1800
   gcgcgtgttc atcatcgaga ccatgggcgg ctactgtggc tacctggcca acatgggggg    1860
   gctcgcggcc ggagctgatg ccgcatacat tttcgaagag cccttcgaca tcagggatct    1920
   gcagtccaac gtggagcacc tgacggagaa aatgaagacc accatccaga gaggccttgt    1980
   gctcagaaat gagagctgca gtgaaaacta caccaccgac ttcatttacc agctgtattc    2040
   agaagagggc aaaggcgtgt ttgactgcag gaagaacgtg ctgggtcaca tgcagcaggg    2100
   tggggcaccc tctccatttg atagaaactt tggaaccaaa atctctgcca gagctatgga    2160
   gtggatcact gcaaaactca aggaggcccg gggcagagga aaaaaattta ccaccgatga    2220
   ttccatttgt gtgctgggaa taagcaaaag aaacgttatt tttcaacctg tggcagagct    2280
   gaagaagcaa acggattttg agcacaggat tcccaaagaa cagtggtggc tcaagctacg    2340
   gcccctcatg aaaatcctgg ccaagtacaa ggccagctat gacgtgtcgg actcaggcca    2400
   gctggaacat gtgcagccct ggagtgtctg acccagtccc gcctgcatgt gcctgcagcc    2460
   accgtggact gtctgttttt gtaacactta agttatttta tcagcacttt atgcacgtat    2520
   tattgacatt aatacctaat cggcgagtgc ccatctgccc cacctgctcc agtgcgtgct    2580
   gtctgtggag tgtgtctcat gctttcagat gtgcatatga gcagaattaa ttaaacattt    2640
   gcctatgact ccaacag                                                  2657
<210> 38
   <211> 591
   <212> DNA
   <213> Homo sapiens
<400> 38
   cttctctggg acacattgcc ttctgttttc tccagcatgc gcttgctcca gctcctgttc      60
   agggccagcc ctgccaccct gctcctggtt ctctgcctgc agttgggggc caacaaagct     120
   caggacaaca ctcggaagat cataataaag aattttgaca ttcccaagtc agtacgtcca     180
   aatgacgaag tcactgcagt gcttgcagtt caaacagaat tgaaagaatg catggtggtt     240
   aaaacttacc tcattagcag catccctcta caaggtgcat ttaactataa gtatactgcc     300
   tgcctatgtg acgacaatcc aaaaaccttc tactgggact tttacaccaa cagaactgtg     360
   caaattgcag ccgtcgttga tgttattcgg gaattaggca tctgccctga tgatgctgct     420
   gtaatcccca tcaaaaacaa ccggttttat actattgaaa tcctaaaggt agaataatgg     480
   aagccctgtc tgtttgccac acccaggtga tttcctctaa agaaacttgg ctggaatttc     540
   tgctgtggtc tataaaataa acttcttaac atgcttaaaa aaaaaaaaaa a              591
<210> 39
   <211> 1880
   <212> DNA
   <213> Homo sapiens
<400> 39
   gggtcggggc cacaaggccg cgctaggcgg acccaggaca cagcccgcgc gcagcccacc      60
   cgcccgccgc ctgccagagc tgctcggccc gcagccaggg ggacagcggc tggtcggagg     120
   ctcgcagtgc tgtcggcgag aagcagtcgg gtttggagcg cttgggtcgc gttggtgcgc     180
   ggtggaacgc gcccagggac cccagttccc gcgagcagct ccgcgccgcg cctgagagac     240
   taagctgaaa ctgctgctca gctcccaaga tggtgccacc caaattgcat gtgcttttct     300
   gcctctgcgg ctgcctggct gtggtttatc cttttgactg gcaatacata aatcctgttg     360
   cccatatgaa atcatcagca tgggtcaaca aaatacaagt actgatggct gctgcaagct     420
   ttggccaaac taaaatcccc cggggaaatg ggccttattc cgttggttgt acagacttaa     480
   tgtttgatca cactaataag ggcaccttct tgcgtttata ttatccatcc caagataatg     540
   atcgccttga caccctttgg atcccaaata aagaatattt ttggggtctt agcaaatttc     600
   ttggaacaca ctggcttatg ggcaacattt tgaggttact ctttggttca atgacaactc     660
   ctgcaaactg gaattcccct ctgaggcctg gtgaaaaata tccacttgtt gttttttctc      720
   atggtcttgg ggcattcagg acactttatt ctgctattgg cattgacctg gcatctcatg     780
   ggtttatagt tgctgctgta gaacacagag atagatctgc atctgcaact tactatttca     840
   aggaccaatc tgctgcagaa ataggggaca agtcttggct ctaccttaga accctgaaac     900
   aagaggagga gacacatata cgaaatgagc aggtacggca aagagcaaaa gaatgttccc     960
   aagctctcag tctgattctt gacattgatc atggaaagcc agtgaagaat gcattagatt    1020
   taaagtttga tatggaacaa ctgaaggact ctattgatag ggaaaaaata gcagtaattg    1080
   gacattcttt tggtggagca acggttattc agactcttag tgaagatcag agattcagat    1140
   gtggtattgc cctggatgca tggatgtttc cactgggtga tgaagtatat tccagaattc    1200
   ctcagcccct cttttttatc aactctgaat atttccaata tcctgctaat atcataaaaa     1260
   tgaaaaaatg ctactcacct gataaagaaa gaaagatgat tacaatcagg ggttcagtcc    1320
   accagaattt tgctgacttc acttttgcaa ctggcaaaat aattggacac atgctcaaat    1380
   taaagggaga catagattca aatgtagcta ttgatcttag caacaaagct tcattagcat    1440
   tcttacaaaa gcatttagga cttcataaag attttgatca gtgggactgc ttgattgaag    1500
   gagatgatga gaatcttatt ccagggacca acattaacac aaccaatcaa cacatcatgt    1560
   tacagaactc ttcaggaata gagaaataca attaggatta aaataggttt tttaaaagtc    1620
   ttgtttcaaa actgtctaaa attatgtgtg tgtgtgtgtg tgtgtgtgtg tgtgtgagag    1680
   agagagagag agagagagag agagagagag agaattttaa tgtattttcc caaaggactc    1740
   atattttaaa atgtaggcta tactgtaatc gtgattgaag cttggactaa gaattttttc     1800
   cctttagatg taaagaaaga atacagtata caatattcaa aaaaaaaaaa aaaaaaaaaa    1860
   aaaaaaaaaa aaaaaaaaaa                                                1880
<210> 40
   <211> 1038
   <212> DNA
   <213> Homo sapiens
<400> 40
   atttgaggcc atataaagtc acctgaggcc ctctccacca cagcccacca gtgaccacga      60
   aggctgtgct gcttgccctg ttgatggcag gcttggccct gcagccaggc actgccctgc     120
   tgtgctactc ctgcaaagcc caggtgagca acgaggactg cctgcaggtg gagaactgca     180
   cccagctggg ggagcagtgc tggaccgcgc gcatccgcgc agttggcctc ctgaccgtca     240
   tcagcaaagg ctgcagcttg aactgcgtgg atgactcaca ggactactac gtgggcaaga     300
   agaacatcac gtgctgtgac accgacttgt gcaacgccag cggggcccat gccctgcagc     360
   cggctgctgc catccttgcg ctgctccctg cactcggcct gctgctctgg ggacccggcc     420
   agctctaggc tctggggggc cccgctgcag cccacactgg gtgtggtgcc ccaggcctct     480
   gtgccactcc tcacacaccc ggcccagtgg gagcctgtcc tggttcctga ggcacatcct     540
   aacgcaagtc tgaccatgta tgtctgcgcc cctgtccccc accctgaccc tcccatggcc     600
   ctctccagga ctcccacccg gcagatcggc tctattgaca cagatccgcc tgcagatggc     660
   ccctccaacc ctctctgctg ctgtttccat ggcccagcat tctccaccct taaccctgtg     720
   ctcaggcacc tcttccccca ggaagccttc cctgcccacc ccatctatga cttgagccag     780
   gtctggtccg tggtgtcccc cgcacccagc aggggacagg cactcaggag ggcccggtaa     840
   aggctgagat gaagtggact gagtagaact ggaggacagg agtcgacgtg agttcctggg     900
   agtctccaga gatggggcct ggaggcctgg aggaaggggc caggcctcac attcgtgggg     960
   ctccctgaat ggcagcctca gcacagcgta ggcccttaat aaacacctgt tggataagcc    1020
   agaaaaaaaa aaaaaaaa                                                  1038
<210> 41
   <211> 2653
   <212> DNA
   <213> Homo sapiens
<400> 41
   ctcaaaaggg gccggatttc cttctcctgg aggcagatgt tgcctctctc tctcgctcgg      60
   attggttcag tgcactctag aaacactgct gtggtggaga aactggaccc caggtctgga     120
   gcgaattcca gcctgcaggg ctgataagcg aggcattagt gagattgaga gagactttac     180
   cccgccgtgg tggttggagg gcgcgcagta gagcagcagc acaggcgcgg gtcccgggag     240
   gccggctctg ctcgcgccga gatgtggaat ctccttcacg aaaccgactc ggctgtggcc     300
   accgcgcgcc gcccgcgctg gctgtgcgct ggggcgctgg tgctggcggg tggcttcttt     360
   ctcctcggct tcctcttcgg gtggtttata aaatcctcca atgaagctac taacattact     420
   ccaaagcata atatgaaagc atttttggat gaattgaaag ctgagaacat caagaagttc     480
   ttatataatt ttacacagat accacattta gcaggaacag aacaaaactt tcagcttgca     540
   aagcaaattc aatcccagtg gaaagaattt ggcctggatt ctgttgagct agcacattat     600
   gatgtcctgt tgtcctaccc aaataagact catcccaact acatctcaat aattaatgaa     660
   gatggaaatg agattttcaa cacatcatta tttgaaccac ctcctccagg atatgaaaat     720
   gtttcggata ttgtaccacc tttcagtgct ttctctcctc aaggaatgcc agagggcgat     780
   ctagtgtatg ttaactatgc acgaactgaa gacttcttta aattggaacg ggacatgaaa     840
   atcaattgct ctgggaaaat tgtaattgcc agatatggga aagttttcag aggaaataag     900
   gttaaaaatg cccagctggc aggggccaaa ggagtcattc tctactccga ccctgctgac     960
   tactttgctc ctggggtgaa gtcctatcca gatggttgga atcttcctgg aggtggtgtc    1020
   cagcgtggaa atatcctaaa tctgaatggt gcaggagacc ctctcacacc aggttaccca    1080
   gcaaatgaat atgcttatag gcgtggaatt gcagaggctg ttggtcttcc aagtattcct    1140
   gttcatccaa ttggatacta tgatgcacag aagctcctag aaaaaatggg tggctcagca    1200
   ccaccagata gcagctggag aggaagtctc aaagtgccct acaatgttgg acctggcttt    1260
   actggaaact tttctacaca aaaagtcaag atgcacatcc actctaccaa tgaagtgaca    1320
   agaatttaca atgtgatagg tactctcaga ggagcagtgg aaccagacag atatgtcatt    1380
   ctgggaggtc accgggactc atgggtgttt ggtggtattg accctcagag tggagcagct    1440
   gttgttcatg aaattgtgag gagctttgga acactgaaaa aggaagggtg gagacctaga    1500
   agaacaattt tgtttgcaag ctgggatgca gaagaatttg gtcttcttgg ttctactgag    1560
   tgggcagagg agaattcaag actccttcaa gagcgtggcg tggcttatat taatgctgac    1620
   tcatctatag aaggaaacta cactctgaga gttgattgta caccgctgat gtacagcttg    1680
   gtacacaacc taacaaaaga gctgaaaagc cctgatgaag gctttgaagg caaatctctt    1740
   tatgaaagtt ggactaaaaa aagtccttcc ccagagttca gtggcatgcc caggataagc    1800
   aaattgggat ctggaaatga ttttgaggtg ttcttccaac gacttggaat tgcttcaggc    1860
   agagcacggt atactaaaaa ttgggaaaca aacaaattca gcggctatcc actgtatcac    1920
   agtgtctatg aaacatatga gttggtggaa aagttttatg atccaatgtt taaatatcac    1980
   ctcactgtgg cccaggttcg aggagggatg gtgtttgagc tagccaattc catagtgctc    2040
   ccttttgatt gtcgagatta tgctgtagtt ttaagaaagt atgctgacaa aatctacagt    2100
   atttctatga aacatccaca ggaaatgaag acatacagtg tatcatttga ttcacttttt    2160
   tctgcagtaa agaattttac agaaattgct tccaagttca gtgagagact ccaggacttt    2220
   gacaaaagca acccaatagt attaagaatg atgaatgatc aactcatgtt tctggaaaga    2280
   gcatttattg atccattagg gttaccagac aggccttttt ataggcatgt catctatgct 2340
   ccaagcagcc acaacaagta tgcaggggag tcattcccag gaatttatga tgctctgttt 2400
   gatattgaaa gcaaagtgga cccttccaag gcctggggag aagtgaagag acagatttat 2460
   gttgcagcct tcacagtgca ggcagctgca gagactttga gtgaagtagc ctaagaggat 2520
   tctttagaga atccgtattg aatttgtgtg gtatgtcact cagaaagaat cgtaatgggt 2580
   atattgataa attttaaaat tggtatattt gaaataaagt tgaatattat atataaaaaa 2640
   aaaaaaaaaa aaa 2653
<210> 42
   <211> 594
   <212> DNA
   <213> Homo sapiens
<400> 42
   aggctcacta taaatagcag ccacctctcc ctggcagaca gggacccgca gctcagctac 60
   agcacagatc agcaccatga agcttctcac gggcctggtt ttctgctcct tggtcctgag 120
   tgtcagcagc cgaagcttct tttcgttcct tggcgaggct tttgatgggg ctcgggacat 180
   gtggagagcc tactctgaca tgagagaagc caattacatc ggctcagaca aatacttcca 240
   tgctcggggg aactatgatg ctgccaaaag gggacctggg ggtgcctggg ctgcagaagt 300
   gatcagcaat gccagagaga atatccagag actcacaggc cgtggtgcgg aggactcgct 360
   ggccgatcag gctgccaata aatggggcag gagtggcaga gaccccaatc acttccgacc 420
   tgctggcctg cctgagaaat actgagcttc ctcttcactc tgctctcagg agacctggct 480
   atgaggccct cggggcaggg atacaaagtt agtgaggtct atgtccagag aagctgagat 540
   atggcatata ataggcatct aataaatgct taagaggtgg aatttgttga aaca 594
<210> 43
   <211> 3220
   <212> DNA
   <213> Homo sapiens
<400> 43
   acaatgactc ctttcggtaa gtgcagtgga agctgtacac tgcccaggca aagcgtccgg      60
   gcagcgtagg cgggcgactc agatcccagc cagtggactt agcccctgtt tgctcctccg     120
   ataactgggg tgaccttggt taatattcac cagcagcctc ccccgttgcc cctctggatc      180
   cactgcttaa atacggacga ggacagggcc ctgtctcctc agcttcaggc accaccactg     240
   acctgggaca gtgaatcgac aatgccgtct tctgtctcgt ggggcatcct cctgctggca     300
   ggcctgtgct gcctggtccc tgtctccctg gctgaggatc cccagggaga tgctgcccag     360
   aagacagata catcccacca tgatcaggat cacccaacct tcaacaagat cacccccaac     420
   ctggctgagt tcgccttcag cctataccgc cagctggcac accagtccaa cagcaccaat     480
   atcttcttct ccccagtgag catcgctaca gcctttgcaa tgctctccct ggggaccaag     540
   gctgacactc acgatgaaat cctggagggc ctgaatttca acctcacgga gattccggag     600
   gctcagatcc atgaaggctt ccaggaactc ctccgtaccc tcaaccagcc agacagccag     660
   ctccagctga ccaccggcaa tggcctgttc ctcagcgagg gcctgaagct agtggataag     720
   tttttggagg atgttaaaaa gttgtaccac tcagaagcct tcactgtcaa cttcggggac     780
   accgaagagg ccaagaaaca gatcaacgat tacgtggaga agggtactca agggaaaatt     840
   gtggatttgg tcaaggagct tgacagagac acagtttttg ctctggtgaa ttacatcttc     900
   tttaaaggca aatgggagag accctttgaa gtcaaggaca ccgaggaaga ggacttccac     960
   gtggaccagg tgaccaccgt gaaggtgcct atgatgaagc gtttaggcat gtttaacatc    1020
   cagcactgta agaagctgtc cagctgggtg ctgctgatga aatacctggg caatgccacc    1080
   gccatcttct tcctgcctga tgaggggaaa ctacagcacc tggaaaatga actcacccac    1140
   gatatcatca ccaagttcct ggaaaatgaa gacagaaggt ctgccagctt acatttaccc    1200
   aaactgtcca ttactggaac ctatgatctg aagagcgtcc tgggtcaact gggcatcact    1260
   aaggtcttca gcaatggggc tgacctctcc ggggtcacag aggaggcacc cctgaagctc    1320
   tccaaggccg tgcataaggc tgtgctgacc atcgacgaga aagggactga agctgctggg    1380
   gccatgtttt tagaggccat acccatgtct atcccccccg aggtcaagtt caacaaaccc     1440
   tttgtcttct taatgattga acaaaatacc aagtctcccc tcttcatggg aaaagtggtg    1500
   aatcccaccc aaaaataact gcctctcgct cctcaacccc tcccctccat ccctggcccc    1560
   ctccctggat gacattaaag aagggttgag ctggtccctg cctgcatgtg actgtaaatc    1620
   cctcccatgt tttctctgag tctccctttg cctgctgagg ctgtatgtgg gctccaggta    1680
   acagtgctgt cttcgggccc cctgaactgt gttcatggag catctggctg ggtaggcaca    1740
   tgctgggctt gaatccaggg gggactgaat cctcagctta cggacctggg cccatctgtt    1800
   tctggagggc tccagtcttc cttgtcctgt cttggagtcc ccaagaagga atcacagggg    1860
   aggaaccaga taccagccat gaccccaggc tccaccaagc atcttcatgt ccccctgctc   1920
   atcccccact cccccccacc cagagttgct catcctgcca gggctggctg tgcccacccc    1980
   aaggctgccc tcctgggggc cccagaactg cctgatcgtg ccgtggccca gttttgtggc    2040
   atctgcagca acacaagaga gaggacaatg tcctcctctt gacccgctgt cacctaacca    2100
   gactcgggcc ctgcacctct caggcacttc tggaaaatga ctgaggcaga ttcttcctga    2160
   agcccattct ccatggggca acaaggacac ctattctgtc cttgtccttc catcgctgcc    2220
   ccagaaagcc tcacatatct ccgtttagaa tcaggtccct tctccccaga tgaagaggag    2280
   ggtctctgct ttgttttctc tatctcctcc tcagacttga ccaggcccag caggccccag    2340
   aagaccatta ccctatatcc cttctcctcc ctagtcacat ggccataggc ctgctgatgg    2400
   ctcaggaagg ccattgcaag gactcctcag ctatgggaga ggaagcacat cacccattga    2460
   cccccgcaac ccctcccttt cctcctctga gtcccgactg gggccacatg cagcctgact    2520
   tctttgtgcc tgttgctgtc cctgcagtct tcagagggcc accgcagctc cagtgccacg    2580
   gcaggaggct gttcctgaat agcccctgtg gtaagggcca ggagagtcct tccatcctcc    2640
   aaggccctgc taaaggacac agcagccagg aagtcccctg ggcccctagc tgaaggacag    2700
   cctgctccct ccgtctctac caggaatggc cttgtcctat ggaaggcact gccccatccc    2760
   aaactaatct aggaatcact gtctaaccac tcactgtcat gaatgtgtac ttaaaggatg    2820
   aggttgagtc ataccaaata gtgatttcga tagttcaaaa tggtgaaatt agcaattcta    2880
   catgattcag tctaatcaat ggataccgac tgtttcccac acaagtctcc tgttctctta    2940
   agcttactca ctgacagcct ttcactctcc acaaatacat taaagatatg gccatcacca    3000
   agccccctag gatgacacca gacctgagag tctgaagacc tggatccaag ttctgacttt   3060
   tccccctgac agctgtgtga ccttcgtgaa gtcgccaaac ctctctgagc cccagtcatt    3120
   gctagtaaga cctgcctttg agttggtatg atgttcaagt tagataacaa aatgtttata    3180
   cccattagaa cagagaataa atagaactac atttcttgca                          3220
<210> 44
   <211> 3783
   <212> DNA
   <213> Homo sapiens
<400> 44
   gtattctgag gcgcgtggta gtgatggcgg cgctcagtga gactttcctg tcactggcta     60
   ctactactcc caaccctcct caaagccgcc ggagcaaccc ccaggtcttt actttacaat    120
   cggcaatttg acttgctctg ctgcatgtct ggagggacca aggaaagtgt ggagacgctc    180
   caaggattag gtgatcggag cttgaaaaga aaaaaagcca aacaaataaa caaaacccac    240
   ccaccctaac aaatatgagg ctgctggaga gaatgaggaa agactggttc atggtcggaa    300
   tagtgctggc gatcgctgga gctaaactgg agccgtccat aggggtgaat gggggaccac    360
   tgaagccaga aataactgta tcctacattg ctgttgcaac aatattcttt aacagtggac    420
   tatcattgaa aacagaggag ctgaccagtg ctttggtgca tctaaaactg catcttttta    480
   ttcagatctt tactcttgca ttcttcccag caacaatatg gctttttctt cagcttttat    540
   caatcacacc catcaacgaa tggcttttaa aaggtttgca gacagtaggt tgcatgcctc    600
   cgcctgtgtc ttctgcagtg attttaacca aggcagttgg tggaaatgag gcagctgcaa    660
   tatttaattc agcctttgga agttttttgg gcatcgttat aacacccctg ctcctgctgc    720
   tttttcttgg ttcatcttct tctgtgcctt tcacatctat tttttctcag ctttttatga    780
   ctgttgtggt tcctctcatc attggacaga ttgtccgaag atacatcaag gattggcttg    840
   agagaaagaa gcctcctttt ggtgctatca gcagcagtgt actcctcatg atcatctaca    900
   caacattctg tgacacgttc tctaacccaa atattgacct ggataaattc agccttgttc    960
   tcatactgtt cataatattt tctatccagc tgagttttat gcttttaact ttcatctttt   1020
   caacaaggaa taattcgggt ttcacaccag cagacacagt ggctatcatt ttctgttcta   1080
   cacacaaatc ccttacattg ggaattccga tgctgaagat cgtgtttgca ggccatgagc   1140
   atctctcttt aatatctgta cccttgctca tctaccaccc agctcagatc cttctgggaa   1200
   gtgtgttggt gccaacaatc aagtcttgga tggtatcaag gcagaaggga gtgaagctga   1260
   caaggccgac agtataacaa aggaggtgga ctttctgtag caatgtatat atgtacagga   1320
   ttgtacatac tagcaattct gaagacttgt acttgtgaat gttgcctcaa tgcatatttt   1380
   atttttttac acaaaaatat gagatcctgt ttaagtgcct taaaatgtat ttgacaagag   1440
   cgttatttcc aaaatatgct ttgttgatta ctgccagggg tggtacaata tttgggggtt   1500
   aattttgctt tcctaatgca ggaatcagtc atggtaagtg acaaaaagca aacatgcttt   1560
   ccctgcagca cctttgtgca atacaaccct atagtagtta ctgtaatgtt tgaaatgagg   1620
   tcacaccatc aggaaaatgc ccttctgatg acagtgaaaa tttccaaagt cttattcatg   1680
   catactttga tttactgtgt gattcttttt ttctacgact gtgacatgcc tcttccttat   1740
   caactcagca ggggtcatag atcgaataga tgctgaaaag cgtaagatat atgcattcct   1800
   tgacatcatt tttaaagaca ttccttcaaa tagtttccac acagaaattc ctcactccca   1860
   ttatgagaga ttgtggttat atgtcttaaa tttattataa gctgcttcaa agaaagggtc   1920
   tgaatgtttg aattatgagt gaaatcatgt gaaattttga gttaaactct gtgatttgat   1980
   tttcagggtc tttaaaatat atcttaatat cttcttcctc tttattcaat aatttctgtc   2040
   ttgcacttac acactcataa cagccaaata tgaggcacaa aaatgttaca atcagtttga   2100
   aagcagcatc aattaatggt agattctatt cacattccac aacccagacc aaattttttt   2160
   cctattacgc agatgtgctg agcactttcc agattgcccc tgttggccaa aagcagcctg   2220
   ttacatcctg gaattaagca cacttaaggt atttgagaca atttattaat gaaaatttcc   2280
   ttggcagatt tgacaaatgt tggcaatatt tttttagaag ttaaatcata ttgctttcat   2340
   gaataaatga aaatataaag gtcatggatg caaacaaatg ttacatatac acattctgtc   2400
   tctccagatg aaaagaacat gcaaaaccat ttaataacca aaatatcaag taaaattagt   2460
   tcccaacggg gcagcagctt tcaaatgagt gtccaatatt tgcttctgct atagctgcaa   2520
   gaactgtaac tggacccaag tagagaatga agccacgtat agaactacga gaacactttt   2580
   ctgtgtttcc cccatgccgt cctgtcacat cctcttacac gtcctctctt gatttgatag   2640
   acaatattgg catcctgggt ctcactgagg ccgtgctatg tcctcagcag ctgtttttgt   2700
   tgtttcgtta ttatgcccac aacaaaaaat cattccttag aaactcacca agtttatcta   2760
   ctgtgtaaat ttatattatt gttactacca ggtctcatct tttgtcaatg tcattgaata   2820
   aatttcataa gagttattct cagtgtgaat tttaaggcta atgccagatc ctgcaaaaat   2880
   ctatgctaac caggctgtag tacacactgt tataaagaat tttacttgtg tctaaaacta   2940
   cagtaatttt gcttaggtaa ttgtgcttac ctatggagca caggaaggct cttaggtttt   3000
   gttcctacaa gtttctttga attttggagt aaatggaagt gtctgtctgt ctgtcatcta   3060
   tctgccctat cataaaaatc tttctcccta acattaaaat actgatcccc gcccccaact   3120
   tatctacctc tattgtctaa cacctatagt aggtgtgatc atgggataaa attcaactga   3180
   aaatgctatg ataacatttt atcgtttgct ttaaaaatgt gctttgtttt caaataatct   3240
   ttacatagtg aactttggtg gcgttagtga tatgtttatg cctatttctt ttttttacac   3300
   aaattccttg gcatattttt tcataaagaa caaaaaataa aatcaaaatt tatttttaat   3360
   tcatgcttat tgggatttaa ttattcagag cttaaaatat tttgttatgt ttatacactg   3420
   taaagctatc tgttttatgc atttgttttg tctaaatgta tttatgaaag aaatacatta   3480
   gattatattt atgtttactc atttttccac ctggattttt tttaatggtt gttacaaaat   3540
   tagatttttt aatgggtaat aatgttggta ttttcatgtt ttttcttagt attaaaattt   3600
   ttgtgggttt tttaaaattt ttccctattc tgttaaaaat taacacacct ctagctaatg   3660
   ttcagtgttt gtgctaaata ccaaattttt tcaaaaggat tggttaagtc ataaagtgga   3720
   ttatttatga tgactggaag atgaaaataa ttatatgatt aaacaaagaa tgtttcagaa   3780
   atc                                                                 3783
<210> 45
   <211> 6937
   <212> DNA
   <213> Homo sapiens
<400> 45
   ccgggtcctg ggcgagcggg cgccgtgcgc gtgtcccgcg gccgagctgc taataaagtt      60
   gcagcgagga gaagcgcagc gacggcgtcg ggagagcgcg cctagccggc tcgcgagact     120
   tgacccaatg aaagaagcat atggcacttg tgaagataaa tgttactcct ccctttttaa    180
   ttggaacttc tgcttaggac ctgtgtatga cgtttcacct gtgatctgtt ctttcggtag     240
   ccactgactt tgagttacag gaaggtctcc gaagatttgt gtcaaatgac gtcaatggcc     300
   agcttgtttt cttttactag tccagcagta aagcgattgt tgggctggaa acaaggtgat     360
   gaggaggaga aatgggcaga aaaggcagtt gatgctttgg tgaagaaact aaaaaagaaa     420
   aagggtgcca tggaggaact ggagaaagcc ttgagcagtc caggacagcc gagtaaatgt     480
   gtcactattc ccagatcttt agatggacgc ctgcaggttt ctcacagaaa aggcttaccc     540
   catgttatat attgtcgtgt ttggcgctgg ccggatttgc agagtcatca tgagctaaag     600
   ccgttggata tttgtgaatt tccttttgga tctaagcaaa aagaagtttg tatcaaccca     660
   taccactata agagagtgga gagtccagtc ttacctccag tattagtgcc tcgtcataat     720
   gaattcaatc cacaacacag ccttctggtt cagtttagga acctgagcca caatgaacca     780
   cacatgccac aaaatgccac gtttccagat tctttccacc agcccaacaa cactcctttt     840
   cccttatctc caaacagccc ttatccccct tctcctgcta gcagcacata tcccaactcc    900
   ccagcaagtt ctggaccagg aagtccattt cagctcccag ctgatacgcc tcctcctgcc     960
   tatatgccac ctgatgatca gatgggtcaa gataattccc agcctatgga tacaagcaat    1020
   aatatgattc ctcagattat gcccagtata tccagcaggg atgttcagcc tgttgcctat    1080
   gaagagccta aacattggtg ttcaatagtc tactatgaat taaacaatcg tgttggagaa    1140
   gcttttcatg catcttctac tagtgtgtta gtagatggat tcacagatcc ttcaaataac    1200
   aaaagtagat tctgcttggg tttgttgtca aatgttaatc gtaattcgac aattgaaaac    1260
   actaggcgac atattggaaa aggtgttcat ctgtactatg ttggtggaga ggtgtatgcg    1320
   gaatgcctca gtgacagcag catatttgta cagagtagga actgcaactt tcatcatggc    1380
   tttcatccca ccactgtctg taagattccc agcagctgca gcctcaaaat ttttaacaat    1440
   caggagtttg ctcagcttct ggctcaatct gtcaaccatg ggtttgaggc agtatatgag    1500
   ctcaccaaaa tgtgtaccat tcggatgagt tttgtcaagg gttggggagc agaatatcac    1560
   cggcaggatg taaccagcac cccatgttgg attgagattc atcttcatgg gcctcttcag    1620
   tggctggata aagtccttac tcagatgggc tcccctctga accccatatc ttctgtttca   1680
   taatgcagaa gtattctttt caattatatt gttagtggac ttgttttaat tttagagaaa    1740
   ctttgagtac agatactgtg agcttacatt gaaaacagat attacagctt atttttttct    1800
   acataattgt gaccaataca tttgtatttt gtgatgaatc tacatttgtt tgtattcatg    1860
   ttcatgtgat taactcttag aagtgttgta aaagatgcag agtaagtatt atgccccagt    1920
   tcagaaattt ggcattgatc ttaaactgga acatgctttt actttattgc cctaacaatt    1980
   ttttattaaa tttatttgaa aatgcatcac atgatgaaaa attatagtag cttataagag    2040
   ggcatataca gtgaagagta agttttccct cctactctcg atcttccaga agctgtactt    2100
   ttaccagttt ctttgtccca ccaacttaaa aaaaaaaagt acaattcatt gttttgcaaa    2160
   agtgtatggt aggggcttaa aagaaactat aaagttttat ttgaatgaac actatgcact    2220
   gctgtaactg gtagtgttca gtaaaagcaa aatgatagtt ttctagatga cataaaattt    2280
   acatttaata cagataagtg ttcttcagtg taatgtgact tcatgctata tatcttttgt    2340
   aagacatttc cttttttaaa aaaatttttg caaataactg atctcaagta tatgtcattt   2400
   actcaaaatc tgtcataagc attactttat agctagtgac agtgcatgca cagccttgtt    2460
   caactatgtt tgctgctttt ggacaatgtt gcaagaactc tatttttgac atgcattaat 2520
   cttttatttt gcacttttat gggtgacagt ttttagcata acctttgata aaatacactc 2580
   aagtgacttg gacttagatg cttatcctta cgtccttggt accttttttg tattaacaaa 2640
   cactgcaatt tatagattac atttgtagga agttatgctt ttttctggtt tttgttttac 2700
   tttcaaccta ggttataaga ctgttattct atagctccaa cttaaggtgc ctttttaatt 2760
   ccctacagtt ttatgggtgt tatcagtgct ggagaatcat gtagttaatc ccattgctct 2820
   tacaagtgtc agcttacttg tatcagcctc cctacgcaag gacctatgca ctggagccgt 2880
   aggaggctct tcagttgggc cccaaggata aggctactga tttgatacta aatgaatcag 2940
   cagtggatgt agggatagct gattttaaaa cactcggctg ggcacagtgg ctcacacctg 3000
   taatcccagc actttgggag gctgaggcag gcagatcatg atgtcaggag tttgagacca 3060
   gcctggccaa tatggtgaaa ccctgtctct acaaaaaata caaaaattag ctgggcatgg 3120
   tggtgcgtgc ctgaagtccc agctactcgg gaagctgagg cagaagaatc acttgaacct 3180
   gggaggcgga ggttgtggtg agccgagatc gcaccactgc actccagcct gggcgacaga 3240
   gcgagactct gcctcaaaaa acaaaacaaa acaaaacact cacccatcaa cgaatataga 3300
   ctcttctctc atttatcgat gatcctcttt ttccattttt taagtactta tgtggaagct 3360
   agtctcccaa aacacaatct ttagagagaa aagacatgaa cgaactccaa aatatccatt 3420
   taatcaatca tgtttttggc tttggataaa gaactttgaa ccagtttttt tctcaggagc 3480
   tgtcaaatgg acacttaatt atgacatgag aatgaagaaa ttattttgga aaaaaaaaat 3540
   gacctaattt acctatcagt gaaagcttta ttttctggtg ccttttgaaa gtatatggag 3600
   tcatatcatt cttctgttta aaatgttagt ttggtttgac tttccacttt gtcctttctg 3660
   ctcttgtgaa gaaaaaaaaa agcattttcg aggaaagaat tatgcaattt cttttgtttt 3720
   ctgtgtcatt atttattgct ttttcaatgt gcagccagtg gatggtttta gttctttcag 3780
   atgaactgcc atttgtgttt cagctcacag ttctttgctg ggtaaaagaa atactttctg 3840
   acagtcacct gagccttaaa tgtaagtatt acatgacatg cattctgttt cttccagagt 3900
   tctgtctgcc acacgaaaga gaatatttgc ttacttgata gaactttggc attttcatca 3960
   ttcttttact taaccaggct tatggcatga tctctggaac aaatttgtag gaaaaaatta 4020
   ctccaattga atgactgatg tatgtaatca acttcattgg gctgcagtaa actagtggaa 4080
   attagagagt tgttttattg gtgttttcta ctgtgagtta attaaaaatt gtttttattt 4140
   ggggtcatta tgtcacagtc ttgagttaac aagatcttac gtgattggcc ttttctttgt 4200
   tttctcttag gagttgtgtc tcatgaatga cagtactaaa gctattaaca actaagagtt 4260
   tgacagagaa ctataagcct gttgtatctc ctaaaagttg tcaactcccc acccttggac 4320
   tttaaatgaa aattttattc agtccagcta ttcttacagt ccctaaggat tttcatatat 4380
   ctatgtatag gagataaaat ttgctagtaa gatttttaaa aactggctag tgaaaggaaa 4440
   gtacctctga aagaaaccat tttagcaaat tatggttata tgttttaatt taatctacag 4500
   aatgttttat agtaaaattc tagcaccact agaataatca catagcatgt acaatatatt 4560
   tatgctggct gaaaagacag aatctgggaa taataaaatt gcaaccagtt tggtaatgca 4620
   aacagcagaa tagaatgaaa tctcagtaat gaattaaagc aacaaaaaga tattgattgg 4680
   caaaaagcaa gatataagag attcatttgc ttaacatttc tacataatat ttatggtctg 4740
   gtcagtattg gtctggtcag tattgcctgg ctgacgtgaa atgtaaacta gtaggcgtgt 4800
   tattgatctg ctaaaactaa ccctcttttt aagaggagat ttaaggaaga cgtcaatcaa 4860
   aatgtcaaat atgtgtgtca gaatataaat aatttttcac attgtattgt tgctatataa 4920
   aaaaaataat agaattggtt gggtttctga ggtgaaatcc agagtaagag tactagacag 4980
   ttcaacaagc cacatctaat ggcacagata gaggatgtag ctattttata cctttcataa 5040
   catttgagag taagatatcc ttcaggatgt gaagtgatta ttaagtactc atacctgaaa 5100
   tctgttgtca agattagaac tggggttcat gttaaaaacc ttccatatta cctgagggta 5160
   cctgtgggga acagttcctt cccctgtgtg gtagtatttt gttggaagag aatgtttata 5220
   caaaaaatga aattcttcca acagcagaga aactctaaaa agtttgatag tacctatcaa 5280
   agtgctgtac ttctgtgata gagaacatct gatgtaccaa tttagatcta tttctttata 5340
   ctttttctaa tcaattgctt aatagtactt tggatgatta tcacctttgc cacttaaaat 5400
   atataaatat cctttttact tcatgaggaa ggaagaattt tttgataatt actgagttca 5460
   gccttttgtg atgacttata ttttggactt acattttaac tttaaagaat gtcagatccc 5520
   ttctttgtct tactagttaa atcctcacct aatctcttgg gtatgaatat aaatgtgtgt 5580
   catcgttata ttgttcagct agatgagcaa gtatcttagg gtagtaggta gcctggtggt 5640
   tttagaagtg tttggtgatt tttatggaga gagttttcct aagtggtggt ttataggtgg 5700
   tatcagatat tattagggca gctttttggg gagtaatctc aggtctccca gagcagcagc 5760
   atttttctca ttgatataag taagattctt aggagctttt cttatcacac aagatgcctg 5820
   aatcgaatgt gagaattgaa ggcatttctt ctgcataaac aaagaattct acctgctgga 5880
   cagaaacctg gaaagttctt tggaattcgc tgaattacag tttagtatgt cctgattaca 5940
   gagtgacaat atttatcaag cctttgttat attggattat cttctctctt aaaatacaac 6000
   tgtattataa ttgaaatgac agcccaaaat tggatggttt accaaaacca atgaaaggga 6060
   tttcacacat caatttttat ttctgttttg aagagcacat gctatataat aattgctagt 6120
   agcaactgca gtaaaacagg tgataagtta ttttctctga aaagatccag tcctagagca 6180
   ggattcttcg atcattcatg gcagagtgaa aaaggtttgt atggttcttg tccaaataac 6240
   tcagttctta aaattcttaa aatgatcgta aaccattatc ctttaaaggt ttatttgaag 6300
   atgctgttaa agtacagaat tttgtgtaca ggtagatttt tccgtccctc attaatagtg 6360
   ccttcttaat taatacagac tggtgttagc tataacaaaa ctccagtaag gccaaagaat 6420
   cccaagttct ttgtggaaaa aaaaaaaaaa tcttttaggg tcagattttc ccttctaata 6480
   tcattgaaga tgatgttgca ttgatttatt cataaagtat tttaactata ggaactctag 6540
   aagataatgg ttaggcaagt gatttttttt ttaaatatgg ttggcgtaag ttgtattttg     6600
   aaattcactt attttaaaat cgaagaggat tgtaatcatg gaaatagaat gtttgtatct     6660
   acctgcccac attttcttaa aaagatattt catatacaga taatgaagac caagctagtg     6720
   gctgcactgt aggtctgctg cttatttgta tttgttgtgc ttctgtttat gttgtagaag     6780
   ctgaaattct agcaacatgc ttcaattctg ttattttgat acttatgaaa atgtattagg     6840
   ttttactata ttgtgctttt gaaagccata actcttaaga actttgtttt tgcatattgt     6900
   ttgctaattc tttactttaa taaacctcaa aacctgc                              6937
<210> 46
   <211> 1883
   <212> DNA
   <213> Homo sapiens
<400> 46
   gggaggggag gggagggata ggacagacag acaaagaaag gggtgcggca gcactgccag       60
   gggaagaggg tgatccgacc cggggaaggt cgctgggcag ggcgagttgg gaaagcggca      120
   gcccccgccg cccccgcagc cccttctcct cctttctccc acgtcctatc tgcctctcgc      180
   tggaggccag gccgtgcagc atcgaagaca ggaggaactg gagcctcatt ggccggcccg      240
   gggcgccggc ctcgggctta aataggagct ccgggctctg gctgggaccc gaccgctgcc      300
   ggccgcgctc ccgctgctcc tgccgggtga tggaaaaccc cagcccggcc gccgccctgg      360
   gcaaggccct ctgcgctctc ctcctggcca ctctcggcgc cgccggccag cctcttgggg      420
   gagagtccat ctgttccgcc agagccccgg ccaaatacag catcaccttc acgggcaagt      480
   ggagccagac ggccttcccc aagcagtacc ccctgttccg cccccctgcg cagtggtctt      540
   cgctgctggg ggccgcgcat agctccgact acagcatgtg gaggaagaac cagtacgtca      600
   gtaacgggct gcgcgacttt gcggagcgcg gcgaggcctg ggcgctgatg aaggagatcg      660
   aggcggcggg ggaggcgctg cagagcgtgc acgcggtgtt ttcggcgccc gccgtcccca      720
   gcggcaccgg gcagacgtcg gcggagctgg aggtgcagcg caggcactcg ctggtctcgt      780
   ttgtggtgcg catcgtgccc agccccgact ggttcgtggg cgtggacagc ctggacctgt      840
   gcgacgggga ccgttggcgg gaacaggcgg cgctggacct gtacccctac gacgccggga      900
   cggacagcgg cttcaccttc tcctccccca acttcgccac catcccgcag gacacggtga      960
   ccgagataac gtcctcctct cccagccacc cggccaactc cttctactac ccgcggctga     1020
   aggccctgcc tcccatcgcc agggtgacac tggtgcggct gcgacagagc cccagggcct     1080
   tcatccctcc cgccccagtc ctgcccagca gggacaatga gattgtagac agcgcctcag     1140
   ttccagaaac gccgctggac tgcgaggtct ccctgtggtc gtcctgggga ctgtgcggag     1200
   gccactgtgg gaggctcggg accaagagca ggactcgcta cgtccgggtc cagcccgcca     1260
   acaacgggag cccctgcccc gagctcgaag aagaggctga gtgcgtccct gataactgcg     1320
   tctaagacca gagccccgca gcccctgggg ccccccggag ccatggggtg tcgggggctc     1380
   ctgtgcaggc tcatgctgca ggcggccgag ggcacagggg gtttcgcgct gctcctgacc     1440
   gcggtgaggc cgcgccgacc atctctgcac tgaagggccc tctggtggcc ggcacgggca     1500
   ttgggaaaca gcctcctcct ttcccaacct tgcttcttag gggcccccgt gtcccgtctg     1560
   ctctcagcct cctcctcctg caggataaag tcatccccaa ggctccagct actctaaatt     1620
   atgtctcctt ataagttatt gctgctccag gagattgtcc ttcatcgtcc aggggcctgg     1680
   ctcccacgtg gttgcagata cctcagacct ggtgctctag gctgtgctga gcccactctc     1740
   ccgagggcgc atccaagcgg gggccacttg agaagtgaat aaatggggcg gtttcggaag     1800
   cgtcagtgtt tccatgttat ggatctctct gcgtttgaat aaagactatc tctgttgctc     1860
   acaaaaaaaa aaaaaaaaaa aaa                                             1883
<210> 47
   <211> 1616
   <212> DNA
   <213> Homo sapiens
<400> 47
   ctccctgtgt tggtggagga tgtctgcagc agcatttaaa ttctgggagg gcttggttgt      60
   cagcagcagc aggaggaggc agagcacagc atcgtcggga ccagactcgt ctcaggccag     120
   ttgcagcctt ctcagccaaa cgccgaccaa ggaaaactca ctaccatgag aattgcagtg     180
   atttgctttt gcctcctagg catcacctgt gccataccag ttaaacaggc tgattctgga     240
   agttctgagg aaaagcagct ttacaacaaa tacccagatg ctgtggccac atggctaaac     300
   cctgacccat ctcagaagca gaatctccta gccccacaga cccttccaag taagtccaac     360
   gaaagccatg accacatgga tgatatggat gatgaagatg atgatgacca tgtggacagc     420
   caggactcca ttgactcgaa cgactctgat gatgtagatg acactgatga ttctcaccag     480
   tctgatgagt ctcaccattc tgatgaatct gatgaactgg tcactgattt tcccacggac     540
   ctgccagcaa ccgaagtttt cactccagtt gtccccacag tagacacata tgatggccga     600
   ggtgatagtg tggtttatgg actgaggtca aaatctaaga agtttcgcag acctgacatc     660
   cagtaccctg atgctacaga cgaggacatc acctcacaca tggaaagcga ggagttgaat     720
   ggtgcataca aggccatccc cgttgcccag gacctgaacg cgccttctga ttgggacagc     780
   cgtgggaagg acagttatga aacgagtcag ctggatgacc agagtgctga aacccacagc     840
   cacaagcagt ccagattata taagcggaaa gccaatgatg agagcaatga gcattccgat     900
   gtgattgata gtcaggaact ttccaaagtc agccgtgaat tccacagcca tgaatttcac     960
   agccatgaag atatgctggt tgtagacccc aaaagtaagg aagaagataa acacctgaaa    1020
   tttcgtattt ctcatgaatt agatagtgca tcttctgagg tcaattaaaa ggagaaaaaa    1080
   tacaatttct cactttgcat ttagtcaaaa gaaaaaatgc tttatagcaa aatgaaagag    1140
   aacatgaaat gcttctttct cagtttattg gttgaatgtg tatctatttg agtctggaaa    1200
   taactaatgt gtttgataat tagtttagtt tgtggcttca tggaaactcc ctgtaaacta    1260
   aaagcttcag ggttatgtct atgttcattc tatagaagaa atgcaaacta tcactgtatt    1320
   ttaatatttg ttattctctc atgaatagaa atttatgtag aagcaaacaa aatactttta    1380
   cccacttaaa aagagaatat aacattttat gtcactataa tcttttgttt tttaagttag    1440
   tgtatatttt gttgtgatta tctttttgtg gtgtgaataa atcttttatc ttgaatgtaa    1500
   taagaatttg gtggtgtcaa ttgcttattt gttttcccac ggttgtccag caattaataa    1560
   aacataacct tttttactgc ctaaaaaaaa aaaaaaaaaa aaaaaaaaaa aaaaaa        1616
<210> 48
   <211> 4044
   <212> DNA
   <213> Homo sapiens
<400> 48
<210> 49
   <211> 4510
   <212> DNA
   <213> Homo sapiens
<400> 49
   gctgaggcca ggagggcgca ctggggattg gaggcgaggg aagtgcaggg cgcatcccag     60
   gcggcagggc tcccagcatc ggcagtcgcc atcaccgcca gaccgcagag acaggttcgg    120
   atccgcggtc ctcttgcctc tttccaggcc tcgatgagtg ttaaatcgcc atttaatgtg    180
   atgtcaagaa ataatttgga agcacctcct tgtaagatga cagagccatt taattttgag    240
   aaaaatgaaa acaagcttcc accacatgag tctttaagaa gtcctggaac acttcctaac    300
   caccctaatt tcaggctgaa aagctcagag aatggaaata aaaagaacaa ttttttgctt    360
   tgtgagcaaa ccaaacaata tttggctagt caggaagaca attcagtttc ttcaaacccg    420
   aatggcatca acggagaagt agttggctcc aaaggagaca ggaaaaaatt gccagcagga    480
   aactcagtgt caccaccaag tgctgaaagt aattcaccac ccaaagaagt gaatattaag    540
   cctggaaata atgtacgtcc tgcaaaatca aaaaaactaa acaagttggt cgagaattcc    600
   ttgtccataa gtaatccagg gctcttcacc tccttaggac ctcctcttcg gtccacaact    660
   tgccatcgct gtggcctatt tggatcgctg aggtgctctc agtgcaagca gacctactat    720
   tgctccacag catgtcaaag aagagactgg tctgcacaca gcatcgtgtg caggcctgtt    780
   cagccaaatt tccacaaact tgaaaataaa tcatctattg aaacaaagga tgtggaggta    840
   aacaataaga gtgactgtcc acttggagtt actaaggaaa tagccatttg ggctgagaga    900
   ataatgtttt ctgatttgag aagtctacaa ctcaagaaaa ccatggaaat aaagggtacg    960
   gttaccgaat tcaaacaccc aggggacttc tacgtgcagt tatattcttc agaagtttta   1020
   gaatacatga accaactctc tgccagctta aaagaaacat atgcaaatgt gcatgaaaaa   1080
   gactatattc ctgttaaggg ggaagtttgt attgccaagt acactgttga tcagacctgg   1140
   aacagagcaa tcatacaaaa cgttgatgtg cagcaaaaga aggcacatgt cttatatatt   1200
   gattatggaa atgaagaaat aattccatta aacagaattt accacctcaa caggaacatt   1260
   gacttgtttc ctccttgtgc cataaagtgc tttgtagcca atgttatccc agcagaaggg   1320
   aattggagca gtgattgtat caaagctact aaaccactgt taatggagca gtactgctcc   1380
   ataaagattg tcgacatctt ggaagaggaa gtggttacct ttgctgtaga agttgagctg   1440
   ccaaattcag gaaaactttt agaccatgtg cttatagaaa tgggatatgg cttgaaaccc   1500
   agtggacaag attctaagaa ggaaaatgca gatcaaagtg atcctgaaga tgttggaaaa   1560
   atgacaactg aaaacaacat tgtcgtagac aaaagtgacc taatcccaaa agtgttaact   1620
   ttgaatgtag gtgatgagtt ttgtggtgtg gttgcccaca ttcaaacacc agaagacttc   1680
   ttttgtcaac aactgcaaag tggccgaaag cttgctgaac ttcaggcatc ccttagcaag   1740
   tactgtgatc agttgcctcc acgctctgat ttttatccag ccattggtga tatatgttgt   1800
   gctcagttct cagaggatga tcagtggtac cgtgcctctg ttttggctta cgcttctgaa   1860
   gaatctgtac tggtcggata tgtagattat ggaaactttg aaatccttag tttgatgaga   1920
   ctttgtccca taatcccaaa gttgttggaa ttgccaatgc aagctataaa gtgtgtacta   1980
   gcaggagtaa agccatcatt aggaatttgg actccagaag ctatttgtct catgaaaaaa   2040
   cttgtacaga acaaaataat cacagtgaaa gtggtggaca agttggaaaa cagttccctg   2100
   gtggagctta ttgataaatc cgagacgcct catgtcagtg ttagcaaagt tctcctagat   2160
   gcaggctttg ctgtgggaga acagagtatg gtgacagata aacccagtga cgtgaaagaa   2220
   accagtgttc ccttgggtgt ggaaggaaaa gtaaatccat tggagtggac atgggttgaa   2280
   cttggtgttg accaaacagt agatgttgtg gtctgtgtga tatatagtcc tggagaattt   2340
   tattgccatg tgcttaaaga ggatgcttta aagaaactca atgatttgaa caagtcatta   2400
   gcagaacact gccagcagaa gttacctaat ggtttcaagg cagagatagg acaaccttgt     2460
   tgtgcttttt ttgcaggtga tggtagttgg tatcgtgctt tagtcaagga aatcttacca     2520
   aatggacatg ttaaagtaca ttttgtggat tatggaaaca tcgaagaagt tactgcagat     2580
   gaactccgaa tgatatcatc aacattttta aaccttccct ttcagggaat acggtgccag     2640
   ttagcagata tacagtctag aaacaaacat tggtctgaag aagccataac aagattccag     2700
   atgtgtgttg ctgggataaa attgcaagcc agagtggttg aagtcactga aaatgggata     2760
   ggagttgaac tcaccgatct ctccacttgt tatcccagaa taattagtga tgttctgatt     2820
   gatgaacatc tggttttaaa atctgcttca ccacataaag acttaccaaa tgacagactt     2880
   gttaataaac atgagcttca agttcatgta cagggacttc aagctacctc ttcagctgag     2940
   caatggaaga cgatagaatt gccagtggat aaaactatac aagcaaatgt attagaaatc     3000
   ataagcccaa acttgtttta tgctctacca aaagggatgc cagaaaatca ggaaaagctg     3060
   tgcatgttga cagctgaatt attagaatac tgcaatgctc cgaaaagtcg accaccctat     3120
   agaccaagaa ttggagacgc atgctgtgcc aaatacacaa gtgatgattt ttggtatcgt     3180
   gcagttgttc tggggacatc agacactgat gtggaagtgc tctatgcaga ctatggaaac     3240
   attgaaaccc tgcctctttg cagagtgcaa ccaatcacct ctagccacct ggcgcttcct     3300
   ttccaaatta ttagatgttc acttgaagga ttaatggaat tgaatggaag ctcttctcaa     3360
   ttaataataa tgctattaaa aaatttcatg ttgaatcaga atgtaatgct ttctgtgaaa     3420
   ggaattacaa agaatgtcca tacagtgtca gttgagaaat gttctgagaa tgggactgtc     3480
   gatgtagctg ataagctagt gacatttggt ctggcaaaaa acatcacacc tcaaaggcag     3540
   agtgctttaa atacagaaaa gatgtatagg atgaattgct gctgcacaga gttacagaaa     3600
   caagttgaaa aacatgaaca tattcttctc ttcctcttaa acaattcaac caatcaaaat     3660
   aaatttattg aaatgaaaaa actgttaaaa aaaacagcat ctcttggagg taaaccctta     3720
   tgagacagga aacagcaaag gctagcttta ggagagaaag tacagcacct ggtgttttta     3780
   tttatgagaa ccttttcttt gtccactttc tctgtaatga ccttctatcc ctccgttttt     3840
   gcctgcctgc cattctccta ttaggttggt ggtttttatt ttcctctaag ttccttccac     3900
   caaataaata ttacgtaaaa aattcatacc aaatcaatga gaatactggc aaggaataca     3960
   tagggacttt ctgctatata tgtaactttt tattacttaa aggtaccgaa ggaaggccag     4020
   gtgcagtggc tcacgcccag cactttggga ggctgaggtg ggaggatccc ttgaggccag     4080
   gagttcaagg ttacagtgag ctatgatagt gccactgcac tccagcctgg gtgacagatt     4140
   ttgtcttaaa aaaaaaaaaa aaaaagttga tatgagtttt attttctgtc cgtttgaaat     4200
   attttgtaat attccctgca ttctctgtcg tctgcctctt ccacataatg tcctttgctt     4260
   tcatgtttgt tatcttcttt ttctgttcac tcagaggtca tcaatttctt tctctccgtc     4320
   cttaattgga ttatttttct tttggccttt gggcacagag tctgacctct ggaccactct     4380
   aactggagaa ggaactttat gttccctctc ctgctgtgtc cacaacctta gaaatctgta     4440
   gctagatttt tgttgttata gatagaattt actgtttctg aaacccaaat acagttatca     4500
   gtttaaggtt                                                            4510
<210> 50
   <211> 3300
   <212> DNA
   <213> Homo sapiens
<400> 50
   acgggagctc tttcccttct ctcctcctcc tcgcccttct cctcgccctc ctcctcctcc     60
   tcgccctcct cttcctcctc ctcctccttg ccctcctcct ctccctcctc cttctcctcc    120
   tccacctcct ctccctcctc ctcctcctcc tgcgctcacc gccggcagcc agcactttgc    180
   gctcacccag agagtagctc cacttgggtg cgagaccgag aggggcatat ccgttcacgc    240
   cgatccatga aaatgctttg gaaattgacg gataatatca agtacgagga ctgcgaggac    300
   cgtcacgacg gcaccagcaa cgggacggca cggttgcccc agctgggcac tgtaggtcaa    360
   tctccctaca cgagcgcccc gccgctgtcc cacaccccca atgccgactt ccagccccca    420
   tacttccccc caccctacca gcctatctac ccccagtcgc aagatcctta ctcccacgtc    480
   aacgacccct acagcctgaa ccccctgcac gcccagccgc agccgcagca cccaggctgg    540
   cccggccaga ggcagagcca ggagtctggg ctcctgcaca cgcaccgggg gctgcctcac    600
   cagctgtcgg gcctggatcc tcgcagggac tacaggcggc acgaggacct cctgcacggc    660
   ccacacgcgc tcagctcagg actcggagac ctctcgatcc actccttacc tcacgccatc    720
   gaggaggtcc cgcatgtaga agacccgggt attaacatcc cagatcaaac tgtaattaag    780
   aaaggccccg tgtccctgtc caagtccaac agcaatgccg tctccgccat ccctattaac    840
   aaggacaacc tcttcggcgg cgtggtgaac cccaacgaag tcttctgttc agttccgggt    900
   cgcctctcgc tcctcagctc cacctcgaag tacaaggtca cggtggcgga agtgcagcgg    960
   cggctctcac cacccgagtg tctcaacgcg tcgctgctgg gcggagtgct ccggagggcg   1020
   aagtctaaaa atggaggaag atctttaaga gaaaaactgg acaaaatagg attaaatctg   1080
   cctgcaggga gacgtaaagc tgccaacgtt accctgctca catcactagt agagggagaa   1140
   gctgtccacc tagccaggga ctttgggtac gtgtgcgaaa ccgaatttcc tgccaaagca   1200
   gtagctgaat ttctcaaccg acaacattcc gatcccaatg agcaagtgac aagaaaaaac   1260
   atgctcctgg ctacaaaaca gatatgcaaa gagttcaccg acctgctggc tcaggaccga   1320
   tctcccctgg ggaactcacg gcccaacccc atcctggagc ccggcatcca gagctgcttg   1380
   acccacttca acctcatctc ccacggcttc ggcagccccg cggtgtgtgc cgcggtcacg   1440
   gccctgcaga actatctcac cgaggccctc aaggccatgg acaaaatgta cctcagcaac   1500
   aaccccaaca gccacacgga caacaacgcc aaaagcagtg acaaagagga gaagcacaga   1560
   aagtgaggct ctcctcccgc cccgcccctc ccacgcctca ccagcccccc gcgcgcccac   1620
   cctccggcgg gtgacagctc cgggatcagc aacccttcct gctgctgcta ctgctgctgc   1680
   tgctgccgcc gccgccgccg ccgctgccct tgggtccccc cgagtctccg ggactgccct   1740
   ctcgactgtc agtggggcag cctctccgac tctgcacccg cctcgacctc cccacccgct   1800
   cccacacccc tgtgccctca tgtggagcct aagagaacag aacaggccgt gaagccagca   1860
   gagaaaagtt ctgccaagtt tgtgaaccct ttttttttta aacaaaacaa caaatcaaca   1920
   acagcaacaa caacaacaaa aattaaaaac ttttttctaa aaaaaaagtg aaaataaaaa   1980
   aaattatatg cgcttcatgg gactgagtca ccaccttccc ttacatactt cagttcagat   2040
   tgtagccata cttaaaaaaa aaaaaaaagc caaaagatga tgacaacatt tttatcagta   2100
   ttgtgaataa acttgaacac aaatacacga agttccatgt catgtcttca gttgtagaag   2160
   tttttcctct ttaaggtaaa gcgaccaact tgaactttct ctggcaacac gattcgcagt   2220
   tatataaggg aatcagtgtt cacgtctctg tatatattta tttatgtgta atttaatggg   2280
   aattgtaaat atggtgagtc tgttttaagc cttttttttt ttatttatct gatcttgttt   2340
   acctcttgtt tagtgggttt tgaatcttcc ctattagttc ttcatgtggt tcatggtact   2400
   gatttagaaa tccagtgttt gggggatttt tttctctggg attcatgaat ttagccctgt   2460
   tgtagcatgt taaaggtgac aaacagctgg acaaattttt aaaaagtaaa ataaaatttt   2520
   atctataatt agtattatta catttagctt ttcattgaac cgaaagaaaa aaagtgatat   2580
   tggaccctgg aaagattttg aaacttgagt ggtttgataa cccttctatg tattgtaggg    2640
   agaaaaaaaa aagtttattt tattccactg tcctccctta aaagcatcat ttgagcaata    2700
   aatgaatatt gtctttaaac caagggttag ggaattttcc tctctctctc tctctcctct   2760
   ctctttctgt tcaaagaact tcaaacattt gggaccacct ggtattctgt attttcactg   2820
   gccatattgg aagcagttct agttgcattg tattgagttg tgctggcagt agtttccatg   2880
   cctgtcaatg tatcatagtc ctttgttgcc cagataaata aatatttgat acgctttatg   2940
   tcgatttttt tttattcagt ggctgtcttt acccaggcgt atttttgttc ttggcagtat   3000
   tttttattca gtatggttac agtaattgag tttaactctc ccttggcaat tgctccttgc   3060
   aataagcagc tgaacccatt gtttccctca agtataataa aaacttactt tcaacttgga   3120
   gttcagagca gggtatcatt tagatattcc actgtgtctg tattcagaca aatgacacaa   3180
   taaaacccaa tgtattcttt tggataaaag attgtttgta ctgctaaagg aatgacatac   3240
   tgtcttttcc ttactagaaa cattaatttt attattaaaa ataaagtttt atttatttta   3300
<210> 51
   <211> 4872
   <212> DNA
   <213> Homo sapiens
<400> 51
   aacttgggac ctgagttagg ccttcatcgt cgcggactca gtagacagag gccataggtg      60
   cagctgtgat ggtagaggtg gaagactgat ttgcattttc accaggtgag acctggttac     120
   tcattggttg atgaggaaag agatgatagc cagggcggca taaaacggca atgagctcag     180
   taaacaaccc ccttcccgtg atcttcgtgg cttcccgccc agacgtgtga ccaagatagc     240
   aaggagcagt ctttaaggcg acacggcaga gaggcagaaa aagatgggcc ttaccggtta     300
   gttaagaaaa aaagaggacc tgttgcctgt ccctctagct ttgaactaca aagtggaggg     360
   gaagtttgtc tggattttcc tgtagaactg agggcaggac atgaggctct gcctgcagtc     420
   agcaacttgg aatattcaga cttcagacca gcatcacaga ttataaccct ccgtaaatca     480
   tctgcatccc agctcccatc aaaagccagc ctgaaggacc catggacacg tgactccagt     540
   gttctcaaca acatcttaag atcaagttgg tttgcacaac atttgcatct acttgggaca     600
   aagcaagaac aataagggca aaaaagtgaa aaaaaaaaaa gatccctgag taattgcaaa     660
   tgctgggaca gtttaccact ccagggtgaa gagtccatac caacatgtct gcctactaca     720
   ggaataactg gtctgaggaa gacccagatt accctgacta ttcagggtct cagaaccgta     780
   cgcaggggta tttgaaaact caaggttatc cagatgttcc aggtcctctg aacaatccag     840
   actaccccgg caccaggagc aatccatact ctgtagcctc cagaacacgt ccagactatc     900
   ctgggtctct ggcagaacca aattatccta gatctctgag taatccagac tattctggca     960
   ccagaagcaa tgcatactct gcagcctcta gaacaagccc agaccatcct acctctctac    1020
   cagagccaga ttatagtgaa tttcagagtc atccctacca ccgagcatca tccagacaac    1080
   cagactaccc tggatctcaa cgaaatcctg attttgcagg ctccagcagc agtggaaact    1140
   atgcaggctc cagaacacat ccagatcatt ttggctcctt agaaccggac taccctggag    1200
   ctcagagcaa ctctgatcat cctggaccca gagccaattt gaaccatcca ggatccagaa    1260
   agaatctgga acatacaagt tttagaatca atccatacgc agactctctg ggaaagcctg    1320
   attatccagg cgctgacatt caacctaact ctccaccctt ttttggggag ccagactatc    1380
   ccagtgctga ggacaatcag aacttgccaa gcacttggag agaacctgat tattcagatg    1440
   ctgagaatgg tcatgattat ggctcttctg agaccccaaa gatgaccagg ggggtgctca    1500
   gcagaacatc ttcaatccag ccctcatttc gtcacaggag tgatgacccc gtgggcagtc    1560
   tttggggaga gaatgattac cctgaaggca ttgaaatggc atccatggag atggcaaact    1620
   catatggcca ctctctgcca ggtgctcctg gaagtggcta tgtgaaccct gcttatgtag    1680
   gtgaaagtgg tcctgtccat gcttatggaa acccaccatt gtctgaatgt gattggcaca    1740
   agtcacccca aggacagaag ttaatcgcat cccttatacc catgacatcc agagacagaa    1800
   ttaaagccat caggaaccag ccaaggacca tggaagagaa aaggaacctt aggaaaatag    1860
   ttgacaaaga aaaaagcaaa cagacccatc gtatccttca gctcaattgc tgtattcagt 1920
   gtctgaactc catttcccgg gcttatcgga gatccaagaa cagcctgtcg gaaattctga 1980
   attccatcag cctgtggcag aagacgctga agatcattgg aggcaagttt ggaaccagcg 2040
   tcctctccta tttcaacttt ctgagatggc ttttgaagtt caacattttc tcattcatcc 2100
   tgaacttcag cttcatcata atccctcagt ttaccgtggc caaaaagaac accctccagt 2160
   tcactgggct ggagtttttc actggggtgg gttattttag ggacacagtg atgtactatg 2220
   gcttttacac caattccacc atccagcacg ggaacagcgg ggcatcctac aacatgcagc 2280
   tggcctacat cttcacaatc ggagcatgct tgaccacctg cttcttcagt ttgctgttca 2340
   gcatggccaa gtatttccgg aacaacttca ttaatcccca catttactcc ggagggatca 2400
   ccaagctgat cttttgctgg gacttcactg tcactcatga aaaagctgtg aagctaaaac 2460
   agaagaatct tagcactgag ataagggaga acctgtcaga gctccgtcag gagaattcca 2520
   agttgacgtt caatcagctg ctgacccgct tctctgccta catggtagcc tgggttgtct 2580
   ctacaggagt ggccatagcc tgctgtgcag ccgtttatta cctggctgag tacaacttag 2640
   agttcctgaa gacacacagt aaccctgggg cggtgctgtt actgcctttc gttgtgtcct 2700
   gcattaatct ggccgtgcca tgcatctact ccatgttcag gcttgtggag aggtacgaga 2760
   tgccacggca cgaagtctac gttctcctga tccgaaacat ctttttgaaa atatcaatca 2820
   ttggcattct ttgttactat tggctcaaca ccgtggccct gtctggtgaa gagtgttggg 2880
   aaaccctcat tggccaggac atctaccggc tccttctgat ggattttgtg ttctctttag 2940
   tcaattcctt cctgggggag tttctgagga gaatcattgg gatgcaactg atcacaagtc 3000
   ttggccttca ggagtttgac attgccagga acgttctaga actgatctat gcacaaactc 3060
   tggtgtggat tggcatcttc ttctgccccc tgctgccctt tatccaaatg attatgcttt 3120
   tcatcatgtt ctactccaaa aatatcagcc tgatgatgaa tttccagcct ccgagcaaag 3180
   cctggcgggc ctcacagatg atgactttct tcatcttctt gctctttttc ccatccttca 3240
   ccggggtctt gtgcaccctg gccatcacca tctggagatt gaagccttca gctgactgtg 3300
   gcccttttcg aggtctgcct ctcttcattc actccatcta cagctggatc gacaccctaa 3360
   gtacacggcc tggctacctg tgggttgttt ggatctatcg gaacctcatt ggaagtgtgc 3420
   acttcttttt catcctcacc ctcattgtgc taatcatcac ctatctttac tggcagatca 3480
   cagagggaag gaagattatg ataaggctgc tccatgagca gatcattaat gagggcaaag 3540
   ataaaatgtt cctgatagaa aaattgatca agctgcagga tatggagaag aaagcaaacc 3600
   ccagctcact tgttctggaa aggagagagg tggagcaaca aggctttttg catttggggg 3660
   aacatgatgg cagtcttgac ttgcgatcta gaagatcagt tcaagaaggt aatccaaggg 3720
   cctgatgact cttttggtaa ccagacacca atcaaataag gggaggagac gaaaatggaa 3780
   tgatttcttc catgccacct gtgcctttag gaactgccca gaagaaaatc caaggcttta 3840
   gccaggagcg gaaactgact accatgtaat tatcaaagta aaattgggca ttccatgcta 3900
   tttttaatac ctggattgct gatttttcaa gacaaaatac ttggggtttt ccaataaaga 3960
   ttgttgtaat attgaaatga gcctacaaaa acctaggaag agataactag ggaataatgt 4020
   atattatctt caagaagtgt gtgcaggaat gattggttct tagaaatctc tcctgccaga 4080
   cttcccagac ctggcaaagg tttagaaact gttgctaaga aaagtggtcc atcctgaata 4140
   aacatgtaat actccagcag ggatatgaag cctctgaatt gtagaacctg catttatttg 4200
   tgactttgaa ctaaagacat cccccatgtc ccaaaggtgg aatacaacca gaggtctcat 4260
   ctctgaactt tcttgcgtac tgattacatg agtctttgga gtcggggatg gaggaggttc 4320
   tgcccctgtg aggtgttata catgaccatc aaagtcctac gtcaagctag ctttgcagtg 4380
   gcagtaccgt agccaatgag atttatccga gacgcgatta ttgctaattg gaaattttcc 4440
   caatacccca ccgtgatgac ttgaaatata atcagcgctg gcaatttttg acagtctcta 4500
   cggagactga ataagaaaaa agaaaagaaa agaaattagc tgggtgcgat ggcttatgcc 4560
   tgtaatcccg gcactttggg aggctgaggc aagcggatca cttaatgtca ggagttcaag 4620
   accagcctgg ccaacatggt gaaaccccgt ctctactaaa aataaaaaaa ctagctgggc 4680
   gtggtggtac atgcctataa tcccagctac tcgggaggct gaggcaggag aattgcttga 4740
   acctgggagg cagaggttgc agtgaggcga gattgtacca ctgcattcca gcctgggcaa 4800
   cagtgagact ctgcctcaaa aaaataaata aataaataaa taaagtaaat taaaagtctt 4860
   attcaaacca aa                                                    4872
<210> 52
   <211> 3685
   <212> DNA
   <213> Homo sapiens
<400> 52
   agtggactca cgcaggcgca ggagactaca cttcccagga actccgggcc gcgttgttcg     60
   ctggtacctc cttctgactt ccggtattgc tgcggtctgt agggccaatc gggagcctgg    120
   aattgctttc ccggcgctct gattggtgca ttcgactagg ctgcctgggt tcaaaatttc    180
   aacgatactg aatgagtccc gcggcgggtt ggctcgcgct tcgttgtcag atctgaggcg    240
   aggctaggtg agccgtggga agaaaagagg gagcagctag ggcgcgggtc tccctcctcc    300
   cggagtttgg aacggctgaa gttcaccttc cagcccctag cgccgttcgc gccgctaggc    360
   ctggcttctg aggcggttgc ggtgctcggt cgccgcctag gcggggcagg gtgcgagcag    420
   gggcttcggg ccacgcttct cttggcgaca ggattttgct gtgaagtccg tccgggaaac    480
   ggaggaaaaa aagagttgcg ggaggctgtc ggctaataac ggttcttgat acatatttgc    540
   cagacttcaa gatttcagaa aaggggtgaa agagaagatt gcaactttga gtcagacctg    600
   taggcctgat agactgatta aaccacagaa ggtgacctgc tgagaaaagt ggtacaaata    660
   ctgggaaaaa cctgctcttc tgcgttaagt gggagacaat gtcacaagtt aaaagctctt    720
   attcctatga tgccccctcg gatttcatca atttttcatc cttggatgat gaaggagata    780
   ctcaaaacat agattcatgg tttgaggaga aggccaattt ggagaataag ttactgggga    840
   agaatggaac tggagggctt tttcagggca aaactccttt gagaaaggct aatcttcagc    900
   aagctattgt cacacctttg aaaccagttg acaacactta ctacaaagag gcagaaaaag    960
   aaaatcttgt ggaacaatcc attccgtcaa atgcttgttc ttccctggaa gttgaggcag   1020
   ccatatcaag aaaaactcca gcccagcctc agagaagatc tcttaggctt tctgctcaga   1080
   aggatttgga acagaaagaa aagcatcatg taaaaatgaa agccaagaga tgtgccactc   1140
   ctgtaatcat cgatgaaatt ctaccctcta agaaaatgaa agtttctaac aacaaaaaga   1200
   agccagagga agaaggcagt gctcatcaag atactgctga aaagaatgca tcttccccag   1260
   agaaagccaa gggtagacat actgtgcctt gtatgccacc tgcaaagcag aagtttctaa   1320
   aaagtactga ggagcaagag ctggagaaga gtatgaaaat gcagcaagag gtggtggaga   1380
   tgcggaaaaa gaatgaagaa ttcaagaaac ttgctctggc tggaatagag caacctgtga   1440
   agaaatcagt gagccaggtc accaaatcag ttgacttcca cttccgcaca gatgagcgaa   1500
   tcaaacaaca tcctaagaac caggaggaat ataaggaagt gaactttaca tctgaactac   1560
   gaaagcatcc ttcatctcct gcccgagtga ctaagggatg taccattgtt aagcctttca   1620
   acctgtccca aggaaagaaa agaacatttg atgaaacagt ttctacatat gtgccccttg   1680
   cacagcaagt tgaagacttc cataaacgaa cccctaacag atatcatttg aggagcaaga   1740
   aggatgatat taacctgtta ccctccaaat cttctgtgac caagatttgc agagacccac   1800
   agactcctgt actgcaaacc aaacaccgtg cacgggctgt gacctgcaaa agtacagcag   1860
   agctggaggc tgaggagctc gagaaattgc aacaatacaa attcaaagca cgtgaacttg   1920
   atcccagaat acttgaaggt gggcccatct tgcccaagaa accacctgtg aaaccaccca   1980
   ccgagcctat tggctttgat ttggaaattg agaaaagaat ccaggagcga gaatcaaaga   2040
   agaaaacaga ggatgaacac tttgaatttc attccagacc ttgccctact aagattttgg   2100
   aagatgttgt gggtgttcct gaaaagaagg tacttccaat caccgtcccc aagtcaccag   2160
   cctttgcatt gaagaacaga attcgaatgc ccaccaaaga agatgaggaa gaggacgaac   2220
   cggtagtgat aaaagctcaa cctgtgccac attatggggt gccttttaag ccccaaatcc   2280
   cagaggcaag aactgtggaa atatgccctt tctcgtttga ttctcgagac aaagaacgtc   2340
   agttacagaa ggagaagaaa ataaaagaac tgcagaaagg ggaggtgccc aagttcaagg   2400
   cacttccctt gcctcatttt gacaccatta acctgccaga gaagaaggta aagaatgtga   2460
   cccagattga acctttctgc ttggagactg acagaagagg tgctctgaag gcacagactt   2520
   ggaagcacca gctggaagaa gaactgagac agcagaaaga agcagcttgt ttcaaggctc   2580
   gtccaaacac cgtcatctct caggagccct ttgttcccaa gaaagagaag aaatcagttg   2640
   ctgagggcct ttctggttct ctagttcagg aaccttttca gctggctact gagaagagag   2700
   ccaaagagcg gcaggagctg gagaagagaa tggctgaggt agaagcccag aaagcccagc   2760
   agttggagga ggccagacta caggaggaag agcagaaaaa agaggagctg gccaggctac   2820
   ggagagaact ggtgcataag gcaaatccaa tacgcaagta ccagggtctg gagataaagt   2880
   caagtgacca gcctctgact gtgcctgtat ctcccaaatt ctccactcga ttccactgct   2940
   aaactcagct gtgagctgcg gataccgccc ggcaatggga cctgctctta acctcaaacc   3000
   taggaccgtc ttgctttgtc attgggcatg gagagaaccc atttctccag acttttacct   3060
   acccgtgcct gagaaagcat acttgacaac tgtggactcc agttttgttg agaattgttt   3120
   tcttacatta ctaaggctaa taatgagatg taactcatga atgtctcgat tagactccat   3180
   gtagttactt cctttaaacc atcagccggc cttttatatg ggtcttcact ctgactagaa   3240
   tttagtctct gtgtcagcac agtgtaatct ctattgctat tgccccttac gactctcacc   3300
   ctctccccac tttttttaaa aattttaacc agaaaataaa gatagttaaa tcctaagata   3360
   gagattaagt catggtttaa atgaggaaca atcagtaaat cagattctgt cctcttctct   3420
   gcataccgtg aatttatagt taaggatccc tttgctgtga gggtagaaaa cctcaccaac   3480
   tgcaccagtg aggaagaaga ctgcgtggat tcatggggag cctcacagca gccacgcagc   3540
   aggctctggg tggggctgcc gttaaggcac gttctttcct tactggtgct gataacaaca   3600
   gggaaccgtg cagtgtgcat tttaagacct ggcctggaat aaatacgttt tgtctttccc   3660
   tcaaaaaaaa aaaaaaaaaa aaaaa                                         3685
<210> 53
   <211> 3658
   <212> DNA
   <213> Homo sapiens
<400> 53
   aaggaggagt gcactggccg ggatcggtgc agcgctcaca ctcactcaca gtcactctct      60
   ctgagcgcgt ctcgctcgct ctcatacacg cccggagccc aggagcgctc aggatcccga     120
   gcgccgcgaa aaagttcccc cggcttttgc tggagactca tcgttttggg aagtgcattt     180
   gcttcgtggc tccgccgagc ctgctgaatc ctgtcctcgc ggcacgggac cccgggatcg     240
   ctgaccgctg ccgccgccgc ctctgcctcc cggactatcg gcagcctcgg caacaatagt     300
   ggcggccgcc cccagcgagg ctccgggagc ccttgcctgc gggggtccgg ggactcgagc     360
   cggcctccgc ctcccggacg cacagccagc gtggtccccg cgtgcaacgc gagcgccggg     420
   gagtggctcc tgctttgccc ctcgtggggg ccgagccaag accagtctgc aaactccatc     480
   ccgccggctg gaagaagtcg cggagccggc accaaacccg cagcgtcttc ccgcgcggat     540
   cccgggactt aaaaagccgg ggccaccccg gcccaggacg ggatgcgggt cggtccggtg 600
   cgctctgcca tgagcggcgc ctcgcagccc cgcggcccgg ccctgctctt cccagccacc 660
   cgaggcgtcc cggccaaacg cctgctggac gccgacgacg cggcggctgt ggcggccaag 720
   tgcccgcgcc tctccgagtg ctccagcccc ccggactacc tcagcccccc cggctcgccc 780
   tgcagcccgc agcccccgcc tgccgctccg ggggccggcg gaggctccgg gagcgcgccg 840
   gggcccagcc gcatcgccga ctacctgctg ctgcccctag ccgagcgcga gcatgtgtcc 900
   cgggcgctgt gcatccacac tggacgcgag ctgcgctgca aggtgtttcc cattaaacac 960
   taccaggaca aaatcaggcc ttacatccag ctgccatcgc acagcaacat tactggcatt 1020
   gtggaagtga tccttgggga aaccaaggcc tatgtcttct ttgagaagga ctttggggac 1080
   atgcactcct atgtgcgaag ccggaagagg ctgcgggaag aggaagccgc ccggctcttc 1140
   aagcagattg tctccgccgt cgcccactgc caccagtcag ccatcgtgct gggggacctg 1200
   aagcttagga agttcgtctt ctccacggag gagagaaccc agcttagact agaaagtcta 1260
   gaagacacac acataatgaa gggggaagat gatgctttgt cagacaaaca tggctgccca 1320
   gcctacgtga gccctgagat cctcaacacc actgggacct actccggaaa ggctgcggac 1380
   gtttggagcc tgggggtgat gctctacacc cttctggttg gacgataccc cttccatgac 1440
   tcagacccca gtgccctttt ctccaaaatt cggcgtggac agttctgcat tcctgagcac 1500
   atttccccca aagccaggtg cctcattcgc agcctcttga gacgggagcc ctccgagaga 1560
   ctcactgccc ccgagatcct actgcacccc tggtttgagt ccgtcttgga acccgggtac 1620
   atcgactcag aaataggaac ttcagaccag attgttccag agtaccagga ggacagtgac 1680
   attagttcct tcttctgcta atccccaaaa cctcagaaac ctcataattc ttaacacctg 1740
   gcatttccat ttctaaagat ggacaggccc tttggcgtgg taccaaccag ataatgactg 1800
   catcaggatg aaagctgctg aactcggcat ggcgcctcct cttctctgtt gggatgagtg 1860
   actttattga tttgagcagc atatgctgtg attggctgcc ctgcaaattt gtttccctta 1920
   aggaaccctc accaactatc tctgctggat ttgggagttc cgcatctttt gtggagggca 1980
   gagtatggac atcttacacc cggtggtcaa gtgtgtaata aacttgagca ttcgaatggg 2040
   agaaaaagca aatcgcacaa tgacatattt tgagtaataa ccgtattttt cacagggtga 2100
   caaattgggc caataaatct gccatctttg aactcatctt tggtggctag actgctacgg 2160
   cagcttctct gatgggaaag ttcctttttt ggcttaacac tcaccctttc ttcacactca 2220
   catttaccaa tgactctgct ccgtttttgg agcagactgt tttaagttgc tcaggagcct 2280
   gatggaacca tgaaccgaga ctcttctctg tttcctgcca agacctcatc tgcactaatg 2340
   ccttctccct gaccttgaca cttccccctt tagctataaa agcacttacc agccgaacgt 2400
   ggaacagtat cacaaaagat tccatctccc aacgatttca gaactctgag ctcagagaga 2460
   ctccagattt taaaaaataa tttgagtgct tggaaactat tagcttttta agttccttcc 2520
   aaatatgtta gtacctaccc tttacttttt ccccaagacc atctcagggt ggagcattct 2580
   gtctaagaga agaaagataa ggaggctccc acccacctct cccaagagca gacattaaac 2640
   atctttgtgc tttgaagaga gtgaattttg gatagtcttg tgattctcag actaacttcc 2700
   agaattatac tttaacccct cccagatatg gtccgccttt ggcattgtgt gtacatctgc 2760
   agttttgcat ggtgggttgt taatatttca aatgtgtggt ttatgaatac gtctgtataa 2820
   tcggcttctg gagtgaaaca gcaaacccca aatcttcaaa gttggaagga actttaaaaa 2880
   tcatccggtc caatctcttt cctctttctg ccacctccca aggcagaaat cccctcttca 2940
   gcttcttttg taggtgggaa tccagcctct gttagatatg tccagagatg gaaactcact 3000
   cccctacaaa agatggagct taatggagaa attgcaactt tcattaaaaa acaaattcag 3060
   atgaaatatc agtaactgtc ttggacagtg ctgaaatcag gtggttaaac gggtaaacaa 3120
   aatatactgt attttgagaa atggcacaaa aacaggcagt catctttaag ggctatgcct 3180
   aggcaaacta ctaacatgca ttgtgagaat gccgtgtata cctcacgtac tgtgtacttt 3240
   gtacatatat tttacctttt atacctatgt tcgattttgt tttgttttgt tttgttctgg 3300
   ctttgaggct tgttttgttg tctgtgtctg tctgaataac ctgcgtgtct aaaaccacgt 3360
   gaaatgtgaa tgattattgg caatattacc ttgacagaat catgggactt tgagaagagg 3420
   gaggacagag gcctctgtcg cactaacgct ctcgtggttg ctcgactgtt gtatctgtga 3480
   tacattatcc gactaaggac tctgggctgg cagggccttc tgccgggaaa gctagaaaca 3540
   ctaggttctt cctgtacata cgtgtatata tgtgaacagt gagatggccg tttctgactt 3600
   gtagagaaat tttaataaac ctggtttcgt aaaaaaaaaa aaaaaaaaaa aaaaaaaa 3658
<210> 54
   <211> 1912
   <212> DNA
   <213> Homo sapiens
<400> 54
   aggggctggg cggggctcgg gctcctgctc cggctcagct gcggcggccg caggttccaa      60
   agcgggtccg agccgccgcc gcgcgcgcgc cgcgcactgc agccccaggc cccggccccc     120
   cacccacgtc tgcgttgctg ccccgcctgg gccaggcccc aaaggcaagg acaaagcagc     180
   tgtcagggaa cctccgccgg agtcgaattt acgtgcagct gccggcaacc acaggttcca     240
   agatggtttg cgggggcttc gcgtgttcca agaactgcct gtgcgccctc aacctgcttt     300
   acaccttggt tagtctgctg ctaattggaa ttgctgcgtg gggcattggc ttcgggctga     360
   tttccagtct ccgagtggtc ggcgtggtca ttgcagtggg catcttcttg ttcctgattg     420
   ctttagtggg tctgattgga gctgtaaaac atcatcaggt gttgctattt ttttatatga     480
   ttattctgtt acttgtattt attgttcagt tttctgtatc ttgcgcttgt ttagccctga     540
   accaggagca acagggtcag cttctggagg ttggttggaa caatacggca agtgctcgaa     600
   atgacatcca gagaaatcta aactgctgtg ggttccgaag tgttaaccca aatgacacct     660
   gtctggctag ctgtgttaaa agtgaccact cgtgctcgcc atgtgctcca atcataggag     720
   aatatgctgg agaggttttg agatttgttg gtggcattgg cctgttcttc agttttacag     780
   agatcctggg tgtttggctg acctacagat acaggaacca gaaagacccc cgcgcgaatc     840
   ctagtgcatt cctttgatga gaaaacaagg aagatttcct ttcgtattat gatcttgttc     900
   actttctgta attttctgtt aagctccatt tgccagttta aggaaggaaa cactatctgg     960
   aaaagtacct tattgatagt ggaattatat atttttactc tatgtttctc tacatgtttt    1020
   tttctttccg ttgctgaaaa atatttgaaa cttgtggtct ctgaagctcg gtggcacctg    1080
   gaatttactg tattcattgt cgggcactgt ccactgtggc ctttcttagc atttttacct    1140
   gcagaaaaac tttgtatggt accactgtgt tggttatatg gtgaatctga acgtacatct    1200
   cactggtata attatatgta gcactgtgct gtgtagatag ttcctactgg aaaaagagtg    1260
   gaaatttatt aaaatcagaa agtatgagat cctgttatgt taagggaaat ccaaattccc    1320
   aatttttttt ggtcttttta ggaaagattg ttgtggtaaa aagtgttagt ataaaaatga    1380
   taatttactt gtagtctttt atgattacac caatgtattc tagaaatagt tatgtcttag    1440
   gaaattgtgg tttaattttt gacttttaca ggtaagtgca aaggagaagt ggtttcatga    1500
   aatgttctaa tgtataataa catttacctt cagcctccat cagaatggaa cgagttttga    1560
   gtaatcagga agtatatcta tatgatcttg atattgtttt ataataattt gaagtctaaa    1620
   agactgcatt tttaaacaag ttagtattaa tgcgttggcc cacgtagcaa aaagatattt    1680
   gattatctta aaaattgtta aataccgttt tcatgaaatt tctcagtatt gtaacagcaa    1740
   cttgtcaaac ctaagcatat ttgaatatga tctcccataa tttgaaattg aaatcgtatt    1800
   gtgtggctct gtatattctg ttaaaaaatt aaaggacaga aacctttctt tgtgtatgca    1860
   tgtttgaatt aaaagaaagt aatggaagaa ttgatcgatg aaaaaaaaaa aa            1912
<210> 55
   <211> 2264
   <212> DNA
   <213> Homo sapiens
<400> 55
   ggtctccttg gcatgcacct attcagactg ttagtattat gtatttactt caaattttag      60
   cagttatatt ttaacttgat tgatttttcc tcagatataa gtatgagaaa tgacagaaag     120
   aaacaacaac tggaaaagaa gcattgcata agaccaggat gtctctgaaa tggacgtcag     180
   tctttctgct gatacagctc agttgttact ttagctctgg aagctgtgga aaggtgctag     240
   tgtggcccac agaatacagc cattggataa atatgaagac aatcctggaa gagcttgttc     300
   agaggggtca tgaggtgact gtgttgacat cttcggcttc tactcttgtc aatgccagta     360
   aatcatctgc tattaaatta gaagtttatc ctacatcttt aactaaaaat tatttggaag     420
   attctcttct gaaaattctc gatagatgga tatatggtgt ttcaaaaaat acattttggt     480
   catatttttc acaattacaa gaattgtgtt gggaatatta tgactacagt aacaagctct     540
   gtaaagatgc agttttgaat aagaaactta tgatgaaact acaagagtca aagtttgatg     600
   tcattctggc agatgccctt aatccctgtg gtgagctact ggctgaacta tttaacatac     660
   cctttctgta cagtcttcga ttctctgttg gctacacatt tgagaagaat ggtggaggat     720
   ttctgttccc tccttcctat gtacctgttg ttatgtcaga attaagtgat caaatgattt     780
   tcatggagag gataaaaaat atgatacata tgctttattt tgacttttgg tttcaaattt     840
   atgatctgaa gaagtgggac cagttttata gtgaagttct aggaagaccc actacattat     900
   ttgagacaat ggggaaagct gaaatgtggc tcattcgaac ctattgggat tttgaatttc     960
   ctcgcccatt cttaccaaat gttgattttg ttggaggact tcactgtaaa ccagccaaac    1020
   ccctgcctaa ggaaatggaa gagtttgtgc agagctctgg agaaaatggt attgtggtgt    1080
   tttctctggg gtcgatgatc agtaacatgt cagaagaaag tgccaacatg attgcatcag    1140
   cccttgccca gatcccacaa aaggttctat ggagatttga tggcaagaag ccaaatactt    1200
   taggttccaa tactcgactg tacaagtggt taccccagaa tgaccttctt ggtcatccca    1260
   aaaccaaagc ttttataact catggtggaa ccaatggcat ctatgaggcg atctaccatg    1320
   ggatccctat ggtgggcatt cccttgtttg cggatcaaca tgataacatt gctcacatga    1380
   aagccaaggg agcagccctc agtgtggaca tcaggaccat gtcaagtaga gatttgctca    1440
   atgcattgaa gtcagtcatt aatgaccctg tctataaaga gaatgtcatg aaattatcaa    1500
   gaattcatca tgaccaacca atgaagcccc tggatcgagc agtcttctgg attgagtttg    1560
   tcatgcgcca caaaggagcc aagcaccttc gagtcgcagc tcacaacctc acctggatcc    1620
   agtaccactc tttggatgtg atagcattcc tgctggcctg cgtggcaact gtgatattta    1680
   tcatcacaaa attttgcctg ttttgtttcc gaaagcttgc caaaaaagga aagaagaaga    1740
   aaagagatta gttatatcaa aagcctgaag tggaatgact gaaagatggg actcctcctt    1800
   tatttcagca tggagggttt taaatggagg atttcctttt tcctgtgaca aaacatcttt    1860
   tcacaactta ccttgttaag acaaaattta ttttccaggg atttaatacg tactttagct    1920
   gaattattct atgtcaatga tttttaagct atgaaaaata caatgggggg aaggatagca    1980
   tttggagata tacctaatgt taaatgacga gttactggat gcagcacgcc aacatggcac    2040
   atgtatacat atgtagctaa cctgcacgtt gtgcacatgt accctaaaac ttaaagtata    2100
   atttaaaaaa agcaaaaaaa aaaaatacaa ctcttttttt taaaccagga aggaaaatgt    2160
   gaacatggaa acaacttcta gtattggatc tgaaaataaa gtgtcatcca agccataaaa    2220
   aaaaaagaaa agaaaaataa aaataatata aaaccttaaa aaaa                    2264
<210> 56
   <211> 3223
   <212> DNA
   <213> Homo sapiens
<400> 56
   tcaatccctt aattaaatag cttcccctct acaggctttt gaagtggtag cagttcctcc      60
   taactcctgc cagaaacagc tctcctcaac atgagagctg cacccctcct cctggccagg     120
   gcagcaagcc ttagccttgg cttcttgttt ctgctttttt tctggctaga ccgaagtgta     180
   ctagccaagg agttgaagtt tgtgactttg gtgtttcggc atggagaccg aagtcccatt     240
   gacacctttc ccactgaccc cataaaggaa tcctcatggc cacaaggatt tggccaactc     300
   acccagctgg gcatggagca gcattatgaa cttggagagt atataagaaa gagatataga     360
   aaattcttga atgagtccta taaacatgaa caggtttata ttcgaagcac agacgttgac     420
   cggactttga tgagtgctat gacaaacctg gcagccctgt ttcccccaga aggtgtcagc     480
   atctggaatc ctatcctact ctggcagccc atcccggtgc acacagttcc tctttctgaa     540
   gatcagttgc tatacctgcc tttcaggaac tgccctcgtt ttcaagaact tgagagtgag     600
   actttgaaat cagaggaatt ccagaagagg ctgcaccctt ataaggattt tatagctacc     660
   ttgggaaaac tttcaggatt acatggccag gacctttttg gaatttggag taaagtctac      720
   gaccctttat attgtgagag tgttcacaat ttcactttac cctcctgggc cactgaggac     780
   accatgacta agttgagaga attgtcagaa ttgtccctcc tgtccctcta tggaattcac     840
   aagcagaaag agaaatctag gctccaaggg ggtgtcctgg tcaatgaaat cctcaatcac     900
   atgaagagag caactcagat accaagctac aaaaaactca tcatgtattc tgcgcatgac     960
   actactgtga gtggcctaca gatggcgcta gatgtttaca acggactcct tcctccctat    1020
   gcttcttgcc acttgacgga attgtacttt gagaaggggg agtactttgt ggagatgtac    1080
   tatcggaatg agacgcagca cgagccgtat cccctcatgc tacctggctg cagccccagc    1140
   tgtcctctgg agaggtttgc tgagctggtt ggccctgtga tccctcaaga ctggtccacg    1200
   gagtgtatga ccacaaacag ccatcaaggt actgaagaca gtacagatta gtgtgcacag    1260
   agatctctgt agaaggagta gctgcccttt ctcagggcag atgatgcttt gagaacatac    1320
   tttggccatt acccccagct ttgaggaaaa tgggctttgg atgattattt tatgttttag    1380
   ggacccccaa cctcaggcaa ttcctacctc ttcacctgac cctgccccca cttgccataa    1440
   aacttagcta agttttgttt tgtttttcag cgttaatgta aaggggcagc agtgccaaaa    1500
   tataatcaga gataaagctt aggtcaaagt tcatagagtt cccatgaact atatgactgg    1560
   ccacacagga tcttttgtat ttaaggattc tgagattttg cttgagcagg attagataag    1620
   gctgttcttt aaatgtctga aatggaacag atttcaaaaa aaaaccccac aatctaggat    1680
   gggaacaagg aaggaaagat gtgaataggc tgatgggcaa aaaaccaatt tacccatcag    1740
   ttccagcctt ctctcaagga gaggcaaaga aaggagatac agtggagaca tctggaaagt    1800
   tttctccact ggaaaactgc tactatctgt ttttatattt ctgttaaaat atatgaggct    1860
   acagaactaa aaattaaaac ctctttgtgt cccttggtcc tggaacattt atgttccttt    1920
   taaagaaaca aaaatcaaac tttacagaaa gatttgatgt atgtaataca tatagcagct    1980
   cttgaagtat atatatcata gcaaataagt catctgatga gaacaagcta tttgggcaca    2040
   acacatcagg aaagagagca ccacgtgatg gagtttctct agaagctcca gtgataagag    2100
   atgttgactc taaagttgat ttaaggccag gcatggtggt ttacgcctat aatcccagca    2160
   ttttgggagt ccgaggtggg cagatcactt gagctcagga ggtcaagatc agcctgggca    2220
   acatggtgaa acctggtctc tacataaaat acaaaaactt agatgggcat ggtggtgtgt    2280
   gcctatagtc ccactacttg tggggctaag gcaggaggat cacttgagcc ccggaggtcg    2340
   aggctacagt gagccaagag tgcactactg tactccagcc agggcaagag agcgagaccc    2400
   tgtctcaata aataaataaa taaataaata aataaataaa taaataaata aaaacaaagt    2460
   tgattaagaa aggaagtata ggccaggcac agtggctcac acctgtaatc cttgcatttt    2520
   ggaaggctga ggcaggagga tcactttagg cctggtgtgt tcaagaccag cctggtcaac    2580
   atagtgagac actgtctcta ccaaaaaaag gaaggaaggg acacatatca aactgaaaca    2640
   aaattagaaa tgtaattatg ttctaagtgc ctccaagttc aaaacttatt ggaatgttga    2700
   gagtgtggtt acgaaatacg ttaggaggac aaaaggaatg tgtaagtctt taatgccgat    2760
   atcttcagaa aacctaagca aacttacagg tcctgctgaa actgcccact ctgcaagaag    2820
   aaatcatgat atagctttgc catgtggcag atctacatgt ctagagaaca ctgtgctcta    2880
   ttaccattat ggataaagat gagatggttt ctagagatgg tttctactgg ctgccagaat    2940
   ctagagcaaa gccatccccg ctcctggttg gtcacagaat gactgacaaa gacatcgatt    3000
   gatatgcttc tttgtgttat ttccctccca agtaaatgtt tgtccttggg tccattttct    3060
   atgcttgtaa ctgtcttcta gcagtgagcc aaatgtaaaa tagtgaataa agtcattatt    3120
   aggaagttca aaagcattgc ttttataatg aacttagaaa aacgtatgtg tgtgtgttta    3180
   attagaataa aattcctcta ggcagatttc aggagctcca aaa                       3223
<210> 57
   <211> 1278
   <212> DNA
   <213> Homo sapiens
<400> 57
   ccattggcct gtagattcac ctcccctggg cagggcccca ggacccagga taatatctgt      60
   gcctcctgcc cagaaccctc caagcagaca caatggtaag aatggtgcct gtcctgctgt     120
   ctctgctgct gcttctgggt cctgctgtcc cccaggagaa ccaagatggt cgttactctc     180
   tgacctatat ctacactggg ctgtccaagc atgttgaaga cgtccccgcg tttcaggccc     240
   ttggctcact caatgacctc cagttcttta gatacaacag taaagacagg aagtctcagc     300
   ccatgggact ctggagacag gtggaaggaa tggaggattg gaagcaggac agccaacttc     360
   agaaggccag ggaggacatc tttatggaga ccctgaaaga catcgtggag tattacaacg     420
   acagtaacgg gtctcacgta ttgcagggaa ggtttggttg tgagatcgag aataacagaa     480
   gcagcggagc attctggaaa tattactatg atggaaagga ctacattgaa ttcaacaaag     540
   aaatcccagc ctgggtcccc ttcgacccag cagcccagat aaccaagcag aagtgggagg     600
   cagaaccagt ctacgtgcag cgggccaagg cttacctgga ggaggagtgc cctgcgactc     660
   tgcggaaata cctgaaatac agcaaaaata tcctggaccg gcaagatcct ccctctgtgg     720
   tggtcaccag ccaccaggcc ccaggagaaa agaagaaact gaagtgcctg gcctacgact     780
   tctacccagg gaaaattgat gtgcactgga ctcgggccgg cgaggtgcag gagcctgagt     840
   tacggggaga tgttcttcac aatggaaatg gcacttacca gtcctgggtg gtggtggcag     900
   tgcccccgca ggacacagcc ccctactcct gccacgtgca gcacagcagc ctggcccagc     960
   ccctcgtggt gccctgggag gccagctagg aagcaagggt tggaggcaat gtgggatctc    1020
   agacccagta gctgcccttc ctgcctgatg tgggagctga accacagaaa tcacagtcaa    1080
   tggatccaca aggcctgagg agcagtgtgg ggggacagac aggaggtgga tttggagacc    1140
   gaagactggg atgcctgtct tgagtagact tggacccaaa aaatcatctc accttgagcc    1200
   cacccccacc ccattgtcta atctgtagaa gctaataaat aatcatccct ccttgcctag    1260
   cataaaaaaa aaaaaaaa                                                  1278
<210> 58
   <211> 3012
   <212> DNA
   <213> Homo sapiens
<400> 58
   ggcaggaaca ctggcagggc agcctgctgt cggcttagag gggatgggca gtgtggaggg      60
   cctggcagag caagaggact catccttcca aagggacttt ctctgggaag cctgctcctc     120
   gggccactgc gaaccctctc tactctccga agggaattgt ccttcctggc ttccactact     180
   tccacccctg aatgcacagg cagcccggcc caagtctccc actagggatg cagatggatt     240
   cggtgtgaag ggctggctgc tgttgcctcc ggctcttgaa agtcaagttc agaggcgtgc     300
   aaagactcca gaattggagg catgatgaag actctgctgc tgtttgtggg gctgctgctg     360
   acctgggaga gtgggcaggt cctgggggac cagacggtct cagacaatga gctccaggaa     420
   atgtccaatc agggaagtaa gtacgtcaat aaggaaattc aaaatgctgt caacggggtg     480
   aaacagataa agactctcat agaaaaaaca aacgaagagc gcaagacact gctcagcaac     540
   ctagaagaag ccaagaagaa gaaagaggat gccctaaatg agaccaggga atcagagaca     600
   aagctgaagg agctcccagg agtgtgcaat gagaccatga tggccctctg ggaagagtgt     660
   aagccctgcc tgaaacagac ctgcatgaag ttctacgcac gcgtctgcag aagtggctca     720
   ggcctggttg gccgccagct tgaggagttc ctgaaccaga gctcgccctt ctacttctgg     780
   atgaatggtg accgcatcga ctccctgctg gagaacgacc ggcagcagac gcacatgctg     840
   gatgtcatgc aggaccactt cagccgcgcg tccagcatca tagacgagct cttccaggac     900
   aggttcttca cccgggagcc ccaggatacc taccactacc tgcccttcag cctgccccac     960
   cggaggcctc acttcttctt tcccaagtcc cgcatcgtcc gcagcttgat gcccttctct    1020
   ccgtacgagc ccctgaactt ccacgccatg ttccagccct tccttgagat gatacacgag    1080
   gctcagcagg ccatggacat ccacttccat agcccggcct tccagcaccc gccaacagaa    1140
   ttcatacgag aaggcgacga tgaccggact gtgtgccggg agatccgcca caactccacg    1200
   ggctgcctgc ggatgaagga ccagtgtgac aagtgccggg agatcttgtc tgtggactgt    1260
   tccaccaaca acccctccca ggctaagctg cggcgggagc tcgacgaatc cctccaggtc    1320
   gctgagaggt tgaccaggaa atacaacgag ctgctaaagt cctaccagtg gaagatgctc    1380
   aacacctcct ccttgctgga gcagctgaac gagcagttta actgggtgtc ccggctggca    1440
   aacctcacgc aaggcgaaga ccagtactat ctgcgggtca ccacggtggc ttcccacact    1500
   tctgactcgg acgttccttc cggtgtcact gaggtggtcg tgaagctctt tgactctgat    1560
   cccatcactg tgacggtccc tgtagaagtc tccaggaaga accctaaatt tatggagacc    1620
   gtggcggaga aagcgctgca ggaataccgc aaaaagcacc gggaggagtg agatgtggat    1680
   gttgcttttg cacctacggg ggcatctgag tccagctccc cccaagatga gctgcagccc    1740
   cccagagaga gctctgcacg tcaccaagta accaggcccc agcctccagg cccccaactc    1800
   cgcccagcct ctccccgctc tggatcctgc actctaacac tcgactctgc tgctcatggg    1860
   aagaacagaa ttgctcctgc atgcaactaa ttcaataaaa ctgtcttgtg agctgatcgc    1920
   ttggagggtc ctctttttat gttgagttgc tgcttcccgg catgccttca ttttgctatg    1980
   gggggcaggc aggggggatg gaaaataagt agaaacaaaa aagcagtggc taagatggta    2040
   tagggactgt cataccagtg aagaataaaa gggtgaagaa taaaagggat atgatgacaa    2100
   ggttgatcca cttcaagaat tgcttgcttt caggaagaga gatgtgtttc aacaagccaa    2160
   ctaaaatata ttgctgcaaa tggaagcttt tctgttctat tataaaactg tcgatgtatt    2220
   ctgaccaagg tgcgacaatc tcctaaagga atacactgaa agttaaggag aagaatcagt    2280
   aagtgtaagg tgtacttggt attataatgc ataattgatg ttttcgttat gaaaacattt    2340
   ggtgcccaga agtccaaatt atcagtttta tttgtaagag ctattgcttt tgcagcggtt    2400
   ttatttgtaa aagctgttga tttcgagttg taagagctca gcatcccagg ggcatcttct    2460
   tgactgtggc atttcctgtc caccgccggt ttatatgatc ttcatacctt tccctggacc    2520
   acaggcgttt ctcggctttt agtctgaacc atagctgggc tgcagtaccc tacgctgcca    2580
   gcaggtggcc atgactaccc gtggtaccaa tctcagtctt aaagctcagg cttttcgttc    2640
   attaacattc tctgatagaa ttctggtcat cagatgtact gcaatggaac aaaactcatc    2700
   tggctgcatc ccaggtgtgt agcaaagtcc acatgtaaat ttatagctta gaatattctt    2760
   aagtcactgt cccttgtctc tctttgaagt tataaacaac aaacttaaag cttagcttat    2820
   gtccaaggta agtattttag catggctgtc aaggaaattc agagtaaagt cagtgtgatt    2880
   cacttaatga tatacattaa ttagaattat ggggtcagag gtatttgctt aagtgatcat    2940
   aattgtaaag tatatgtcac attgtcacat taatgtcaca ctgtttcaaa agttaaaaaa    3000
   aaaaaaaaaa aa                                                        3012
<210> 59
   <211> 1970
   <212> DNA
   <213> Homo sapiens
<400> 59
   ggagggcagc accgccgggc ttcgcgccat gacatcagcc ctggagtggt gcagtgtgca      60
   aagcccactg gttggcgtgg cccgggacac gccttccgcg gagcggaaca aaacggcgcg     120
   caggccgggc gcacccagcc gccacttccg agagcgcctg ccgcccctgc gccgccgagc     180
   cagctgccag aatgccgaac tggggaggag gcaagaaatg tggggtgtgt cagaagacgg     240
   tttactttgc cgaagaggtt cagtgcgaag gcaacagctt ccataaatcc tgcttcctgt     300
   gcatggtctg caagaagaat ctggacagta ccactgtggc cgtgcatggt gaggagattt     360
   actgcaagtc ctgctacggc aagaagtatg ggcccaaagg ctatggctac gggcagggcg     420
   caggcaccct cagcactgac aagggggagt cgctgggtat caagcacgag gaagcccctg     480
   gccacaggcc caccaccaac cccaatgcat ccaaatttgc ccagaagatt ggtggctccg     540
   agcgctgccc ccgatgcagc caggcagtct atgctgcgga gaaggtgatt ggtgctggga     600
   agtcctggca taaggcctgc tttcgatgtg ccaagtgtgg caaaggcctt gagtcaacca     660
   ccctggcaga caaggatggc gagatttact gcaaaggatg ttatgctaaa aacttcgggc     720
   ccaagggctt tggttttggg caaggagctg ggcccttggt ccactctgag tgaggccacc     780
   atcacccacc acaccctgcc cactcctgcg cttttcatcg ccattccatt cccagcagct     840
   ttggagacct ccaggattat ttctctgtca gccctgccac atatcactaa tgacttgaac     900
   ttgggcatct ggctcccttt ggtttggggg tctgcctgag gtcccacccc actaaagggc     960
   tccccaggcc tgggatctga caccatcacc agtaggagac ctcagtgttt tgggtctagg    1020
   tgagagcagg cccctctccc cacacctcgc cccacagagc tctgttctta gcctcctgtg    1080
   ctgcgtgtcc atcatcagct gaccaagaca cctgaggaca catcttggca cccagaggag    1140
   cagcagcaac aggctggagg gagagggaag caagaccaag atgaggaggg gggaaggctg    1200
   ggttttttgg atctcagaga ttctcctctg tgggaaagag gttgagcttc ctggtgtccc    1260
   tcagagtaag cctgaggagt cccagcttag ggagtcacta ttggaggcag agaggcatgc    1320
   aggcagggtc ctaggagccc ctgcttctcc aggcctcttg cctttgagtc tttgtggaat    1380
   ggatagcctc ccactaggac tgggaggaga ataacccagg tcttaaggac cccaaagtca    1440
   ggatgttgtt tgatcttctc aaacatctag ttccctgctt gatgggagga tcctaatgaa    1500
   atacctgaaa catatattgg catttatcaa tggctcaaat cttcatttat ctctggcctt    1560
   aaccctggct cctgaggctg cggccagcag agcccaggcc agggctctgt tcttgccaca    1620
   cctgcttgat cctcagatgt ggagggaggt aggcactgcc tcagtcttca tccaaacacc    1680
   tttccctttg ccctgagacc tcagaatctt ccctttaacc caagaccctg cctcttccac    1740
   tccacccttc tccagggacc cttagatcac atcactccac ccctgccagg ccccaggtta    1800
   ggaatagtgg tgggaggaag gggaaagggc tgggcctcac cgctcccagc aactgaaagg    1860
   acaacactat ctggagccac ccactgaaag ggctgcaggc atgggctgta cccaagctga    1920
   tttctcatct ggtcaataaa gctgtttaga ccagaaaaaa aaaaaaaaaa               1970
<210> 60
   <211> 4412
   <212> DNA
   <213> Homo sapiens
<400> 60
   gcgccgctgc tcgaggaaac gctttcggcc gggagctgcg gccgccgcca gcagttttca      60
   tgtttgggat tcaggagaat attccgcgcg gggggacgac catgaaggag gagccgctgg     120
   gcagcggcat gaacccggtg cgctcgtgga tgcacacggc gggcgtggtg gacgccaaca     180
   cggccgccca gagcggcgtg gggctggcgc gggcgcactt cgagaagcag ccgccttcca     240
   acctccggaa atccaatttc ttccacttcg tgctggcgct ctacgatagg caggggcagc     300
   cggtggagat tgaaaggacc gcttttgtgg actttgtgga gaaagagaaa gagccaaaca     360
   acgagaaaac caacaacggc atccactata aactccagtt attgtacagc aacggagtca     420
   gaacagagca agatctgtat gttcgcctca tagattcaat gaccaaacag gccatcgtct     480
   acgagggcca ggacaagaac ccggagatgt gccgtgtgct gctgacccac gagatcatgt     540
   gcagccggtg ctgtgacaag aaaagttgtg gcaatagaaa cgaaacgccc tcagaccctg 600
   taatcattga cagattcttt ctaaagtttt tcctcaagtg caatcagaac tgtttgaaga 660
   atgcaggcaa ccctcgagat atgcggagat tccaggttgt tgtatcgaca acagtcaacg 720
   tggacggcca cgtgctggcc gtgtcagaca acatgtttgt gcacaacaat tccaaacacg 780
   ggaggcgggc ccgccgccta gacccgtcag aagccactcc gtgcatcaag gccatcagtc 840
   ccagtgaagg ctggaccacg gggggtgcca ccgtcatcat aattggcgac aacttctttg 900
   acgggctgca agttgtattc ggaactatgt tggtgtggag cgagctgata actccccatg 960
   ccatccgagt ccagaccccg ccgaggcaca ttcctggcgt cgtcgaagtg accctctcct 1020
   acaaatccaa gcagttctgc aaaggtgctc ctgggcgctt tgtctacacc gcccttaatg 1080
   aaccaaccat agattacggc tttcagaggt tgcagaaagt gatcccaaga catccgggtg 1140
   atcccgaaag gttacccaag gaggtgttac tgaagcgggc ggcggacctg gtggaagcct 1200
   tatacggaat gcctcacaac aaccaggaga tcatcttgaa gcgagcggcg gacatcgccg 1260
   aggcgctgta cagcgttccc cgcaatcaca accagatccc caccctgggc aacaaccctg 1320
   cacacacggg catgatgggc gtcaactcct tcagcagcca gctagccgtc aacgtgtcag 1380
   agacgtcaca agccaacgac caagtcggct acagtcgcaa tacaagcagc gtgtccccgc 1440
   gaggctacgt ccccagcagt actccccagc agtccaatta caacacagtc agcactagca 1500
   tgaatggata tggaagtggc gccatggcca gtctaggggt ccctggctcg cctggatttc 1560
   ttaatggctc ctccgctaac tctccctacg gcatgaaaca gaagagcgcc ttcgcgcccg 1620
   tggtccggcc ccaagcctct cctcctcctt cctgcaccag cgccaacggg aatggactgc 1680
   aagctatgtc tgggctggta gtcccgccaa tgtgagggac ttctgtttac cttccgcagc 1740
   acccagcatc aaaggacgga cttcagggga cacgtttagt atattaagac atgctgatgg 1800
   aaacagtatc ttcaaaaaaa tcagcagcaa ttgaaatgct acaaaagact ttgtttaaag 1860
   atttatttta aactattaag aatcaacatg caaacagcct acttcttcat gaacaattcc 1920
   attttattga ctgaactttt ctcatatttt cacatttctc agtcctgaag aataaggaaa 1980
   acaaagcgac gcctattttg tataaagttt ccgactccgt cttggccatg tctagtaatt 2040
   gctatgtgtt gggagaaact ttgtgaatgc accattttga tgatcatgaa acgctgatga 2100
   aaaatgcctc caaacatttt tctgtactca tacttagatt cacaatggtt gtgtatctct 2160
   ataatgtgaa atattttttt gtggtgataa aaagagggcc aaggaggtat gagccatcag 2220
   actggaaaaa aggatgacta tatgatgagg agaaactggg gtggcaggga gggagggagg 2280
   gtttatcact gcttaacttc atcttcatga aatgaaactt tgtaacttat tgtagttaga 2340
   aattgtaact ttgatattga attctcttgc cttcaacaag cacactgaca gagaaaaaat 2400
   gctactgtct gttggttcca atattctccc actgctagag cttcctgtta agcaagtgtg 2460
   atctgcaaca ttttttcaac ttttgctagc actgtatact attgcattct taggctactg 2520
   tgaggtctat gtttcttgta ccagaaattg tccttttgac ttctagatcc ttcttcccta 2580
   atgtgttttg tatgtggtta taaaattgta gacttttgtg attttgccaa agttgtagct 2640
   aaatatttat acacttgtct tgaatttttt tcagatccac ttaaaatatt tagaaaaaca 2700
   agttttattc cttatgtgtc ttataaggaa taaaatggtc ttcatttgac acttactttc 2760
   ccatgaacac ttgcagttgc taagggactt tattttgtaa catatcaatt ataaatattg 2820
   tatttatctt tgaaattttg tacattgctt ttcccacctt ttcctttttc ttctttcttg 2880
   tctgtattgg tttttgatca cggcctggtg ttgtgatact gggaagagca ttagccaaga 2940
   acttgtctct ttgatctgtt tcgttaagct gaaccagtgt ctttacattt catttgtact 3000
   tcaaaaaata tgctattgtt tagactttcc atcctttttt tttttatttt gaggaaaagt 3060
   caaattcatt gtttattttt atattatttt taagttatct gaacaaatac ttttgaaaaa 3120
   aaagtttgtt gtatagtcaa aacaaatcgg tgccacccgg ccgtgacaaa tcctagtaga 3180
   ttctgtgcat gtggagcggc cgcgaagagg tgacaccgtt tggggctgtg tccttatttt 3240
   attttatttt tttgtagaat gtaaaaagtc attttagatg ccacccattg actttgccac 3300
   atagctgaac tgtgtttact ggaaaaattc agaggcctaa agtttaaaat aaaatttact 3360
   tctgatgttt taattaaaat gtttgccaca ttaacttttc tgatgcctta aaagtgaact 3420
   tctttaaaga acctttgtgc tattttatca caggcttaca ctacaattgt taataaatac 3480
   ttcatttgga gatgtatggt gtaaaacaca caaacacaca caaaaaagca caagcccgct 3540
   gcatgacccg tctctccttt ctgggactat ttctgctgcg tcctgcaccc tcctgggccc 3600
   acctccgatt cacagaggtt tcagggggac ccaaatcact gctggttttc aatttttttt 3660
   taacaataca tttttgtggt cagttccaac agcactgtcc gtacttttaa aactggaatg 3720
   acctccttca gatatcgtgc ctcttagtgc caaacccaca gtgagaccaa aagtgtcagg 3780
   tgtttttttt tttttctttc tcctttgcac taagtgcttt gcagacacgg cacagcaaac 3840
   attttgcaaa ctgcagcaga aatcgaattt aaaacaaaaa ggagggactt taaaatacct 3900
   tcttgacaaa aatcaacaat gcacaactta caaagtgttc attctaggga caaaattaaa 3960
   taaacagaat gtccccagga gtcagcaggt cacagtctgg ctttgtgatg gttgacaagg 4020
   tctagctaca tgggaaagcc tgagaagtca ctttggaact aaattgcctc cattttattt 4080
   tgtacgagta agggtttgat ctacaaaaga gctcacatgg acgcactgag aacgcctgcc 4140
   agcttcccca tgccctcact tggtttgtgt tttaggttaa gtagtcaatg cccacatcac 4200
   ttcactgtct caagactgag cacttcacta aatggtagat tttactgtta aagaccctac 4260
   aataagattg ttttatctgt acattttttc agatatttaa ctgtataaaa atgttcattt 4320
   tacacaatat ttaattaaag tatttcttgt ctgtgaattt cacttttggt aattttctct 4380
   gtttttgatt attaaaatga ctaaacacta ac 4412
<210> 61
   <211> 1727
   <212> DNA
   <213> Homo sapiens
<400> 61
   aaagtgaggt ccctttctgg tgctggtggc cttcccagtt tagccaccct ttggattcct      60
   ggaaaatatg aggcctcgtg tgtccttgaa gcaggcagga caaacgagga catggcagcc     120
   ccttcccatc tgggagctga ggcttcttta atctggagac cttaaaaggc acgtgggagc     180
   caggggctgg gttcttatct cctaggcctg gccaatgtcc agacgtgcct tatttatagc     240
   gccactgaca agggcagcgc agctggctgg cagctgcaca gaaggttggg gccatccaac     300
   caggcagtcc gcctgcacag cagctgtccc tgctcatcgg gctggaggac agaagacaga     360
   accctaaaac cacaggttgc tgaaaagcca ggagtcaaaa tgactgagcg ctttgactgc     420
   caccattgca acgaatctct ctttggcaag aagtacatcc tgcgggagga gagcccctac     480
   tgcgtggtgt gctttgagac cctgttcgcc aacacctgcg aggagtgtgg gaagcccatc     540
   ggctgtgact gcaaggactt gtcttacaag gaccggcact ggcatgaagc ctgtttccac     600
   tgctcgcagt gcagaaactc actggtggac aagccctttg ctgccaagga ggaccagctg     660
   ctctgtacag actgctattc caacgagtac tcatccaagt gccaggaatg caagaagacc     720
   atcatgccag gtacccgcaa gatggagtac aagggcagca gctggcatga gacctgcttc     780
   atctgccacc gctgccagca gccaattgga accaagagtt tcatccccaa agacaatcag     840
   aatttctgtg tgccctgcta tgagaaacaa catgccatgc agtgcgttca gtgcaaaaag     900
   cccatcacca cgggaggggt cacttaccgg gagcagccct ggcacaagga gtgcttcgtg     960
   tgcaccgcct gcaggaagca gctgtctggg cagcgcttca cagctcgcga tgactttgcc    1020
   tactgcctga actgcttctg tgacttgtat gccaagaagt gtgctgggtg caccaacccc    1080
   atcagcggac ttggtggcac aaaatacatc tcctttgagg aacggcagtg gcataacgac    1140
   tgctttaact gtaagaagtg ctccctctca ctggtggggc gtggcttcct cacagagagg    1200
   gacgacatcc tgtgccccga ctgtgggaaa gacatctgaa ttcaacacag agaagttgct    1260
   gcttgtgatc tcacacacag atttttatgt tttctttctc acccaggcaa tcttgccttc    1320
   tggtttcttc cagccacatt gagactttct tctagtgctt ttcagtgata ctcacgtttg    1380
   cttaaaccct ttagtgcttt gtgatagttc agtcccaggg aaagagaaaa ctcgccctag    1440
   gccctaggtg ggaagatggt ttgaaatttt tgtaatcgag taaggcacac ccaaatgtaa    1500
   aaatcctttt gaatgatgcc tttataaatc tttctctcac tgtctattta agtgcaatta    1560
   acatatgtca cgaacttgaa agttttctaa actcaataag gtaatgacca gttgttattt    1620
   acagctctgt aacctcccgt tgcgtcaagt ctaaaccaag attatgtgac ttgcaataaa    1680
   gttattcaga acagaaaaaa aaaaaaaaaa aaaaaaaaaa aaaaaaa                  1727
<210> 62
   <211> 8533
   <212> DNA
   <213> Homo sapiens
<400> 62
   attcgcgtgg aggcgcgtcg cgcgcagcgg acgccgacag aatccccgag gcgcctggcg      60
   cgggcgcggg cgcgaaggcg atccgggcgc caccccgcgg tcatcggtca ccggtcgctc     120
   tcaggaacag cagcgcaacc tctgctccct gcctcgcctc ccgcgcgcct aggtgcctgc     180
   gactttaatt aaagggccgt cccctcgccg aggctgcagc accgcccccc cggcttctcg     240
   cgcctcaaaa tgagtagctc ccactctcgg gcgggccaga gcgcagcagg cgcggctccg     300
   ggcggcggcg tcgacacgcg ggacgccgag atgccggcca ccgagaagga cctggcggag     360
   gacgcgccgt ggaagaagat ccagcagaac actttcacgc gctggtgcaa cgagcacctg     420
   aagtgcgtga gcaagcgcat cgccaacctg cagacggacc tgagcgacgg gctgcggctt     480
   atcgcgctgt tggaggtgct cagccagaag aagatgcacc gcaagcacaa ccagcggccc     540
   actttccgcc aaatgcagct tgagaacgtg tcggtggcgc tcgagttcct ggaccgcgag     600
   agcatcaaac tggtgtccat cgacagcaag gccatcgtgg acgggaacct gaagctgatc     660
   ctgggcctca tctggaccct gatcctgcac tactccatct ccatgcccat gtgggacgag     720
   gaggaggatg aggaggccaa gaagcagacc cccaagcaga ggctcctggg ctggatccag     780
   aacaagctgc cgcagctgcc catcaccaac ttcagccggg actggcagag cggccgggcc     840
   ctgggcgccc tggtggacag ctgtgccccg ggcctgtgtc ctgactggga ctcttgggac     900
   gccagcaagc ccgttaccaa tgcgcgagag gccatgcagc aggcggatga ctggctgggc     960
   atcccccagg tgatcacccc cgaggagatt gtggacccca acgtggacga gcactctgtc    1020
   atgacctacc tgtcccagtt ccccaaggcc aagctgaagc caggggctcc cttgcggccc    1080
   aaactgaacc cgaagaaagc ccgtgcctac gggccaggca tcgagcccac aggcaacatg    1140
   gtgaagaagc gggcagagtt cactgtggag accagaagtg ctggccaggg agaggtgctg    1200
   gtgtacgtgg aggacccggc cggacaccag gaggaggcaa aagtgaccgc caataacgac    1260
   aagaaccgca ccttctccgt ctggtacgtc cccgaggtga cggggactca taaggttact    1320
   gtgctctttg ctggccagca catcgccaag agccccttcg aggtgtacgt ggataagtca    1380
   cagggtgacg ccagcaaagt gacagcccaa ggtcccggcc tggagcccag tggcaacatc    1440
   gccaacaaga ccacctactt tgagatcttt acggcaggag ctggcacggg cgaggtcgag    1500
   gttgtgatcc aggaccccat gggacagaag ggcacggtag agcctcagct ggaggcccgg    1560
   ggcgacagca cataccgctg cagctaccag cccaccatgg agggcgtcca caccgtgcac    1620
   gtcacgtttg ccggcgtgcc catccctcgc agcccctaca ctgtcactgt tggccaagcc    1680
   tgtaacccga gtgcctgccg ggcggttggc cggggcctcc agcccaaggg tgtgcgggtg 1740
   aaggagacag ctgacttcaa ggtgtacaca aagggcgctg gcagtgggga gctgaaggtc 1800
   accgtgaagg gccccaaggg agaggagcgc gtgaagcaga aggacctggg ggatggcgtg 1860
   tatggcttcg agtattaccc catggtccct ggaacctata tcgtcaccat cacgtggggt 1920
   ggtcagaaca tcgggcgcag tcccttcgaa gtgaaggtgg gcaccgagtg tggcaatcag 1980
   aaggtacggg cctggggccc tgggctggag ggcggcgtcg ttggcaagtc agcagacttt 2040
   gtggtggagg ctatcgggga cgacgtgggc acgctgggct tctcggtgga agggccatcg 2100
   caggctaaga tcgaatgtga cgacaagggc gacggctcct gtgatgtgcg ctactggccg 2160
   caggaggctg gcgagtatgc cgttcacgtg ctgtgcaaca gcgaagacat ccgcctcagc 2220
   cccttcatgg ctgacatccg tgacgcgccc caggacttcc acccagacag ggtgaaggca 2280
   cgtgggcctg gattggagaa gacaggtgtg gccgtcaaca agccagcaga gttcacagtg 2340
   gatgccaagc acggtggcaa ggccccactt cgggtccaag tccaggacaa tgaaggctgc 2400
   cctgtggagg cgttggtcaa ggacaacggc aatggcactt acagctgctc ctacgtgccc 2460
   aggaagccgg tgaagcacac agccatggtg tcctggggag gcgtcagcat ccccaacagc 2520
   cccttcaggg tgaatgtggg agctggcagc caccccaaca aggtcaaagt atacggcccc 2580
   ggagtagcca agacagggct caaggcccac gagcccacct acttcactgt ggactgcgcc 2640
   gaggctggcc agggggacgt cagcatcggc atcaagtgtg cccctggagt ggtaggcccc 2700
   gccgaagctg acatcgactt cgacatcatc cgcaatgaca atgacacctt cacggtcaag 2760
   tacacgcccc ggggggctgg cagctacacc attatggtcc tctttgctga ccaggccacg 2820
   cccaccagcc ccatccgagt caaggtggag ccctctcatg acgccagtaa ggtgaaggcc 2880
   gagggccctg gcctcagtcg cactggtgtc gagcttggca agcccaccca cttcacagta 2940
   aatgccaaag ctgctggcaa aggcaagctg gacgtccagt tctcaggact caccaagggg 3000
   gatgcagtgc gagatgtgga catcatcgac caccatgaca acacctacac agtcaagtac    3060
   acgcctgtcc agcagggtcc agtaggcgtc aatgtcactt atggagggga tcccatccct    3120
   aagagccctt tctcagtggc agtatctcca agcctggacc tcagcaagat caaggtgtct 3180
   ggcctgggag agaaggtgga cgttggcaaa gaccaggagt tcacagtcaa atcaaagggt 3240
   gctggtggtc aaggcaaagt ggcatccaag attgtgggcc cctcgggtgc agcggtgccc 3300
   tgcaaggtgg agccaggcct gggggctgac aacagtgtgg tgcgcttcct gccccgtgag 3360
   gaagggccct atgaggtgga ggtgacctat gacggcgtgc ccgtgcctgg cagccccttt 3420
   cctctggaag ctgtggcccc caccaagcct agcaaggtga aggcgtttgg gccggggctg 3480
   cagggaggca gtgcgggctc ccccgcccgc ttcaccatcg acaccaaggg cgccggcaca 3540
   ggtggcctgg gcctgacggt ggagggcccc tgtgaggcgc agctcgagtg cttggacaat 3600
   ggggatggca catgttccgt gtcctacgtg cccaccgagc ccggggacta caacatcaac 3660
   atcctcttcg ctgacaccca catccctggc tccccattca aggcccacgt ggttccctgc 3720
   tttgacgcat ccaaagtcaa gtgctcaggc cccgggctgg agcgggccac cgctggggag 3780
   gtgggccaat tccaagtgga ctgctcgagc gcgggcagcg cggagctgac cattgagatc 3840
   tgctcggagg cggggcttcc ggccgaggtg tacatccagg accacggtga tggcacgcac 3900
   accattacct acattcccct ctgccccggg gcctacaccg tcaccatcaa gtacggcggc 3960
   cagcccgtgc ccaacttccc cagcaagctg caggtggaac ctgcggtgga cacttccggt 4020
   gtccagtgct atgggcctgg tattgagggc cagggtgtct tccgtgaggc caccactgag 4080
   ttcagtgtgg acgcccgggc tctgacacag accggagggc cgcacgtcaa ggcccgtgtg 4140
   gccaacccct caggcaacct gacggagacc tacgttcagg accgtggcga tggcatgtac 4200
   aaagtggagt acacgcctta cgaggaggga ctgcactccg tggacgtgac ctatgacggc 4260
   agtcccgtgc ccagcagccc cttccaggtg cccgtgaccg agggctgcga cccctcccgg 4320
   gtgcgtgtcc acgggccagg catccaaagt ggcaccacca acaagcccaa caagttcact 4380
   gtggagacca ggggagctgg cacgggcggc ctgggcctgg ctgtagaggg cccctccgag 4440
   gccaagatgt cctgcatgga taacaaggac ggcagctgct cggtcgagta catcccttat 4500
   gaggctggca cctacagcct caacgtcacc tatggtggcc atcaagtgcc aggcagtcct 4560
   ttcaaggtcc ctgtgcatga tgtgacagat gcgtccaagg tcaagtgctc tgggcccggc 4620
   ctgagcccag gcatggttcg tgccaacctc cctcagtcct tccaggtgga cacaagcaag 4680
   gctggtgtgg ccccattgca ggtcaaagtg caagggccca aaggcctggt ggagccagtg 4740
   gacgtggtag acaacgctga tggcacccag accgtcaatt atgtgcccag ccgagaaggg 4800
   ccctacagca tctcagtact gtatggagat gaagaggtac cccggagccc cttcaaggtc 4860
   aaggtgctgc ctactcatga tgccagcaag gtgaaggcca gtggccccgg gctcaacacc 4920
   actggcgtgc ctgccagcct gcccgtggag ttcaccatcg atgcaaagga cgccggggag 4980
   ggcctgctgg ctgtccagat cacggatccc gaaggcaagc cgaagaagac acacatccaa 5040
   gacaaccatg acggcacgta tacagtggcc tacgtgccag acgtgacagg tcgctacacc 5100
   atcctcatca agtacggtgg tgacgagatc cccttctccc cgtaccgcgt gcgtgccgtg 5160
   cccaccgggg acgccagcaa gtgcactgtc acaggtgctg gcatcggccc caccattcag 5220
   attggggagg agacggtgat cactgtggac actaaggcgg caggcaaagg caaagtgacg 5280
   tgcaccgtgt gcacgcctga tggctcagag gtggatgtgg acgtggtgga gaatgaggac 5340
   ggcactttcg acatcttcta cacggccccc cagccgggca aatacgtcat ctgtgtgcgc 5400
   tttggtggcg agcacgtgcc caacagcccc ttccaagtga cggctctggc tggggaccag 5460
   ccctcggtgc agccccctct acggtctcag cagctggccc cacagtacac ctacgcccag 5520
   ggcggccagc agacttgggc cccggagagg cccctggtgg gtgtcaatgg gctggatgtg 5580
   accagcctga ggccctttga ccttgtcatc cccttcacca tcaagaaggg cgagatcaca 5640
   ggggaggttc ggatgccctc aggcaaggtg gcgcagccca ccatcactga caacaaagac 5700
   ggcaccgtga ccgtgcggta tgcacccagc gaggctggcc tgcacgagat ggacatccgc 5760
   tatgacaaca tgcacatccc aggaagcccc ttgcagttct atgtggatta cgtcaactgt 5820
   ggccatgtca ctgcctatgg gcctggcctc acccatggag tagtgaacaa gcctgccacc 5880
   ttcaccgtca acaccaagga tgcaggagag gggggcctgt ctctggccat tgagggcccg 5940
   tccaaagcag aaatcagctg cactgacaac caggatggga catgcagcgt gtcctacctg 6000
   cctgtgctgc cgggggacta cagcattcta gtcaagtaca atgaacagca cgtcccaggc 6060
   agccccttca ctgctcgggt cacaggtgac gactccatgc gtatgtccca cctaaaggtc 6120
   ggctctgctg ccgacatccc catcaacatc tcagagacgg atctcagcct gctgacggcc 6180
   actgtggtcc cgccctcggg ccgggaggag ccctgtttgc tgaagcggct gcgtaatggc 6240
   cacgtgggga tttcattcgt gcccaaggag acgggggagc acctggtgca tgtgaagaaa 6300
   aatggccagc acgtggccag cagccccatc ccggtggtga tcagccagtc ggaaattggg 6360
   gatgccagtc gtgttcgggt ctctggtcag ggccttcacg aaggccacac ctttgagcct 6420
   gcagagttta tcattgatac ccgcgatgca ggctatggtg ggctcagcct gtccattgag 6480
   ggccccagca aggtggacat caacacagag gacctggagg acgggacgtg cagggtcacc 6540
   tactgcccca cagagccagg caactacatc atcaacatca agtttgccga ccagcacgtg 6600
   cctggcagcc ccttctctgt gaaggtgaca ggcgagggcc gggtgaaaga gagcatcacc 6660
   cgcaggcgtc gggctccttc agtggccaac gttggtagtc attgtgacct cagcctgaaa 6720
   atccctgaaa ttagcatcca ggatatgaca gcccaggtga ccagcccatc gggcaagacc 6780
   catgaggccg agatcgtgga aggggagaac cacacctact gcatccgctt tgttcccgct 6840
   gagatgggca cacacacagt cagcgtgaag tacaagggcc agcacgtgcc tgggagcccc 6900
   ttccagttca ccgtggggcc cctaggggaa gggggagccc acaaggtccg agctgggggc 6960
   cctggcctgg agagagctga agctggagtg ccagccgaat tcagtatctg gacccgggaa 7020
   gctggtgctg gaggcctggc cattgctgtc gagggcccca gcaaggctga gatctctttt 7080
   gaggaccgca aggacggctc ctgtggtgtg gcttatgtgg tccaggagcc aggtgactac 7140
   gaagtctcag tcaagttcaa cgaggaacac attcccgaca gccccttcgt ggtgcctgtg 7200
   gcttctccgt ctggcgacgc ccgccgcctc actgtttcta gccttcagga gtcagggcta 7260
   aaggtcaacc agccagcctc ttttgcagtc agcctgaacg gggccaaggg ggcgatcgat 7320
   gccaaggtgc acagcccctc aggagccctg gaggagtgct atgtcacaga aattgaccaa 7380
   gataagtatg ctgtgcgctt catccctcgg gagaatggcg tttacctgat tgacgtcaag 7440
   ttcaacggca cccacatccc tggaagcccc ttcaagatcc gagttgggga gcctgggcat 7500
   ggaggggacc caggcttggt gtctgcttac ggagcaggtc tggaaggcgg tgtcacaggg 7560
   aacccagctg agttcgtcgt gaacacgagc aatgcgggag ctggtgccct gtcggtgacc 7620
   attgacggcc cctccaaggt gaagatggat tgccaggagt gccctgaggg ctaccgcgtc 7680
   acctataccc ccatggcacc tggcagctac ctcatctcca tcaagtacgg cggcccctac 7740
   cacattgggg gcagcccctt caaggccaaa gtcacaggcc cccgtctcgt cagcaaccac 7800
   agcctccacg agacatcatc agtgtttgta gactctctga ccaaggccac ctgtgccccc 7860
   cagcatgggg ccccgggtcc tgggcctgct gacgccagca aggtggtggc caagggcctg 7920
   gggctgagca aggcctacgt aggccagaag agcagcttca cagtagactg cagcaaagca 7980
   ggcaacaaca tgctgctggt gggggttcat ggcccaagga ccccctgcga ggagatcctg 8040
   gtgaagcacg tgggcagccg gctctacagc gtgtcctacc tgctcaagga caagggggag 8100
   tacacactgg tggtcaaatg gggggacgag cacatcccag gcagccccta ccgcgttgtg 8160
   gtgccctgag tctggggccc gtgccagccg gcagccccca agcctgcccc gctacccaag 8220
   cagccccgcc ctcttcccct caaccccggc ccaggccgcc ctggccgccc gcctgtcact 8280
   gcagccgccc ctgccctgtg ccgtgctgcg ctcacctgcc tccccagcca gccgctgacc 8340
   tctcggcttt cacttgggca gagggagcca tttggtggcg ctgcttgtct tctttggttc 8400
   tgggaggggt gagggatggg ggtcctgtac acaaccaccc actagttctc ttctccagcc 8460
   aagaggaata aagttttgct tccattaaaa aaaaaaaaaa aaaaaaaaaa aaaaaaaaaa 8520
   aaaaaaaaaa aaa                                                    8533
<210> 63
   <211> 5091
   <212> DNA
   <213> Homo sapiens
<400> 63
   gtcctctctc gcggtatcat ccggttgctg aggccctgta ataaaggtct cgcgaaattt      60
   gttctagagg tccaagtttg cttcttagct tactccaccc cacccccaac ctgtccctcc     120
   ttttctttcc aagtcacaaa attctcccct cccctacccc ggagtttacg gccctcctcc     180
   tgtttccgat ttcagcccgg aaccggaagt gtagtgggcg gggcccgtcg gcggaaaacg     240
   cagcggagcc agagccggac acggctgtgg ccgctgcctc tacccccgcc acggatcgcc     300
   gggtagtagg actgcgcggc tccaggctga gggtcggtcc ggaggcgggt gggcgcgggt     360
   ctcacccgga ttgtccgggt ggcaccgttc ccggccccac cgggcgccgc gagggatcat     420
   gtctacagcc tctgccgcct cctcctcctc ctcgtcttcg gccggtgaga tgatcgaagc     480
   cccttcccag gtcctcaact ttgaagagat cgactacaag gagatcgagg tggaagaggt     540
   tgttggaaga ggagcctttg gagttgtttg caaagctaag tggagagcaa aagatgttgc     600
   tattaaacaa atagaaagtg aatctgagag gaaagcgttt attgtagagc ttcggcagtt     660
   atcccgtgtg aaccatccta atattgtaaa gctttatgga gcctgcttga atccagtgtg     720
   tcttgtgatg gaatatgctg aagggggctc tttatataat gtgctgcatg gtgctgaacc     780
   attgccatat tatactgctg cccacgcaat gagttggtgt ttacagtgtt cccaaggagt     840
   ggcttatctt cacagcatgc aacccaaagc gctaattcac agggacctga aaccaccaaa     900
   cttactgctg gttgcagggg ggacagttct aaaaatttgt gattttggta cagcctgtga     960
   cattcagaca cacatgacca ataacaaggg gagtgctgct tggatggcac ctgaagtttt    1020
   tgaaggtagt aattacagtg aaaaatgtga cgtcttcagc tggggtatta ttctttggga    1080
   agtgataacg cgtcggaaac cctttgatga gattggtggc ccagctttcc gaatcatgtg    1140
   ggctgttcat aatggtactc gaccaccact gataaaaaat ttacctaagc ccattgagag    1200
   cctgatgact cgttgttggt ctaaagatcc ttcccagcgc ccttcaatgg aggaaattgt    1260
   gaaaataatg actcacttga tgcggtactt tccaggagca gatgagccat tacagtatcc    1320
   ttgtcagtat tcagatgaag gacagagcaa ctctgccacc agtacaggct cattcatgga    1380
   cattgcttct acaaatacga gtaacaaaag tgacactaat atggagcaag ttcctgccac    1440
   aaatgatact attaagcgct tagaatcaaa attgttgaaa aatcaggcaa agcaacagag    1500
   tgaatctgga cgtttaagct tgggagcctc ccgtgggagc agtgtggaga gcttgccccc    1560
   aacctctgag ggcaagagga tgagtgctga catgtctgaa atagaagcta ggatcgccgc    1620
   aaccacaggc aacggacagc caagacgtag atccatccaa gacttgactg taactggaac    1680
   agaacctggt caggtgagca gtaggtcatc cagtcccagt gtcagaatga ttactacctc    1740
   aggaccaacc tcagaaaagc caactcgaag tcatccatgg acccctgatg attccacaga    1800
   taccaatgga tcagataact ccatcccaat ggcttatctt acactggatc accaactaca    1860
   gcctctagca ccgtgcccaa actccaaaga atctatggca gtgtttgaac agcattgtaa    1920
   aatggcacaa gaatatatga aagttcaaac agaaattgca ttgttattac agagaaagca    1980
   agaactagtt gcagaactgg accaggatga aaaggaccag caaaatacat ctcgcctggt    2040
   acaggaacat aaaaagcttt tagatgaaaa caaaagcctt tctacttact accagcaatg    2100
   caaaaaacaa ctagaggtca tcagaagtca gcagcagaaa cgacaaggca cttcatgatt    2160
   ctctgggacc gttacatttt gaaatatgca aagaaagact ttttttttaa ggaaaggaaa    2220
   accttataat gacgattcat gagtgttagc tttttggcgt gttctgaatg ccaactgcct    2280
   atatttgctg catttttttc attgtttatt ttccttttct catggtggac atacaatttt    2340
   actgtttcat tgcataacat ggtagcatct gtgacttgaa tgagcagcac tttgcaactt    2400
   caaaacagat gcagtgaact gtggctgtat atgcatgctc attgtgtgaa ggctagccta    2460
   acagaacagg aggtatcaaa ctagctgcta tgtgcaaaca gcgtccattt tttcatatta    2520
   gaggtggaac ctcaagaatg actttattct tgtatctcat ctcaaaatat taataatttt    2580
   tttcccaaaa gatggtatat accaagttaa agacagggta ttataaattt agagtgattg    2640
   gtggtatatt acggaaatac ggaaccttta gggatagttc cgtgtaaggg ctttgatgcc    2700
   agcatccttg gatcagtact gaactcagtt ccatccgtaa aatatgtaaa ggtaagtggc    2760
   agctgctcta tttaatgaaa gcagttttac cggattttgt tagactaaaa tttgattgtg    2820
   atacattgaa caaaatggaa ctcatttttt tttaaggagt aaagattttt aattctgtga    2880
   ttgtgtgtat gtgtgttgaa actgtaaagc ttttatgact ctaatattaa tctcttaaat    2940
   gaaattaaaa ggcaaaagaa catgattgag cttaaatgat catttcttcc tgcagtgatt    3000
   cttggattgt tttctcatgt atttgaaaaa aaaaaaatga agaaaaataa tggaaaatgg    3060
   aagtaattac tccagctaaa aaaagcttgg acttagattt ctttttatga taccaaatga    3120
   gaaataaacc aggcaaatca gaaggaagtt aaagaagcaa atataaattc aacaagtgtc    3180
   ctaattatcc tggatattgg aatatttgat tttccttaca atcccgttct agaatgcctg    3240
   ccgcctttca acactttcaa gagaatttca acacttacag agtatttata ttgttaacag    3300
   taattttgct accaaaacct tcagaataac tttaataata aaatgacact gaaataatag    3360
   actatacaaa ctctatattt tttcagttgg tgttaaaata agtcacattt tgataccagt    3420
   acaagatgtc ttttaaataa ggatgtcatc agtctgattt ttatagcatt aatgttttat    3480
   gaagaaaaag ttcaaaatga aagcattaat tgctgtgatt attagaattc tatcatgact    3540
   gtattgtagt ttttgctcta tttcagataa gcaagatcta agaagttatc aaaactattc    3600
   tttaaaatgc taaagcaggt aactttttct tccattattt tttcctccta ccactgagtt    3660
   ttgtaatgaa ttccttgtgt atacaagcaa tacaggtgaa tactaaactg ttatttttag    3720
   cttcttcaaa agctatttta gaaagcttcc tggaaataaa tgtcttctgt catttaattt    3780
   aaataaaagg agtgttaatt gttcccaaat ttgattacct agctgaaaca gatattggat    3840
   tcagcatagt aatagtaaaa taaaattggc agagaaaata actgtagact aggataaagc    3900
   atgactcctc catagcattg ttgcttgtat gtacatctgt cactccactg aaagggacct    3960
   agtcacttta aagaggcctt ttatctcagc acgagatatc caagcgtgaa tgctaaggct    4020
   tgatgtttcc attaggattt agcattcgag atttcagatt ttatttatag tcattgatgt    4080
   gttttgctgt attataacac atttaaggga aattttatta tggtttttac atgtagtcat    4140
   ttcataaaaa tatccagata ggtaaatgga agaaaatagt agattttagt catggtacca    4200
   aatgcaagag gctgttgaac agttgcttgt ttgtttacag taagttcctt gagattttca    4260
   gtactgtagt tgccctggac attgagtaca gttcagcctt actctgtttt taagtagttg    4320
   tgttgtcaat ttcatgcttt taagtcctga tgatccttgg agatgggaga aaaagatacc    4380
   agggataatt aaaatccaca gccagtttca tcagtttcct catccatcct gcaaataaaa    4440
   atgttaacaa ggagccaaac ttattcgttc tggttttaca atttattttg accgtttttt    4500
   gggtgaaaat tgcaaacagc caagcaagtg gctggaatgc cccagtctaa gaaattcaaa    4560
   aacaatcact gagtaagccc taattacatt aattacattt tactcttgac tccaggaaaa    4620
   gaaagcaagc ttttatctaa tctagaggga agtattcgtg agcaataaaa atcacttttt    4680
   tgagttgaat aataatcagt taagaatatt taccgcatag caacacaaca tataaagatt    4740
   tgttacaagt tgtttacgga tgtttgacta tttttgctga agtattttag agtattgaat    4800
   gtcttctctc ttcaagtttc tttcatgttc ctaatttcag ctcctgtagc cagagatcac 4860
   aggtcttccc tgtgaaactt tggtttcttt ctataaatgt gtgtggtttt cagcgctcaa 4920
   ctcctgtctt caaatggtag taagttctac ttctacttct gtcattcaga acattttatg 4980
   tcaaatgatg taatgcagaa attcttgtgc atatttgtaa ctgaaggaag ctttttagat 5040
   ttatttttgt ttttaataaa attcagattc ctattctaaa ctggtaaaaa a 5091
<210> 64
   <211> 503
   <212> DNA
   <213> Homo sapiens
<400> 64
   gtacctgtct ataaggagtc ctgcttatca caatgaatgt tctcctgggc agcgttgtga 60
   tctttgccac cttcgtgact ttatgcaatg catcatgcta tttcatacct aatgagggag 120
   ttccaggaga ttcaaccagg aaatgcatgg atctcaaagg aaacaaacac ccaataaact 180
   cggagtggca gactgacaac tgtgagacat gcacttgcta cgaaacagaa atttcatgtt 240
   gcacccttgt ttctacacct gtgggttatg acaaagacaa ctgccaaaga atcttcaaga 300
   aggaggactg caagtatatc gtggtggaga agaaggaccc aaaaaagacc tgttctgtca 360
   gtgaatggat aatctaatgt gcttctagta ggcacagggc tcccaggcca ggcctcattc 420
   tcctctggcc tctaatagtc aatgattgtg tagccatgcc tatcagtaaa aagatttttg 480
   agcaaacact tgaaaaaaaa aaa 503
<210> 65
   <211> 7852
   <212> DNA
   <213> Homo sapiens
<400> 65
   ggcgctgagc gagctcggag cccgcgctgt gcgcctgcgg ccggggcgcc ccgccgagcg      60
   ccggtgcccc ggctcccggg ccgccttcgc cgcgcgggaa ggattcttca aaattaacag     120
   aaaccaattc gggccagctg aagagaaaaa ataaaggtgg ctcccggctg cctctgctgc     180
   agttcagagc aacttcagga gcttcccagc cgagagcttc aggacgcctt tcctgtccca     240
   ctggcccagt tgccacaaca aacaacagag aagacggtga ccatggggga tgtgaagctg     300
   gttgcctcgt cacacatttc caaaacctcc ctcagtgtgg atccctcaag agttgactcc     360
   atgcccctga cagaggcccc tgctttcatt ttgccccctc ggaacctctg catcaaagaa      420
   ggagccaccg ccaagttcga agggcgggtc cggggttacc cagagcccca ggtgacatgg     480
   cacagaaacg ggcaacccat caccagcggg ggccgcttcc tgctggattg cggcatccgg     540
   gggactttca gccttgtgat tcatgctgtc catgaggagg acaggggaaa gtatacctgt     600
   gaagccacca atggcagtgg tgctcgccag gtgacagtgg agttgacagt agaaggaagt     660
   tttgcgaagc agcttggtca gcctgttgtt tccaaaacct taggggatag attttcagct     720
   ccagcagtgg agacccgtcc tagcatctgg ggggagtgcc caccaaagtt tgctaccaag     780
   ctgggccgag ttgtggtcaa agaaggacag atgggacgat tctcctgcaa gatcactggc     840
   cggccccaac cgcaggtcac ctggctcaag ggaaatgttc cactgcagcc gagtgcccgt     900
   gtgtctgtgt ctgagaagaa cggcatgcag gttctggaaa tccatggagt caaccaagat     960
   gacgtgggag tgtacacgtg cctggtggtg aacgggtcgg ggaaggcctc gatgtcagct    1020
   gaactttcca tccaaggttt ggacagtgcc aataggtcat ttgtgagaga aacaaaagcc    1080
   accaattcag atgtcaggaa agaggtgacc aatgtaatct caaaggagtc gaagctggac    1140
   agtctggagg ctgcagccaa aagcaagaac tgctccagcc cccagagagg tggctcccca    1200
   ccctgggctg caaacagcca gcctcagccc ccaagggagt ccaagctgga gtcatgcaag    1260
   gactcgccca gaacggcccc gcagaccccg gtccttcaga agacttccag ctccatcacc    1320
   ctgcaggccg caagagttca gccggaacca agagcaccag gcctgggggt cctatcacct    1380
   tctggagaag agaggaagag gccagctcct ccccgtccag ccaccttccc caccaggcag    1440
   cctggcctgg ggagccaaga tgttgtgagc aaggctgcta acaggagaat ccccatggag    1500
   ggccagaggg attcagcatt ccccaaattt gagagcaagc cccaaagcca ggaggtcaag    1560
   gaaaatcaaa ctgtcaagtt cagatgtgaa gtttccggga ttccaaagcc tgaagtggcc    1620
   tggttcctgg aaggcacccc cgtgaggaga caggaaggca gcattgaggt ttatgaagat    1680
   gctggctccc attacctctg cctgctgaaa gcccggacca gggacagtgg gacatacagc    1740
   tgcactgctt ccaacgccca aggccagctg tcctgtagct ggaccctcca agtggaaagg    1800
   cttgccgtga tggaggtggc cccctccttc tccagtgtcc tgaaggactg cgctgttatt    1860
   gagggccagg attttgtgct gcagtgctcc gtacggggga ccccagtgcc ccggatcact    1920
   tggctgctga atgggcagcc catccagtac gctcgctcca cctgcgaggc cggcgtggct    1980
   gagctccaca tccaggatgc cctgccggag gaccatggca cctacacctg cctagctgag    2040
   aatgccttgg ggcaggtgtc ctgcagcgcc tgggtcaccg tccatgaaaa gaagagtagc    2100
   aggaagagtg agtaccttct gcctgtggct cccagcaagc ccactgcacc catcttcctg    2160
   cagggcctct ctgatctcaa agtcatggat ggaagccagg tcactatgac tgtccaagtg    2220
   tcagggaatc caccccctga agtcatctgg ctgcacaatg ggaatgagat ccaagagtca     2280
   gaggacttcc actttgaaca gagaggaact cagcacagcc tttgtatcca ggaagtgttc 2340
   ccggaggaca cgggcacgta cacctgcgag gcctggaaca gcgctggaga ggtccgcacc 2400
   caggccgtgc tcacggtaca agagcctcac gatggcaccc agccctggtt catcagtaag 2460
   cctcgctcag tgacagcctc cctgggccag agtgtcctca tctcctgcgc catagctggt 2520
   gacccctttc ctaccgtgca ctggctcaga gatggcaaag ccctctgcaa agacactggc 2580
   cacttcgagg tgcttcagaa tgaggacgtg ttcaccctgg ttctaaagaa ggtgcagccc 2640
   tggcatgccg gccagtatga gatcctgctc aagaaccggg ttggcgaatg cagttgccag 2700
   gtgtcactga tgctacagaa cagctctgcc agagcccttc cacgggggag ggagcctgcc 2760
   agctgcgagg acctctgtgg tggaggagtt ggtgctgatg gtggtggtag tgaccgctat 2820
   gggtccctga ggcctggctg gccagcaaga gggcagggtt ggctagagga ggaagacggc 2880
   gaggacgtgc gaggggtgct gaagaggcgc gtggagacga ggcagcacac tgaggaggcg 2940
   atccgccagc aggaggtgga gcagctggac ttccgagacc tcctggggaa gaaggtgagt 3000
   acaaagaccc tatcggaaga cgacctgaag gagatcccag ccgagcagat ggatttccgt 3060
   gccaacctgc agcggcaagt gaagccaaag actgtgtctg aggaagagag gaaggtgcac 3120
   agcccccagc aggtcgattt tcgctctgtc ctggccaaga aggggacttc caagaccccc 3180
   gtgcctgaga aggtgccacc gccaaaacct gccaccccgg attttcgctc agtgctgggt 3240
   ggcaagaaga aattaccagc agagaatggc agcagcagtg ccgagaccct gaatgccaag 3300
   gcagtggaga gttccaagcc cctgagcaat gcacagcctt cagggccctt gaaacccgtg 3360
   ggcaacgcca agcctgctga gaccctgaag ccaatgggca acgccaagcc tgccgagacc 3420
   ctgaagccca tgggcaatgc caagcctgat gagaacctga aatccgctag caaagaagaa 3480
   ctcaagaaag acgttaagaa tgatgtgaac tgcaagagag gccatgcagg gaccacagat 3540
   aatgaaaaga gatcagagag ccaggggaca gccccagcct tcaagcagaa gctgcaagat 3600
   gttcatgtgg cagagggcaa gaagctgctg ctccagtgcc aggtgtcttc tgacccccca 3660
   gccaccatca tctggacgct gaacggaaag accctcaaga ccaccaagtt catcatcctc 3720
   tcccaggaag gctcactctg ctccgtctcc atcgagaagg cactgcctga ggacagaggc 3780
   ttatacaagt gtgtagccaa gaatgacgct ggccaggcgg agtgctcctg ccaagtcacc 3840
   gtggatgatg ctccagccag tgagaacacc aaggccccag agatgaaatc ccggaggccc 3900
   aagagctctc ttcctcccgt gctaggaact gagagtgatg cgactgtgaa aaagaaacct 3960
   gcccccaaga cacctccgaa ggcagcaatg ccccctcaga tcatccagtt ccctgaggac 4020
   cagaaggtac gcgcaggaga gtcagtggag ctgtttggca aagtgacagg cactcagccc 4080
   atcacctgta cctggatgaa gttccgaaag cagatccagg aaagcgagca catgaaggtg 4140
   gagaacagcg agaatggcag caagctcacc atcctggccg cgcgccagga gcactgcggc 4200
   tgctacacac tgctggtgga gaacaagctg ggcagcaggc aggcccaggt caacctcact 4260
   gtcgtggata agccagaccc cccagctggc acaccttgtg cctctgacat tcggagctcc 4320
   tcactgaccc tgtcctggta tggctcctca tatgatgggg gcagtgctgt acagtcctac 4380
   agcatcgaga tctgggactc agccaacaag acgtggaagg aactagccac atgccgcagc 4440
   acctctttca acgtccagga cctgctgcct gaccacgaat ataagttccg tgtacgtgca 4500
   atcaacgtgt atggaaccag tgagccaagc caggagtctg aactcacaac ggtaggagag 4560
   aaacctgaag agccgaagga tgaagtggag gtgtcagatg atgatgagaa ggagcccgag 4620
   gttgattacc ggacagtgac aatcaatact gaacaaaaag tatctgactt ctacgacatt 4680
   gaggagagat taggatctgg gaaatttgga caggtctttc gacttgtaga aaagaaaact 4740
   cgaaaagtct gggcagggaa gttcttcaag gcatattcag caaaagagaa agagaatatc 4800
   cggcaggaga ttagcatcat gaactgcctc caccacccta agctggtcca gtgtgtggat 4860
   gcctttgaag aaaaggccaa catcgtcatg gtcctggaga tcgtgtcagg aggggagctg 4920
   tttgagcgca tcattgacga ggactttgag ctgacggagc gtgagtgcat caagtacatg 4980
   cggcagatct cggagggagt ggagtacatc cacaagcagg gcatcgtgca cctggacctc 5040
   aagccggaga acatcatgtg tgtcaacaag acgggcacca ggatcaagct catcgacttt 5100
   ggtctggcca ggaggctgga gaatgcgggg tctctgaagg tcctctttgg caccccagaa 5160
   tttgtggctc ctgaagtgat caactatgag cccatcggct acgccacaga catgtggagc 5220
   atcggggtca tctgctacat cctagtcagt ggcctttccc ccttcatggg agacaacgat 5280
   aacgaaacct tggccaacgt tacctcagcc acctgggact tcgacgacga ggcattcgat 5340
   gagatctccg acgatgccaa ggatttcatc agcaatctgc tgaagaaaga tatgaaaaac 5400
   cgcctggact gcacgcagtg ccttcagcat ccatggctaa tgaaagatac caagaacatg 5460
   gaggccaaga aactctccaa ggaccggatg aagaagtaca tggcaagaag gaaatggcag 5520
   aaaacgggca atgctgtgag agccattgga agactgtcct ctatggcaat gatctcaggg 5580
   ctcagtggca ggaaatcctc aacagggtca ccaaccagcc cgctcaatgc agaaaaacta 5640
   gaatctgaag aagatgtgtc ccaagctttc cttgaggctg ttgctgagga aaagcctcat 5700
   gtaaaaccct atttctctaa gaccattcgc gatttagaag ttgtggaggg aagtgctgct 5760
   agatttgact gcaagattga aggataccca gaccccgagg ttgtctggtt caaagatgac 5820
   cagtcaatca gggagtcccg ccacttccag atagactacg atgaggacgg gaactgctct 5880
   ttaattatta gtgatgtttg cggggatgac gatgccaagt acacctgcaa ggctgtcaac 5940
   agtcttggag aagccacctg cacagcagag ctcattgtgg aaacgatgga ggaaggtgaa 6000
   ggggaagggg aagaggaaga agagtgaaac aaagccagag aaaagcagtt tctaagtcat 6060
   attaaaagga ctatttctct aaaactcaaa aaaaaaaaaa aaactcaaga tagtaaaagc 6120
   acctagtgtg atagattatc ggttaggtca tttgtgggtt gattcttcag aaacagcagt 6180
   tgatacctag cagcgttatt gatgggcatt aatctatgtt agttggcacc ttaagatact 6240
   agtgcagcta gatttcattt agggaaatca ccagtaactt gactgaccaa ttgattttag 6300
   agagaaagta accaaaccaa atatttatct gggcaaagtc ataaattctc cacttgaatg 6360
   cgctcatgaa aaataaggcc aaaacaagag ttctgggcca cagctcagcc cagagggttc     6420
   ctggggatgg gaggcctctc tctccccacc ccctgactct agagaactgg gttttctccc     6480
   agtactccag caattcattt ctgaaagcag ttgagccact ttattccaaa gtacactgca     6540
   gatgttcaaa ctctccattt ctctttcccc ttccacctgc cagttttgct gactctcaac     6600
   ttgtcatgag tgtaagcatt aaggacatta tgcttcttcg attctgaaga caggtccctg     6660
   ctcatggatg actctggctt ccttaggaaa atatttttct tccaaaatca gtaggaaatc     6720
   taaacttatc ccctctttgc agatgtctag cagcttcaga catttggtta agaacccatg     6780
   ggaaaaaaaa aatccttgct aatgtggttt cctttgtaaa ccaggattct tatttgtgct     6840
   gttatagaat atcagctctg aacgtgtggt aaagattttt gtgtttgaat ataggagaaa     6900
   tcagtttgct gaaaagttag tcttaattat ctattggcca cgatgaaaca gatttcaact     6960
   gataaagagc tggagaactc catgtacttt ggaatctcct ccaagatagc cagagtttaa     7020
   tacatcttca ttctcaacac tctccaaaga acttgaccta ccttatgggt tccatatttt     7080
   tcttcttaaa tgtgcatcaa tcatgccttg cccccaacct ttaaatatat tcttagacct     7140
   ggtaaatgca ctcagacttg cgtctttagg aatttttaac tttctttcac tacattggca     7200
   cttaaatttt ttctttataa agctttttga aggtcataaa caaagaccat aattgatgat     7260
   agacctaata catttcctct gtgtgtgtgt gtaacattcc aaatactttt tttttctttt     7320
   ccactgtttg taaggtgcaa caatttaata tttttaaggg actttttaag agttccttaa     7380
   gaaccaattt aaaattactt cagtgcaatc ctacacagta tcaacattag aattttgata     7440
   ttagtcttat gttatcttcc attctatttt tatctgcttt ttgctgctag tttcaaactg     7500
   ccagtatttt tccttttgct tttaaaatag ttacaatatt tttcatgata gccacagtat     7560
   tgccacagtt tattataata aagggttttt atttgattta gcgcattcaa agcttttttc     7620
   tatcactttt gtgttcagaa tataaccttt gtgtgcgtgt atgttgtgtg tgtgcatgtg     7680
   tggcgtatat gtgtgttaca ggttaatgcc ttcttggaat tgtgttaatg ttctcttggt     7740
   ttattatgcc atcagaatgg taaatgagaa cactacaact gtagtcagct cacaattttt     7800
   aaataaagga taccacagtg catgctgttt gttcaaaaaa aaaaaaaaaa aa             7852
<210> 66
   <211> 1545
   <212> DNA
   <213> Homo sapiens
<400> 66
   tttccgcgag cgccggcact gcccgctccg agcccgtgtc tgtcgggtgc cgagccaact       60
   ttcctgcgtc catgcagccc cgccggcaac ggctgcctgc tccctggtcc gggcccaggg      120
   gcccgcgccc caccgccccg ctgctcgcgc tgctgctgtt gctcgccccg gtggcggcgc      180
   ccgcggggtc cggggacccc gacgaccctg ggcagcctca ggatgctggg gtcccgcgca      240
   ggctcctgca gcaggcggcg cgcgcggcgc ttcacttctt caacttccgg tccggctcgc      300
   ccagcgcgct acgagtgctg gccgaggtgc aggagggccg cgcgtggatt aatccaaaag      360
   agggatgtaa agttcacgtg gtcttcagca cagagcgcta caacccagag tctttacttc      420
   aggaaggtga gggacgtttg gggaaatgtt ctgctcgagt gtttttcaag aatcagaaac      480
   ccagaccaac catcaatgta acttgtacac ggctcatcga gaaaaagaaa agacaacaag      540
   aggattacct gctttacaag caaatgaagc aactgaaaaa ccccttggaa atagtcagca      600
   tacctgataa tcatggacat attgatccct ctctgagact catctgggat ttggctttcc      660
   ttggaagctc ttacgtgatg tgggaaatga caacacaggt gtcacactac tacttggcac      720
   agctcactag tgtgaggcag tggaaaacta atgatgatac aattgatttt gattatactg      780
   ttctacttca tgaattatca acacaggaaa taattccctg tcgcattcac ttggtctggt      840
   accctggcaa acctcttaaa gtgaagtacc actgtcaaga gctacagaca ccagaagaag      900
   cctccggaac tgaagaagga tcagctgtag taccaacaga gcttagtaat ttctaaaaag      960
   aaaaaatgat ctttttccga cttctaaaca agtgactata ctagcataaa tcattcttct     1020
   agtaaaacag ctaaggtata gacattctaa taatttggga aaacctatga ttacaagtaa     1080
   aaactcagaa atgcaaagat gttggttttt tgtttctcag tctgctttag cttttaactc     1140
   tggaagcgca tgcacactga actctgctca gtgctaaaca gtcaccagca ggttcctcag     1200
   ggtttcagcc ctaaaatgta aaacctggat aatcagtgta tgttgcacca gaatcagcat     1260
   ttttttttta actgcaaaaa atgatggtct catctctgaa tttatatttc tcattctttt     1320
   gaacatacta tagctaatat attttatgtt gctaaattgc ttctatctag catgttaaac     1380
   aaagataata tactttcgat gaaagtaaat tataggaaaa aaattaactg ttttaaaaag     1440
   aacttgatta tgttttatga tttcaggcaa gtattcattt ttaacttgct acctactttt     1500
   aaataaatgt ttacatttct aaataaaaaa aaaaaaaaaa aaaaa                     1545
<210> 67
   <211> 739
   <212> DNA
   <213> Homo sapiens
<400> 67
   cgtggcgcag cgactcggag gttcgcctcc agcttgcgca tcatctgcgg ccgggtcccg      60
   atgagcctcc tgttgcctcc gctggcgctg ctgctgcttc tcgcggcgct tgtggcccca     120
   gccacagccg ccactgccta ccggccggac tggaaccgtc tgagcggcct aacccgcgcc     180
   cgggtagaga cctgcggggg atgacagctg aaccgcctaa aggaggtgaa ggctttcgtc     240
   acgcaggaca ttccattcta tcacaacctg gtgatgaaac acctccctgg ggccgaccct     300
   gagctcgtgc tgctgggccg ccgctacgag gaactagagc gcatcccact cagtgaaatg     360
   acccgcgaag agatcaatgc gctagtgcag gagctcggct tctaccgcaa ggcggcgccc     420
   gacgcgcagg tgccccccga gtacgtgtgg gcgcccgcga agcccccaga ggaaacttcg     480
   gaccacgctg acctgtaggt ccgggggcgc ggcggagctg ggacctacct gcctgagtcc     540
   tggagacaga atgaagcgct cagcatcccg ggaatacttc tcttgctgag agccgatgcc     600
   cgtccccggg ccagcaggga tggggttggg gaggttctcc caaccccact ttcttccttc     660
   cccagctcca ctaaattccc tcctgcctta aaaaaaaaaa aaaaaaaaaa aaaaaaaaaa     720
   aaaaaaaaaa aaaaaaaaa                                                  739
<210> 68
   <211> 4482
   <212> DNA
   <213> Homo sapiens
<400> 68
   gcactccagc cctgcagcct ccggagtcag tgccgcgcgc ccgccgcccc gcgccttcct 60
   gctcgccgca cctccgggag ccggggcgca cccagcccgc agcgccgcct ccccgcccgc 120
   gccgcctccg accgcaggcc gagggccgcc actggccggg gggaccgggc agcagcttgc 180
   ggccgcggag ccgggcaacg ctggggactg cgccttttgt ccccggaggt ccctggaagt 240
   ttgcggcagg acgcgcgcgg ggaggcggcg gaggcagccc cgacgtcgcg gagaacaggg 300
   cgcagagccg gcatgggcat cgggcgcagc gaggggggcc gccgcggggc agccctgggc 360
   gtgctgctgg cgctgggcgc ggcgcttctg gccgtgggct cggccagcga gtacgactac 420
   gtgagcttcc agtcggacat cggcccgtac cagagcgggc gcttctacac caagccacct 480
   cagtgcgtgg acatccccgc ggacctgcgg ctgtgccaca acgtgggcta caagaagatg 540
   gtgctgccca acctgctgga gcacgagacc atggcggagg tgaagcagca ggccagcagc 600
   tgggtgcccc tgctcaacaa gaactgccac gccggcaccc aggtcttcct ctgctcgctc 660
   ttcgcgcccg tctgcctgga ccggcccatc tacccgtgtc gctggctctg cgaggccgtg 720
   cgcgactcgt gcgagccggt catgcagttc ttcggcttct actggcccga gatgcttaag 780
   tgtgacaagt tccccgaggg ggacgtctgc atcgccatga cgccgcccaa tgccaccgaa 840
   gcctccaagc cccaaggcac aacggtgtgt cctccctgtg acaacgagtt gaaatctgag 900
   gccatcattg aacatctctg tgccagcgag tttgcactga ggatgaaaat aaaagaagtg 960
   aaaaaagaaa atggcgacaa gaagattgtc cccaagaaga agaagcccct gaagttgggg 1020
   cccatcaaga agaaggacct gaagaagctt gtgctgtacc tgaagaatgg ggctgactgt 1080
   ccctgccacc agctggacaa cctcagccac cacttcctca tcatgggccg caaggtgaag 1140
   agccagtact tgctgacggc catccacaag tgggacaaga aaaacaagga gttcaaaaac 1200
   ttcatgaaga aaatgaaaaa ccatgagtgc cccacctttc agtccgtgtt taagtgattc 1260
   tcccgggggc agggtgggga gggagcctcg ggtggggtgg gagcgggggg gacagtgccc 1320
   cgggaacccg gtgggtcaca cacacgcact gcgcctgtca gtagtggaca ttgtaatcca 1380
   gtcggcttgt tcttgcagca ttcccgctcc cttccctcca tagccacgct ccaaacccca 1440
   gggtagccat ggccgggtaa agcaagggcc atttagatta ggaaggtttt taagatccgc 1500
   aatgtggagc agcagccact gcacaggagg aggtgacaaa ccatttccaa cagcaacaca 1560
   gccactaaaa cacaaaaagg gggattgggc ggaaagtgag agccagcagc aaaaactaca 1620
   ttttgcaact tgttggtgtg gatctattgg ctgatctatg cctttcaact agaaaattct 1680
   aatgattggc aagtcacgtt gttttcaggt ccagagtagt ttctttctgt ctgctttaaa 1740
   tggaaacaga ctcataccac acttacaatt aaggtcaagc ccagaaagtg ataagtgcag 1800
   ggaggaaaag tgcaagtcca ttatgtaata gtgacagcaa agggaccagg ggagaggcat 1860
   tgccttctct gcccacagtc tttccgtgtg attgtctttg aatctgaatc agccagtctc 1920
   agatgcccca aagtttcggt tcctatgagc ccggggcatg atctgatccc caagacatgt 1980
   ggaggggcag cctgtgcctg cctttgtgtc agaaaaagga aaccacagtg agcctgagag 2040
   agacggcgat tttcgggctg agaaggcagt agttttcaaa acacatagtt aaaaaagaaa 2100
   caaatgaaaa aaattttaga acagtccagc aaattgctag tcagggtgaa ttgtgaaatt 2160
   gggtgaagag cttaggattc taatctcatg ttttttcctt ttcacatttt taaaagaaca 2220
   atgacaaaca cccacttatt tttcaaggtt ttaaaacagt ctacattgag catttgaaag 2280
   gtgtgctaga acaaggtctc ctgatccgtc cgaggctgct tcccagagga gcagctctcc 2340
   ccaggcattt gccaagggag gcggatttcc ctggtagtgt agctgtgtgg ctttccttcc 2400
   tgaagagtcc gtggttgccc tagaacctaa caccccctag caaaactcac agagctttcc 2460
   gtttttttct ttcctgtaaa gaaacatttc ctttgaactt gattgcctat ggatcaaaga 2520
   aattcagaac agcctgcctg tccccccgca ctttttacat atatttgttt catttctgca 2580
   gatggaaagt tgacatgggt ggggtgtccc catccagcga gagagtttca aaagcaaaac 2640
   atctctgcag tttttcccaa gtaccctgag atacttccca aagcccttat gtttaatcag 2700
   cgatgtatat aagccagttc acttagacaa ctttaccctt cttgtccaat gtacaggaag 2760
   tagttctaaa aaaaatgcat attaatttct tcccccaaag ccggattctt aattctctgc 2820
   aacactttga ggacatttat gattgtccct ctgggccaat gcttataccc agtgaggatg 2880
   ctgcagtgag gctgtaaagt ggccccctgc ggccctagcc tgacccggag gaaaggatgg 2940
   tagattctgt taactcttga agactccagt atgaaaatca gcatgcccgc ctagttacct     3000
   accggagagt tatcctgata aattaacctc tcacagttag tgatcctgtc cttttaacac     3060
   cttttttgtg gggttctctc tgacctttca tcgtaaagtg ctggggacct taagtgattt     3120
   gcctgtaatt ttggatgatt aaaaaatgtg tatatatatt agctaattag aaatattcta     3180
   cttctctgtt gtcaaactga aattcagagc aagttcctga gtgcgtggat ctgggtctta     3240
   gttctggttg attcactcaa gagttcagtg ctcatacgta tctgctcatt ttgacaaagt     3300
   gcctcatgca accgggccct ctctctgcgg cagagtcctt agtggagggg tttacctgga     3360
   acattagtag ttaccacaga atacggaaga gcaggtgact gtgctgtgca gctctctaaa     3420
   tgggaattct caggtaggaa gcaacagctt cagaaagagc tcaaaataaa ttggaaatgt     3480
   gaatcgcagc tgtgggtttt accaccgtct gtctcagagt cccaggacct tgagtgtcat     3540
   tagttacttt attgaaggtt ttagacccat agcagctttg tctctgtcac atcagcaatt     3600
   tcagaaccaa aagggaggct ctctgtaggc acagagctgc actatcacga gcctttgttt     3660
   ttctccacaa agtatctaac aaaaccaatg tgcagactga ttggcctggt cattggtctc     3720
   cgagagagga ggtttgcctg tgatttccta attatcgcta gggccaaggt gggatttgta     3780
   aagctttaca ataatcattc tggatagagt cctgggaggt ccttggcaga actcagttaa     3840
   atctttgaag aatatttgta gttatcttag aagatagcat gggaggtgag gattccaaaa     3900
   acattttatt tttaaaatat cctgtgtaac acttggctct tggtacctgt gggttagcat     3960
   caagttctcc ccagggtaga attcaatcag agctccagtt tgcatttgga tgtgtaaatt     4020
   acagtaatcc catttcccaa acctaaaatc tgtttttctc atcagactct gagtaactgg     4080
   ttgctgtgtc ataacttcat agatgcagga ggctcaggtg atctgtttga ggagagcacc     4140
   ctaggcagcc tgcagggaat aacatactgg ccgttctgac ctgttgccag cagatacaca     4200
   ggacatggat gaaattcccg tttcctctag tttcttcctg tagtactcct cttttagatc     4260
   ctaagtctct tacaaaagct ttgaatactg tgaaaatgtt ttacattcca tttcatttgt     4320
   gttgtttttt taactgcatt ttaccagatg ttttgatgtt atcgcttatg ttaatagtaa     4380
   ttcccgtacg tgttcatttt attttcatgc tttttcagcc atgtatcaat attcacttga     4440
   ctaaaatcac tcaattaatc aatgataaaa aaaaaaaaaa aa                        4482
<210> 69
   <211> 7355
   <212> DNA
   <213> Homo sapiens
<400> 69
   agtctgcggg cctccggggc agcggcgagg ccggagcgtc gcggcggaga ggacgagacc      60
   gggacaagac cagggcagga gggagccggc cagccgcgag aaccccgcac gcccggcaag     120
   atgctgtcct ggcggctgca gacgggcccc gagaaggccg agctccagga gctcaacgcc     180
   cggctctatg actacgtgtg tcgggtgcgg gagctggagc gcgaaaacct actcctggag     240
   gaggagctgc gcggccggcg cgggcgagag ggcctgtggg ccgaggggca ggcccgctgc     300
   gccgaggagg cgcgcagctt gcggcagcag ctggacgagc tgagctgggc cactgcgctg     360
   gcggagggcg agcgggacgc tctgcggcgc gagctgcggg agctgcagcg cctggatgcg     420
   gaggagcgcg ccgcccgcgg ccgcctggac gccgagctgg gtgcgcagca gcgcgagctg     480
   caggaggcgc tgggcgcgcg cgccgccctc gaggcgctgc tgggccggct gcaggccgag     540
   cgccgaggcc tcgacgcggc ccacgaacgc gacgtgaggg agctgcgcgc gcgcgccgcc     600
   agccttacca tgcatttccg cgcccgcgcc accggccccg ccgcgccgcc gccacgcctg     660
   cgggaggtgc acgacagcta cgcactgctg gtggccgagt cgtggcggga gacggtgcag     720
   ctgtacgagg acgaggtgcg cgagctggag gaggcgctgc ggcgcggcca ggagagcaga     780
   ctccaggcgg aggaagagac gcggctgtgc gcgcaggagg cagaggcgct gcggcgcgag     840
   gcgctcgggt tggagcagct gcgcgcgcgg ctggaggacg cgctgctgcg gatgcgcgag     900
   gagtacggga tacaggccga ggagcggcag agagtgattg actgcctgga ggatgagaag     960
   gcaaccctca ccttggccat ggctgactgg ctgcgggact atcaggacct cctgcaggtg    1020
   aagaccggcc tcagtctgga ggtggcgacc taccgggcct tattggaagg agaaagtaat    1080
   ccagagatag tgatctgggc tgagcacgtt gaaaacatgc cgtcagaatt cagaaacaaa    1140
   tcctatcact ataccgactc actactacag agggaaaatg aaaggaatct attttcaagg    1200
   cagaaagcac ctttggcaag tttcaatcac agctcggcac tgtattctaa cctgtcaggg    1260
   caccgtggat ctcagacggg cacatctatt ggaggtgatg ccagaagagg cttcttgggc    1320
   tcgggatatt cttcctcggc cactacccag caggaaaact catacggaaa agccgtcagc    1380
   agtcaaacca acgtcagaac tttctctcca acctatggcc ttttaagaaa tactgaggct    1440
   caagtgaaaa cattccctga cagaccaaaa gccggagata caagggaggt ccccgtttac    1500
   ataggtgaag attccacaat tgcccgcgag tcgtaccggg atcgccgaga caaggtggca    1560
   gcaggtgctt cggaaagcac acggtcaaat gagaggaccg tcattctggg aaagaaaaca    1620
   gaagtgaaag ccacgaggga gcaagaaaga aacagaccag aaaccatccg aacaaagcca    1680
   gaagagaaaa tgttcgattc taaagagaag gcttccgagg agagaaacct aagatgggaa    1740
   gaattgacaa agttagataa ggaagcgaga cagagagaaa gccagcagat gaaggagaag    1800
   gctaaggaga aggactcacc gaaggagaag agcgtgcgag agagagaggt gccgattagt    1860
   ctagaagtat cccaggacag aagagcagag gtgtccccga aaggtttgca gacgcctgtg    1920
   aaggatgctg gtggtgggac cggtagagag gcagaagcaa gagagctacg gttcaggttg    1980
   ggcaccagtg atgccactgg ttctctgcaa ggcgattcca tgacagaaac cgtagcagaa    2040
   aacatcgtta ccagtatcct gaagcagttc actcagtctc cagagacaga agcatctgct    2100
   gattcttttc cagacacaaa agtcacttac gtggacagga aagagcttcc tggggaaagg    2160
   aaaacaaaga ctgaaatagt tgtggagtct aaactgactg aggatgttga tgtttccgat    2220
   gaagctggcc tggactacct tttaagcaag gatattaagg aagtggggct gaaaggcaag    2280
   tcagccgagc agatgatagg agacatcatc aacctcggcc tgaaagggag ggaggggaga    2340
   gcaaaggtcg tcaacgtgga gatcgtggag gagcccgtga gttatgtcag cggggagaag    2400
   ccggaggagt tttccgtccc attcaaagtg gaggaggtcg aagatgtgtc gccaggcccc    2460
   tgggggttgg ttaaggagga ggaaggttat ggagaaagcg atgtcacatt ctcagttaat    2520
   cagcatcgaa ggaccaagca gcctcaggag aacacgactc acgtggaaga agtgacagag    2580
   gcaggtgatt cagagggcga gcagagttat tttgtgtcca ctccagatga acaccccggg    2640
   gggcacgaca gagatgacgg ctcggtgtac gggcagatcc acatcgagga ggaatccacc    2700
   atcaggtact cttggcagga tgaaatcgtg caggggactc gaaggaggac acagaaggac    2760
   ggtgcagtgg gcgagaaggt tgtgaagccc ttggatgtcc cagcgccctc tctggagggg    2820
   gacctgggtt ccactcactg gaaagaacaa gctagaagcg gtgaatttca tgccgaaccc    2880
   acagtcattg aaaaagaaat taaaataccc cacgaattcc acacctccat gaagggcatc    2940
   tcctccaagg agccccggca gcagctggtg gaggtcatcg ggcagctgga ggaaaccctt    3000
   cccgagcgca tgagggagga gctgtccgcc ctcaccagag aggggcaggg tgggccgggg    3060
   agcgtttccg tggatgtcaa gaaggtccag ggtgctggtg gcagttccgt gaccctggtt    3120
   gctgaagtca acgtctcaca aactgtggat gccgatcggt tagacctgga ggagctgagc    3180
   aaagatgagg ccagtgagat ggagaaggct gtggagtcgg tggttcggga gagcctgagc    3240
   aggcaacgca gcccagcgcc tggcagccca gatgaggaag gtggagcgga ggccccggct    3300
   gctggcattc gctttaggcg ttgggccacc cgggagctgt acatcccttc aggcgagagc    3360
   gaggttgctg gtggggcctc tcacagctcg ggacagcgca ctccccaggg cccagtgtcg    3420
   gccactgtgg aggtcagcag ccccacaggc tttgcccagt cacaggtgct ggaggatgtg    3480
   agccaggctg caaggcacat aaaactcggc ccctctgaag tctggaggac tgagcgaatg    3540
   tcatatgaag gacccactgc agaagtggtg gaggtaagtg cgggaggtga cctaagtcag    3600
   gcagcgagcc cgaccggagc cagccggtct gtgaggcatg tcacgctggg tcccggtcaa    3660
   agtccactgt ccagagaagt catcttccta ggccctgccc ctgcctgtcc agaggcatgg    3720
   ggctcgccag aacctggccc agcagagtct tctgcagata tggacggatc agggaggcac    3780
   agcacatttg gctgcagaca atttcatgct gaaaaggaga ttatttttca gggccccatt    3840
   tctgctgcag ggaaggttgg tgattatttt gcaacagaag agtcagtggg tacccagact    3900
   tctgtcaggc aactccagtt aggccctaaa gaagggttca gtgggcaaat ccagttcaca    3960
   gctccacttt cagacaaggt ggagttgggt gtcataggag attctgtaca catggaaggg    4020
   ttgccaggga gcagcacatc catcaggcac atcagcattg ggcctcagag gcatcagacc    4080
   acccagcaga tagtttacca tgggctggtt ccccaactgg gggaatctgg tgactcagag    4140
   agcactgtgc acggagaggg ctcagcagat gtgcaccagg ccactcacag tcatacctcg    4200
   ggtagacaaa ccgttatgac tgaaaagagc accttccaaa gtgtcgtttc tgaatctccc    4260
   caggaggata gtgcagagga cacatcaggg gcagaaatga catcgggtgt tagcagatcc    4320
   tttaggcaca ttcgactagg tcctacagaa acggaaacct ctgaacacat tgccatccgt    4380
   ggacccgtgt ccagaacatt tgtgcttgct ggttcagcgg actcccctga gctaggcaag    4440
   ttagcagaca gcagcagaac gctaaggcac attgcaccag ggcccaaaga aacttcgttt    4500
   acctttcaga tggatgtgag taacgtagag gcgatccgca gccggacaca ggaagcggga    4560
   gctctcggtg tgtctgaccg tggttcctgg agagacgcgg acagtaggaa tgaccaggca    4620
   gttggtgtga gctttaaggc ctctgctggg gaaggagacc aggcccacag agaacagggc    4680
   aaggagcagg ccatgtttga taagaaggtg cagctccaga gaatggtaga ccaaaggtcg    4740
   gtgatttcag atgaaaagaa agttgccctc ctctatctag acaatgagga ggaggagaat    4800
   gatgggcatt ggttttaata agcagaaaca ttttgtttta atggcagcct gttggcgacg    4860
   tgccaacatc caaaggcctt aacttatttt aagaggccga gggagtctat gaaaatctcc    4920
   ccttttttac ttttttaaag agtactcccg gcatggtcaa tttcctttat agttaatccg    4980
   taaaggtttc cagttaattc atgccttaaa aggcactgca attttatttt tgagttggga    5040
   cttttacaaa acactttttt ccctggagtc ttctctccac ttctggagat gaatttctat    5100
   gttttgcacc tggtcacaga catggcttgc atctgtttga aactacaatt aattatagat    5160
   gtcaaaacat taaccagatt aaagtaatat atttaagagt aaattttgct tgcatgtgct    5220
   aatatgaaat aacagactaa cattttaggg gaaaaataaa tacaatttag actctaaaaa    5280
   gtcttttcaa aaagaaatgg gaaataggca gactgtttat gttaaaaaaa ttcttgctaa    5340
   atgatttcat ctttaggaaa aaattacttg ccatatagag ctaaattcat cttaagactt    5400
   gaatgaattg ctttctatgt acagaacttt aaacaatata gtatttatgg cgaggacagc    5460
   tgtagtctgt tgtgatattt cacattctat ttgcacaggt tccctggcac tggtagggta    5520
   gatgattatt gggaatcgct tacagtacca tttcattttt tggcactagg tcattaagta    5580
   gcacacagtc tgaatgccct tttctggagt ggccagttcc tatcagactg tgcagacttg    5640
   cgcttctctg caccttatcc cttagcaccc aaacatttaa tttcactggt gggaggtaga    5700
   ccttgaagac aatgaagaga atgccgatac tcagactgca gctggaccgg caagctggct    5760
   gtgtacagga aaattggaag cacacagtgg actgtgcctc ttaaagatgc ctttcccaac    5820
   cctccattca tgggatgcag gtctttctga gctcaagggt gaaagatgaa tacaataaca    5880
   accatgaacc cacctcacgg aagctttttt tgcactttga acagaagtca ttgcagttgg    5940
   ggtgttttgt ccagggaaac agtttattaa atagaaggat gttttgggga aggaactgga    6000
   tatctctcct gcagcccagc accgagatac ccaggacggg cctggggggc gagaaaggcc    6060
   cccatgctca tgggccgcgg agtgtggacc tgtagatagg caccaccgag tttaagatac    6120
   tgggatgagc atgcttcatt ggattcattt tattttacac gtcagtattg ttttaaagtt    6180
   tctgtctgta aagtgtagca tcatatataa aaagagtttc gctagcagcg catttttttt 6240
   agttcaggct agcttctttc acataatgct gtctcagctg tatttccagt aacacagcat 6300
   catcgcactg actgtggcgc actggggaat aacagtctga gctagcacca ccctcagcca 6360
   ggctacaacg acagcactgg agggtcttcc ctctcagatt cacctggagg ccctcagacc 6420
   cccagggtgc acgtctcccc aggtcctggg agtggctacc gcaggtagtt tctggagagc 6480
   acgttttctt cattgataag tggaggagaa atgcagcaca gctttcaaga tactatttta 6540
   aaaacaccat gaatcagata gggaaagaaa gttgattgga atagcaagtt taaacctttg 6600
   ttgtccatct gccaaatgaa ctagtgattg tcagactggt atggaggtga ctgctttgta 6660
   aggttttgtc gtttctaata cagacagaga tgtgctgatt ttgttttagc tgtaacaggt 6720
   aatggttttt ggatagatga ttgactggtg agaatttggt caaggtgaca gcctcctgtc 6780
   tgatgacagg acagactggt ggtgaggagt ctaagtgggc tcagtttgat gtcagtgtct 6840
   gggctcatga cttgtaaatg gaagctgatg tgaacaggta attaatatta tgacccactt 6900
   ctatttactt tgggaaatat cttggatctt aattatcatc tgcaagtttc aagaagtatt 6960
   ctgccaaaag tatttacaag tatggactca tgagctattg ttggttgcta aatgtgaatc 7020
   acgcgggagt gagtgtgccc ttcacactgt gacattgtga cattgtgaca agctccatgt 7080
   cctttaaaat cagtcactct gcacacaaga gaaatcaact tcgtggttgg atggggccgg 7140
   aacacaacca gtctttttgt atttattgtt actgagacaa aacagtactc actgagtgtt 7200
   tttcagtttc ctactggtgg ttttgatatt gtttgtttaa gatgtatatt tagaatgaca 7260
   tcatctaaga agctgatttt gctaaactcc tgttccctac aatgggaaat gtcacaagaa 7320
   tgtgcaaaaa taaaaatctg aggaaaaaac ccaca 7355
<210> 70
   <211> 9950
   <212> DNA
   <213> Homo sapiens
<400> 70
   gagcccatta gccgcacaaa ttcgcagcag gcggctgggg cggcggctgg ggcagcggct      60
   gcagcagcgg cggacgctct gcattaccca gtcttgcgtc ctcggcaggc gcccgaagct     120
   gagtgcgcat cctctaccgc acccaagctt cgtctgtctc gtcaagctct tcatgctgcc     180
   caactaaaag gaaaacatgg gcacagggga ttttatctgc atttccatga ctggaggggc     240
   gccctggggg ttcagattgc aaggtggcaa ggagcagaag cagcccttac aagttgcaaa     300
   gattcgaaat cagagcaaag cctctgggtc tgggctctgt gagggagatg aagtggtttc     360
   catcaatggc aacccttgtg cagatctcac ctaccctgaa gtcatcaagc tcatggaaag     420
   cataacagac tctctccaaa tgctcatcaa aagaccatcc agtggaataa gtgaggcttt     480
   gatatctgaa aatgaaaaca aaaacctcga gcatctcaca catgggggtt atgtggaaag      540
   taccaccctg cagattcgac cggccacaaa gacccagtgc acagaattct tcctcgcccc     600
   tgtcaagact gaagttcccc tagctgagaa ccaaagaagt ggtcccgact gtgcaggcag     660
   cttgaaagaa gaaacaggcc cgagctacca aagggctccc caaatgcctg actcccaaag     720
   aggacgcgtg gcagaagagc tgatcttaag ggagaaggta gaagcggtac agcctgggcc     780
   tgtggttgag ctgcaactgt ccctttcaca ggagagacat aagggcgcta gtggcccttt     840
   agtggctctc ccgggagctg aaaaatctaa gtctcctgac ccagacccta acttgtcaca     900
   tgacaggatt gtccacataa attcgatccc tactaatgag aaagcagacc ctttcctgag     960
   gtccagcaag ataatccaga tctccagtgg cagagagttg agagtgatcc aggaaagtga    1020
   agcaggagat gcgggactgc cccgggtgga agtgatcctc gactgctctg acaggcagaa    1080
   gacagaaggg tgcaggcttc aggcaggaaa ggagtgtgtg gattctccag tggaaggagg    1140
   gcagtcagaa gcacctcctt ctctggtatc ctttgccgtc tcatcagaag gcacagagca    1200
   gggagaagat ccacgctcgg aaaaagatca cagcagacct cacaagcacc gagcgcggca    1260
   tgcacggctc aggaggagtg aaagcctgtc agaaaaacaa gtgaaggaag caaaatctaa    1320
   atgcaaaagc attgcccttc ttctaacgga tgctcccaac cccaactcca agggggtgtt     1380
   gatgtttaag aagcgacgtc ggagggccag gaaatacacc ctagttagct acggtactgg    1440
   cgagcttgag cgagaggcgg acgaggagga agaaggtgac aaggaggata catgtgaagt    1500
   agcatttctt ggtgcaagcg aatcagaggt ggatgaagag ttattgtctg acgttgacga    1560
   caacacacaa gttgtgaact ttgactggga ttctggactg gtggacattg aaaagaaact    1620
   gaacagaggg gacaagatgg agatgttacc agacaccaca ggcaagggag ccctcatgtt    1680
   tgccaagagg agggagagaa tggatcagat cacagcccaa aaagaagagg acaaggtagg    1740
   tggaacgcca agcagagaac aagatgctgc ccagaccgat ggcctgagaa ccacgacttc    1800
   ttaccaaaga aaggaggaag agtcggtaag aacgcagagc tctgtgagca aaagctacat    1860
   cgaggtgagt catggtcttg gccatgttcc ccaacagaat ggcttcagtg ggacatctga    1920
   gacagcaaac atccagagga tggtccccat gaatagaacg gccaaaccct tcccagggtc    1980
   tgtgaatcag ccagctaccc ccttctcgcc aacccgaaac atgacgagtc ccattgctga    2040
   ctttcctgca cctccacctt actctgcagt cactcctccc cctgacgcct tctccagagg    2100
   ggtttcaagt ccgattgctg gcccagcaca gccccctcca tggccccagc ctgccccgtg    2160
   gtcccagcca gccttttacg attcgtctga gcgaatagct tcccgagatg agaggatctc    2220
   agtgccagca aaaagaacag gaatattgca ggaggccaaa aggagaagca cgacaaaacc    2280
   catgtttact tttaaagagc ccaaagtaag cccaaatcct gaactcttgt cactccttca    2340
   aaattcagaa ggcaaacggg gcactggagc tggaggtgat tccggaccgg aagaagacta 2400
   cctcagcttg ggggcagagg cttgtaattt catgcaaagc tcctctgcca aacaaaagac 2460
   ccctcctcct gttgctccaa aacctgcagt caagtcctca tcctcccaac cagtaactcc 2520
   agtttcccca gtctggtctc caggagtggc tcccacccaa cctcctgcct tccccacatc 2580
   caacccatca aagggcaccg ttgtctcctc catcaaaata gcccagcctt cttaccctcc 2640
   tgcccggcct gcaagtactt tgaacgtggc tggtcccttc aaaggaccac aagcagcagt 2700
   agccagtcag aattacacac ccaaaccaac agtttccaca ccaacagtca atgctgttca 2760
   gcctggtgca gtgggaccat ccaatgagct tccaggaatg agtgggagag gagctcagct 2820
   ctttgctaaa aggcagtcga gaatggagaa gtatgtggtc gattcagaca cggtgcaggc 2880
   ccacgctgct cgagctcagt ctcccactcc atctctcccg gccagttgga agtactcctc 2940
   caatgtccga gcacctcctc ctgtggccta taatcctatc cactcgccgt cttacccact 3000
   ggctgctctc aagtctcagc catcagctgc acagccctcc aaaatgggca agaaaaaggg 3060
   aaagaaaccc ctcaatgcat tagatgtcat gaagcaccaa ccgtatcagc tcaatgcatc 3120
   cttgtttact ttccaacctc cagatgcaaa ggatggcctc ccccagaagt catcagtcaa 3180
   ggtcaattca gccctggcca tgaagcaagc tcttcctccc cggccagtga atgctgcctc 3240
   acctacgaat gtgcaggctt cgtcagtgta ctcggtacca gcctatacct ctcctccttc 3300
   cttctttgca gaggcctcct caccagtcag tgcatcccca gtgcctgtgg gcattcccac 3360
   ctcgccaaag caagaatcag cctcatcatc ttattttgtg gcaccaaggc caaagttctc 3420
   agccaagaaa agtggtgtca caattcaggt gtggaaacca tctgttgtgg aagagtaatc 3480
   ttgtagctga agctgagtgt ccactttgct tgaaatgaat tgtttgcagt gtttcttgag 3540
   tccctgagaa tgcctagcaa agtcctcaac ttacttaatt tcagatatgt cacctcctaa 3600
   tctgggtcca aggagtataa tatttttaat gagtcaaaaa tccaactcag attgacctaa 3660
   aatatattta tcttctttgc acacttaaaa aatccaggag caccccaaaa tagacatgta 3720
   ccgttatatt aagtaagcag gagacttagg atttgtgctg tagccacaag aaagacagtg 3780
   atcagtgata tcaaacatca ggaatcagcc tttatgtaac ataacagctg tcctcctatg 3840
   gtgaaaggtt caaatgtagt gaaggtataa cctatattga ctgagatttc ccttttaggt 3900
   agtgccttat ctctattact agtgttaaag gaataaggaa tctatgaagg acagggagca 3960
   gctctggtct gtcaatctca gccacctgtt tgatatcaca gagaagatac tcggaggatt 4020
   gttggaatgt atatagttta gtaagaagtg ggtaagaaag agggtcttaa ttactgagca 4080
   cttattatgt attaggttct ttgccagatg tttttacata tataaactca tttcagaaaa 4140
   cttatttaaa gtaaatgggg ccgggtatgg tggttcatgc ctggaatcct agcactttgg 4200
   gaggctgagg taggaggact gcttgaggcc gggagttgga gaccagcctg agcaacatag 4260
   tgagaccctg tctcaataat aataataata atagtaataa tgaagtaaat gggataagga 4320
   aagaaggata attatcttta aaggttgatt cccaccctcc ctccccagtt acttaaggaa 4380
   ctaagtgagt acatctccag ttgcccatga aagcataagt ttgttttcct cagctgaggc 4440
   aagtggtaga gtatacagga taacgaagta acatgtaaaa ggcaggacgc acataaaggt 4500
   gtacatggct attgtttcac ctggagaaac cacatgattg ggacctgaag gtttactgac 4560
   tgactacagg ggctgattgt gaagcacgag gaaccccatg tgtgtggaga ctgtagggtg 4620
   agagcacaca attattagca tcatttctga gtgatctcac agattttttt tcttgtgttt 4680
   gctttgcttt ttgacaactg cttctcccac gttccttgca attctattct ctcaccttca 4740
   ctttactatt tgtattcgat ggaccaggat aattcaggca aggttacctt gtaaacttta 4800
   attggccaca caccatgttg tcacccagct ggctatgaag tgaataatgg tactgaaagt 4860
   aaacctgaag acctttctca gatctatttt aagtctgagt ctgaccaacc atggaaaata 4920
   ttcgacatga attaatgtag agaactataa agcatttatg acagctccaa gaaaaatcat 4980
   ctactctatg caggagatat gtttagagac ctctcagaaa aacttgcctg gtttgagggt 5040
   acacagtacc attttaatct tctgaaaata tctgtattcc tgctcttttt ctgctgtcac 5100
   tgtcaatctg ctatattttt cactatccta ttaaaatatt actgtctcct ttatctgttc 5160
   aatgtccata ttttaaaaaa atcttccttg tatgagctat tctgatctaa ataatttctc 5220
   tgatatttct ctatatggct cccacaacaa tttcattgtt gttagcatat ctatttctcc 5280
   atacattgta aaactgtaat ccttaggtat ttctaaaaca taaagaggag aattaagtca 5340
   gctgcagaac aatggggctg attcttctgc tttttctctg gaaaatcttt cattgctttt 5400
   ggtggaaatt tacctagagg ttacaaccac aggatgtagc ttggtctctt atttgccttt 5460
   ttgggaaacc aattaagatt aatacaggat aaaggaaaaa agcaatctat tcattatata 5520
   acacagttgt ttgtattact tgttccctgc aaaggaaatc tgttgaatgc ttgcattttg 5580
   aattcttttc taatagaaca accaaaaaag gcttcttatg gtgcagcagg aaaaaagatc 5640
   atttttatag ctttgcattc ttaacatagc atttaaagag cggcatgaat tagaggaaag 5700
   acatggaaca cacaggtagt cggtttgaga tcatcggctt aaaagtatcc taggatggta 5760
   atgacccaga agtatttcca gttgtctagt ggtgtggtat gcaggaatga gaagtgtttt 5820
   ctttccattt cctgttggac aggtggcaat cttagcagag ccactatttg gagttgataa 5880
   ctaaagatgc aaataacatg actatgcctt ctggtcatcc taggactatt tggagttctc 5940
   caaaaccttg taagaggcat gtcaggcatg cagtaaaagc atctacaact tcagctgggc 6000
   actggcagca taggtctcat cttggaccat acagtcccac tttatagaag agggtggaag 6060
   ttctccaaaa caatatccac aacaaagtct gacctcactc tgagggagat gggaagtggg 6120
   aggaagaagg actaaccagc tccctggagt aagaggaatt tgctttccct gtctgcccac 6180
   caggggctat atgtgccacc tttcaggttg gggccaagga agtgatgtca gtgtgacaga 6240
   agggagagtt agacctccag acgtcagcct ccctcccatg gggtacattt tcaatctgag 6300
   tgttgttgcc ttagctgtgt tggtattagc ttgattggtt ggtccgctgg ttatgaggtg 6360
   tagggaggca gtttttgttt agtttttagg actttgcctc ttcctttgtc cttagcataa 6420
   tttctaggca gagcatccac gaagtcggtt ttcattgcca gctcaagagc gacaatcatt      6480
   tacgagttcc tatgttatgt taggtgcctt atgtatatta tcccaaatcc actgcatggt      6540
   ttaaatacag gcactggaat ataaatgaaa aaggtcatta cagtcactga ctttctgcag      6600
   gaccttaaac atttctcttt ccacaagttt ccccttaatc atgtgtcaaa cctctcttcc      6660
   tgacgggaat gttgtgctat aatgaatctg cataacgctt gggattctag gaggaaggaa      6720
   ggttccatgg acatgtaagt acagcatatt cccctcagtc ttctaggagg gcagagtgaa      6780
   tcccagaact ggtaagattg ggaatctgag cattgccact ttaatcttag aatatttatc      6840
   attttgacac atcctgtttt ttagagagga aaacaaacac agtttctgca ttggtagtgt      6900
   aaagcatacc ttgttaggaa cgtgttttgt aagacacatt tgggttgtca ctctagagca      6960
   tgtcaaactt tgtacttcaa aatatattta gtatgattgt tagtggtaac atatatcaag      7020
   gctttgaatt aactgtttta tttaattttc acaagaagca cttattttag ccataggaaa      7080
   accaatctga gctacaaata gttctttaaa ataagcccag gttatttagc tattctagaa      7140
   agtgccgact tctttcaaga agcaggcatt gtaggacagc tgagaattat cacatagcct      7200
   aaattctagc ctggcagcaa gagtcacatc tgagatgtcc aaaaaaaaaa aaaaaacacc      7260
   tgatctacat tgaaaggggg tagactaacg tatgtgagac cattttccta tttgcagtta      7320
   caaggttaaa gaactttgaa ggtcattcgg ctgctaagag gcatgtcgaa cactctgtgt      7380
   ggctctttca cagtaaaccc tcctaagagc agaagacaca tggctgttag tgtctgcgtt      7440
   tagatttaat ttctcaaata aaggcccttg gctgcgtatc atttcatcca gttataaact      7500
   agggctcctg caagcacccc cattctaagg gtgaattatt gaaatcagtt gctatttgat      7560
   gagtcacaac tggcccagca ggcagggcat ttgaagtcat ggtcatcaaa aagaaatgat      7620
   tgtttttgga aaagctaaat gcttaaaatg cttctagagg gaagtcgtgg ggcgtgtgct      7680
   cattctcttt aaaatcaggg ttgttgagtt tgtttttaaa catttttata agttcatgag      7740
   aaaaaatata taaattctaa gaaccaacac tgtattccca gaaacatgac cctcgctggt      7800
   cttgggtcca catatcattg gactctgggg gacacaaaga tgcctgtgac actttggtgt      7860
   tgccgagtta gtcaacaatt attctgggaa aaagcagaat tgaattcttc tctagatgtc      7920
   ctaccagggt tggccaaggg ccacaaagca ggctaataaa ttcccacagg atccagacac      7980
   caggcaaaat tgctctaaga agccagttac tgtcatccct ctatggttct agaaaaaata      8040
   gtacaaaaat gacaggtcat cctatgagcg tcatgccaat gaaaccccat cttctggaga      8100
   agcccttgaa tcagaattat cttttttctt gatgtcgtca gatgcagcca gtttcttaat      8160
   ttttttaaaa actgtatgtt tctgtggtat gtatatttgt acacctaact acctggcact      8220
   tggaaatcac agcactactc agaggcaatt gaataaagag aaatttaatt ttaaatatca      8280
   agtcctgtca aacatttctc aaacttctga ttttatcaaa ggtttgccag ccaataaagt      8340
   gcatcccaag tatacagggg agaaagctag actcctacag ggtcctagag tttaagtaat      8400
   ttttttgtta ttaatatagg taataatttt tctaattttt attttttggt tccaaatgta      8460
   aagctccttg tgtttacctc tgtttatgtc attcttgaca tgtttatcta aattatgtgt      8520
   gctctgtgac aggtgaaatg taaatctggg atccatagtc aagatatcat aaggacctac      8580
   ttcccagcct acctttcttc ctctacctga taatgataat actcaaaata acaacattca      8640
   aaggaaacac aaagaaatcc tgctttcaca tctcctattt cttgggctcc ttaataacta      8700
   ctgatggttt gttcatgaaa aaaaattttt aaatcaaaag attgtacttg gccctgagtt      8760
   gaaaaaattt caaaaatcaa aagtttgtac ttggccctga gttgaaaaaa aaaattcaca      8820
   ttctaagaat aaacagaaaa atgttcttct tggaagtaaa taacaaaagc catagtgttt      8880
   tcatttgtct tttcttcagg atacacggta gaagtcagag aatctttgat acttttattt      8940
   ggtgcaataa tcaaggccat gcaacaaccc aaaatcaagc attttggttc aagtcaggat      9000
   gacatgagtg gggacagaag ctgtggcagt cattcaaata atctcatggg tcctgaggaa      9060
   aagacaggag ttaatgtatt aagtttctac tatatgcagg aactgtgtta aatattttac      9120
   ataagttttg ataatagcta acattagctg agcacaaaat ttgggccctg atttgtgctg      9180
   agtatctttc acagattact gcttttaatc agcagtcctt gtgagctagg tatgatcatt      9240
   atccccattt tatagattac agatgagatt ctgaggcaca aagaggctaa gtaacttgcc      9300
   aaagatcata cgatgttaag taatggcccc tggattcagt ctgcagcctg aattcttaac      9360
   caattatact gtgatttcat tattcttcag aattacacta aaaagaaggt attattccca      9420
   ttttacagat gaggtatcta agctcagaga agctaaacaa cttgtgcaac aatcactaag      9480
   cttataagca gtggattagg gttagattta gatatttgtc tggcatccaa acctgtgctc      9540
   tccctacagt accacatggt ttccacagtc tcatcagacc ccggaatttc actccctgag      9600
   actgcttaat tgtgaatttc ccaaactgat tcaccaagag cctactgtct ctgctttgta      9660
   gatagctttg accacattca atgacattag gaaagactcc atttcccaag atggctcaga      9720
   aaatcagatg ctatgacgca tgttgaaagt gaaaacccat ctctgagaaa gaagcatctg      9780
   ttttattagt aaaaaaaaaa aatgaaattt acagcaatgt tgtgtgactt ctcaaaattc      9840
   tttcattttc ttatttcaga atgaatagtg ttgttcgttg gctgggaatg gggaagaatg      9900
   tgatttttaa aaataaagca taatcaaact ctgcaaaaaa aaaaaaaaaa                 9950
<210> 71
   <211> 1182
   <212> DNA
   <213> Homo sapiens
<400> 71
   gcggccgcac cccccggccg ggccgtgctt ctgcccctac aaggtttggg ccgaggtggg      60
   ggagggtcct ggttgccggc cccgcccggt ccctccccgc cttttaggcg cccgcgtggc     120
   cgggacgtcc cagtcccgct ccgtcctcct cgcctgccac cggtgcaccc agtccgctca     180
   cccagcccag tccgtccggt cctcaccgcc tgccggccgg cccacccccc accgcagcca     240
   tggacgccat caagaagaag atgcagatgc tgaagctgga caaggagaac gccatcgacc     300
   gcgccgagca ggccgaagcc gacaagaagc aagctgagga ccgctgcaag cagctggagg     360
   aggagcagca ggccctccag aagaagctga aggggacaga ggatgaggtg gaaaagtatt     420
   ctgaatccgt gaaggaggcc caggagaaac tggagcaggc cgagaagaag gccactgatg     480
   ctgaggcaga tgtggcctcc ctgaaccgcc gcattcagct ggttgaggag gagctggacc     540
   gggcccagga gcgcctggct acagccctgc agaagctgga ggaggccgag aaggcggctg     600
   atgagagcga gagaggaatg aaggtcatcg aaaaccgggc catgaaggat gaggagaaga     660
   tggaactgca ggagatgcag ctgaaggagg ccaagcacat cgctgaggat tcagaccgca     720
   aatatgaaga ggtggccagg aagctggtga tcctggaagg agagctggag cgctcggagg     780
   agagggctga ggtggccgag agccgagcca gacagctgga ggaggaactt cgaaccatgg     840
   accaggccct caagtccctg atggcctcag aggaggagta ttccaccaaa gaagataaat     900
   atgaagagga gatcaaactg ttggaggaga agctgaagga ggctgagacc cgagcagagt     960
   ttgccgagag gtctgtggca aagttggaga aaaccatcga tgacctagaa gagaccttgg    1020
   ccagtgccaa ggaggagaac gtcgagattc accagacctt ggaccagacc ctgctggaac    1080
   tcaacaacct gtgagggcca gccccacccc cagccaggct atggttgcca ccccaaccca    1140
   ataaaactga tgttactagc ctctcaaggc cctttaatcc tt                       1182
<210> 72
   <211> 7158
   <212> DNA
   <213> Homo sapiens
<400> 72
   tcctagtgag tatcgagttg gtcttattat cgcgtgaact gggagccttt gtttcctgcg      60
   tgtcgcagga agtgacgttt cgggtacagc cgctaccaga gtccctttct cgcgaggcgg     120
   aagaaccccg atcgctgagg agcaaggggg cgctaggaaa gggaactggg ttgcgacggt     180
   ccggcgagag agagctgggg tgctggggtg cggggaagtt ggggagcaga ggccgcttgg     240
   tgtccgagta gggtaagacc gcaccgaccc agtccgttag gaaagaaggg aaacgaggca     300
   attgtcgggc ggatccccgg acggagggct aaggttgtgt ggaaggcgct gctccccgga     360
   tggcgaccgc agatactccg gccccggcct ccagtggcct ctcgccgaag gaagaagggg     420
   agcttgaaga tggggaaatc agtgacgacg ataataacag ccagatacgg agtcggagca     480
   gcagcagcag cagcggcggc gggctgttac cctatccgcg gcgaaggcct cctcactcgg     540
   cccggggcgg tggatctggc ggaggcggtg gctcttcctc gtcatcgtcc tcttctcagc     600
   agcagctgag gaatttctca cgctcgcggc acgcgtctga gcggggccac ctcaggggac     660
   ccagcagcta ccgacccaaa gaaccgttcc ggtctcatcc gccttctgta cggatgcctt     720
   cgagctcact gtccgaaagc agtccccggc cgtctttctg ggagcggagc cacctcgcct     780
   tggaccgttt ccgctttcga ggcaggcctt accggggtgg gagtcgctgg agtcgggggc     840
   gaggagtggg tgagcgagga ggcaagccgg ggtgcagacc tcctctggga ggaggagcag     900
   gatccgggtt cagcagcagt cagagctggc gagagccctc tccacctcgg aagagctcca     960
   aaagttttgg aaggtctcca tcaagaaaac aaaattattc atcaaaaaat gaaaactgtg    1020
   tggaagaaac ttttgaagat ttgcttttaa agtataaaca aatacagttg gaactagaat    1080
   gcatcaataa ggatgaaaaa ctagcattga gtagcaaaga agagaatgtg caggaagatc    1140
   ctaaaacatt gaacttcgag gaccaaacta gcactgataa tgtcagtatt acaaaggatt    1200
   caagtaaaga agtagctcct gaggagaaaa cacaagtcaa aacttttcag gcatttgaat    1260
   taaaaccact caggcaaaaa ttgactttac caggagataa gaaccgtttg aaaaaagtta    1320
   aagatggagc aaaaccactt tccctgaaat ccgacactac tgattctagt caaggattac    1380
   aagataaaga acaaaattta acaagaagaa ttagtacctc agatattctg tctgaaaaga    1440
   aacttggtga agatgaagag gaactatctg aattacagct tcgccttttg gctcttcagt    1500
   cagccagtaa aaaatggcaa caaaaagaac agcaggtgat gaaagaaagc aaagaaaagt    1560
   tgactaagac gaaaactgta cagcaaaaag ttaaaacaag tacaaaaaca cattcggcca    1620
   aaaaagttag cactacagct aaacaagcat tgaggaagca gcaaacaaag gcatggaaga    1680
   aactacaaca acaaaaagag caggaaagac agaaagaaga ggatcagcgg aaacaagctg    1740
   aagaagaaga gagaaggaaa agagaggaag aaatcagaaa aattcgagat ctctcaaatc    1800
   aggaagaaca gtacaatcga ttcatgaaat tggttggtgg caagaggaga tcaagaagta    1860
   aatcttcaga tcctgacctg aggcgatcct tagataagca acctactgat agtggaggag    1920
   gcatttatca gtatgataac tatgaagaag ttgctatgga tacagatagt gaaaccagtt    1980
   ctccagctcc ttcaccagtg caaccgccat tttctctgaa atgttcattg ggtatttttt    2040
   ctccagcacc atctctttct ttgcctccac cacctcaggt ttcttctctg ccacctttga    2100
   gccagcctta tgtggaaggc ttgtgtgttt ctcttgaacc tctacctcct ctaccaccat    2160
   taccacctct cccacctgaa gatccagaac agcctccaaa accacctttt gcagatgagg    2220
   aagaggagga agaaatgctg cttcgagaag aactacttaa atctctagca aataaaagag    2280
   cttttaagcc agaggaaaca tccagtaata gtgacccacc ttcacctcca gttctgaaca    2340
   attcacatcc tgtgccaaga agcaatctat caatagtcag tattaacaca gtgtctcagc    2400
   ctaggataca gaatccaaag tttcacagag gaccccgtct tccacgaact gtgatctcgc    2460
   ttccaaagca taaatcagtg gttgtaacac taaatgattc agatgatagt gaatctgatg 2520
   gagaggcttc caagtcaaca aatagtgttt ttggtggatt agagtccatg attaaagaag 2580
   caagacgaac tgctgagcaa gcttcaaaac cgaaagtacc tccaaaatct gaaaaagaaa 2640
   atgatcctct gcgaacaccg gaggctttgc ctgaagaaaa gaagattgaa tatagattgt 2700
   taaaggaaga gattgccaac cgtgagaaac agcgtttgat taaatcagat cagctgaaga 2760
   caagttcatc atccccagca aactctgatg tggaaattga tggtattggc aggatagcaa 2820
   tggttactaa gcaggttaca gatgcagaat caaaactgaa aaaacatagg attctcttga 2880
   tgaaagatga atctgtttta aagaatttag tgcaacaaga agctaagaag aaagaatctg 2940
   ttagaaatgc tgaagcaaag attacaaaac ttacagaaca gcttcaagca actgaaaaaa 3000
   ttcttaatgt taacagaatg tttttgaaga agcttcagga acaaattcac agagttcaac 3060
   agcgtgttac aattaagaaa gctttgactc taaaatatgg agaagagctt gctcgggcaa 3120
   aggcagtggc cagtaaagaa ataggaaaac gtaaactgga acaagatcgc tttgggccaa 3180
   acaaaatgat gagactggac agttctccag tatcaagtcc aagaaagcat tcagcagaac 3240
   taattgctat ggagaaaaga cggttacaaa agctagaata tgaatatgcc ctgaaaattc 3300
   aaaaattaaa agaagcccgt gcccttaaag caaaggaaca acaaaatatc tctccagttg 3360
   tggaagagga acccgaattt tctttacctc aaccctcact tcatgatctg acacaagata 3420
   aattaaccct ggacactgaa gaaaatgatg ttgatgatga aattttgtct ggttcaagca 3480
   gagagcgaag aagatctttt ttagaatcca attattttac taaacctaac cttaagcaca 3540
   ctgatactgc taacaaagaa tgcataaaca aacttaataa aaatactgta gaaaaaccag 3600
   aactttttct agggttaaaa attggtgaat tgcaaaaatt gtattcaaaa gctgacagcc 3660
   taaaacagct gattttaaaa accaccacag gcattacaga gaaggttttg catggtcagg 3720
   agatttctgt agatgtggat tttgtcacag cacaaagtaa aacaatggaa gtgaagccat 3780
   gtccttttag accctaccat agtcctcttc tagtttttaa gtcctacaga tttagtccat 3840
   attatcgaac caaggaaaaa cttcccctga gctcagtatc atacagtaat atgattgaac 3900
   cggatcagtg tttctgccgt tttgatttaa caggaacatg taatgatgat gattgtcaat 3960
   ggcagcatat acaagactat acacttagcc gaaaacagtt attccaggac attctgtcat 4020
   ataatctgtc tttgattggt tgtgcagaga caagtactaa tgaagaaatt actgcttcag 4080
   cagaaaaata tgttgagaaa ctttttggag taaacaaaga tcgaatgtca atggaccaga 4140
   tggctgttct ccttgttagc aatatcaatg aaagtaaagg tcatactcct ccatttacaa 4200
   cctacaaaga taaaagaaag tggaagccaa agttttggag aaaacctatt tcagataata 4260
   gcttcagtag tgatgaggaa cagtctacag gaccaattaa gtatgctttc cagccagaga 4320
   accaaataaa tgttccagct ctggatacag ttgtcactcc agatgatgtc agatacttta 4380
   caaatgagac tgatgacatc gctaatttag aagcaagtgt gcttgaaaat ccttctcatg 4440
   tacaactttg gctcaagctt gcgtacaagt acttgaatca aaatgagggg gagtgctcag 4500
   aatccttgga ttctgcttta aatgttctgg cgcgagcatt ggaaaataac aaagacaatc 4560
   cagaaatttg gtgccattac ctcagattgt tctcaaaaag aggaaccaag gacgaggtgc 4620
   aggaaatgtg tgaaacagct gttgaatatg ctccagatta tcaaagcttt tggacttttc 4680
   tacacctaga aagtaccttt gaagaaaagg attacgtatg tgagagaatg ttggagtttc 4740
   tgatgggagc agccaagcag gaaacatcca atattttgtc ctttcagctt ttagaggctc 4800
   ttttgtttag agttcagctg cacatattta ctggaagatg ccaaagtgca ctggcaattt 4860
   tacagaatgc attgaaatct gctaatgatg gaatagtagc tgaatacctt aaaaccagtg 4920
   atcgatgttt ggcatggttg gcctacatac atcttattga attcaacatt ctcccttcaa 4980
   aattttatga tccatctaat gataatcctt caagaattgt taacactgaa tcatttgtaa 5040
   tgccatggca agctgttcaa gatgtaaaga ctaatcctga catgttgtta gcagtttttg 5100
   aagatgcagt gaaagcttgc acagatgaga gccttgctgt tgaggaaaga atagaggcct 5160
   gccttccact ttacacaaac atgattgctc tgcaccaact cctggagagg tatgaggctg 5220
   caatggagct ttgtaaatct ttattggaat catgtcctat taactgccag ttgctggaag 5280
   ctcttgttgc attatatttg caaacaaatc agcatgacaa agccagagca gtgtggctta 5340
   ctgcatttga aaaaaatcct cagaatgcag aggtttttta tcatatgtgc aaattcttca 5400
   tcttacagaa tcgaggcgat aatcttcttc catttttgcg gaaatttatt gcatccttct 5460
   ttaaaccggg gtttgagaag tataataact tggatctgtt tcggtatctc ttaaatattc 5520
   caggaccaat tgacattcca tctcgtttat gtaaagggaa ttttgatgat gatatgttta 5580
   accaccaagt tccttatttg tggctgattt actgcctttg tcatcctctt caatcaagta 5640
   ttaaagaaac agtggaggca tatgaggcag cattaggggt ggctatgaga tgtgatatag 5700
   tacagaagat atggatggat tatcttgtct ttgcaaataa tagagctgct ggatccagaa 5760
   acaaagttca agaattcaaa ttttttactg atttagtgaa tagatgtttg gttacagtcc 5820
   ctgcccgata ccccattcct tttagcagtg ctgattactg gtccaactat gaatttcata 5880
   atagggttat tttcttttat ttgagctgtg ttccaaagac ccagcattcc aaaaccttgg 5940
   aacggttttg ttcagttatg ccagctaatt ctggacttgc attgaggtta cttcaacatg 6000
   aatgggaaga aagcaatgtt cagattctga aacttcaagc caagatgttt acatataata 6060
   tcccaacatg cctggccacc tggaaaatag ccattgctgc tgagattgtt ctaaagggac 6120
   aaagagaggt ccaccgttta tatcagagag ccttacagaa gttacctctt tgtgcatcac 6180
   tgtggaaaga tcaactcttg tttgaagcat cagaaggagg taaaactgat aacctgagaa 6240
   aactagtttc caagtgccaa gagattggag tcagcctaaa tgagctctta aatttaaaca 6300
   gtaacaaaac agaaagcaag aatcactgaa cactgggtgc agtcagttct aagtccttat 6360
   aataattgcc aaaattattt gaatgattct tcaagattag gctgatccct ggctaaggtc 6420
   tgtgtaaggc agacaagcgt tattgatcat atcaagttcc ctacaatatc ctgtcctcaa 6480
   aaccggaagc aatgaacatg atcctcttcg gttggataaa tgaacttcct gtttggcctg 6540
   cttctaggcc ctgccagatt ctcataacat catatacgta agtatagttc ctcaaagtga     6600
   ctgacattta ttttaatttt gctttgtttt tttttatttt ctcccccatt cctttatttt     6660
   gtgttattcc tgactcactt gacactctct gatgcctgag agattcctgt ttgggattta     6720
   atatccaggg ctgtgtttac agtaaaaaaa gcaggcagtc ccttttagtt tttccttttt     6780
   aaattttttt gagattcttc atttcaggat ttaaaactat agcagtccat cttaaggaaa     6840
   gtgtaactgc catggccaca agtctgctag ttgcacttga atgctctatc agggttgttt     6900
   attacccttt ctacgttctg gactccttgc cgagactgtt taacttgaag attaaagaaa     6960
   ctattgcaaa tgccagtgca tcagaaccta agagtggtca aatattatgt gcaatttttt     7020
   tgtaaagaaa ttttaattta taataaagtt taacagttta aagaacagtt aatatttgaa     7080
   ctgctttgta ttgaaatact actttgtagt attgaaattg tttatatact tttttaatat     7140
   aagttaactt tctaaaaa                                                   7158
<210> 73
   <211> 4336
   <212> DNA
   <213> Homo sapiens
<400> 73
   agttcgccct gccggaaagc agcacgccgc cgcggcattt tacgacgtcg gcggtgacag      60
   gccctgggac tctgggaata cccagcttcc tccccgcaac ccggtgaaag ccaacgcaat     120
   gttcggtgcg ggggacgagg acgacaccga tttcctctcg ccgagcggcg gtgccagatt     180
   ggcctcactt tttggactgg atcaggcagc tgctggccat ggaaatgaat ttttccagta     240
   cacagcccca aaacagccta agaaaggcca gggaacggca gcaacaggaa atcaggcaac     300
   accaaaaaca gcaccagcca ccatgagcac tcccacaata ctggtcgcaa cagcagtcca     360
   tgcatatcga tacacaaatg gtcaatatgt aaagcagggc aaatttggtg ctgcagttct     420
   ggggaaccac acagccagag agtataggat tcttctttat atcagtcaac aacagccagt     480
   tacggttgct aggattcatg tgaactttga gctaatggtt cggcccaata actatagcac     540
   cttttatgat gaccagagac agaactggtc catcatgttt gagtcggaaa aggctgctgt     600
   ggagttcaat aagcaggtgt gcattgctaa gtgcaacagt acctcttccc tggatgcagt     660
   gctctcccag gacctcattg tggcagacgg ccctgctgta gaagttggag attctttgga     720
   agtggcctat accggctggc tctttcagaa tcatgtgctg ggccaggttt tcgactccac     780
   tgctaacaaa gataagttgc ttcgcttgaa gttaggatca ggaaaagtca tcaagggctg     840
   ggaggatgga atgctgggca tgaaaaaagg aggaaagcga ttgcttattg tccctccagc     900
   ctgtgctgtt ggctcagaag ggtaataggg ctggactcaa gcaacggact cgatcctggt      960
   gttcgaggtg gaggttaggc gggtgaagtt tgccagagat tctggctctg atggtcacag    1020
   tgttagttcc cgcgattctg cagctccgtc tcccatccct ggtgctgaca acctctctgc    1080
   tgatcctgtt gtgtcaccac ccacatcaat acctttcaaa tcaggggagc cagctcttcg    1140
   taccaaatct aactccctca gtgaacaact tgcaataaat acaagtcccg atgcagtcaa    1200
   agccaagttg atctctcgga tggctaaaat gggccagccc atgctgccca tccttccacc    1260
   acagctggat tccaatgatt cagaaatcga agatgtgaac actctgcaag gaggtgggca    1320
   gcctgtggtg actccgtccg tccagccctc tcttcatccg gcccatccag cgttaccaca    1380
   gatgacctca caggcacctc agccatctgt tactgggctc caggcacctt ctgctgcctt    1440
   aatgcaagtg tcatctctcg attcccactc agctgtatct ggaaatgccc aatcctttca    1500
   gccctatgca ggtatgcaag cctacgctta tccccaggca tctgccgtca cctcccagct    1560
   gcagcccgtt cggcctttgt acccagcacc gctctctcag cctccccatt tccaaggatc    1620
   aggtgatatg gcttcatttc tcatgactga agcccggcaa cataacactg aaattcgaat    1680
   ggcagtcagc aaagtggctg ataaaatgga tcatctcatg actaaggttg aagagttaca    1740
   gaaacatagt gctggcaatt ccatgcttat tcctagcatg tcagttacaa tggaaacaag    1800
   catgattatg agcaacatcc agcgaatcat tcaggaaaat gaaagattga agcaagagat    1860
   ccttgaaaag agcaatcgga tagaagaaca gaatgacaag attagtgaac taattgaacg    1920
   aaatcagagg tatgttgagc agagtaacct gatgatggag aagaggaaca actcacttca    1980
   gacagccaca gaaaacacac aggcaagagt attgcatgct gaacaagaga aggccaaggt    2040
   gacagaggag ttagcagcgg ccactgcaca ggtctctcat ctgcagctga aaatgactgc    2100
   tcaccaaaaa aaggaaacag agctgcagat gcagctgaca gaaagcctga aggagacaga    2160
   tcttctcagg ggccagctca ccaaagtgca ggcaaagctc tcagagctcc aagaaacctc    2220
   tgagcaagca cagtccaaat tcaaaagtga aaagcagaac cggaaacaac tggaactcaa    2280
   ggtgacatcc ctggaggagg aactgactga ccttcgagtt gagaaggagt ccttggaaaa    2340
   gaacctctca gaaaggaaaa agaagtcagc tcaagagcgt tctcaggccg aggaggagat    2400
   agatgaaatt cgcaagtcat accaggagga attggacaaa cttcgacagc tcttgaaaaa    2460
   gactcgagtg tccacagacc aagcagctgc agagcagctg tctttagtac aggctgagct    2520
   acagacccag tgggaagcaa aatgtgaaca tttgttggcc tccgccaagg atgagcacct    2580
   gcagcagtac caggaggtgt gcgcacagag agatgcctac cagcagaagc tggtacaact    2640
   tcaggaaaag tgtttagccc tccaggccca aatcacagct ctcaccaagc aaaatgaaca    2700
   gcacatcaag gaactagaga agaacaagtc ccagatgtct ggggttgaag ctgctgcatc    2760
   tgacccctca gagaaggtca agaagatcat gaaccaggtg ttccagtcct tacggagaga    2820
   gtttgagctg gaggaatctt acaatggcag gaccattctg ggaaccatca tgaatacgat    2880
   caagatggtg actcttcagc tgttaaacca acaggagcaa gagaaggaag agagcagcag 2940
   tgaagaagaa gaagaaaaag cagaagagcg gccacgaaga ccttcccagg agcagtcagc 3000
   ctcagccagt tctgggcagc ctcaagcacc cctgaatagg gagaggccag agtcccccat 3060
   ggtgccctca gagcaggtgg tcgaggaagc tgtcccgttg cctcctcagg ccctcaccac 3120
   ttcccaggat ggacacagaa ggaaagggga ctcagaagct gaggcactct cagagataaa 3180
   agatggttcc cttccacccg aactgtcttg catcccatcc cacagagttc tagggccccc 3240
   gacttcaatt ccacctgagc ccctaggccc tgtatccatg gactctgagt gtgaggagtc 3300
   acttgctgcc agcccaatgg cagctaagcc cgacaaccca tcaggaaagg tctgtgtcag 3360
   ggaagtagca ccagatggcc cactacaaga aagctccaca agactgtccc tgacttcaga 3420
   ccccgaggag ggggacccac tggccttagg gcctgaaagc ccaggagagc ctcagcctcc 3480
   acagctcaag aaagatgatg tcactagctc caccggtccc cacaaggagc tgtcaagcac 3540
   agaggcaggt tccacagttg caggagcagc cctcagaccc agccatcatt cccagcgttc 3600
   cagtctctct ggggatgaag aggatgaact gtttaaaggg gcaactctga aagctctgag 3660
   gcccaaagca cagcctgagg aggaggatga agacgaggtg agcatgaagg gacgcccgcc 3720
   cccaacgccc ctttttggag atgatgatga tgacgatgac attgactggc tgggatgaag 3780
   acccaggaaa ctggtgcaaa ggtttctctg caacccttcc ctaagcatga ttttgcacag 3840
   ccaaccctgg gtctaggcga accacagggt gaggtcaagg tgagcattct gggaacaata 3900
   tttgggctca gagggtgggt tggccacctt ctgagcccca cccccgccag acctggtgaa 3960
   gaggatcata accctgtctt caagaacact gggatttcag cagcaagttg gaagaaggac 4020
   tggtaggttc ccctccaagc cagtcacctg taagagtcct gtcctctgcc agacttttta 4080
   atctcttcat taactctcag actgacctgg gagccctcct ctacctgaat ccagtgctca 4140
   actgtgcccc ggcaacaaga cctgggctga ggtctccctg gtagaactaa gggagattac 4200
   accatctaaa tcccagtgca gtcaacagcc tggcctatag tcctgggaca tgtatcttct 4260
   tctttgcctt aaatctgata caagaggtca atgactttga aaataaaact aaaataaatg 4320
   tctataatga aacttg                                                4336
<210> 74
   <211> 1496
   <212> DNA
   <213> Homo sapiens
<400> 74
   gtctaagccc cgccctttcc tgtcgtgact taacgcacgc aagcggctcc agggtacgtc   60
   cccgccacgc gcgctcgcag gatcggtgcg tggtgacgtt tcgccggcgc gggcgccatc  120
   ccggaagcgc gagcaaggcc gccagatgtg caggtgccgc cgctaccgac gccggggccg  180
   agtttggggc agcggaggag gagaaagaga tggacctccc ggactcggcc tcgagggtct  240
   tctgcggccg catcctgagc atggtgaaca cagatgatgt caacgccatc atcctggccc  300
   agaagaacat gctggaccgc tttgagaaga ccaatgagat gctgctcaac ttcaacaacc  360
   tgtccagtgc ccgcctgcag cagatgagcg aacgcttcct gcaccacacg aggaccctag  420
   tagagatgaa acgggacctg gacagcatct tccgccgtat caggacgctg aaagggaaac  480
   tggccaggca gcacccagag gccttcagcc atatcccaga ggcatccttc ctggaggaag  540
   aggatgaaga ccccatccca cccagcacca cgaccaccat tgccacctca gaacagagca  600
   cgggctcatg tgacaccagc cccgacaccg tctcgccctc cctgagcccc ggcttcgagg  660
   acctgtccca tgtccagcct ggctccccag ccatcaacgg ccgcagccag acagatgacg  720
   aggagatgac gggcgaatag ccctgctgcc cggtgccttg agggggtctc agggcagcag  780
   catacaaggt ggcagcgggt aaccctgcct tgttctgtca tccagggctc ctttgctgcc  840
   ccgttctgtc acccagggct cctaggggga caaggctctc tcccgagggg tgtggaattc  900
   ctgggggggt ctttaattct ggctccttcc ttcctcagaa catctctatt ctgcaagacc  960
   cctctgccat gccagggcac gcccattcca gctggagtcg tggggctggg cacaggggaa 1020
   tttttccaga gctgagcctg acgtctgctc tgaagaatgc ttagaaggtt cccagacacc 1080
   agagccagat gtcccccacc accggtcagg acctccttga ggtgcacaag cacggtctcc 1140
   tctgagttca ccccagccca cccccgcacc cactaattct gcttttcctg ccccttgctc 1200
   cgtaaaagta tcaaatactt tctccttggt atctcaagga ggtttctgag ataggtagaa 1260
   gtcttgagac ggaggctggc catccattca gccctgagcg tgctgagttc tgtgtttctc 1320
   tgaatagagg tgtggaacct gaggggccag caggcctctc tgaaggcctc catggagcaa 1380
   acggagccac ctcgggaaag agtttaatgg aatatttttg tacccgatgt ttacagatgc 1440
   tgttgggaag ttatcaataa aaagacacca ttactaaaaa gggaaaagta caccta     1496
<210> 75
   <211> 1874
   <212> DNA
   <213> Homo sapiens
<400> 75
   caggccttaa aggggccgca actatttttt taagccactt tttcctctct ccaattttgc 60
   aacgacgggc gcggaggagg aggttcccgg aagccacgcg cagctggagc agcggcgacc 120
   gcagctggag gcccggagcg cctgcggggc tggcagaggc gagggaggtt gcgggtagga 180
   agggcggact gcgcgcgccc cctgcgtccc gcgcacctcg gggccggtcc atgctcccga 240
   cggctgcggg cttcagcatc tggggccagg ttggggcggc ggggtccagg gcgcaggtgg 300
   tgcggccgat gcgccgggcc ggaggctgag gccgcgctgc ggggctggga cagcactggc 360
   atctccagag caggcccggg gcagcaaggg aggcgccgcg atgccagacg aaaatatctt 420
   cctgttcgtg cccaacctca tcggttatgc ccggattgtc ttcgccatca tttctttcta 480
   cttcatgccc tgctgccccc tcacggcctc ctccttctac ctgctcagcg gcctgctgga 540
   cgctttcgat ggacacgctg ctcgcgctct taatcaagga acccggtttg gggccatgct 600
   ggacatgctg acggaccgct gctccaccat gtgcctgttg gtcaacctgg ccctgctgta 660
   ccctggagcc acgctgttct tccaaatcag catgagtttg gatgtggcca gtcactggct 720
   gcacctccac agttctgtgg tccgaggcag tgagagtcac aagatgatcg acttgtccgg 780
   gaatccggtg cttcggatct actacacctc gaggcctgct ctgttcacct tgtgtgctgg 840
   gaatgagctc ttctactgcc tcctctacct gttccatttc tctgagggac ctttagttgg 900
   ctctgtggga ctgttccgga tgggcctctg ggtcactgcc cccatcgcct tgctgaagtc 960
   gctcatcagc gtcatccacc tgatcacggc cgcccgcaac atggctgccc tggacgcagc 1020
   agaccgcgcc aagaagaagt gacgctggag ccccgggtcc tggctgccca cctgccctgg 1080
   gagtcttgct gtgccacaca gctccccacc ccctgctagg aggtcccagt ctcacgcctt 1140
   cctcatgtgt tgttctacct gctgggatgg gggtcagcct ctctttggtg acgtcacgtt 1200
   ctctgggatc ctgaggaccc gggcctcaaa tcagggagga tacgcgggag gccccctcca 1260
   tccaggcggt gctcctgggg tgccgggacc gggcagtgtc acaccctgcc tgctcagtcc 1320
   tggggtccga gatgctaggg acgcttgagt gagggaggtg gtgtgagggc caggtttcct 1380
   gaaaggcggg agtcagacct ccgcccccag ccagagcaag cttggggcac catgcccagg 1440
   agggaagaag ccatccacag ccttccctgt caccggctcc tctgtcctgc ctaccctggt 1500
   cctggcggga cttcactatt tgacttggtt tcctttcaga tattcttggc tcagggcctg 1560
   ggttgaggga gcttagggaa ggacgtccgt ctgggtgctt ttcctccagt ttgctggctg 1620
   gcttctccgt ctacccacag tgacctcaca gagaggccct cctgccaccc atgctcatgt 1680
   ggtgtcccca ccgcccactt gtttgatgtc actgactgtc tacatgtatt tatattcttg 1740
   atattttcta ccctcactag aatgtaaact ccatgaaggc acagactttt cttgttctct 1800
   tctctatccc tagagtaaga ccaacttgaa cctggcatat agtagctgct taataaatac 1860
   tcgtctgtca atga 1874
<210> 76
   <211> 20
   <212> DNA
   <213> Artificial Sequence
<220>
   <223> Made in a lab
<400> 76
   acttcggagt tttgccattg 20
<210> 77
   <211> 19
   <212> DNA
   <213> Artificial Sequence
<220>
   <223> Made in a lab
<400> 77
   acggaaacca gcttcatcc 19
<210> 78
   <211> 19
   <212> DNA
   <213> Artificial Sequence
<220>
   <223> Made in a lab
<400> 78
   aggtgggtga ggaaatcca 19
<210> 79
   <211> 20
   <212> DNA
   <213> Artificial Sequence
<220>
   <223> Made in a lab
<400> 79
   gctccaggtt tgactgtggt 20
<210> 80
   <211> 20
   <212> DNA
   <213> Artificial Sequence
<220>
   <223> Made in a lab
<400> 80
   gtcctggcca agagctacaa 20
<210> 81
   <211> 21
   <212> DNA
   <213> Artificial Sequence
<220>
   <223> Made in a lab
<400> 81
   ctaggccatc tatggctttc a 21
<210> 82
   <211> 20
   <212> DNA
   <213> Artificial Sequence
<220>
   <223> Made in a lab
<400> 82
   ccgcatcaaa cagagtgaac 20
<210> 83
   <211> 20
   <212> DNA
   <213> Artificial Sequence
<220>
   <223> Made in a lab
<400> 83
   gtcacccttc aggtcctcat 20
<210> 84
   <211> 20
   <212> DNA
   <213> Artificial Sequence
<220>
   <223> Made in a lab
<400> 84
   tccagttccg atttgtaggc 20
<210> 85
   <211> 20
   <212> DNA
   <213> Artificial Sequence
<220>
   <223> Made in a lab
<400> 85
   gtcgcttttg ggattacctg 20
<210> 86
   <211> 20
   <212> DNA
   <213> Artificial Sequence
<220>
   <223> Made in a lab
<400> 86
   taccagctca ccaagctcct 20
<210> 87
   <211> 20
   <212> DNA
   <213> Artificial Sequence
<220>
   <223> Made in a lab
<400> 87
   aaagtccacg ctcaccatgt 20
<210> 88
   <211> 20
   <212> DNA
   <213> Artificial Sequence
<220>
   <223> Made in a lab
<400> 88
   atcccagtcc cacttgtgtc 20
<210> 89
   <211> 19
   <212> DNA
   <213> Artificial Sequence
<220>
   <223> Made in a lab
<400> 89
   aaacatggtc cctggcagt 19
<210> 90
   <211> 20
   <212> DNA
   <213> Artificial Sequence
<220>
   <223> Made in a lab
<400> 90
   gaaccttggg aaactgtgga 20
<210> 91
   <211> 20
   <212> DNA
   <213> Artificial Sequence
<220>
   <223> Made in a lab
<400> 91
   catttggtca ccctttggac 20
<210> 92
   <211> 20
   <212> DNA
   <213> Artificial Sequence
<220>
   <223> Made in a lab
<400> 92
   tcacaagcaa tccaaagctg 20
<210> 93
   <211> 20
   <212> DNA
   <213> Artificial Sequence
<220>
   <223> Made in a lab
<400> 93
   ttgggacagt aggcatttcc 20
<210> 94
   <211> 20
   <212> DNA
   <213> Artificial Sequence
<220>
   <223> Made in a lab
<400> 94
   cgagcagaga gggagtagga 20
<210> 95
   <211> 20
   <212> DNA
   <213> Artificial Sequence
<220>
   <223> Made in a lab
<400> 95
   ccacagacag ctgagttcca 20
<210> 96
   <211> 20
   <212> DNA
   <213> Artificial Sequence
<220>
   <223> Made in a lab
<400> 96
   tagccgttca ctccgctatt 20
<210> 97
   <211> 20
   <212> DNA
   <213> Artificial Sequence
<220>
   <223> Made in a lab
<400> 97
   ggagaaaagg gcttctggat 20
<210> 98
   <211> 20
   <212> DNA
   <213> Artificial Sequence
<220>
   <223> Made in a lab
<400> 98
   ccagccatga attacgacaa 20
<210> 99
   <211> 19
   <212> DNA
   <213> Artificial Sequence
<220>
   <223> Made in a lab
<400> 99
   cgggctcaca cacaaactt 19
<210> 100
   <211> 21
   <212> DNA
   <213> Artificial Sequence
<220>
   <223> Made in a lab
<400> 100
   catacaggct gaaatcctcc a 21
<210> 101
   <211> 20
   <212> DNA
   <213> Artificial Sequence
<220>
   <223> Made in a lab
<400> 101
   tgacacggat aaacctggaa 20
<210> 102
   <211> 21
   <212> DNA
   <213> Artificial Sequence
<220>
   <223> Made in a lab
<400> 102
   tggccatctc aatggagttt a 21
<210> 103
   <211> 19
   <212> DNA
   <213> Artificial Sequence
<220>
   <223> Made in a lab
<400> 103
   ataccaccgg gttttccaa 19
<210> 104
   <211> 20
   <212> DNA
   <213> Artificial Sequence
<220>
   <223> Made in a lab
<400> 104
   tggaattgct gttcctgatg 20
<210> 105
   <211> 22
   <212> DNA
   <213> Artificial Sequence
<220>
   <223> Made in a lab
<400> 105
   ggttgccatt ttgttaggat tc 22
<210> 106
   <211> 20
   <212> DNA
   <213> Artificial Sequence
<220>
   <223> Made in a lab
<400> 106
   gtctgtcctc ccaggctcag 20
<210> 107
   <211> 18
   <212> DNA
   <213> Artificial Sequence
<220>
   <223> Made in a lab
<400> 107
   gaaggcctca tccctgtg 18
<210> 108
   <211> 20
   <212> DNA
   <213> Artificial Sequence
<220>
   <223> Made in a lab
<400> 108
   tccctgtggc aagagaagtt 20
<210> 109
   <211> 20
   <212> DNA
   <213> Artificial Sequence
<220>
   <223> Made in a lab
<400> 109
   tggtggtcaa aatggacaga 20
<210> 110
   <211> 20
   <212> DNA
   <213> Artificial Sequence
<220>
   <223> Made in a lab
<400> 110
   tcatccaaga agccctaacg 20
<210> 111
   <211> 20
   <212> DNA
   <213> Artificial Sequence
<220>
   <223> Made in a lab
<400> 111
   cgctttctct gagcattctg 20
<210> 112
   <211> 20
   <212> DNA
   <213> Artificial Sequence
<220>
   <223> Made in a lab
<400> 112
   gatggcagat tccgactgag 20
<210> 113
   <211> 20
   <212> DNA
   <213> Artificial Sequence
<220>
   <223> Made in a lab
<400> 113
   cttgcacatg tgaggccata 20
<210> 114
   <211> 20
   <212> DNA
   <213> Artificial Sequence
<220>
   <223> Made in a lab
<400> 114
   ccagcaagca acccatagtc 20
<210> 115
   <211> 19
   <212> DNA
   <213> Artificial Sequence
<220>
   <223> Made in a lab
<400> 115
   acttgctgcc gggtatagg 19
<210> 116
   <211> 20
   <212> DNA
   <213> Artificial Sequence
<220>
   <223> Made in a lab
<400> 116
   ggaactcctc tgaagcaagc 20
<210> 117
   <211> 20
   <212> DNA
   <213> Artificial Sequence
<220>
   <223> Made in a lab
<400> 117
   cacgggcttc tcttcactct 20
<210> 118
   <211> 20
   <212> DNA
   <213> Artificial Sequence
<220>
   <223> Made in a lab
<400> 118
   cagccggttt attgtgcttc 20
<210> 119
   <211> 20
   <212> DNA
   <213> Artificial Sequence
<220>
   <223> Made in a lab
<400> 119
   aacaaagcct ggacaaatgg 20
<210> 120
   <211> 20
   <212> DNA
   <213> Artificial Sequence
<220>
   <223> Made in a lab
<400> 120
   cgaggagaac agcaacgata 20
<210> 121
   <211> 20
   <212> DNA
   <213> Artificial Sequence
<220>
   <223> Made in a lab
<400> 121
   cagatcttgc catccaccag 20
<210> 122
   <211> 20
   <212> DNA
   <213> Artificial Sequence
<220>
   <223> Made in a lab
<400> 122
   gtccctggat gtcaaccact 20
<210> 123
   <211> 20
   <212> DNA
   <213> Artificial Sequence
<220>
   <223> Made in a lab
<400> 123
   gatgtagcca tgctcgtcct 20
<210> 124
   <211> 20
   <212> DNA
   <213> Artificial Sequence
<220>
   <223> Made in a lab
<400> 124
   tatgatggct cgaaggctct 20
<210> 125
   <211> 20
   <212> DNA
   <213> Artificial Sequence
<220>
   <223> Made in a lab
<400> 125
   cctgtgcctt ggctaaactc 20
<210> 126
   <211> 18
   <212> DNA
   <213> Artificial Sequence
<220>
   <223> Made in a lab
<400> 126
   ccagtgggtc ctcacagc 18
<210> 127
   <211> 20
   <212> DNA
   <213> Artificial Sequence
<220>
   <223> Made in a lab
<400> 127
   agctgtggct gacctgaaat 20
<210> 128
   <211> 22
   <212> DNA
   <213> Artificial Sequence
<220>
   <223> Made in a lab
<400> 128
   agcattgaac cagaggagtt ct 22
<210> 129
   <211> 18
   <212> DNA
   <213> Artificial Sequence
<220>
   <223> Made in a lab
<400> 129
   cccgagcagg tgcttttg 18
<210> 130
   <211> 20
   <212> DNA
   <213> Artificial Sequence
<220>
   <223> Made in a lab
<400> 130
   gtggacacct gtgtcagcat 20
<210> 131
   <211> 19
   <212> DNA
   <213> Artificial Sequence
<220>
   <223> Made in a lab
<400> 131
   cctcccacaa tccgagact 19
<210> 132
   <211> 19
   <212> DNA
   <213> Artificial Sequence
<220>
   <223> Made in a lab
<400> 132
   ctgattgttg acgggaacg 19
<210> 133
   <211> 20
   <212> DNA
   <213> Artificial Sequence
<220>
   <223> Made in a lab
<400> 133
   aggcattttt gcttcacacc 20
<210> 134
   <211> 18
   <212> DNA
   <213> Artificial Sequence
<220>
   <223> Made in a lab
<400> 134
   cccctgctgt gccaaaac 18
<210> 135
   <211> 18
   <212> DNA
   <213> Artificial Sequence
<220>
   <223> Made in a lab
<400> 135
   gcagtgccct ggaggaga 18
<210> 136
   <211> 20
   <212> DNA
   <213> Artificial Sequence
<220>
   <223> Made in a lab
<400> 136
   tgagcgagta ccccacctac 20
<210> 137
   <211> 20
   <212> DNA
   <213> Artificial Sequence
<220>
   <223> Made in a lab
<400> 137
   cccctacaag ttggcagaag 20
<210> 138
   <211> 25
   <212> DNA
   <213> Artificial Sequence
<220>
   <223> Made in a lab
<400> 138
   ttgcaggaag aataggatct tctaa 25
<210> 139
   <211> 18
   <212> DNA
   <213> Artificial Sequence
<220>
   <223> Made in a lab
<400> 139
   agtcccatcc ccattcct 18
<210> 140
   <211> 20
   <212> DNA
   <213> Artificial Sequence
<220>
   <223> Made in a lab
<400> 140
   tatctgcatc tggctgctgt 20
<210> 141
   <211> 20
   <212> DNA
   <213> Artificial Sequence
<220>
   <223> Made in a lab
<400> 141
   tgcaaggagc actcaatgac 20
<210> 142
   <211> 20
   <212> DNA
   <213> Artificial Sequence
<220>
   <223> Made in a lab
<400> 142
   aagcgattga cccttgagac 20
<210> 143
   <211> 20
   <212> DNA
   <213> Artificial Sequence
<220>
   <223> Made in a lab
<400> 143
   tgttgcgagt tgtttctgct 20
<210> 144
   <211> 20
   <212> DNA
   <213> Artificial Sequence
<220>
   <223> Made in a lab
<400> 144
   gcactgagtc atcaggagca 20
<210> 145
   <211> 20
   <212> DNA
   <213> Artificial Sequence
<220>
   <223> Made in a lab
<400> 145
   caccagttcc ggacacacta 20
<210> 146
   <211> 20
   <212> DNA
   <213> Artificial Sequence
<220>
   <223> Made in a lab
<400> 146
   agtcctggtg ggaacagatg 20
<210> 147
   <211> 20
   <212> DNA
   <213> Artificial Sequence
<220>
   <223> Made in a lab
<400> 147
   ttggagccac agagaaggtt 20
<210> 148
   <211> 19
   <212> DNA
   <213> Artificial Sequence
<220>
   <223> Made in a lab
<400> 148
   tgttctgctg ggagtgcat 19
<210> 149
   <211> 20
   <212> DNA
   <213> Artificial Sequence
<220>
   <223> Made in a lab
<400> 149
   tgccgaaggt agatgagctt 20
<210> 150
   <211> 20
   <212> DNA
   <213> Artificial Sequence
<220>
   <223> Made in a lab
<400> 150
   tatggtattc acgccctgct 20
<210> 151
   <211> 20
   <212> DNA
   <213> Artificial Sequence
<220>
   <223> Made in a lab
<400> 151
   tgacacacat gacgatgcag 20
<210> 152
   <211> 20
   <212> DNA
   <213> Artificial Sequence
<220>
   <223> Made in a lab
<400> 152
   cgatgattcc atttgtgtgc 20
<210> 153
   <211> 20
   <212> DNA
   <213> Artificial Sequence
<220>
   <223> Made in a lab
<400> 153
   tgagccacca ctgttctttg 20
<210> 154
   <211> 20
   <212> DNA
   <213> Artificial Sequence
<220>
   <223> Made in a lab
<400> 154
   ctggttctct gcctgcagtt 20
<210> 155
   <211> 20
   <212> DNA
   <213> Artificial Sequence
<220>
   <223> Made in a lab
<400> 155
   gcagtgactt cgtcatttgg 20
<210> 156
   <211> 20
   <212> DNA
   <213> Artificial Sequence
<220>
   <223> Made in a lab
<400> 156
   ctggcttatg ggcaacattt 20
<210> 157
   <211> 20
   <212> DNA
   <213> Artificial Sequence
<220>
   <223> Made in a lab
<400> 157
   ccccaagacc atgagaaaaa 20
<210> 158
   <211> 19
   <212> DNA
   <213> Artificial Sequence
<220>
   <223> Made in a lab
<400> 158
   ctgcctgcag gtggagaac 19
<210> 159
   <211> 20
   <212> DNA
   <213> Artificial Sequence
<220>
   <223> Made in a lab
<400> 159
   gagtcatcca cgcagttcaa 20
<210> 160
   <211> 23
   <212> DNA
   <213> Artificial Sequence
<220>
   <223> Made in a lab
<400> 160
   tcatgtttct ggaaagagca ttt 23
<210> 161
   <211> 23
   <212> DNA
   <213> Artificial Sequence
<220>
   <223> Made in a lab
<400> 161
   tgctttcaat atcaaacaga gca 23
<210> 162
   <211> 20
   <212> DNA
   <213> Artificial Sequence
<220>
   <223> Made in a lab
<400> 162
   gccgatgtaa ttggcttctc 20
<210> 163
   <211> 20
   <212> DNA
   <213> Artificial Sequence
<220>
   <223> Made in a lab
<400> 163
   tggttttctg ctccttggtc 20
<210> 164
   <211> 22
   <212> DNA
   <213> Artificial Sequence
<220>
   <223> Made in a lab
<400> 164
   gcatcactaa ggtcttcagc aa 22
<210> 165
   <211> 20
   <212> DNA
   <213> Artificial Sequence
<220>
   <223> Made in a lab
<400> 165
   tcagtccctt tctcgtcgat 20
<210> 166
   <211> 22
   <212> DNA
   <213> Artificial Sequence
<220>
   <223> Made in a lab
<400> 166
   tcatcttctt ctgtgccttt ca 22
<210> 167
   <211> 22
   <212> DNA
   <213> Artificial Sequence
<220>
   <223> Made in a lab
<400> 167
   tccttgatgt atcttcggac aa 22
<210> 168
   <211> 20
   <212> DNA
   <213> Artificial Sequence
<220>
   <223> Made in a lab
<400> 168
   ggagtttgct cagcttctgg 20
<210> 169
   <211> 20
   <212> DNA
   <213> Artificial Sequence
<220>
   <223> Made in a lab
<400> 169
   tgatattctg ctccccaacc 20
<210> 170
   <211> 20
   <212> DNA
   <213> Artificial Sequence
<220>
   <223> Made in a lab
<400> 170
   ccagcaggga caatgagatt 20
<210> 171
   <211> 18
   <212> DNA
   <213> Artificial Sequence
<220>
   <223> Made in a lab
<400> 171
   agtggcctcc gcacagtc 18
<210> 172
   <211> 20
   <212> DNA
   <213> Artificial Sequence
<220>
   <223> Made in a lab
<400> 172
   gccgaggtga tagtgtggtt 20
<210> 173
   <211> 20
   <212> DNA
   <213> Artificial Sequence
<220>
   <223> Made in a lab
<400> 173
   tgaggtgatg tcctcgtctg 20
<210> 174
   <211> 21
   <212> DNA
   <213> Artificial Sequence
<220>
   <223> Made in a lab
<400> 174
   ggagtttgct ggactttctc a 21
<210> 175
   <211> 20
   <212> DNA
   <213> Artificial Sequence
<220>
   <223> Made in a lab
<400> 175
   acgtagttca tccgctccac 20
<210> 176
   <211> 20
   <212> DNA
   <213> Artificial Sequence
<220>
   <223> Made in a lab
<400> 176
   cagagtgcaa ccaatcacct 20
<210> 177
   <211> 22
   <212> DNA
   <213> Artificial Sequence
<220>
   <223> Made in a lab
<400> 177
   aattgagaag agcttccatt ca 22
<210> 178
   <211> 20
   <212> DNA
   <213> Artificial Sequence
<220>
   <223> Made in a lab
<400> 178
   ggagacgtaa agctgccaac 20
<210> 179
   <211> 20
   <212> DNA
   <213> Artificial Sequence
<220>
   <223> Made in a lab
<400> 179
   agctactgct ttggcaggaa 20
<210> 180
   <211> 20
   <212> DNA
   <213> Artificial Sequence
<220>
   <223> Made in a lab
<400> 180
   acaaggcttt ttgcatttgg 20
<210> 181
   <211> 22
   <212> DNA
   <213> Artificial Sequence
<220>
   <223> Made in a lab
<400> 181
   tggtgtctgg ttaccaaaag ag 22
<210> 182
   <211> 20
   <212> DNA
   <213> Artificial Sequence
<220>
   <223> made in a lab
<400> 182
   tggaggaggc cagactacag 20
<210> 183
   <211> 20
   <212> DNA
   <213> Artificial Sequence
<220>
   <223> Made in a lab
<400> 183
   ctccagaccc tggtacttgc 20
<210> 184
   <211> 18
   <212> DNA
   <213> Artificial Sequence
<220>
   <223> Made in a lab
<400> 184
   gccgactacc tgctgctg 18
<210> 185
   <211> 25
   <212> DNA
   <213> Artificial Sequence
<220>
   <223> Made in a lab
<400> 185
   gattttgtcc tggtagtgtt taatg 25
<210> 186
   <211> 20
   <212> DNA
   <213> Artificial Sequence
<220>
   <223> Made in a lab
<400> 186
   gcattggcct gttcttcagt 20
<210> 187
   <211> 22
   <212> DNA
   <213> Artificial Sequence
<220>
   <223> Made in a lab
<400> 187
   tcatcaaagg aatgcactag ga 22
<210> 188
   <211> 23
   <212> DNA
   <213> Artificial Sequence
<220>
   <223> Made in a lab
<400> 188
   tcaagtagag atttgctcaa tgc 23
<210> 189
   <211> 19
   <212> DNA
   <213> Artificial Sequence
<220>
   <223> Made in a lab
<400> 189
   ggcttcattg gttggtcat 19
<210> 190
   <211> 20
   <212> DNA
   <213> Artificial Sequence
<220>
   <223> Made in a lab
<400> 190
   tgccacttga cggaattgta 20
<210> 191
   <211> 20
   <212> DNA
   <213> Artificial Sequence
<220>
   <223> Made in a lab
<400> 191
   ccaggtagca tgaggggata 20
<210> 192
   <211> 20
   <212> DNA
   <213> Artificial Sequence
<220>
   <223> Made in a lab
<400> 192
   ggtggaagga atggaggatt 20
<210> 193
   <211> 20
   <212> DNA
   <213> Artificial Sequence
<220>
   <223> Made in a lab
<400> 193
   aaccttccct gcaatacgtg 20
<210> 194
   <211> 20
   <212> DNA
   <213> Artificial Sequence
<220>
   <223> Made in a lab
<400> 194
   agcagctgaa cgagcagttt 20
<210> 195
   <211> 20
   <212> DNA
   <213> Artificial Sequence
<220>
   <223> Made in a lab
<400> 195
   aaggaacgtc cgagtcagaa 20
<210> 196
   <211> 20
   <212> DNA
   <213> Artificial Sequence
<220>
   <223> Made in a lab
<400> 196
   ctttcgatgt gccaagtgtg 20
<210> 197
   <211> 20
   <212> DNA
   <213> Artificial Sequence
<220>
   <223> Made in a lab
<400> 197
   ggcccgaagt ttttagcata 20
<210> 198
   <211> 20
   <212> DNA
   <213> Artificial Sequence
<220>
   <223> Made in a lab
<400> 198
   atttgcccag aagattggtg 20
<210> 199
   <211> 20
   <212> DNA
   <213> Artificial Sequence
<220>
   <223> Made in a lab
<400> 199
   catcgaaagc aggccttatg 20
<210> 200
   <211> 20
   <212> DNA
   <213> Artificial Sequence
<220>
   <223> Made in a lab
<400> 200
   cagagacgtc acaagccaac 20
<210> 201
   <211> 22
   <212> DNA
   <213> Artificial Sequence
<220>
   <223> Made in a lab
<400> 201
   tgctgactgt gttgtaattg ga 22
<210> 202
   <211> 20
   <212> DNA
   <213> Artificial Sequence
<220>
   <223> Made in a lab
<400> 202
   gcctactgcc tgaactgctt 20
<210> 203
   <211> 20
   <212> DNA
   <213> Artificial Sequence
<220>
   <223> Made in a lab
<400> 203
   ccgttcctca aaggagatgt 20
<210> 204
   <211> 20
   <212> DNA
   <213> Artificial Sequence
<220>
   <223> Made in a lab
<400> 204
   ccctggagga gtgctatgtc 20
<210> 205
   <211> 18
   <212> DNA
   <213> Artificial Sequence
<220>
   <223> Made in a lab
<400> 205
   gtgccgttga acttgacg 18
<210> 206
   <211> 21
   <212> DNA
   <213> Artificial Sequence
<220>
   <223> Made in a lab
<400> 206
   ccaagacgta gatccatcca a 21
<210> 207
   <211> 20
   <212> DNA
   <213> Artificial Sequence
<220>
   <223> Made in a lab
<400> 207
   tctgaggttg gtcctgaggt 20
<210> 208
   <211> 20
   <212> DNA
   <213> Artificial Sequence
<220>
   <223> Made in a lab
<400> 208
   gtgatctttg ccaccttcgt 20
<210> 209
   <211> 20
   <212> DNA
   <213> Artificial Sequence
<220>
   <223> Made in a lab
<400> 209
   cagtctgcca ctccgagttt 20
<210> 210
   <211> 21
   <212> DNA
   <213> Artificial Sequence
<220>
   <223> Made in a lab
<400> 210
   tgctgctaga tttgactgca a 21
<210> 211
   <211> 20
   <212> DNA
   <213> Artificial Sequence
<220>
   <223> Made in a lab
<400> 211
   tcccgtcctc atcgtagtct 20
<210> 212
   <211> 21
   <212> DNA
   <213> Artificial Sequence
<220>
   <223> Made in a lab
<400> 212
   agtgtgaggc agtggaaaac t 21
<210> 213
   <211> 20
   <212> DNA
   <213> Artificial Sequence
<220>
   <223> Made in a lab
<400> 213
   ccagggtacc agaccaagtg 20
<210> 214
   <211> 24
   <212> DNA
   <213> Artificial Sequence
<220>
   <223> Made in a lab
<400> 214
   gacattccat tctatcacaa cctg 24
<210> 215
   <211> 18
   <212> DNA
   <213> Artificial Sequence
<220>
   <223> Made in a lab
<400> 215
   gcgggtcatt tcactgag 18
<210> 216
   <211> 20
   <212> DNA
   <213> Artificial Sequence
<220>
   <223> Made in a lab
<400> 216
   tctgaggcca tcattgaaca 20
<210> 217
   <211> 20
   <212> DNA
   <213> Artificial Sequence
<220>
   <223> Made in a lab
<400> 217
   tcaggggctt cttcttcttg 20
<210> 218
   <211> 20
   <212> DNA
   <213> Artificial Sequence
<220>
   <223> Made in a lab
<400> 218
   cgttgaaaac atgccgtcag 20
<210> 219
   <211> 20
   <212> DNA
   <213> Artificial Sequence
<220>
   <223> Made in a lab
<400> 219
   cttgccaaag gtgctttctg 20
<210> 220
   <211> 20
   <212> DNA
   <213> Artificial Sequence
<220>
   <223> Made in a lab
<400> 220
   tatcctttgc cgtctcatca 20
<210> 221
   <211> 20
   <212> DNA
   <213> Artificial Sequence
<220>
   <223> Made in a lab
<400> 221
   ggctttcact cctcctgagc 20
<210> 222
   <211> 20
   <212> DNA
   <213> Artificial Sequence
<220>
   <223> Made in a lab
<400> 222
   agagtttgcc gagaggtctg 20
<210> 223
   <211> 20
   <212> DNA
   <213> Artificial Sequence
<220>
   <223> Made in a lab
<400> 223
   tggtccaagg tctggtgaat 20
<210> 224
   <211> 20
   <212> DNA
   <213> Artificial Sequence
<220>
   <223> Made in a lab
<400> 224
   ttacagtccc tgcccgatac 20
<210> 225
   <211> 20
   <212> DNA
   <213> Artificial Sequence
<220>
   <223> Made in a lab
<400> 225
   atgctgggtc tttggaacac 20
<210> 226
   <211> 20
   <212> DNA
   <213> Artificial Sequence
<220>
   <223> Made in a lab
<400> 226
   cagctgacag aaagcctgaa 20
<210> 227
   <211> 20
   <212> DNA
   <213> Artificial Sequence
<220>
   <223> Made in a lab
<400> 227
   tccggttctg cttttcactt 20
<210> 228
   <211> 20
   <212> DNA
   <213> Artificial Sequence
<220>
   <223> Made in a lab
<400> 228
   gcggaggagg agaaagagat 20
<210> 229
   <211> 20
   <212> DNA
   <213> Artificial Sequence
<220>
   <223> Made in a lab
<400> 229
   actagggtcc tcgtgtggtg 20
<210> 230
   <211> 20
   <212> DNA
   <213> Artificial Sequence
<220>
   <223> Made in a lab
<400> 230
   gttgagcagc atctcattgg 20
<210> 231
   <211> 22
   <212> DNA
   <213> Artificial Sequence
<220>
   <223> Made in a lab
<400> 231
   tgtggtccga ggcagtgaga gt 22
<210> 232
   <211> 21
   <212> DNA
   <213> Artificial Sequence
<220>
   <223> Made in a lab
<400> 232
   acagagcagg cctcgaggtg t 21
<210> 233
   <211> 18
   <212> DNA
   <213> Artificial Sequence
<220>
   <223> Made in a lab
<400> 233
   acgacgctct tccgatct 18
<210> 234
   <211> 18
   <212> DNA
   <213> Artificial Sequence
<220>
   <223> Made in a lab
<400> 234
   cgtgtgctct tccgatct 18
<210> 235
   <211> 1587
   <212> DNA
   <213> Homo sapiens
<400> 235
   cggattgtca gactgctcct ccctcttctt tagactgcca cgaggaaaaa gcagatgtga     60
   gaactcaagg ttcagggctg ctcttctaag aaacaagtct gccataatct ccatctgtgt    120
   tggaatctgt taactaatga actggtctct gtgcaaatcc tgagtgctaa agcttccaac    180
   aagactgatg ctagctcgtg tcaccaggaa gatgctacgt catgccaagt gttttcagcg    240
   cctagcaatt tttggttctg tgagggcact gcataaagat aatagaacag caacccctca    300
   gaatttctcc aactatgaat ccatgaaaca ggacttcaaa ctggggattc cagagtattt    360
   caactttgct aaagatgtcc tggaccaatg gactgataag gaaaaggctg gaaagaaacc    420
   ttcaaatcca gccttctggt ggatcaacag aaatggagaa gagatgcgat ggagttttga    480
   ggaactggga tctctgtcca gaaaatttgc caatatactt tcagaagcct gttccctaca    540
   aagaggagat cgggtaattc tgattctgcc cagggtccca gagtggtggc ttgcaaatgt    600
   ggcctgtctg cgaacaggga cagttttaat tccaggaacc actcagctga cccagaaaga    660
   cattctctac agactacaat cttcaaaagc aaactgcatt atcaccaatg atgttttagc    720
   cccagcagta gacgctgttg catccaaatg tgaaaatctg cactccaagc tgattgtatc    780
   agagaactcc agagaggggt gggggaacct caaggagttg atgaaacatg ccagtgacag    840
   ccacacctgt gtgaagacaa aacacaatga gatcatggcc atattcttta ccagtggaac    900
   aagtggatat ccgaaaatga ctgcacacac ccacagcagt tttggtttag gattatctgt    960
   aaatggaagg ttctggctag atttgacacc ctcagatgtg atgtggaata cctcagatac   1020
   gggctgggca aagtctgcat ggagtagtgt tttttctccg tggatccagg gagcatgtgt   1080
   attcacacac catttacccc gttttgagcc gacttctatc ttgcaaacac tctccaagta   1140
   ccccatcaca gtcttctgtt cagcaccaac tgtataccga atgcttgtac agaatgatat   1200
   aaccagctat aagtttaaaa gcttaaagca ctgtgtgagt gctggggaac caattacccc   1260
   tgacgtgact gaaaaatgga gaaacaagac gggcctggat atctacgaag gatatggaca   1320
   gactgaaacg gtgctaatct gtggaaattt taagggaatg aaaattaaac ctggctcaat   1380
   gggaaaacct tctcctgctt tcgatgttaa ggtttgcaca tccccttcca ggagaatgtt   1440
   taacaaccca atctgtacac tacctaccta ccgcttaccc ccatataaac tttctttgtt   1500
   atgatggtga ttccatttta cttccatgat actttaattt ttataaatat gtgaaaatga   1560
   ttagaaatga aaaaaaaaaa aaaaaaa                                       1587
<210> 236
   <211> 11373
   <212> DNA
   <213> Homo sapiens
<400> 236
   gtgcgcgggg cggcgccgcg gaacatgacg gcgccctggg tggccctcgc cctcctctgg     60
   ggatcgctgt gcgccggctc tgggcgtggg gaggctgaga cacgggagtg catctactac    120
   aacgccaact gggagctgga gcgcaccaac cagagcggcc tggagcgctg cgaaggcgag    180
   caggacaagc ggctgcactg ctacgcctcc tggcgcaaca gctctggcac catcgagctc    240
   gtgaagaagg gctgctggct agatgacttc aactgctacg ataggcagga gtgtgtggcc    300
   actgaggaga acccccaggt gtacttctgc tgctgtgaag gcaacttctg caacgaacgc    360
   ttcactcatt tgccagaggc tgggggcccg gaagtcacgt acgagccacc cccgacagcc    420
   cccaccctgc tcacggtgct ggcctactca ctgctgccca tcgggggcct ttccctcatc    480
   gtcctgctgg ccttttggat gtaccggcat cgcaagcccc cctacggtca tgtggacatc    540
   catgaggacc ctgggcctcc accaccatcc cctctggtgg gcctgaagcc actgcagctg    600
   ctggagatca aggctcgggg gcgctttggc tgtgtctgga aggcccagct catgaatgac    660
   tttgtagctg tcaagatctt cccactccag gacaagcagt cgtggcagag tgaacgggag    720
   atcttcagca cacctggcat gaagcacgag aacctgctac agttcattgc tgccgagaag    780
   cgaggctcca acctcgaagt agagctgtgg ctcatcacgg ccttccatga caagggctcc    840
   ctcacggatt acctcaaggg gaacatcatc acatggaacg aactgtgtca tgtagcagag    900
   acgatgtcac gaggcctctc atacctgcat gaggatgtgc cctggtgccg tggcgagggc    960
   cacaagccgt ctattgccca cagggacttt aaaagtaaga atgtattgct gaagagcgac   1020
   ctcacagccg tgctggctga ctttggcttg gctgttcgat ttgagccagg gaaacctcca   1080
   ggggacaccc acggacaggt aggcacgaga cggtacatgg ctcctgaggt gctcgaggga   1140
   gccatcaact tccagagaga tgccttcctg cgcattgaca tgtatgccat ggggttggtg   1200
   ctgtgggagc ttgtgtctcg ctgcaaggct gcagacggac ccgtggatga gtacatgctg   1260
   ccctttgagg aagagattgg ccagcaccct tcgttggagg agctgcagga ggtggtggtg   1320
   cacaagaaga tgaggcccac cattaaagat cactggttga aacacccggg cctggcccag   1380
   ctttgtgtga ccatcgagga gtgctgggac catgatgcag aggctcgctt gtccgcgggc   1440
   tgtgtggagg agcgggtgtc cctgattcgg aggtcggtca acggcactac ctcggactgt   1500
   ctcgtttccc tggtgacctc tgtcaccaat gtggacctgc cccctaaaga gtcaagcatc   1560
   taagcccagg acatgagtgt ctgtccagac tcagtggatc tgaagaaaaa aggaaaaaaa   1620
   gttgtgtttt gttttggaaa tcccataaaa ccaacaaaca cataaaatgc agctgctatt   1680
   ttaccttgac tttttattat tattattata attattataa ttattattat taatattatt   1740
   ttttggattg gatcagtttt taccagcata ttgctctact gtatcacaaa cagcggacac   1800
   gtcagcaggc gttgaggtgc tgagctgtgg atgcagaacc agcgccatgc tgaagagcct   1860
   cagccacctc ctgtcctttg ggattcgttt ttcccgcttt ctctttgttt gtcgtctcag   1920
   aatctgtgac acaaagaaac ccatctcctg tcttaggaaa cctaatgctg caaactctac   1980
   ctagaggaac ctttgaagac tgttacataa gaacatacct tcctcagaag aggagtttcc   2040
   tctgccctct gcccttctcc cctgcctccc tccctcccct ccttttattt tgttttagtg   2100
   agcttaagaa acagcagatg tgtctttcac ggatctaacg ggtgttgtcc tgatcgagaa   2160
   aaaaactggg atgagaatgg tttggactgg agttggaagg ggaggacggt actgggggta   2220
   gggtttggaa cagagctaca ctggactcgg gcacattcgg agcagcatcc tttagtatgg   2280
   aggctacttc tcaggtaacc aggaattgag gggaaggacc ttgtggaggc cgagcattaa   2340
   cagcaagagc ggggtttgga gaaagtctga gattgggtgc agccctgact tacctgctgg   2400
   ccctgaccag tttcttttca ctaacttggc cttgggcata ggatgaaaca ttttttctgc   2460
   cctaatttta aaactaggtg agggtagaat catcacaggt taggaataca ttcttcataa   2520
   gacacgatgc tgtaaatacc cttaatggac gaaaagttga aatacttttg tttcctcttg   2580
   gagcagttca gggaaatgcc cacaggggat tgtcctgcac agatagggca agaggatttc   2640
   ctgggtggag tctgccaagg cctgcctcgc tggggacccc agagtcctgc acctctggtt   2700
   ccgccccagg tggtgacatt actgtccccg ttctgtggct cgtggacaag actttctcca   2760
   gaccccttaa agtggtacat attctaaaaa actgtttttc tattatgcca taaccttgct   2820
   ctagtcagtg aatgttccta atgctgctgt ttcaacattt gaattctttt taatttatga   2880
   aacatgctaa attttttttt tcaaacaaaa cacacacatc cacatataca catgcttcgc   2940
   tatgtggctt ccaaggttta aattttgaaa agtaaaagaa ttaaaacttc acgaccacag   3000
   atcacctcaa accagaaata cctcagaatt ttctacttat gtaaggttta ttatatattt   3060
   tgttagttgt gttgtcttgt agtaagtata ttttaatgta agttggcttt tgtgacaagg   3120
   aagtttaaaa gaaatagaga aaaagaaaaa agtttgcatc ttctagggag tgctaccatt   3180
   tttgtttgat aacgccccct tgtaaataat tgtcatcaac tgtaggttgg ctgtctgggc   3240
   caagtctggg catttatcag tcttgtttgt gaaggctttt ccttctggtt tctttagatc   3300
   attttattta aaaacagtgc atctcttcat cgtgagggta ggcaaggcgg gggccgtggg   3360
   gagaggttga cctgggtgag aactgaagag gccgcctcct cttgggttgt ttggagcttc   3420
   acatgtaatt cacatgtaac atgtaacttg atcggtcagt gttcagaatg acaagtaacc   3480
   ccgcttaaac ttggtagaag gatggccctt agacctgaat ggggtgattt tacttgggat   3540
   ttaacttctt cagcaaatta acagcaacgt tggaagagat ctgtggcgcc tctgtgaagc   3600
   acaccgtgac tcaggccagt cttttagtgc agcgtgtctg ggagtgaagg gttttgccct   3660
   tgctggtctt ggagtccaca gtgtgagggg cactgcacat gcctgggcat ctacctagtg   3720
   tgctatgttc agtgtctggg gcttactgcc ccggggtcct ttcctctggg tgttggggca   3780
   cagggtgcta tgggaggccc atttgcttcc ctctcggagc tcagtttttg cttcatgggt   3840
   caaaatgtgg gctggccaag tggttacagg aacagggttt cggtaagcta tgttgtcttt   3900
   tttttttttt tttttttttt ttttaatggt ttgattttgt gctgtggtat tttttttccc   3960
   ttagaataat ttttaatggc aaaacaggcc ttacagcagt tgcttttctt taccatttat   4020
   ttctttaaga agctttaaaa tatttattga aaagtgccat atctaatttc tttagctttc   4080
   gcctcaggca gtgcaggcat ctttactttt catcctcaga agaaacaaac gactaacaaa   4140
   tgtagcaaat ttactgcagg aatagttagg tcatgatact acctgaacac taaaccccag   4200
   cctctttgtt tggttttagt tcctctgggt ggtttttctt ttgtgtgctg gcttgattct   4260
   tgtgagaagt tttgacctgg ccaagggagg gttgagccat ggttctggtg tgggactttg   4320
   cggtcaagac acagtacaga caggtcaggc ctgcgtgcct tttctctggg tggcctcccc   4380
   gttaggccca ccgtacgctc agccactata gtgtccctgt ggggccttgc catcagattg   4440
   tgtgtcagga gatggtacct ttttggtgtg gctggggagg agtgtggtcc atgccagttc   4500
   tttgggcttc aggccactct tcccctcatg ctgtggtgta aagtgcaccc atcaggtggt   4560
   atatctggtt ctgatggcaa gaagaaggtg ggggatctcc ttatagggca tgggtctagg   4620
   agcacagatg ggccttttgc cccgggtaaa tgcttgtctg tttgctgtca tgtgttcttt   4680
   gaggagtgag ccatctcgag ccctgctttg aatttactgg gtcatagagc ctctgcctgt   4740
   gctcttttcc ataatgactt catgtgacat gcacttttgg tgggctcaga taattggttt   4800
   ctttttgttt ttgacctcag gctctgtggc agactgggga aaatggggcc tggcatcatt   4860
   ttccctgtca atgggagggg ctgttccatg cagggtggga ggggaccaag ttagcagaga   4920
   gtagccaagg atccttgctt cttcctttct agtgtgctgt catccaagca ggctcctggc   4980
   tgtagggatg ggccttgggg aagaatcttc tttgaaagca tctatgataa ctgagaagtc   5040
   atccctagtt ggagaaatcc agtaatgagc agaaggagga agcaagtgag gacagaggcc   5100
   attgtattac agtgtcacgc agagggccct caatgatggg gcattgggga aggctgtaga   5160
   catagtcatc agaacatcct ggcctggcat aagctgggtt ttctcctggg accattggtc 5220
   ctcagcagga gttctttgca tgagttgctc aggggcaagg gctgcaagtg ggctgtgctt 5280
   aggagaaagt gacacctggc agtgagggaa gatggtgagc attattagcc tttgttgtcc 5340
   agcatggcct tcttgtcctg tctgctctgg agaggagcct gtgggaccag tcctgcctgg 5400
   ggagggcata cccacacgtg ccagctgatt ctgactctga atacatcatg tccggacttg 5460
   ggggtgtttc tgcagaaaaa ggaggttgtt tttcagcctt gaacatcttc aggaggatag 5520
   agactcttgc tcacatattc ttagcaaagg gaagggtctc tcatctccag gccacagaga 5580
   tagttcttcc attgccctaa gaggctaggc taaccctctt gacataactt agacagcaaa 5640
   gcacttcatc ctgtagttgg gctctgtcac ctttctcttc agttggccac attctcgttt 5700
   cctccatcct gctatgcttt gtgtgctcgg gctgtgtgtg gggtttttcc ctggtggaag 5760
   gaagcccagc tgtgtattga atgtccttca tgtgttgtgt gtggctcaga aagcctgtca 5820
   cttggcccct gtgctctgag ccgtgagggt ggggaggtgg ctgttccatt aaagtgggag 5880
   tattggatgg ccctcttgaa actagaattt tgcctttttt agtatgcagt ataaagtttc 5940
   cagcatctat tggtaacaca aagatttgct ggtttttaaa ataatacagt aagcataagt 6000
   atgtaagttt ttagaattgg tactagaagt tggacagcta gttattctcg agaactttat 6060
   ttcactagaa aaatatacta attggaaagc agtttccagg agttaactca gtttaatttt 6120
   cagtctcagt tattttagcc tgttgagttt ttgatggcac acctttggag agatggccac 6180
   gcctgattcc catttcaggg gcatcagacc ataccttttt aagaagctcc gtgaatctag 6240
   tcatctaccc ttcatcctgg gcgaacagcc aaaaagagaa ggggacaagg tgtctttttc 6300
   tccttctcac tggggtgaca tgaattcttt tagttaatgg ctgtttgcaa attctaaact 6360
   aatgaaatac ttagcagcta acatgttcaa tctagtaatg atgagtttaa atctcaattg 6420
   acagtaatgt tttagataaa caggcccagt aattcagttg atgaactgta tatcttctca 6480
   gtctagattt gtaaatgttt aatgaattca gggttataag catagttctt taagtaagat 6540
   tccagatagt tgatttgcaa ccagcagtct acctatgaat gtatcccaaa cctttagaag 6600
   attggaaaag atttttgaaa taatgattta gttttgtagg aaaaacaccc ccttgaaaat 6660
   taattcggtt gacccagtaa cattttttaa aacaattggt ggctccaaaa ggcctgccaa 6720
   caaagaaaag tccaaattat ctagtgggac attttgaatg ttttatgttt attttgggtc 6780
   cactgtaaac tttggttcaa aaaagaattt gaatttaaag aatttaccat tatttaaatt 6840
   attaccaagt ttttacattt tcatgatggt attttccagg tatgaatgaa acatgacttt 6900
   ttgattgtgg tacttcctgt atcccctgta gtgccaaaac cagtgatact ttatttgctc 6960
   ctatggcagc tcatagaggt aaccgaagtg atttttcctc agtaattgaa acacatattc 7020
   tctaaatgcc aatgtgtggt gatgggccct gcactgcctt catttctcta gggcagtgtc 7080
   tttggattgt ctagggccta ggtaattctg agaactactg taaaccaacc acagggcact 7140
   aaagcaatgt acacaccact ctttgtgtgt atggaagggg ttatataaac ctgggctatg 7200
   ctggacatct acagaagagt attacattca cttgcaaagt ttacattttt gagctcacag 7260
   ttatgaaaaa tatgacccac aagtttttca ggcaggtgag gatgggtctt cttgcaaatg 7320
   catgagttct gtcttgagtc ctgggaactt ctctgttggt tgagtgtggg ctcattccct 7380
   gactctccta atcatgtttg cgtcagaatg ttagcattgt aaataaaaga ataggttgta 7440
   taatagatac acaacacttg aaactttact ttaaaaaaat cgatagttct acatatatat 7500
   ttagttatat cacttgacag atttcttcta cacagtgtgg agattgtttt ataccacaga 7560
   ttatttttat aaagttagtg aatttgaatg attttgtaat cagagctaat gagctttacc 7620
   tttcaagaga aacgtacact ggagcatgag tggtgtggaa cttttactta gtgtttatat 7680
   ggattcttgt gatacactgg cagactggag tcaatttgcg ggtctttttt ggccaaaact 7740
   ccacttgtgg ttgtgtagga cagtgatatt cagctcagct tcttgtggat tgggaggaga 7800
   gagggcctgc aatgtgtttt acattggtgc ttcctcctga gatttctgtt gaacaaaggg 7860
   ttctgaggtc aaaaattagt ttgtaagcct ttgccatagg acatagtcat gtgagagtgt 7920
   ttgggggaac agaaattgta taggggtgcc tattggggtg ggatgggact cgaataagat 7980
   tcaggtacaa aaactttgaa atgagaatct ggtggtttga gtaatccacc agactgaatt 8040
   atctaagatc acattatcca ggttgggggg cagaattacc cagttaagta attgttcaga 8100
   aaagtgggga gggtggcatg tggatgcagt gatccaatta aatggagagc tgccaggcac 8160
   attttgtcct ctctggtcag tgagaatggt tgggttggct cgctgcttca atctgtggaa 8220
   tcagccagga gcccagtgag gaagctcaga accccagtaa cagcagagca tctttcagat 8280
   agctccagag ttttcctgct tttctgagga agctcagcat cactgccaca atacggaaag 8340
   tggtcttcat tttagcctat ttatttttag gcagagagtg gatggttatt tgtgtgggac 8400
   ttttggtggc gatatataat gaataattaa gttaatttct ggtatgcata atggccagtc 8460
   ctgaggccca gctgaagacc tgtcccccag accctgcccg ctggcttcag gctgctgctt 8520
   ctagacagag gtgcactgga cgggatagtt ttatcaagag aatccctaat gtgtcatttt 8580
   aaaccagctg tgctttttat tcattctggt tgagcgtata ggtttacact ttaccctttt 8640
   tatacttgga ataaatttag ttccagcaga tctagtagca ctccagaaac caaccccatc 8700
   tgttccccat aaaaagaaca ttttctctgc tctccagcca cgtgtcttgg aatgtaattc 8760
   tgttgtgcct ttgtttttat cactctcttc gccccaaaag caactgctgt aagctttttt 8820
   ctacttgtct tttctagtcc ccaacctcta cctttttcct ttttcccagc cctaatttct 8880
   ggatgcactt ctgtgatcca ggtattttaa gaaccagtta cctcagacct catgttgaac 8940
   agtgtcgcca tctgggtcct cttgatactg cagactttta acgtacacat gcaggaaccc 9000
   tgctgagcgt gggcacttgt tttaaagcaa aactcttccc aaggactgaa gaaagggctt 9060
   ctggcaagct cgtcatggca ttgtggtggg atgggtctag agtgtcatct gaatggtgct 9120
   tcctgtgttc ctctttgaat tctgccattt tcagtattct tgtgtgtctg aataggcaaa 9180
   gcgatttaat tggctggtct tgcacgcaaa ttagttccaa agataagctc tttgtaacac 9240
   atttccagtc gctaatgctc aaatgtagaa cattccttta aatggcagga taaaaaaccc    9300
   actatccacc atagtgcatt ttgggaagat gtctgtagca tatgttgctg tgaaattagg    9360
   ccttgtggga tatggctgtt tgtcattttg atgtatttta aataaatata tatatttttt     9420
   aaagagcctt ttttaccagt tcaaaaagtt taattaacca gcagtcaccg catctgaatt    9480
   tttgtctctg gggcatagat ggcagaccaa gattaaaagt ggtaactcag ctatacgagc    9540
   atgggctacc ttcctgggct ctcctgcagt cctgtagacc tgctgttccg cagaccatgg    9600
   gacacaaggt cagtgtgttc ccagtgaggg tcccaagtca gtcatcttaa gtgtttgttc    9660
   tctgccccat tcagtggact gttgacttca gtccctgcaa gtgctttagc ccgagtgggg    9720
   ttttctcaga gcactgccac gagttaagtg tgtgtttagc caaataattt ctccgtaagg    9780
   gaaaaatgca gtcacccaaa ttttaccaac aatgacagag atgagagtag aaaagattag    9840
   gcaacatctg agttttaact tgaaaagtgt ccaagtcatc atgaaaggcc gactgggagc    9900
   aagtgattat tagagattct tcaggagacc tcatctgaaa atgttaagac tgccagtgag    9960
   ggaaggaatt gttaaaatgc cagcggcttt tttttcctct ttttttctgt aattctgtaa    10020
   aaatgcagag aaagttgagt ggtacttcag aattgaggga gagggttacc gcagagtaga    10080
   aatatatttc tagatttcag ttccacacca caaatccaca acaatgccat ttttcaactg    10140
   tacaaaaatc tgcttatgaa ctggacatga tcttaatggt agtgtcaaag gccaagtttt    10200
   tcacctgtta atatttttcc acatttgtcc ttgaatctga ataactttat acagtactgt    10260
   aaatttaact tacatcgagt ttgttgtcaa ttcttatgaa aagagctttc tgcatgtaac    10320
   acatacggtt aaagaacaca gcaaaggaca aaatttgcag gaacagtttt ggaaccaaca    10380
   gaaaatgtca ccttttattt gccatcttat atatatctat cagttttacc agctacttct    10440
   aaatttgtac attatttgta agggaaagaa ggaaaaccct aagacttgtc taacttagtg    10500
   gagaatgtgt gtgttgggct taggatggat agctaagtct tattgagctg tgttacctaa    10560
   cttgtatata aaaattgtaa ttaaaagttt gggttcacct gtttctcaca gtttaaaatg    10620
   atgagtaatt gcaaactctg gaaatgtgac tagtatatga tttaaggctg tagaagcaag    10680
   gaagctcttt caagtgctaa aactaaagac ttctagtttt tggctcaaat aagtactgtt    10740
   tgtataccag gatatgtgag atgtaaatgt agtaggtcac ttttcaccct tgtagctata    10800
   aaataaaaat tttgtagaac agaaatagct tgtactactg aattaacaaa agttatacta    10860
   aagtatcatg tttaaaaaaa atatatatat atatacagag ttaagcttgt tgctgttacc    10920
   ctgtctggat ttgaaaagtg tgctgattta tatatatata ttacacacac acacacacac    10980
   acacacacac acacacacac acacacacac acacacacac acacacacac acacatacac    11040
   ctaaaatggc ctaaagcaga catccatgta attacagttg caaaatgaaa acattttgga    11100
   aagaacattg tatcatagtt cattcatttg cagtggatct ttgttccttt ttactgtggt    11160
   aattttagaa atgagtgtca agtttgaaat tagatctgct aagttggggt tttgctgctt    11220
   gaactctgca ctgggtcctc aaataaaccg atgtgaatgt agttttttcc ccctgtgtga    11280
   agaagcagtt acaccccaac aataggagga aaaatctaga actatttcaa gttttatctt    11340
   tttgtatatg aaaataaaat aataataaaa caa                                 11373
<210> 237
   <211> 3352
   <212> DNA
   <213> Homo sapiens
<400> 237
   ggggcgtggc gccggggatt gggagggctt cttgcaggct gctgggctgg ggctaagggc      60
   tgctcagttt ccttcagcgg ggcactggga agcgccatgg cactgcaggg catctcggtc     120
   gtggagctgt ccggcctggc cccgggcccg ttctgtgcta tggtcctggc tgacttcggg     180
   gcgcgtgtgg tacgcgtgga ccggcccggc tcccgctacg acgtgagccg cttgggccgg     240
   ggcaagcgct cgctagtgct ggacctgaag cagccgcggg gagccgccgt gctgcggcgt     300
   ctgtgcaagc ggtcggatgt gctgctggag cccttccgcc gcggtgtcat ggagaaactc     360
   cagctgggcc cagagattct gcagcgggaa aatccaaggc ttatttatgc caggctgagt     420
   ggatttggcc agtcaggaag cttctgccgg ttagctggcc acgatatcaa ctatttggct     480
   ttgtcaggtg ttctctcaaa aattggcaga agtggtgaga atccgtatgc cccgctgaat     540
   ctcctggctg actttgctgg tggtggcctt atgtgtgcac tgggcattat aatggctctt     600
   tttgaccgca cacgcactgg caagggtcag gtcattgatg caaatatggt ggaaggaaca     660
   gcatatttaa gttcttttct gtggaaaact cagaaattga gtctgtggga agcacctcga     720
   ggacagaaca tgttggatgg tggagcacct ttctatacga cttacaggac agcagatggg     780
   gaattcatgg ctgttggagc aatagaaccc cagttctacg agctgctgat caaaggactt     840
   ggactaaagt ctgatgaact tcccaatcag atgagcatgg atgattggcc agaaatgaag     900
   aagaagtttg cagatgtatt tgcagagaag acgaaggcag agtggtgtca aatctttgac     960
   ggcacagatg cctgtgtgac tccggttctg acttttgagg aggttgttca tcatgatcac    1020
   aacaaggaac ggggctcgtt tatcaccagt gaggagcagg acgtgagccc ccgccctgca    1080
   cctctgctgt taaacacccc agccatccct tctttcaaaa gggatccttt cataggagaa    1140
   cacactgagg agatacttga agaatttgga ttcagccgcg aagagattta tcagcttaac    1200
   tcagataaaa tcattgaaag taataaggta aaagctagtc tctaacttcc aggcccacgg    1260
   ctcaagtgaa tttgaatact gcatttacag tgtagagtaa cacataacat tgtatgcatg    1320
   gaaacatgga ggaacagtat tacagtgtcc taccactcta atcaagaaaa gaattacaga    1380
   ctctgattct acagtgatga ttgaattcta aaaatggtta tcattagggc ttttgattta    1440
   taaaactttg ggtacttata ctaaattatg gtagttattc tgccttccag tttgcttgat    1500
   atatttgttg atattaagat tcttgactta tattttgaat gggttctagt gaaaaaggaa    1560
   tgatatattc ttgaagacat cgatatacat ttatttacac tcttgattct acaatgtaga    1620
   aaatgaggaa atgccacaaa ttgtatggtg ataaaagtca cgtgaaacag agtgattggt    1680
   tgcatccagg ccttttgtct tggtgttcat gatctccctc taagcacatt ccaaacttta    1740
   gcaacagtta tcacactttg taatttgcaa agaaaagttt cacctgtatt gaatcagaat    1800
   gccttcaact gaaaaaaaca tatccaaaat aatgaggaaa tgtgttggct cactacgtag    1860
   agtccagagg gacagtcagt tttagggttg cctgtatcca gtaactcggg gcctgtttcc    1920
   ccgtgggtct ctgggctgtc agctttcctt tctccatgtg tttgatttct cctcaggctg    1980
   gtagcaagtt ctggatctta tacccaacac acagcaacat ccagaaataa agatctcagg    2040
   accccccagc aagtcgtttt gtgtctcctt ggactgagtt aagttacaag cctttcttat     2100
   acctgtcttt gacaaagaag acgggattgt ctttacataa aaccagcctg ctcctggagc    2160
   ttccctggac tcaacttcct aaaggcatgt gaggaagggg tagattccac aatctaatcc    2220
   gggtgccatc agagtagagg gagtagagaa tggatgttgg gtaggccatc aataaggtcc    2280
   attctgcgca gtatctcaac tgccgttcaa caatcgcaag aggaaggtgg agcaggtttc    2340
   ttcatcttac agttgagaaa acagagactc agaagggctt cttagttcat gtttccctta    2400
   gcgcctcagt gattttttca tggtggctta ggccaaaaga aatatctaac cattcaattt    2460
   ataaataatt aggtccccaa cgaattaaat attatgtcct accaacttat tagctgcttg    2520
   aaaaatataa tacacataaa taaaaaaata tatttttcat ttctatttca ttgttaatca    2580
   caactactta ctaaggagat gtatgcacct attggacact gtgcaacttc tcacctggaa    2640
   tgagattgga cactgctgcc ctcattttct gctccatgtt ggtgtccata tagtacttga    2700
   ttttttatca gatggcctgg aaaacccagt ctcacaaaaa tatgaaatta tcagaaggat     2760
   tatagtgcaa tcttatgttg aaagaatgaa ctacctcact agtagttcac gtgatgtctg    2820
   acagatgttg agtttcattg tgtttgtgtg ttcaaatttt taaatattct gagatactct    2880
   tgtgaggtca ctctaatgcc ctgggtgcct tggcacagtt ttagaaatac cagttgaaaa    2940
   tatttgctca ggaatatgca actaggaagg ggcagaatca gaatttaagc tttcatattc    3000
   tagccttcag tcttgttctt caaccatttt taggaacttt cccataaggt tatgttttcc    3060
   agcccaggca tggaggatca cttgaggcca agagttcgag accagcctgg ggaacttggc    3120
   tggacctccg tttctacgaa ataaaaataa aaaaattatc caggtatggt ggtgtgtgcc    3180
   tgtagtccta tctactcaag ggtggggcag gaggatcact tgagcccagg aatttgaggc    3240
   cacagtgaat taggattgca ccactgcact ctagcccagg caacagaaca agaacctgtc    3300
   tctaaataaa taaataaaaa taataataat aaaaaagatg ttttccctac aa            3352
<210> 238
   <211> 2065
   <212> DNA
   <213> Homo sapiens
<400> 238
<210> 239
   <211> 533
   <212> DNA
   <213> Homo sapiens
<400> 239
<210> 240
   <211> 1864
   <212> DNA
   <213> Homo sapiens
<400> 240
   ctggcggctc ttgggactgg cggggctgcg cgcggggtta gggtgggggt acgggaaggc      60
   tcaacccagg acctgcgtac cttgctttgg gggcgcacta agcacctgcc gggagcaggg     120
   ggcgcaccgg gaactcgcag atttcgccag ttgggcgcac tggggatctg tggactgcgt     180
   ccgggggatg ggctaggggg acatgcgcac gctttgggcc ttacagaatg tgatcgcgcg     240
   agggggaggg cgaagcgtgg cgggagggcg aggcgaagga aggagggcgt gagaaaggcg     300
   acggcggcgg cgcggaggag ggttatctat acatttaaaa accagccgcc tgcgccgcgc     360
   ctgcggagac ctgggagagt ccggccgcac gcgcgggaca cgagcgtccc acgctccctg     420
   gcgcgtacgg cctgccacca ctaggcctcc tatccccggg ctccagacga cctaggacgc     480
   gtgccctggg gagttgcctg gcggcgccgt gccagaagcc cccttggggc gccacagttt     540
   tccccgtcgc ctccggttcc tctgcctgca ccttcctgcg gcgcgccggg acctggagcg     600
   ggcgggtgga tgcaggcgcg atggacggcg gcacactgcc caggtccgcg ccccctgcgc     660
   cccccgtccc tgtcggctgc gctgcccggc ggagacccgc gtccccggaa ctgttgcgct     720
   gcagccggcg gcggcgaccg gccaccgcag agaccggagg cggcgcagcg gccgtagcgc     780
   ggcgcaatga gcgcgagcgc aaccgcgtga agctggtgaa cttgggcttc caggcgctgc     840
   ggcagcacgt gccgcacggc ggcgccagca agaagctgag caaggtggag acgctgcgct     900
   cagccgtgga gtacatccgc gcgctgcagc gcctgctggc cgagcacgac gccgtgcgca     960
   acgcgctggc gggagggctg aggccgcagg ccgtgcggcc gtctgcgccc cgcgggccgc    1020
   cagggaccac cccggtcgcc gcctcgccct cccgcgcttc ttcgtccccg ggccgcgggg    1080
   gcagctcgga gcccggctcc ccgcgttccg cctactcgtc ggacgacagc ggctgcgaag    1140
   gcgcgctgag tcctgcggag cgcgagctac tcgacttctc cagctggtta gggggctact    1200
   gagcgccctc gacctatgag cctcagcccc ggaagccgag cgagcggccg gcgcgctcat    1260
   cgccggggag cccgccaggt ggaccggccc gcgctccgcc cccagcgagc cggggaccca    1320
   cccaccaccc cccgcaccgc cgacgccgcc tcgttcgtcc ggcccagcct gaccaatgcc    1380
   gcggtggaaa cgggcttgga gctggcccca taagggctgg cggcttcctc cgacgccgcc    1440
   cctccccaca gcttctcgac tgcagtgggg cggggggcac caacacttgg agatttttcc    1500
   ggaggggaga ggattttcta agggcacaga gaatccattt tctacacatt aacttgagct    1560
   gctggaggga cactgctggc aaacggagac ctatttttgt acaaagaacc cttgacctgg    1620
   ggcgtaataa agatgacctg gacccctgcc cccactatct ggagttttcc atgctggcca    1680
   agatctggac acgagcagtc cctgaggggc ggggtccctg gcgtgaggcc cccgtgacag    1740
   cccaccctgg ggtgggtttg tgggcactgc tgctctgcta gggagaagcc tgtgtggggc    1800
   acacctcttc aagggagcgt gaactttata aataaatcag ttctgtttaa aaaaaaaaaa    1860
   aaaa                                                                 1864
<210> 241
   <211> 948
   <212> DNA
   <213> Homo sapiens
<400> 241
   acatcctgga agagtggcct aggacagctc ctctcctgcc agagctaggc aggcgccgaa      60
   gtagccgcat ggccccgtca gaagatccca gggactggag agccaacctc aaaggcacca     120
   tccgtgagac aggcctggag accagctccg gtgggaagct ggctggccat cagaagaccg     180
   tccccacggc tcacctgact tttgttattg actgcaccca cgggaagcag ctctccctgg     240
   cagcaaccgc atcaccaccc caagccccca gtcccaatcg agggcttgtc accccaccaa     300
   tgaagactta catcgtgttc tgtggggaaa actggcccca tctgactcgg gtgaccccca     360
   tgggtggggg atgccttgcc caggccaggg ccaccctgcc gctctgcaga gggtctgtgg     420
   cctcagcttc cttcccagtc agcccgctct gcccccagga ggttcccgag gctaagggga     480
   aacccgtgaa ggctgcgcct gtgaggtctt caacttgggg aacagtcaag gactcactga     540
   aagccctctc ctcttgtgtc tgtgggcagg ccgattagct ggaagggccg ggctctgatg     600
   cccagaggct gcaattccca gggcctggcc ctgcttcccc agctaagcag gagtcttttg     660
   tgcttgagcc aaggaaacat cattagatcc gctaaggggc atctgaaaca tccgtcgagt     720
   ggcagaggca ggataagtca cctgcacatg aagagactca ttcattcata cagcaaatat     780
   tactggtaca tcttccacat gccaggccct gcaaagtgct ggggagatac catggttttc     840
   ctggagctgg tatttttggg gtggagggaa cccaccctga ataaataaag taacccaata     900
   aataaagaag atgatttcga aaaaaaaaaa aaaaaaaaaa aaaaaaaa                  948
<210> 242
   <211> 1432
   <212> DNA
   <213> Homo sapiens
<400> 242
   ggggcgtgct cgcggctata aggggcggag gctgggcggc gttgctctgc gctctgcggc      60
   tgacggcgct tttgtctccg gtgagttttg tggcgggaag cttctgcgct ggtgcttagt     120
   aaccgacttt cctccggact cctgcacgac ctgctcctac agccggcgat ccactcccgg     180
   ctgttccccc ggagggtcca gaggcctttc agaaggagaa ggcagctctg tttctctgca     240
   gaggagtagg gtcctttcag ccatgaagca tgtgttgaac ctctacctgt taggtgtggt     300
   actgacccta ctctccatct tcgttagagt gatggagtcc ctagagggct tactagagag     360
   cccatcgcct gggacctcct ggaccaccag aagccaacta gccaacacag agcccaccaa     420
   gggccttcca gaccatccat ccagaagcat gtgataagac ctccttccat actggccata     480
   ttttggaaca ctgacctaga catgtccaga tgggagtccc attcctagca gacaagctga     540
   gcaccgttgt aaccagagaa ctattactag gccttgaaga acctgtctaa ctggatgctc     600
   attgcctggg caaggcctgt ttaggccggt tgcggtggct catgcctgta atcctagcac     660
   tttgggaggc tgaggtgggt ggatcacctg aggtcaggag ttcgagacca gcctcgccaa     720
   catggcgaaa ccccatctct actaaaaata caaaagttag ctgggtgtgg tggcagaggc     780
   ctgtaatccc agctccttgg gaggctgagg cgggagaatt gcttgaaccc ggggacggag     840
   gttgcagtga gccgagatcg cactgctgta cccagcctgg gccacagtgc aagactccat     900
   ctcaaaaaaa aaagaaaaga aaaagcctgt ttaatgcaca ggtgtgagtg gattgcttat     960
   ggctatgaga taggttgatc tcgcccttac cccggggtct ggtgtatgct gtgctttcct    1020
   cagcagtatg gctctgacat ctcttagatg tcccaacttc agctgttggg agatggtgat    1080
   attttcaacc ctacttccta aacatctgtc tggggttcct ttagtcttga atgtcttatg    1140
   ctcaattatt tggtgttgag cctctcttcc acaagagctc ctccatgttt ggatagcagt    1200
   tgaagaggtt gtgtgggtgg gctgttggga gtgaggatgg agtgttcagt gcccatttct    1260
   cattttacat tttaaagtcg ttcctccaac atagtgtgta ttggtctgaa gggggtggtg    1320
   ggatgccaaa gcctgctcaa gttatggaca ttgtggccac catgtggctt aaatgatttt    1380
   ttctaactaa taaagtggaa tatatatttc taaaaaaaaa aaaaaaaaaa aa            1432
<210> 243
   <211> 4880
   <212> DNA
   <213> Homo sapiens
<400> 243
   aacgggcgcc gcggcgggga gaagacgcag agcgctgctg ggctgccggg tctcccgctt      60
   ccccctcctg ctccaagggc ctcctgcatg agggcgcggt agagacccgg acccgcgccg     120
   tgctcctgcc gtttcgctgc gctccgcccg ggcccggctc agccaggccc cgcggtgagc     180
   catgattcgc ctcggggctc cccagacgct ggtgctgctg acgctgctcg tcgccgctgt     240
   ccttcggtgt cagggccagg atgtccggca accaggacca aagggacaga aaggagaacc     300
   tggagacatc aaggatattg taggacccaa aggacctcct gggcctcagg gacctgcagg     360
   ggaacaagga cccagagggg atcgtggtga caaaggtgaa aaaggtgccc ctggacctcg     420
   tggcagagat ggagaacctg ggacccctgg aaatcctggc ccccctggtc ctcccggccc     480
   ccctggtccc cctggtcttg gtggaaactt tgctgcccag atggctggag gatttgatga     540
   aaaggctggt ggcgcccagt tgggagtaat gcaaggacca atgggcccca tgggacctcg     600
   aggacctcca ggccctgcag gtgctcctgg gcctcaagga tttcaaggca atcctggtga     660
   acctggtgaa cctggtgtct ctggtcccat gggtccccgt ggtcctcctg gtccccctgg     720
   aaagcctggt gatgatggtg aagctggaaa acctggaaaa gctggtgaaa ggggtccgcc     780
   tggtcctcag ggtgctcgtg gtttcccagg aaccccaggc cttcctggtg tcaaaggtca 840
   cagaggttat ccaggcctgg acggtgctaa gggagaggcg ggtgctcctg gtgtgaaggg 900
   tgagagtggt tccccgggtg agaacggatc tccgggccca atgggtcctc gtggcctgcc 960
   tggtgaaaga ggacggactg gccctgctgg cgctgcgggt gcccgaggca acgatggtca 1020
   gccaggcccc gcagggcctc cgggtcctgt cggtcctgct ggtggtcctg gcttccctgg 1080
   tgctcctgga gccaagggtg aagccggccc cactggtgcc cgtggtcctg aaggtgctca 1140
   aggtcctcgc ggtgaacctg gtactcctgg gtcccctggg cctgctggtg cctccggtaa 1200
   ccctggaaca gatggaattc ctggagccaa aggatctgct ggtgctcctg gcattgctgg 1260
   tgctcctggc ttccctgggc cacggggccc tcctggccct caaggtgcaa ctggtcctct 1320
   gggcccgaaa ggtcagacgg gtgaacctgg tattgctggc ttcaaaggtg aacaaggccc 1380
   caagggagaa cctggccctg ctggccccca gggagcccct ggacccgctg gtgaagaagg 1440
   caagagaggt gcccgtggag agcctggtgg cgttgggccc atcggtcccc ctggagaaag 1500
   aggtgctccc ggcaaccgcg gtttcccagg tcaagatggt ctggcaggtc ccaagggagc 1560
   ccctggagag cgagggccca gtggtcttgc tggccccaag ggagccaacg gtgaccctgg 1620
   ccgtcctgga gaacctggcc ttcctggagc ccggggtctc actggccgcc ctggtgatgc 1680
   tggtcctcaa ggcaaagttg gcccttctgg agcccctggt gaagatggtc gtcctggacc 1740
   tccaggtcct cagggggctc gtgggcagcc tggtgtcatg ggtttccctg gccccaaagg 1800
   tgccaacggt gagcctggca aagctggtga gaagggactg cctggtgctc ctggtctgag 1860
   gggtcttcct ggcaaagatg gtgagacagg tgctgcagga ccccctggcc ctgctggacc 1920
   tgctggtgaa cgaggcgagc agggtgctcc tgggccatct gggttccagg gacttcctgg 1980
   ccctcctggt cccccaggtg aaggtggaaa accaggtgac cagggtgttc ccggtgaagc 2040
   tggagcccct ggcctcgtgg gtcccagggg tgaacgaggt ttcccaggtg aacgtggctc 2100
   tcccggtgcc cagggcctcc agggtccccg tggcctcccc ggcactcctg gcactgatgg 2160
   tcccaaaggt gcatctggcc cagcaggccc ccctggggct cagggccctc caggtcttca 2220
   gggaatgcct ggcgagaggg gagcagctgg tatcgctggg cccaaaggcg acaggggtga 2280
   cgttggtgag aaaggccctg agggagcccc tggaaaggat ggtggacgag gcctgacagg 2340
   tcccattggc ccccctggcc cagctggtgc taatggcgag aagggagaag ttggacctcc 2400
   tggtcctgca ggaagtgctg gtgctcgtgg cgctccgggt gaacgtggag agactgggcc 2460
   ccccggacca gcgggatttg ctgggcctcc tggtgctgat ggccagcctg ggccaagggg 2520
   tgagcaagga gaggccggcc agaaaggcga tgctggtgcc cctggtcctc agggcccctc 2580
   tggagcacct gggcctcagg gtcctactgg agtgactggt cctaaaggag cccgaggtgc 2640
   ccaaggcccc ccgggagcca ctggattccc tggagctgct ggccgcgttg gacccccagg 2700
   ctccaatggc aaccctggac cccctggtcc ccctggtcct tctggaaaag atggtcccaa 2760
   aggtgctcga ggagacagcg gcccccctgg ccgagctggt gaacccggcc tccaaggtcc 2820
   tgctggaccc cctggcgaga agggagagcc tggagatgac ggtccctctg gtgccgaagg 2880
   tccaccaggt ccccagggtc tggctggtca gagaggcatc gtcggtctgc ctgggcaacg 2940
   tggtgagaga ggattccctg gcttgcctgg cccgtcgggt gagcccggca agcagggtgc 3000
   tcctggagca tctggagaca gaggtcctcc tggccccgtg ggtcctcctg gcctgacggg 3060
   tcctgcaggt gaacctggac gagagggaag ccccggtgct gatggccccc ctggcagaga 3120
   tggcgctgct ggagtcaagg gtgatcgtgg tgagactggt gctgtgggag ctcctggagc 3180
   ccctgggccc cctggctccc ctggccccgc tggtccaact ggcaagcaag gagacagagg 3240
   agaagctggt gcacaaggcc ccatgggacc ctcaggacca gctggagccc ggggaatcca 3300
   gggtcctcaa ggccccagag gtgacaaagg agaggctgga gagcctggcg agagaggcct 3360
   gaagggacac cgtggcttca ctggtctgca gggtctgccc ggccctcctg gtccttctgg 3420
   agaccaaggt gcttctggtc ctgctggtcc ttctggccct agaggtcctc ctggccccgt 3480
   cggtccctct ggcaaagatg gtgctaatgg aatccctggc cccattgggc ctcctggtcc 3540
   ccgtggacga tcaggcgaaa ccggccctgc tggtcctcct ggaaatcctg gaccccctgg 3600
   tcctccaggt ccccctggcc ctggcatcga catgtccgcc tttgctggct taggcccgag 3660
   agagaagggc cccgaccccc tgcagtacat gcgggccgac caggcagccg gtggcctgag 3720
   acagcatgac gccgaggtgg atgccacact caagtccctc aacaaccaga ttgagagcat 3780
   ccgcagcccc gagggctccc gcaagaaccc tgctcgcacc tgcagagacc tgaaactctg 3840
   ccaccctgag tggaagagtg gagactactg gattgacccc aaccaaggct gcaccttgga 3900
   cgccatgaag gttttctgca acatggagac tggcgagact tgcgtctacc ccaatccagc 3960
   aaacgttccc aagaagaact ggtggagcag caagagcaag gagaagaaac acatctggtt 4020
   tggagaaacc atcaatggtg gcttccattt cagctatgga gatgacaatc tggctcccaa 4080
   cactgccaac gtccagatga ccttcctacg cctgctgtcc acggaaggct cccagaacat 4140
   cacctaccac tgcaagaaca gcattgccta tctggacgaa gcagctggca acctcaagaa 4200
   ggccctgctc atccagggct ccaatgacgt ggagatccgg gcagagggca atagcaggtt 4260
   cacgtacact gccctgaagg atggctgcac gaaacatacc ggtaagtggg gcaagactgt 4320
   tatcgagtac cggtcacaga agacctcacg cctccccatc attgacattg cacccatgga 4380
   cataggaggg cccgagcagg aattcggtgt ggacataggg ccggtctgct tcttgtaaaa 4440
   acctgaaccc agaaacaaca caatccgttg caaacccaaa ggacccaagt actttccaat 4500
   ctcagtcact ctaggactct gcactgaatg gctgacctga cctgatgtcc attcatccca 4560
   ccctctcaca gttcggactt ttctcccctc tctttctaag agacctgaac tgggcagact 4620
   gcaaaataaa atctcggtgt tctatttatt tattgtcttc ctgtaagacc ttcgggtcaa 4680
   ggcagaggca ggaaactaac tggtgtgagt caaatgcccc ctgagtgact gcccccagcc 4740
   caggccagaa gacctccctt caggtgccgg gcgcaggaac tgtgtgtgtc ctacacaatg 4800
   gtgctattct gtgtcaaaca cctctgtatt ttttaaaaca tcaattgata ttaaaaatga 4860
   aaagattatt ggaaagtaca 4880
<210> 244
   <211> 2831
   <212> DNA
   <213> Homo sapiens
<400> 244
   agcgccggga gactctgccg tcggtgcgtg cgcggacacg cacccgtccc ccttggtctc 60
   gccgccagcc atggccgccg ctacggcctc cccccgcagc ctccttgttc tcctccaggt 120
   ggtagtgctc gctctggcgc agattagagg tccaccggga gagcggggcc ccccgggtcc 180
   cccgggaccg ccgggagtgc ctggatccga cggcatcgac ggtgacaatg ggccccctgg 240
   aaaagctggc cctccgggac ccaagggcga gcctggcaaa gctgggccag atgggccaga 300
   cgggaagccc gggattgatg gtttaactgg agccaagggg gagcctggcc ccatggggat 360
   ccctggagtc aagggccagc ccgggcttcc tggtcctcct ggccttccgg gccctggttt 420
   tgctggacct cctgggcctc ctggacctgt tggcctccct ggtgagattg gaatccgagg 480
   ccccaagggg gaccctggac cagatggacc atcggggccc ccaggacccc ctgggaaacc 540
   tggtcgcccg ggaaccatcc agggtctgga aggcagtgcg gatttcctgt gtccaaccaa 600
   ctgtccaccc ggaatgaaag gtcccccagg gctgcaggga gtgaaggggc atgcgggcaa 660
   acgcgggatt ctgggtgatc ctggccacca ggggaagccg ggtcccaagg gagatgtggg 720
   tgcctctgga gagcaaggca tccctggacc accgggtccc cagggcatca ggggctaccc 780
   aggcatggca gggcccaagg gagagacggg ccctcatgga tataaaggca tggtgggcgc 840
   tatcggtgcc actgggccac cgggtgagga aggtcctagg ggaccgccag gccgagctgg 900
   ggagaagggt gacgagggca gcccaggtat tcgtggaccc caggggatca caggcccgaa 960
   aggagcaacg ggccccccag gcatcaacgg caaggatggg accccaggca cgcctggcat 1020
   gaagggcagt gcaggacagg cgggacagcc cggaagtcca ggccaccagg gcctagcggg 1080
   tgtgccaggc cagcctggga caaaaggagg ccctggagac cagggtgagc cgggcccgca 1140
   gggccttcct ggattctctg gtccccctgg gaaagaggga gagccagggc ctcgaggaga 1200
   aattggtccc cagggcatca tgggacagaa gggtgaccaa ggcgagaggg gtccagtggg 1260
   gcaaccaggc cctcagggaa ggcagggccc taagggggag cagggccccc ccggaattcc 1320
   agggccccaa ggcttgccag gcgtcaaagg agacaagggc tccccaggga agaccgggcc 1380
   ccgcggcaaa gtgggtgacc caggggtggc cggcctcccc ggagagaaag gcgagaaggg 1440
   cgagtccggc gagccggggc ccaagggaca gcaaggagta cgtggagaac ccggctaccc 1500
   tggccccagc ggggatgcgg gcgccccagg ggttcagggc taccctggtc cccccggccc 1560
   tcgaggactg gccgggaacc gaggcgtgcc aggacagccc gggagacagg gcgtggaggg 1620
   ccgggatgcc actgaccagc acatcgtgga tgtggcgctg aagatgctgc aagagcaact 1680
   ggcagaggtc gccgtgagtg ccaagcggga agccctgggt gcggtgggca tgatgggtcc 1740
   tccaggacct cctgggcccc ctgggtaccc aggcaagcag ggcccccatg ggcaccctgg 1800
   ccctcggggc gttcctggca tcgtgggagc cgtgggtcag atcggcaaca cggggcccaa 1860
   gggaaaacgt ggagagaagg gtgatccagg agaagtggga cgggggcacc ccgggatgcc 1920
   tgggccccca gggatcccag gactccctgg ccggcctggc caggcaatca acggcaagga 1980
   tggagatcga gggtccccag gggctccagg agaggcaggt cgacctggcc tgccaggccc 2040
   cgtggggctg ccgggcttct gtgaacctgc cgcctgcctt ggagcttcgg cctatgcctc 2100
   tgcccgcctt acagagcctg gatccatcaa ggggccttga gcatcaggcc cagacagagc 2160
   ctggcaggca tcctggcggg aaggaccagg tcccctctgg gtggacatgc acccatcccc 2220
   agtccaggaa accatctccc ccaggacctt ctgtctggga ctcaggagtc ctaaggaaaa 2280
   ggaattctaa aacatggggg aaggggaggt agagcactga tgggtgaaaa agtgaggcca 2340
   acacacaggg caagtggtgt cgatggagtc gaagcgctga aggaataggg cggctttcct 2400
   tccagcgagc atcattcggc tgttaccaaa acaaacatct taatctgcac ctttctccac 2460
   tggccatctt gtccttgggt cagtgggaca tgggcacctc gggaggcccg ggccctgccc 2520
   agctacagtt ccacccctca gcttgaggac caatgactga ggtctatgcc agttcctgat 2580
   cccatctcac tctctggacc taccaggtga ctgctgctgg gtgactcccc tgaggcggct 2640
   atacccttaa gccagcccca ctacttcctt ccctgcctcc cagctcagta tttaaacatc 2700
   atctcccttc tctttctcgc ataactcccc acccctttct ccccgatcca cccaggcctt 2760
   tctgtaaata aaagctccca agttgggtac aaaccaggat attggagtta ctctatcctg 2820
   gagttaacta g 2831
<210> 245
   <211> 2471
   <212> DNA
   <213> Homo sapiens
<400> 245
   agaaagcgag cagccaccca gctccccgcc accgccatgg tccccgacac cgcctgcgtt      60
   cttctgctca ccctggctgc cctcggcgcg tccggacagg gccagagccc gttgggctca     120
   gacctgggcc cgcagatgct tcgggaactg caggaaacca acgcggcgct gcaggacgtg     180
   cgggagctgc tgcggcagca ggtcagggag atcacgttcc tgaaaaacac ggtgatggag     240
   tgtgacgcgt gcgggatgca gcagtcagta cgcaccggcc tacccagcgt gcggcccctg     300
   ctccactgcg cgcccggctt ctgcttcccc ggcgtggcct gcatccagac ggagagcggc     360
   gcgcgctgcg gcccctgccc cgcgggcttc acgggcaacg gctcgcactg caccgacgtc     420
   aacgagtgca acgcccaccc ctgcttcccc cgagtccgct gtatcaacac cagcccgggg     480
   ttccgctgcg aggcttgccc gccggggtac agcggcccca cccaccaggg cgtggggctg     540
   gctttcgcca aggccaacaa gcaggtttgc acggacatca acgagtgtga gaccgggcaa     600
   cataactgcg tccccaactc cgtgtgcatc aacacccggg gctccttcca gtgcggcccg     660
   tgccagcccg gcttcgtggg cgaccaggcg tccggctgcc agcggcgcgc acagcgcttc     720
   tgccccgacg gctcgcccag cgagtgccac gagcatgcag actgcgtcct agagcgcgat     780
   ggctcgcggt cgtgcgtgtg tgccgttggc tgggccggca acgggatcct ctgtggtcgc     840
   gacactgacc tagacggctt cccggacgag aagctgcgct gcccggagcg ccagtgccgt     900
   aaggacaact gcgtgactgt gcccaactca gggcaggagg atgtggaccg cgatggcatc     960
   ggagacgcct gcgatccgga tgccgacggg gacggggtcc ccaatgaaaa ggacaactgc    1020
   ccgctggtgc ggaacccaga ccagcgcaac acggacgagg acaagtgggg cgatgcgtgc    1080
   gacaactgcc ggtcccagaa gaacgacgac caaaaggaca cagaccagga cggccggggc    1140
   gatgcgtgcg acgacgacat cgacggcgac cggatccgca accaggccga caactgccct    1200
   agggtaccca actcagacca gaaggacagt gatggcgatg gtatagggga tgcctgtgac    1260
   aactgtcccc agaagagcaa cccggatcag gcggatgtgg accacgactt tgtgggagat    1320
   gcttgtgaca gcgatcaaga ccaggatgga gacggacatc aggactctcg ggacaactgt    1380
   cccacggtgc ctaacagtgc ccaggaggac tcagaccacg atggccaggg tgatgcctgc    1440
   gacgacgacg acgacaatga cggagtccct gacagtcggg acaactgccg cctggtgcct    1500
   aaccccggcc aggaggacgc ggacagggac ggcgtgggcg acgtgtgcca ggacgacttt    1560
   gatgcagaca aggtggtaga caagatcgac gtgtgtccgg agaacgctga agtcacgctc    1620
   accgacttca gggccttcca gacagtcgtg ctggacccgg agggtgacgc gcagattgac    1680
   cccaactggg tggtgctcaa ccagggaagg gagatcgtgc agacaatgaa cagcgaccca    1740
   ggcctggctg tgggttacac tgccttcaat ggcgtggact tcgagggcac gttccatgtg    1800
   aacacggtca cggatgacga ctatgcgggc ttcatctttg gctaccagga cagctccagc    1860
   ttctacgtgg tcatgtggaa gcagatggag caaacgtatt ggcaggcgaa ccccttccgt    1920
   gctgtggccg agcctggcat ccaactcaag gctgtgaagt cttccacagg ccccggggaa    1980
   cagctgcgga acgctctgtg gcatacagga gacacagagt cccaggtgcg gctgctgtgg    2040
   aaggacccgc gaaacgtggg ttggaaggac aagaagtcct atcgttggtt cctgcagcac    2100
   cggccccaag tgggctacat cagggtgcga ttctatgagg gccctgagct ggtggccgac    2160
   agcaacgtgg tcttggacac aaccatgcgg ggtggccgcc tgggggtctt ctgcttctcc    2220
   caggagaaca tcatctgggc caacctgcgt taccgctgca atgacaccat cccagaggac    2280
   tatgagaccc atcagctgcg gcaagcctag ggaccagggt gaggacccgc cggatgacag    2340
   ccaccctcac cgcggctgga tgggggctct gcacccagcc ccaaggggtg gccgtcctga    2400
   gggggaagtg agaagggctc agagaggaca aaataaagtg tgtgtgcagg gaaaaaaaaa    2460
   aaaaaaaaaa a                                                         2471
<210> 246
   <211> 4334
   <212> DNA
   <213> Homo sapiens
<400> 246
   gcaataaatg ctcgcgaacg cgagagagcg acggaagcgc taggcgcctg gttctgcgcg      60
   tactggctgt acggagcagg agcaagaggt cgccgccagc ctccgccgcc gagcctcgtt     120
   cgtgtccccg cccctcgctc ctgcagctac tgctcagaaa cgctggggcg cccaccctgg     180
   cagactaacg aagcagctcc cttcccaccc caactgcagg tctaattttg gacgctttgc     240
   ctgccatttc ttccaggttg agggagccgc agaggcggag gctcgcgtat tcctgcagtc     300
   agcacccacg tcgcccccgg acgctcggtg ctcaggccct tcgcgagcgg ggctctccgt     360
   ctgcggtccc ttgtgaaggc tctgggcggc tgcagaggcc ggccgtccgg tttggctcac     420
   ctctcccagg aaacttcaca ctggagagcc aaaaggagtg gaagagcctg tcttggagat     480
   tttcctgggg aaatcctgag gtcattcatt atgaagtgta ccgcgcggga gtggctcaga     540
   gtaaccacag tgctgttcat ggctagagca attccagcca tggtggttcc caatgccact     600
   ttattggaga aacttttgga aaaatacatg gatgaggatg gtgagtggtg gatagccaaa     660
   caacgaggga aaagggccat cacagacaat gacatgcaga gtattttgga ccttcataat     720
   aaattacgaa gtcaggtgta tccaacagcc tctaatatgg agtatatgac atgggatgta     780
   gagctggaaa gatctgcaga atcctgggct gaaagttgct tgtgggaaca tggacctgca     840
   agcttgcttc catcaattgg acagaatttg ggagcacact ggggaagata taggcccccg     900
   acgtttcatg tacaatcgtg gtatgatgaa gtgaaagact ttagctaccc atatgaacat     960
   gaatgcaacc catattgtcc attcaggtgt tctggccctg tatgtacaca ttatacacag    1020
   gtcgtgtggg caactagtaa cagaatcggt tgtgccatta atttgtgtca taacatgaac    1080
   atctgggggc agatatggcc caaagctgtc tacctggtgt gcaattactc cccaaaggga    1140
   aactggtggg gccatgcccc ttacaaacat gggcggccct gttctgcttg cccacctagt    1200
   tttggagggg gctgtagaga aaatctgtgc tacaaagaag ggtcagacag gtattatccc    1260
   cctcgagaag aggaaacaaa tgaaatagaa cgacagcagt cacaagtcca tgacacccat    1320
   gtccggacaa gatcagatga tagtagcaga aatgaagtca taagcgcaca gcaaatgtcc    1380
   caaattgttt cttgtgaagt aagattaaga gatcagtgca aaggaacaac ctgcaatagg    1440
   tacgaatgtc ctgctggctg tttggatagt aaagctaaag ttattggcag tgtacattat     1500
   gaaatgcaat ccagcatctg tagagctgca attcattatg gtataataga caatgatggt     1560
   ggctgggtag atatcactag acaaggaaga aagcattatt tcatcaagtc caatagaaat     1620
   ggtattcaaa caattggcaa atatcagtct gctaattcct tcacagtctc taaagtaaca     1680
   gttcaggctg tgacttgtga aacaactgtg gaacagctct gtccatttca taagcctgct     1740
   tcacattgcc caagagtata ctgtcctcgt aactgtatgc aagcaaatcc acattatgct     1800
   cgtgtaattg gaactcgagt ttattctgat ctgtccagta tctgcagagc agcagtacat     1860
   gctggagtgg ttcgaaatca cggtggttat gttgatgtaa tgcctgtgga caaaagaaag     1920
   acctacattg cttcttttca gaatggaatc ttctcagaaa gtttacagaa tcctccagga     1980
   ggaaaggcat tcagagtgtt tgctgttgtg tgaaactgaa tacttggaag aggaccataa     2040
   agactattcc aaatgcaata tttctgaatt ttgtataaaa ctgtaacatt actgtacaga     2100
   gtacatcaac tattttcagc ccaaaaaggt gccaaatgca tataaatctt gataaacaaa     2160
   gtctataaaa taaaacatgg gacattagct ttgggaaaag taatgaaaat ataatggttt     2220
   tagaaatcct gtgttaaata ttgctatatt ttcttagcag ttatttctac agttaattac     2280
   atagtcatga ttgttctacg tttcatatat tatatggtgc tttgtatatg ccactaataa     2340
   aatgaatcta aacattgaat gtgaatggcc ctcagaaaat catctagtgc atttaaaaat     2400
   aatcgactct aaaactgaaa gaaaccttat cacattttcc ccagttcaat gctatgccat     2460
   taccaactcc aaataatctc aaataatttt ccacttaata actgtaaagt ttttttctgt     2520
   taatttaggc atatagaata ttaaattctg atattgcact tcttatttta tataaaataa     2580
   tcctttaata tccaaatgaa tctgttaaaa tgtttgattc cttgggaatg gccttaaaaa     2640
   taaatgtaat aaagtcagag tggtggtatg aaaacattcc tagtgatcat gtagtaaatg     2700
   tagggttaag catggacagc cagagctttc tatgtactgt taaaattgag gtcacatatt     2760
   ttcttttgta tcctggcaaa tactcctgca ggccaggaag tataatagca aaaagttgaa     2820
   caaagatgaa ctaatgtatt acattaccat tgccactgat ttttttttaa atggtaaatg     2880
   accttgtata taaatattgc catatcatgg tacctataat ggtgatatat ttgtttctat     2940
   gaaaaatgta ttgtgctttg atactaaaaa tctgtaaaat gttagttttg gtaatttttt     3000
   ttctgctggt ggatttacat attaaatttt ttctgctggt ggataaacat taaaattaat     3060
   catgtttcaa agttttattt tcagttcctt ttgcatgcct attttgattt agaaatcact     3120
   ttaagataaa tgaacaaaat tattgtaagt cttctaaact tggtttattg acgttagtat     3180
   aaataacata caataccaga tgtctacaaa atcgacctga ttatttaggt atttgtatgt     3240
   gaaagagaaa cacatattta gaaacacagc aagggagatt ttgaataaag agagagatga     3300
   atttataaag taggaaaaaa agaatctgaa agatacaaag tgattataaa agaattgaca     3360
   ggacaataaa tacttcaaat acttttagga agtaaatgag taggagttta ggagggataa     3420
   acgagataaa ttccatgcaa tgatcaagga aagcaagaga gcagataata caaaagaaaa     3480
   agaagaaggt tcacataaat agcttctgga gtgcaaatta taaagacatc aacatttttg     3540
   atcatataat aagtactggc ttacagctaa gtggttccaa atactggtaa ttaaaacaat     3600
   gatgtatacg ttaaagattg caaaacttaa aagcagattt ttatgggaac ttttttcgaa     3660
   aggatacatg accaccttct taaatagtat gactttacat agactctttt tctggtcctt     3720
   actgctcctc ccacaacagg gaagcccgat cagttctgtc agtataaggt tttcttctat     3780
   ttgcccttag tttagatctc catcatcaat tccacttact attgtaatac cttcagctgg     3840
   agcctgtgtc tttctccatt cctttttaat acattcgtat ttcccagccc cagaacccct     3900
   gaaattaact cttccatgag ttttcattgc tgaagaaaaa ccaaacattc ttgtaaaaac     3960
   atttaagaac ttccatagtc ttgttcatat tgtttaccta tcttctacac aaggcgtgtc     4020
   cttgggtaag ttatttaact tcaatttacc ttacctgtaa aatggaacaa ataatatctc     4080
   cttaagattg ctctacgaat taattgacac attatgtgtg atgtacttaa aggaataata     4140
   aatgcataat atgaggtcag tggacgccat tttctgttat aatcattttt gttatctaat     4200
   ttggggaaac tattcatctg tcatatacta ttgatccttt ggcacttaaa taaatgtgaa     4260
   atcctccagg aattttcctg tgattctcct tgtaagaatt aaatgctctc taatatgata     4320
   tccctttaaa aaaa                                                       4334
<210> 247
   <211> 2024
   <212> DNA
   <213> Homo sapiens
<400> 247
   aaggcaagag atctaggact tctagcccct gaactttcag ccgaatacat cttttccaaa 60
   ggagtgaatt caggcccttg tatcactggc agcaggacgt gaccatggag aagctgttgt 120
   gtttcttggt cttgaccagc ctctctcatg cttttggcca gacagacatg tcgaggaagg 180
   cttttgtgtt tcccaaagag tcggatactt cctatgtatc cctcaaagca ccgttaacga 240
   agcctctcaa agccttcact gtgtgcctcc acttctacac ggaactgtcc tcgacccgtg 300
   ggtacagtat tttctcgtat gccaccaaga gacaagacaa tgagattctc atattttggt 360
   ctaaggatat aggatacagt tttacagtgg gtgggtctga aatattattc gaggttcctg 420
   aagtcacagt agctccagta cacatttgta caagctggga gtccgcctca gggatcgtgg 480
   agttctgggt agatgggaag cccagggtga ggaagagtct gaagaaggga tacactgtgg 540
   gggcagaagc aagcatcatc ttggggcagg agcaggattc cttcggtggg aactttgaag 600
   gaagccagtc cctggtggga gacattggaa atgtgaacat gtgggacttt gtgctgtcac 660
   cagatgagat taacaccatc tatcttggcg ggcccttcag tcctaatgtc ctgaactggc 720
   gggcactgaa gtatgaagtg caaggcgaag tgttcaccaa accccagctg tggccctgag 780
   gcccagctgt gggtcctgaa ggtacctccc ggttttttac accgcatggg ccccacgtct 840
   ctgtctctgg tacctcccgc ctttttacac tgcatggttc ccacgtctct gtctctgggc 900
   ctttgttccc ctatatgcat tgcaggcctg ctccaccctc ctcagcgcct gagaatggag 960
   gtaaagtgtc tggtctggga gctcgttaac tatgctggga aacggtccaa aagaatcaga 1020
   atttgaggtg ttttgttttc atttttattt caagttggac agatcttgga gataatttct 1080
   tacctcacat agatgagaaa actaacaccc agaaaggaga aatgatgtta taaaaaactc 1140
   ataaggcaag agctgagaag gaagcgctga tcttctattt aattccccac ccatgacccc 1200
   cagaaagcag gagggcattg cccacattca cagggctctt cagtctcaga atcaggacac 1260
   tggccaggtg tctggtttgg gtccagagtg ctcatcatca tgtcatagaa ctgctgggcc 1320
   caggtctcct gaaatgggaa gcccagcaat accacgcagt ccctccactt tctcaaagca 1380
   cactggaaag gccattagaa ttgccccagc agagcagatc tgcttttttt ccagagcaaa 1440
   atgaagcact aggtataaat atgttgttac tgccaagaac ttaaatgact ggtttttgtt 1500
   tgcttgcagt gctttcttaa ttttatggct cttctgggaa actcctcccc ttttccacac 1560
   gaaccttgtg gggctgtgaa ttctttcttc atccccgcat tcccaatata cccaggccac 1620
   aagagtggac gtgaaccaca gggtgtcctg tcagaggagc ccatctccca tctccccagc 1680
   tccctatctg gaggatagtt ggatagttac gtgttcctag caggaccaac tacagtcttc 1740
   ccaaggattg agttatggac tttgggagtg agacatcttc ttgctgctgg atttccaagc 1800
   tgagaggacg tgaacctggg accaccagta gccatcttgt ttgccacatg gagagagact 1860
   gtgaggacag aagccaaact ggaagtggag gagccaaggg attgacaaac aacagagcct 1920
   tgaccacgtg gagtctctga atcagccttg tctggaacca gatctacacc tggactgccc 1980
   aggtctataa gccaataaag cccctgttta cttgaaaaaa aaaa 2024
<210> 248
   <211> 782
   <212> DNA
   <213> Homo sapiens
<400> 248
   gggctccctg cctcgggctc tcaccctcct ctcctgcagc tccagctttg tgctctgcct 60
   ctgaggagac catggcccag tatctgagta ccctgctgct cctgctggcc accctagctg 120
   tggccctggc ctggagcccc aaggaggagg ataggataat cccgggtggc atctataacg 180
   cagacctcaa tgatgagtgg gtacagcgtg cccttcactt cgccatcagc gagtataaca 240
   aggccaccaa agatgactac tacagacgtc cgctgcgggt actaagagcc aggcaacaga 300
   ccgttggggg ggtgaattac ttcttcgacg tagaggtggg ccgcaccata tgtaccaagt 360
   cccagcccaa cttggacacc tgtgccttcc atgaacagcc agaactgcag aagaaacagt 420
   tgtgctcttt cgagatctac gaagttccct gggagaacag aaggtccctg gtgaaatcca 480
   ggtgtcaaga atcctaggga tctgtgccag gccattcgca ccagccacca cccactccca 540
   ccccctgtag tgctcccacc cctggactgg tggcccccac cctgcgggag gcctccccat 600
   gtgcctgcgc caagagacag acagagaagg ctgcaggagt cctttgttgc tcagcagggc 660
   gctctgccct ccctccttcc ttcttgcttc taatagccct ggtacatggt acacaccccc 720
   ccacctcctg caattaaaca gtagcatcgc ctccctctga aaaaaaaaaa aaaaaaaaaa 780
   aa 782
<210> 249
   <211> 449
   <212> DNA
   <213> Homo sapiens
<400> 249
   atatccactc ctgctctccc tcctgcaggt gaccccagcc atgaggacca tcgccatcct      60
   tgctgccatt ctcctggtgg ccctgcaggc ccaggctgag tcactccagg aaagagctga     120
   tgaggctaca acccagaagc agtctgggga agacaaccag gaccttgcta tctcctttgc     180
   aggaaatgga ctctctgctc ttagaacctc aggttctcag gcaagagcca cctgctattg     240
   ccgaaccggc cgttgtgcta cccgtgagtc cctctccggg gtgtgtgaaa tcagtggccg     300
   cctctacaga ctctgctgtc gctgagcttc ctagatagaa accaaagcag tgcaagattc     360
   agttcaaggt cctgaaaaaa gaaaaacatt ttactctgtg taccttgtgt ctttctaaat     420
   ttctctctcc aaaataaagt tcaagcatt                                       449
<210> 250
   <211> 4727
   <212> DNA
   <213> Homo sapiens
<400> 250
<210> 251
   <211> 1322
   <212> DNA
   <213> Homo sapiens
<400> 251
<210> 252
   <211> 1901
   <212> DNA
   <213> Homo sapiens
<400> 252
   gcggcgagtg gagcgggagc cgactggaag aagggctcta gggagggggc tgtggctgct      60
   ggggtccgag gtggggccgg gtacaccagc cccatcactg tttgcagaga gtcagggagg     120
   cggaaaagac acgcgctcta ggctcccatc agggcacatg gcccgggccc atcccccgcg     180
   cgtctccccg gctgcggggc gcggggggct gccgggtgcg cttggctgtg gcgcggcgcg     240
   ttggagactt tattgcgatg ggacgataag aggggcgggg gcggggtcct gggggccgag     300
   gcggcagcgc tttaattaaa acggaaattg cggccccggg ccgcgcgggg gccggagggt     360
   tccaagcggc cccttagctg gaagcgtttc tccaggaccc ccccgcaacc cccgccacgc     420
   ccgggctgcc ccctcccgcc aggccctgcc ggacccggcg ccgtcttctc ctccttgtca     480
   cccgcggtcg cttcgggcgg ggatcggtgc caccgagcgc aaagcctgcc tcgcccccct     540
   tccccgtccc ccccatctcc caccgcccag tccccggcgg cgatgagaca gagcggcgcc     600
   tcccagcccc tgctgatcaa catgtacctg ccagatcccg tcggagacgg tctcttcaag     660
   gacgggaaga acccgagctg ggggccgctg agccccgcgg ttcagaaagg cagcggacag     720
   atccagctgt ggcagtttct gctggagctg ctggctgacc gcgcgaacgc cggctgcatc     780
   gcgtgggagg gcggtcacgg cgagttcaag ctcacggacc cggacgaggt ggcgcggcgg     840
   tggggcgagc gcaagagcaa gcccaacatg aactacgaca agctgagccg cgccctgcgc     900
   tactactacg acaagaacat catgagcaag gtgcatggca agcgctacgc ctaccgcttc     960
   gacttccagg gcctggcgca ggcctgccag ccgccgcccg cgcacgctca tgccgccgcc    1020
   gcagctgctg ccgccgccgc ggccgcccag gacggcgcgc tctacaagct gcccgccggc    1080
   ctcgccccgc tgcccttccc cggcctctcc aaactcaacc tcatggccgc ctcggccggg    1140
   gtcgcgcccg ccggcttctc ctactggccg ggcccgggcc ccgccgccac cgctgccgcc    1200
   gccaccgccg cgctctaccc cagtcccagc ttgcagcccc cgcccgggcc cttcggggcc    1260
   gtggccgcag cctcgcactt ggggggccat taccactaga cggggcggtc gggtgcctgc    1320
   ggcctcgccc gcacgcctag agtctcgccc gatcccatcg gcatcccggg gagggcccgg    1380
   gagcctccgt caaccgtcct ctaatccaga gtttactcca cctgccgcac ttagcagggg    1440
   gacgggaccg aagctccctc aatccttgtc tggtactaga tttgctcctg tcccaccccg    1500
   cagtcccctg aggagggcga tgtgcgccct ctttcacttt ttttcttcta ggtctccagg    1560
   tcccggaggg gatttgtgga cctctcttgt ctccccacca ctccagtgca tttccgcctg    1620
   gctcctagaa gccccattca atatcactac tctttaacga gtgccaaatc ttttcccact    1680
   tttgctcttc cccaaggaac tgctcccacc tcagcacgtg gaggcctctc acggtcctcc    1740
   ttcctgggac ctgagcaggt ttggtgaaag ccaccgtcct ccgtgacaca cggccccctt    1800
   cctcctgtcc ccacactccc aggagaaact cccggtgtgt ttctgaccct ttcagcccca    1860
   ttaaagctcc tgagctctca aaaaaaaaaa aaaaaaaaaa a                        1901
<210> 253
   <211> 1401
   <212> DNA
   <213> Homo sapiens
<400> 253
   ggctgggact ggctgagcct ggcgggaggc ggggtccgag tcaccgcctg ccgccgcgcc      60
   cccggtttct ataaattgag cccgcagcct cccgcttcgc tctctgctcc tcctgttcga     120
   cagtcagccg catcttcttt tgcgtcgcca gccgagccac atcgctcaga caccatgggg     180
   aaggtgaagg tcggagtcaa cggatttggt cgtattgggc gcctggtcac cagggctgct     240
   tttaactctg gtaaagtgga tattgttgcc atcaatgacc ccttcattga cctcaactac     300
   atggtttaca tgttccaata tgattccacc catggcaaat tccatggcac cgtcaaggct     360
   gagaacggga agcttgtcat caatggaaat cccatcacca tcttccagga gcgagatccc     420
   tccaaaatca agtggggcga tgctggcgct gagtacgtcg tggagtccac tggcgtcttc     480
   accaccatgg agaaggctgg ggctcatttg caggggggag ccaaaagggt catcatctct     540
   gccccctctg ctgatgcccc catgttcgtc atgggtgtga accatgagaa gtatgacaac     600
   agcctcaaga tcatcagcaa tgcctcctgc accaccaact gcttagcacc cctggccaag     660
   gtcatccatg acaactttgg tatcgtggaa ggactcatga ccacagtcca tgccatcact     720
   gccacccaga agactgtgga tggcccctcc gggaaactgt ggcgtgatgg ccgcggggct     780
   ctccagaaca tcatccctgc ctctactggc gctgccaagg ctgtgggcaa ggtcatccct     840
   gagctgaacg ggaagctcac tggcatggcc ttccgtgtcc ccactgccaa cgtgtcagtg     900
   gtggacctga cctgccgtct agaaaaacct gccaaatatg atgacatcaa gaaggtggtg     960
   aagcaggcgt cggagggccc cctcaagggc atcctgggct acactgagca ccaggtggtc    1020
   tcctctgact tcaacagcga cacccactcc tccacctttg acgctggggc tggcattgcc    1080
   ctcaacgacc actttgtcaa gctcatttcc tggtatgaca acgaatttgg ctacagcaac    1140
   agggtggtgg acctcatggc ccacatggcc tccaaggagt aagacccctg gaccaccagc    1200
   cccagcaaga gcacaagagg aagagagaga ccctcactgc tggggagtcc ctgccacact    1260
   cagtccccca ccacactgaa tctcccctcc tcacagttgc catgtagacc ccttgaagag    1320
   gggaggggcc tagggagccg caccttgtca tgtaccatca ataaagtacc ctgtgctcaa    1380
   ccaaaaaaaa aaaaaaaaaa a                                              1401
<210> 254
   <211> 5822
   <212> DNA
   <213> Homo sapiens
<400> 254
   actttttgtt tttaaaacag cagcgcggct ctcagggatg actctgtgag actgggagga      60
   tcatagctgg gggaggctga gcgtgggagc ggtgctgcca gtcctgcctg aaaacgcgaa     120
   atgagtcttg cttggttctc cctccactgg gcgtgagagc ccctgcccag gaggcccagg     180
   acaaatggcc ccatagtgga aactgggaag cttttaggca tctgatcaga gcgggagcca     240
   gccgggggac cacagtgctg gacaggccaa ccaactcaaa cttgaagaca tgaaatcccc     300
   aaggagaacc actttgtgcc tcatgtttat tgtgatttat tcttccaaag ctgcactgaa     360
   ctggaattac gagtctacta ttcatccttt gagtcttcat gaacatgaac cagctggtga     420
   agaggcactg aggcaaaaac gagccgttgc cacaaaaagt cctacggctg aagaatacac     480
   tgttaatatt gagatcagtt ttgaaaatgc atccttcctg gatcctatca aagcctactt     540
   gaacagcctc agttttccaa ttcatgggaa taacactgac caaattaccg acattttgag     600
   cataaatgtg acaacagtct gcagacctgc tggaaatgaa atctggtgct cctgcgagac     660
   aggttatggg tggcctcggg aaaggtgtct tcacaatctc atttgtcaag agcgtgacgt     720
   cttcctccca gggcaccatt gcagttgcct taaagaactg cctcccaatg gacctttttg     780
   cctgcttcag gaagatgtta ccctgaacat gagagtcaga ctaaatgtag gctttcaaga     840
   agacctcatg aacacttcct ccgccctcta taggtcctac aagaccgact tggaaacagc     900
   gttccggaag ggttacggaa ttttaccagg cttcaagggc gtgactgtga cagggttcaa     960
   gtctggaagt gtggttgtga catatgaagt caagactaca ccaccatcac ttgagttaat    1020
   acataaagcc aatgaacaag ttgtacagag cctcaatcag acctacaaaa tggactacaa    1080
   ctcctttcaa gcagttacta tcaatgaaag caatttcttt gtcacaccag aaatcatctt    1140
   tgaaggggac acagtcagtc tggtgtgtga aaaggaagtt ttgtcctcca atgtgtcttg    1200
   gcgctatgaa gaacagcagt tggaaatcca gaacagcagc agattctcga tttacaccgc    1260
   acttttcaac aacatgactt cggtgtccaa gctcaccatc cacaacatca ctccaggtga 1320
   tgcaggtgaa tatgtttgca aactgatatt agacattttt gaatatgagt gcaagaagaa 1380
   aatagatgtt atgcccatcc aaattttggc aaatgaagaa atgaaggtga tgtgcgacaa 1440
   caatcctgta tctttgaact gctgcagtca gggtaatgtt aattggagca aagtagaatg 1500
   gaagcaggaa ggaaaaataa atattccagg aacccctgag acagacatag attctagctg 1560
   cagcagatac accctcaagg ctgatggaac ccagtgccca agcgggtcgt ctggaacaac 1620
   agtcatctac acttgtgagt tcatcagtgc ctatggagcc agaggcagtg caaacataaa 1680
   agtgacattc atctctgtgg ccaatctaac aataaccccg gacccaattt ctgtttctga 1740
   gggacaaaac ttttctataa aatgcatcag tgatgtgagt aactatgatg aggtttattg 1800
   gaacacttct gctggaatta aaatatacca aagattttat accacgagga ggtatcttga 1860
   tggagcagaa tcagtactga cagtcaagac ctcgaccagg gagtggaatg gaacctatca 1920
   ctgcatattt agatataaga attcatacag tattgcaacc aaagacgtca ttgttcaccc 1980
   gctgcctcta aagctgaaca tcatggttga tcctttggaa gctactgttt catgcagtgg 2040
   ttcccatcac atcaagtgct gcatagagga ggatggagac tacaaagtta ctttccatac 2100
   gggttcctca tcccttcctg ctgcaaaaga agttaacaaa aaacaagtgt gctacaaaca 2160
   caatttcaat gcaagctcag tttcctggtg ttcaaaaact gttgatgtgt gttgtcactt 2220
   taccaatgct gctaataatt cagtctggag cccatctatg aagctgaatc tggttcctgg 2280
   ggaaaacatc acatgccagg atcccgtaat aggtgtcgga gagccgggga aagtcatcca 2340
   gaagctatgc cggttctcaa acgttcccag cagccctgag agtcccattg gcgggaccat 2400
   cacttacaaa tgtgtaggct cccagtggga ggagaagaga aatgactgca tctctgcccc 2460
   aataaacagt ctgctccaga tggctaaggc tttgatcaag agcccctctc aggatgagat 2520
   gctccctaca tacctgaagg atctttctat tagcatagac aaagcggaac atgaaatcag 2580
   ctcttctcct gggagtctgg gagccattat taacatcctt gatctgctct caacagttcc 2640
   aacccaagta aattcagaaa tgatgacgca cgtgctctct acggttaatg tcatccttgg 2700
   caagcccgtc ttgaacacct ggaaggtttt acaacagcaa tggaccaatc agagttcaca 2760
   gctactacat tcagtggaaa gattttccca agcattacag tcgggagata gccctccttt 2820
   gtccttctcc caaactaatg tgcagatgag cagcatggta atcaagtcca gccacccaga 2880
   aacctatcaa cagaggtttg ttttcccata ctttgacctc tggggcaatg tggtcattga 2940
   caagagctat ctagaaaact tgcagtcgga ttcgtctatt gtcaccatgg ctttcccaac 3000
   tctccaagcc atccttgccc aggatatcca ggaaaataac tttgcagaga gcttagtgat 3060
   gacaaccact gtcagccaca atacaactat gccattcagg atttcaatga cttttaagaa 3120
   caatagccct tcaggcggcg aaacgaagtg tgtcttctgg aacttcaggc ttgccaacaa 3180
   cacagggggg tgggacagca gtgggtgcta tgtagaagaa ggtgatgggg acaatgtcac 3240
   ctgtatctgt gaccacctaa catcattctc catcctcatg tcccctgact ccccagatcc 3300
   tagttctctc ctgggaatac tcctggatat tatttcttat gttggggtgg gcttttccat 3360
   cttgagcttg gcagcctgtc tagttgtgga agctgtggtg tggaaatcgg tgaccaagaa 3420
   ccggacttct tatatgcgcc acacctgcat agtgaatatc gctgcctccc ttctggtcgc 3480
   caacacctgg ttcattgtgg tcgctgccat ccaggacaat cgctacatac tctgcaagac 3540
   agcctgtgtg gctgccacct tcttcatcca cttcttctac ctcagcgtct tcttctggat 3600
   gctgacactg ggcctcatgc tgttctatcg cctggttttc attctgcatg aaacaagcag 3660
   gtccactcag aaagccattg ccttctgtct tggctatggc tgcccacttg ccatctcggt 3720
   catcacgctg ggagccaccc agccccggga agtctatacg aggaagaatg tctgttggct 3780
   caactgggag gacaccaagg ccctgctggc tttcgccatc ccagcactga tcattgtggt 3840
   ggtgaacata accatcacta ttgtggtcat caccaagatc ctgaggcctt ccattggaga 3900
   caagccatgc aagcaggaga agagcagcct gtttcagatc agcaagagca ttggggtcct 3960
   cacaccactc ttgggcctca cttggggttt tggtctcacc actgtgttcc cagggaccaa 4020
   ccttgtgttc catatcatat ttgccatcct caatgtcttc cagggattat tcattttact 4080
   ctttggatgc ctctgggatc tgaaggtaca ggaagctttg ctgaataagt tttcattgtc 4140
   gagatggtct tcacagcact caaagtcaac atccctgggt tcatccacac ctgtgttttc 4200
   tatgagttct ccaatatcaa ggagatttaa caatttgttt ggtaaaacag gaacgtataa 4260
   tgtttccacc ccagaagcaa ccagctcatc cctggaaaac tcatccagtg cttcttcgtt 4320
   gctcaactaa gaacaggata atccaaccta cgtgacctcc cggggacagt ggctgtgctt 4380
   ttaaaaagag atgcttgcaa agcaatgggg aacgtgttct cggggcaggt ttccgggagc 4440
   agatgccaaa aagacttttt catagagaag aggctttctt ttgtaaagac agaataaaaa 4500
   taattgttat gtttctgttt gttccctccc cctccccctt gtgtgatacc acatgtgtat 4560
   agtatttaag tgaaactcaa gccctcaagg cccaacttct ctgtctatat tgtaatatag 4620
   aatttcgaag agacattttc actttttaca cattgggcac aaagataagc tttgattaaa 4680
   gtagtaagta aaaggctacc taggaaatac ttcagtgaat tctaagaagg aaggaaggaa 4740
   gaaaggaagg aaagaaggga gggaaacagg gagaaaggga aaaagaagaa aaagagaaag 4800
   atgaaaatag gaacaaataa agacaaacaa cattaagggc catattgtaa gatttccatg 4860
   ttaatgatct aatataatca ctcagtgcaa cattgagaat ttttttttaa tggctcaaaa 4920
   atggaaactg aaagcaagtc atggggaatg aatactttgg gcagtatctt cctgatgtct 4980
   tcttagctaa gaggaggaaa aaaaggctga aaaaataggg aggaaattcc ttcatcagaa 5040
   cgacttcaag tggataacaa tatttataag aaatgaatgg aaggaaatat gatcctcctg 5100
   agactaactt tgtatgttaa ggtttgaact aagtgaatgt atctgcagag gaagtattac 5160
   aaagatatgt cattagatcc aagtgctgat taaattttta tagtttatca gaaaagcctt 5220
   atattttagt ttgttccaca ttttgaaagc aaaaaatata tatttgatat acccttcaat 5280
   tgccaaattt gatatgttgc actgaagaca gaccctgtca tatatttaat ggcttcaagc 5340
   aggtacttct ctgtgcatta tagaatagat tttaataatc ttatagcatt gtatattatt 5400
   attgctgttg tcactgttat tattattgtg gatactggcc cttggtgtgt tgcatagctc 5460
   cctatgtatt ctctgtttcc atctttaagt tcccagacca atatacatta agagttttgc 5520
   atggtctaaa ttgtgtttat tccaaccacg tggaaagctc ctggaaagaa attttacatt 5580
   cggttgttct gtgctcctaa tgacacttga ccttgttgaa caaatggcag agcctttccc 5640
   aaggatttga ttgtttgtga attatctgca tgtgtgcttt tttttggtgt gtatttcatt 5700
   aaaaaatata aatatttatg aaaattgcac gcatattaga gttaaccatg tactattgat 5760
   acagcaacgc tacattgcaa ataaaagtcc gatcccaaaa ggagaatgag acaaaaaaaa 5820
   aa 5822
<210> 255
   <211> 2681
   <212> DNA
   <213> Homo sapiens
<400> 255
   aaaggtatct gttaagctag gtaggaactg cagtcggctg gttgcttctc atctggagaa 60
   agcaggcaac tgggcagtga ttgaagtgtc cagcaggggg ctggcattct ctgtctataa 120
   gtaacactgg ttcctcttca gagcctcagc tcagcggagc tgccgtttgc tggtgaagcc 180
   cgtgacgtgc aaagcatcct gcctatagga tttgaggatt tctcagtgca gtttttttct 240
   acccacttta aacctccaga ttctaaatat caggaaagac gctgtgggaa aatagcaggc 300
   caaaagttct tagtaaactg cagccaggga gactcagact agaatggagg tagaaagaac 360
   tgatgcagag tgggtttaat tctaagcctt tttgtggcta agttttgttg ttgttaactt 420
   attgaattta gagttgtatt gcactggtca tgtgaaagcc agagcagcac cagtgtcaaa 480
   atagtgacag agagttttga ataccatagt tagtatatat gtactcagag tatttttatt 540
   aaagaaggca aagagcccgg catagatctt atcttcatct tcactcggtt gcaaaatcaa 600
   tagttaagaa atagcatcta agggaacttt taggtgggaa aaaaaatcta gagatggctc 660
   taaatgactg tttccttctg aacttggagg tggaccattt catgcactgc aacatctcca 720
   gtcacagtgc ggatctcccc gtgaacgatg actggtccca cccggggatc ctctatgtca 780
   tccctgcagt ttatggggtt atcattctga taggcctcat tggcaacatc actttgatca 840
   agatcttctg tacagtcaag tccatgcgaa acgttccaaa cctgttcatt tccagtctgg 900
   ctttgggaga cctgctcctc ctaataacgt gtgctccagt ggatgccagc aggtacctgg 960
   ctgacagatg gctatttggc aggattggct gcaaactgat cccctttata cagcttacct 1020
   ctgttggggt gtctgtcttc acactcacgg cgctctcggc agacagatac aaagccattg 1080
   tccggccaat ggatatccag gcctctcatg ccctgatgaa gatctgcctc aaagccgcct 1140
   ttatctggat catctccatg ctgctggcca ttccagaggc cgtgttttct gacctccatc 1200
   ccttccatga ggaaagcacc aaccagacct tcattagctg tgccccatac ccacactcta 1260
   atgagcttca ccccaaaatc cattctatgg cttcctttct ggtcttctac gtcattccac 1320
   tgtcgatcat ctctgtttac tactacttca ttgctaaaaa tctgatccag agtgcttaca 1380
   atcttcccgt ggaagggaat atacatgtca agaagcagat tgaatcccgg aagcgacttg 1440
   ccaagacagt gctggtgttt gtgggcctgt tcgccttctg ctggctcccc aatcatgtca 1500
   tctacctgta ccgctcctac cactactctg aggtggacac ctccatgctc cactttgtca 1560
   ccagcatctg tgcccgcctc ctggccttca ccaactcctg cgtgaacccc tttgccctct 1620
   acctgctgag caagagtttc aggaaacagt tcaacactca gctgctctgt tgccagcctg 1680
   gcctgatcat ccggtctcac agcactggaa ggagtacaac ctgcatgacc tccctcaaga 1740
   gtaccaaccc ctccgtggcc acctttagcc tcatcaatgg aaacatctgt cacgagcggt 1800
   atgtctagat tgacccttga ttttgccccc tgagggacgg ttttgcttta tggctagaca 1860
   ggaacccttg catccattgt tgtgtctgtg ccctccaaag agccttcaga atgctcctga 1920
   gtggtgtagg tgggggtggg gaggcccaaa tgatggatca ccattatatt ttgaaagaag 1980
   ccatcaagtc ttaagttttt catttcaact tgtgaacgtt tcttctgatg tgaagcaaac 2040
   cttccctttt cagaaaaggg aacaagtaga aaattatttt ttaagcctca agccctgtta 2100
   aatggtcgtg gccaattatg tcatagaaac tgtatgaaca accagattta catagcagag 2160
   aaatcataca ttgaatgctt actttgtgaa agacttcacc ttgtcatttc tttaagcaga 2220
   cgctagtact ttagaaatat aacttgactc tgttttcagg aatatctgta atacacaaac 2280
   caaggaacaa cttttattta cactcctaat atgaaaagtc aatcctgtga gagagctcca 2340
   tgtatgaggg acactctcca agttgataac aatggaagcg agtttaatat aaaacaattc 2400
   cctaagcatt tatttttttt ttaaaaagat gttactgagg acctagaaga aatgctcaat 2460
   acatactttg aaagcaaaaa tacaatcaaa cacattgaca cgtatataaa gatccacgcg 2520
   tggctgtgcg tgatatctca cactctgaat tcttacttga tggaggtttt gtttgctgct 2580
   acggttttaa tcatccaggg tgccattcca ccatagaaga gcaatccttt taggaaaaaa 2640
   aaaatcatgc tattaattaa tcaaatatct ataaatgcat a 2681
<210> 256
   <211> 3047
   <212> DNA
   <213> Homo sapiens
<400> 256
   tcttgcgtca agacggccgt gctgagcgaa tgcaggcgac ttgcgagctg ggagcgattt      60
   aaaacgcttt ggattccccc ggcctgggtg gggagagcga gctgggtgcc ccctagattc     120
   cccgcccccg cacctcatga gccgaccctc ggctccatgg agcccggcaa ttatgccacc     180
   ttggatggag ccaaggatat cgaaggcttg ctgggagcgg gaggggggcg gaatctggtc     240
   gcccactccc ctctgaccag ccacccagcg gcgcctacgc tgatgcctgc tgtcaactat     300
   gcccccttgg atctgccagg ctcggcggag ccgccaaagc aatgccaccc atgccctggg     360
   gtgccccagg ggacgtcccc agctcccgtg ccttatggtt actttggagg cgggtactac     420
   tcctgccgag tgtcccggag ctcgctgaaa ccctgtgccc aggcagccac cctggccgcg     480
   taccccgcgg agactcccac ggccggggaa gagtacccca gccgccccac tgagtttgcc     540
   ttctatccgg gatatccggg aacctaccag cctatggcca gttacctgga cgtgtctgtg     600
   gtgcagactc tgggtgctcc tggagaaccg cgacatgact ccctgttgcc tgtggacagt     660
   taccagtctt gggctctcgc tggtggctgg aacagccaga tgtgttgcca gggagaacag     720
   aacccaccag gtcccttttg gaaggcagca tttgcagact ccagcgggca gcaccctcct     780
   gacgcctgcg cctttcgtcg cggccgcaag aaacgcattc cgtacagcaa ggggcagttg     840
   cgggagctgg agcgggagta tgcggctaac aagttcatca ccaaggacaa gaggcgcaag     900
   atctcggcag ccaccagcct ctcggagcgc cagattacca tctggtttca gaaccgccgg     960
   gtcaaagaga agaaggttct cgccaaggtg aagaacagcg ctacccctta agagatctcc    1020
   ttgcctgggt gggaggagcg aaagtggggg tgtcctgggg agaccaggaa cctgccaagc    1080
   ccaggctggg gccaaggact ctgctgagag gcccctagag acaacaccct tcccaggcca    1140
   ctggctgctg gactgttcct caggagcggc ctgggtaccc agtatgtgca gggagacgga    1200
   accccatgtg acagcccact ccaccagggt tcccaaagaa cctggcccag tcataatcat    1260
   tcatcctgac agtggcaata atcacgataa ccagtactag ctgccatgat cgttagcctc    1320
   atattttcta tctagagctc tgtagagcac tttagaaacc gctttcatga attgagctaa    1380
   ttatgaataa atttggaagg cgatcccttt gcagggaagc tttctctcag acccccttcc   1440
   attacacctc tcaccctggt aacagcagga agactgagga gaggggaacg ggcagattcg    1500
   ttgtgtggct gtgatgtccg tttagcattt ttctcagctg acagctgggt aggtggacaa    1560
   ttgtagaggc tgtctcttcc tccctccttg tccaccccat agggtgtacc cactggtctt    1620
   ggaagcaccc atccttaata cgatgatttt tctgtcgtgt gaaaatgaag ccagcaggct    1680
   gcccctagtc agtccttcct tccagagaaa aagagatttg agaaagtgcc tgggtaattc    1740
   accattaatt tcctccccca aactctctga gtcttccctt aatatttctg gtggttctga    1800
   ccaaagcagg tcatggtttg ttgagcattt gggatcccag tgaagtagat gtttgtagcc    1860
   ttgcatactt agcccttccc aggcacaaac ggagtggcag agtggtgcca accctgtttt    1920
   cccagtccac gtagacagat tcacagtgcg gaattctgga agctggagac agacgggctc    1980
   tttgcagagc cgggactctg agagggacat gagggcctct gcctctgtgt tcattctctg    2040
   atgtcctgta cctgggctca gtgcccggtg ggactcatct cctggccgcg cagcaaagcc    2100
   agcgggttcg tgctggtcct tcctgcacct taggctgggg gtggggggcc tgccggcgca    2160
   ttctccacga ttgagcgcac aggcctgaag tctggacaac ccgcagaacc gaagctccga    2220
   gcagcgggtc ggtggcgagt agtggggtcg gtggcgagca gttggtggtg ggccgcggcc    2280
   gccactacct cgaggacatt tccctcccgg agccagctct cctagaaacc ccgcggcggc    2340
   cgccgcagcc aagtgtttat ggcccgcggt cgggtgggat cctagccctg tctcctctcc    2400
   tgggaaggag tgagggtggg acgtgactta gacacctaca aatctattta ccaaagagga    2460
   gcccgggact gagggaaaag gccaaagagt gtgagtgcat gcggactggg ggttcagggg    2520
   aagaggacga ggaggaggaa gatgaggtcg atttcctgat ttaaaaaatc gtccaagccc    2580
   cgtggtccag cttaaggtcc tcggttacat gcgccgctca gagcaggtca ctttctgcct    2640
   tccacgtcct ccttcaagga agccccatgt gggtagcttt caatatcgca ggttcttact    2700
   cctctgcctc tataagctca aacccaccaa cgatcgggca agtaaacccc ctccctcgcc    2760
   gacttcggaa ctggcgagag ttcagcgcag atgggcctgt ggggaggggg caagatagat    2820
   gagggggagc ggcatggtgc ggggtgaccc cttggagaga ggaaaaaggc cacaagaggg    2880
   gctgccaccg ccactaacgg agatggccct ggtagagacc tttgggggtc tggaacctct    2940
   ggactcccca tgctctaact cccacactct gctatcagaa acttaaactt gaggattttc    3000
   tctgtttttc actcgcaata aattcagagc aaacaaaaaa aaaaaaa                   3047
<210> 257
   <211> 4617
   <212> DNA
   <213> Homo sapiens
<400> 257
   atctaagctt ctctgtcttc ctccctccct cccttcctct tactctcatt catttcatac      60
   acactggctc acacatctac tctctctctc tatctctctc agaatgacaa ttctaggtac     120
   aacttttggc atggtttttt ctttacttca agtcgtttct ggagaaagtg gctatgctca     180
   aaatggagac ttggaagatg cagaactgga tgactactca ttctcatgct atagccagtt     240
   ggaagtgaat ggatcgcagc actcactgac ctgtgctttt gaggacccag atgtcaacat     300
   caccaatctg gaatttgaaa tatgtggggc cctcgtggag gtaaagtgcc tgaatttcag     360
   gaaactacaa gagatatatt tcatcgagac aaagaaattc ttactgattg gaaagagcaa     420
   tatatgtgtg aaggttggag aaaagagtct aacctgcaaa aaaatagacc taaccactat     480
   agttaaacct gaggctcctt ttgacctgag tgtcgtctat cgggaaggag ccaatgactt 540
   tgtggtgaca tttaatacat cacacttgca aaagaagtat gtaaaagttt taatgcacga 600
   tgtagcttac cgccaggaaa aggatgaaaa caaatggacg catgtgaatt tatccagcac 660
   aaagctgaca ctcctgcaga gaaagctcca accggcagca atgtatgaga ttaaagttcg 720
   atccatccct gatcactatt ttaaaggctt ctggagtgaa tggagtccaa gttattactt 780
   cagaactcca gagatcaata atagctcagg ggagatggat cctatcttac taaccatcag 840
   cattttgagt tttttctctg tcgctctgtt ggtcatcttg gcctgtgtgt tatggaaaaa 900
   aaggattaag cctatcgtat ggcccagtct ccccgatcat aagaagactc tggaacatct 960
   ttgtaagaaa ccaagaaaaa atttaaatgt gagtttcaat cctgaaagtt tcctggactg 1020
   ccagattcat agggtggatg acattcaagc tagagatgaa gtggaaggtt ttctgcaaga 1080
   tacgtttcct cagcaactag aagaatctga gaagcagagg cttggagggg atgtgcagag 1140
   ccccaactgc ccatctgagg atgtagtcat cactccagaa agctttggaa gagattcatc 1200
   cctcacatgc ctggctggga atgtcagtgc atgtgacgcc cctattctct cctcttccag 1260
   gtccctagac tgcagggaga gtggcaagaa tgggcctcat gtgtaccagg acctcctgct 1320
   tagccttggg actacaaaca gcacgctgcc ccctccattt tctctccaat ctggaatcct 1380
   gacattgaac ccagttgctc agggtcagcc cattcttact tccctgggat caaatcaaga 1440
   agaagcatat gtcaccatgt ccagcttcta ccaaaaccag tgaagtgtaa gaaacccaga 1500
   ctgaacttac cgtgagcgac aaagatgatt taaaagggaa gtctagagtt cctagtctcc 1560
   ctcacagcac agagaagaca aaattagcaa aaccccacta cacagtctgc aagattctga 1620
   aacattgctt tgaccactct tcctgagttc agtggcactc aacatgagtc aagagcatcc 1680
   tgcttctacc atgtggattt ggtcacaagg tttaaggtga cccaatgatt cagctattta 1740
   aaaaaaaaag aggaaagaat gaaagagtaa aggaaatgat tgaggagtga ggaaggcagg 1800
   aagagagcat gagaggaaag aaagaaagga aaataaaaaa tgatagttgc cattattagg 1860
   atttaatata tatccagtgc tttgcaagtg ctctgcgcac cttgtctcac tccatcctga 1920
   caataatcct gggaggtgtg tgcaattact acgactactc tcttttttat agatcattaa 1980
   attcagaact aaggagttaa gtaacttgtc caagttgttc acacagtgaa gggaggggcc 2040
   aagatatgat ggctgggagt ctaattgcag ttccctgagc catgtgcctt tctcttcact 2100
   gaggactgcc ccattcttga gtgccaaacg tcactagtaa cagggtgtgc ctagataatt 2160
   tatgatccaa actgagtcag tttggaaagt gaaagggaaa cttacatata atccctccgg 2220
   gacaatgagc aaaaactagg actgtcccca gacaaatgtg aacatacata tcatcactta 2280
   aattaaaatg gctatgagaa agaaagaggg ggagaaacag tcttgcgggt gtgaagtccc 2340
   atgaccagcc atgtcaaaag aaggtaaaga agtcaagaaa aagccatgaa gcccatttgg 2400
   tttcattttt ctgaaaatag gctcaagagg gaataaatta gaaactcaca atttctcttg 2460
   tttgttacca agacagtgat tctcttgctg ctaccaccca actgcatccg tccatgatct 2520
   cagaggaaac tgtcgctgac cctggacatg ggtacgtttg acgagtgaga ggaggcatga 2580
   cccctcccat gtgtatagac actaccccaa cctaaattca tccctaaatt gtcccaagtt 2640
   ctccagcaat agaggctgcc acaaacttca gggagaaaga gttacaagta catgcaatga 2700
   gtgaactgac tgtggctaca atcttgaaga tatacggaag agacgtatta ttaatgcttg 2760
   acatatatca tcttgccttt cttggtctag actgacttct aatgactaac tcaaagtcaa 2820
   ggcaactgag taatgtcagc tcagcaaagt gcagcaaacc catctcccac aggcctccaa 2880
   accctggctg ttcacagaac cacaaagggc agatgctgca cagaaaacta gagaaggggt 2940
   cataggttca tggttttgtt tgagatttgt tgctactgtt tttctgtttt gaattttctt 3000
   ctttgttctg tttttacttt atttaggggg actaggtgtt tctgatattt tagttttctt 3060
   gtttgttttg ttttgtgttg tctgtgaatg gggttttaac tgtggatgaa tggaccttat 3120
   ctgttggctt aaaggactgg taagatcaga ccatcttatt cttcaggtga atgttttact 3180
   ttccaaagtg ctctcctctg caccagcagt aataaataca atgccataat cccttaggtt 3240
   tgcctagtgc ttttgcaatt ttcaaagcac ttccataagc attccttcca cctccttgat 3300
   aggcatttat ggaaagcctg ctacatgtca atcatactgt taggcacagg ggacctaaag 3360
   acacataaaa ggatggcatt ctgcctcata aattgcaaaa cctaatgaaa gtgactgctt 3420
   ggtaaacaaa ttattattat attataaaat gctataaaag agccatattg aaagtgccct 3480
   gttggagaca gggcaaatgc cacaaaaatg atgtaaattt acatggagga aaagtagaat 3540
   ctgcctggtt tgtaggcagc agaagacatt tttcatcagt gggcaggtgt tctttacctt 3600
   ttgtagaaat gggagtcaag tctcaaatag gaggctccac aaaatctcat gccaggtctc 3660
   tgatacctta ttcacagaag ttctttgaag tatttattgt tattttcttt gacttatggg 3720
   aaaactggga cacaggaaga caggtaaatt acccaacctc acacgttaag tcagaactgg 3780
   gagccataat tttgtatccc tggtataaat agacaatctc ttgaagaaat gaagagatga 3840
   ccatagaaaa acatcgagat atctccagct ctaaaatcct ttgtttcaat gttgtttggc 3900
   atatgttatc tttggaattt agtgtctgag cctctgtctg ttactgtagt atttaaaatg 3960
   catgtattat aatcatataa tcataactgc tgttaattct tgattatata cctagggaca 4020
   atgtgtaatg taagattact aattggttct gcccaatctc ctttcagatt ttattaggaa 4080
   aaaaaaataa acctcctgat cggagacaat gtattaatca gaagtgtaaa ctgccagttc 4140
   tatatagcat gaaatgaaaa gacagctaat ttggtccaac aaacatgact gggtctaggg 4200
   cacccaggct gattcagctg atttcctacc agcctttgcc tcttccttca atgtggtttc 4260
   catgggaatt tgcttcagaa aagccaagta tgggctgttc agaggtgcac acctgcattt 4320
   tcttagctct tctagagggg ctaagagact tggtacgggc caggaagaat atgtggcaga 4380
   gctcctggaa atgatgcaga ttaggtggca tttttgtcag ctctgtggtt tattgttggg 4440
   actattcttt aaaatatcca ttgttcacta cagtgaagat ctctgattta accgtgtact 4500
   atccacatgc attacaaaca tttcgcagag ctgcttagta tataagcgta caatgtatgt 4560
   aataaccatc tcatatttaa ttaaatggta tagaagaaca aaaaaaaaaa aaaaaaa 4617
<210> 258
   <211> 3407
   <212> DNA
   <213> Homo sapiens
<400> 258
   actctttcct gagccctgtg cttcggatgg cggcgggagg ttgatggcga gtggtgctga      60
   agggacagct ccagcagtgg ctgatttggg ggagaaacaa aatctgcaga tggaatccga     120
   gcagggcgac ttcaccttca agtggtgagc tctcctgacc tgcggccagt ctccactcca     180
   ttcacggcca gccgatctgc ccgctcccgg aggggtcggg cagtgccggc tggacccgcc     240
   ccgagctcca tggtttgccc aaccctgcgc gatggtgact ctgggcgcgg aggttggcga     300
   ctggcaaatc cgcagatcac agaatgaagg cggggagcgc ggccggcggc cggcgggggc     360
   tttctccccc accccagcgc ccagggaagc ggctcaacca cctgaatccg gaaaacgcca     420
   acaagtagtt tctcgtcgga gaagggcggc tcacctgggc gccaagactc agtcccgctg     480
   cccagagaac ctcgtccact cggaaaccaa agcagaacca cttttctctc ggtctcgtta     540
   agtcatgtct gagtcacaga gatgggcaag atcgagaaca acgagagggt gatcctcaat     600
   gtcgggggca cccggcacga aacctaccgc agcaccctca agaccctgcc tggaacacgc     660
   ctggcccttc ttgcctcctc cgagccccca ggcgactgct tgaccacggc gggcgacaag     720
   ctgcagccgt cgccgcctcc actgtcgccg ccgccgagag cgcccccgct gtcccccggg     780
   ccaggcggct gcttcgaggg cggcgcgggc aactgcagtt cccgcggcgg cagggccagc     840
   gaccatcccg gtggcggccg cgagttcttc ttcgaccggc acccgggcgt cttcgcctat     900
   gtgctcaatt actaccgcac cggcaagctg cactgccccg cagacgtgtg cgggccgctc     960
   ttcgaggagg agctggcctt ctggggcatc gacgagaccg acgtggagcc ctgctgctgg    1020
   atgacctacc ggcagcaccg cgacgccgag gaggcgctgg acatcttcga gacccccgac    1080
   ctcattggcg gcgaccccgg cgacgacgag gacctggcgg ccaagaggct gggcatcgag    1140
   gacgcggcgg ggctcggggg ccccgacggc aaatctggcc gctggaggag gctgcagccc    1200
   cgcatgtggg ccctcttcga agacccctac tcgtccagag ccgccaggtt tattgctttt    1260
   gcttctttat tcttcatcct ggtttcaatt acaacttttt gcctggaaac acatgaagct    1320
   ttcaatattg ttaaaaacaa gacagaacca gtcatcaatg gcacaagtgt tgttctacag    1380
   tatgaaattg aaacggatcc tgccttgacg tatgtagaag gagtgtgtgt ggtgtggttt    1440
   acttttgaat ttttagtccg tattgttttt tcacccaata aacttgaatt catcaaaaat    1500
   ctcttgaata tcattgactt tgtggccatc ctacctttct acttagaggt gggactcagt    1560
   gggctgtcat ccaaagctgc taaagatgtg cttggcttcc tcagggtggt aaggtttgtg    1620
   aggatcctga gaattttcaa gctcacccgc cattttgtag gtctgagggt gcttggacat    1680
   actcttcgag ctagtactaa tgaatttttg ctgctgataa ttttcctggc tctaggagtt    1740
   ttgatatttg ctaccatgat ctactatgcc gagagagtgg gagctcaacc taacgaccct    1800
   tcagctagtg agcacacaca gttcaaaaac attcccattg ggttctggtg ggctgtagtg    1860
   accatgacta ccctgggtta tggggatatg tacccccaaa catggtcagg catgctggtg   1920
   ggagccctgt gtgctctggc tggagtgctg acaatagcca tgccagtgcc tgtcattgtc    1980
   aataattttg gaatgtacta ctccttggca atggcaaagc agaaacttcc aaggaaaaga     2040
   aagaagcaca tccctcctgc tcctcaggca agctcaccta ctttttgcaa gacagaatta    2100
   aatatggcct gcaatagtac acagagtgac acatgtctgg gcaaagacaa tcgacttctg    2160
   gaacataaca gatcagataa ctgcaaagag gttgtcatta ctggttacac gcaagccgag    2220
   gccagatctc ttacttaatg acttggggaa ggcacaaaac atgagagaaa gtgttgtaca    2280
   gaatttatca tggattattg actgctgaga aagggacagt ggaatttagc cataccaagg    2340
   actatactgg aaacagactt ctgctgctga atgtgccctg atgtgaccag gttgcacttg    2400
   gaagagatcc tcgcgtcttc atgaggcact taaagcttat aaaagaactg cggctggaac    2460
   tcatctggtg ctccccatga gagtgctctg cttgtagact ggccagtgtc catgaaacaa    2520
   ctgtaaatac caacatgtgt gcatgggtca acagtcttgg ccatttctca tcaaaagaag    2580
   ccaaattcat gatcaacatc tctgaagttt caagtaaggc ccacacttct ttgaattact    2640
   cttcatgggc ccacattagg ttgtgctgtg aattacttaa ggcagtgata ctgatgtagt    2700
   atagttttgt cttaatttcc cttatttcta cttctttggt tgaatctatg aacttgattg    2760
   tataattttc ttataaatta ctgatgtaat cagcttgtca attatgttgt gaaattgtta     2820
   gtattcattt atcaaaaatg acctatgttt agtcacatat ttgtttagtt ctgggaaatt    2880
   gttatagctt aaatggaact caccaacatt attcatagtt taagtctttt atcattatta    2940
   cctcaattat aaatattaca aaaacataat tctggcaatg agagtatttt tttattcaat    3000
   gatcaaggag caatgtcagt atatagtaga atatcaatta aattatatcc taaaatgtat    3060
   attttgcata aaagagatat tctttaatca attacttttt tgtgagtttt gtggcaaatg    3120
   aagcttgtac gtgtctttaa aactgttgta gatgaaactg tataagattt ttacatcttg    3180
   cttaatcaat attttcagag tctattagtt cccctgggat tctgaatata acatatagcc    3240
   tattataaat ccctgtatcg tggacctttt gtgaacattt caaggcgcat gcacaacctt    3300
   gatgataacc agtggaaatg taactaactg aaatgaagaa taaaaggcaa atgagctggg    3360
   gataaacttg aatgttatct gattaaatta ctcaaattat aaaaaaa                  3407
<210> 259
   <211> 2095
   <212> DNA
   <213> Homo sapiens
<400> 259
   gcggccgccg gagcccgagc tgacgccgcc ttggcacccc tcctggagtt agaaactaag      60
   gccggggccc gcggcgctcg gcgcgcaggc cgcccggctt cctgcgtcca tttccgcgtg     120
   ctttcaaaga agacagagag aggcactggg ttgggcttca tttttttcct ccccatcccc     180
   agtttctttc tctttttaaa aataataatt atcccaataa ttaaagccaa ttcccccctc      240
   ccctccccca gtccctcccc ccaactcccc cctcccccgc ccgccggggc aggggagcgc     300
   cacgaattga ccaagtgaag ctacaacttt gcgacataaa ttttggggtc tcgaaccatg     360
   tcgctgacca acacaaagac ggggttttcg gtcaaggaca tcttagacct gccggacacc     420
   aacgatgagg agggctctgt ggccgaaggt ccggaggaag agaacgaggg gcccgagcca     480
   gccaagaggg ccgggccgct ggggcagggc gccctggacg cggtgcagag cctgcccctg     540
   aagaacccct tctacgacag cagcgacaac ccgtacacgc gctggctggc cagcaccgag     600
   ggccttcagt actccctgca cggtctggct gccggggcgc cccctcagga ctcaagctcc     660
   aagtccccgg agccctcggc cgacgagtca ccggacaatg acaaggagac cccgggcggc     720
   gggggggacg ccggcaagaa gcgaaagcgg cgagtgcttt tctccaaggc gcagacctac     780
   gagctggagc ggcgctttcg gcagcagcgg tacctgtcgg cgcccgagcg cgaacacctg     840
   gccagcctca tccgcctcac gcccacgcag gtcaagatct ggttccagaa ccaccgctac     900
   aagatgaagc gcgcccgggc cgagaaaggt atggaggtga cgcccctgcc ctcgccgcgc     960
   cgggtggccg tgcccgtctt ggtcagggac ggcaaaccat gtcacgcgct caaagcccag    1020
   gacctggcag ccgccacctt ccaggcgggc attccctttt ctgcctacag cgcgcagtcg    1080
   ctgcagcaca tgcagtacaa cgcccagtac agctcggcca gcacccccca gtacccgaca   1140
   gcacaccccc tggtccaggc ccagcagtgg acttggtgag cgccgcccca acgagactcg    1200
   cggccccagg cccaggcccc accccggcgg cggtggcggc gaggaggcct cggtccttat    1260
   ggtggttatt attattatta taattattat tatggagtcg agttgactct cggctccact    1320
   agggaggcgc cgggaggttg cctgcgtctc cttggagtgg cagattccac ccacccagct    1380
   ctgcccatgc ctctccttct gaaccttggg agagggctga actctacgcc gtgtttacag    1440
   aatgtttgcg cagcttcgct tctttgcctc tccccggggg gaccaaaccg tcccagcgtt    1500
   aatgtcgtca cttgaaaacg agaaaaagac cgacccccca cccctgcttt cgtgcatttt   1560
   gtaaaatatg tttgtgtgag tagcgatatt gtcagccgtc ttctaaagca agtggagaac    1620
   actttaaaaa tacagagaat ttcttccttt ttttaaaaaa aaataagaaa atgctaaata    1680
   tttatggcca tgtaaacgtt ctgacaactg gtggcagatt tcgcttttcg ttgtaaatat    1740
   cggtggtgat tgttgccaaa atgaccttca ggaccggcct gtttcccgtc tgggtccaac    1800
   tcctttcttt gtggcttgtt tgggtttgtt ttttgttttg tttttgtttt tgcgttttcc    1860
   cctgctttct tcctttctct ttttatttta ttgtgcaaac atttctcaaa tatggaaaag    1920
   aaaaccctgt aggcagggag ccctctgccc tgtcctccgg gccttcagcc ccgaacttgg    1980
   agctcagcta ttcggcgcgg ttccccaaca gcgccgggcg cagaaagctt tcgatttttt    2040
   aaataagaat tttaataaaa atcctgtgtt taaaaaagaa aaaaaaaaaa aaaaa         2095
<210> 260
   <211> 2926
   <212> DNA
   <213> Homo sapiens
<400> 260
   ccgggttctc tcccggcgtg ccccgcgccg ggtttgttgg ggggtactcg gcagtgcagc      60
   catgactata ctccccaaaa agaagccgcc gcctcccgac gccgaccccg ccaacgagcc     120
   gccgccgccc gggccgatgc ccccggcgcc gcggcgcggc ggaggtgtgg gcgtgggcgg     180
   cggcggcacg ggcgtgggcg gcggcgatcg cgaccgtgac tccggcgtcg tgggggcccg     240
   tccgcgagct tcgccaccgc ctcaaggccc gctaccagga ccgccgggcg ctcttcatcg     300
   ctgggcgctg gccgtgccgc ctggtgcagt ggcgggtccc cggccacaac aggcttctcc     360
   acctccttgc gggggcccag gtggtcccgg cggcggtccc ggcgacgcgc tgggcgcagc     420
   ggcggcgggt gtgggtgccg cgggcgtggt ggtgggtgtg ggtggtgccg taggcgtggg     480
   cggctgctgc tccgggcctg ggcacagcaa gcggcgacgt caagctcccg gggttggcgc     540
   ggttggcggg ggcagtcccg agcgtgagga ggtcggcgca ggctacaaca gtgaggacga     600
   gtatgaggcg gctgcagcac gcatcgaggc tatggaccct gccactgtcg agcagcagga     660
   gcattggttt gaaaaggccc tacgagacaa gaagggcttc atcatcaagc agatgaagga     720
   ggatggcgcc tgtctcttcc gggctgtagc tgaccaggtg tatggagacc aggacatgca     780
   tgaggttgtg cgaaagcatt gcatggacta tctgatgaag aatgccgact acttctccaa     840
   ctatgtcaca gaggacttta ccacctacat taacaggaag cggaaaaaca attgccatgg     900
   caaccacatt gagatgcagg ccatggcaga gatgtacaac cgtcctgtgg aggtgtacca     960
   gtacagcaca gaacccatca acacattcca tgggatacat caaaacgagg acgaacccat    1020
   tcgtgttagc taccatcgga atatccacta taattcagtg gtgaatccta acaaggccac    1080
   cattggtgtg gggctgggcc  tgccatcatt caaaccaggg tttgcagagc agtctctgat    1140
   gaagaatgcc ataaaaacat cggaggagtc atggattgaa cagcagatgc tagaagacaa    1200
   gaaacgggcc  acagactggg aggccacaaa tgaagccatc gaggagcagg tggctcggga    1260
   atcctacctg cagtggttgc gggatcagga gaaacaggct cgccaggtcc gaggccccag    1320
   ccagccccgg aaagccagcg ccacatgcag ttcggccaca gcagcagcct ccagtggcct     1380
   ggaggagtgg actagccggt ccccgcggca gcggagttca gcctcgtcac ctgagcaccc     1440
   tgagctgcat gctgaattgg gcatgaagcc cccttcccca ggcactgttt tagctcttgc     1500
   caaacctcct tcgccctgtg cgccaggtac aagcagtcag ttctcggcag gggccgaccg     1560
   ggcaacttcc ccccttgtgt ccctctaccc tgctttggag tgccgggccc tcattcagca     1620
   gatgtccccc tctgcctttg gtctgaatga ctgggatgat gatgagatcc tagcttcggt     1680
   gctggcagtg tcccaacagg aatacctaga cagtatgaag aaaaacaaag tgcacagaga     1740
   cccgccccca gacaagagtt gatggagacc cagggattgg acaccatctc ccaaccccag     1800
   tactcctgct ctccggtgcc acctcacctt ctttggcttc ttccctcttg cctccttctg     1860
   ttctttctgc tctcccctct tttccctcct cctcacttcc ctctggctag cccacccctg     1920
   cactctctct cattgccgct gccactatca cctgtctctc tgccagctga tgtgccctgt     1980
   tgccccccac cccatcccgc acagaaccat ccctgcattc cacaggggac tcgggcaagg     2040
   gtgccgaaga tagacaagag gcacacagag acagaccaac tggcagccag gcagccccag     2100
   aggagagaga cattcagaca gaggaaagtc tccctgcccc tcattccttc caagatgaga     2160
   aaaacttgcc gccacccccc gacactgatg ccagggaggt gggaggaaga agtgggaaat     2220
   ttcccttccc agtaccccca agaacgtctg agccttcaat gttgaatttt ttctttatta     2280
   aaattacttt tatcttataa aatcaactaa tcaaaaatga tatagacgac agcactggct     2340
   ctgtgaaggt ggcatctttc tgggcaggca ggccatgggg catggaggag ggtgcaaaga     2400
   tatgggttgc tgtcttctgg cctccagctg catggaggcc ggcccagggt ctagggtgtg     2460
   cactgggcaa gggcagggcg gcaggtgtca ggccggcttg gacaatgaaa ccctgacctt     2520
   gctgcattcc ttttgcttcc accaccacta gcttctttgg aatcttgggg tgggggtcat     2580
   ctttggggat tatggctgcc acccgggatt tgagtgtagg gagtgtggga gcagccttgg     2640
   cagatggggc acccgtgccc tgcaggtgtt gacaagatcc gccatctgta atgtccttgg     2700
   cacaataaaa ccaaatgtca gtttccctga gcgactctgt tctgtgtggg gcaggggttg     2760
   ggcgggcctc tgggcagagg atgcaatggc acggaccttg gcttgacctc agaggtgtga     2820
   atgctctcca gcagggtctg tctgggggcc tggagtttgt atttgatttg ctgcttatta     2880
   aacctccttc tggacctatt gccactggaa aaaaaaaaaa aaaaaa                    2926
<210> 261
   <211> 3675
   <212> DNA
   <213> Homo sapiens
<400> 261
   ctgcagccga gggagaccag gaagatctgc atggtgggaa ggacctgatg atacagaggt      60
   gagaaataag aaaggctgct gactttacca tctgaggcca cacatctgct gaaatggaga     120
   taattaacat cactagaaac agcaagatga caatataatg tctaagtagt gacatgtttt     180
   tgcacatttc cagccccttt aaatatccac acacacagga agcacaaaag gaagcacaga     240
   gatccctggg agaaatgccc ggccgccatc ttgggtcatc gatgagcctc gccctgtgcc     300
   tggtcccgct tgtgagggaa ggacattaga aaatgaattg atgtgttcct taaaggatgg     360
   gcaggaaaac agatcctgtt gtggatattt atttgaacgg gattacagat ttgaaatgaa     420
   gtcacaaagt gagcattacc aatgagagga aaacagacga gaaaatcttg atggcttcac     480
   aagacatgca acaaacaaaa tggaatactg tgatgacatg aggcagccaa gctggggagg     540
   agataaccac ggggcagagg gtcaggattc tggccctgct gcctaaactg tgcgttcata     600
   accaaatcat ttcatatttc taaccctcaa aacaaagctg ttgtaatatc tgatctctac     660
   ggttccttct gggcccaaca ttctccatat atccagccac actcattttt aatatttagt     720
   tcccagatct gtactgtgac ctttctacac tgtagaataa cattactcat tttgttcaaa     780
   gacccttcgt gttgctgcct aatatgtagc tgactgtttt tcctaaggag tgttctggcc     840
   caggggatct gtgaacaggc tgggaagcat ctcaagatct ttccagggtt atacttacta     900
   gcacacagca tgatcattac ggagtgaatt atctaatcaa catcatcctc agtgtctttg     960
   cccatactga aattcatttc ccacttttgt gcccattctc aagacctcaa aatgtcattc    1020
   cattaatatc acaggattaa cttttttttt taacctggaa gaattcaatg ttacatgcag    1080
   ctatgggaat ttaattacat attttgtttt ccagtgcaaa gatgactaag tcctttatcc    1140
   ctcccctttg tttgattttt tttccagtat aaagttaaaa tgcttagcct tgtactgagg    1200
   ctgtatacag ccacagcctc tccccatccc tccagcctta tctgtcatca ccatcaaccc    1260
   ctcccatgca cctaaacaaa atctaacttg taattccttg aacatgtcag gcatacatta    1320
   ttccttctgc ctgagaagct cttccttgtc tcttaaatct agaatgatgt aaagttttga    1380
   ataagttgac tatcttactt catgcaaaga agggacacat atgagattca tcatcacatg    1440
   agacagcaaa tactaaaagt gtaatttgat tataagagtt tagataaata tatgaaatgc    1500
   aagagccaca gagggaatgt ttatggggca cgtttgtaag cctgggatgt gaagcaaagg    1560
   cagggaacct catagtatct tatataatat acttcatttc tctatctcta tcacaatatc    1620
   caacaagctt ttcacagaat tcatgcagtg caaatcccca aaggtaacct ttatccattt    1680
   catggtgagt gcgctttaga attttggcaa atcatactgg tcacttatct caactttgag    1740
   atgtgtttgt ccttgtagtt aattgaaaga aatagggcac tcttgtgagc cactttaggg    1800
   ttcactcctg gcaataaaga atttacaaag agctactcag gaccagttgt taagagctct    1860
   gtgtgtgtgt gtgtgtgtgt gagtgtacat gccaaagtgt gcctctctct ctttgaccca    1920
   ttatttcaga cttaaaaaca agcatgtttt caaatggcac tatgagctgc caatgatgta    1980
   tcaccaccat atctcattat tctccagtaa atgtgataat aatgtcatct gttaacataa    2040
   aaaaagtttg acttcacaaa agcagctgga aatggacaac cacaatatgc ataaatctaa    2100
   ctcctaccat cagctacaca ctgcttgaca tatattgtta gaagcacctc gcatttgtgg    2160
   gttctcttaa gcaaaatact tgcattaggt ctcagctggg gctgtgcatc aggcggtttg    2220
   agaaatattc aattctcagc agaagccaga atttgaattc cctcatcttt taggaatcat    2280
   ttaccaggtt tggagaggat tcagacagct caggtgcttt cactaatgtc tctgaacttc    2340
   tgtccctctt tgtgttcatg gatagtccaa taaataatgt tatctttgaa ctgatgctca    2400
   taggagagaa tataagaact ctgagtgata tcaacattag ggattcaaag aaatattaga    2460
   tttaagctca cactggtcaa aaggaaccaa gatacaaaga actctgagct gtcatcgtcc    2520
   ccatctctgt gagccacaac caacagcagg acccaacgca tgtctgagat ccttaaatca    2580
   aggaaaccag tgtcatgagt tgaattctcc tattatggat gctagcttct ggccatctct    2640
   ggctctcctc ttgacacata ttagcttcta gcctttgctt ccacgacttt tatcttttct    2700
   ccaacacatc gcttaccaat cctctctctg ctctgttgct ttggacttcc ccacaagaat    2760
   ttcaacgact ctcaagtctt ttcttccatc cccaccacta acctgaatgc ctagaccctt    2820
   atttttatta atttccaata gatgctgcct atgggctata ttgctttaga tgaacattag    2880
   atatttaaag ctcaagaggt tcaaaatcca actcattatc ttctctttct ttcacctccc    2940
   tgctcctctc cctatattac tgattgcact gaacagcatg gtccccaatg tagccatgca    3000
   aatgagaaac ccagtggctc cttgtggtac atgcatgcaa gactgctgaa gccagaagga    3060
   tgactgatta cgcctcatgg gtggagggga ccactcctgg gccttcgtga ttgtcaggag    3120
   caagacctga gatgctccct gccttcagtg tcctctgcat ctcccctttc taatgaagat    3180
   ccatagaatt tgctacattt gagaattcca attaggaact cacatgtttt atctgcccta    3240
   tcaatttttt aaacttgctg aaaattaagt tttttcaaaa tctgtccttg taaattactt    3300
   tttcttacag tgtcttggca tactatatca actttgattc tttgttacaa cttttcttac    3360
   tcttttatca ccaaagtggc ttttattctc tttattatta ttattttctt ttactactat    3420
   attacgttgt tattattttg ttctctatag tatcaattta tttgatttag tttcaattta    3480
   tttttattgc tgacttttaa aataagtgat tcggggggtg ggagaacagg ggagggagag    3540
   cattaggaca aatacctaat gcatgtggga cttaaaacct agatgatggg ttgataggtg    3600
   cagcaaacca ctatggcaca cgtatacctg tgtaacaaac ctacacattc tgcacatgta    3660
   tcccagaacg taaag                                                      3675
<210> 262
   <211> 2692
   <212> DNA
   <213> Homo sapiens
<400> 262
   tttaaagctg ggaggttctg ccaccaagca cggccttccc actgggaaca caaacttgct      60
   ggcgggaaga gcccggaaag aaacctgtgg atctcccttc gagatcatcc aaagagaaga     120
   aaggtgacct cacattcgtg ccccttagca gcactctgca gaaatgcctc ctcagctgca     180
   aaacggcctg aacctctcgg ccaaagttgt ccagggaagc ctggacagcc taccccaggc     240
   agtgagggag tttctcgaga ataacgctga gctgtgtcag cctgatcaca tccacatctg     300
   tgacggctct gaggaggaga atgggcggct tctgggccag atggaggaag agggcatcct     360
   caggcggctg aagaagtatg acaactgctg gttggctctc actgacccca gggatgtggc     420
   caggatcgaa agcaagacgg ttatcgtcac ccaagagcaa agagacacag tgcccatccc     480
   caaaacaggc ctcagccagc tcggtcgctg gatgtcagag gaggattttg agaaagcgtt     540
   caatgccagg ttcccagggt gcatgaaagg tcgcaccatg tacgtcatcc cattcagcat     600
   ggggccgctg ggctcgcctc tgtcaaagat cggcatcgag ctgacggatt caccctacgt     660
   ggtggccagc atgcggatca tgacgcggat gggcacgccc gtcctggaag cagtgggcga     720
   tggggagttt gtcaaatgcc tccattctgt ggggtgccct ctgcctttac aaaagccttt     780
   ggtcaacaac tggccctgca acccggagct gacgctcatc gcccacctgc ctgaccgcag     840
   agagatcatc tcctttggca gtgggtacgg cgggaactcg ctgctcggga agaagtgctt     900
   tgctctcagg atggccagcc ggctggccaa ggaggaaggg tggctggcag agcacatgct     960
   gattctgggt ataaccaacc ctgagggtga gaagaagtac ctggcggccg catttcccag    1020
   cgcctgcggg aagaccaacc tggccatgat gaaccccagc ctccccgggt ggaaggttga    1080
   gtgcgtcggg gatgacattg cctggatgaa gtttgacgca caaggtcatt taagggccat    1140
   caacccagaa aatggctttt tcggtgtcgc tcctgggact tcagtgaaga ccaaccccaa    1200
   tgccatcaag accatccaga agaacacaat ctttaccaat gtggccgaga ccagcgacgg    1260
   gggcgtttac tgggaaggca ttgatgagcc gctagcttca ggtgtcacca tcacgtcctg    1320
   gaagaataag gagtggagct cagaggatgg ggaaccttgt gcccacccca actcgaggtt   1380
   ctgcacccct gccagccagt gccccatcat tgatgctgcc tgggagtctc cggaaggtgt    1440
   tcccattgaa ggcattatct ttggaggccg tagacctgct ggtgtccctc tagtctatga    1500
   agctctcagc tggcaacatg gagtctttgt gggggcggcc atgagatcag aggccacagc    1560
   ggctgcagaa cataaaggca aaatcatcat gcatgacccc tttgccatgc ggcccttctt    1620
   tggctacaac ttcggcaaat acctggccca ctggcttagc atggcccagc acccagcagc    1680
   caaactgccc aagatcttcc atgtcaactg gttccggaag gacaaggaag gcaaattcct    1740
   ctggccaggc tttggagaga actccagggt gctggagtgg atgttcaacc ggatcgatgg    1800
   aaaagccagc accaagctca cgcccatagg ctacatcccc aaggaggatg ccctgaacct    1860
   gaaaggcctg gggcacatca acatgatgga gcttttcagc atctccaagg aattctggga    1920
   gaaggaggtg gaagacatcg agaagtatct ggaggatcaa gtcaatgccg acctcccctg    1980
   tgaaatcgag agagagatcc ttgccttgaa gcaaagaata agccagatgt aatcagggcc    2040
   tgagtgcttt acctttaaaa tcattccctt tcccatccat aaggtgcagt aggagcaaga    2100
   gagggcaagt gttcccaaat tgacgccacc ataataatca tcaccacacc gtgagcagat    2160
   ctgaaaggca cactttgatt tttttaagga taagaaccac agaacactgg gtagtagcta    2220
   atgaaattga gaagggaaat cttagcatgc ctccaaaaat tcacatccaa tgcatagttt    2280
   gttcaaattt aaggttactc aggcattgat cttttcagtg ttttttcact ttagctatgt    2340
   ggattagcta gaatgcacac caaaaaaata cttgagctgt atatatatat gtgtgtgtgt    2400
   gtgtgtgtgt gtgtgtgtgt gtgtgcatgt atgtgcacat gtgtctgtgt ggtatatttg    2460
   tgtatgtgta tttgtatgta ctgttattga aatatatttt aatacctttg gaaaaatctt    2520
   gggcaagatg acctactagt tttccttgaa aaaaagttgc tttgttatta atattgtgct    2580
   taaattattt ttatacacca ttgttcctta cctttacata attgcaatat ttccccctta    2640
   ctacttcttg gaaaaaaatt acaaaatgaa gttttataga aaagaaaaaa aa            2692
<210> 263
   <211> 6602
   <212> DNA
   <213> Homo sapiens
<400> 263
   acacgcgctt caacttcggt tggtgtgtgt cgaagaaacc tgactgcgcc ctgaggagaa 60
   cagcggagaa ggtccaccga gcctggcgaa aggtccgctg agcgggctgt cgtccggagc 120
   cactccgggc tgcggagcac ccagtggaga ccgcgcctgg ctcaggtgtg ggaccccatc 180
   cttcctgtct tcgcagagga gtcctcgcgt ggtgagtatg cgaaataagc gggttttgaa 240
   aacaaaaaaa agaaggagtg gaagaggggg ccaggatcca ggcctccatc cccacagaag 300
   tgaagctaca gctgggaggt ctcctcccac cccaaccgtc accctgggtc ccgactgccc 360
   acctcctcct cctccccctc cccccaacaa caacaacaac aacaactcca agcacaccgg 420
   ccataagagt gcgtgtgtcc ccaacatgac cgaacgaaga agggacgagc tctctgaaga 480
   gatcaacaac ttaagagaga aggtcatgaa gcagtcggag gagaacaaca acctgcagag 540
   ccaggtgcag aagctcacag aggagaacac cacccttcga gagcaagtgg aacccacccc 600
   tgaggatgag gatgatgaca tcgagctccg cggtgctgca gcagctgctg ccccaccccc 660
   tccaatagag gaagagtgcc cagaagacct cccagagaag ttcgatggca acccagacat 720
   gctggctcct ttcatggccc agtgccagat cttcatggaa aagagcacca gggatttctc 780
   agttgatcgt gtccgtgtct gcttcgtgac aagcatgatg accggccgtg ctgcccgttg 840
   ggcctcagca aagctggagc gctcccacta cctgatgcac aactacccag ctttcatgat 900
   ggaaatgaag catgtctttg aagaccctca gaggcgagag gttgccaaac gcaagatcag 960
   acgcctgcgc caaggcatgg ggtctgtcat cgactactcc aatgctttcc agatgattgc 1020
   ccaggacctg gattggaacg agcctgcgct gattgaccag taccacgagg gcctcagcga 1080
   ccacattcag gaggagctct cccacctcga ggtcgccaag tcgctgtctg ctctgattgg 1140
   gcagtgcatt cacattgaga gaaggctggc cagggctgct gcagctcgca agccacgctc 1200
   gccaccccgg gcgctggtgt tgcctcacat tgcaagccac caccaggtag atccaaccga 1260
   gccggtggga ggtgcccgca tgcgcctgac gcaggaagaa aaagaaagac gcagaaagct 1320
   gaacctgtgc ctctactgtg gaacaggagg tcactacgct gacaattgtc ctgccaaggc 1380
   ctcaaagtct tcgccggcgg gaaactcccc ggccccgctg tagagggacc ttcagcgacc 1440
   gggccagaaa taataaggtc cccacaagat gatgcctcat ctccacactt gcaagtgatg 1500
   ctccagattc atcttccggg cagacacacc ctgttcgtcc gagccatgat cgattctggt 1560
   gcttctggca acttcattga tcacgaatat gttgctcaaa atggaattcc tctaagaatc 1620
   aaggactggc caatacttgt ggaagcaatt gatgggcgcc ccatagcatc gggcccagtt 1680
   gtccacgaaa ctcacgacct gatagttgac ctgggagatc accgagaggt gctgtcattt 1740
   gatgtgactc agtctccatt cttccctgtc gtcctagggg ttcgctggct gagcacacat 1800
   gatcccaata tcacatggag cactcgatct atcgtctttg attctgaata ctgccgctac 1860
   cactgccgga tgtattctcc aataccacca tcgctcccac caccagcacc acaaccgcca 1920
   ctctattatc cagtagatgg atacagagtt taccaaccag tgaggtatta ctatgtccag 1980
   aatgtgtaca ctccagtaga tgagcacgtc tacccagatc accgcctggt tgaccctcac 2040
   atagaaatga tacctggagc acacagtatt cccagtggac atgtgtattc actgtccgaa 2100
   cctgaaatgg cagctcttcg agattttgtg gcaagaaatg taaaagatgg gctaattact 2160
   ccaacgattg cacctaatgg agcccaagtt ctccaggtga agagggggtg gaaactgcaa 2220
   gtttcttatg attgccgagc tccaaacaat tttactatcc agaatcagta tcctcgccta 2280
   tctattccaa atttagaaga ccaagcacac ctggcaacgt acactgaatt cgtacctcaa 2340
   atacctggat accaaacata ccccacatat gccgcgtacc cgacctaccc agtaggattc 2400
   gcctggtacc cagtgggacg agacggacaa ggaagatcac tatatgtacc tgtgatgatc 2460
   acttggaatc cacactggta ccgccagcct ccggtaccac agtacccgcc gccacagccg 2520
   ccgcctccac caccaccacc gccgccgcct ccatcttaca gtaccctgta aatacctgtc 2580
   atgtccttca ggatctctgc cctcaaaatt tattcctgtt cagcttctca atcagtgact 2640
   gtgtgctaaa ttttaggcta ctgtatcttc aggccacctg aggcacatcc tctctgaaac 2700
   ggctatggaa ggttagggcc actctggact ggcacacatc ctaaagcacc aaaagacctt 2760
   caacattttc tgagagcaac agagtatttg ccaataaatg atctctcatt tttccacctt 2820
   gactgccaat ctaactaaaa taattaataa gtttactttc cagccagtcc tggaagtctg 2880
   ggttttacct gccaaaacct ccatcaccat ctaaattata ggctgccaaa tttgctgttt 2940
   aacatttaca gagaagctga tacaaacgca ggaaatgctg atttctttat ggagggggag 3000
   acgaggagga ggaggacatg acttttcttg cggtttcggt accctctttt taaatcactg 3060
   gaggactgag gccttattaa ggaagccaaa attatcggtg cagtgtggaa aggcttccgt 3120
   gatcctctcg ctgcaccctt agaaacttca ccgtcttcaa actccatttc catggttctg 3180
   ttaattctca aggagcagca actcgactgg ttctcccagg agcaggaaaa acccttgtga 3240
   catgaaacat ctcaggcctg aaaagaaagt gctctctcag atggactctt gcatgttaag 3300
   actatgtctt cacatcatgg tgcaaatcac atgtacccaa tgactccggc tttgacacaa 3360
   caccttacca tcatcatgcc atgatggctt ccacaaagca ttaaacctgg taaccagaga 3420
   ttactggtgg ctccagcgtt gttagatgtt catgaaatgt gaccacctct caatcacctt 3480
   tgagggctaa agagtagcac atcaaaagga ctccaaaatc ccatacccaa ctcttaagag 3540
   atttgtcctg gtacttcaga aagaattttc atgagtgttc ttaattggct ggaaaagcac 3600
   cagctgacgt tttggaagaa tctatccatg tgtctgcctc catatgcatc tgggcatttc 3660
   atcttcagtc ccctcattag actgtagcat taggatgtgt ggagagagga gaaatgattt 3720
   agcacccaga ttcacactcc tatgcctgga agggggacat ctttgaagaa gaggaattag 3780
   ggctgtggac actgtcttga ggatgtggac ttccttagtg agctccacat tacttgatgg 3840
   taaccacttc aaaaggatca gaatccacgt aatgaaaaag gtccctctag aggatggagc 3900
   tgatgtgaag ctgccaatgg atgaaaagcc tcagaaagca actcaaagga ctcaaagcaa 3960
   cggacaacac aagagttgtc ttcagcccag tgacacctct gatgtcccct ggaagctttg 4020
   tgctaacctg ggactgcctg acttccttta gcctggtccc ttgctactac cttgaactgt 4080
   tttatctaac ctctcttttt ctgtttaatt ctttgctact gccattgacc ctgctgcagg 4140
   atttgtgtca ttttcctgcc tggttgctga gactccattt tgctgccaca cacagagatg 4200
   taagaggcag gctttaattg ccaaagcaca gtttgagcag tagaaaacaa catggtgtat 4260
   atctcaaatt gcctgacatg aagaggagtc taacggtgaa gtttcacttt tcatcagcat 4320
   catctttcac atgttcatta tcatctgctc ttattcttgc atgtttaaac acttaaaatt 4380
   tttagtataa tttttagtgt gttttgaagt ggtgactagg ctttcaaaaa cttccattga 4440
   attacaaagc actatccagt tcttattgtt aaactaagta aaaatgataa gtaacatagt 4500
   gtaaaatatt cctttactgt gaacttctta caatgctgtg aatgagaggc tcctcagaac 4560
   tggagcattt gtataataat tcatcctgtt catcttcaat tttaacatca tatataattt 4620
   caattctatc aattgggcct ttaaaaatca tataaaagga tataaaattt gaaaagagaa 4680
   acctaattgg ctatttaatc caaaacaact ttttttttcc ttcaatggaa tcagaaagct 4740
   tgtcaatcac tcatgtgttt tagagtaatt acttttaaaa tggtgcattt gtgcttctga 4800
   actattttga agagtcactt ctgtttacct caagtatcaa ttcatcctcc atacatttga 4860
   attcaagttg tttttttgtc aaatttacag ttgtcaattg atcttcaagc tgcagggtgc 4920
   ctagaaatgg gccgttgtct gtagccctgg catgtgcaca cggacatttg ccaccactgc 4980
   aagcaaaagt ctggagaagt tcaccaacga caagaacgat tagggaaaat atgctgctgt 5040
   gggttaacaa ctcagaaagt ccctgatcca catttggctg tttactaaag cttgtgatta 5100
   actttttggc agtgtgtact atgctctatt gctatatatg ctatctataa atgtagatgt 5160
   taaggataag taattctaaa tttattattc tatagttttg aagtttggtt aagtttcctt 5220
   tcactcaatt gatttatttt gttgttaatc aaatttatgt taattggatc ctttaaattt 5280
   tttttggcat tttccaacaa aaatggcttt attcataaga aaggaaaaaa atcaatggaa 5340
   tttgatatct aaagaagtta gaaagggagc aaaataaaaa acataaagga gatagatgaa 5400
   ttagtaagca aatcagtagt cgagtttttc aaactggcaa aattaattaa ttgactttta 5460
   gcccaaattt acattgttaa ttaaatcaag aaggaagaag atctaagagc tcccattgat 5520
   aggcaagcct agagagaact agctaaattt atcatgctag gatattgaaa cacagaaagt 5580
   ttacatacat ttatgaaggg tcaatttagt ttggacagtg aggtatttgt cttagtggaa 5640
   aaaaggagaa ttagtctgat caaatcgtga agtaatacag tgaacttgca ggtgcacaaa 5700
   ataagagggc cacatctata tggtgcagtc tggaattctg tttaagtttg taggtacctc 5760
   ttggacttct gaattgatcc agttgtcatc caccacagac atctcacatc agatacagac 5820
   agttccaaga ttgacaacag agaacaacct gctggaaaga cctgggcaga aatggagagc 5880
   cctgcgggaa ccatgctaca ttttcatcta aagagagaat gcacatctga tgagactgaa 5940
   agttctttgt tgttttagat tgtagaatgg tattgaattg gtctgtggaa aattgcattg 6000
   cttttatttc tttgtgtaat caagtttaag taatagggga tatataatca taagcatttt 6060
   agggtgggag ggactattaa gtaattttaa gtgggtgggg ttatttagaa tgttagaata 6120
   atattatgta ttagatatcg ctataagtgg acatgcgtac ttacttgtaa ccctttaccc 6180
   tataattgct atccttaaag atttcaaata aactcggagg gaactgcagg gagaccaact 6240
   tatttagagc gaattggaca tggataaaaa ccccagtggg agaaagttca aaggtgatta 6300
   gattaataat ttaatagagg atgagtgacc tctgataaat tactgctaga atgaacttgt 6360
   caatgatgga tggtaaattt tcatggaagt tataaaagtg ataaataaaa acccttgctt 6420
   ttacccctgt cagtagccct cctcctacca ctgaacccca ttgcccctac ccctccttct 6480
   aactttattg ctgtattctc ttcactctat atttctctct atttgctaat attgcattgc 6540
   tgttacaata aaaattcaat aaagatttag tggttaagtg caaaaaaaaa aaaaaaaaaa 6600
   aa                                                                6602
<210> 264
   <211> 1647
   <212> DNA
   <213> Homo sapiens
<400> 264
   gggttatatg atctctttgg ctttagggaa ttactccata ccagctctga gatttccagc      60
   tcagcgatgc ccccaggtcc ctgggagagc tgcttctggg tggggggcct cattttgtgg     120
   ctcagcgttg gaagttcagg gttttaggaa caaagccttc ctggattgac acatttatta     180
   gaacccttct gcgtgcaacg aatgctaatg tgattgccgt ggactggatt tatgggtcta     240
   caggagtcta cttctcagct gtgaaaaatg tgattaagtt gagcctcgag atctcccttt     300
   tcctcaataa actcctggtg ctgggtgtgt cggaatcctc aatccacatc attggtgtta     360
   gcctgggggc ccacgttggg ggcatggtgg gacagctctt cggaggccag ctgggacaga     420
   tcacaggcct ggaccccgct ggacctgagt acaccagggc cagtgtggaa gagcgcttgg     480
   atgctggaga tgccctcttc gtggaagcca tccacacaga caccgacaat ttgggtattc     540
   ggattcccgt tggacatgtg gactacttcg tcaacggagg ccaagaccaa cctggctgcc     600
   ccaccttctt ttacgcaggt tatagttatc tgatctgtga tcacatgagg gctgtgcacc     660
   tctacatcag cgccctggag aattcctgtc cactgatggc ctttccctgt gccagctaca     720
   aggccttcct tgctggacgc tgtctggatt gctttaaccc ttttctgctt tcctgcccaa     780
   ggataggact ggtggaacaa ggtggtgtca agatagagcc gctccccaag gaagtgaaag     840
   tctacctcct gactacttcc agtgctccgt actgcatgca tcacagcctc gtggagtttc     900
   acttgaagga actgagaaac aaggacacca acatcgaggt taccttcctt agcagtaaca     960
   tcacctcttc atctaagatc accataccta agcagcaacg ctatgggaaa ggaatcatag    1020
   cccatgccac cccacaatgc cagataaacc aagtgaaatt caagtttcag tcttccaacc    1080
   gagtttggaa aaaagaccgg actaccatta ttgggaagtt ctgcactgcc cttttgcctg    1140
   tcaatgacag agaaaagatg gtctgcttac ctgaaccagt gaacttacaa gcaagtgtga    1200
   ctgtttcctg tgacctgaag atagcctgtg tgtagtttaa cctgggcagg acacatctcc    1260
   ctgcattttt tttttttttt tgagagagag gtgtgatgag ggatgtgtgt gtgcagctta    1320
   ttgtagacca ttactactaa ggagaaaagc aaagctcttt cttattttcc tcataatcag    1380
   ctaccctgga ggggagggag aactcatttt acagaacttg gtttcctttg ccgatcttat    1440
   gtacataccc attttagctt tcccatgcat acttaactgc acttgcttta tctccttggg    1500
   cattcgtact taggattcaa tagaaacatg tacagggtaa acaatttttt aaaaataaaa    1560
   cttcatggag tatctgaaaa aaaaaaaaaa aaaaaaaaaa aaaaaaaaaa aaaaaaaaaa    1620
   aaaaaaaaaa aaaaaaaaaa aaaaaaa                                        1647
<210> 265
   <211> 1798
   <212> DNA
   <213> Homo sapiens
<400> 265
   gccgcccggc cgagcgcgga gcgcagccac tcgccgctgc ccagggagcg cccaagatgt      60
   ggggggaccg gggcggcagc ggccgtagca gcgccaggga cgggggcacg cagcagcctc     120
   cgctcgcccg cctgtcctga cctgcctcgc ttgcccccaa agaatgtcag ccaagtccaa     180
   ggggaacccc tcctcgtcct gtccagccga gggaccgccg gcagcctcca aaaccaaggt     240
   gaaggaacag atcaagatca tcgtggagga tttggaatta gtcctgggcg acctgaagga     300
   cgtggccaag gaacttaagg agatgaagtc ccactctgtt gcccaggcta gagtgcaatg     360
   gcacaatctg ggctcactgc aacctctgcc tcccaggttc aagctattct cctgcctcag     420
   cctgcctcag tgcgccacta cgcctgggtg gttgaccaga ttgacaccct gacctctgac     480
   ctacagctgg aggatgagat gactgacagc tccaaaacgg acacgctgaa tagtagctca     540
   agtggcacaa cagcctccag cctagagaag atcaaagtgc aggctaatgc accgcttatt     600
   aaacccccag cacacccatc tgctatcctc acggtcctga gaaagccaaa ccctccacca     660
   cctcctccaa ggttgacacc tgtgaagtgt gaagacccca aaagggtggt tccaactgcc     720
   aatcctgtaa aaaccaatgg cacccttcta cgaaatggag gcttaccagg tggacctaac     780
   aaaattccaa atggagatat ctgctgcata cccaacagta acttggacaa ggctccagtc     840
   cagcttctga tgcatagacc tgaaaaagac agatgtcccc aggcagggcc tcgagaacga     900
   gttcggttta atgaaaaagt acagtaccat ggctattgtc ctgactgtga tacccggtat     960
   aacataaaaa acagggaggt ccacttacac agtgaacctg tccacccacc gggaaagatt    1020
   cctcaccaag gccctcccct ccctcctaca ccccatctcc ctcctttccc actagaaaat    1080
   gggggaatgg gaataagcca cagtaacagc ttccccccta tcagacctgc aactgtgcct    1140
   cctcccactg caccaaaacc acagaagacg atcttgagga agtcaaccac tacaaccgtg    1200
   tgatgtatgc cattaaaaaa attgtttttt taattttcta tattataaac ataaaataag    1260
   taatgagcac tttctactca agcaataaaa agcccaaata tattaatcct gcattcagca    1320
   aagtggcata aaaatcacct ggtaagtatg cagcacattg cttatatcct gggtatgcat    1380
   tattttaaat gttgtatcat taaaaacctc agaatgatga aaaatatgaa tgatgcattg    1440
   tttttgcaat tgacctatga caaactgtga acctgcagat ttcacctatt ttgatttact    1500
   ataagagctg ggatttgatt cattttattt atgcctaagt catctatgca ttaacatgtc    1560
   atattcttaa ctttgatcta atgcttttta ctaggaaatt ttaatactga aggactattt    1620
   tattattttt ttctaaagat gtttgtcact agtttttcat tattaaatgc tgaggccaat    1680
   accaagaagt ttattttcta tattatacaa ttatgaatta catgctcagc tatatatgta    1740
   ataaaatact ttggtctgtg gaaaaaaaaa aaaaaaaaaa aaaaaaaaaa aaaaaaaa      1798
<210> 266
   <211> 1982
   <212> DNA
   <213> Homo sapiens
<400> 266
   agacaagatt tttcaagcaa gatgaagtcc atcatcctct ttgtcctttc cctgctcctt      60
   atcttggaga agcaagcagc tgtgatggga caaaaaggtg gatcaaaagg ccaattgcca     120
   agcggatctt cccaatttcc acatggacaa aagggccagc actattttgg acaaaaagac     180
   caacaacata ctaaatccaa aggcagtttt tctattcaac acacatatca tgtagacatc     240
   aatgatcatg actggacccg aaaaagtcag caatatgatt tgaatgccct acataaggcg     300
   acaaaatcaa aacaacacct aggtggaagt caacaactgc tcaattataa acaagaaggc     360
   agagaccatg ataaatcaaa aggtcatttt cacatgatag ttatacatca taaaggaggc     420
   caagctcatc atgggacaca aaatccttct caagatcagg ggaatagccc atctggaaag     480
   ggattatcca gtcaatgttc aaacacagaa aaaaggctat gggttcatgg actaagtaaa     540
   gaacaagctt cagcctctgg tgcacaaaaa ggtagaacac aaggtggatc ccaaagcagt     600
   tatgttctcc aaactgaaga actagtagtt aacaaacaac aacgtgagac taaaaattct     660
   catcaaaata aagggcatta ccaaaatgtg gttgacgtga gagaggaaca ttcaagtaaa     720
   ctacaaactt cactccatcc tgcacatcaa gacagactcc aacatggacc caaagacatt     780
   tttactaccc aagatgagct cctagtatat aacaagaatc aacaccagac aaaaaatctc     840
   agtcaagatc aagagcatgg ccggaaggca cataaaatat catacccgtc ttcacgtaca     900
   gaagaaagac aacttcacca tggagaaaag agtgtacaga aagatgtatc caaaggcagc     960
   atttctatcc aaactgaaga gaaaatacat ggcaagtctc aaaaccaggt aacaattcat    1020
   agtcaagatc aagagcatgg ccataaggaa aataaaatat cataccaatc ttcaagtaca    1080
   gaagaaagac atctcaactg tggagaaaag ggcatccaga aaggtgtatc caaaggcagt    1140
   atttcgatcc aaactgaaga gcaaatacat ggcaagtctc aaaaccaggt aagaattcct    1200
   agtcaagctc aagagtatgg ccataaggaa aataaaatat cataccaatc ttcgagtaca    1260
   gaagaaagac gtctcaacag tggagaaaag gatgtacaga aaggtgtatc caaaggcagt    1320
   atttctatcc aaactgaaga gaaaatacat ggcaagtctc aaaaccaggt aacaattcct    1380
   agtcaagatc aagagcatgg ccataaggaa aataaaatgt cataccaatc ttcaagtaca    1440
   gaagaaagac gactcaacta tggaggaaag agcacgcaga aagatgtatc ccaaagcagt    1500
   atttctttcc aaattgaaaa gctagtagaa ggcaagtctc aaatccagac accaaatcct    1560
   aatcaagatc aatggtctgg ccaaaatgca aaaggaaagt ctggtcaatc tgcagatagc    1620
   aaacaagacc tactcagtca tgaacaaaaa ggcagataca aacaggaatc cagtgagtca    1680
   cataatattg taattactga gcatgaggtt gcccaagatg atcatttgac acaacaatat    1740
   aatgaagaca gaaatccaat atctacatag ccctgttgct tagcaaccac ttgaaaagct    1800
   ggaccaatag caaggtgtca cccgacctca gtgaagtctt tgatgtttct gagaggcaga    1860
   ctcccatgtg gtcccagatc cttggtccat ggatgacacc accttcccat gcttccttgc    1920
   attaggcttt ctaaacccgg agccccttca aacttccaat aaagggatca ttttctgctt    1980
   ta                                                                   1982
<210> 267
   <211> 2894
   <212> DNA
   <213> Homo sapiens
<400> 267
   atatggatgc acacagagcc tgtagacctg agtggatgga cactgcctct tagaactaga      60
   acttagaact ttatcttgaa aatgtaccac tgttgcagaa gctcctcaca gagtatgtgt     120
   caggcatttt taacctgcta aaggcaagaa gaagtgttca ccacatagtt gcaaaggtct     180
   tcaacttgcc acagccaaca gaaaaatcaa aatgattgaa ccctttggga atcagtatat     240
   tgtggccagg ccagtgtatt ctacaaatgc ttttgaggaa aatcataaaa agacaggaag     300
   acatcataag acatttctgg atcatctcaa agtgtgttgt agctgttccc cacaaaaggc     360
   caagagaatt gtcctctctt tgttccccat agcatcttgg ttgccagcat accggcttaa     420
   agaatggttg ctcagtgata ttgtttctgg tatcagcaca gggattgtgg ccgtactaca     480
   aggtttagca tttgctctgc tggtcgacat tcccccagtc tatgggttgt atgcatcctt     540
   tttcccagcc ataatctacc ttttcttcgg cacttccaga cacatatccg tgggtccgtt     600
   tccgattctg agtatgatgg tgggactagc agtttcagga gcagtttcaa aagcagtccc     660
   agatcgcaat gcaactactt tgggattgcc taacaactcg aataattctt cactactgga     720
   tgacgagagg gtgagggtgg cggcggcggc atcagtcaca gtgctttctg gaatcatcca     780
   gttggctttt gggattctgc ggattggatt tgtagtgata tacctgtctg agtccctcat     840
   cagtggcttc actactgctg ctgctgttca tgttttggtt tcccaactca aattcatttt     900
   tcagttgaca gtcccgtcac acactgatcc agtttcaatt ttcaaagtac tatactctgt     960
   attctcacaa atagagaaga ctaatattgc agacctggtg acagctctga ttgtcctttt    1020
   ggttgtatcc attgttaaag aaataaatca gcgcttcaaa gacaaacttc cagtgcccat    1080
   tccaatcgaa ttcattatga ccgtgattgc agcaggtgta tcctacggct gtgactttaa    1140
   aaacaggttt aaagtggctg tggttgggga catgaatcct ggatttcagc cccctattac    1200
   acctgacgtg gagactttcc aaaacaccgt aggagattgc ttcggcatcg caatggttgc     1260
   atttgcagtg gccttttcag ttgccagcgt ctattccctc aaatacgatt atccacttga     1320
   tggcaatcag gagttaatag ccttgggact gggtaacata gtctgtggag tattcagagg     1380
   atttgctggg agtactgccc tctccagatc agcagttcag gagagcacag gaggcaaaac     1440
   acagattgct gggcttattg gtgccatcat cgtgctgatt gtcgttctag ccattggatt     1500
   tctcctggcg cctctacaaa agtccgtcct ggcagcttta gcattgggaa acttaaaggg     1560
   aatgctgatg cagtttgctg aaataggcag attgtggcga aaggacaaat atgattgttt     1620
   aatttggatc atgaccttca tcttcaccat tgtcctggga ctcgggttag gcctggcagc     1680
   tagtgtggca tttcaactgc taaccatcgt gttcaggacc caatttccaa aatgcagcac     1740
   gctggctaat attggaagaa ccaacatcta taagaataaa aaagattatt atgatatgta     1800
   tgagccagaa ggagtgaaaa ttttcagatg tccatctcct atctactttg caaacattgg     1860
   tttctttagg cggaaactta tcgatgctgt tggctttagt ccacttcgaa ttctacgcaa     1920
   gcgcaacaaa gctttgagga aaatccgaaa actgcagaag caaggcttgc tacaagtgac     1980
   accaaaagga tttatatgta ctgttgacac cataaaagat tctgacgaag agctggacaa     2040
   caatcagata gaagtactgg accagccaat caataccaca gacctgcctt tccacattga     2100
   ctggaatgat gatcttcctc tcaacattga ggtccccaaa atcagcctcc acagcctcat     2160
   tcttgacttt tcagcagtgt cctttcttga tgtttcttca gtgaggggcc ttaaatcgat     2220
   tttgcaagaa tttatcagga tcaaggtaga tgtgtatatc gttggaactg atgatgactt     2280
   cattgagaag cttaaccggt atgaattttt tgatggtgaa gtgaaaagct caatattttt     2340
   cttaacaatc catgatgctg ttttgcatat tttgatgaag aaagattaca gtacttcaaa     2400
   gtttaatccc agtcaggaaa aagatggaaa aattgatttt accataaata caaatggagg     2460
   attacgtaat cgggtatatg aggtgccagt tgaaacaaaa ttctaatcaa catataattc     2520
   agaaggatct tcatctgact atgacataaa aacaacttta tacccagaaa gttattgata     2580
   agttcataca ttgtacgaag agtatttttg acagaatatg tttcaaactt tggaacaaga     2640
   tggttctagc atggcatatt tttcacatat ctagtatgaa attatataag tattctaaat     2700
   tttatatctt gtagctttat caaagggtga aaattatttt gttcatacat atttttgtag     2760
   cactgacaga tttccatcct agtcactacc ttcatgcata ggtttagcag tatagtggcg     2820
   ccactgtttt gaatctcata atttatacag gtcatattaa tatatttcca ttaaaaaatc     2880
   agttgtacag tgga                                                      2894
<210> 268
   <211> 3894
   <212> DNA
   <213> Homo sapiens
<400> 268
   agtagtgaat cgtagcatgt tagagttaga aggttcagtg ttgatggagt tattgaagaa      60
   atgatggagt aagagactct tttctaagca actcaagttt gcagtgattc aggcctactt     120
   ctgaagagac agccttttat ctcaatgaat gacacagaaa aaccagcaga tactccctct     180
   gaggaagagg actttggtga tccaaggaca tatgacccag atttcaaggg gcctgttgcc     240
   aacaggagtt gtacagatgt tctgtgctgt atgatcttcc tactgtgtat tattggctac    300
   attgttttag gacttgtggc ctgggtacat ggggacccca gaagagcagc ctatcctaca    360
   gacagccagg gccacttttg tggccagaag ggcactccca atgagaacaa gaccattttg     420
   ttttacttta acctgttacg ctgtaccagt ccctccgtgt tgctaaacct acagtgccct     480
   accacacaga tctgtgtctc caagtgccca gaaaaatttt taacctatgt ggaaatgcaa     540
   cttttgtaca caaaagacaa aagctactgg gaagactacc gtcagttctg taagaccact     600
   gctaagcctg tgaagtctct cacacagctt ttactggatg atgattgtcc aacagcgatt     660
   tttcccagca aaccttttct ccagagatgt ttccctgact tctctaccaa aaatggcact     720
   ttaacaatag gaagtaagat gatgtttcaa gatggaaatg gagggacaag aagtgttgta     780
   gaactcggga ttgctgcaaa tggtatcaat aaacttcttg atgcaaagtc acttggattg     840
   aaagtgtttg aagactatgc aagaacttgg tattggattc tcattggcct gacgattgcc     900
   atggtcctta gttggatatt tttgatactt ctgaggttca tagctggatg cctcttctgg     960
   gtcttcatga ttggtgtgat tggaattata ggttatggaa tatggcactg ttaccagcag    1020
   tacaccaatc ttcaggaacg cccaagttct gtattaacta tctatgacat cgggattcag    1080
   actaacataa gcatgtactt tgaactgcaa caaacatggt tcacatttat gataatactc    1140
   tgcatcattg aagtgattgt catcctcatg ctgatcttcc tcaggaatcg aatccgagtc    1200
   gccattatcc tgctgaagga aggaagcaaa gccattggat atgttcctag tacattagtc    1260
   tatccagctt taactttcat tttgctctca atctgcattt gctactgggt cgtgacagca    1320
   gttttcttgg cgacatcggg ggtacctgta tacaaagtca tagctccagg ggggcattgt     1380
   atacatgaaa atcaaacctg tgacccagag atttttaata caactgaaat tgccaaagct    1440
   tgccctgggg ctctgtgtaa ctttgctttc tatggtggaa agagcttgta ccatcagtac    1500
   atccctacct tccatgtata caacttattt gtctttctct ggcttataaa cttcgtcatt    1560
   gcattaggtc agtgcgccct tgctggtgca ttcgctactt attactgggc catgaaaaaa    1620
   cctgatgaca tcccacgata tccacttttt actgcatttg gacgagccat acgatatcac    1680
   acaggatccc tagcatttgg atctttaatt attgcattaa ttcaaatgtt taaaattgta    1740
   ctagaatact tggaccaccg tcttaaacgt acccagaaca cattgtctaa attcctacaa    1800
   tgctgcctga gatgctgctt ctggtgtttg gaaaatgcaa taaagttttt aaacagaaat    1860
   gcctatatta tgattgcaat atatggcaga aacttctgca ggtcagcaaa agatgctttc    1920
   aatctgctga tgagaaatgt tttgaaagtt gcagttacag atgaagttac atactttgta    1980
   ttattcctgg ggaaacttct agttgctgga agtataggtg ttctggcctt cctattcttc    2040
   acacaaagac tgccagtgat tgcacaagga ccagcatctt taaattacta ctgggtacct    2100
   ttgctgacag tcatttttgg gtcttacctg attgcacatg ggttcttcag cgtctatgca    2160
   atgtgtgttg aaacaatttt catctgcttc ttggaagatt tagaaagaaa tgatggttct    2220
   actgcaagac cttattatgt gagtcaacct ttgctgaaga ttttccagga ggaaaatcca    2280
   caaactagga agcagtagaa gagcaaactg gtcgtcctac agctgtgtgt taccttttct   2340
   ccatctgctg tgtctgtgca acatttgttt cataagtgct ttgtgtttag caacactgta    2400
   ttcacgacct tgttggcttg catttgcatg ttttatacca aagcttatac tgtactatgt    2460
   gaagccatca gaagtcgcaa gggaattgtt aataacataa aacattttta tactaagatc    2520
   atttgttttg taattcgttt ttaaagagtg gcttggatgt tttgaaaata ctactgaata    2580
   tgttaatatt cttttaaatc ttagattgaa aaatgataca ttacttaaat tgatagctcc    2640
   taatatattt ttaaaattac aactaaaaga agacttcttc tgcagggaaa attggtcagc    2700
   aaagtgaaat taaaaatttt aaagtttttc ccactctcgt tggacagtaa atcagtgaaa     2760
   ggactgcccc agttgagagt ttgctctctt taagtataga atgtttcctc ttaaacaaat    2820
   tgccaatcat ccagccttta ctacttagcc ctctgacaaa gtgccttact ggctatttaa    2880
   tattacccag cttttatggg caagtttaca aacattgttt tttaaaaaat taaaacctgc    2940
   aatgtttcgt gattaaaaca agtcttattg catttgtttc actcttagct cactgattgg    3000
   aaaacatttg tcattttgct ctgtttgata tcctcactat tatggaatac attgtgcagc    3060
   taaacaattt cccttgcgcc tagtggacat tcatgaatgt gtactacacg caagaagaaa    3120
   caaaccccga aagaacactt gttggatttc tttgtttttt tttttactaa aagagaagtt    3180
   ttaaaatgaa atgttttcta tagtagatct ttgaaaatac aataggtata atactgcatt    3240
   tctcagtgtt ttacaaagat cagaaagaga aacttctagg aattgcaaag ggaaacttta    3300
   ctcctcgaaa gggtgctcac agatgtcatg tactgaatag ctccctttta aatgatcatt    3360
   tattttcatc aaagcctgtt ctatatatgc cacttcattt tctaactttt ggtatgaaaa    3420
   aatcagttta cttacagtat gttaattgta ttgtactact ataaacagga acataatttc    3480
   caattcagtt ttaaataatt ttaccagtac tactaacttt taaggaaatt aattcagttg    3540
   gttactcagt gtttgttaca gaaagagtcc agaaaagtat tcaccctaag agaatgtcaa    3600
   tcatataatg ataatttgtg aaagctttga gaatcaatca tcagtaagtt actatcagtt    3660
   tataaaatat tatcacattt gtttaaatgt gactttagat acttttatgc caaaaataaa    3720
   ctcacatgag cacatgacag tctgagctct ataatcagtg tgcttctgct gtgcagaaat    3780
   gttagaaacg tattgtctaa atatctttga taattaaaat gtttaatatt taatgaaatt    3840
   tgttgttact tgttttaaat cttttttctt ttaataaaga tttaaataag aaat          3894
<210> 269
   <211> 2799
   <212> DNA
   <213> Homo sapiens
<400> 269
   agcttcgagg ccagtgggag gagggagggg ccaggcagct gagggccagg aaagatgtga      60
   aaaactctag ctggtgaccg agaggaggag tagagtgtgc ccttagttca tatgaactag     120
   agggagttgg tatttgcaca gcagtcaggg tcacatgagt gatcatggta cagtgagaag     180
   ttctccctcc cagggccagg tcacagggtt tgtttctgtt caatccggat tcttccagta     240
   aaagcttcaa cttcccacac tgaagctgag agcctcccaa agtgctggct acctgctgag     300
   cgcccccgta actctgacac agtagtaatt tgagcctctg caattgccgt ctgcttcctg     360
   tgaaagtcct ttccgtgccc actgaccctt gagtgggcct ttgagctgct gactttcagc     420
   tggaacttga agggacccca accctgagac actatggccc tgacctcaga cctggggaaa     480
   cagataaaac tgaaagaggt ggaggggacc ctcctgcagc ctgcaactgt ggacaactgg     540
   agccagatcc agagcttcga ggccaaacca gatgatctcc tcatctgcac ctaccctaaa     600
   gcagggacaa cgtggattca ggaaattgtg gatatgattg aacagaatgg ggacgtggag     660
   aagtgccagc gagccatcat ccaacaccgc catcctttca ttgagtgggc tcggccaccc     720
   caaccttctg gtgtggaaaa agccaaagca atgccctctc cacggatact aaagactcac     780
   ctttccactc agctgctgcc accgtctttc tgggaaaaca actgcaagtt cctttatgta     840
   gctcgaaatg ccaaagactg tatggtttcc tactaccatt tccaaaggat gaaccacatg     900
   cttcctgacc ctggtacctg ggaagagtat tttgaaacct tcatcaatgg aaaagtggtt     960
   tggggttcct ggtttgacca cgtgaaagga tggtgggaga tgaaagacag acaccagatt    1020
   ctcttcctct tctatgagga cataaagagg gacccaaagc atgaaattcg gaaggtgatg    1080
   cagttcatgg gaaagaaggt ggatgaaaca gtgctagata aaattgtcca ggagacgtca    1140
   tttgagaaaa tgaaagaaaa tcccatgaca aatcgttcta cagtttccaa atctatcttg    1200
   gaccagtcaa tttcctcctt catgagaaaa ggaactgtgg gggattggaa aaaccacttc    1260
   actgttgccc agaatgagag gtttgatgaa atctatagaa gaaagatgga aggaacctcc    1320
   ataaacttct gcatggaact ctgagcaaga tgtaaataaa attaaaaggt ggatggcaag    1380
   agtgcaaata ctatcttcaa tccttcagtc ccagccagaa gaatctctga aagcatattg    1440
   tgaatgtata caatgtagta caaacaatct ctgtgatgat taacagtatg tcaccacttc    1500
   attttttaaa aaggatcacg tctaatgccc attttcccaa ctattctttc caaagtaaga    1560
   tataaggtag cttaataaac taagtaaaac gtatgacttg agtacaaaag gattgtttta    1620
   atccccatta ttctggaaag tgcatcctag tctcccagtc tataacatca taataccttg    1680
   agtataagtc caaatattag gttatatcta tattaaaaac aaaatttctg tcatctgtcc    1740
   tggccattca ggcaactcca gcctgggctc aatcctggag ttctgtctgg tcactatcag    1800
   aaggaacact ttgagggaaa ccctggtgca gccagccctg aggaaacatg gcctgagtgc    1860
   cctcactggt gggtgggaat aaaatggaag tgcacagagg agatgtcaga agaccaaaac    1920
   ttggtgaata gtcccagtgc taggtcatat aggaaacaga aagcatgaca gtggcctttt    1980
   gggaacccaa gttacgtcct ggtgaaagca gaaaggagga gacaggatgg ctgtcaacaa    2040
   cgtcagcatg ggcatggctc ccaggcatgg aggtaattgg gtcttggctt caatgccacc    2100
   cttttgggaa gccctaaacc aagtgaggtg ttcttttccc ttgcttccca tatttgtccc    2160
   agttgcagcc ctgttgttat gtatttactc ttcaatttct tatttatacc tctcactcaa    2220
   ttgttaattc tttgggttca gtctcatgcc atatttactc ctagtaccta atgcagtgcc    2280
   tggcacacag caagtgccct tgagaacttg tagagggagt aaaaaagtgc atggataaat    2340
   gactgcagca aatcacaaat tttgaacttt gacccttttc ttttagtccc aaatagtggc    2400
   attccatagc tgagggtagt aggagcagtt caccctggga gcagacaata agaggaagaa    2460
   tgaagccaac taaaattctg aatgcttttt gttggatttc gtagggtatt ttttttaaaa     2520
   tagggtcttg ctatgttgcc caggctggtc ttaactgact gctttttatt attaccatgc    2580
   actggcaatt ccaaacaatg tcagtgttaa aatgcttctc cctgaaaaag agaaaaaaaa    2640
   aaggaaaaaa agaaaagaaa agtgaaaaga aaaactctta ttggcctaag ttctaaataa    2700
   tagctaggtt accactgagt tttaactata tgtatatgag cttcaaataa gcaccttttt    2760
   attatgtaaa taaataaaag ttatctgtat ccccaaaaa                           2799
<210> 270
   <211> 1054
   <212> DNA
   <213> Homo sapiens
<400> 270
   gccaaaacag tgggggctga actgacctct cccctttggg agagaaaaac tgtctgggag      60
   cttgacaaag gcatgcagga gagaacagga gcagccacag ccaggaggga gagccttccc     120
   caagcaaaca atccagagca gctgtgcaaa caacggtgca taaatgaggc ctcctggacc     180
   atgaagcgag tcctgagctg cgtcccggag cccacggtgg tcatggctgc cagagcgctc     240
   tgcatgctgg ggctggtcct ggccttgctg tcctccagct ctgctgagga gtacgtgggc     300
   ctgtctgcaa accagtgtgc cgtgccagcc aaggacaggg tggactgcgg ctacccccat     360
   gtcaccccca aggagtgcaa caaccggggc tgctgctttg actccaggat ccctggagtg     420
   ccttggtgtt tcaagcccct gcaggaagca gaatgcacct tctgaggcac ctccagctgc     480
   ccccggccgg gggatgcgag gctcggagca cccttgcccg gctgtgattg ctgccaggca     540
   ctgttcatct cagcttttct gtccctttgc tcccggcaag cgcttctgct gaaagttcat     600
   atctggagcc tgatgtctta acgaataaag gtcccatgct ccacccgagg acagttcttc     660
   gtgcctgaga ctttctgagg ttgtgcttta tttctgctgc gtcgtgggag agggcgggag     720
   ggtgtcaggg gagagtctgc ccaggcctca agggcaggaa aagactccct aaggagctgc     780
   agtgcatgca aggatatttt gaatccagac tggcacccac gtcacaggaa agcctaggaa     840
   cactgtaagt gccgcttcct cgggaaagca gaaaaaatac atttcaggta gaagttttca     900
   aaaatcacaa gtctttcttg gtgaagacag caagccaata aaactgtctt ccaaagtggt     960
   cctttatttc acaaccactc tcgctactgt tcaatacttg tactattcct gggttttgtt    1020
   tctttgtaca gtaaacatta tgaacaaaca ggca                                1054
<210> 271
   <211> 1669
   <212> DNA
   <213> Homo sapiens
<400> 271
   gaggtataag agcctccaag tctgcagctc tcgcccaact cccagacacc tcgcgggctc      60
   tgcagcaccg gcaccgtttc caggaggcct ggcggggtgt gcgtccagcc gttgggcgct     120
   ttctttttgg acctcggggc catccacacc gtcccctccc cctcccgcct ccctccccgc     180
   ctcccccgcg cgccctcccc gcggaggtcc ctcccgtccg tcctcctgct ctctcctccg     240
   cgggccgcat cgcccgggcc ggcgccgcgc gcgggggaag ctggcgggct gaggcgcccc     300
   gctcttctcc tctgccccgg gcccgcgagg ccacgcgtcg ccgctcgaga gatgatgcag     360
   gacgtgtcca gctcgccagt ctcgccggcc gacgacagcc tgagcaacag cgaggaagag     420
   ccagaccggc agcagccgcc gagcggcaag cgcgggggac gcaagcggcg cagcagcagg     480
   cgcagcgcgg gcggcggcgc ggggcccggc ggagccgcgg gtgggggcgt cggaggcggc     540
   gacgagccgg gcagcccggc ccagggcaag cgcggcaaga agtctgcggg ctgtggcggc     600
   ggcggcggcg cgggcggcgg cggcggcagc agcagcggcg gcgggagtcc gcagtcttac     660
   gaggagctgc agacgcagcg ggtcatggcc aacgtgcggg agcgccagcg cacccagtcg     720
   ctgaacgagg cgttcgccgc gctgcggaag atcatcccca cgctgccctc ggacaagctg     780
   agcaagattc agaccctcaa gctggcggcc aggtacatcg acttcctcta ccaggtcctc     840
   cagagcgacg agctggactc caagatggca agctgcagct atgtggctca cgagcggctc     900
   agctacgcct tctcggtctg gaggatggag ggggcctggt ccatgtccgc gtcccactag   960
   caggcggagc cccccacccc ctcagcaggg ccggagacct agatgtcatt gtttccagag    1020
   aaggagaaaa tggacagtct agagactctg gagctggata actaaaaata aaaatatatg    1080
   ccaaagattt tcttggaaat tagaagagca aaatccaaat tcaaagaaac agggcgtggg    1140
   gcgcactttt aaaagagaaa gcgagacagg cccgtggaca gtgattccca gacgggcagc    1200
   ggcaccatcc tcacacctct gcattctgat agaagtctga acagttgttt gtgttttttt    1260
   tttttttttt tttgacgaag aatgttttta tttttatttt tttcatgcat gcattctcaa    1320
   gaggtcgtgc caatcagcca ctgaaaggaa aggcatcact atggactttc tctattttaa    1380
   aatggtaaca atcagaggaa ctataagaac acctttagaa ataaaaatac tgggatcaaa    1440
   ctggcctgca aaaccatagt cagttaattc tttttttcat ccttcctctg aggggaaaaa   1500
   caaaaaaaaa cttaaaatac aaaaaacaac attctattta tttattgagg acccatggta    1560
   aaatgcaaat agatccggtg tctaaatgca ttcatatttt tatgattgtt ttgtaaatat    1620
   ctttgtatat ttttctgcaa taaataaata taaaaaattt agagaaaaa                1669
<210> 272
   <211> 823
   <212> DNA
   <213> Homo sapiens
<400> 272
   aaacgcgggc gggcgggccc gcagtcctgc agttgcagtc gtgttctccg agttcctgtc      60
   tctctgccaa cgccgcccgg atggcttccc aaaaccgcga cccagccgcc actagcgtcg     120
   ccgccgcccg taaaggagct gagccgagcg ggggcgccgc ccggggtccg gtgggcaaaa     180
   ggctacagca ggagctgatg accctcatga tgtctggcga taaagggatt tctgccttcc     240
   ctgaatcaga caaccttttc aaatgggtag ggaccatcca tggagcagct ggaacagtat      300
   atgaagacct gaggtataag ctctcgctag agttccccag tggctaccct tacaatgcgc     360
   ccacagtgaa gttcctcacg ccctgctatc accccaacgt ggacacccag ggtaacatat     420
   gcctggacat cctgaaggaa aagtggtctg ccctgtatga tgtcaggacc attctgctct     480
   ccatccagag ccttctagga gaacccaaca ttgatagtcc cttgaacaca catgctgccg     540
   agctctggaa aaaccccaca gcttttaaga agtacctgca agaaacctac tcaaagcagg     600
   tcaccagcca ggagccctga cccaggctgc ccagcctgtc cttgtgtcgt ctttttaatt     660
   tttccttaga tggtctgtcc tttttgtgat ttctgtatag gactctttat cttgagctgt     720
   ggtatttttg ttttgttttt gtcttttaaa ttaagcctcg gttgagccct tgtatattaa     780
   ataaatgcat ttttgtcctt ttttagacaa aaaaaaaaaa aaa                        823
<210> 273
   <211> 473
   <212> DNA
   <213> Homo sapiens
<400> 273
   ttttttttac ataatatgtt gtttatttga tattctggag aagtccaaac acacaaagtg      60
   attctgtatt tgcgagaaat ttaaggagat gatgaaaatg ggtaaaaaat agatttaaaa     120
   gggtgatgaa agtattatgt ataatattat aatggtaaat atgtgatatg aatttgttga     180
   aatcaacaga atatacagca taaagggtta attccaattc acaaaaatat aaataaatag     240
   gagattagga attccaggat agaatgcaga caatatagaa aatatctaat gtcattacaa     300
   atgtatgaaa tcagaagagg tgccaagtga cctcagaaat agtgtagtca ataaaagaat     360
   aaagaaagtg cacgtcagaa ctgtacccca gctgatgatg ttccacaaaa gagcaaaaca     420
   tacacaatct ggttccactc tacagaaatc ctggaactgg actacaaagg gaa             473
<210> 274
   <211> 1415
   <212> DNA
   <213> Homo sapiens
<400> 274
   gaggagagca ggccaagggg ctatataacc cttcagcttt cagcttccct gaacaccacc      60
   cagtgtggag cagcccagcc aagcactgtc aggaatcctg tgaagcagct ccagctatgt     120
   gtgaagaaga ggacagcact gccttggtgt gtgacaatgg ctctgggctc tgtaaggccg     180
   gctttgctgg ggacgatgct cccagggctg ttttcccatc cattgtggga cgtcccagac     240
   atcagggggt gatggtggga atgggacaaa aagacagcta cgtgggtgac gaagcacaga     300
   gcaaaagagg aatcctgacc ctgaagtacc cgatagaaca tggcatcatc accaactggg     360
   acgacatgga aaagatctgg caccactctt tctacaatga gcttcgtgtt gcccctgaag     420
   agcatcccac cctgctcacg gaggcacccc tgaaccccaa ggccaaccgg gagaaaatga     480
   ctcaaattat gtttgagact ttcaatgtcc cagccatgta tgtggctatc caggcggtgc     540
   tgtctctcta tgcctctgga cgcacaactg gcatcgtgct ggactctgga gatggtgtca     600
   cccacaatgt ccccatctat gagggctatg ccttgcccca tgccatcatg cgtctggatc     660
   tggctggccg agatctcact gactacctca tgaagatcct gactgagcgt ggctattcct     720
   tcgttactac tgctgagcgt gagattgtcc gggacatcaa ggagaaactg tgttatgtag     780
   ctctggactt tgaaaatgag atggccactg ccgcatcctc atcctccctt gagaagagtt     840
   acgagttgcc tgatgggcaa gtgatcacca tcggaaatga acgtttccgc tgcccagaga 900
   ccctgttcca gccatccttc atcgggatgg agtctgctgg catccatgaa accacctaca 960
   acagcatcat gaagtgtgat attgacatca ggaaggacct ctatgctaac aatgtcctat 1020
   cagggggcac cactatgtac cctggcattg ccgaccgaat gcagaaggag atcacggccc 1080
   tagcacccag caccatgaag atcaagatca ttgcccctcc ggagcgcaaa tactctgtct 1140
   ggatcggtgg ctccatcctg gcctctctgt ccaccttcca gcagatgtgg atcagcaaac 1200
   aggaatacga tgaagccggg ccttccattg tccaccgcaa atgcttctaa aacactttcc 1260
   tgctcctctc tgtctctagc acacaactgt gaatgtcctg tggaattatg ccttcagttc 1320
   ttttccaaat cattcctagc caaagctctg actcgttacc tatgtgtttt ttaataaatc 1380
   tgaaataggc tactggtaaa aaaaaaaaaa aaaaa 1415
<210> 275
   <211> 1216
   <212> DNA
   <213> Homo sapiens
<400> 275
   gcctctgggg ttttatattg ctctggtatt catgccaaag acacaccagc cctcagtcac 60
   tgggagaaga acctctcata ccctcggtgc tccagtcccc agctcactca gccacacaca 120
   ccatgtgtga agaggagacc accgcgctcg tgtgtgacaa tggctctggc ctgtgcaagg 180
   caggcttcgc aggagatgat gccccccggg ctgtcttccc ctccattgtg ggccgccctc 240
   gccaccagat ctggcaccac tccttctaca atgagctgcg tgtagcacct gaagagcacc 300
   ccaccctgct cacagaggct cccctaaatc ccaaggccaa cagggaaaag atgacccaga 360
   tcatgtttga aaccttcaat gtccctgcca tgtacgtcgc cattcaagct gtgctctccc 420
   tctatgcctc tggccgcacg acaggcatcg tcctggattc aggtgatggc gtcacccaca 480
   atgtccccat ctatgaaggc tatgccctgc cccatgccat catgcgcctg gacttggctg 540
   gccgtgacct cacggactac ctcatgaaga tcctcacaga gagaggctat tcctttgtga 600
   ccacagctga gagagaaatt gtgcgagaca tcaaggagaa gctgtgctat gtggccctgg 660
   attttgagaa tgagatggcc acagcagctt cctcttcctc cctggagaag agctatgagc 720
   tgccagatgg gcaggttatc accattggca atgagcgctt ccgctgccct gagaccctct 780
   tccagccttc ctttattggc atggagtccg ctggaattca tgagacaacc tacaattcca 840
   tcatgaagtg tgacattgac atccgtaagg acttatatgc caacaatgtc ctctctgggg 900
   gcaccaccat gtaccctggc attgctgaca ggatgcagaa ggagatcaca gccctggccc 960
   ccagcaccat gaagatcaag attattgctc ccccagagcg gaagtactca gtctggatcg 1020
   ggggctctat cctggcctct ctctccacct tccagcagat gtggatcagc aagcctgagt 1080
   atgatgaggc agggccctcc attgtccaca ggaagtgctt ctaaagtcag aacaggttct 1140
   ccaaggatcc cctcgagact actctgttac cagtcatgaa acattaaaac ctacaagcct 1200
   taaaaaaaaa aaaaaa 1216
<210> 276
   <211> 672
   <212> DNA
   <213> Homo sapiens
<400> 276
   caggccagcc ctggggcgcc ttaaaaaccg gagctggcgc ttggcatcgc cactctgggc 60
   aggatccaac gtcgctccag ctgctcttga cgactccaca gataccccga agccatggca 120
   agcaagggct tgcaggacct gaagcaacag gtggagggga ccgcccagga agccgtgtca 180
   gcggccggag cggcagctca gcaagtggtg gaccaggcca cagaggcggg gcagaaagcc 240
   atggaccagc tggccaagac cacccaggaa accatcgaca agactgctaa ccaggcctct 300
   gacaccttct ctgggattgg gaaaaaattc ggcctcctga aatgacagca gggagacttg 360
   ggtcggcctc ctgaaatgac agcagggaga cttgggtgac cccccttcca ggcgccatct 420
   agcacagcct ggccctgatc tccgggcagc caccacctcc tcggtctgcc ccctcattaa 480
   aattcacgtt cccaccctgt gtccacttca tgattcctcg caagctgggc ccagtcctct 540
   catcccaaga gcagagccac cgtagccgga gtcctagcct cccaaattcg gaaatccaat 600
   ccaacggtct caggaatgtt ttccatcccg ccacgcgcct cccgaagctc ccagaccgga 660
   ggctcagccc cc 672
<210> 277
   <211> 1599
   <212> DNA
   <213> Homo sapiens
<400> 277
   ggcaggcccc tacagccaat ggaacggccc tggaagagac ccgggtcgcc tccggagctt      60
   caaaaacatg tgaggaggga agagtgtgca gacggaactt cagccgctgc ctctgttctc     120
   agcgtcagtg ccgccactgc ccccgccaga gcccaccggc cagcatgtcc tctgctcact     180
   tcaaccgagg ccctgcctac gggctgtcag ccgaggttaa gaacaagctg gcccagaagt     240
   atgaccacca gcgggagcag gagctgagag agtggatcga gggggtgaca ggccgtcgca     300
   tcggcaacaa cttcatggac ggcctcaaag atggcatcat tctttgcgaa ttcatcaata     360
   agctgcagcc aggctccgtg aagaagatca atgagtcaac ccaaaattgg caccagctgg     420
   agaacatcgg caacttcatc aaggccatca ccaagtatgg ggtgaagccc cacgacattt     480
   ttgaggccaa cgacctgttt gagaacacca accatacaca ggtgcagtcc accctcctgg     540
   ctttggccag catggcgaag acgaaaggaa acaaggtgaa cgtgggagtg aagtacgcag     600
   agaagcagga gcggaaattc gagccgggga agctaagaga agggcggaac atcattgggc     660
   tgcagatggg caccaacaag tttgccagcc agcagggcat gacggcctat ggcacccggc     720
   gccacctcta cgaccccaag ctgggcacag accagcctct ggaccaggcg accatcagcc     780
   tgcagatggg caccaacaaa ggagccagcc aggctggcat gactgcgcca gggaccaagc     840
   ggcagatctt cgagccgggg ctgggcatgg agcactgcga cacgctcaat gtcagcctgc     900
   agatgggcag caacaagggc gcctcgcagc ggggcatgac ggtgtatggg ctgccacgcc     960
   aggtctacga ccccaagtac tgtctgactc ccgagtaccc agagctgggt gagcccgccc    1020
   acaaccacca cgcacacaac tactacaatt ccgcctaggg ccacaaggcc ttccctgttt    1080
   tccccccaag ggaggctgct gctgctcttg gctggaccca gccaggccca gccgaccccc    1140
   tctccctgca tggcatcctc cagcccctgt agaactcaac ctctacaggg ttagagtttg    1200
   gagagagcag actggcgggg ggcccattgg ggggaagggg accctccgct ctgtagtgct    1260
   acagggtcca acatagagcc gggtgtcccc aacagcgccc aaaggacgca ctgagcaacg    1320
   ctattccagc tgtcccccca ctccctcaca agtgggtacc cccaggacca gaagctcccc    1380
   cagcaaagcc cccagagccc aggctcggcc tgcccccacc ccattcccgc agtgggagca    1440
   aactgcatgc ccagagaccc agcggacaca cgcggtttgg tttgcagcga ctggcatact    1500
   atgtggatgt gacagtggcg tttgtaatga gagcactttc ttttttttct atttcactgg      1560
   agcacaataa atggctgtaa aatctcaaaa aaaaaaaaa                            1599
<210> 278
   <211> 1538
   <212> DNA
   <213> Homo sapiens
<400> 278
   tgggaagggg tgggctgcag cactggagga agggaaccct ccaccctgag atctctgtct      60
   ctatcctatc ctgtccctgg ccttctgagg caagcggggc caattaaggg gaaaacgtac     120
   ctcctccatt tgtgctgaac caatccctcc aacccctctc aggagggcat gatatggaga     180
   gttgggcatt ggctgtgttc cctgaataca gagtatctct cttgtggtgc ctggaactgg     240
   catccccttt gtggagctta gggcaagccc cgcctctgca tgagacttgg tttgtgggac     300
   acacttggtt tcagggaagg ggaaagaggt caccaagggc agaggtgtcc aggccggagc     360
   caggggcccc actgttggga tgctggctgc agtggggcgc cccaagccca ggtcccctct     420
   gtcttctctt tcgactttgc agctgtactt gttttgctcc tctacccgca ggagctgaca     480
   tggacccaaa tcctcgggcc gccctggagc gccagcagct ccgccttcgg gagcggcaaa     540
   aattcttcga ggacatttta cagccagaga cagagtttgt ctttcctctg tcccatctgc     600
   atctcgagtc gcagagaccc cccataggta gtatctcatc catggaagtg aatgtggaca     660
   cactggagca agtagaactt attgaccttg gggacccgga tgcagcagat gtgttcttgc     720
   cttgcgaaga tcctccacca accccccagt cgtctggtat gcccctctgc tttggggact     780
   tcagtgccag tcagccagag ccggatgtca ggctctgaaa cgaggctaca aggctgggct     840
   ggggaagtac acaaggatgg acaaccattt ggaggagctg agcctgccgg tgcctacatc     900
   agacaggacc acatctagga cctcctcctc ctcctcctcc gactcctcca ccaacctgca     960
   tagcccaaat ccaagtgatg atggagcaga tacgcccttg gcacagtcgg atgaagagga    1020
   ggaaaggggt gatggagggg cagagcctgg agcctgcagc tagcagtggg cccctgccta    1080
   cagactgacc acgctggcta ttctccacat gagaccacag gcccagccag agcctgtcgg    1140
   gagaagacca gactctttac ttgcagtagg caccagaggt gggaaggatg gtgggattgt    1200
   gtacctttct aagaattaac cctctcctgc tttactgcta attttttcct gctgcaaccc   1260
   tcccaccagt ttttggctta ctcctgagat atgatttgca aatgaggaga gagaagatga    1320
   ggttggacaa gatgccactg cttttcttag cactcttccc tcccctaaac catcccgtag    1380
   tcttctaata cagtctctca gacaagtgtc tctagatgga tgtgaactcc ttaactcatc    1440
   aagtaaggtg gtactcaagc catgctgcct ccttacatcc tttttggaac agagcacggt    1500
   ataaataata aactaataat aatatgccaa ccaaaaaa                             1538
<210> 279
   <211> 2579
   <212> DNA
   <213> Homo sapiens
<400> 279
   gcagagcagc ggcggcagcg gcggcggcgg cagcagccac ccgatgtctt cggcgcccga      60
   gaagcagcag ccaccgcacg gcggcggcgg cggcggcggc gggggaggcg gcgcggccat     120
   ggaccccgcg tcgtccggcc cgtccaaggc caagaagacc aacgccggca tccggcgccc     180
   ggagaagccg ccctattcct acatcgcgct catcgtcatg gccatccaga gttcacccac     240
   caagcgcctg acgctgagcg agatctacca gttcctgcag agccgcttcc ccttcttccg     300
   gggctcctac cagggctgga agaactccgt gcgccacaac ctctcgctca acgagtgctt 360
   catcaagcta cccaagggcc ttgggcggcc cggcaagggc cactactgga ccatcgaccc 420
   ggccagcgag ttcatgttcg aggagggctc ctttcggcgg cggccgcgcg gcttccgaag 480
   gaaatgccag gcgctcaagc ccatgtacag catgatgaac gggctcggct tcaaccacct 540
   cccggacacc tacggcttcc agggctcggc cggcggcctc tcgtgcccgc ccaacagcct 600
   ggcgctggag ggcggcctgg gcatgatgaa cggccacttg ccgggcaacg tggacggcat 660
   ggccctgccc agccactcgg tgccccacct gccttccaac ggcggccact cgtacatggg 720
   cggctgcggc ggcgcggcgg ccggcgagta cccgcaccac gacagctcgg tgcccgcctc 780
   cccgctgctg cccaccggcg ccggtggggt catggagccg cacgccgtct actcgggctc 840
   ggcggcggcc tggccgccct cggcgtccgc ggcgctcaac agcggcgcct cttatatcaa 900
   gcagcagccc ctgtccccct gtaaccccgc ggccaacccc ctgtccggca gcctctccac 960
   gcactccctg gagcagccgt atctgcacca gaacagccac aacgccccag ccgagctgca 1020
   aggcatcccg cggtatcact cgcagtcgcc cagcatgtgt gaccgaaagg agtttgtctt 1080
   ctctttcaac gccatggcgt cctcttccat gcactcggcc ggcgggggct cctactacca 1140
   ccagcaggtc acctaccaag acatcaagcc ttgcgtgatg tgaggctgcc gccgcaggcc 1200
   ctcctggtgc aggcaggcgg gtcacaggga ccctggaccg gcacaagaaa ctgctttctt 1260
   ctcgaggtat aaccgtcggc agaagaaaag ggttccacct ctccccaacc ggagtttttg 1320
   gcaaggagtc cccaatgcaa agacacagcg ctgcggttgg cacctccttc ctcactcctt 1380
   caaaattgtt aagaaatgtt agtggtgggt ctgatctgac tgcagccatc ggtaaataaa 1440
   agtttttgat cctgttgaac ccgcctgaga cggtgctgtg caggggaaag cccccgcacc 1500
   cacacaggaa ttctgctgag gtcccccctc cttccggcca atggcagaag tgggggaaaa 1560
   tttttagaag aaaagcaaac atgtgagacc aatcattatc aaatactttt attttttggt 1620
   tgagtattta tctttttatt ttttattttt tttttgaaag aatgtcttgg aatgcgcaag 1680
   tctcccttta gagccgtctt ttgcagggag cgggaagtga caagagctca gatctccctc 1740
   ccgatctccc tccccacctc cgaagtctcc tccgtggacc acaggtggat ctttgtgcga 1800
   acaacttgca tttcggaagc cactgtccgt ctttaaacag aaagtcaaag gagccacgaa 1860
   gcaagcggcc gtccgggcgt ccgcctccgt ccccttccat gttcctcctc ttccttcgct 1920
   tcagcctctt ctgttatgtt ttgtcttgaa ttttatttag actttttcag tgggtatttt 1980
   tctgtctccc aacctctact gtaaactttc tggtccgaga acgagccgaa cacagcgcga 2040
   cgcagggact aggacggccc ggtgaccgcg cggattcagg attgcgggga cgcagaaagg 2100
   ttaaggcact tttaaaaact atagcaaggc tcctgtttat ttattctact ttctttccct 2160
   aataatcaaa acaccgcgta ggctcctccg tttatcagta ttaatggtgt aactttgttg 2220
   gcaatatttg ccgtgtagaa ttttttttag atatccattg taaatttgaa acaaagaccg 2280
   atctgtgtaa aaacaaattt ccatatgttt tatataaata tatatataat atgaaggact 2340
   accctccttt tttttttttg tattttggct gctagagtgc agcatttgtg acacgtattt 2400
   gaaatttgaa atttccttct gcactgtata aaaggaccat ttgaggatgt tttgcctttt 2460
   gtgtattttt tcctaaaaaa agaacaaaaa taaaaatgta taacatttgt acatggcctt 2520
   taaaattgta tcaactagaa ataaaattgc atgagtattt taaaaaaaaa aaaaaaaaa 2579
<210> 280
   <211> 986
   <212> DNA
   <213> Homo sapiens
<400> 280
   tgggaaagag ggaaaggctt ccccggccag ctgcgcggcg actccgggga ctccagggcg 60
   cccctctgcg gccgacgccc ggggtgcagc ggccgccggg gctggggccg gcgggagtcc 120
   gcgggaccct ccagaagagc ggccggcgcc gtgactcagc actggggcgg agcggggcgg 180
   gaccaccctt ataaggctcg gaggccgcga ggccttcgct ggagtttcgc cgccgcagtc 240
   ttcgccacca tgccgcccta caccgtggtc tatttcccag ttcgaggccg ctgcgcggcc 300
   ctgcgcatgc tgctggcaga tcagggccag agctggaagg aggaggtggt gaccgtggag 360
   acgtggcagg agggctcact caaagcctcc tgcctatacg ggcagctccc caagttccag 420
   gacggagacc tcaccctgta ccagtccaat accatcctgc gtcacctggg ccgcaccctt 480
   gggctctatg ggaaggacca gcaggaggca gccctggtgg acatggtgaa tgacggcgtg 540
   gaggacctcc gctgcaaata catctccctc atctacacca actatgaggc gggcaaggat 600
   gactatgtga aggcactgcc cgggcaactg aagccttttg agaccctgct gtcccagaac 660
   cagggaggca agaccttcat tgtgggagac cagatctcct tcgctgacta caacctgctg 720
   gacttgctgc tgatccatga ggtcctagcc cctggctgcc tggatgcgtt ccccctgctc 780
   tcagcatatg tggggcgcct cagtgcccgg cccaagctca aggccttcct ggcctcccct 840
   gagtacgtga acctccccat caatggcaac gggaaacagt gagggttggg gggactctga 900
   gcgggaggca gagtttgcct tcctttctcc aggaccaata aaatttctaa gagagctaaa 960
   aaaaaaaaaa aaaaaaaaaa aaaaaa 986
<210> 281
   <211> 6120
   <212> DNA
   <213> Homo sapiens
<400> 281
   cgccaggtcg cgcacagcgc cccgagccca ggcgcctccc cgcccccctc ccgcgctccg 60
   cggcggcggc ggcggcggca gcagtagcag caatatggct gttgatgggt gtttggggtg 120
   gcgctggcgg cgggaggagc tcccccgagc ccctgcgccg gctgcccgtt gctagctatg 180
   gcaaatggtg gcggcggcgg cggcggcagc agcggcggcg gcggcggcgg cggaggcagc 240
   agtcttagaa tgagtagcaa tatccacgcg aaccatctca gcctagacgc gtcctcctcc 300
   tcctcctcct cctcttcctc ttcttcttct tcctcctcct cttcctcctc gtcctcggtc 360
   cacgagccca agatggatgc gctcatcatc ccggtgacca tggaggtgcc gtgcgacagc 420
   cggggccaac gcatgtggtg ggctttcctg gcctcctcca tggtgacttt cttcgggggc 480
   ctcttcatca tcttgctctg gcggacgctc aagtacctgt ggaccgtgtg ctgccactgc 540
   gggggcaaga cgaaggaggc ccagaagatt aacaatggct caagccaggc ggatggcact 600
   ctcaaaccag tggatgaaaa agaggaggca gtggccgccg aggtcggctg gatgacctcc 660
   gtgaaggact gggcgggggt gatgatatcc gcccagacac tgactggcag agtcctggtt 720
   gtcttagtct ttgctctcag catcggtgca cttgtaatat acttcataga ttcatcaaac 780
   ccaatagaat cctgccagaa tttctacaaa gatttcacat tacagatcga catggctttc 840
   aacgtgttct tccttctcta cttcggcttg cggtttattg cagccaacga taaattgtgg 900
   ttctggctgg aagtgaactc tgtagtggat ttcttcacgg tgccccccgt gtttgtgtct 960
   gtgtacttaa acagaagttg gcttggtttg agatttttaa gagctctgag actgatacag 1020
   ttttcagaaa ttttgcagtt tctgaatatt cttaaaacaa gtaattccat caagctggtg 1080
   aatctgctct ccatatttat cagcacgtgg ctgactgcag ccgggttcat ccatttggtg 1140
   gagaattcag gggacccatg ggaaaatttc caaaacaacc aggctctcac ctactgggaa 1200
   tgtgtctatt tactcatggt cacaatgtcc accgttggtt atggggatgt ttatgcaaaa 1260
   accacacttg ggcgcctctt catggtcttc ttcatcctcg ggggactggc catgtttgcc 1320
   agctacgtcc ctgaaatcat agagttaata ggaaaccgca agaaatacgg gggctcctat 1380
   agtgcggtta gtggaagaaa gcacattgtg gtctgcggac acatcactct ggagagtgtt 1440
   tccaacttcc tgaaggactt tctgcacaag gaccgggatg acgtcaatgt ggagatcgtt 1500
   tttcttcaca acatctcccc caacctggag cttgaagctc tgttcaaacg acattttact 1560
   caggtggaat tttatcaggg ttccgtcctc aatccacatg atcttgcaag agtcaagata 1620
   gagtcagcag atgcatgcct gatccttgcc aacaagtact gcgctgaccc ggatgcggag 1680
   gatgcctcga atatcatgag agtaatctcc ataaagaact accatccgaa gataagaatc 1740
   atcactcaaa tgctgcagta tcacaacaag gcccatctgc taaacatccc gagctggaat 1800
   tggaaagaag gtgatgacgc aatctgcctc gcagagttga agttgggctt catagcccag 1860
   agctgcctgg ctcaaggcct ctccaccatg cttgccaacc tcttctccat gaggtcattc 1920
   ataaagattg aggaagacac atggcagaaa tactacttgg aaggagtctc aaatgaaatg 1980
   tacacagaat atctctccag tgccttcgtg ggtctgtcct tccctactgt ttgtgagctg 2040
   tgttttgtga agctcaagct cctaatgata gccattgagt acaagtctgc caaccgagag 2100
   agccgtatat taattaatcc tggaaaccat cttaagatcc aagaaggtac tttaggattt 2160
   ttcatcgcaa gtgatgccaa agaagttaaa agggcatttt tttactgcaa ggcctgtcat 2220
   gatgacatca cagatcccaa aagaataaaa aaatgtggct gcaaacggct tgaagatgag 2280
   cagccgtcaa cactatcacc aaaaaaaaag caacggaatg gaggcatgcg gaactcaccc 2340
   aacacctcgc ctaagctgat gaggcatgac cccttgttaa ttcctggcaa tgatcagatt 2400
   gacaacatgg actccaatgt gaagaagtac gactctactg ggatgtttca ctggtgtgca 2460
   cccaaggaga tagagaaagt catcctgact cgaagtgaag ctgccatgac cgtcctgagt 2520
   ggccatgtcg tggtctgcat ctttggcgac gtcagctcag ccctgatcgg cctccggaac 2580
   ctggtgatgc cgctccgtgc cagcaacttt cattaccatg agctcaagca cattgtgttt 2640
   gtgggctcta ttgagtacct caagcgggaa tgggagacgc ttcataactt ccccaaagtg 2700
   tccatattgc ctggtacgcc attaagtcgg gctgatttaa gggctgtcaa catcaacctc 2760
   tgtgacatgt gcgttatcct gtcagccaat cagaataata ttgatgatac ttcgctgcag 2820
   gacaaggaat gcatcttggc gtcactcaac atcaaatcta tgcagtttga tgacagcatc 2880
   ggagtcttgc aggctaattc ccaagggttc acacctccag gaatggatag atcctctcca 2940
   gataacagcc cagtgcacgg gatgttacgt caaccatcca tcacaactgg ggtcaacatc 3000
   cccatcatca ctgaactagt gaacgatact aatgttcagt ttttggacca agacgatgat 3060
   gatgaccctg atacagaact gtacctcacg cagccctttg cctgtgggac agcatttgcc 3120
   gtcagtgtcc tggactcact catgagcgcg acgtacttca atgacaatat cctcaccctg 3180
   atacggaccc tggtgaccgg aggagccacg ccggagctgg aggctctgat tgctgaggaa 3240
   aacgccctta gaggtggcta cagcaccccg cagacactgg ccaataggga ccgctgccgc 3300
   gtggcccagt tagctctgct cgatgggcca tttgcggact taggggatgg tggttgttat 3360
   ggtgatctgt tctgcaaagc tctgaaaaca tataatatgc tttgttttgg aatttaccgg 3420
   ctgagagatg ctcacctcag cacccccagt cagtgcacaa agaggtatgt catcaccaac 3480
   ccgccctatg agtttgagct cgtgccgacg gacctgatct tctgcttaat gcagtttgac 3540
   cacaatgccg gccagtcccg ggccagcctg tcccattcct cccactcgtc gcagtcctcc 3600
   agcaagaaga gctcctctgt tcactccatc ccatccacag caaaccgaca gaaccggccc 3660
   aagtccaggg agtcccggga caaacagaag tacgtgcagg aagagcggct ttgatatgtg 3720
   tatccaccgc cactgtgtga aactgtatct gccactcatt tccccagttg gtgtttccaa 3780
   caaagtaact ttccctgttt tcccctgtag tccccccctt tttttttaca catatttgca 3840
   tatgtatgat agtgtgcatg tggttgtcat ttttatttca ccaccataaa acccttgagc 3900
   acaacagcaa ataagcagac ggaccaaaag ttatttatga ttctggggga aaaataaccc 3960
   aaaggcatgc tccagacata aatagctcac tgcaggaacg agttcacaga ttagaaggga 4020
   gcacttgtga tcaacgtcag ttaggcagag caagtttatt taatgtaaaa gaaaagttga     4080
   ttctgattta tcaggattat cagggtgctt tgggttttga ttttgttgtt gttgttgttt     4140
   tcctttcttt ctttttttat acacacaata agttagcaca tgtttatttg aaacaagcaa     4200
   ccaaacagca atgaaaacat attgattgtt tccagtctct gggccgaagt attgcgaagc     4260
   atttgaaaag ctttcacgat ttgtgtagat gattatgaag gacctgcttg ttgcaagaga     4320
   acatcagtga tttttttagt tactcaccaa ggccttttgt cccagagcca gttccctctg     4380
   ggagttctta tgaacatttc tcaccttaat atggaggaga gaatagtatt ccaatcatgg     4440
   atgtatcaaa ttctagtcat ttagtttaag tgaaaagagg tttgattgca tattaaattg     4500
   ttattctgtc tccttatgtt gccatatgaa tagctatttt ttttctttca cttttgacat     4560
   ttgggatgaa aagccatatg tatcataaat atcagatgta agtcattaaa aactgccttc     4620
   ctgggacttt tacatctttt aaaaggtgaa ttacttacct tatgtacaga ataaataatg     4680
   ctcaggaaag agcaagtatt tttccatgca ttctcagggg atctttttac tcccctttgt     4740
   ttgattagtt agggccccaa tgccaggtag gaggaagggc tggggcaatg gtagagtgag     4800
   aggaagacaa acccagctgc agatcatgct tttctaggag ccgacatgct aaataaatta     4860
   gaatgtagga ggatcagcca cagttgactc aacaaagaca aaagccagcc accaccttca     4920
   actgttggca cagctgtgcg gtgctggctg tcccaatgca gaaagctggt gggaaggaat     4980
   tcctcatcat cactttcttt aatgtagcca atttaggcag ggtaatgacg gcaatagaga     5040
   gctgctcctt gtcattatga gacgtgggat aagaagagtg caacagtgag ccaaacacat     5100
   tttggtatag ttattttttt cttcttttgt tttctttctt ttttaacact tagtaagcat     5160
   gagaggagag gtagaaaaat accctttttt caacatatag ttgtcagatg ctttgtgcat     5220
   gcaaatcatg ctttaggcag tgcggtattt cttaaaaact ggccaattca ccataaccaa     5280
   tttcccttat ggatggacta ggctggtata tacatatttg aaaagtttta cttcaaagaa     5340
   ttccatcgaa tagaataggg gtaaaaggga ggaggaaaac atgtcacagc tgtaccatct     5400
   ctaaaaaggt gtttttatgg tgaatgtttt ggatttagat tttggatccc ccgtcccctc     5460
   aagcatgata gttttggata tttgcttgct gtgtgaattg acaagcactt ttactgacaa     5520
   atggtgaggc tcagtcagaa cctccaccct cccccacacc aaagacaggg gcagcgtagt     5580
   attcaaacca gtattgtggt ggggaataat tgtatacatg taaattatca agccctatga     5640
   gtggaagaat tttttcaaat tatttttgtc cctctatata ttgatttata ttatgtataa     5700
   ctatctcttt atataaacta tatataatta tatatatata actatataat tatatatata     5760
   taactatata tataactata tatatgtatc ccctagtatt ggatcatgaa gagctcttca     5820
   tgcattcttt gcaaaggagg ttataaagtt acgccctcag aacatttata actataagaa     5880
   tgtgccagtt aaagtgctca acaggaaata tgacagttta aaagcattgt aaaactcaca     5940
   tagcttactt ctctctctaa agtgcaacaa ggatgaatag aatgggccaa ggtatgacaa     6000
   ttaatggttc tgcatgacct agccactgct gggggttttc ttctataacg ttgtccttgt     6060
   gaaaactttt gtgaaattaa aaaaaaagga gttacaaatt ttaaaaaaaa aaaaaaaaaa     6120
<210> 282
   <211> 8220
   <212> DNA
   <213> Homo sapiens
<400> 282
   gaaacttcgg cgtgaagtgc agctccgctc cggctccctc tagctttctt tccttctctg       60
   gaatccgagg cgcggatctt cctcgcccca ccgccctagt tttttcggga gctcgccggt      120
   gccctctagg gtgtcggctc gtgctgggaa gtgccctcca tcctggtaat ggggggcggc      180
   gaggcaccgt aggagtggcg aggcggcgcc cagggtggca ctgccccgga acggggcgct      240
   gggtgcgcgc gggagggtcc ccgcgcgggc tccgccgctg ccgcagctgc gagcgcgccg      300
   cgccaccgag cctcctgcag caatggctcg tccgtgaaac gcgagccacg gctgctcttt      360
   ttaagagtgc ctgcatcctc cgtttgcgct tcgcaactgt cctgggtgaa aatggctgtc      420
   tagactaaaa tgtggcagaa gggaccaagc agtggatatt gagcctgtga agtccaactc      480
   ttaagctccg agacctgggg gactgagagc ccagctctga aaagtgcatc atgaattccg      540
   gagttgccat gaaatatgga aacgactcct cggccgagct gagtgagctc cattcagcag      600
   ccctggcatc actaaaggga gatatagtgg aacttaataa acgtctccag caaacagaga      660
   gggaacggga ccttctggaa aagaaattgg ccaaggcaca gtgcgagcag tcccacctca      720
   tgagagagca tgaggatgtc caggagcgaa cgacacttcg ctatgaggaa cgcatcacag      780
   agctccacag cgtcattgcg gagctcaaca agaagataga ccgtctgcaa ggcaccacca      840
   tcagggagga agatgagtac tcagaactgc gatcagaact cagccagagc caacacgagg      900
   tcaacgagga ctctcgaagc atggaccaag accagacctc tgtctctatc cccgaaaacc      960
   agtctaccat ggttactgct gacatggaca actgcagtga cctgaactca gaactgcaga     1020
   gggtgctgac agggctggag aatgttgtct gcggcaggaa gaagagcagc tgcagcctct     1080
   ccgtggccga ggtggacagg cacattgagc agctcaccac agccagcgag cactgtgacc     1140
   tggctattaa gacagtcgag gagattgagg gggtgcttgg ccgggacctg tatcccaacc     1200
   tggctgaaga gaggtctcgg tgggagaagg agctggctgg gctgagggaa gagaatgaga     1260
   gcctgactgc catgctgtgc agcaaagagg aagaactgaa ccggactaag gccaccatga     1320
   atgccatccg ggaagagcgg gaccggctcc ggaggagggt cagagagctt caaactcgac     1380
   tacagagcgt gcaggccaca ggtccctcca gccctggccg cctcacttcc accaaccgcc 1440
   cgattaaccc cagcactggg gagctgagca caagcagcag cagcaatgac attcccatcg 1500
   ccaagattgc tgagagggtg aagctatcaa agacaaggtc cgaatcgtca tcatctgatc 1560
   ggccagtcct gggctcagaa atcagtagca taggggtatc cagcagtgtg gctgaacacc 1620
   tggcccactc acttcaggac tgctccaata tccaagagat tttccaaaca ctctactcac 1680
   acggatctgc catctcagaa agcaagatta gagagtttga ggtggaaaca gaacggctga 1740
   atagccggat tgagcacctc aaatcccaaa atgacctcct gaccataacc ttggaggaat 1800
   gtaaaagcaa tgccgagagg atgagcatgc tggtgggaaa atacgaatcc aatgccacag 1860
   cgctgaggct ggccttgcag tacagcgagc agtgcatcga agcctacgaa ctcctcctgg 1920
   cgctggcaga gagtgagcag agcctcatcc tggggcagtt ccgagcggcg ggcgtggggt 1980
   cctcccctgg agaccagtcg gggatgaaaa acatcactca gatgctcaag cgagctcatg 2040
   actgccggaa gacagctgag aacgctgcca aggccctgct catgaagctg gacggcagct 2100
   gtgggggagc ctttgccgtg gccggctgca gcgtgcagcc ctgggagagc ctttcctcca 2160
   acagccacac cagcacaacc agctccacag ccagtagttg cgacaccgag ttcactaaag 2220
   aagacgagca gaggctgaag gattatatcc agcagctcaa gaatgacagg gctgcggtca 2280
   agctgaccat gctggagctg gaaagcatcc acatcgatcc tctcagctat gacgtcaagc 2340
   ctcggggaga cagccagagg ctggatctgg aaaacgcagt gcttatgcag gagctcatgg 2400
   ccatgaagga ggagatggcc gagttgaagg cccagctcta cctactggag aaagagaaga 2460
   aggccctgga gctgaagctg agcacgcggg aggcccagga gcaggcctac ctggtgcaca 2520
   ttgagcacct gaagtccgag gtggaggagc agaaggagca gcggatgcga tccctcagct 2580
   ccaccagcag cggcagcaaa gacaaacctg gcaaggagtg tgctgatgct gcctccccag 2640
   ctctgtccct agccgaactc aggacaacgt gcagcgagaa tgagctggct gcggagttca 2700
   ccaacgccat tcgtcgagaa aagaagttga aggccagagt tcaagagctg gtgagtgcct 2760
   tggagagact caccaagagc agtgaaatcc gacatcagca atctgcagag ttcgtgaatg 2820
   atctaaagcg ggccaacagc aacctggtgg ctgcctatga gaaagcaaag aaaaagcatc 2880
   aaaacaaact gaagaagtta gagtcgcaga tgatggccat ggtggagaga catgagaccc 2940
   aagtgaggat gctcaagcaa agaatagctc tgctagagga ggagaactcc aggccacaca 3000
   ccaatgaaac ttcgctttaa tcagcactca cgcaccggag ttctgcccat gggaagtaaa 3060
   ctgcagcagg ccactgggga cagaagggcc catgtacttg ttgggaggag gaggaaaggg 3120
   aaggctggca ggtaggtcgg cacttggaca atggagtgcc ccaactcaac ccttggggcg 3180
   actggccatg gtgacattgt ggactgtatc cagaggtgcc cgctcttccc tcctgggccc 3240
   acaacagcgt gtaaacacat gttctgtgcc tgctcagcag agcctcgttt ctgctttcag 3300
   cactcactct ccccctcctc ttctggtctg gcggctgtgc atcagtggga tcccagacat 3360
   ttgtttctgt aagattttcc attgtatcct ctttttggta gatgctgggc tcatcttcta 3420
   gaatctcgtt tctcctcttt cctcctgctt catgggaaaa cagacctgtg tgtgcctcca 3480
   gcatttaaaa ggactgctga tttgtttact acagcaaggc tttggtttcc aagtcccggg 3540
   tctcaacttt aagatagagg cggccataag aggtgatctc tgggagttat aggtcatggg 3600
   aagagcgtag acaggtgtta cttacagtcc cagatacact aaagttacaa acagaccacc 3660
   accaggactg tgcctgaaca attttgtatt gagagaataa aaacttcctt caatcttcat 3720
   tttggaggca gggctgggaa gggagcgctc tcttgattct gggatttctc cctctcagtg 3780
   gagccttatt aatatccaag acttagagct gggaatcttt ttgatacctg tagtggaact 3840
   aaaattctgt caggggtttc ttcaagagct gagaaacatt attagcactt cccgccccag 3900
   ggcactacat aattgctgtt ctgctgaatc aaatctcttc cacatgggtg catttgtagc 3960
   tctggacctg tctctaccta aggacaagac actgaggaga tactgaacat tttgcaaaac 4020
   ttatcacgcc tacttaagag tgctgtgtaa cccccagttc aagacttagc tcctgttgtc 4080
   atgacgggga cagagtgagg gaatggtagt taaggcttct tttttgcccc cagatacatg 4140
   gtgatggtta gcatatggtg cttaaaaggt taaatttcaa gcaaaatgct tacagggcta 4200
   ggcagtacca aagtaactga attatttcag gaaggtcttc aatcttaaaa caaattcatt 4260
   attctttttc agttttacct cttctctctc agttctacac tgatacactt gaaggaccat 4320
   ttactgtttt tttctgtagc accagagaat ccatccaaag ttccctatga aaaatgtgtt 4380
   ccattgccat agctgactac aaattaaagt tgaggaggtt tctgcataga gtctttatgt 4440
   ccataagcta cgggtaggtc tattttcaga gcatgataca aattccacag ccttctgttc 4500
   gctggaggat actccagcca ccagttcgga gggcagacag ctgtatccta aaagcaacca 4560
   ctgagaggcc agcagtgagg ctgcccatct ccaaaacgaa acaacagatc aactggtatc 4620
   agtctagaag gaccagcttt ggggtgcttt gatcactatg aaaatgttca ctggtttctg 4680
   tcaatcattt ggtcaaagtt agttcctgtg attttttttt tttttttttg agatggagtt 4740
   ttgctcgtca ccaggctgaa gtgcagtggc gtgatcttgg ctcactgcaa cctctgcctc 4800
   ctgagtagct ggaattacag gtgcccgcca ccatacccag ctagtttttt tgtattttta 4860
   gtagagacag ggtttcacca tgttggccag gctggtctcg aactcctgac cactgatcca 4920
   cccacctcgg cctcccaaag tgctgggatt acaggcatga gccaatgtgc ctggccattc 4980
   ctgtgatttt ttagaatttt tcacttttca tataaagttg cattttatca tgaggaaaat 5040
   gttttggctg aaaaacatat gccccaaaaa aaaaaaaatc agttttcact atttttccag 5100
   cttgcctagg gaaacgtcct cccctgagca aagtgggatg tgcatggctc ttttttgtga 5160
   ttaagcaggc atcaaatggg cagttttcat tttcactgac acagaaacat gtggctgaag 5220
   caggaataaa atcctgccta ctgacttctg tctccattct tgctccaggt aaaatcagag 5280
   aacctcccct tctctgagaa ttcccccagg cagaggcggc tccagctcct gttaagggcc 5340
   ccagttttac tcatgctacc tgaccttagt tttagtactt tcaaatgtga tttgggtaaa 5400
   aactattttg caggcaaaag tgttaatttt ttataaatat gtataataaa aaagctataa 5460
   aagtttttcc ttttcttgta tttttcaaat actccccaag acacaatcct tgaaggtgaa     5520
   ctaggtggca cttgattatg ctaattgtgg gagctactga actaaactat actgattaaa     5580
   ttaaatgctt gcaggttcct gggtaatttt cctttgtgaa taacctggcg ctacctgaat     5640
   ttttctctaa caaacatctc caatgagggt aaacaatgat cttgattcta atttatatga     5700
   ggttaaaaac cttcagattt cgtattttga attgttctcc ctagtttcct cagtggttac     5760
   ccccttttag tcagtaacca tctctgttca gaattcagtc tttggctttt gaagaaatgg     5820
   gcacagagcc aaaggaccca tttcacagtt cccttggaat atatttccca ttgttggact     5880
   gaggcaaaca aaaccaactg tgcaaatcac aagcaaaaga gacttctgtc cgtagttgcg     5940
   agtgccatca gtgtgagctt accagctaca aaaatctgtc atggactggg gaacagtatg     6000
   attccttatt gaaactttca tcctcatcgg ctggcttgtc agcgttctga agttaatatc     6060
   catgctgcct gaaacaaatg ttatgtttaa ctagcactta acttttaaaa ttgcttaatt     6120
   ctcctttcag atgcatgcac taagtcttct ctgagcctgc agcaatattt tagagggacc     6180
   tcttctaagt cagagccttg acattctact ccaaaaggaa gtccaaatta gagcccatag     6240
   gctttttctt aaaagctgtt aaggagtagt aaagaaagtt accactctaa ggaaaacatg     6300
   aaactatctg aaagtcttct ggttcctaac tggatacaga ttagattatt ttaaatgaga     6360
   aaaaagtgca ccgtagttac ccgatcaaga ttggactgcc tgtgtccctg gcatgaatta     6420
   aagaacctcc atagttgctt gttcccttct gaaggtgttt cctatggccc acgagaatta     6480
   taaagcaatc actttttaag tctatcagtc aaccttaata gctctcaaga gtttcgatta     6540
   tcaagcttta aagtgataac atatcaagat gactattaaa aaataaacat ctctgaaagt     6600
   gtcctttcct ccataaggct ttaaacaatt tgcaaattct taattgggag tatgtcccaa     6660
   tcattctatt aaactattta cagctctacc tacttctaga ttcagcaaac agagctacta     6720
   gttagagtat tttgagagcc aaaatagtaa gagacttctg taatggttga gatttctgca     6780
   ctgaccttaa ttagggtacc gccatagggc taccaaagtt tttagtgagg gctggggagg     6840
   tggagcataa gtaacatttt ttttctgcag aaggtgatgg ctttgtagaa cactataaaa     6900
   tttttcagga aaacatacag caaattttct tatccagtgg gaacagccta tttgacaagg     6960
   aatgatatac aagtttgatt cacctttgag cactcttata gagtttacag aagaggcgta     7020
   actggttaca aaacaaaagt tacgccttca ctatccagtt cttaaagttt atccacatcc     7080
   tacaaatctt atgttcatgt aggtcaggtt aaagtaagct ccaaacttgt ggccttcaga     7140
   agcatctcct gtttaacaca cctgaaatta ctctaactta atacatttgt attctctaag     7200
   aaataatgct gactgctgtc accacagcat cactgatctc tcagtggctg ttaaaatata     7260
   aacaagtgga aaaaaagtat acaagttgat agcagctgtt catgtattct tgttttgcta     7320
   atacctgtgt agagaaagac agtttcacat gcagatcacc atacattttt gcttaaaggt     7380
   tacactgggt aaactgtttg tttatcacat tacatctggg tgagattttg catgtgacca     7440
   agaggaagaa atcattcagt cccggaaata gcaactgcta tgtaaaacca catccttgac     7500
   tagtacttca tctttccaca gtcatccatc tgctaatcag gtgaaatatc agctcagcag     7560
   aacaaagcaa gttatacaca aatttccccc acattcacac tgatgccgta cttagtaatt     7620
   ctggttctac cattcccttt tgcacttttt gcaagtcaaa ttcatgtgga aaaatgttct     7680
   gtttgtagaa ctcatagtat tcacttatag tgctcatgtc aggaatggca agtgagtaag     7740
   agcccctgat aatctgcttt tgttagaacg taatgcaaag ataataaaac atgtccactt     7800
   cttttctagt tctaatcagt ttttagacat caagatttat ttactttgca tgagatttgt     7860
   ttcctattat ttgaatatgt gctatacact tgaattagaa aaacctgctt gaatgtattg     7920
   tcagttatta aagcacatgt gactaagttt gcatttgtca gtattgttct actgtttgta     7980
   ctgaaagtct cattttaaaa ttgctactta acactgtact ataccttgta attttttaga    8040
   ttctccactt gtattcttta aatgtttcca gatgccttta gttttagctg ctgtaaaata     8100
   gctactttgc gttatttgat atagaattgt tttttaaaaa ctccatttat ttatacagag     8160
   acatttataa tattttacaa acattcttat attctgaata aatatttcta attccacagt     8220
<210> 283
   <211> 6882
   <212> DNA
   <213> Homo sapiens
<400> 283
   gggagatttg gacgctccgg cctgggaggt gcgtcagatc cgagctcgcc atccagtttc      60
   ctctccacta gtccccccag ttggagatct gggaccaaca aggcaccatg gcgcagaagg     120
   gccaactcag tgacgatgag aagttcctct ttgtggacaa aaacttcatc aacagcccag     180
   tggcccaggc tgactgggcc gccaagagac tcgtctgggt cccctcggag aagcagggct     240
   tcgaggcagc cagcattaag gaggagaagg gggatgaggt ggttgtggag ctggtggaga     300
   atggcaagaa ggtcacggtt gggaaagatg acatccagaa gatgaaccca cccaagttct     360
   ccaaggtgga ggacatggcg gagctgacgt gcctcaacga agcctccgtg ctacacaacc     420
   tgagggagcg gtacttctca gggctaatat atacgtactc tggcctcttc tgcgtggtgg     480
   tcaaccccta taaacacctg cccatctact cggagaagat cgtcgacatg tacaagggca     540
   agaagaggca cgagatgccg cctcacatct acgccatcgc agacacggcc taccggagca     600
   tgcttcaaga tcgggaggac cagtccattc tatgcacagg cgagtctgga gccgggaaaa     660
   ccgaaaacac caagaaggtc attcagtacc tggccgtggt ggcctcctcc cacaagggca     720
   agaaagacac aagtatcacg ggagagctgg aaaagcagct tctacaagca aacccgattc 780
   tggaggcttt cggcaacgcc aaaacagtga agaacgacaa ctcctcacga ttcggcaaat 840
   tcatccgcat caacttcgac gtcacgggtt acatcgtggg agccaacatt gagacctatc 900
   tgctagaaaa atcacgggca attcgccaag ccagagacga gaggacattc cacatctttt 960
   actacatgat tgctggagcc aaggagaaga tgagaagtga cttgcttttg gagggcttca 1020
   acaactacac cttcctctcc aatggctttg tgcccatccc agcagcccag gatgatgaga 1080
   tgttccagga aaccgtggag gccatggcaa tcatgggttt cagcgaggag gagcagctat 1140
   ccatattgaa ggtggtatca tcggtcctgc agcttggaaa tatcgtcttc aagaaggaaa 1200
   gaaacacaga ccaggcgtcc atgccagata acacagctgc tcagaaagtt tgccacctca 1260
   tgggaattaa tgtgacagat ttcaccagat ccatcctcac tcctcgtatc aaggttggcc 1320
   gagatgtggt acagaaagct cagacaaaag aacaggctga ctttgctgta gaggctttgg 1380
   ccaaggcaac atatgagcgc cttttccgct ggatactcac ccgcgtgaac aaagccctgg 1440
   acaagaccca tcggcaaggg gcttccttcc tggggatcct ggatatagct ggatttgaga 1500
   tctttgaggt gaactccttc gagcagctgt gcatcaacta caccaacgag aagctgcagc 1560
   agctcttcaa ccacaccatg ttcatcctgg agcaggagga gtaccagcgc gagggcatcg 1620
   agtggaactt catcgacttt gggctggacc tacagccctg catcgagctc atcgagcgac 1680
   cgaacaaccc tccaggtgtg ctggccctgc tggacgagga atgctggttc cccaaagcca 1740
   cggacaagtc tttcgtggag aagctgtgca cggagcaggg cagccacccc aagttccaga 1800
   agcccaagca gctcaaggac aagactgagt tctccatcat ccattatgct gggaaggtgg 1860
   actataatgc gagtgcctgg ctgaccaaga atatggaccc gctgaatgac aacgtgactt 1920
   ccctgctcaa tgcctcctcc gacaagtttg tggccgacct gtggaaggac gtggaccgca 1980
   tcgtgggcct ggaccagatg gccaagatga cggagagctc gctgcccagc gcctccaaga 2040
   ccaagaaggg catgttccgc acagtggggc agctgtacaa ggagcagctg ggcaagctga 2100
   tgaccacgct acgcaacacc acgcccaact tcgtgcgctg catcatcccc aaccacgaga 2160
   agaggtccgg caagctggat gcgttcctgg tgctggagca gctgcggtgc aatggggtgc 2220
   tggaaggcat tcgcatctgc cggcagggct tccccaaccg gatcgtcttc caggagttcc 2280
   gccaacgcta cgagatcctg gcggcgaatg ccatccccaa aggcttcatg gacgggaagc 2340
   aggcctgcat tctcatgatc aaagccctgg aacttgaccc caacttatac aggatagggc 2400
   agagcaaaat cttcttccga actggcgtcc tggcccacct agaggaggag cgagatttga 2460
   agatcaccga tgtcatcatg gccttccagg cgatgtgtcg tggctacttg gccagaaagg 2520
   cttttgccaa gaggcagcag cagctgaccg ccatgaaggt gattcagagg aactgcgccg 2580
   cctacctcaa gctgcggaac tggcagtggt ggaggctttt caccaaagtg aagccactgc 2640
   tgcaggtgac acggcaggag gaggagatgc aggccaagga ggatgaactg cagaagacca 2700
   aggagcggca gcagaaggca gagaatgagc ttaaggagct ggaacagaag cactcgcagc 2760
   tgaccgagga gaagaacctg ctacaggaac agctgcaggc agagacagag ctgtatgcag 2820
   aggctgagga gatgcgggtg cggctggcgg ccaagaagca ggagctggag gagatactgc 2880
   atgagatgga ggcccgcctg gaggaggagg aagacagggg ccagcagcta caggctgaaa 2940
   ggaagaagat ggcccagcag atgctggacc ttgaagaaca gctggaggag gaggaagctg 3000
   ccaggcagaa gctgcaactt gagaaggtca cggctgaggc caagatcaag aaactggagg 3060
   atgagatcct ggtcatggat gatcagaaca ataaactatc aaaagaacga aaactccttg 3120
   aggagaggat tagtgactta acgacaaatc ttgcagaaga ggaagaaaag gccaagaatc 3180
   ttaccaagct gaaaaacaag catgaatcta tgatttcaga actggaagtg cggctaaaga 3240
   aggaagagaa gagccgacag gagctggaga agctgaaacg gaagctggag ggtgatgcca 3300
   gcgacttcca cgagcagatc gctgacctcc aggcgcagat cgcagagctc aagatgcagc 3360
   tggccaagaa ggaggaggag ctgcaggcgg ccctggccag gcttgacgat gaaatcgctc 3420
   agaagaacaa tgccctgaag aagatccggg agctggaggg ccacatctca gacctccagg 3480
   aggacctgga ctcagagcgg gccgccagga acaaggctga aaagcagaag cgagacctcg 3540
   gcgaggagct ggaggcccta aagacagagc tggaagacac actggacagc acagccactc 3600
   agcaggagct cagggccaag agggagcagg aggtgacggt gctgaagaag gccctggatg 3660
   aagagacgcg gtcccatgag gctcaggtcc aggagatgag gcagaaacac gcacaggcgg 3720
   tggaggagct cacagagcag cttgagcagt tcaagagggc caaggcgaac ctagacaaga 3780
   ataagcagac gctggagaaa gagaacgcag acctggccgg ggagctgcgg gtcctgggcc 3840
   aggccaagca ggaggtggaa cataagaaga agaagctgga ggcgcaggtg caggagctgc 3900
   agtccaagtg cagcgatggg gagcgggccc gggcggagct caatgacaaa gtccacaagc 3960
   tgcagaatga agttgagagc gtcacaggga tgcttaacga ggccgagggg aaggccatta 4020
   agctggccaa ggacgtggcg tccctcagtt cccagctcca ggacacccag gagctgcttc 4080
   aagaagaaac ccggcagaag ctcaacgtgt ctacgaagct gcgccagctg gaggaggagc 4140
   ggaacagcct gcaagaccag ctggacgagg agatggaggc caagcagaac ctggagcgcc 4200
   acatctccac tctcaacatc cagctctccg actcgaagaa gaagctgcag gactttgcca 4260
   gcaccgtgga agctctggaa gaggggaaga agaggttcca gaaggagatc gagaacctca 4320
   cccagcagta cgaggagaag gcggccgctt atgataaact ggaaaagacc aagaacaggc 4380
   ttcagcagga gctggacgac ctggttgttg atttggacaa ccagcggcaa ctcgtgtcca 4440
   acctggaaaa gaagcagagg aaatttgatc agttgttagc cgaggagaaa aacatctctt 4500
   ccaaatacgc ggatgagagg gacagagctg aggcagaagc cagggagaag gaaaccaagg 4560
   ccctgtccct ggctcgggcc cttgaagagg ccttggaagc caaagaggaa ctcgagcgga 4620
   ccaacaaaat gctcaaagcc gaaatggaag acctggtcag ctccaaggat gacgtgggca 4680
   agaacgtcca tgagctggag aagtccaagc gggccctgga gacccagatg gaggagatga 4740
   agacgcagct ggaagagctg gaggacgagc tgcaagccac ggaggacgcc aaactgcggc 4800
   tggaagtcaa catgcaggcg ctcaagggcc agttcgaaag ggatctccaa gcccgggacg    4860
   agcagaatga ggagaagagg aggcaactgc agagacagct tcacgagtat gagacggaac    4920
   tggaagacga gcgaaagcaa cgtgccctgg cagctgcagc aaagaagaag ctggaagggg    4980
   acctgaaaga cctggagctt caggccgact ctgccatcaa ggggagggag gaagccatca    5040
   agcagctacg caaactgcag gctcagatga aggactttca aagagagctg gaagatgccc    5100
   gtgcctccag agatgagatc tttgccacag ccaaagagaa tgagaagaaa gccaagagct    5160
   tggaagcaga cctcatgcag ctacaagagg acctcgccgc cgctgagagg gctcgcaaac    5220
   aagcggacct cgagaaggag gaactggcag aggagctggc cagtagcctg tcgggaagga    5280
   acgcactcca ggacgagaag cgccgcctgg aggcccggat cgcccagctg gaggaggagc    5340
   tggaggagga gcagggcaac atggaggcca tgagcgaccg ggtccgcaaa gccacacagc    5400
   aggccgagca gctcagcaac gagctggcca cagagcgcag cacggcccag aagaatgaga    5460
   gtgcccggca gcagctcgag cggcagaaca aggagctccg gagcaagctc cacgagatgg    5520
   agggggccgt caagtccaag ttcaagtcca ccatcgcggc gctggaggcc aagattgcac    5580
   agctggagga gcaggtcgag caggaggcca gagagaaaca ggcggccacc aagtcgctga    5640
   agcagaaaga caagaagctg aaggaaatct tgctgcaggt ggaggacgag cgcaagatgg    5700
   ccgagcagta caaggagcag gcagagaaag gcaatgccag ggtcaagcag ctcaagaggc    5760
   agctggagga ggcagaggag gagtcccagc gcatcaacgc caaccgcagg aagctgcagc    5820
   gggagctgga tgaggccacg gagagcaacg aggccatggg ccgcgaggtg aacgcactca    5880
   agagcaagct caggcgagga aacgagacct ctttcgttcc ttctagaagg tctggaggac    5940
   gtagagttat tgaaaatgca gatggttctg aggaggaaac ggacactcga gacgcagact    6000
   tcaatggaac caaggccagt gaataagcaa ctttctacag ttttgcacca cggcaagaaa    6060
   accaaaaacc aaaacaaaca aacaaaaaaa acccaacaac aacccagaac aaagcaaaac    6120
   ccagcagact gtacttagca ttgtctaaat ccattctcaa attccaaata tcacagacac    6180
   ccctcacaca aggaatataa aaaccaccac cctccagcct gggcaacgta gtaaaacctc    6240
   atctatacaa gaatttaaaa ataagctggg cgtggtggta cacacctgtg gtcccagcta    6300
   ctagggaggc tgagccagga agaacgctcc agcccaggac ttcgaggctg caatgagcta    6360
   taattgcatc attgcactcc agcctgggca acagagaccc tgtctcaacc accaccacca    6420
   ccaccacccc tactacccct gtattcaagg taaaaattga agtttgtatg atgtaagaga    6480
   tgagaaaaac ccaacaggaa acacagacac atcctccagt tctatcaatg gattgtgcag    6540
   acactgagtt tttagaaaaa catatccacg gtaaccggtc cctggcaatt ctgtttacat    6600
   gaaatgggga gaaagtcacc gaaatgggtg ccgccggccc ccactcccaa ttcattccct    6660
   aacctgcaaa cctttccaac ttctcacgtc aggcctttga gaattctttc cccctctcct    6720
   ggtttccaca cctcagacac gcacagttca ccaagtgcct tctgtagtca catgaattga    6780
   aaaggagacg ctgctcccac ggaggggagc aggaatgctg cactgtttac accctgactg    6840
   tgcttaaaaa cactttcact aataaatggt tataaatcac aa                       6882
<210> 284
   <211> 6921
   <212> DNA
   <213> Homo sapiens
<400> 284
   gggagatttg gacgctccgg cctgggaggt gcgtcagatc cgagctcgcc atccagtttc      60
   ctctccacta gtccccccag ttggagatct gggaccaaca aggcaccatg gcgcagaagg     120
   gccaactcag tgacgatgag aagttcctct ttgtggacaa aaacttcatc aacagcccag     180
   tggcccaggc tgactgggcc gccaagagac tcgtctgggt cccctcggag aagcagggct     240
   tcgaggcagc cagcattaag gaggagaagg gggatgaggt ggttgtggag ctggtggaga     300
   atggcaagaa ggtcacggtt gggaaagatg acatccagaa gatgaaccca cccaagttct     360
   ccaaggtgga ggacatggcg gagctgacgt gcctcaacga agcctccgtg ctacacaacc     420
   tgagggagcg gtacttctca gggctaatat atacgtactc tggcctcttc tgcgtggtgg     480
   tcaaccccta taaacacctg cccatctact cggagaagat cgtcgacatg tacaagggca     540
   agaagaggca cgagatgccg cctcacatct acgccatcgc agacacggcc taccggagca     600
   tgcttcaaga tcgggaggac cagtccattc tatgcacagg cgagtctgga gccgggaaaa     660
   ccgaaaacac caagaaggtc attcagtacc tggccgtggt ggcctcctcc cacaagggca     720
   agaaagacac aagtatcacg ggagagctgg aaaagcagct tctacaagca aacccgattc     780
   tggaggcttt cggcaacgcc aaaacagtga agaacgacaa ctcctcacga ttcggcaaat     840
   tcatccgcat caacttcgac gtcacgggtt acatcgtggg agccaacatt gagacctatc     900
   tgctagaaaa atcacgggca attcgccaag ccagagacga gaggacattc cacatctttt     960
   actacatgat tgctggagcc aaggagaaga tgagaagtga cttgcttttg gagggcttca    1020
   acaactacac cttcctctcc aatggctttg tgcccatccc agcagcccag gatgatgaga    1080
   tgttccagga aaccgtggag gccatggcaa tcatgggttt cagcgaggag gagcagctat    1140
   ccatattgaa ggtggtatca tcggtcctgc agcttggaaa tatcgtcttc aagaaggaaa    1200
   gaaacacaga ccaggcgtcc atgccagata acacagctgc tcagaaagtt tgccacctca    1260
   tgggaattaa tgtgacagat ttcaccagat ccatcctcac tcctcgtatc aaggttgggc    1320
   gagatgtggt acagaaagct cagacaaaag aacaggctga ctttgctgta gaggctttgg    1380
   ccaaggcaac atatgagcgc cttttccgct ggatactcac ccgcgtgaac aaagccctgg    1440
   acaagaccca tcggcaaggg gcttccttcc tggggatcct ggatatagct ggatttgaga 1500
   tctttgaggt gaactccttc gagcagctgt gcatcaacta caccaacgag aagctgcagc 1560
   agctcttcaa ccacaccatg ttcatcctgg agcaggagga gtaccagcgc gagggcatcg 1620
   agtggaactt catcgacttt gggctggacc tacagccctg catcgagctc atcgagcgac 1680
   cgaacaaccc tccaggtgtg ctggccctgc tggacgagga atgctggttc cccaaagcca 1740
   cggacaagtc tttcgtggag aagctgtgca cggagcaggg cagccacccc aagttccaga 1800
   agcccaagca gctcaaggac aagactgagt tctccatcat ccattatgct gggaaggtgg 1860
   actataatgc gagtgcctgg ctgaccaaga atatggaccc gctgaatgac aacgtgactt 1920
   ccctgctcaa tgcctcctcc gacaagtttg tggccgacct gtggaaggac gtggaccgca 1980
   tcgtgggcct ggaccagatg gccaagatga cggagagctc gctgcccagc gcctccaaga 2040
   ccaagaaggg catgttccgc acagtggggc agctgtacaa ggagcagctg ggcaagctga 2100
   tgaccacgct acgcaacacc acgcccaact tcgtgcgctg catcatcccc aaccacgaga 2160
   agaggtccgg caagctggat gcgttcctgg tgctggagca gctgcggtgc aatggggtgc 2220
   tggaaggcat tcgcatctgc cggcagggct tccccaaccg gatcgtcttc caggagttcc 2280
   gccaacgcta cgagatcctg gcggcgaatg ccatccccaa aggcttcatg gacgggaagc 2340
   aggcctgcat tctcatgatc aaagccctgg aacttgaccc caacttatac aggatagggc 2400
   agagcaaaat cttcttccga actggcgtcc tggcccacct agaggaggag cgagatttga 2460
   agatcaccga tgtcatcatg gccttccagg cgatgtgtcg tggctacttg gccagaaagg 2520
   cttttgccaa gaggcagcag cagctgaccg ccatgaaggt gattcagagg aactgcgccg 2580
   cctacctcaa gctgcggaac tggcagtggt ggaggctttt caccaaagtg aagccactgc 2640
   tgcaggtgac acggcaggag gaggagatgc aggccaagga ggatgaactg cagaagacca 2700
   aggagcggca gcagaaggca gagaatgagc ttaaggagct ggaacagaag cactcgcagc 2760
   tgaccgagga gaagaacctg ctacaggaac agctgcaggc agagacagag ctgtatgcag 2820
   aggctgagga gatgcgggtg cggctggcgg ccaagaagca ggagctggag gagatactgc 2880
   atgagatgga ggcccgcctg gaggaggagg aagacagggg ccagcagcta caggctgaaa 2940
   ggaagaagat ggcccagcag atgctggacc ttgaagaaca gctggaggag gaggaagctg 3000
   ccaggcagaa gctgcaactt gagaaggtca cggctgaggc caagatcaag aaactggagg 3060
   atgagatcct ggtcatggat gatcagaaca ataaactatc aaaagaacga aaactccttg 3120
   aggagaggat tagtgactta acgacaaatc ttgcagaaga ggaagaaaag gccaagaatc 3180
   ttaccaagct gaaaaacaag catgaatcta tgatttcaga actggaagtg cggctaaaga 3240
   aggaagagaa gagccgacag gagctggaga agctgaaacg gaagctggag ggtgatgcca 3300
   gcgacttcca cgagcagatc gctgacctcc aggcgcagat cgcagagctc aagatgcagc 3360
   tggccaagaa ggaggaggag ctgcaggcgg ccctggccag gcttgacgat gaaatcgctc 3420
   agaagaacaa tgccctgaag aagatccggg agctggaggg ccacatctca gacctccagg 3480
   aggacctgga ctcagagcgg gccgccagga acaaggctga aaagcagaag cgagacctcg 3540
   gcgaggagct ggaggcccta aagacagagc tggaagacac actggacagc acagccactc 3600
   agcaggagct cagggccaag agggagcagg aggtgacggt gctgaagaag gccctggatg 3660
   aagagacgcg gtcccatgag gctcaggtcc aggagatgag gcagaaacac gcacaggcgg 3720
   tggaggagct cacagagcag cttgagcagt tcaagagggc caaggcgaac ctagacaaga 3780
   ataagcagac gctggagaaa gagaacgcag acctggccgg ggagctgcgg gtcctgggcc 3840
   aggccaagca ggaggtggaa cataagaaga agaagctgga ggcgcaggtg caggagctgc 3900
   agtccaagtg cagcgatggg gagcgggccc gggcggagct caatgacaaa gtccacaagc 3960
   tgcagaatga agttgagagc gtcacaggga tgcttaacga ggccgagggg aaggccatta 4020
   agctggccaa ggacgtggcg tccctcagtt cccagctcca ggacacccag gagctgcttc 4080
   aagaagaaac ccggcagaag ctcaacgtgt ctacgaagct gcgccagctg gaggaggagc 4140
   ggaacagcct gcaagaccag ctggacgagg agatggaggc caagcagaac ctggagcgcc 4200
   acatctccac tctcaacatc cagctctccg actcgaagaa gaagctgcag gactttgcca 4260
   gcaccgtgga agctctggaa gaggggaaga agaggttcca gaaggagatc gagaacctca 4320
   cccagcagta cgaggagaag gcggccgctt atgataaact ggaaaagacc aagaacaggc 4380
   ttcagcagga gctggacgac ctggttgttg atttggacaa ccagcggcaa ctcgtgtcca 4440
   acctggaaaa gaagcagagg aaatttgatc agttgttagc cgaggagaaa aacatctctt 4500
   ccaaatacgc ggatgagagg gacagagctg aggcagaagc cagggagaag gaaaccaagg 4560
   ccctgtccct ggctcgggcc cttgaagagg ccttggaagc caaagaggaa ctcgagcgga 4620
   ccaacaaaat gctcaaagcc gaaatggaag acctggtcag ctccaaggat gacgtgggca 4680
   agaacgtcca tgagctggag aagtccaagc gggccctgga gacccagatg gaggagatga 4740
   agacgcagct ggaagagctg gaggacgagc tgcaagccac ggaggacgcc aaactgcggc 4800
   tggaagtcaa catgcaggcg ctcaagggcc agttcgaaag ggatctccaa gcccgggacg 4860
   agcagaatga ggagaagagg aggcaactgc agagacagct tcacgagtat gagacggaac 4920
   tggaagacga gcgaaagcaa cgtgccctgg cagctgcagc aaagaagaag ctggaagggg 4980
   acctgaaaga cctggagctt caggccgact ctgccatcaa ggggagggag gaagccatca 5040
   agcagctacg caaactgcag gctcagatga aggactttca aagagagctg gaagatgccc 5100
   gtgcctccag agatgagatc tttgccacag ccaaagagaa tgagaagaaa gccaagagct 5160
   tggaagcaga cctcatgcag ctacaagagg acctcgccgc cgctgagagg gctcgcaaac 5220
   aagcggacct cgagaaggag gaactggcag aggagctggc cagtagcctg tcgggaagga 5280
   acgcactcca ggacgagaag cgccgcctgg aggcccggat cgcccagctg gaggaggagc 5340
   tggaggagga gcagggcaac atggaggcca tgagcgaccg ggtccgcaaa gccacacagc 5400
   aggccgagca gctcagcaac gagctggcca cagagcgcag cacggcccag aagaatgaga 5460
   gtgcccggca gcagctcgag cggcagaaca aggagctccg gagcaagctc cacgagatgg 5520
   agggggccgt caagtccaag ttcaagtcca ccatcgcggc gctggaggcc aagattgcac 5580
   agctggagga gcaggtcgag caggaggcca gagagaaaca ggcggccacc aagtcgctga 5640
   agcagaaaga caagaagctg aaggaaatct tgctgcaggt ggaggacgag cgcaagatgg 5700
   ccgagcagta caaggagcag gcagagaaag gcaatgccag ggtcaagcag ctcaagaggc 5760
   agctggagga ggcagaggag gagtcccagc gcatcaacgc caaccgcagg aagctgcagc 5820
   gggagctgga tgaggccacg gagagcaacg aggccatggg ccgcgaggtg aacgcactca 5880
   agagcaagct cagagggccc cccccacagg aaacttcgca gtgatgcacc aggcgaggaa 5940
   acgagacctc tttcgttcct tctagaaggt ctggaggacg tagagttatt gaaaatgcag 6000
   atggttctga ggaggaaacg gacactcgag acgcagactt caatggaacc aaggccagtg 6060
   aataagcaac tttctacagt tttgcaccac ggcaagaaaa ccaaaaacca aaacaaacaa 6120
   acaaaaaaaa cccaacaaca acccagaaca aagcaaaacc cagcagactg tacttagcat 6180
   tgtctaaatc cattctcaaa ttccaaatat cacagacacc cctcacacaa ggaatataaa 6240
   aaccaccacc ctccagcctg ggcaacgtag taaaacctca tctatacaag aatttaaaaa 6300
   taagctgggc gtggtggtac acacctgtgg tcccagctac tagggaggct gagccaggaa 6360
   gaacgctcca gcccaggact tcgaggctgc aatgagctat aattgcatca ttgcactcca 6420
   gcctgggcaa cagagaccct gtctcaacca ccaccaccac caccacccct actacccctg 6480
   tattcaaggt aaaaattgaa gtttgtatga tgtaagagat gagaaaaacc caacaggaaa 6540
   cacagacaca tcctccagtt ctatcaatgg attgtgcaga cactgagttt ttagaaaaac 6600
   atatccacgg taaccggtcc ctggcaattc tgtttacatg aaatggggag aaagtcaccg 6660
   aaatgggtgc cgccggcccc cactcccaat tcattcccta acctgcaaac ctttccaact 6720
   tctcacgtca ggcctttgag aattctttcc ccctctcctg gtttccacac ctcagacacg 6780
   cacagttcac caagtgcctt ctgtagtcac atgaattgaa aaggagacgc tgctcccacg 6840
   gaggggagca ggaatgctgc actgtttaca ccctgactgt gcttaaaaac actttcacta 6900
   ataaatggtt ataaatcaca a 6921
<210> 285
   <211> 566
   <212> DNA
   <213> Homo sapiens
<400> 285
   gccagaaccg gtggagcagc gacccctgag cagtgttctc tgtgctgagc ggcgggactg 60
   agctgttgag ttagagccaa catgagtgag cgacaaggtg ctggggcaac caatggaaaa 120
   gacaagacat ctggtgaaaa tgatggacag aagaaagttc aagaagaatt tgacattgac 180
   atggatgcac cagagacaga acgtgcagcg gtggccattc agtctcagtt cagaaaattc 240
   cagaagaaga aggctgggtc tcagtcctag tgggagaacc ccctcctagt ccacctgaaa 300
   acaccaaatt caaccatcat ctgtcaagaa attaaaagaa caacacccta gagagaagtc 360
   atccacacac aatccacaca cgcatagcaa acctccaatg catgtacaga aacctgtgat 420
   atttataccc ttgtaggaag gtatagacaa tggaattgtg agtagcttaa tctctatgtt 480
   tctctccatt ttcattcctc ctgcaactat tttccttgat gttgtaataa aatgaagtta 540
   cgatgagtga attcaaaaaa aaaaaa 566
<210> 286
   <211> 2090
   <212> DNA
   <213> Homo sapiens
<400> 286
   gccagcagct gcagctcggc ctctgctgcc tgccggtgct cttcgtggct ctgggcatgg     60
   cctcggaccc catcttcacg ctggcgcccc cgctgcattg ccactacggg gccttccccc    120
   ctaatgcctc tggctgggag cagcctccca atgccagcgg cgtcagcgtc gccagcgctg    180
   ccctagcagc cagcgccgcc agccgtgtcg ccaccagtac cgacccctcg tgcagcggct    240
   tcgccccgcc ggacttcaac cattgcctca aggattggga ctataatggc cttcctgtgc    300
   tcaccaccaa cgccatcggc cagtgggatc tggtgtgtga cctgggctgg caggtgatcc    360
   tggagcagat cctcttcatc ttgggctttg cctccggcta cctgttcctg ggttaccccg    420
   cagacagatt tggccgtcgc gggattgtgc tgctgacctt ggggctggtg ggcccctgtg    480
   gagtaggagg ggctgctgca ggctcctcca caggcgtcat ggccctccga ttcctcttgg    540
   gctttctgct tgccggtgtt gacctgggtg tctacctgat gcgcctggag ctgtgcgacc    600
   caacccagag gcttcgggtg gccctggcag gggagttggt gggggtggga gggcacttcc    660
   tgttcctggg cctggccctt gtctctaagg attggcgatt cctacagcga atgatcaccg    720
   ctccctgcat cctcttcctg ttttatggct ggcctggttt gttcctggag tccgcacggt    780
   ggctgatagt gaagcggcag attgaggagg ctcagtctgt gctgaggatc ctggctgagc    840
   gaaaccggcc ccatgggcag atgctggggg aggaggccca ggaggccctg caggacctgg    900
   agaatacctg ccctctccct gcaacatcct ccttttcctt tgcttccctc ctcaactacc    960
   gcaacatctg gaaaaatctg cttatcctgg gcttcaccaa cttcattgcc catgccattc   1020
   gccactgcta ccagcctgtg ggaggaggag ggagcccatc ggacttctac ctgtgctctc   1080
   tgctggccag cggcaccgca gccctggcct gtgtcttcct gggggtcacc gtggaccgat   1140
   ttggccgccg gggcatcctt cttctctcca tgacccttac cggcattgct tccctggtcc   1200
   tgctgggcct gtgggattgt gagcatccta tcttccccac agtgtgggct caacaaggga   1260
   accccaacag agatctgaac gaggctgcca tcaccacttt ctctgtcctt gggctcttct   1320
   cctcccaagc tgccgccatc ctcagcaccc tccttgctgc tgaggtcatc cccaccactg   1380
   tccggggccg tggcctgggc ctgatcatgg ctctaggggc gcttggagga ctgagcggcc   1440
   cggcccagcg cctccacatg ggccatggag ccttcctgca gcacgtggtg ctggcggcct   1500
   gcgccctcct ctgcattctc agcattatgc tgctgccgga gaccaagcgc aagctcctgc   1560
   ccgaggtgct ccgggacggg gagctgtgtc gccggccttc cctgctgcgg cagccacccc   1620
   ctacccgctg tgaccacgtc ccgctgcttg ccacccccaa ccctgccctc tgagcggcct   1680
   ctgagtaccc tggcgggagg ctggcccaca cagaaaggtg gcaagaagat cgggaagact   1740
   gagtagggaa ggcagggctg cccagaagtc tcagaggcac ctcacgccag ccatcgcgga   1800
   gagctcagag ggccgtcccc accctgcctc ctccctgctg ctttgcattc acttccttgg   1860
   ccagagtcag gggacaggga gagagctcca cactgtaacc actgggtctg ggctccatcc   1920
   tgcgcccaaa gacatccacc cagacctcat tatttcttgc tctatcattc tgtttcaata   1980
   aagacatttg gaataaacga gcaaaaaaaa aaaaaaaaaa aaaaaaaaaa aaaaaaaaaa   2040
   aaaaaaaaaa aaaaaaaaaa aaaaaaaaaa aaaaaaaaaa aaaaaaaaaa              2090
<210> 287
   <211> 2989
   <212> DNA
   <213> Homo sapiens
<400> 287
   ggggggagcg cgccgggcgc agggagctga gtggacggct cgagacggcg gcgcgtgcag 60
   cagctccaga aagcagcgag ttggcagagc agggctgcat ttccagcagg agctgcgagc 120
   acagtgctgg ctcacaacaa gatgctcaag gtgtcagccg tactgtgtgt gtgtgcagcc 180
   gcttggtgca gtcagtctct cgcagctgcc gcggcggtgg ctgcagccgg ggggcggtcg 240
   gacggcggta attttctgga tgataaacaa tggctcacca caatctctca gtatgacaag 300
   gaagtcggac agtggaacaa attccgagac gaagtagagg atgattattt ccgcacttgg 360
   agtccaggaa aacccttcga tcaggcttta gatccagcta aggatccatg cttaaagatg 420
   aaatgtagtc gccataaagt atgcattgct caagattctc agactgcagt ctgcattagt 480
   caccggaggc ttacacacag gatgaaagaa gcaggagtag accataggca gtggaggggt 540
   cccatattat ccacctgcaa gcagtgccca gtggtctatc ccagccctgt ttgtggttca 600
   gatggtcata cctactcttt tcagtgcaaa ctagaatatc aggcatgtgt cttaggaaaa 660
   cagatctcag tcaaatgtga aggacattgc ccatgtcctt cagataagcc caccagtaca 720
   agcagaaatg ttaagagagc atgcagtgac ctggagttca gggaagtggc aaacagattg 780
   cgggactggt tcaaggccct tcatgaaagt ggaagtcaaa acaagaagac aaaaacattg 840
   ctgaggcctg agagaagcag attcgatacc agcatcttgc caatttgcaa ggactcactt 900
   ggctggatgt ttaacagact tgatacaaac tatgacctgc tattggacca gtcagagctc 960
   agaagcattt accttgataa gaatgaacag tgtaccaagg cattcttcaa ttcttgtgac 1020
   acatacaagg acagtttaat atctaataat gagtggtgct actgcttcca gagacagcaa 1080
   gacccacctt gccagactga gctcagcaat attcagaagc ggcaaggggt aaagaagctc 1140
   ctaggacagt atatccccct gtgtgatgaa gatggttact acaagccaac acaatgtcat 1200
   ggcagtgttg gacagtgctg gtgtgttgac agatatggaa atgaagtcat gggatccaga 1260
   ataaatggtg ttgcagattg tgctatagat tttgagatct ccggagattt tgctagtggc 1320
   gattttcatg aatggactga tgatgaggat gatgaagacg atattatgaa tgatgaagat 1380
   gaaattgaag atgatgatga agatgaaggg gatgatgatg atggtggtga tgaccatgat 1440
   gtatacattt gattgatgac agttgaaatc aataaattct acatttctaa tatttacaaa 1500
   aatgatagcc tatttaaaat tatcttcttc cccaataaca aaatgattct aaacctcaca 1560
   tatattttgt ataattattt gaaaaattgc agctaaagtt atagaacttt atgtttaaat 1620
   aagaatcatt tgctttgagt ttttatattc cttacacaaa aagaaaatac atatgcagtc 1680
   tagtcagaca aaataaagtt ttgaagtgct actataataa gtttttcacg agaacaaact 1740
   ttgtaaatct tccataagca aaatgacagc tagtgcttgg gatcgtacat gttaattttc 1800
   tgaaagataa ttctaagtga aatttaaaat aaataaattt ttaatgacct gggtcttaag 1860
   gatttaggaa aaatatgcat gctttaattg catttccaaa gtagcatctt gctagaccta 1920
   gttgagtcag gataacagag agataccaca tggcaagaaa aacaaagtga caattgtaga 1980
   gtcctcaatt gtgtttacat taatagtggt gtttttacct atgaaattat tctggatcta 2040
   ataggacatt ttacaaaatg gcaagtatgg aaaaccatgg attctgaaag ttaaaaattt 2100
   agttgttctc cccaatgtgt attttaattt ggatggcagt ctcatgcaga ttttttaaaa 2160
   gattctttaa taacatgatt tgtttgcctt tctagatttc tttatctttc tgaccagcaa 2220
   cttagggagc agaatttaaa ttaggaagac aaagggaaag attcatttaa accatatttt 2280
   tacaaagttt gtcatttgcc ccaaggtcaa attttaaatt cttaattttc attttatttc 2340
   ccattttagg taaaagtttg catttaatct tagaattatg ttatttttgt tagtagtgtg 2400
   gaaacttaga gaacttattg tatggtgcct tgcaaaaata gagatagaaa gattttagca 2460
   tgcataccaa tatagtatat tacgcaatat ataagcacac ctaattaaca gattaatatc 2520
   agtaaaggta ttgctgctgg aatgaagaaa atgggatacg tttgtttctt tttttctatt 2580
   gttacataat tgccatgtgg acttgtttat gattattgtg tagagtagca tttaagattt 2640
   aactgtagca aaaattactt taaccgctgt atttaagtta gcatgttaat taattgtgta 2700
   gacattttgg cacaccatca cttttaacta tatcatacca atggttttgt gcccataata 2760
   aaaatggaaa aacctgttga atgttacgta ttggtatctt taatttcaac agtgggtaaa 2820
   ctggtttccc agtatacaat tcattgaaag caaaattgat taattatttc catttaattt 2880
   atacacactc aatacaaaat ttaatgttga ctttacgtaa taaagtataa tgcattttct 2940
   tttttactgt ttatgtatag tttacaaaat aaagaatctt gtaaccaaa 2989
<210> 288
   <211> 3083
   <212> DNA
   <213> Homo sapiens
<400> 288
   aggcgcgcgc tgtttccgga agtcgcggcc ggcgtcaccg ctgcggctgc ctcagctact      60
   gccgcagtcg ccgcggaatt cggcgagtag aaccgctgag gcgggcgcgg gcccgggtgg     120
   ggccaaggtt ccggccactc tgcagaatgg agataatcag gagcaatttt aagagtaatc     180
   ttcacaaagt gtaccaggcc atagaggagg ccgacttctt cgccatcgat ggggagtttt     240
   caggaatcag tgatggacct tcagtctctg cattaacaaa tggttttgac actccagaag     300
   agaggtatca gaagcttaaa aagcattcca tggacttttt gctatttcag tttggccttt     360
   gcacttttaa gtatgactac acagattcaa agtatataac gaagtcattt aacttctatg     420
   ttttcccgaa acccttcaat agatcctcac cagatgtcaa atttgtttgt cagagctcca     480
   gcattgactt tctagcaagc cagggatttg attttaataa agtttttcga aatggaattc     540
   catatttaaa tcaggaagaa gaaagacagt taagagagca gtatgatgaa aaacgttcac     600
   aggcgaatgg tgcaggagct ctgtcctatg tatctcctaa cacttcaaaa tgtcctgtca     660
   cgattcctga ggatcaaaag aagtttattg accaagtggt agagaaaata gaggatttat     720
   tacaaagtga agaaaacaag aacttggatt tagagccatg taccgggttc caaagaaaac     780
   taatttatca gactttgagc tggaagtatc cgaaaggcat tcatgttgag actttagaaa     840
   ctgaaaagaa ggagcgatat atagttatca gcaaagtaga tgaagaagaa cgcaaaagaa     900
   gagagcagca gaaacatgcc aaagaacagg aggagctgaa tgatgctgtg ggattttcta     960
   gagtcattca cgccattgct aattcgggaa aacttgttat tggacacaat atgctcttgg    1020
   acgtcatgca cacagttcat cagttctact gccctctgcc tgcggactta agtgagttta    1080
   aagagatgac aacatgtgtt ttccccagac tcttggatac taaattgatg gccagcacac    1140
   aaccttttaa ggatatcatt aacaacacat cccttgcgga attggaaaag cggttaaaag    1200
   agacaccttt caaccctcct aaagttgaaa gtgccgaagg ttttccaagt tatgacacag    1260
   cctctgaaca actccacgag gcaggctacg atgcctacat cacagggctg tgcttcatct    1320
   ccatggccaa ttacctaggt tcttttctca gccctccaaa aattcatgtg tctgccagat    1380
   caaaactcat tgaacctttt tttaacaagt tatttcttat gagggtcatg gatatcccct    1440
   atctaaactt ggaaggacca gacttgcagc ctaaacgtga tcatgttctc catgtgacat    1500
   tccccaaaga atggaaaacc agcgaccttt accagctttt cagtgccttt ggtaacattc    1560
   agatatcctg gattgatgac acatcagcat ttgtttccct tagccagccc gagcaagtaa    1620
   agattgctgt caataccagc aaatatgcag aaagctatcg gatccaaacc tatgctgaat    1680
   atatggggag aaaacaggaa gagaagcaga tcaaaagaaa gtggactgaa gatagctgga    1740
   aggaggctga cagcaaacgg ttaaaccccc agtgcatacc ctacaccctg cagaatcact    1800
   attaccgcaa caatagtttt acagctccca gcacagtagg aaagagaaat ttgagtccta    1860
   gtcaagagga agctggcctg gaggacggag tgtcagggga gatttccgac actgagcttg    1920
   agcagaccga ttcctgtgca gagcccctct cagagggaag gaaaaaggcc aagaaattaa    1980
   aaagaatgaa gaaggagctt tctccagcag gaagcatctc gaagaacagc cctgccacac    2040
   tctttgaagt tcctgacaca tggtaaccaa gacctgaggg cagcaaaccg ctggtgctgt    2100
   cgctgtgagc aagagccggc tggcacattt ggaagccgca ctgtatttaa cttaatcaaa    2160
   tgtggtatgg gaggggttgg aaaccaagtt gtctcctggg ggggagaaaa caggttttat    2220
   ttttgtggct gtggtttttt ccccttttta atctaactgc ctgttgacat tgacactcat    2280
   cacggttgta ggctgtcatg aatgtgtacg tgcttaacca gtgaattccg tgttgctctt    2340
   gtgaggcctt tcctgtcatg acccagtgtg cttaagaacc tgcctgatgg ggagtgtcgg    2400
   ctgtgaaatc tgcaaaaaga gctgacattc cagctgctgt gatcatgaat ttgggggtgt    2460
   actgtcctgc ctgtgcatct tctcgcactg agattttgag gcagttgcag ccctcggtta    2520
   gtctcccagt ggaaaaatcg gttgtgcctc cctgcttccc accatagctg cctgaaaaca    2580
   tgacgctctc aagcttgtcc ttccttcagg aagatgtcca ctcatgccca cccatgagag    2640
   ggcttgccgt atgccctggc ctttgggcat atttatgtag agttcctttc tcctaagacg    2700
   tgagtttctc atgggggatg tacgagtaaa aaggttaact tctgttctta tgcgtggcgc    2760
   tgtgttcact ttccagagtc tctgttcgtt tgtttggatg gcggtctcgg ggtacggcag    2820
   cgtgtgtgcg tacgtgtctg tgtgtgtgtg tgtgtgtgtg tgtgtgtgtg tgtgtgtgtg    2880
   tgtgtgaaat cgtgcaaatc tacaacatgt cccagcccat tctccgttga aacagatcac    2940
   agcaacgaca aacgctcatg gcgctgcttt gctccacccg cttcagatag atcattgtta    3000
   gatatttcac atttttgtat ggtggaaata aaaatgaaaa atgtatttcc aaaagatgaa    3060
   aattaaaaac attttcatag gac                                             3083
<210> 289
   <211> 2153
   <212> DNA
   <213> Homo sapiens
<400> 289
   cgaccccgag aggcccggtt cctttaggcc gcctgcccgc ctccagctct cggggtcggc     60
   tccaggaggc gccctcagga gaggggcggg cgctctattc cagagaccga gtggcagggc    120
   ggccactgtg gcggggctct ttccccgttt cgcctcagct acccctcagc tccggtagtc    180
   gccagtccgg ggtcgtcgcc gtttggggcg ggagctgctc ggccccgccg ccgtccccgt    240
   cgccgcttcc gggtccaggc ccctcgggcc gcctgccgcc gtcatgaggc tgcgggtgcg    300
   gcttctgaag cggacctggc cgctggaggt gcccgagacg gagccgacgc tggggcattt    360
   gcgctcgcac ctgaggcagt ccctgctgtg cacctggggg tacagttcta atacccgatt    420
   tacaattaca ttgaactaca aggatcccct cactggagat gaagagacct tggcttcata    480
   tgggattgtt tctggggact tgatatgttt gattcttcaa gatgacattc cagcgcctaa    540
   tataccttca tccacagatt cagagcattc ttcactccag aataatgagc aaccctcttt    600
   ggccaccagc tccaatcaga ctagcatgca ggatgaacaa ccaagtgatt cattccaagg    660
   acaggcagcc cagtctggtg tttggaatga cgacagtatg ttagggccta gtcaaaattt    720
   tgaagctgag tcaattcaag ataatgcgca tatggcagag ggcacaggtt tctatccctc    780
   agaacccatg ctctgtagtg aatcggtgga agggcaagtg ccacattcat tagagacctt    840
   gtatcaatca gctgactgtt ctgatgccaa tgatgccttg atagtgttga tacatcttct    900
   catgttggag tcaggttaca tacctcaggg caccgaagcc aaagcactgt ccatgccgga    960
   gaagtggaag ttgagcgggg tgtataagct gcagtacatg catcctctct gcgagggcag   1020
   ctccgctact ctcacctgtg tgcctttggg aaacctgatt gttgtaaatg ctacactaaa   1080
   aatcaacaat gagattagaa gtgtgaaaag attgcagctg ctaccagaat cttttatttg   1140
   caaagagaaa ctaggggaaa atgtagccaa catatacaaa gatcttcaga aactctctcg   1200
   cctctttaaa gaccagctgg tgtatcctct tctggctttt acccgacaag cactgaacct   1260
   accagatgta tttgggttgg tcgtcctccc attggaactg aaactacgga tcttccgact   1320
   tctggatgtt cgttccgtct tgtctttgtc tgcggtttgt cgtgacctct ttactgcttc   1380
   aaatgaccca ctcctgtgga ggtttttata tctgcgtgat tttcgagaca atactgtcag   1440
   agttcaagac acagattgga aagaactgta caggaagagg cacatacaaa gaaaagaatc   1500
   cccgaaaggg cggtttgtga tgctcctgcc atcgtcaact cacaccattc cattctatcc   1560
   caaccccttg caccctaggc catttcctag ctcccgcctt cctccaggaa ttatcggggg   1620
   tgaatatgac caaagaccaa cacttcccta tgttggagac ccaatcagtt cactcattcc   1680
   tggtcctggg gagacgccca gccagtttcc tccactgaga ccacgctttg atccagttgg   1740
   cccacttcca ggacctaacc ccatcttgcc agggcgaggc ggccccaatg acagatttcc   1800
   ctttagaccc agcaggggtc ggccaactga tggccggctg tcattcatgt gattgatttg   1860
   taatttcatt tctggagctc catttgtttt tgtttctaaa ctacagatgt caactccttg   1920
   gggtgctgat ctcgagtgtt attttctgat tgtggtgttg agagttgcac tcccagaaac   1980
   cttttaagag atacatttat agccctaggg gtggtatgac ccaaaggttc ctctgtgaca   2040
   aggttggcct tgggaatagt tggctgccaa tctccctgct cttggttctc ctctagattg   2100
   aagtttgttt tctgatgctg ttcttaccag attaaaaaaa agtgtaaatt aca          2153
<210> 290
   <211> 790
   <212> DNA
   <213> Homo sapiens
<400> 290
   tgtgacgtca cggcgtcgtt ggtaaggggc tggcggccgg ggagctgcgt agctcccggc     60
   cccgcggcca tgcccaagcg gagctgcccc ttcgcggacg tggccccgct acagctcaag    120
   gtccgcgtga gccagaggga gttgagccgc ggcgtgtgcg ccgagcgcta ctcgcaggag    180
   gtcttcgaga agaccaagcg actcctgttc ctcggggccc aggcctacct ggaccacgtg    240
   tgggatgaag gctgtgccgt cgttcacctg ccagagtccc caaagcctgg ccctacaggg    300
   gccccgaggg ctgcacgtgg gcagatgctg attggaccag acggccgcct gatcaggagc    360
   cttgggcagg cctccgaagc tgacccatct ggggtagcgt ccattgcctg ttcctcatgc    420
   gtgcgagccg tggatgggaa ggcggtctgc ggtcagtgtg agcgagccct gtgcgggcag    480
   tgtgtgcgca cctgctgggg ctgcggctcc gtggcctgta ccctgtgtgg cctcgtggac    540
   tgcagtgaca tgtacgagaa agtgctgtgc accagctgtg ccatgttcga gacctgaggc    600
   tggctcaagc cggctgcctt caccgggagc cacgccgtgc atggcagcct tccctggacg    660
   agcgctcggt gttcacactg aactgtgggg tcgacgggag ggtgcctttt tacatgttct    720
   attttgtatc ctaatgacag aatgaataaa cctctttata tttgcacaag aaaaaaaaaa    780
   aaaaaaaaaa                                                           790
<210> 291
   <211> 2119
   <212> DNA
   <213> Homo sapiens
<400> 291
   atttcctctg ggttacggcg caggcgcaag ataagctagg agccgcgcga gtcgtagtgt 60
   cgctgtttgc gggtctccgc gcgggaccgg ggcgcagcgg ggtcgctgag gcgagggtgt 120
   catgtcagac aacgaggaca attttgatgg cgacgacttt gatgatgtgg aggaggatga 180
   agggctagat gacttggaga atgccgaaga ggaaggccag gagaatgtcg agatcctccc 240
   ctctggggag cgaccgcagg ccaaccagaa gcgaatcacc acaccataca tgaccaagta 300
   cgagcgagcc cgcgtgctgg gcacccgagc gctccagatt gcgatgtgtg cccctgtgat 360
   ggtggagctg gagggggaga cagatcctct gctcattgcc atgaaggaac tcaaggcccg 420
   aaagatcccc atcatcattc gccgttacct gccagatggg agctatgaag actggggggt 480
   ggacgagctc atcatcaccg actgagctgg agtcatcttc ctgcccttgc cccatgccca 540
   attttcattc tcactttata tgtgtaaata ataaaatatt caactttcca acccccttcc 600
   cctctgctta tctgcaatgt caccacctgt tgcttccccg ttaccgccat gctgcgtgga 660
   gcatgcacct attccagtgg ccctgtgact gtcagctcct taagaagcac caggggccct 720
   tagccccttt ggatccccca catccttccc tccatctccc tgttccccag agcaaaggct 780
   gctgcagggg agacacctca gctgccttcc aagcagacag acaagtcttt gtgcccaggg 840
   agctggttgc cacggaaacc cccaatttcc tttccagtgg ggactggctg caggggcttc 900
   tcccttctca ggagtatcac agagcaggtc tcatcaagcc acccattgtt tcctaaggac 960
   ctgcttcgag cctcttatcg tgggctcgga tcccctttca ggagcagtgc cccagcagga 1020
   agcgtggggg tgtgctgatc tcccaccctc cccaggcaga gccctgctgg gcaagtcagc 1080
   agctggagca aggaccgagc acctgcctac ccctgccccc atggctctgt ccccactcct 1140
   cctcaggact ctgcccacac gctgtcctct gtggcccacc tctttggtgt gccacaccca 1200
   tgccttcttc ctagcctcta taagatatcc tttccctcct atttggggct ggtgatcccc 1260
   tgaggccttg gggacatggt gctggggtgg tggtgctcct gggttccagg tgttacatta 1320
   agtcagagct ggagttcccc cttcctctgc ctgaggttct aatgggtcct cttcctttct 1380
   ccaccctgct tacccaacct gaggtaagac cagtcacact ggctcctccc tcctagaggg 1440
   ggtcaggggg agggtgtata ttgacatgaa cagggataga gggtaaactg gctccctgaa 1500
   tatgccagcc ttaacctcca ttccactgcc agctcccctt caaagaggag gagctgggct 1560
   tccctaacct ctgcaggagg cagggcctcc aggcctaggt gcagcctggc cctgggatgg 1620
   gatgtgggga gtgaatggtg aggatctgca ttggtgggag gggtgtccgc tgccctggag 1680
   aagggttaat tcagggagca gtggacttca cactcccatc caccctcctc caagcctgtg 1740
   gaatccttta atcaagttgg gtgctgaaat ttcagctctg aaatgccgcc tttgtgctgg 1800
   catccaggca gccgccccag agtgcggggg tcactttctc ggccccattt ctctaaatgg 1860
   tctctttgtt ccctgctggg ctgctcagta tcagatgtga ttaaagggag atggggtttg 1920
   ggctggggag gaggggaatt gggggttaac ccttagggga tagagtgcaa gggaaatggg 1980
   acagaagggg tgtttttcct cctgtcttcc tcttcccatt ctcctctttt ggggagtccc 2040
   ctgctattca gctgtctggg cctggctttt gacttctctt gaataaaatg tcccggtcac 2100
   caaaaaaaaa aaaaaaaaa 2119
<210> 292
   <211> 2438
   <212> DNA
   <213> Homo sapiens
<400> 292
   aaacatgggg cggggcggcg cggccgggga agcgtgatga aggcctacga gtgcggcgcg      60
   gcctgaaggg gcacgcgggg gacctgcaaa gctagtgagg ggcggggcag gcggcgcggt     120
   gggggcgggc cgagcccgga ggccagatga gcggacacag ccccacgcgc ggggccatgc     180
   aggtggccat gaacggtaag gcccgcaaag aggcggtgca gactgcggct aaggaactcc     240
   tcaagttcgt gaaccggagt ccctctcctt tccatgctgt ggctgaatgc cgcaaccgcc     300
   ttctccaggc tggcttcagt gaactcaagg agactgagaa atggaatatt aagcccgaga     360
   gcaagtactt catgaccagg aactcctcca ccatcatagc ttttgctgta gggggccagt     420
   acgttcctgg caatggcttc agcctcatcg gggcccacac ggacagcccc tgcctccggg     480
   tgaaacgtcg gtctcgccgc agccaggtgg gcttccagca agtcggtgtg gagacctatg     540
   gtggtgggat ctggagcacc tggtttgacc gtgacctgac tctggctgga cgcgtcattg     600
   tcaagtgccc tacctcaggt cggctggagc agcagctggt gcacgtggag cggcccattc     660
   ttcgcatccc acacctggcc atccatctgc agcgaaatat caacgagaac tttgggccca     720
   acacagagat gcatctagtc cccattcttg ccacagccat ccaggaggag ctggagaagg     780
   ggactcctga gccagggcct ctcaatgctg tggatgagcg gcaccattcg gtcctcatgt     840
   ccctgctctg tgcccatctg gggctgagcc ccaaggacat agtggagatg gagctctgcc     900
   ttgcagacac ccagcctgcg gtcttgggtg gtgcctatga tgagttcatc tttgctcctc     960
   ggctggacaa tctgcacagc tgcttctgtg ccctgcaggc cttgatagat tcctgtgcag    1020
   gccctggctc cctggccaca gagcctcacg tgcgcatggt cacactctat gacaacgaag    1080
   aggtggggtc tgagagtgca cagggagcac agtcactgct gacagagctg gtgctgcggc    1140
   ggatctcagc ctcgtgccag cacccgacag ccttcgagga agccataccc aagtccttca    1200
   tgatcagcgc agacatggcc catgctgtgc atcccaacta cctggacaag catgaggaga    1260
   accaccggcc tttattccac aagggccccg tgatcaaggt gaacagcaag caacgctatg    1320
   cttcaaacgc ggtgtcagag gccctgatcc gagaggtggc caacaaagtc aaggtccccc    1380
   tgcaggatct catggtccgg aatgacaccc cctgtggaac caccattgga cctatcttgg    1440
   cttctcggct ggggctgcgg gtgctggatt taggcagccc ccaactggcc atgcactcta    1500
   tccgggagat ggcctgcacc acaggagtcc tccagaccct caccctcttc aagggcttct    1560
   ttgagctgtt cccttctcta agccataatc tcttagtgga ttgagccctc ttggaaagac    1620
   ttctctgcca tccctttgca cctgagaggg gaagttctca gctgagctga agctggatta    1680
   ttaaagtgga ttgtcactca gactctccgt gctacgctta tttggagact agaggagtgg    1740
   gagttgagcc tggcttgaac ctttggaacc agaaaagttg gggagcaggt ggaggaggcc    1800
   acactcctgg gagctgatgg ttttaaatct ggttttaaat ctcttctctg tctcaagtcc    1860
   atgtctgaag tgggtgaagg gtggactgga tcctcaagca gaaggtcact tctcccaccc    1920
   ctagtcctcc acctgggaaa tggcctcaac ggtcttccct ctttccatcc ccagaatggg    1980
   tgtcccgctc tgccttcaga gatcctgttc tgcactgggc caccttcaga gctgctctgg    2040
   gcagacaaag ctgtttctca ccctcccaaa atcccttccc atcccctctc aggagctgca    2100
   aagagaaggg cacagtctat accttatcac ccttcatcat ccacttctca gcagtccctg    2160
   gtattacttg attgtgaaca tattatcagg aaaaaagatt atcattgaaa aaattggaaa    2220
   agtcaaagtc tggccgggtg cagcggctca catctgtatt cccagcattt tgggaggcca    2280
   aggcaggtgg atcacttgag gtcagagttc aagaccagcc ttgccaacat ggtgaaaccc    2340
   atctctactt aaaaaaaaat acaaaaaaat tagccaggca tggtggcaca cacctgtaat    2400
   ctcagctatt caggagactg aggcaggaga atagcttg                            2438
<210> 293
   <211> 22
   <212> DNA
   <213> Artificial Sequence
<220>
   <223> Made in a lab
<400> 293
   tgaaaaatgg agaaacaaga cg 22
<210> 294
   <211> 20
   <212> DNA
   <213> Artificial Sequence
<220>
   <223> Made in a lab
<400> 294
   ttcccattga gccaggttta 20
<210> 295
   <211> 20
   <212> DNA
   <213> Artificial sequence
<220>
   <223> Made in a lab
<400> 295
   ctggagagcc aacctcaaag 20
<210> 296
   <211> 23
   <212> DNA
   <213> Artificial sequence
<220>
   <223> Made in a lab
<400> 296
   tcaataacaa aagtcaggtg age 23
<210> 297
   <211> 23
   <212> DNA
   <213> Artificial Sequence
<220>
   <223> Made in a lab
<400> 297
   atcactagac aaggaagaaa gca 23
<210> 298
   <211> 26
   <212> DNA
   <213> Artificial Sequence
<220>
   <223> Made in a lab
<400> 298
   ttagagactg tgaaggaatt agcaga 26
<210> 299
   <211> 20
   <212> DNA
   <213> Artificial Sequence
<220>
   <223> Made in a lab
<400> 299
   cagccagaac tgcagaagaa 20
<210> 300
   <211> 20
   <212> DNA
   <213> Artificial Sequence
<220>
   <223> Made in a lab
<400> 300
   aggcttcttg acacctggaa 20
<210> 301
   <211> 20
   <212> DNA
   <213> Artificial Sequence
<220>
   <223> Made in a lab
<400> 301
   gggacagctg ggtcttcata 20
<210> 302
   <211> 19
   <212> DNA
   <213> Artificial sequence
<220>
   <223> Made in a lab
<400> 302
   ctctggccat ccctcgtat 19
<210> 303
   <211> 20
   <212> DNA
   <213> Artificial Sequence
<220>
   <223> Made in a lab
<400> 303
   tgacttgggg agttcctacg 20
<210> 304
   <211> 20
   <212> DNA
   <213> Artificial Sequence
<220>
   <223> Made in a lab
<400> 304
   tgtttgtgtc tgggctgttc 20
<210> 305
   <211> 20
   <212> DNA
   <213> Artificial Sequence
<220>
   <223> Made in a lab
<400> 305
   gggttttggt ctcaccactg 20
<210> 306
   <211> 19
   <212> DNA
   <213> Artificial Sequence
<220>
   <223> Made in a lab
<400> 306
   atcccagagg catccaaag 19
<210> 307
   <211> 20
   <212> DNA
   <213> Artificial Sequence
<220>
   <223> Made in a lab
<400> 307
   agaagctggc atcagaaaaa 20
<210> 308
   <211> 21
   <212> DNA
   <213> Artificial Sequence
<220>
   <223> Made in a lab
<400> 308
   gcctcagatg gtaaagtcag c 21
<210> 309
   <211> 20
   <212> DNA
   <213> Artificial Sequence
<220>
   <223> Made in a lab
<400> 309
   gctggaccaa tagcaaggtg 20
<210> 310
   <211> 19
   <212> DNA
   <213> Artificial Sequence
<220>
   <223> Made in a lab
<400> 310
   aaggaagcat gggaaggtg 19
<210> 311
   <211> 20
   <212> DNA
   <213> Artificial Sequence
<220>
   <223> Made in a lab
<400> 311
   gggttcttca gcgtctatgc 20
<210> 312
   <211> 21
   <212> DNA
   <213> Artificial Sequence
<220>
   <223> Made in a lab
<400> 312
   ttcagcaaag gttgactcac a 21
<210> 313
   <211> 22
   <212> DNA
   <213> Artificial Sequence
<220>
   <223> Made in a lab
<400> 313
   aaaatcccat gacaaatcgt tc 22
<210> 314
   <211> 20
   <212> DNA
   <213> Artificial Sequence
<220>
   <223> Made in a lab
<400> 314
   gggcaacagt gaagtggttt 20
<210> 315
   <211> 20
   <212> DNA
   <213> Artificial Sequence
<220>
   <223> Made in a lab
<400> 315
   actccacaga taccccgaag 20
<210> 316
   <211> 19
   <212> DNA
   <213> Artificial sequence
<220>
   <223> Made in a lab
<400> 316
   tccaccactt gctgagctg 19
<210> 317
   <211> 20
   <212> DNA
   <213> Artificial sequence
<220>
   <223> Made in a lab
<400> 317
   acatttttga ggccaacgac 20
<210> 318
   <211> 20
   <212> DNA
   <213> Artificial Sequence
<220>
   <223> Made in a lab
<400> 318
   ctcccacgtt caccttgttt 20
<210> 319
   <211> 19
   <212> DNA
   <213> Artificial Sequence
<220>
   <223> Made in a lab
<400> 319
   cctggagcag ccgtatctg 19
<210> 320
   <211> 21
   <212> DNA
   <213> Artificial sequence
<220>
   <223> Made in a lab
<400> 320
   gacaaactcc tttcggtcac a 21
<210> 321
   <211> 20
   <212> DNA
   <213> Artificial sequence
<220>
   <223> Made in a lab
<400> 321
   cccagtcagt gcacaaagag 20
<210> 322
   <211> 20
   <212> DNA
   <213> Artificial sequence
<220>
   <223> Made in a lab
<400> 322
   gcattgtggt caaactgcat 20
<210> 323
   <211> 20
   <212> DNA
   <213> Artificial Sequence
<220>
   <223> Made in a lab
<400> 323
   gcaatctgca gagttcgtga 20
<210> 324
   <211> 20
   <212> DNA
   <213> Artificial sequence
<220>
   <223> Made in a lab
<400> 324
   gccatcatct gcgactctaa 20
<210> 325
   <211> 21
   <212> DNA
   <213> Artificial Sequence
<220>
   <223> Made in a lab
<400> 325
   tggagcagat cctcttcatc t 21
<210> 326
   <211> 19
   <212> DNA
   <213> Artificial Sequence
<220>
   <223> Made in a lab
<400> 326
   ccaaggtcag cagcacaat 19

## Description

### TECHNICAL FIELD

The present disclosure relates to methods and compositions for diagnosing and defining the staging or progress of disorders such as prostate cancer.

### BACKGROUND

The use of prostate specific antigen (PSA) as a diagnostic biomarker for prostate cancer was approved by the US Federal Drug Agency in 1994. In the nearly two decades since this approval, the PSA test has remained the primary tool for use in prostate cancer diagnosis, in monitoring for recurrence of prostate cancer, and in following the efficacy of treatments. However the PSA test has multiple shortcomings and, despite its widespread use, has resulted in only small changes in the death rate from advanced prostate cancers. To reduce the death rate and the negative impacts on quality of life caused by prostate cancer, new tools are required not only for more accurate primary diagnosis, but also for assessing the risk of spread of primary prostate cancers, and for monitoring responses to therapeutic interventions.

Today, a blood serum level of around 4 ng per ml of PSA is considered indicative of prostate cancer, while a PSA level of 10 ng per ml or higher is considered highly suggestive of prostate cancer. The PSA blood test is not used in isolation when checking for prostate cancer; a digital rectal examination (DRE) is usually also performed. If the results of the PSA test or the DRE are abnormal, a biopsy is generally performed in which small samples of tissue are removed from the prostate and examined. If the results are positive for prostate cancer, further tests may be needed to determine the stage of progression of the cancer, such as a bone scan, a computed tomography (CT) scan or a pelvic lymph node dissection.

While the PSA test has a good sensitivity (80%), it suffers from a false positive rate that approaches 75%. For example, it has been estimated that for PSA values of 4-10 ng/ml, only one true diagnosis of prostate cancer was found in approximately four biopsies performed (Catalona et al. J. Urol. 151(5):1283-90, 1994). Tests that measure the ratio of free to total (i.e., free plus bound) PSA do not have significantly greater specificity or sensitivity than the standard PSA test.

Higher PSA levels often lead to biopsies to determine the presence or absence of cancer cells in the prostate, and may lead to the surgical removal of the localized prostate gland. While surgery removes the localized cancer and often improves prostate cancer-specific mortality, it also masks the fact that many patients with prostate cancer, even in the absence of surgery, do not experience disease progression to metastasis or death.

The high false positive rate associated with the PSA test leads to many unnecessary biopsies. In addition to the physical discomfort and psychological distress associated with biopsies, it has been suggested that performing a biopsy may promote inflammation of cancerous tissue and increase the risk of cancer metastasis.

Currently, the established prognostic factors of histological grade and cancer stage from biopsy results, and prostate-specific antigen level in blood at diagnosis are insufficient to separate prostate cancer patients who are at high risk for cancer progression from those who are likely to die of another cause.

Once high risk or virulent forms of prostate cancer have been diagnosed, control strategies may involve surgery to remove the prostate gland if identified before metastasis, radiation to destroy cancer cells within the prostate and drug-based testosterone repression, generally referred to as androgen depletion therapy. These various treatments may bring about cures in some instances, or slow the time to death. However, for those with the most virulent forms of prostate cancer, the cancer will usually recur after surgery or radiation therapy and progress to resistance to androgen depletion therapy, with death a frequent outcome.

Early detection of virulent forms of prostate cancer is critical but the conclusion of specialist physicians is that the PSA test alone is inadequate for distinguishing patients whose cancers will become virulent and progress to threaten life expectancy from those with indolent cancers.

The following are some key reasons why the PSA test does not meet the needs of men's health:

### i) The type of cancer

There are at least two basic cell types involved in prostate cancer. Adenocarcinoma is a cancer of epithelial cells in the prostate gland and accounts for approximately 95% of prostate cancers. Neuroendocrine cancers may arise from cells of the endocrine (hormonal) and nervous systems of the prostate gland and account for approximately 5% of prostate cancers. Neuroendocrine cells have common features such as special secretory granules, produce biogenic amines and polypeptide hormones, and are most common in the intestine, lung, salivary gland, pituitary gland, pancreas, liver, breast and prostate. Neuroendocrine cells co-proliferate with malignant adenocarcinomas and secrete factors which appear to stimulate adenocarcinoma cell growth. Neuroendocrine cancers are rarer, and are considered non-PSA secreting and androgen-independent for their growth.

### ii) Asymptomatic men

Some 15 to 17% of men with prostate cancer have cancers that grow but do not produce increasing or high blood levels of PSA. In these patients, who are termed asymptomatic, the PSA test often returns false negative test results as the cancer grows.

### iii) BPH, prostatitis and PIN

Benign prostate hypertrophy (BPH), a non-malignant growth of epithelial cells, and prostatitis are diseases of the prostate that are usually caused by an infection of the prostate gland. Both BPH and prostatitis are common in men over 50 and can result in increased PSA levels. Incidence rates increase from 3 cases per 1000 man-years at age 45-49 years, to 38 cases per 1000 man-years by the age of 75-79 years. Whereas the prevalence rate is 2.7% for men aged 45-49, it increases to at least 24% by the age of 80 years. While prostate cancer results from the deregulated proliferation of epithelial cells, BPH commonly results from proliferation of normal epithelial cells and frequently does not lead to malignancy (Ziada et al. (1999) Urology 53(3 Suppl 3D): 1-6). Bacterial infection of the prostate can be demonstrated in only about 10% of men with symptoms of chronic prostatitis/chronic pelvic pain syndrome. Bacteria able to be cultured from patients suffering chronic bacterial prostatitis are mainly Gram-negative uropathogens. The role of Gram-positives, such as staphylococci and enterococci, and atypicals, such as chlamydia, ureaplasmas, mycoplasmas, are still debatable.

Another condition, known as prostate intraepithelial neoplasia (PIN), may precede prostate cancer by five to ten years. Currently there are no specific diagnostic tests for PIN, although the ability to detect and monitor this potentially pre-cancerous condition would contribute to early detection and enhanced survival rates for prostate cancer.

### iv) The phenotype of the prostate cancer

The phenotype of prostate cancer varies from one patient to another. More specifically, in different individuals prostate cancers display heterogeneous cellular morphologies, growth rates, responsiveness to androgens and pharmacological blocking agents for androgens, and varying metastatic potential. Each prostate cancer has its own unique progression involving multiple steps, including progression from localized carcinoma to invasive carcinoma to metastasis. The progression of prostate cancer likely proceeds, as seen for other cancers, via events that include the loss of function of cell regulators such as cancer suppressors, cell cycle and apoptosis regulators, proteins involved in metabolism and stress response, and metastasis related molecules (Abate-Shen et al. Polypeptides Dev. 14(19):2410-34, 2000; Ciocca et al. Cell Stress Chaperones 10(2):86-103, 2005).

At present health authorities do not universally recommend widespread screening for prostate cancer with the PSA test. There are concerns that many men may be diagnosed and treated unnecessarily as a result of being screened, at high cost to health systems as well as risking the patient's quality of life, such as through incontinence or impotence. Despite these concerns, prostate cancer is the most prevalent form of cancer and the second most common cause of cancer death in New Zealand, Australian and North American males (Jemal et al. CA Cancer J. Clin., 57(1):43-66, 2007). In reality, at least some of the men incubating life threatening forms of prostate cancer are being missed until their cancer is too advanced, due to the economic costs of national screening, the need to avoid unnecessary over-treatment, and/or the presence of progressive cancers producing only low or background levels of PSA. The need for a better diagnostic test could not be clearer.

The lack of a diagnostic test that distinguishes a non-life threatening from a potentially life-threatening cancer raises the important clinical question as to how aggressively to treat patients with localized prostate cancer. Treatment options for more aggressive cancers are invasive and include radical prostatectomy and/or radiation therapy. Androgen-depletion therapy, for example using gonadotropin-releasing hormone agonists (e.g., leuprolide, goserelin, etc.), is designed to reduce the amount of testosterone that enters the prostate gland and is used in patients with metastatic disease, some patients who have a rising PSA and choose not to have surgery or radiation, and some patients with a rising PSA after surgery or radiation. Treatment options usually depend on the stage of the prostate cancer. Men with a 10-year life expectancy or less, who have a low Gleason score from a biopsy and whose cancer has not spread beyond the prostate are often not treated. Younger men with a low Gleason score and a prostate-restricted cancer may enter a phase of "watchful waiting" in which treatment is withheld until signs of progression are identified. However, these prognostic indicators do not accurately predict clinical outcome for individual patients.

Unlike many cancer types, specific patterns of gene expression have not been consistently identified in prostate cancer progression, although a number of candidate genes and pathways likely to be important in individual cases have been identified (Tomlins et al., Annu. Rev. Pathol. 1:243-71, 2006). Several groups have attempted to examine prostate cancer progression by comparing gene expression of primary carcinomas to normal prostate tissue. Because of differences in technique, the integrity of the tissue samples used as well as the biological heterogeneity of prostate cancers, these studies have reported thousands of candidate genes that share only moderate consensus. Also sample type differences could contribute to the lack of consensus seen from these studies. For example formalin fixed paraffin embedded (FFPE) tissues allow a convenient comparison of tumor and adjacent tissues but many of the cDNA microarray studies have used snap frozen tissues (Bibikova et al., Genomics 89:666-72, 2007; van't Veer et al., Nature 415:530-6, 2002). In addition, some studies have included accident victim donors as controls to overcome potential field effects (Aryee et al. Sci Transl Med 5, 169ra10 2013; Chandran et al. BMC Cancer, 5:45 doi:10.1186/1471-2407-5-45, 2005). However, a few genes have emerged including hepsin (HPN; Rhodes et al., Cancer Res. 62:4427-33, 2002), alpha-methylacyl-CoA racemase (AMACR; Rubin et al., JAMA 287:1662-70, 2002, Lin et al. Biosensors 2:377-387, 2012), enhancer of Zeste homolog 2 (EZH2; Varambally et al. Nature, 419:624-9, 2002), L-dopa decarboxylase (DDC; Koutalellis et al. BJU International, 110:E267-E273, 2012) and anterior-gradient 2 (AGR2; Hu et al. Carcinogenesis 33:1178-1186, 2012) which have been shown experimentally to have probable roles in prostate carcinogenesis.

More recently, bioinformatic approaches employing data from gene expression profiling using both microarray and RNA-seq have generated lists of dysregulated genes in prostate cancer. RNA-seq is a technique based on enumeration of RNA transcripts using next-generation sequencing methodologies. However, because of their different experimental approaches, these studies have also shown few consensus genes, (Aryee et al. Sci Transl Med 5, 169ra10, 2013; Chandran et al. BMC Cancer, 5:1471-2407 2005; Pflueger et al. Genome Res. 21:56-67, 2011; Prensner et al. Nature Biotechnology 29:742-749, 2011; Shancheng Ren et al. Cell Research 22:806-821, 2012).

A number of studies have also shown distinct classes of prostate cancers separable by their gene expression profiles (Glinsky et al., J. Clin. Invest. 113:913-23, 2004; Hsieh et al., Nature doi:10.1038/nature.10912, 2012; Lapointe et al., Proc. Natl. Acad. Sci. USA 101:811-6, 2004; LaTulippe et al., Cancer Res. 62:4499-506, 2002; Markert et al., Proc. Natl. Acad. Sci. doi:10.1073/pnas.1117029108, 2012; Rhodes et al., Cancer Res. 62:4427-33, 2002; Singh et al., Cancer Cell 1:203-9, 2002; Yu et al., J. Clin. Oncol. 22:2790-9, 2004; Varambally et al., Nature 419:624-9, 2002). Additionally, these approaches have been used to identify the genomic fusion of androgen-regulated genes including transmembrane protease, serine 2 (TMPRSS2) with members of the erythroblast transformation specific (ETS) DNA transcription factor family (Tomlins et al., Science 310:644-8, 2005, Tomlins, Nature 448: 595-599, 2007). These fusions appear commonly in prostate cancers and have been shown to be prevalent in more aggressive cancers (Attard et al., Oncogene 27:253-63, 2008; Barwick et al. Br. J. Cancer 102:570-576, 2010; Demichelis et al., Oncogene 26:4596-9, 2007; Nam et al., Br. J. Cancer 97:1690-5, 2007). Transcriptional modulation of TMPRSS2-ERG fusions has been shown to be associated with prostate cancer biomarkers and TGF-beta signalling (Brase et al., BMC Cancer 11:507 doi: 10.1186/1471, 2011). In addition to specific gene fusions, a vast array of mutational changes, including copy number variants, have been associated with prostate cancer tumours (Berger et al., Nature 470:214-220, 2011; Demichellis et al., Proc. Natl. Acad. Sci. doi:10.1073/pnas.117405109, 2012; Kumar et al., Proc. Natl. Acad. Sci. 108:17087-17092, 2011). Intratumor heterogeneity has also been found which has been suggested to result in underestimation of the degree of tumor heterogeneity (Gerlinger et al., New Eng, J. Med. 66:883-892, 2012). In particular mutations involving the substrate binding cleft of SPOP, which was found in 6-15% of prostate tumors, lacked ETS family gene rearrangements suggesting that tumors with SPOP mutations define a new class of prostate tumors. Also tumors with SPOP mutations lacked PTEN deletions in primary tumors but not in metastatic tumors (Barbieri et al., Nature Gen. 44:685-689, 2012).

Gene expression is the transcription of DNA into messenger RNA by RNA polymerase. Up-regulation describes a gene which has been observed to have higher expression (higher RNA levels) in one sample (for example, from cancer tissue) compared to another (usually healthy tissue from a control sample). Down-regulation describes a gene which has been observed to have lower expression (lower RNA levels) in one sample (for example, from cancer tissue) compared to another (usually healthy tissue from a control sample).

A common technology used for measuring RNA abundance is RT-qPCR where reverse transcription (RT) is followed by real-time quantitative PCR (qPCR). Reverse transcription first generates a DNA template from the RNA. This single-stranded template is called cDNA. The cDNA template is then amplified in the quantitative step, during which the fluorescence emitted by labeled hybridization probes or intercalating dyes changes as the DNA amplification process progresses. Quantitative PCR produces a measurement of an increase or decrease in copies of the original RNA and has been used to attempt to define changes of gene expression in cancer tissue as compared to comparable healthy tissues (Nolan T, et al. Nat Protoc 1:1559-1582, 2006; Paik S. The Oncologist 12:631-635, 2007; Costa C, et al. Transl Lung Cancer Research 2:87-91, 2013).

Massive parallel sequencing made possible by next generation sequencing (NGS) technologies is another way to approach the enumeration of RNA transcripts in a tissue sample and RNA-seq is a method that utilizes this. It is currently the most powerful analytical tool used for transcriptome analyses, including gene expression level difference between different physiological conditions, or changes that occur during development or over the course of disease progression. Specifically, RNA-seq can be used to study phenomena such as gene expression changes, alternative splicing events, allele-specific gene expression, and chimeric transcripts, including gene fusion events, novel transcripts and RNA editing.

High throughput assays for the detection and/or sequencing of particular target nucleic acids are known in the art and are disclosed in WO2010/115154 and Levin et al. WO2010/115154 discloses amplification methods in which target nucleotide sequences are labeleld with tags and barcode nucleotide sequences to allow rapid identification of the target nucleic acids in a plurality of samples. Levin et al (Genome Biology, vol 10, issue 10, Article R115) discloses a targeted RNA-sequencing method allowing further analysis of a specific subset of a transcriptome such as cancer-related genes for mutations, structural alterations and expression levels.

However, there are currently no methods that allow the use of RNA-seq for the accurate and reproducible quantification of multiple specific RNAs for reliable applications in the field of diagnostics.

### Why is it important to detect multiple biomarkers?

Using multiple biomarkers in a diagnostic or prognostic test is preferable to using a single biomarker because of the following:
Each individual tumor is heterogeneous with respect to all of the different aspects of their genome, transcriptome and proteome;
Multiple tumor foci are commonly found in tissues;
A single biomarker does not allow tumors of different lethality, aggressiveness or specificity to be differentiated;
A single biomarker may be affected by a treatment regime or other environmental influence;
A single biomarker may be affected by a field effect either as part of the progression of the disease or due to the tumor itself; and
A single biomarker may be less effective in particular ethnic groups.

### Why does RT-qPCR not allow the accurate detection of multiple biomarkers?

RT-qPCR is a time consuming technique as expression differences are determined for a single gene at a time, which does not allow multiple biomarkers to be compared/assessed at one time.

Comparing expression levels for genes across different experiments is often difficult, and can require complicated normalization methods that may not be suitable for integration into a diagnostic.

RT-qPCR does not allow the accurate detection of down-regulated genes because it is limited in its fluorescence detection range, compared to NGS based methods. This causes genes that are at a low and/or high abundance to be problematic. Very often these transcripts, for which differential expression is difficult to measure, are the ones with the most diagnostic and/or progonostic value. RT-PCR does not allow multiplexing which causes a rise in cost per RNA biomarker, and hence the overall cost of the diagnostic test.

There thus remains a need in the art for an accurate test for prostate cancer.

### SUMMARY

The present invention provides methods for determining the presence and progression of a disorder in a subject. Such methods employ modified RNA-seq techniques to determine the relative frequency of one or more RNA biomarkers (also referred to as gene transcript biomarkers) specific for the disorder in the subject compared to that in healthy controls. Determination of the relative frequency of expression levels of specific combinations of RNA biomarkers using the methods disclosed herein can also be used to determine the type and/or stage of a disorder, and to monitor the progression of a disorder and/or the effectiveness of treatment. Disorders that can be diagnosed and monitored using the methods disclosed herein include, but are not limited to, cancers, such as prostate and breast cancers.

The methods disclosed herein allow the determination of the frequency of multiple RNA biomarkers simultaneously using a process known as multiplexing. Multiplexing is a process wherein oligonucleotides specific for multiple biomarkers are amplified together to produce a pool of amplicons. The advantages of multiplexing are that it allows simultaneous testing of multiple RNA biomarkers in one or a small number of tubes, which in turn:
Reduces cost;
Reduces the amount of tissue required;
Increases the level of reproducibility due to less hands-on manipulation;
Reduces time involved in set-up; and
Increases throughput.

More specifically, the disclosed methods employ oligonucleotides specific for RNA biomarkers known to be associated with the presence and/or progression of a disorder, such as prostate cancer, at specific steps of a RNA-seq protocol to selectively identify cDNAs for the RNA biomarkers, and compare their relative frequency of expression between prostate cancer donors and healthy donors, as well as defining differences in expression between different stages of the disorder.

In conventional RNA-seq methodologies, the actual frequency of expression of each transcript is determined for the whole genome. These frequencies can be biased by differences in the efficiency of the cDNA production and subsequent PCR amplification steps for each transcript. The inventors believe that the methods disclosed herein avoid these biases by determining the relative, rather than actual, frequency of expression of RNA biomarkers. The biases are not relevant as long as they are neutral with respect to the comparisons made. The relative changes in frequency of expression of RNA biomarkers specific for prostate cancer allows detection of prostate cancers, distinguishing prostate cancers from benign prostate hypertrophy (BPH) and prostatitis, and detection of prostate cancers in asymptomatic men whose prostate cancer may produce low levels of PSA with high sensitivity and specificity. In certain embodiments, the disclosed methods determine changes in frequency of expression of RNA biomarkers in order to distinguish between indolent cancers, which have a low likelihood of progressing to a lethal disease, and more aggressive forms of prostate cancer which are life threatening and require treatment.

In one aspect, the present disclosure provides methods for detecting the presence of a disorder in a subject, comprising: (a) determining the relative frequency of expression of at least one RNA biomarker in a biological sample obtained from the subject using RNA sequencing; and (b) comparing the relative frequency of expression of the at least one RNA biomarker in the biological sample with a predetermined threshold value, wherein increased or decreased relative frequency of expression of the at least one RNA biomarker in the biological sample indicates the presence of the disorder in the subject. In related aspects, the disclosed methods comprise: (a) determining the relative frequency of expression of a plurality of RNA biomarkers in the biological sample; and (b) comparing the relative frequency of expression of the plurality of RNA biomarkers in the biological sample with predetermined threshold values, wherein increased or decreased relative frequency of expression of at least two or more of the RNA biomarkers in the biological sample indicates the presence of the disorder in the subject.

In one embodiment, the relative frequency of expression of at least one RNA biomarker is determined by: (a) isolating total RNA from the biological sample; (b) generating first strand cDNA from the total RNA using a first oligonucleotide primer specific for the at least one RNA biomarker; (c) synthesizing second strand cDNA to provide double-stranded cDNA (dsDNA); (d) addingat least one sequencing adapter to the double-stranded cDNA; (e) amplifying the double-stranded cDNA to provide a cDNA library from the double-stranded cDNA; and (f) sequencing the cDNA library and determining the relative frequency of expression of the at least one RNA biomarker. Optionally, such methods also comprise: (i) removing rRNA from the total RNA prior to step (b); (ii) end repairing the double stranded cDNA and adding an overhanging adenine (A) base to the 3' end of the double stranded cDNA after step (c) and prior to step (d); and/or (iii) purifying and, optionally, size selecting the cDNA in the cDNA library after step (e) and prior to step (f).

In a related embodiment, such methods further comprise the option of synthesizing cDNA by polymerase chain reaction (PCR) using an oligonucleotide primer pair specific for the at least RNA biomarker after step (b) and prior to step (d) or by the standard methods. In certain embodiments, one of the oligonucleotides in the primer pair will be the same as the oligonucleotide primer used in the generation of the first strand cDNA.

In a further embodiment, the relative frequency of expression of the at least one RNA biomarker is determined by: (a) isolating total RNA from a biological sample; (b) generating first strand cDNA from the total RNA; (c) amplifying cDNA by polymerase chain reaction using an oligonucleotide primer pair specific for the at least one RNA biomarker to provide amplified double-stranded cDNA; (d) adding at least one sequencing adapter to the amplified double-stranded cDNA; (e) further amplifying the amplified double-stranded cDNA using primers specific for the at least one sequencing adapter to provide a cDNA library; and (f) sequencing the cDNA library and determining the relative frequency of expression of the at least one RNA biomarker. Optionally, such methods also comprise: (i) removing rRNA from the total RNA prior to step (b); (ii) end repairing the double stranded cDNA and adding an overhanging adenine (A) base to the 3' end of the double stranded cDNA after step (c) and prior to step (d); and/or (iii) purifying and, optionally, size selecting the cDNA in the cDNA library after step (e) and prior to step (f).

In certain embodiments, the disclosed methods comprise determining the expression level of multiple RNA biomarkers corresponding to polynucleotide biomarkers selected from the group consisting of those listed in Tables 1, 2 and 3. Oligonucleotide primers that can be employed in the methods disclosed herein include, but are not limited to, those provided in SEQ ID NO: 76-232 and 293-326. In certain embodiments, the methods disclosed herein include detecting the relative frequency of expression of a RNA biomarker comprising an RNA sequence that corresponds to a DNA sequence of SEQ ID NO: 1-75 and 235-287 or a variant thereof, as defined herein. Those of skill in the art will appreciate that the RNA sequences for the disclosed RNA biomarkers are identical to the cDNA sequences disclosed herein except for the substitution of thymine (T) residues with uracil (U) residues.

The present disclosure provides an oligonucleotide primer comprising, or consisting of, a sequence selected from the group consisting of SEQ ID NO: 76-232 and 293-326, and variants thereof. The disclosure relates to oligonucleotide primers that have a length equal to or less than 30 nucleotides. The disclosed oligonucleotide primers can be effectively employed in methods for diagnosing the presence of, and/or monitoring the progression of, prostate cancer using methods well known to those of skill in the art, including quantitative real time PCR or small scale oligonucleotide microarrays.

Biological samples that can be effectively employed in the disclosed methods include, but are not limited to, urine, blood, serum, cell lines, peripheral blood mononuclear cells (PBMCs), biopsy tissue and prostatectomy tissue.

### BRIEF DESCRIPTION OF THE DRAWINGS

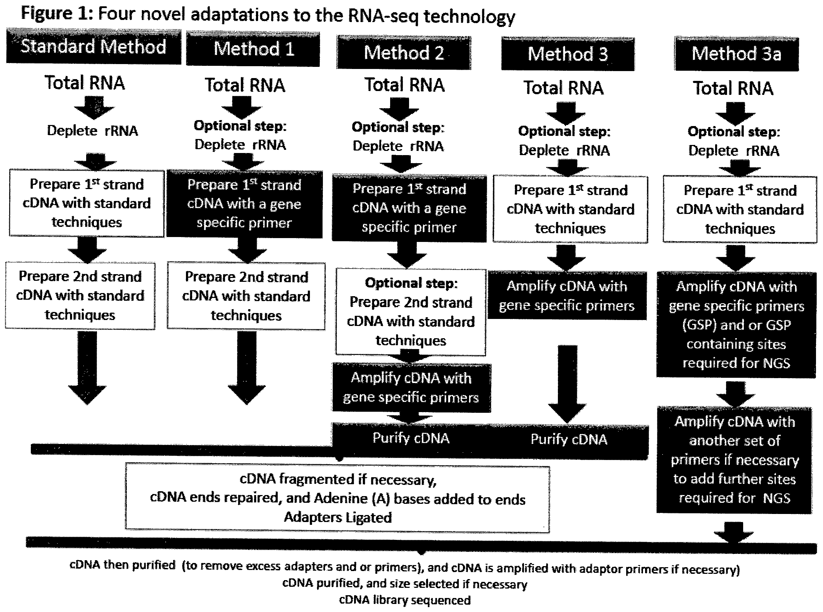

Fig. 1 shows four adaptations to conventional RNA-seq technology that are employed in the disclosed methods.

### DEFINITIONS

As used herein, the term "biomarker" refers to a molecule that is associated either quantitatively or qualitatively with a biological change. Examples of biomarkers include polypeptides, proteins, fragments of a polypeptide or protein; polynucleotides, such as a gene product, RNA or RNA fragment; and other body metabolites.

As used herein, the term "RNA biomarker" or "gene transcript biomarker" refers to an RNA molecule produced by transcription of a gene that is associated either quantitatively or qualitatively with a biological change.

As used herein the term "RNA sequence corresponding to a DNA sequence" refers to a sequence that is identical to the DNA sequence except for the substitution of all thymine (T) residues with uracil (U) residues.

As used herein, the term "oligonucleotide specific for a biomarker" refers to an oligonucleotide that specifically hybridizes to a polynucleotide biomarker or a polynucleotide encoding a polypeptide biomarker, and that does not significantly hybridize to unrelated polynucleotides. In certain embodiments, the oligonucleotide hybridizes to a gene, a gene fragment or a gene transcript. In specific embodiments, the oligonucleotide hybridizes to the polynucleotide of interest under stringent conditions, such as, but not limited to, prewashing in a solution of 6X SSC, 0.2% SDS; hybridizing at 65°C, 6X SSC, 0.2% SDS overnight; followed by two washes of 30 minutes each in 1X SSC, 0.1% SDS at 65°C and two washes of 30 minutes each in 0.2X SSC, 0.1% SDS at 65°C.

As used herein the term "oligonucleotide primer pair" refers to a pair of oligonucleotide primers that span an intron in the cognate RNA biomarker.

As used, herein the term "polynucleotide(s)," refers to a single or double-stranded polymer of deoxyribonucleotide or ribonucleotide bases and includes DNA and corresponding RNA molecules, including hnRNA, mRNA, and non-coding RNA, molecules, both sense and anti-sense strands, and includes cDNA, genomic DNA and recombinant DNA, as well as wholly or partially synthesized polynucleotides. An hnRNA molecule contains introns and corresponds to a DNA molecule in a generally one-to-one manner. An mRNA molecule corresponds to an hnRNA and DNA molecule from which the introns have been excised. A non-coding RNA is a functional RNA molecule that is not translated into a protein, although in some circumstances non-coding RNA can be coding and vice a versa.

As used herein, the term "subject" refers to a mammal, preferably a human, who may or may not have a disorder, such as prostate cancer. Typically, the terms "subject" and "patient" are used interchangeably herein in reference to a human subject.

As used herein, the term "healthy subject" refers to a subject who is not inflicted with a disorder of interest.

As used herein in connection with prostate cancer, the term "healthy male" refers to a male who has an undetectable PSA level in serum or non-rising PSA levels up to 1ng/ml, no evidence of prostate gland abnormality following a DRE and no clinical symptoms of prostatic disorders.

As used herein in connection with prostate cancer, the term "asymptomatic male" refers to a male who has a PSA level in serum of greater than 4ng/ml, which is considered indicative of prostate cancer, but whose DRE is inconclusive and who has no symptoms of clinical disease.

The term "benign prostate hypertrophy" (BPH) refers to a prostatic disease with a non-malignant growth of epithelial cells in the prostate gland and the term "prostatitis" refers to another prostatic disease of the prostate, usually due to a microbial infection of the prostate gland. Both BPH and prostatitis can result in increased PSA levels.

As used herein, the term "metastatic prostate cancer" refers to prostate cancer which has spread beyond the prostate gland to a distant site, such as lymph nodes or bone.
As used herein, the term "biopsy tissue" refers to a sample of tissue (e.g., prostate tissue) that is removed from a subject for the purpose of determining if the sample contains cancerous tissue. The biopsy tissue is then examined (e.g., by microscopy) for the presence or absence of cancer.

As used herein, the term "prostatectomy" refers to the surgical removal of the prostate gland.

As used herein, the term "sample" is used herein in its broadest sense to include a sample, specimen or culture obtained from any source. Biological samples include blood products (such as plasma, serum and whole blood), urine, saliva and the like. Biological samples also include tissue samples, such as biopsy tissues or pathological tissues, that have previously been fixed (e.g., formalin, snap frozen, cytological processing, etc.).

As used herein, the term "predetermined threshold value of expression" of a RNA biomarker refers to the level of expression of the same RNA biomarker in a corresponding control/normal sample or group of control/normal samples obtained from normal, or healthy, subjects, e.g. from males who do not have prostate cancer.

As used herein, the term "altered frequency of expression" of a RNA biomarker in a test biological sample refers to a frequency that is either below or above the predetermined threshold value of expression for the same RNA biomarker in a control sample and thus encompasses either high (increased) or low (decreased) expression levels.

As used herein, the term "relative frequency of expression" refers to the frequency of expression of a RNA biomarker in a test biological sample relative to the frequency of expression of the same RNA biomarker in a corresponding control/normal sample or group of control/normal samples obtained from normal, or healthy, subjects, (e.g., from males who do not have prostate cancer). In preferred embodiments, the frequency of expression of the RNA biomarker is also normalized to the frequency of an internal reference transcript.

As used herein, the term "prognosis" or "providing a prognosis" for a disorder, such as prostate cancer, refers to providing information regarding the likely impact of the presence of prostate cancer (e.g., as determined by the diagnostic methods) on a subject's future health (e.g., the risk of metastasis).

### DETAILED DESCRIPTION

As outlined above, the present disclosure provides methods for detecting the presence or absence of a disorder, such as prostate cancer, in a subject, monitoring progression of the disorder by determining the frequency of expression of specific RNA biomarkers in a biological sample obtained from the subject. The methods disclosed herein employ one or more modifications of standard RNA-seq protocols. RNA-seq is a relatively new technology that has been employed for mass sequencing of whole transcriptomes, and that offers significant advantages over other methods employed for transcriptome sequencing, such as microarrays, including low levels of background noise, the ability to detect low levels of expression, the ability to detect novel mutations and transcripts, and the ability to use relatively small amounts of RNA (for a review of RNA-seq, see Wang et al., Nat. Rev. Genet. (2009) 10:57-63).

The disclosed methods employ oligonucleotides specific for one or more RNA biomarker in combination with RNA-seq technology to perform directed sequencing and thereby determine the relative frequency of expression of the RNA biomarker(s). Such methods have significant advantages over other technologies typically employed to determine expression levels of polynucleotide biomarkers, including improved accuracy, reproducibility and speed, the ability to easily determine the frequency of expression of a multitude of RNA biomarkers in a large number of samples at a relatively low cost, and the ability to identify novel mutations and transcripts.

In specific embodiments, such methods use oligonucleotides specific for one or more biomarkers selected from those shown in Tables 1, 2 and 3.

In one embodiment, the disclosed methods comprise determining the relative frequency of expression levels of at least two, three, four, five, six, seven, eight, nine, ten or more RNA biomarkers selected from the group consisting of: SEQ ID NO: 76-223 and 293-326 in a biological sample taken from a subject, and comparing the relative frequency of expression levels with predetermined threshold values.

The disclosed methods can be employed to diagnose the presence of prostate cancer in subjects with early stage prostate cancer; subjects who have had surgery to remove the prostate (radical prostatectomy); subjects who have had radiation treatment for prostate cancer; subjects who are undergoing, or have completed, androgen ablation therapy; subjects who have become resistant to hormone ablation therapy; and/or subjects who are undergoing, or have had, chemotherapy.

In certain embodiments, the RNA biomarkers disclosed herein appear in subjects with prostate cancer at levels that are at least two-fold higher or lower than, or at least two standard deviations above or below, the mean level in normal, healthy individuals, or are at least two-fold higher or lower than, or at least two standard deviations above or below, a predetermined threshold of expression.

All of the biomarkers and oligonucleotides disclosed herein are isolated and purified, as those terms are commonly used in the art. Preferably, the biomarkers and oligonucleotides are at least about 80% pure, more preferably at least about 90% pure, and most preferably at least about 99% pure.

In certain embodiments, the oligonucleotides employed in the disclosed methods specifically hybridize to a variant of a polynucleotide biomarker disclosed herein. As used herein, the term "variant" comprehends nucleotide or amino acid sequences different from the specifically identified sequences, wherein one or more nucleotides or amino acid residues is deleted, substituted, or added. Variants may be naturally occurring allelic variants, or non-naturally occurring variants. Variant sequences (polynucleotide or polypeptide) preferably exhibit at least 80%, 85%, 90%, 95%, 96%, 97%, 98% or 99% identity to a sequence disclosed herein. The percentage identity is determined by aligning the two sequences to be compared as described below, determining the number of identical residues in the aligned portion, dividing that number by the total number of residues in the inventive (queried) sequence, and multiplying the result by 100.

In addition to exhibiting the recited level of sequence identity, variants of the disclosed biomarkers are preferably themselves expressed in subjects with prostate cancer at a frequency that are higher or lower than the levels of expression in normal, healthy individuals.

Polypeptide and polynucleotide sequences may be aligned, and percentages of identical amino acids or nucleotides in a specified region may be determined against another polypeptide or polynucleotide sequence, using computer algorithms that are publicly available. The percentage identity of a polynucleotide or polypeptide sequence is determined by aligning polynucleotide and polypeptide sequences using appropriate algorithms, such as BLASTN or BLASTP, respectively, set to default parameters; identifying the number of identical nucleic or amino acids over the aligned portions; dividing the number of identical nucleic or amino acids by the total number of nucleic or amino acids of the polynucleotide or polypeptide of the present invention; and then multiplying by 100 to determine the percentage identity.

Two exemplary algorithms for aligning and identifying the identity of polynucleotide sequences are the BLASTN and FASTA algorithms. The alignment and identity of polypeptide sequences may be examined using the BLASTP algorithm. BLASTX and FASTX algorithms compare nucleotide query sequences translated in all reading frames against polypeptide sequences. The FASTA and FASTX algorithms are described in Pearson and Lipman, Proc. Natl. Acad. Sci. USA 85:2444-2448, 1988; and in Pearson, Methods in Enzymol. 183:63-98, 1990. The FASTA software package is available from the University of Virginia, Charlottesville, VA 22906-9025. The FASTA algorithm, set to the default parameters described in the documentation and distributed with the algorithm, may be used in the determination of polynucleotide variants. The readme files for FASTA and FASTX Version 2.0x that are distributed with the algorithms describe the use of the algorithms and describe the default parameters.

The BLASTN software is available on the NCBI anonymous FTP server and is available from the National Center for Biotechnology Information (NCBI), National Library of Medicine, Building 38A, Room 8N805, Bethesda, MD 20894. The BLASTN algorithm Version 2.0.6 [Sep-10-1998] and Version 2.0.11 [Jan-20-2000] set to the default parameters described in the documentation and distributed with the algorithm, is preferred for use in the determination of variants according to the present invention. The use of the BLAST family of algorithms, including BLASTN, is described at NCBI's website and in the publication of Altschul, et al., "Gapped BLAST and PSI-BLAST: a new generation of protein database search programs," Nucleic Acids Res. 25:3389-3402, 1997.

Variant sequences generally differ from the specifically identified sequence only by conservative substitutions, deletions or modifications. As used herein with regards to amino acid sequences, a "conservative substitution" is one in which an amino acid is substituted for another amino acid that has similar properties, such that one skilled in the art of peptide chemistry would expect the secondary structure and hydropathic nature of the polypeptide to be substantially unchanged. In general, the following groups of amino acids represent conservative changes: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his. Variants may also, or alternatively, contain other modifications, including the deletion or addition of amino acids that have minimal influence on the antigenic properties, secondary structure and hydropathic nature of the polypeptide. For example, a polypeptide may be conjugated to a signal (or leader) sequence at the N-terminal end of the protein which co-translationally or post-translationally directs transfer of the protein. The polypeptide may also be conjugated to a linker or other sequence for ease of synthesis, purification or identification of the polypeptide (e.g., poly-His), or to enhance binding of the polypeptide to a solid support. For example, a polypeptide may be conjugated to an immunoglobulin Fc region.

In another embodiment, variant polypeptides are encoded by polynucleotide sequences that hybridize to a disclosed polynucleotide under stringent conditions. Stringent hybridization conditions for determining complementarity include salt conditions of less than about 1 M, more usually less than about 500 mM, and preferably less than about 200 mM. Hybridization temperatures can be as low as 5°C, but are generally greater than about 22°C, more preferably greater than about 30°C, and most preferably greater than about 37°C. Longer DNA fragments may require higher hybridization temperatures for specific hybridization. Since the stringency of hybridization may be affected by other factors such as probe composition, presence of organic solvents and extent of base mismatching, the combination of parameters is more important than the absolute measure of any one alone. An example of "stringent conditions" is prewashing in a solution of 6X SSC, 0.2% SDS; hybridizing at 65°C, 6X SSC, 0.2% SDS overnight; followed by two washes of 30 minutes each in 1X SSC, 0.1% SDS at 65°C and two washes of 30 minutes each in 0.2X SSC, 0.1% SDS at 65°C.

The expression levels of one or more RNA biomarkers in a biological sample can be determined, for example, using one or more oligonucleotides that are specific for the RNA biomarker. In one method, the expression level of one or more RNA biomarkers disclosed herein is determined by first collecting urine from a subject following DRE or prostate massage via a bicycle or exocycle. RNA is isolated from the urine sample, and the frequency of expression of the RNA biomarker is determined as described below using modified RNA-seq technology in combination with oligonucleotides specific for the RNA biomarker of interest.

In other embodiments, the levels of mRNA corresponding to a prostate cancer biomarker disclosed herein can be detected using oligonucleotides in Southern hybridizations, *in situ* hybridizations, or quantitative real-time PCR amplification (qRT-PCR). Solid phase substrates, or carriers, that can be effectively employed in such assays are well known to those of skill in the art and include, but are not limited to, microporous membranes constructed, for example, of nitrocellulose, nylon, polyvinylidene difluoride, polyester, cellulose acetate, mixed cellulose esters and polycarbonate. Suitable microporous membranes include, for example, those described in US Patent Application Publication no. US2010/0093557A1. Methods for performing such assays are well known to those of skill in the art.

The oligonucleotides employed in the disclosed methods are generally single-stranded molecules, such as synthetic antisense molecules or cDNA fragments, and are, for example, 6-60 nt, 15-30 or 20-25 nt in length.

Oligonucleotides specific for a polynucleotide, or RNA, biomarker disclosed herein are prepared using techniques well known to those of skill in the art. For example, oligonucleotides can be designed using known computer algorithms to identify oligonucleotides of a defined length that are unique to the polynucleotide, have a GC content within a range suitable for hybridization, and lack predicted secondary structure that may interfere with hybridization. Oligonucleotides can be synthesized using methods well known to those in the art. In specific embodiments, the oligonucleotides employed in the disclosed methods and compositions are selected from the group consisting of: SEQ ID NO: 76-223 and 293-326.

For tests involving alterations in RNA expression levels, it is important to ensure adequate standardization. Accordingly, in tests such as the adapted RNA-seq technology disclosed herein, quantitative real time PCR or small scale oligonucleotide microarrays, at least one expression standard is employed. Expression standards that can be employed in such methods include, but are not limited to, those listed in Table 3 below.

The present disclosure further provides methods employing a plurality of oligonucleotides that are specific for a plurality of the prostate cancer RNA biomarkers disclosed herein.

The following examples are intended to illustrate, but not limit, this disclosure.

### EXAMPLES

### MATERIALS AND METHODS

### RNA extraction

### a) Cell lines

RNA was isolated from LNCaP and A549 cell lines that had been harvested from cell culture and stored in Trizol using a ZYMO Direct-zol™ kit (Ngaio Diagnostics Ltd.) following the manufacturer's instructions. RNA quality was assessed using the Agilent BioAnalyser and the Agilent RNA 6000 nano assay protocol. The LNCaP and A549 RNA had a RIN value of 9.5 and 9.8 respectively. The RNA was also checked on the NanoDrop 2000 spectrophotometer, (Thermo Scientific), and its concentration ascertained by the Qubit® 2.0 Fluorometer (Life Technologies).

### b) FFPE prostatectomy tissue

Histological blocks from subjects were reviewed by a clinical histopathologist, and tumor and histologically adjacent regions deemed "normal" were identified. These sections were then excised and reset in paraffin. Approximately fifteen freshly cut sections at a thickness of ten microns were then processed using a Qiagen RNeasy FFPE kit (Cat No: 74404, 73504). The method used in all extractions for deparaffinization step was the original method from the Cat no: 74404 kit, and the remainder of the protocol was performed following the manufacturer's instructions. The RNA was checked on the NanoDrop, and its concentration ascertained by the Qubit® 2.0 Fluorometer (Life Technologies).

### c) Urine

RNA was isolated from one or more separate fresh urine samples from donors by sedimentation of the cellular material using centrifgation at 1000g for five minutes at 4°C. The urine was decanted and the cell pellet resuspended in 1.8 ml of ice cold 1X PBS containing 2.5% Fetal Bovine Serum (Invitrogen). The cell suspension was transferred to a 2ml Eppendorf tube and the cellular material collected by centrifugation at 400g for 5 minutes at 4°C. The supernatant was removed (leaving around 50µl) and the cell pellet resuspended in 1.8 ml of ice cold 1X PBS containing 2.5% Fetal Bovine Serum (Invitrogen). The cells were again collected by centrifugation at 400g for 5 minutes at 4°C. The supernatant was removed (leaving around 50µl) and the cell pellet resuspended in 1.8 ml of ice cold 1X PBS containing 2.5% Fetal Bovine Serum (Invitrogen). The cells were collected by centrifµgation at 400g for 5 minutes at 4°C and all but 100 µl of the supernatant removed. The cells were resuspended in the remaining 100 µl of supernatant, and 8µl was taken for microscopic analysis. A total of 300 µl of Trizol LS (Invitrogen) and 5µg of E. coli 5S rRNA was added and the cell suspension was stored at -80°C. RNA was extracted as described by ZYMO using the Direct-zol™ kit, or as described by Invitrogen and further purified using Qiagen RNeasy™ spin columns. RNA was stored at -80°C prior to use.

### cDNA preparation

cDNA was produced from approximately 1-1.5ug of total RNA from either cell lines, biopsy tissue or urine extracts using random primers for the production of the first strand cDNA using the SuperScript® VILO™ cDNA Synthesis Kit (Life Technologies) or RNA biomarker-specific primers.The cDNA preparations were stored at -80°C prior to use and then diluted 1/5 in sterile water prior to qRT-PCR.

### qRT-PCR methods

RNA biomarker specific primers were used to perform real time SYBR green PCR quantification from cell line-, biopsy- or urine-derived cDNA using the Roche Lightcycler 480 using standard protocols for determining the specificity and efficiency of the amplification. The relative amount of the marker gene in each of the samples tested was determined by comparing the cycle threshold (Ct value: number of PCR cycles required for the SYBR green fluorescent signal to cross the threshold exceeding background level within the exponential growth phase of the amplification curve). Following 30 cycle RT-PCR reactions, the amplicons were electrophoresed on a 2% agarose gel and sequenced with standard Sanger chemistry using an Applied Biosystems 3130x1 DNA sequencer.

### RNA biomarker amplicon production

The relative frequency of expression of specific RNA biomarkers was determined using the isolated RNA in one or more of the four methods described below. Each of these methods includes at least one modification of conventional RNA-seq technologies. Conventional RNA-seq technologies are well known to those of skill in the art and are described, for example, in Wang et al. (Nat. Rev. Genet. (2009) 10:57-63), and Marguerat and Bahler (Cell. Mol. Life Sci. (2010) 67:569-579).

### Method 1

In a first method, sequence specific priming is employed during the generation of first strand cDNA. An optional first step in this method is to deplete the total RNA of rRNA using an industry-provided kit, if necessary. An industry-provided first strand cDNA kit is used to combine total RNA or rRNA-depleted total RNA with at least one strand specific oligonucleotide primer (i.e. an oligonucleotide primer specific for the RNA biomarker of interest) and generate first strand cDNA according to the manufacturer's protocol. Second strand cDNA is then synthesized in an unbiased manner using standard techniques. The resulting double-stranded cDNA is fragmented if necessary using standard methods, and the cDNA ends are repaired using standard methods in which any overhangs at the cDNA ends are converted into blunt ends using T4 DNA polymerase. An overhanging adenine (A) base is added to the 3' end of the blunt DNA fragments by the use of Klenow fragment to assist with ligation of adapters required for the sequencing process. The adapters are ligated to the ends of the cDNA fragments using standard procedures, and then the cDNA fragments are run on a gel for purification and removal of excess adapters. The cDNA is amplified using adapter primers, purified, denatured and further diluted for cluster generation and sequencing, for example on a HiSeq2000 according to Illumina Corporation's standard protocols (208 cycles sequencing program, paired-end with indexing). The cDNA library is sequenced, and the relative frequency of expression of the specific RNA biomarkers in cancer patients and healthy controls is determined.

### Method 2

As in method 1, sequence specific priming is employed during the generation of first strand cDNA. This is achieved using an industry provided first strand cDNA kit and at least one strand specific oligonucleotide primer to generate first strand cDNA from total RNA (or rRNA depleted total RNA if necessary) according to the manufacturer's protocol. The second strand cDNA can either be prepared in an unbiased manner using standard techniques, or it can be directly amplified using a set of specific oligonucleotide primers (i.e. oligonucleotide primers specific for the RNA biomarkers of interest) to amplify a specific set of PCR amplicons by either primer limited or cycle limited PCR. In preferred embodiments, the oligonucleotide primer employed to generate the first strand cDNA can be the same as one of the pair of oligonucleotide primers used to amplify the double-stranded cDNA. The cDNA is then purified via a cleanup procedure to remove excess PCR reagents. The cDNA is fragmented if necessary using standard methods, and the cDNA ends are repaired using standard methods in which any overhangs at the cDNA ends are converted into blunt ends using T4 DNA polymerase. An overhanging adenine (A) base is added to the 3' end of the blunt DNA fragments by the use of Klenow fragment to assist with ligation of adapters required for the sequencing process. The adapters are ligated to the ends of the cDNA fragments using standard procedures, and the cDNA fragments are then purified to remove excess adapters. The cDNA is amplified using adapter primers, purified, denatured and further diluted for cluster generation and sequencing, for example on a HiSeq2000 according to Illumina Corporation's standard protocols (208 cycles sequencing program, paired-end with indexing). The cDNA library is sequenced and the relative frequency of expression of the specific RNA biomarkers in cancer patients and healthy controls is determined.

### Method 3

This method employs total RNA or rRNA-depleted RNA if necessary. The first strand cDNA is synthesized using standard methods. The first strand cDNA is then directly amplified using a set of specific oligonucleotide primers (i.e. oligonucleotide primers specific for the RNA biomarkers of interest) to amplify a specific set of PCR amplicons using either primer limited or cycle limited PCR. The cDNA is purified via a cleanup procedure to remove excess PCR reagents. The cDNA is fragmented if necessary using standard methods, and the cDNA ends are repaired using standard methods, in which any overhangs at the cDNA ends are converted into blunt ends using T4 DNA polymerase. An overhanging adenine (A) base is added to the 3'end of the blunt DNA fragments by the use of Klenow fragment to assist with ligation of adapters required for the sequencing process. Adapters are ligated to the ends of the cDNA fragments using standard procedures, and the cDNA is purified to remove excess adapters. The cDNA is then amplified using adapter primers and purified. The cDNA can be size selected via gel electrophoresis using standard methods if necessary. The cDNA library is sequenced, and the relative frequency of expression of the specific RNA biomarkers in cancer patients and healthy controls is determined.

### Method 4

Method 4 differs from Method 3 in that all sequences necessary for next generation sequencing are incorporated via either a one or two step PCR amplification.
An optional first step in this method is to deplete the total RNA of rRNA using an industry-provided kit, if necessary. The first strand cDNA is then synthesized using standard methods. The first strand cDNA is directly amplified using a set of specific oligonucleotide primers (i.e. oligonucleotide primers specific for the RNA biomarkers of interest) also containing Next Generation Sequencing (NGS) primer sites, using either primer limited or cycle limited PCR. The cDNA is then purified via a cleanup procedure to remove excess PCR reagents, and re-amplified with another set of primers, if necessary, in order to add further sites required for NGS using either primer limited or cycle limited PCR. The cDNA is then purified to remove excess PCR reagents and, if necessary, is again amplified using adaptor primers and purified. The cDNA is amplified using adapter primers, purified, denatured and further diluted for cluster generation and sequencing, for example on a HiSeq2000 according to Illumina Corporation's standard protocols (208 cycles sequencing program, paired-end with indexing). The cDNA library is sequenced, and the relative frequency of expression of the specific RNA biomarkers in cancer patients and healthy controls is determined.

### Identification of Prostate Cancer Biomarkers

RNA biomarkers were selected using annotation and analysis of publicly available RNA expression profile data in the NCBI databases GSE6919 and GSE38241 as these data-sets include data from cancer free donors. The biomarkers shown in Table 1 below is a unique set identified as being over-expressed in subjects with prostate cancer. Similarly, the biomarkers shown in Table 2 is a second unique combination of RNA biomarkers identified as being under-expressed in subjects with prostate cancer.

The NCBI database GSE6919, which was developed at the University of Pittsburgh, contains data from three Affymetrix chips (U95A, U95B and U95C), representing more than 36,000 gene reporters. The database, which has been analyzed by Chandran et al. (BMC Cancer 2005, 5:45; BMC Cancer 2007, 9:64), and Yu et al. (J Clin Oncol 2004, 22:2790-2799), contains RNA profiles from more than 200 individual prostate tumor samples, combined with adjacent "normal" or "healthy" tissues, or prostate tissues from individuals believed to be free of prostate cancer.

**Table 1: RNA Biomarkers with Elevated Expression Levels in Prostate Cancer Patients**

| **REPORTER** | **GENBANK ACCESSION** | **GENE DESCRIPTION** | **GENE SYMBOL** | **SEQ ID NO:** | **PRIMER SEQ ID NOS:** | **PRIMER IDS** |
|---|---|---|---|---|---|---|
| 34777_at | D14874 | Adrenomedullin | ADM | 1 | 76, 77 | ND654, ND655 |
| 38827_at | AF038451 | Anterior gradient 2 homolog | AGR2 | 2 | 78, 79 | ND543, ND544 |
| 37399_at | D17793 | Aldo-keto reductase family 1, member C3 | AKR1C3 | 3 | 80, 81 | ND498, ND499 |
| 41764_at | AA976838 | Apolipoprotein C-I | ApoC1 | 4 | 82, 83 | ND414, ND599 |
| 608_at | M12529 | Apolipoprotein E | ApoE | 5 | 84, 85 | CH350, CH351 |
| 1577_at | M23263 | Androgen receptor | AR | 6 | 86, 87 | ND460, ND461, ND532, ND533 |
| | | | | | 88, 89 | |
| 56999_at | AI625959 | Chromosome 15 open reading frame 48 | C15ORF48 | 7 | 90, 91 | CH075, CH076 |
| 36464_at | X94323 | cysteine-rich secretory | CRISP3 | 8 | 92, 93 | ND536, |
| | | protein 3 | | | | ND537 |
| 40201_at | M76180 | Dopa decarboxylase | DDC | 9 | 94, 95 | CH127, CH128 |
| 37156_at | AF070641 | ets variant gene 1 | ETV1 | 10 | 96, 97 | ND440, ND441 |
| 2084_s_at | D12765 | ets variant gene 4 (E1A enhancer binding protein, E1AF) | ETV4 | 11 | 98, 99 | ND410, ND411 |
| 35245_at | M16967 | F5, Coagulation factor V | F5 | 12 | 100, 101 | ND714, ND715 |
| 36622_at | AI989422 | Fibrinogen | FGG | 13 | 102, 103 | ND442, ND443 |
| 36201_at | D13315 | Glycoxalase 1 | GLO1 | 14 | 104, 105 | CH186, CH187 |
| 39135_at | AB018310 | GRAM domain containing 4 | GRAMD4 | 15 | 106, 107 | ND484, ND589 |
| 48885_at | R61847 | Glutamate receptor, ionotropic N-methyl-D-aspartate 3A | GRIN3A | 16 | 108, 109 | CH328, CH329 |
| 1039_s_at | U22431 | Hypoxia inducible factor 1, alpha subunit | HIF-1A | 17 | 110, 111 | ND700, ND701 |
| 37851_at | AF055019 | Homeodomain interacting protein kinase: TF kinase | HIPK2 | 18 | 112, 113 | ND612, ND613 |
| 32480_at | X07495 | Homeobox C4 | HOXC4 | 19 | 114, 115 | ND422, ND423 |
| 56429_at | AI525822 | Homo sapiens hematological and neurological expressed 1 | HN1 | 20 | 116, 117 | ND490, ND491 |
| 32570_at | L76465 | Hydroxyprostaglandin dehydrogenase 15-(NAD) | HPGD | 21 | 118, 119 | ND528, ND529 |
| 37639_at | X07732 | hepsin (transmembrane protease, serine 1) | HPN | 22 | 120, 121 | ND595, ND596 |
| 63673_at | AI635057 | HSBP1 - Heat shock protein 27A | HSBP1 | 23 | 122, 123 | ND702, 703 |
| 1232_s_at | M74587 | Insulin like growth factor binding protein 1 precursor | IGFBP1 | 24 | 124, 125 | ND608, 609 |
| 1804_at | X07730 | kallikrein-related peptidase 3 | KLK3 | 25 | 126, 127 128, 129 | ND438, ND439 ND470, ND471 |
| 217_at, 41721_at | S39329 | kallikrein-related peptidase 2 | KLK2 | 26 | 130, 131 | ND418, ND419 |
| 62175_at | AI50156 | Homo sapiens laminin, alpha 1 | LAMA1 | 27 | 132, 133 | ND662, ND663 |
| 60019_at, 56912_at | AA947309.1 | Leucine rich repeat neuronal 1 - Homo sapiens leucine-rich repeats and calponin homology (CH) domain containing 4 (LRCH4) | LRRN1 | 28 | 134, 135 | ND428, ND429 |
| 1083_s_at, 927_at | M35093 | Mucin1 cell surface associated protein | MUC1 | 29 | 136, 137 | CH284, CH285 |
| 52116_at | AI697679 | Myelin expression factor 2 | MYEF2 | 30 | 138, 139 | ND396, ND397 |
| 35024_at | L37362 | OPRK1 receptor | OPRK1 | 31 | 140, 141 | ND404, ND405 |
| ------ | ------ | *Homo sapiens* SET domain and mariner transposase fusion gene (SETMAR) transcript variant 3, non coding RNA | PCAT1 | 32 | 142, 143 | ND492, ND493 |
| ------ | ------ | *Homo sapiens* uncharacterized LOC100506990, transcript variant 2 non-coding RNA | PCAT14 | 33 | 144, 145 | ND488, ND489 |
| 51776_s_at | AI749525 | PDZK1 interacting protein 1 | PDZK1IP1 | 34 | 146, 147 | ND500, ND501 |
| 31610_at | U21049 | | | | | |
| 59794_g_at | AA872415 | | | | | |
| 41281_s_at | AF060502 | Peroxisomal biogenesis factor 10 | PEX10 | 35 | 148, 149 | CH139, CH140 |
| 40116_at | X16911 | Homo sapiens phosphofructokinase, liver (PFKL) | PFKL | 36 | 150, 151 | ND708, ND709 |
| 39175_at | D25328 | Homo sapiens phosphofructokinase, platelet (PFKP) gene | PFKP | 37 | 152, 153 | ND696, ND697 |
| 41094_at | Y10179 | Prolactin Induced Protein | PIP | 38 | 154, 155 | ND502, ND503 |
| 37068_at | U24577 | phospholipase A2, group VII (platelet-activating factor acetylhydrolase, plasma) | PLA2G7 | 39 | 156, 157 | CH212, CH213 |
| 63958_at | AI583077 | prostate stem cell antigen | PSCA | 40 | 158, 159 | ND380, ND381 |
| 1739_at, 1740_g_at | M99487 | **Prostate**-specific membrane antigen | PSMA | 41 | 160, 161 | ND402, ND403 |
| 33272_at | AA829286 | Serum amyloid A2 | SAA2 | 42 | 162, 163 | CH320, CH321 |
| 36781_at | X01683 | Serpin peptidase inhibitor clade A | SERPINA1 | 43 | 164, 165 | ND446, ND447 |
| 54293_at | N30034 | Solute carrier family 10, member 7 | SLC10A7 | 44 | 166, 167 | ND734, ND735 |
| 39926_at | U59913 | Homo sapiens SMAD family member 5 (SMAD5) | SMAD5 | 45 | 168, 169 | ND710, ND711 |
| 52576_s_at | AW007426 | Spondin 2 extracellular matrix protein | SPON2 | 46 | 170, 171 | ND358, ND359 |
| 34342_s_at | AF052124 | Osteopontin:secreted phophoprotein | SPP1 | 47 | 172, 173 | ND472, ND473 |
| 1938_at | K03218 | Homo sapiens v-src sarcoma (Schmidt-Ruppin A-2) viral oncogene homolog | SRC | 48 | 174, 175 | ND704, ND705 |
| ---- | ----- | Homo sapiens tudor domain containing 1 (TDRD1) | TDRD1 | 49 | 176, 177 | ND726, ND727 |
| 32154_at | M36711 | transcription factor AP-2 alpha (activating enhancer binding protein 2 alpha) | TFAP2A | 50 | 178, 179 | ND494, ND495 |
| 47890_at | AI921465 | Homo sapiens transmembrane channel-like 5 (TMC5) | TMC5 | 51 | 180, 181 | ND670, ND671 |
| 45574_g_at | AA534688 | TPX2-microtubule associated | TPX2 | 52 | 182, 183 | ND436, ND437 |
| 57239_at | AI439109 | Homo sapiens isolate TRIB1-VI-T tribbles-like protein 1 | TRIB1 | 53 | 184, 185 | ND718,719 |
| 56508_at | W22687 | Tetraspanin 13 | TSPAN13 | 54 | 186, 187 | ND386, ND387 |
| 6315_f_at | T50788 | UDP glucuronosyltransferase 2 family polypeptide B15 | UGT2B15 | 55 | 188, 189 | ND452, ND453 |
| 33279_at | X80062 | acyl-CoA synthetase medium-chain family member 3 | ACSM3 | 235 | 293, 294 | |
| | NM_001106.3 | | ACVR2B | 236 | - | |
| 41706_at | AJ130733 | alpha-methylacyl-CoA racemase | AMACR | 237 | - | |
| | NM_000479.3 | | AMH | 238 | - | |
| 36106_at | X01388 | Apolipoprotein C-III | ApoCIII | 239 | - | |
| 31355_at | U77629.1 | Achaete-scute complex homolog 2 | ASCL2 | 240 | - | |
| 56999_at | AI625959 | Chromosome 15 open reading frame 48 | C15ORF4 8 | 241 | - | |
| | NM_178840.2 | | C1orf64 | 242 | 295, 296 | |
| | NM_033150.2 | | COL2A1 | 243 | - | |
| 39925_at | M95610 | collagen, type IX, alpha 2 | COL9A2 | 244 | - | |
| 40162_s_at | AC003107 | Cartilage Oligomeric Matrix protein precursor | COMP | 245 | - | |
| 45399_at | T77033 | Cysteine-rich secretory protein LCCL domain containing 1 | CRISPLD 1 | 246 | 297, 298 | |
| 37020_at | X56692 | C-reactive protein | CRP | 247 | - | |
| 35506_s_at | J03870 | Cystatin S | CST4 | 248 | 299, 300 | |
| 34623_at | M97925 | Defensin alpha 5, Paneth cell specific | DEFA5 | 249 | - | |
| 52138_at | AI351043, AI351043 | v-ets erythroblastosis virus E26 oncogene like (avian) | ERG | 250 | - | |
| 45394_s_at | AA563933 | Family with sequence similarity 3, member D | FAM3D | 251 | 301-304 | |
| 31685_at | Y08976 | FEV (ETS oncogene family) | FEV | 252 | - | |
| | NM_002046.4 | | GAPDH | 253 | - | |
| | | | | | | |
| | NM_0010985 18.1 | | GPR116 | 254 | 305, 306 | |
| 32430_at | M73481 | Gastrin releasing peptide receptor | GRPR | 255 | - | |
| 40327_at | U57052 | homeo box B13 | HOXB13 | 256 | - | |
| 36227_at | AF043129 | Interleukin 7 receptor | IL7R | 257 | - | |
| 46958_at | AI868421 | Potassium voltage gated channel, Shaw-related subfamily, member 2 | KCNC2 | 258 | - | |
| 33606_g_at | AF019415 | NK2 homeobox | NKX2-2 | 259 | - | |
| | | NM_001136157.1 | OTUD5 | 260 | - | |
| | | NR_015342.1 | PCA3 | 261 | 307, 308 | |
| 33703_f_at, 33702_f_at | L05144 | Phophoenol pyruvate carboxy kinase I | PCK1 | 262 | - | |
| 39696_at | AB028974 | Paternally expressed 10 | PEG10 | 263 | - | |
| 58941_at | AI765967 | Phospholipase A1 | PLA1A | 264 | - | |
| 62240_at | AI096692 | Proline rich 16 | PRR16 | 265 | - | |
| 33259_at | M81652 | Semenogelin II | SEMG2 | 266 | 309, 310 | |
| 928_at | L02785 | Solute carrier 26, member 3 | SLC26A3 | 267 | - | |
| 51847_at | AA001450 | Solute carrier family 44, member 5 | SLC44A5 | 268 | 311, 312 | |
| 35716_at | AB008164 | Sulfotransferase | SULT1C2 | 269 | 313, 314 | |
| | NM_003226.3 | | TFF3 | 270 | - | |
| 40328_at | X99268 | TWIST homolog 1 | TWIST1 | 271 | - | |
| 1651_at | U73379 | Ubiquitin-conjugating enzyme E2C | UBE2C | 272 | - | |
| 44403_at | AI873501 | Clone HH0011_E05 mRNA sequence | | 273 | - | |

**Table 2: RNA Biomarkers Showing Reduced Expression Levels in Prostate Cancer Patients**

| **REPORTER** | **GENBANK ACCESSION** | **GENE DESCRIPTION** | **GENE SYMBOL** | **SEQ ID NO:** | **PRIMER SEQ ID NOS:** | **PRIMER ID'S** |
|---|---|---|---|---|---|---|
| 32200_at | M24902 | acid phosphatase, prostate | ACPP | 56 | 190, 191 | ND496, ND497 |
| 35834_at | X59766 | Alpha-2-glycoprotein 1, zinc-binding | AZGP1 | 57 | 192, 193 | CH161, CH162 |
| 36780_at | M25915 | Clusterin | CLU | 58 | 194, 195 | ND698, ND699 |
| 38700_at | M33146 | Cysteine and glycine-rich protein 1 | CSRP1 | 59 | 196, 197, 198, 199 | DR583, DR584, ND690, ND691 |
| 65988_at | W19285 | Early b-cell factor 3 | EBF3 | 60 | 200, 201 | ND730, ND731 |
| 38422_s_at | U29332 | 4.5 LIM domains | FHL2 | 61 | 202, 203 | DR569, DR570 |
| 32749_s_at | AL050396 | filamin A | FLNA | 62 | 204, 205 | ND624, ND625 |
| 53270_s_at | AW021867 | Homo sapiens mitogen-activated protein kinase kinase kinase 7 | MAP3K7 | 63 | 206, 207 | ND682, ND683 |
| 32149_at | AA532495 | microseminoprotein, beta- | MSMB | 64 | 208, 209 | CH143, CH144 |
| 32847_at | U48959 | Myosin kinase | MYLK | 65 | 210, 211 | DR567, DR568 |
| 33505_at, 1042_at, 62940_f_at | AI887421 | Retinoic acid responder | RARRES1 | 66 | 212, 213 | DR575, DR576 |
| | U27185 | | | | | |
| | AI669229 | | | | | |
| 64449_at | AI810399 | Selenoprotein M | SELM1 | 67 | 214, 215 | DR559, DR560 |
| 32521_at | AF056087 | Secreted frizzled-related protein 1 | SFRP1 | 68 | 216, 217 | DR555, DR556 |
| 39544_at | AB002351 | Synemin | SYNM | 69 | 218, 219 | DR579, DR580 |
| 48039_at | AI634580 | Synaptopodin 2 | SYNPO2 | 70 | 220, 221 | DR737,738 |
| 32314_g_at | M75165 | Tropomyosin 2 | TPM2 | 71 | 222, 223 | DR565, DR566 |
| 32755_at | X13839 | Actin SM | ACTA2 | 274 | - | |
| 1197_at | D00654 | Actin gamma2 | ACTG2 | 275 | - | |
| 32527_at | AI381790 | Unknown | C10orf116 | 276 | 315, 316 | |
| 34203_at | D17408 | Calponin 1, basic, smooth muscle | CNN1 | 277 | 317, 318 | |
| 57241_at | AI928870 | Dystrobrevin binding protein 1 | DBNDD2 | 278 | - | |
| 38183_at | U13219 | Forkhead box F1 | FOXF1 | 279 | 319, 320 | |
| 33396_at | U12472 | glutathione S-transferase P1 | GSTP1 | 280 | - | |
| 53796_at | AI819282 | Potassium channel | KCNMA1 | 281 | 321, 322 | |
| 49502_i_at | AI379607 | Mutated in CRC | MCC | 282 | 323, 324 | |
| 767_at | AF001548 | Myosin, heavy chain 11, smooth muscle | MYH11 | 283, 284 | - | |
| 37407_s_at | AF013570 | | | | | |
| 773_at | D10667 | | | | | |
| 774_g_at | D10667 | | | | | |
| 32582_at | X69292 | | | | | |
| 37576_at | U52969 | Purkinje cell protein 4 | PCP4 | 285 | - | |
| 63827_at | AI479999 | Solute carrier family 22, member 17 | SLC22A1 7 | 286 | 325, 326 | |
| | NM_016950. 2 | | SPOCK3 | 287 | | |

For tests measuring the changes in frequency of RNA expression levels, it is essential to ensure adequate standardization. For this reason we have analyzed the NCBI database to identify reporters with the least variation between gene expression profiles, as shown in Table 3 below, in prostate cancer and healthy donor tissues. These reporters form a robust set of RNA expression standards that can be used where appropriate in tests involving quantification of RNA expression, such as in the modified RNA-seq technology described herein.

**Table 3: Reporters with Least Variation between Gene Expression Profiles**

| **REPORTER** | **PROBE** | **GENE SYMBOL** | **GENE DESCRIPTION** | **SEQ ID NO:** | **PRIMER SEQ ID NOS:** | **PRIMER ID'S** |
|---|---|---|---|---|---|---|
| 35184_at | AB011118 | ZFC3H1 | zinc finger, C3H1-type containing CCDC 131 | 72 | 224, 225 | ND514, ND515 |
| 31826_at | AB014574 | FKBP15 | FK506 binding protein 15, 133kDa | 73 | 226, 227 | ND468, ND469 |
| 39811_at | AA402538 | C19orf50 | chromosome 19 open reading frame 50 | 74 | 228, 229, 230, 231 | CH035, CH036, ND505 |
| 33397_at | AL050383 | CDIPT | CDP-diacylglycerol--inositol 3-phosphatidyltransferase | 75 | 231, 232 | CH103, CH104 |
| 36003_at | AJ005698 | PARN | poly(A)-specific ribonuclease (deadenylation nuclease) | 288 | - | |
| 35337_at | AL050254 | FBXO7 | F-box protein 7 | 289 | - | |
| F39020_at | U82938 | SIVA | CD27-binding (Siva) protein polymerase | 290 | - | |
| 36027_at | AA418779 | POLR2F | PDGFA associated protein 1 | 291 | - | |
| 38703_at | AF005050 | DNPEP | Aspartyl aminopeptidase | 292 | - | |

Primers for the production of an RNA biomarker specific amplicon were created using a multistep primer design strategy. Specific intron-spanning primers were created to amplify an amplicon of a specific size (60-300bp) that can be used for Next Generation Sequencing (NGS).

The primers were designed using Primer3 (v. 0.4.0) software and the primers were checked to ensure that certain criteria were met:
- No more than three C's or G's in the last five base pairs;
- No runs (more than three) of G's in either primer;
- No or limited self-complementarity, or hairpin formation; and
- Primer BLAST of the primer set hits the cognate RNA target of the expected size.

In order to use these RNA specific amplicon primer sets for the RNA Biomarker Amplicon Sequencing (RBAS), nucleotides incorporating sequencing primers were added to the 5' end of the primers in the first round PCR as described in Table 4 below, and a second set of primers used for a second round of PCR were used to add further sequences containing an index and adaptor sequence.

**Table 4: Specification of the added sequence to the RNA biomarker specific primer use for the first round PCR for biomarker specific amplicon**

| 1st round PCR | |
|---|---|
| Sequence added to forward primer 5' end | ACGACGCTCTTCCGATCT (SEQ ID NO: 233) |
| Sequence added to reverse primer 5' end | CGTGTGCTCTTCCGATCT (SEQ ID NO: 234) |

All primers used in the studies described herein were designed by the inventors and supplied by Invitrogen or IDT, except for a set of primers for PSA (KLK3) which are taught by Hessels et al. (European Urology 44: 8-16, 2003.

### EXAMPLE 1

### Use of RNA Biomarker Amplicon Sequencing to compare RNA biomarker expression profiles in a prostate adenocarcinoma cell line (LNCaP) and a lung adenocarcinoma cell line (A549)

The ability of RNA Biomarker Amplicon Sequencing (RBAS) to be used for the accurate detection and relative quantification of multiple RNA biomarkers was demonstrated by:
a) producing a selected set of 25 specific RNA biomarker amplicons from LNCaP cells (epithelial cell line derived from androgen-sensitive human prostate adenocarcinoma lymph node metastasis) and A549 cells (epithelial cell line derived from lung alveolar basal tissue); and
b) detecting and measuring the relative abundance of the LNCaP- and A549-derived RNA biomarker specific amplicons by massive parallel sequencing.

### 1) Amplicon production

An amplicon is defined as the specific amplification product obtained by PCR using a pair of oligonucleotide primers targeted to a specific RNA biomarker. The template used for the amplicon production was the single strand DNA complementary to the RNA extracted from LNCaP and A549 cells (see method section above). The cDNA was produced using random primers in this example but biomarker specific primers can also be used to initiate the reverse transcription from the extracted RNA.

DNA amplicons compatible with Illumina Corporation's Next Generation Sequencing technology were produced in this example. Amplicons compatible for sequencing using other NGS technology can also be prepared using the same rationale. The 25 specific primer pairs were targeted to 21 prostate cancer RNA biomarkers and 4 reference RNA biomarkers and contained added sequences for adaptor introduction to the 5' and 3' ends of the amplicons according to Illumina's specification (the RNA biomarker selection and primer design strategies are presented in the method section above).

Technical triplicates for each individual RNA biomarker were produced during a first round of PCR. The same cDNAs produced from RNA of LNCaP or A549 cells were used as a template for each of the three separate first round PCR amplifications. Six amplicon pools were then prepared by combining equal volumes of each of the 25 biomarker specific amplicons produced individually during the first round PCRs. These six amplicon pools, technical triplicates for each of the two cell types, were purified to remove residual primers and dNTPs using Agencourt AMPureXP system (Beckman Coulter, Inc.), and then analyzed with the 2100 Bioanalyser (Agilent Technologies Inc.) and Qubit® 2.0 Fluorometer (Life Technologies) to ascertain quality, average size distribution and the concentration of amplicons in each pool.

### 2) Preparation of amplicon libraries

After dilution, the six cleaned amplicon pools were used as individual templates for the second round PCR performed with sequencing primers specific for the adaptor added during the first round PCR. The sequencing primers also contained a barcode sequence for indexing and a tag sequence for clustering. The amplicon libraries produced during the second round PCR were analyzed and the concentration determined using the 2100 Bioanalyser (Agilent Technologies, Inc.) and Qubit® (Life Technologies - Invitrogen). Residual primers and dNTPs were removed using Agencourt AMPureXP system (Beckman Coulter, Inc.) and then pooled together at equimolar concentration to produce a single amplicon library sequencing pool. The sequencing pool was denatured and further diluted for cluster generation and sequenced on a HiSeq2000 according to Illumina Corporation's standard protocols (208 cycles sequencing program, paired-end with indexing).

### 3) Amplicons relative quantification

Illumina bcl2fastq conversion software (version 1.8.3) was used for the demultiplexing of the sequence reads acquired during the sequencing program and base call conversion to fastq paired end read data. Quality statistics for percentage of bases > Q30 and mean QScore for all reads showed that all amplicon libraries sequenced and de-multiplexed very well. This data set was used to generate the read counts per amplicon (Read counts (Rc) Tables 5 and 6). This is the number of sequencing reads of at least 50bp in length that map to the corresponding amplicon. This number is directly proportional to the amount of the amplicon in the library, and is also proportional to the specific RNA biomarker abundance from which the amplicon was derived.

By using the read count obtained for each amplicon it is thus possible to establish a precise assessment of the relative abundance of the corresponding RNA biomarkers in each sample studied.

Different methods can be used for the normalization of the read count to minimize biases generated by the acquisition of wide count distribution by massive parallel sequencing. The average of the read counts obtained from the four reference amplicons were used to normalize the raw read counts of the amplicons produced from the LNCaP and A549 RNA using the 21 primer pairs specific for the prostate cancer RNA biomarkers. The reference amplicons were made with specific primers targeted to four different RNA biomarkers selected due to their low level of expression variation between different prostate cancer and healthy donor control tissues. The raw counts obtained for the four reference amplicons derived from A549 and LNCaP RNA were consistent between replicates and between the two cell types compared (Table 5). The data confirms the low level of differential expression of these reference RNAs and validates the selection of these RNA biomarkers as reference amplicons.

**Table 5: Read counts obtained in triplicate (Rep. 1, 2, 3) for the four Reference Amplicons (Ref)**

| **a) Reference read Counts from A549 amplicons** | | | | | |
|---|---|---|---|---|---|
| **Ref.** | **Rep.1** | **Rep.2** | **Rep.2** | **Avr.** | **StDev** |
| CDIPT | 520,522 | 513,026 | 531,305 | 242,890 | 13,173 |
| C19orf50 | 209,037 | 211,595 | 210,174 | 210,268 | 1,282 |
| ZFC3HI. | 207,606 | 222,590 | 311,090 | 247,095 | 55,925 |
| FKBP15 | 11,112 | 40,746 | 23,749 | 25,202 | 14,870 |
| **Avr. Ref.** | **237,069** | **246,989** | **269,079** | **160,855** | |
| | | | | | |

| **b) Reference read Counts from LNCaP amplicons** | | | | | |
|---|---|---|---|---|---|
| **Ref.** | **Rep.1** | **Rep.2** | **Rep.2** | **Avr.** | **StDev** |
| CDIPT | 473,707 | 590,290 | 533,300 | 267,674 | 44,723 |
| C19orf50 | 236,952 | 283,338 | 380,160 | 300,150 | 73,069 |
| ZFC3HI. | 96,551 | 201,322 | 160,785 | 152,886 | 52,830 |
| FKBP15 | 37,939 | 80,900 | 39,426 | 52,755 | 24,386 |
| **Avr. Ref**. | **211,287** | **288,962** | **278,418** | **168,597** | |

In Table 6, the normalization of the read count for each of the non-reference RNA biomarker specific amplicons derived from LNCaP and A549 RNA (termed target amplicons) was calculated by dividing each target read count by the average read count calculated from the mean of the four reference amplicons either from LNCaP or A549 RNA. This normalization was performed for each replicate (Table 6: target amplicon read counts/ average references read counts).

The assessment of the RNA biomarker differential expression fold change (FC) between the LNCaP and A549 cells was performed by comparing the normalized read counts per amplicon converted to a log₂ number. The log₂ FC was calculated for the read counts before (raw read counts) and after normalization (Normalised read counts) and was compared in order to assess the effect of the amplicon library count distributions on the evaluation of the differential expression (Table 6). The data in Table 6 compares the expression of 21 target RNA biomarkers in LNCaP and A549 cells. A negative log₂ number indicates a decrease, or down regulation of RNA biomarkers while a positive log₂ number indicates an increase, or up regulation of RNA biomarkers.

**Table 6: Read counts and relative quantification (Log₂ FC) of RNA biomarker specific amplicons derived from LNCaP RNA compared with A549 RNA**

| | **Fold change (FC) calculated with the raw read count (Rc)** | | | | | | **FC calculated with the normalized count normalised read count (Rc)** | | | | |
|---|---|---|---|---|---|---|---|---|---|---|---|
| | | **Rc** | | **Log₂** | | **Log₂ FC LNCaP/ A549** | **Rc** | | **Log**₂ | | **Log₂ FC LNCaP**/ **A549** |
| | | **A549** | **LNCaP** | **A549** | **LNCaP** | | **A549** | **LNCaP** | **A549** | **LNCaP** | |
| **ACPP** | Rep.1 | 108 | 52,877 | 6.8 | 15.7 | **8.9** | 0.0005 | 0.2503 | -11.1 | -2 | 9.1 |
| | Rep.2 | 145 | 51,052 | 7.2 | 15.6 | **8.9** | 0.0006 | 0.1767 | -10.7 | -2.5 | 8.2 |
| | Rep.3 | 143 | 63,492 | 7.2 | 16 | **9.2** | 0.0005 | 0.2280 | -10.9 | -2.1 | 8.7 |
| | **Avr.** | **132** | **55,807** | 7 | **15.8** | **9** | **0.0005** | **0.2183** | **-10.9** | **-2.2** | **8.7** |
| | **Stdev** | **21** | **6,718** | **0.2** | **0.2** | **-0.2** | **0.0001** | **0.0377** | **0.2** | **0.3** | **0.4** |
| **AGR2** | Rep.1 | 676,547 | 48,098 | 19.4 | 15.6 | **-3.8** | 2.8538 | 0.2276 | 1.5 | -2.1 | -3.6 |
| | Rep.2 | 703,769 | 63,188 | 19.4 | 15.9 | **-3.4** | 2.8494 | 0.2187 | 1.5 | -2.2 | -3.7 |
| | Rep.3 | 712,083 | 71,317 | 19.4 | 16.1 | **-3.2** | 2.6464 | 0.2562 | 1.4 | -2 | -3.4 |
| | **Avr.** | **697,466** | **60,868** | **19.4** | **15.9** | **-3.5** | **2.7832** | **0.2342** | **1.5** | **-2.1** | **-3.6** |
| | **Stdev** | **18,587** | **11,782** | **0** | **0.3** | **0.3** | **0.1185** | **0.0196** | **0.1** | **0.1** | **0.2** |
| **AKRI C3** | Rep.1 | 773,556 | 10,121 | 19.6 | 13.3 | **-6.3** | 3.2630 | 0.0479 | 1.7 | -4.4 | -6.1 |
| | Rep.2 | 763,968 | 12,768 | 19.5 | 13.6 | **-5.9** | 3.0931 | 0.0442 | 1.6 | -4.5 | -6.1 |
| | Rep.3 | 721,042 | 16,204 | 19.5 | 14 | **-5.6** | 2.6797 | 0.0582 | 1.4 | -4.1 | -5.5 |
| | **Avr.** | **752,855** | **13,031** | **19.5** | **13.6** | **-5.9** | **3.0119** | **0.0501** | **1.6** | **-4.3** | **-5.9** |
| | **Stdev** | **27,965** | **3,050** | **0.1** | **0.3** | **0.3** | **0.3000** | **0.0073** | **0.1** | **0.2** | **0.3** |
| **AR460** | Rep.1 | 147,236 | 257,216 | 17.2 | 18 | 0.8 | 0.6211 | 1.2174 | -0.7 | 0.3 | 1 |
| | Rep.2 | 145,185 | 272,469 | 17.1 | 18.1 | 0.9 | 0.5878 | 0.9429 | -0.8 | -0.1 | 0.7 |
| | Rep.3 | 146,121 | 237,525 | 17.2 | 17.9 | 0.7 | 0.5430 | 0.8531 | -0.9 | -0.2 | 0.7 |
| | **Avr.** | **146,181** | **255,737** | **17.2** | **18** | **0.8** | **0.5840** | **1.0045** | **-0.8** | **0** | **0.8** |
| | **Stdev** | **1,027** | **17,519** | **0** | **0.1** | **0.1** | **0.0392** | **0.1898** | **0.1** | **0.3** | **0.2** |
| **AR532** | Rep.1 | 267,160 | 1,062,23 0 | 18 | 20 | 2 | 1.1269 | 5.0274 | 0.2 | 2.3 | 2.2 |
| | Rep.2 | 267,201 | 431,144 | 18 | 18.7 | 0.7 | 1.0818 | 1.4920 | 0.1 | 0.6 | 0.5 |
| | Rep.3 | 295,910 | 448,932 | 18.2 | 18.8 | 0.7 | 1.0997 | 1.6124 | 0.1 | 0.7 | 0.6 |
| | **Avr.** | **276,757** | **647,435** | **18.1** | **19.2** | **1.1** | **1.1028** | **2.7106** | **0.1** | 1.2 | 1.1 |
| | **Stdev** | **16,587** | **359,333** | **0.1** | **0.7** | **-0.7** | **0.0227** | **2.0073** | **0** | 1 | 1 |
| **AZGP 1** | Rep.1 | 324 | 129,118 | 8.3 | 17 | **8.6** | 0.0014 | 0.6111 | -9.5 | -0.7 | 8.8 |
| | Rep.2 | 240 | 104,903 | 7.9 | 16.7 | **8.3** | 0.0010 | 0.3630 | -10 | -1.5 | 8.5 |
| | Rep.3 | 308 | 79,348 | 8.3 | 16.3 | **7.9** | 0.0011 | 0.2850 | -9.8 | -1.8 | 8 |
| | **Avr.** | **291** | **104,456** | **8.2** | **16.6** | **8.3** | **0.0012** | **0.4197** | **-9.8** | **-1.3** | **8.4** |
| | **Stdev** | **45** | **24**,**888** | **0.2** | **0.4** | **0.4** | **0.0002** | **0**.**1703** | **0.2** | **0.6** | **0.4** |
| **CRISP 3** | Rep.1 | 74 | 9,068 | 6.2 | 13.1 | **6.9** | 0.0003 | 0.0429 | -11.6 | -4.5 | 7.1 |
| | Rep.2 | 131 | 6,967 | 7 | 12.8 | **6.6** | 0.0005 | 0.0241 | -10.9 | -5.4 | 5.5 |
| | Rep.3 | 302 | 7,297 | 8.2 | 12.8 | **6.6** | 0.0011 | 0.0262 | -9.8 | -5.3 | 4.5 |
| | **Avr.** | **169** | 7,777 | **7.2** | **12.9** | **6.7** | **0.0007** | **0.0311** | **-10.8** | **-5.1** | **5.7** |
| | **Stdev** | **119** | **1,130** | **1** | **0.2** | **0.2** | **0.0004** | **0.0103** | **0.9** | **0.4** | **1.3** |
| **DDC** | Rep.1 | 11,844 | 403,659 | 13.5 | 18.6 | **5.1** | 0.0500 | 1.9105 | -4.3 | 0.9 | 5.3 |
| | Rep.2 | 13,632 | 448,386 | 13.7 | 18.8 | **5.2** | 0.0552 | 1.5517 | -4.2 | 0.6 | 4.8 |
| | Rep.3 | 47,271 | 404,380 | 15.5 | 18.6 | **5.1** | 0.1757 | 1.4524 | -2.5 | 0.5 | 3 |
| | **Avr.** | **24,249** | **418,808** | **14.3** | **18.7** | **5.1** | **0.0936** | **1.6382** | **-3.7** | **0.7** | **4.4** |
| | **Stdev** | **19,958** | **25,618** | **1.1** | **0.1** | **0.1** | **0.0711** | **0.2410** | **1** | **0.2** | **1.2** |
| **ETV1** | Rep.1 | 80,571 | 574,119 | 16.3 | 19.1 | **2.8** | 0.3399 | 2.7172 | -1.6 | 1.4 | 3.0 |
| | Rep.2 | 65,909 | 594,479 | 16 | 19.2 | **2.9** | 0.2668 | 2.0573 | -1.9 | 1 | 2.9 |
| | Rep.3 | 76,805 | 645,353 | 16.2 | 19.3 | **3** | 0.2854 | 2.3179 | -1.8 | 1.2 | 3.0 |
| | **Avr.** | **74,428** | **604,650** | **16.2** | **19.2** | **2.9** | **0.2974** | **2.3642** | **-1.8** | **1.2** | **2.9** |
| | **Stdev** | **7,614** | **36,690** | **0.2** | **0.1** | **0.1** | **0.0379** | **0.3324** | **0.2** | **0.2** | **0** |
| **ETV4** | Rep.1 | 222,417 | 1,426 | 17.8 | 10.5 | -7.3 | 0.9382 | 0.0067 | -0.1 | -7.2 | -7.1 |
| | Rep.2 | 197,816 | 2,018 | 17.6 | 11 | -6.8 | 0.8009 | 0.0070 | -0.3 | -7.2 | -6.8 |
| | Rep.3 | 187,812 | 2,698 | 17.5 | 11.4 | -6.4 | 0.6980 | 0.0097 | -0.5 | -6.7 | -6.2 |
| | **Avr.** | **202,682** | **2,047** | **17.6** | **11** | **-6.8** | **0.8124** | **0.0078** | **-0.3** | -7 | **-6.7** |
| | **Stdev** | **17,808** | **637** | **0.1** | **0.5** | **-0.5** | **0.1205** | **0.0016** | **0.2** | **0.3** | **0.5** |
| **HN1** | Rep.1 | 292,321 | 311,090 | 18.2 | 18.2 | 0.1 | 1.2331 | 1.4724 | 0.3 | 0.6 | 0.3 |
| | Rep.2 | 257,665 | 362,158 | 18 | 18.5 | 0.3 | 1.0432 | 1.2533 | 0.1 | 0.3 | 0.3 |
| | Rep.3 | 246,021 | 348,395 | 17.9 | 18.4 | 0.3 | 0.9143 | 1.2513 | -0.1 | 0.3 | 0.5 |
| | **Avr.** | **265,336** | **340,548** | **18** | **18.4** | **0.2** | **1.0635** | **1.3257** | **0.1** | **0.4** | **0.3** |
| | **Stdev** | **24,084** | **26,423** | **0.1** | **0.1** | **-0.1** | **0.1603** | **0.1270** | **0.2** | **0.1** | **0.1** |
| **MUC1** | Rep.1 | 13,230 | 924 | 13.7 | 9.9 | -3.8 | 0.0558 | 0.0044 | -4.2 | -7.8 | -3.7 |
| | Rep.2 | 13,647 | 902 | 13.7 | 9.8 | -3.9 | 0.0553 | 0.0031 | -4.2 | -8.3 | -4.1 |
| | Rep.3 | 17,202 | 941 | 14.1 | 9.9 | -3.8 | 0.0639 | 0.0034 | -4 | -8.2 | -4.2 |
| | **Avr.** | **14,693** | **922** | **13.8** | **9.8** | **-3.8** | **0.0583** | **0.0036** | **-4.1** | **-8.1** | **-4.3** |
| | **Stdev** | **2,183** | **20** | **0.2** | **0** | **0.1** | **0.0049** | **0.0007** | **0.1** | **0.3** | **0.3** |
| **MYL K** | Rep.1 | 293,518 | 24,448 | 18.2 | 14.6 | -3.6 | 1.2381 | 0.1157 | 0.3 | -3.1 | -3.4 |
| | Rep.2 | 276,460 | 31,241 | 18.1 | 14.9 | -3.2 | 1.1193 | 0.1081 | 0.2 | -3.2 | -3.4 |
| | Rep.3 | 251,537 | 22,665 | 17.9 | 14.5 | -3.7 | 0.9348 | 0.0814 | -0.1 | -3.6 | -3.5 |
| | **Avr.** | **273,838** | **26,118** | **18.1** | **14.7** | **-3.5** | **1.0974** | **0.1017** | **0.1** | **-3.3** | **-3.4** |
| | **Stdev** | **21,113** | **4,525** | **0.1** | **0.2** | **0.2** | **0.1528** | **0.0180** | **0.2** | **0.3** | **0.1** |
| **PCAT 1** | Rep.1 | 114,546 | 386,617 | 16.8 | 18.6 | 1.8 | 0.4832 | 1.8298 | -1 | 0.9 | 1.9 |
| | Rep.2 | 124,881 | 385,426 | 16.9 | 18.6 | 1.8 | 0.5056 | 1.3338 | -1 | 0.4 | 1.4 |
| | Rep.3 | 208,422 | 413,859 | 17.7 | 18.7 | 1.9 | 0.7746 | 1.4865 | -0.4 | 0.6 | 0.9 |
| | **Avr.** | **149,283** | **395,301** | **17.1** | **18.6** | **1.8** | **0.5878** | **1.5500** | **-0.8** | **0.6** | **1.4** |
| | **Stdev** | **51,476** | **16,083** | **0.5** | **0.1** | **0.1** | **0.1622** | **0.2540** | **0.4** | **0.2** | **0.5** |
| **PDZK1 IP1** | Rep.1 | 125,239 | 4,428 | 16.9 | 12.1 | -4.8 | 0.5283 | 0.0210 | -0.9 | -5.6 | -4.7 |
| | Rep.2 | 118,631 | 11,141 | 16.9 | 13.4 | -3.5 | 0.4803 | 0.0386 | -1.1 | -4.7 | -3.6 |
| | Rep.3 | 111,850 | 8,550 | 16.8 | 13.1 | -3.9 | 0.4157 | 0.0307 | -1.3 | -5 | -3.8 |
| | **Avr.** | **118,573** | **8,040** | **16.9** | **12.9** | **-4.1** | **0.4748** | **0.0301** | **-1.1** | **-5.1** | **-4.3** |
| | **Stdev** | **6,695** | **3,385** | **0.1** | **0.7** | **0.7** | **0.0565** | **0.0088** | **0.2** | **0.4** | **0.6** |
| **PEX10** | Rep.1 | 115,769 | 308,004 | 16.8 | 18.2 | 1.4 | 0.4883 | 1.4578 | -1 | 0.5 | 1.6 |
| | Rep.2 | 137,943 | 378,401 | 17.1 | 18.5 | 1.7 | 0.5585 | 1.3095 | -0.8 | 0.4 | 1.2 |
| | Rep.3 | 231,140 | 344,061 | 17.8 | 18.4 | 1.6 | 0.8590 | 1.2358 | -0.2 | 0.3 | 0.5 |
| | **Avr.** | **161,617** | **343,489** | **17.2** | **18.4** | **1.6** | **0.6353** | **1.3343** | **-0.7** | **0.4** | **1.1** |
| | **Stdev** | **61,221** | **35,202** | **0.5** | **0.1** | **0.1** | **0.1969** | **0.1131** | **0.4** | **0.1** | **0.5** |
| **PSCA** | Rep.1 | 4,960 | 24,551 | 12.3 | 14.6 | 2.3 | 0.0209 | 0.1162 | -5.6 | -3.1 | 2.5 |
| | Rep.2 | 2,638 | 27,668 | 11.4 | 14.8 | 2.5 | 0.0107 | 0.0957 | -6.5 | -3.4 | 3.2 |
| | Rep.3 | 2,396 | 23,267 | 11.2 | 14.5 | 2.2 | 0.0089 | 0.0836 | -6.8 | -3.6 | 3.2 |
| | **Avr.** | **3,331** | **25,162** | **11.6** | **14.6** | **2.3** | **0.0135** | **0.0985** | **-6.3** | **-3.4** | **2.9** |
| | **Stdev** | **1,416** | **2,263** | **0.6** | **0.1** | **0.1** | **0.0065** | **0.0165** | **0.6** | **0.2** | **0.4** |
| **SYNM** | Rep.1 | 177,946 | 14,501 | 17.4 | 13.8 | -3.6 | 0.7506 | 0.0686 | -0.4 | -3.9 | -3.5 |
| | Rep.2 | 164,377 | 16,199 | 17.3 | 14 | -3.5 | 0.6655 | 0.0561 | -0.6 | -4.2 | -3.6 |
| | Rep.3 | 154,079 | 14,466 | 17.2 | 13.8 | -3.6 | 0.5726 | 0.0520 | -0.8 | -4.3 | -3.5 |
| | **Avr**. | **165,467** | **15,055** | **17.3** | **13.9** | **-3.6** | **0.6629** | **0.0589** | **-0.6** | **-4.1** | **-3.5** |
| | **Stdev** | **11**,**971** | **991** | **0.1** | **0.1** | **0.1** | **0.0890** | **0.0087** | **0.2** | **0.2** | **0.1** |
| **TFAP 2A** | Rep.1 | 94,299 | 27,021 | 16.5 | 14.7 | -1.8 | 0.3978 | 0.1279 | -1.3 | -3 | -1.6 |
| | Rep.2 | 106,592 | 25,883 | 16.7 | 14.7 | -1.9 | 0.4316 | 0.0896 | -1.2 | -3.5 | -2.3 |
| | Rep.3 | 127,323 | 28,986 | 17 | 14.8 | -1.7 | 0.4732 | 0.1041 | -1.1 | -3.3 | -2.2 |
| | **Avr.** | **109,405** | **27,297** | **16.7** | **14.7** | **-1.8** | **0.4342** | **0.1072** | **-1.2** | **-3.2** | **-2.0** |
| | **Stdev** | **16**,**691** | **1,570** | **0.2** | **0.1** | **0.1** | **0.0378** | **0.0193** | **0.1** | **0.3** | **0.3** |
| **TPM2** | Rep.1 | 647,658 | 18,974 | 19.3 | 14.2 | -5.1 | 2.7319 | 0.0898 | 1.4 | -3.5 | -4.9 |
| | Rep.2 | 571,092 | 21,325 | 19.1 | 14.4 | -4.9 | 2.3122 | 0.0738 | 1.2 | -3.8 | -5 |
| | Rep.3 | 570,539 | 27,813 | 19.1 | 14.8 | -4.5 | 2.1203 | 0.0999 | 1.1 | -3.3 | -4.4 |
| | **Avr**. | **596,430** | **22,704** | **19.2** | **14.5** | **-4.9** | **2.3882** | **0.0878** | **1.2** | **-3.5** | **-4.8** |
| | **Stdev** | **44,366** | **4,578** | **0.1** | **0.3** | **0.3** | **0.3128** | **0.0132** | **0.2** | **0.2** | **0.3** |
| **UGT2 B15** | Rep.1 | 524 | 317,083 | 9 | 18.3 | 9.2 | 0.0022 | 1.5007 | -8.8 | 0.6 | 9.4 |
| | Rep.2 | 535 | 154,557 | 9.1 | 17.2 | 8.2 | 0.0022 | 0.5349 | -8.9 | -0.9 | 7.9 |
| | Rep.3 | 2,478 | 294,434 | 11.3 | 18.2 | 9.1 | 0.0092 | 1.0575 | -6.8 | 0.1 | 6.8 |
| | **Avr.** | **1,179** | **255,358** | **9.8** | **17.9** | **8.9** | **0.0045** | **1.0310** | **-8.1** | **-0.1** | **8.1** |
| | **Stdev** | **1,125** | **88,028** | **1.3** | **0.6** | **0.6** | **0.0041** | **0.4835** | **1.2** | **0.8** | **1.3** |

The data shows that the difference between FC values calculated either using the log₂ value for raw counts or the log₂ value for the normalized counts is not large. However, the normalization process allows a more accurate detection of the relative difference in expression of RNA biomarkers in A549 and LNCaP cells.

For the data in Table 7 we have accepted Log₂ FC values greater than 2 are significant and grouped the expression levels of the 21 prostate cancer specific RNA biomarkers tested using LNCaP and A549 RNA in two groups: Log₂FC >2; and Log₂FC <2.

**Table 7: Comparison of Log₂ FC expression levels of RNA biomarkers in LNCaP and A549 RNA**

| **Log₂ Fc** | **Elevated expression in LNCaP RNA** | **Elevated expression in A549 RNA** |
|---|---|---|
| **Log₂ Fc>2** | ACPP, AZGP1, CRISP3, DDC, UGT2B15, ETV1 PSCA | AKRIC3, ETV4 , MUC1,PDZK1IP1, TPM2, AGR2, MYLK,, SYNM |
| **Log₂ Fc<2** | AR460, AR532, HN1, PCAT1, PEX10 | TFAP2A |

The data reveals an even split of RNA biomarkers with Log₂ FC >2 between the two RNAs.

The data contained in Table 8 are basic statistical analyses of the Log₂ FC differences between the 21 RNA biomarkers expressed in LNCaP and A549 RNA calculated by dividing the normalized Log₂ FC of each RNA biomarker from LNCaP RNA by the corresponding Log₂ FC from A549 RNA. The level of differential expression calculated by the limma-based linear model fit analysis (T= limma moderated t- statistic) highlights some significant levels of differential expression of the RNA biomarker between the LNCaP and A549 cell types (T value) with correlating P value.

**Table 8: Significance levels comparing the differential expression of each RNA biomarker between LNCaP and A549 cells**

| **Target** | **Log₂ FC difference** | **t** | **P.Value** | **adj.P.Val** |
|---|---|---|---|---|
| **ACPP** | 8.7 | 30 | 9.E-14 | 2.E-12 |
| **AZGP1** | 8.4 | 24 | 3.E-12 | 6.E-11 |
| **UGT2B15** | 8.1 | 15 | 1.E-09 | 2.E-08 |
| **ETV4** | -6.7 | -24 | 2.E-12 | 6.E-11 |
| **AKRIC3** | -5.9 | -22 | 6.E-12 | 1.E-10 |
| **CRISP3** | 5.7 | 13 | 4.E-09 | 7.E-08 |
| **TPM2** | -4.9 | -17 | 1.E-10 | 3.E-09 |
| **DDC** | 4.4 | --10 | 2.E-07 | 2.E-06 |
| **MUC1** | -4.3 | -15 | 9.E-10 | 2.E-08 |
| **PDZKIP1** | -4.3 | -14 | 2.E-09 | 3.E-08 |
| **AGR2** | -3.6 | -14 | 2.E-09 | 3.E-08 |
| **SYNM** | -3.5 | -13 | 5.E-09 | 9.E-08 |
| **MYLK** | -3.4 | -13 | 7.E-09 | 1.E-07 |
| **PSCA** | 2.9 | 7 | 5.E-06 | 5.E-05 |
| **ETV1** | 2.9 | 9 | 3.E-07 | 4.E-06 |
| **TFAP2A** | -2.0 | -8 | 2.E-06 | 2.E-05 |
| **PCAT1** | 1.4 | 4 | 2.E-03 | 2.E-02 |
| **AR532** | 1.1 | 2 | 8.E-02 | 5.E-01 |
| **AR460** | 0.8 | 2 | 9.E-02 | 5.E-01 |
| **PEX10** | 1.1 | 3 | 1.E-02 | 9.E-02 |
| **HN1** | 0.3 | 0 | 8.E-01 | 1.E+00 |

These two cell lines, LNCAP and A549, were chosen for this example to demonstrate a proof of concept by comparing RNA biomarker expression in two cell lines; one (LNCaP cells) of prostate origin and the other (A549 cells) of lung origin. As might be expected, there is significant differential expression between these two cell lines of the RNA biomarkers chosen on the basis of their possible involvement in prostate cancer.

The data provided in the above example shows that it is possible to detect the change in expression of specific RNA biomarkers through quantitative amplicon synthesis followed by enumeration using a Next Generation DNA sequencing methodology.

### EXAMPLE 2

### RNA amplicon biomarker sequencing (RBAS) in the analysis of differential gene expression profile using prostate cancer tissue from formalin-fixed paraffin embedded (FFPE) human prostatectomy tissue

This example demonstrates that the RNA amplicon biomarker sequencing (RBAS) method is diagnostically and prognostically relevant by quantifying the relative expression of 79 RNA biomarkers using amplicon production and NGS to establish their RNA expression profile in prostate cancer tissues.

Stored formalin-fixed paraffin embedded (FFPE) prostatectomy tissue blocks were reviewed by a clinical histopathologist to select tissues for analysis. Prostatectomy tissue from two subjects was selected.

Subject 1 is a 63 year old male who underwent a prostate biopsy in 2007 and was diagnosed with prostate cancer with a Gleason score of 4+5. The subject underwent a radical prostatectomy at the age of 58. A stored FFPE block containing the original prostatectomy tissue was re-examined and a tumor region was identified with a Gleason score of 4+5. The region identified was reset in paraffin and then sectioned. Three tissue samples were selected from Subject 1 for RNA extraction: Tumor tissue 4+5 (T); adjacent glandular tissue (Adj.G); and adjacent muscle tissue (Adj.M) deemed histologically normal.

Subject 2 is a 67 year old male who underwent a prostate biopsy in 2012 and was diagnosed with prostate cancer with a Gleason score of 3+4. The subject underwent a radical prostatectomy at the age of 66. A stored FFPE block containing the prostatectomy tissue was re-examined. Three tumors were identified with different Gleason scores, 4+5 (T1), 3+4 (T2) and 3+3 (T3) respectively. The different regions from the blocks were reset, and then sectioned. Tissue samples were selected from each of the three tumor regions as well as an adjacent glandular tissue (Adj.G) deemed histologically normal. No Adj.M region was identified in Subject 2 tissue samples.

Total RNA was extracted separately from the seven selected tissue samples from Subject 1 and 2 using a Qiagen FFPE RNeasy extraction kit (Cat No: 74404, 73504). The RNA was then used to generate cDNA for each tissue sample as described above in the methods section. This cDNA was used for amplicon production in triplicate, using a total of 79 RNA biomarker primer pairs that included five reference amplicons from four RNA biomarkers. The second round PCR sequencing of the 79 RNA biomarker specific amplicons produced in the first round PCR was done in two separate runs. During the second round PCR, the barcode sequence for indexing and a tag sequence were added and the amplicon libraries were pooled together for clustering and sequencing on the Illumina Hiseq2500 instrument as described in Example 1.

As described in Example 1, Illumina bcl2fastq conversion software (version 1.8.3) was used to obtain the number of sequence reads per amplicon (read counts).

The raw counts of the five reference amplicons from each of the sequencing runs (Run1, Run2) is presented in Table 9. The sequence counts for all the reference amplicons were lower in run 1 than the run 2. However, the ratio of the individual reference RNA biomarkers to each other was very similar in the two runs.

**Table 9A: Subject 1 - Average of raw counts for the triplicates for reference amplicons tested in triplicates from Tumor (T) and adjacent glandular (AdjG) or adjacent muscular (AdjM) RNA samples**

| | **T** | | **Adj.G** | | **Adj.M** | |
|---|---|---|---|---|---|---|
| **Run 1** | **Avr.** | **StDev** | **Avr** | **StDev** | **Avr.** | **StDev** |
| **CDIPT** | 181,602 | 108,375 | 69,387 | 25,776 | 109,665 | 22,597 |
| **FKBP15** | 26,420 | 14,819 | 14,726 | 5,349 | 19,283 | 9,148 |
| **ZFC3H1** | 26,996 | 13,809 | 11,019 | 4,804 | 10,355 | 5,742 |
| **C19orf50.35/36** | 11,518 | 5,887 | 4,873 | 1,696 | 7,909 | 3,387 |
| **C19orf50.35/505** | 11,484 | 5,941 | 4,892 | 1,738 | 8,029 | 3,384 |
| **Avr.** | 51,604 | 28,926 | 20,979 | 6,330 | 31,048 | 7,989 |

| | **T** | | **Adj.G** | | **Adj.M** | |
|---|---|---|---|---|---|---|
| **Run** 2 | **Avr**. | **StDev** | **Avr** | **StDev** | **Avr.** | **StDev** |
| **CDIPT** | 579,696 | 428,581 | 392,492 | 26,856 | 312,658 | 28,339 |
| **FKBP15** | 107,916 | 67,181 | 91,199 | 4,604 | 52,760 | 10,832 |
| **ZFC3H1** | 164,089 | 104,640 | 75,341 | 2,445 | 82,436 | 13,887 |
| **C19orf50.35/36** | 39,019 | 27,178 | 33,147 | 6,143 | 23,112 | 5,425 |
| **C19orf50 .35/505** | 39,049 | 26,955 | 32,880 | 6,194 | 23,372 | 5,712 |
| **Avr.** | 185,954 | 130,620 | 125,012 | 5,966 | 98,868 | 6,648 |
| | | | | | | |

**Table 9B: Subject 2 - Average raw counts for the triplicate reference amplicons from Tumors (T1,T2 and T3) and adjacent glandular (Adj.G) RNA samples**

| | **T1** | | **Adj.G** | | **T2** | |
|---|---|---|---|---|---|---|
| **Run 1** | Avr. | **StDev** | **Avr** | **StDev** | **Avr.** | **StDev** |
| **CDIPT** | 141,808 | 57,175 | 108,540 | 13,054 | 157,843 | 84,787 |
| **FKBP15** | 32,004 | 1,364 | 11,053 | 9,047 | 11,090 | 2,664 |
| **ZFC3H1** | 25,860 | 7,845 | 21,315 | 10,432 | 21,694 | 9,172 |
| **C19orf50 35/36** | 5,514 | 368 | 3,478 | 699 | 4,377 | 2,372 |
| **C19orf50 35/505** | 5,578 | 246 | 3,418 | 792 | 4,278 | 2,306 |
| **Avr.** | 42,153 | 13,400 | 29,561 | 1,977 | 39,856 | 19,405 |

| | **T3** | | **Adj.G** | | **T2** | |
|---|---|---|---|---|---|---|
| **Run 2** | Avr. | StDev | Avr | StDev | Avr. | StDev |
| **CDIPT** | 453,616 | 163,307 | 482,506 | 80,991 | 444,554 | 19,270 |
| **FKBP15** | 82,124 | 40,266 | 69,754 | 10,656 | 90,864 | 19,203 |
| **ZFC3H1** | 124,362 | 54,650 | 99,653 | 31,461 | 138,021 | 19,628 |
| **C19orf50 (35/36)** | 14,934 | 5,048 | 11,097 | 4,414 | 20,073 | 8,693 |
| **C19orf50 (35/505)** | 14,997 | 5,010 | 11,129 | 4,241 | 20,223 | 8,519 |
| **Avr.** | 138,007 | 50,711 | 134,828 | 20,977 | 142,747 | 10,484 |

The raw counts obtained for the reference amplicons presented in Table 9 were generally consistent between replicates across the prostatectomy-derived RNA samples and the data supports the selection of these RNA biomarkers as reference amplicons.

The average of the read counts from the five reference amplicons was used to normalize the raw read counts of the amplicons produced from the appropriate tumor and adjacent glandular and muscular tissue pairings.

### Subject 1 RNA Biomarker Analysis

For the analysis of Subject 1, the data compared the relative expression of the RNA biomarkers between tumor tissue and both adjacent glandular and adjacent muscular tissue. The raw counts of triplicate samples from tumor tissue and both adjacent glandular and adjacent muscular tissue is given followed by the log₂ normalized counts. The log₂ FC expression of each RNA biomarker from the tumor region of the prostatectomy tissue RNA samples is given relative to the adjacent glandular and muscular adjacent muscular tissue RNA. Finally the log₂ FC of the adjacent glandular relative to the muscular adjacent muscular tissue RNA is presented (Table 10).

Those RNA biomarkers with a differential amplicon count (Log₂ FC >2) from Subject 1 were selected from the tumor, adjacent glandular and adjacent muscular samples with the data being presented in Table 11.

**Table 10: Subject 1 - Raw read counts, Log₂ normalization of the read counts and relative quantification (Log₂ FC) of RNA biomarker specific amplicons**

| | | **Raw read counts (Rc)** | | | **Log₂ Normalised Rc** | | | **Differential Expression (Log₂ FC)** | | |
|---|---|---|---|---|---|---|---|---|---|---|
| | | **T** | **Adj**.**G** | **Adj.M** | **T** | **Adj.G** | **Adj**.**M** | **T/ Adj.G** | **T/ Adj.M** | **Adj**.**G/ Adj.M** |
| **ACPP** | Rep.1 | 218,083 | 640,127 | 31,967 | 2.94 | 5.24 | 0.51 | -2.30 | 2.43 | 4.734 |
| | Rep.2 | 163,669 | 656,380 | 30,575 | 1.95 | 5.21 | -0.33 | -3.26 | 2.27 | 5.534 |
| | Rep.3 | 700,788 | 883,399 | 31,581 | 3.06 | 4.97 | -0.04 | -1.91 | 3.10 | 5.001 |
| | Avr. | 360,847 | 726,635 | 31,374 | 2.65 | 5.14 | 0.05 | -2.49 | 2.60 | 5.09 |
| | StDv | 295,652 | 136,004 | 719 | 0.61 | 0.15 | 0.42 | 0.70 | 0.44 | 0.407 |
| **AGR2** | Rep.1 | 131,239 | 31,120 | 6,276 | 2.21 | 0.88 | -1.84 | 1.33 | 4.05 | 2.72 |
| | Rep.2 | 162,340 | 35,938 | 4,981 | 1.94 | 1.02 | -2.94 | 0.92 | 4.88 | 3.961 |
| | Rep.3 | 476,179 | 49,861 | 3,389 | 2.50 | 0.82 | -3.26 | 1.68 | 5.76 | 4.074 |
| | Avr. | 256,586 | 38,973 | 4,882 | 2.22 | 0.91 | -2.68 | 1.31 | 4.90 | 3.585 |
| | StDv | 190,808 | 9,732 | 1,446 | 0.28 | 0.10 | 0.74 | 0.38 | 0.85 | 0.751 |
| **AKR1C3** | Rep.1 | 7,565 | 7,573 | 11,688 | -1.91 | -1.16 | -0.94 | -0.75 | -0.97 | -0.22 |
| | Rep.2 | 11,053 | 8,093 | 27,577 | -1.94 | -1.13 | -0.47 | -0.81 | -1.47 | -0.66 |
| | Rep.3 | 25,510 | 11,563 | 19,632 | -1.72 | -1.29 | -0.72 | -0.43 | -1.00 | -0.57 |
| | Avr. | 14,709 | 9,076 | 19,632 | -1.86 | -1.19 | -0.71 | -0.66 | -1.14 | -0.48 |
| | StDv | 9,515 | 2,169 | 7,945 | 0.12 | 0.08 | 0.24 | 0.20 | 0.28 | 0.234 |
| **ADM** | Rep.1 | 383 | 177 | 45 | -6.21 | -6.58 | -8.96 | 0.37 | 2.75 | 2.386 |
| | Rep.2 | 6,725 | 794 | 2,117 | -2.66 | -4.48 | -4.18 | 1.83 | 1.52 | -0.31 |
| | Rep.3 | 3,618 | 497 | 34 | -4.54 | -5.83 | -9.89 | 1.29 | 5.36 | 4.064 |
| | Avr. | 3,575 | 489 | 732 | -4.47 | -5.63 | -7.68 | 1.16 | 3.21 | 2.049 |
| | StDv | 3,171 | 309 | 1,199 | 1.78 | 1.06 | 3.07 | 0.74 | 1.96 | 2.204 |
| **AR(460)** | Rep.1 | 87,414 | 63,945 | 64,627 | 1.62 | 1.92 | 1.52 | -0.30 | 0.10 | 0.395 |
| | Rep.2 | 106,349 | 75,612 | 98,985 | 1.33 | 2.09 | 1.37 | -0.76 | -0.04 | 0.721 |
| | Rep.3 | 201,173 | 84,483 | 62,643 | 1.26 | 1.58 | 0.95 | -0.32 | 0.31 | 0.626 |
| | Avr. | 131,645 | 74,680 | 75,418 | 1.40 | 1.86 | 1.28 | -0.46 | 0.12 | 0.581 |
| | StDv | 60,952 | 10,301 | 20,433 | 0.19 | 0.26 | 0.30 | 0.26 | 0.18 | 0.168 |
| **AR(532)** | Rep.1 | 42,868 | 43,461 | 22,464 | 0.59 | 1.36 | 0.00 | -0.77 | 0.59 | 1.363 |
| | Rep.2 | 67,215 | 21,630 | 28,560 | 0.67 | 0.29 | -0.42 | 0.38 | 1.09 | 0.709 |
| | Rep.3 | 111,816 | 60,319 | 43,444 | 0.41 | 1.09 | 0.43 | -0.68 | -0.01 | 0.668 |
| | Avr. | 73,966 | 41,803 | 31,489 | 0.56 | 0.91 | 0.00 | -0.36 | 0.56 | 0.913 |
| | StDv | 34,966 | 19,398 | 10,792 | 0.13 | 0.56 | 0.42 | 0.64 | 0.55 | 0.39 |
| **AZGP1** | Rep.1 | 198,131 | 545,971 | 35,292 | 2.80 | 5.01 | 0.65 | -2.21 | 2.15 | 4.362 |
| | Rep.2 | 104,449 | 650,870 | 23,844 | 1.30 | 5.20 | -0.68 | -3.90 | 1.98 | 5.88 |
| | Rep.3 | 672,265 | 871,798 | 40,138 | 3.00 | 4.95 | 0.31 | -1.95 | 2.69 | 4.636 |
| | Avr. | 324,948 | 689,546 | 33,091 | 2.37 | 5.05 | 0.09 | -2.68 | 2.28 | 4.959 |
| | StDv | 304,410 | 166,321 | 8,367 | 0.93 | 0.13 | 0.69 | 1.06 | 0.37 | 0.809 |
| **CLU** | Rep.1 | 26,673 | 24,462 | 48,500 | -0.09 | 0.53 | 1.11 | -0.62 | -1.20 | -0.58 |
| | Rep.2 | 36,616 | 30,951 | 103,633 | -0.21 | 0.80 | 1.44 | -1.01 | -1.65 | -0.63 |
| | Rep.3 | 92,251 | 52,909 | 71,777 | 0.13 | 0.90 | 1.15 | -0.77 | -1.01 | -0.25 |
| | Avr. | 51,847 | 36,107 | 74,637 | -0.06 | 0.75 | 1.23 | -0.80 | -1.29 | -0.49 |
| | StDv | 35,343 | 14,908 | 27,678 | 0.18 | 0.19 | 0.18 | 0.20 | 0.33 | 0.21 |
| **CRISP3** | Rep.1 | 13,110 | 984 | 266 | -1.12 | -4.10 | -6.40 | 2.99 | 5.29 | 2.298 |
| | Rep.2 | 17,388 | 4 | 10 | -1.29 | -12.12 | -11.90 | 10.83 | 10.62 | -0.21 |
| | Rep.3 | 17,838 | 143 | 36 | -2.24 | -7.63 | -9.81 | 5.39 | 7.58 | 2.185 |
| | Avr. | 16,112 | 377 | 104 | -1.55 | -7.95 | -9.37 | 6.40 | 7.83 | 1.423 |
| | StDv | 2,610 | 530 | 141 | 0.60 | 4.02 | 2.78 | 4.02 | 2.67 | 1.418 |
| **DDC** | Rep.1 | 49 | 1 | 2 | -9.18 | -14.05 | -13.46 | 4.87 | 4.28 | -0.59 |
| | Rep.2 | 1 | 1 | 1 | -15.37 | -14.12 | -15.23 | -1.26 | -0.15 | 1.11 |
| | Rep.3 | 199 | 601 | 670 | -8.72 | -5.56 | -5.59 | -3.17 | -3.13 | *0.038* |
| | **Avr**. | 83 | 201 | 224 | -11.09 | -11.24 | -11.43 | 0.15 | 0.33 | 0.186 |
| | **StDv** | 103 | 346 | 386 | 3.71 | 4.92 | 5.13 | 4.20 | 3.73 | 0.859 |
| **ETV1** | Rep.1 | 323,226 | 19,968 | 28,271 | 3.51 | 0.24 | 0.33 | 3.27 | 3.18 | -0.09 |
| | Rep.2 | 470,090 | 16,096 | 42,166 | 3.47 | -0.14 | 0.14 | 3.61 | 3.33 | -0.28 |
| | Rep.3 | 697,535 | 24,370 | 28,491 | 3.05 | -0.21 | -0.18 | 3.27 | 3.24 | -0.03 |
| | **Avr**. | 496,950 | 20,145 | 32,976 | 3.34 | -0.04 | 0.10 | 3.38 | 3.25 | -0.13 |
| | **StDv** | 188,595 | 4,140 | 7,960 | 0.25 | 0.24 | 0.26 | 0.20 | 0.08 | 0.13 |
| **ETV4** | Rep.1 | 501 | 1,011 | 829 | -5.83 | -4.06 | -4.76 | -1.76 | -1.06 | 0.697 |
| | Rep.2 | 2 | 871 | 2 | -14.37 | -4.35 | -14.23 | -10.02 | -0.15 | 9.876 |
| | Rep.3 | 1,636 | 571 | 10 | -5.68 | -5.63 | -11.66 | -0.05 | 5.98 | 6.03 |
| | **Avr**. | 713 | 818 | 280 | -8.63 | -4.68 | -10.22 | -3.95 | 1.59 | 5.534 |
| | **StDv** | 837 | 225 | 475 | 4.98 | 0.83 | 4.89 | 5.33 | 3.83 | 4.61 |
| **FLNA** | Rep.1 | 427,572 | 338,722 | 869,661 | 3.91 | 4.32 | 5.27 | -0.41 | -1.36 | -0.95 |
| | Rep.2 | 374,615 | 451,638 | 1,877,290 | 3.14 | 4.67 | 5.62 | -1.53 | -2.47 | -0.95 |
| | Rep.3 | 1,169,697 | 462,865 | 1,064,855 | 3.80 | 4.03 | 5.04 | -0.23 | -1.24 | -1.01 |
| | **Avr**. | 657,295 | 417,742 | 1,270,602 | 3.62 | 4.34 | 5.31 | -0.72 | -1.69 | -0.97 |
| | **StDv** | 444,543 | 68,663 | 534,395 | 0.41 | 0.32 | 0.29 | 0.70 | 0.68 | 0.034 |
| **GLOI** | Rep.1 | 215272 | 35,392 | 28,114 | 0.62 | 1.33 | 1.78 | 2.42 | 2.40 | 0.46 |
| | Rep.2 | 132276 | 53,092 | 31,252 | 0.65 | 1.00 | 1.58 | 1.96 | 2.23 | 0.31 |
| | Rep.3 | 487668 | 76,360 | 29,474 | 0.55 | 0.65 | 1.85 | 1.20 | 2.40 | 0.00 |
| | Avr. | 278405 | 54948 | 29613 | 0.61 | 0.99 | 1.74 | 1.86 | 2.34 | 0.26 |
| | StDv | 185917 | 20547 | 1574 | 0.05 | 0.34 | 0.14 | 0.62 | 0.10 | 0.23 |
| **HN1** | Rep.1 | 3,784 | 1,871 | 147 | -2.91 | -3.18 | -7.26 | 0.27 | 4.35 | 4.08 |
| | Rep.2 | 2,614 | 2,796 | 4,995 | -4.02 | -2.67 | -2.94 | -1.35 | -1.08 | 0.273 |
| | Rep.3 | 6,432 | 4,393 | 1,246 | -3.71 | -2.69 | -4.70 | -1.02 | 0.99 | 2.013 |
| | **Avr**. | 4,277 | 3,020 | 2,129 | -3.55 | -2.84 | -4.96 | -0.70 | 1.42 | 2.122 |
| | **StDv** | 1,956 | 1,276 | 2,542 | 0.57 | 0.29 | 2.17 | 0.86 | 2.74 | 1.906 |
| **HPGD** | Rep.1 | 10,885 | 6,589 | 11,129 | -1.38 | -1.36 | -1.01 | -0.02 | -0.37 | -0.35 |
| | Rep.2 | 22,378 | 12,952 | 13,946 | -0.92 | -0.45 | -1.46 | -0.47 | 0.54 | 1.003 |
| | Rep.3 | 47,146 | 20,066 | 12,168 | -0.83 | -0.49 | -1.41 | -0.34 | 0.58 | 0.916 |
| | **Avr**. | 26,803 | 13,202 | 12,414 | -1.05 | -0.77 | -1.29 | -0.28 | 0.25 | 0.525 |
| | **StDv** | 18,531 | 6,742 | 1,425 | 0.30 | 0.51 | 0.24 | 0.23 | 0.54 | 0.755 |
| **KLK2** | Rep.1 | 300,931 | 494,877 | 34,461 | 3.40 | 4.87 | 0.62 | -1.47 | 2.79 | 4.254 |
| | Rep.2 | 496,385 | 636,865 | 25,665 | 3.55 | 5.17 | -0.58 | -1.62 | 4.13 | 5.743 |
| | Rep.3 | 858,522 | 630,712 | 27,354 | 3.35 | 4.48 | -0.24 | -1.13 | 3.60 | 4.722 |
| | **Avr**. | 551,946 | 587,485 | 29,160 | 3.44 | 4.84 | -0.07 | -1.40 | 3.50 | 4.906 |
| | **StDv** | 282,917 | 80,260 | 4,668 | 0.10 | 0.34 | 0.62 | 0.25 | 0.67 | 0.761 |
| **KLK3** | Rep.1 | 1,201,462 | 1,510,521 | 121,070 | 5.40 | 6.48 | 2.43 | -1.08 | 2.97 | 4.052 |
| | Rep.2 | 1,715,345 | 1,465,004 | 121,869 | 5.34 | 6.37 | 1.67 | -1.03 | 3.67 | 4.697 |
| | Rep.3 | 2,869,519 | 1,541,639 | 87,096 | 5.09 | 5.77 | 1.43 | -0.67 | 3.67 | 4.34 |
| | **Avr**. | 1,928,775 | 1,505,721 | 110,012 | 5.28 | 6.21 | 1.84 | -0.93 | 3.44 | 4.363 |
| | **StDv** | 854,265 | 38,542 | 19,850 | 0.16 | 0.38 | 0.52 | 0.22 | 0.40 | 0.323 |
| **LAMA1** | **Rep**.**1** | 38 | 1 | 2 | -9.55 | -14.05 | -13.46 | 4.50 | 3.91 | -0.59 |
| | **Rep.2** | 2 | 2 | 1,480 | -14.37 | -13.12 | -4.69 | -1.26 | -9.68 | -8.42 |
| | **Rep.3** | 526 | 1 | 1 | -7.32 | -14.79 | -14.98 | 7.47 | 7.66 | 0.195 |
| | **Avr**. | 189 | 1 | 494 | -10.41 | -13.98 | -11.04 | 3.57 | 0.63 | -2.94 |
| | **StDv** | 293 | 1 | 854 | 3.60 | 0. 84 | 5.55 | 4.44 | 9.12 | 4.764 |
| **MSMB** | Rep.1 | 671,389 | 929,667 | 51,400 | 4.56 | 5.78 | 1.19 | -1.22 | 3.37 | 4.587 |
| | Rep.2 | 910,538 | 848,857 | 18,772 | 4.43 | 5.58 | -1.03 | -1.15 | 5.45 | 6.609 |
| | Rep.3 | 1,628,017 | 11,765 | 15,852 | 4.28 | -1.26 | -1.03 | 5.54 | 5.31 | -0.24 |
| | **Avr**. | 1,069,981 | 596,763 | 28,675 | 4.42 | 3.37 | -0.29 | 1.06 | 4.71 | 3.654 |
| | **StDv** | 497,846 | 508,232 | 19,735 | 0.14 | 4.01 | 1.28 | 3.88 | 1.16 | 3.516 |
| **MUC1A** | Rep.1 | 262 | 1 | 5 | -6.76 | -14.05 | -12.13 | 7.29 | 5.37 | -1.91 |
| | Rep.2 | 1 | 1 | 1 | -15.37 | -14.12 | -15.23 | -1.26 | -0.15 | 1.11 |
| | Rep.3 | 73 | 2 | 1 | -10.17 | -13.79 | -14.98 | 3.62 | 4.81 | 1.195 |
| | **Avr.** | 112 | 1 | 2 | -10.77 | -13.98 | -14.11 | 3.22 | 3.35 | 0.131 |
| | **StDv** | 135 | 1 | 2 | 4.34 | 0.17 | 1.72 | 4.28 | 3.04 | 1.769 |
| **MYLK** | Rep.1 | 715,065 | 617,785 | 1,953,630 | 4.65 | 5.19 | 6.44 | -0.54 | -1.79 | -1.25 |
| | Rep.2 | 610,439 | 657,898 | 2,799,061 | 3.85 | 5.21 | 6.19 | -1.36 | -2.34 | -0.98 |
| | Rep.3 | 1,951,162 | 943,798 | 1,861,415 | 4.54 | 5.06 | 5.85 | -0.52 | -1.31 | -0.79 |
| | **Avr.** | 1,092,222 | 739,827 | 2,204,702 | 4.35 | 5.15 | 6.16 | -0.81 | -1.81 | -1 |
| | **StDv** | 745,701 | 177,779 | 516,791 | 0.44 | 0.08 | 0.30 | 0.48 | 0.52 | 0.234 |
| **PCAT1** | Rep.1 | 46,874 | 32,022 | 49,088 | 0.72 | 0.92 | 1.13 | -0.20 | -0.40 | -0.21 |
| | Rep.2 | 32,297 | 32,088 | 42,375 | -0.39 | 0.85 | 0.15 | -1.25 | -0.54 | 0.709 |
| | Rep.3 | 108,603 | 34,684 | 44,589 | 0.37 | 0.29 | 0.46 | 0.08 | -0.09 | -0.17 |
| | **Avr.** | 62,591 | 32,931 | 45,351 | 0.23 | 0.69 | 0.58 | -0.46 | -0.34 | 0.112 |
| | StDv | 40,508 | 1,518 | 3,421 | 0.57 | 0.34 | 0.50 | 0.70 | 0.23 | 0.517 |
| **PDZK1IP1** | Rep.1 | 3,534 | 279 | 81 | -3.01 | -5.92 | -8.12 | 2.92 | 5.11 | 2.195 |
| | Rep.2 | 7,452 | 763 | 25 | -2.51 | -4.54 | -10.58 | 2.03 | 8.07 | 6.041 |
| | Rep.3 | 14,745 | 941 | 32 | -2.51 | -4.91 | -9.98 | 2.40 | 7.47 | 5.073 |
| | **Avr.** | 8,577 | 661 | 46 | -2.68 | -5.12 | -9.56 | 2.45 | 6.88 | 4.436 |
| | **StDv** | 5,690 | 343 | 31 | 0.29 | 0.72 | 1.29 | 0.44 | 1.57 | 2.001 |
| **PEX10** | Rep.1 | 4,988 | 2,592 | 142 | -2.51 | -2.71 | -7.31 | 0.20 | 4.80 | 4.601 |
| | Rep.2 | 11,488 | 2,484 | 18 | -1.88 | -2.84 | -11.06 | 0.95 | 9.17 | 8.218 |
| | Rep.3 | 15,027 | 2,866 | 1,354 | -2.48 | -3.30 | -4.58 | 0.82 | 2.10 | 1.277 |
| | **Avr.** | 10,501 | 2,647 | 505 | -2.29 | -2.95 | -7.65 | 0.66 | 5.35 | 4.698 |
| | **StDv** | 5,092 | 197 | 738 | 0.35 | 0.31 | 3.25 | 0.40 | 3.57 | 3.472 |
| **PIP** | Rep.1 | 54 | 20 | 3 | -9.04 | -9.72 | -12.87 | 0.69 | 3.83 | 3.147 |
| | Rep.2 | 1 | 1 | 1 | -15.37 | -14.12 | -15.23 | -1.26 | -0.15 | 1.11 |
| | Rep.3 | 214 | 6 | 2 | -8.62 | -12.20 | -13.98 | 3.59 | 5.36 | 1.78 |
| | Avr. | 90 | 9 | 2 | -11.01 | -12.01 | -14.03 | 1.00 | 3.02 | 2.012 |
| | StDv | 111 | 10 | 1 | 3.78 | 2.20 | 1.18 | 2.44 | 2.84 | 1.039 |
| **PSCA** | Rep.1 | 5,241 | 1,893 | 584 | -2.44 | -3.16 | -5.27 | 0.72 | 2.83 | 2.107 |
| | Rep.2 | 1,732 | 2,623 | 64 | -4.61 | -2.76 | -9.23 | -1.85 | 4.61 | 6.467 |
| | Rep.3 | 21,332 | 1,448 | 64 | -1.98 | -4.29 | -8.98 | 2.31 | 7.00 | 4.695 |
| | **Avr.** | 9,435 | 1,988 | 237 | -3.01 | -3.40 | -7.82 | 0.39 | 4.81 | 4.423 |
| | **StDv** | 10,451 | 593 | 300 | 1.41 | 0.79 | 2.22 | 2.10 | 2.10 | 2.193 |
| **RARRES1** | Rep.1 | 32,243 | 22,582 | 13,675 | 0.18 | 0.42 | -0.72 | -0.23 | 0.90 | 1.134 |
| | Rep.2 | 60,617 | 19,969 | 49,942 | 0.52 | 0.17 | 0.38 | 0.35 | 0.13 | -0.21 |
| | Rep.3 | 95,938 | 25,022 | 22,595 | 0.19 | -0.18 | -0.52 | 0.37 | 0.71 | 0.342 |
| | **Avr.** | 62,933 | 22,524 | 28,737 | 0.30 | 0.14 | -0.28 | 0.16 | 0.58 | 0.421 |
| | **StDv** | 31,911 | 2,527 | 18,898 | 0.19 | 0.30 | 0.59 | 0.34 | 0.40 | 0.677 |
| **SELM1** | Rep.1 | 45,074 | 60,198 | 56,679 | 0.67 | 1.83 | 1.33 | -1.17 | -0.67 | 0.497 |
| | Rep.2 | 81,299 | 74,988 | 256,748 | 0.94 | 2.08 | 2.74 | -1.14 | -1.81 | -0.67 |
| | Rep.3 | 187,357 | 85,734 | 154,857 | 1.16 | 1.60 | 2.26 | -0.44 | -1.10 | -0.66 |
| | **Avr.** | 104,577 | 73,640 | 156,095 | 0.92 | 1.84 | 2.11 | -0.92 | -1.19 | -0.28 |
| | StDv | 73,943 | 12,821 | 100,040 | 0.25 | 0.24 | 0.72 | 0.41 | 0.57 | 0.669 |
| **SFRP1** | Rep.1 | 20,200 | 13,851 | 10,177 | -0.49 | -0.29 | -1.14 | -0.20 | 0.65 | 0.855 |
| | Rep.2 | 38,279 | 14,458 | 25,213 | -0.15 | -0.30 | -0.60 | 0.15 | 0.46 | 0.307 |
| | Rep.3 | 67,428 | 13,976 | 22,144 | -0.32 | -1.02 | -0.55 | 0.70 | 0.23 | -0.47 |
| | **Avr.** | 41,969 | 14,095 | 19,178 | -0.32 | -0.53 | -0.76 | 0.21 | 0.45 | 0.231 |
| | **StDv** | 23,829 | 321 | 7,945 | 0.17 | 0.42 | 0.33 | 0.45 | 0.21 | 0.665 |
| **SPP1** | Rep.1 | 17,123 | 8,549 | 5,130 | -0.73 | -0.98 | -2.13 | 0.25 | 1.40 | 1.147 |
| | Rep.2 | 33,838 | 5,495 | 9,376 | -0.32 | -1.69 | -2.03 | 1.37 | 1.71 | 0.339 |
| | Rep.3 | 47,407 | 5,307 | 7,799 | -0.83 | -2.41 | -2.05 | 1.59 | 1.23 | -0.36 |
| | **Avr.** | 32,789 | 6,450 | 7,435 | -0.63 | -1.70 | -2.07 | 1.07 | 1.44 | 0.375 |
| | **StDv** | 15,169 | 1,820 | 2,146 | 0.27 | 0.71 | 0.05 | 0.71 | 0.24 | 0.755 |
| **SYNM** | Rep.1 | 38,214 | 38,025 | 108,172 | 0.43 | 1.17 | 2.27 | -0.74 | -1.84 | -1.1 |
| | Rep.2 | 24,320 | 27,575 | 136,330 | -0.80 | 0.64 | 1.83 | -1.44 | -2.63 | -1.2 |
| | Rep.3 | 128,472 | 65,055 | 113,498 | 0.61 | 1.20 | 1.81 | -0.59 | -1.20 | -0.61 |
| | **Avr.** | 63,669 | 43,552 | 119,333 | 0.08 | 1.00 | 1.97 | -0.92 | -1.89 | -0.97 |
| | **StDv** | 56,550 | 19,342 | 14,958 | 0.77 | 0.32 | 0.26 | 0.45 | 0.72 | 0.315 |
| **TFAP2** | Rep.1 | 4,593 | 4,894 | 921 | -2.63 | -1.79 | -4.61 | -0.84 | 1.98 | 2.82 |
| | Rep.2 | 12,213 | 5,609 | 12 | -1.80 | -1.66 | -11.64 | -0.13 | 9.85 | 9.978 |
| | Rep.3 | 17,866 | 7,267 | 409 | -2.23 | -1.96 | -6.31 | -0.27 | 4.07 | 4.346 |
| | **Avr.** | 11,557 | 5,923 | 447 | -2.22 | -1.80 | -7.52 | -0.42 | 5.30 | 5.715 |
| | **StDv** | 6,661 | 1,217 | 456 | 0.42 | 0.15 | 3.67 | 0.37 | 4.07 | 3.77 |
| **TMC5** | Rep.1 | 42,344 | 5,080 | 1,449 | 0.58 | -1.74 | -3.96 | 2.31 | 4.53 | 2.22 |
| | Rep.2 | 156,493 | 9,681 | 4,101 | 1.88 | -0.87 | -3.22 | 2.76 | 5.11 | 2.349 |
| | Rep.3 | 184,408 | 12,510 | 344 | 1.13 | -1.18 | -6.56 | 2.31 | 7.69 | 5.379 |
| | **Avr.** | 127,748 | 9,090 | 1,965 | 1.20 | -1.26 | -4.58 | 2.46 | 5.78 | 3.316 |
| | **StDv** | 75,268 | 3,750 | 1,931 | 0.66 | 0.44 | 1.75 | 0.26 | 1.68 | 1.788 |
| **TPM2** | Rep.1 | 349,025 | 360,643 | 697,757 | 3.62 | 4.41 | 4.96 | -0.80 | -1.34 | -0.54 |
| | Rep.2 | 258,123 | 394,476 | 1,498,424 | 2.61 | 4.47 | 5.29 | -1.87 | -2.68 | -0.82 |
| | Rep.3 | 1,091,972 | 518,778 | 1,081,988 | 3.70 | 4.20 | 5.06 | -0.50 | -1.36 | -0.87 |
| | **Avr.** | 566,373 | 424,632 | 1,092,723 | 3.31 | 4.36 | 5.10 | -1.05 | -1.79 | -0.74 |
| | **StDv** | 457,445 | 83,269 | 400,441 | 0.61 | 0.15 | 0.17 | 0.72 | 0.77 | 0.174 |
| **TPX2** | Rep.1 | 148 | 19 | 1,930 | -7.58 | -9.80 | -3.54 | 2.21 | -4.04 | -6.26 |
| | Rep.2 | 2 | 39 | 1,802 | -14.37 | -8.83 | -4.41 | -5.54 | -9.96 | -4.42 |
| | Rep.3 | 648 | 4 | 4 | -7.02 | -12.79 | -12.98 | 5.77 | 5.96 | 0.195 |
| | Avr. | 266 | 21 | 1,245 | -9.66 | -10.47 | -6.98 | 0.81 | -2.68 | -3.49 |
| | StDv | 339 | 18 | 1,077 | 4.09 | 2.06 | 5.22 | 5.78 | 8.05 | 3.324 |
| **UGT2B15** | Rep.1 | 1,427 | 8 | 26 | -4.32 | -11.05 | -9.76 | 6.73 | 5.44 | -1.29 |
| | Rep.2 | 174 | 4 | 3 | -7.93 | -12.12 | -13.64 | 4.19 | 5.71 | 1.525 |
| | Rep.3 | 1,210 | 1,234 | 24 | -6.12 | -4.52 | -10.40 | -1.60 | 4.28 | 5.879 |
| | **Avr.** | 937 | 415 | 18 | -6.12 | -9.23 | -11.26 | 3.11 | 5.14 | 2.038 |
| | **StDv** | 670 | 709 | 13 | 1.81 | 4.11 | 2.08 | 4.27 | 0.76 | 3.612 |
| **ApoCl** | Rep.1 | 174,984 | 60,571 | 15,853 | 0.32 | -1.02 | -2.61 | 1.34 | 2.93 | 1.586 |
| | Rep.2 | 109,280 | 61,628 | 16,719 | 0.37 | -1.10 | -2.48 | 1.47 | 2.86 | 1.388 |
| | Rep.3 | 287,167 | 63,189 | 16,083 | -0.21 | -0.93 | -2.72 | 0.71 | 2.51 | 1.797 |
| | **Avr.** | 190,477 | 61,796 | 16,218 | 0.16 | -1.02 | -2.61 | 1.17 | 2.77 | 1.59 |
| | **StDv** | 89,950 | 1,317 | 449 | 0.33 | 0.08 | 0.12 | 0.41 | 0.22 | 0.205 |
| **ApoE** | Rep.1 | 291,532 | 162,851 | 193,580 | 1.06 | 0.40 | 1.00 | 0.65 | 0.06 | -0.6 |
| | Rep.2 | 176,541 | 148,789 | 166,165 | 1.06 | 0.18 | 0.83 | 0.89 | 0.23 | -0.65 |
| | Rep.3 | 598,834 | 164,006 | 168,695 | 0.85 | 0.45 | 0.67 | 0.40 | 0.18 | -0.22 |
| | **Avr.** | 355,636 | 158,549 | 176,147 | 0.99 | 0.34 | 0.83 | 0.65 | 0.16 | -0.49 |
| | **StDv** | 218,323 | 8,472 | 15,151 | 0.12 | 0.15 | 0.17 | 0.25 | 0.09 | 0.237 |
| **C15orf48** | Rep.1 | 91,710 | 72,158 | 11,140 | -0.61 | -0.77 | -3.12 | 0.16 | 2.51 | 2.348 |
| | Rep.2 | 24,923 | 90,805 | 13,560 | -1.76 | -0.54 | -2.79 | -1.22 | 1.02 | 2.249 |
| | Rep.3 | 335,481 | 73,301 | 9,586 | 0.01 | -0.71 | -3.47 | 0.72 | 3.48 | 2.758 |
| | **Avr.** | 150,705 | 78,755 | 11,429 | -0.79 | -0.67 | -3.13 | -0.11 | 2.34 | 2.452 |
| | **StDv** | 163,468 | 10,452 | 2,003 | 0.90 | 0.12 | 0.34 | 1.00 | 1.24 | 0.27 |
| **CSRP1.583** | Rep.1 | 501,452 | 720,127 | 1,040,681 | 1.84 | 2.55 | 3.43 | -0.71 | -1.59 | -0.88 |
| | Rep.2 | 211,188 | 999,386 | 1,129,536 | 1.32 | 2.92 | 3.60 | -1.60 | -2.27 | -0.67 |
| | Rep.3 | 1,187,574 | 454,677 | 685,654 | 1.83 | 1.92 | 2.69 | -0.09 | -0.86 | -0.77 |
| | **Avr.** | 633,405 | 724,730 | 951,957 | 1.67 | 2.46 | 3.24 | -0.80 | -1.57 | -0.77 |
| | **StDv** | 501,389 | 272,384 | 234,865 | 0.30 | 0.51 | 0.48 | 0.76 | 0.71 | 0.104 |
| **CSRP1.690** | Rep.1 | 428,472 | 677,330 | 878,261 | 1.61 | 2.46 | 3.18 | -0.85 | -1.57 | -0.72 |
| | Rep.2 | 135,826 | 860,624 | 776,997 | 0.69 | 2.71 | 3.06 | -2.02 | -2.37 | -0.35 |
| | Rep.3 | 939,564 | 682,836 | 907,654 | 1.50 | 2.51 | 3.09 | -1.01 | -1.60 | -0.59 |
| | **Avr.** | 501,287 | 740,263 | 854,304 | 1.26 | 2.56 | 3.11 | -1.29 | -1.85 | -0.55 |
| | **StDv** | 406,786 | 104,272 | 68,544 | 0.50 | 0.13 | 0.06 | 0.64 | 0.45 | 0.19 |
| **EBF3** | Rep.1 | 3,600 | 7,994 | 7,110 | -5.28 | -3.95 | -3.77 | -1.34 | -1.51 | -0.18 |
| | Rep.2 | 2,129 | 4,120 | 5,084 | -5.31 | -5.00 | -4.20 | -0.31 | -1.11 | -0.8 |
| | Rep.3 | 11,296 | 3,972 | 4,659 | -4.88 | -4.92 | -4.51 | 0.04 | -0.37 | -0.41 |
| | **Avr.** | 5,675 | 5,362 | 5,618 | -5.16 | -4.62 | -4.16 | -0.54 | -1.00 | -0.46 |
| | **StDv** | 4,923 | 2,281 | 1,310 | 0.24 | 0.59 | 0.37 | 0.71 | 0.58 | 0.313 |
| **F5** | Rep.1 | 358,681 | 9,657 | 4,497 | 1.36 | -3.67 | -4.43 | 5.03 | 5.78 | 0.755 |
| | Rep.2 | 185,570 | 5,448 | 115 | 1.14 | -4.60 | -9.67 | 5.73 | 10.80 | 5.072 |
| | Rep.3 | 282,916 | 3,853 | 2,263 | -0.24 | -4.96 | -5.55 | 4.73 | 5.32 | 0.591 |
| | **Avr.** | 275,722 | 6,319 | 2,292 | 0.75 | -4.41 | -6.55 | 5.16 | 7.30 | 2.139 |
| | **StDv** | 86,779 | 2,999 | 2,191 | 0.86 | 0.66 | 2.76 | 0.52 | 3.04 | 2.541 |
| **FGG** | Rep.1 | 67 | 6 | 321 | -11.03 | -14.33 | -8.24 | 3.30 | -2.79 | -6.09 |
| | Rep.2 | 1 | 1 | 2 | -16.37 | -17.01 | -15.51 | 0.64 | -0.85 | -1.49 |
| | Rep.3 | 341 | 4 | 3 | -9.93 | -14.87 | -15.11 | 4.94 | 5.18 | 0.238 |
| | Avr. | 136 | 4 | 109 | -12.44 | -15.40 | -12.95 | 2.96 | 0.51 | -2.45 |
| | StDv | 180 | 3 | 184 | 3.44 | 1.42 | 4.09 | 2.17 | 4.16 | 3.27 |
| **FHL2** | Rep.1 | 58,230 | 70,377 | 35,517 | -1.27 | -0.81 | -1.45 | -0.46 | 0.18 | 0.639 |
| | Rep.2 | 38,348 | 76,367 | 39,815 | -1.14 | -0.79 | -1.23 | -0.35 | 0.09 | 0.445 |
| | Rep.3 | 116,597 | 69,405 | 35,387 | -1.52 | -0.79 | -1.59 | -0.72 | 0.07 | 0.795 |
| | **Avr.** | 71,058 | 72,050 | 36,906 | -1.31 | -0.80 | -1.42 | -0.51 | 0.11 | 0.626 |
| | **StDv** | 40,671 | 3,770 | 2,520 | 0.19 | 0.01 | 0.18 | 0.19 | 0.06 | 0.175 |
| **GLOI** | Rep.1 | 58,230 | 70,377 | 35,517 | -1.27 | -0.81 | -1.45 | -0.46 | 0.18 | 0.639 |
| | Rep.2 | 38,348 | 76,367 | 39,815 | -1.14 | -0.79 | -1.23 | -0.35 | 0.09 | 0.445 |
| | Rep.3 | 116,597 | 69,405 | 35,387 | -1.52 | -0.79 | -1.59 | -0.72 | 0.07 | 0.795 |
| | **Avr.** | 71,058 | 72,050 | 36,906 | -1.31 | -0.80 | -1.42 | -0.51 | 0.11 | 0.626 |
| | **StDv** | 40,671 | 3,770 | 2,520 | 0.19 | 0.01 | 0.18 | 0.19 | 0.06 | 0.175 |
| **GRAMD4** | Rep.1 | 40,612 | 35,025 | 14,160 | -1.79 | -1.81 | -2.77 | 0.03 | 0.99 | 0.959 |
| | Rep.2 | 15,180 | 47,756 | 17,947 | -2.48 | -1.46 | -2.38 | -1.01 | -0.09 | 0.918 |
| | Rep.3 | 85,337 | 44,607 | 31,849 | -1.97 | -1.43 | -1.74 | -0.54 | -0.23 | 0.309 |
| | **Avr.** | 47,043 | 42,463 | 21,319 | -2.08 | -1.57 | -2.30 | -0.51 | 0.22 | 0.729 |
| | **StDv** | 35,518 | 6,631 | 9,314 | 0.36 | 0.21 | 0.52 | 0.52 | 0.67 | 0.364 |
| **HIF1A** | Rep.1 | 391,182 | 387,463 | 283,585 | 1.48 | 1.65 | 1.55 | -0.17 | -0.07 | 0.103 |
| | Rep.2 | 185,075 | 532,691 | 278,680 | 1.13 | 2.02 | 1.58 | -0.88 | -0.44 | 0.44 |
| | Rep.3 | 905,548 | 469,050 | 235,023 | 1.44 | 1.96 | 1.14 | -0.52 | 0.30 | 0.82 |
| | **Avr.** | 493,935 | 463,068 | 265,763 | 1.35 | 1.88 | 1.42 | -0.53 | -0.07 | 0.454 |
| | **StDv** | 371,065 | 72,799 | 26,734 | 0.19 | 0.20 | 0.24 | 0.36 | 0.37 | 0.359 |
| **HIPK2** | Rep.1 | 166,274 | 152,208 | 52,407 | 0.25 | 0.30 | -0.89 | -0.06 | 1.13 | 1.191 |
| | Rep.2 | 121,045 | 186,578 | 58,276 | 0.52 | 0.50 | -0.68 | 0.02 | 1.20 | 1.184 |
| | Rep.3 | 387,919 | 143,266 | 74,611 | 0.22 | 0.25 | -0.51 | -0.03 | 0.73 | 0.764 |
| | **Avr.** | 225,079 | 160,684 | 61,765 | 0.33 | 0.35 | -0.69 | -0.03 | 1.02 | 1.047 |
| | **StDv** | 142,825 | 22,866 | 11,506 | 0.17 | 0.13 | 0.19 | 0.04 | 0.25 | 0.244 |
| **HOXC4** | Rep.1 | 2,026 | 151 | 3,808 | -6.11 | -9.67 | -4.67 | 3.56 | -1.44 | -5 |
| | Rep.2 | 12,598 | 2,903 | 5,307 | -2.74 | -5.50 | -4.14 | 2.76 | 1.39 | -1.36 |
| | Rep.3 | 22,809 | 57 | 3,547 | -3.87 | -11.04 | -4.91 | 7.17 | 1.04 | -6.14 |
| | **Avr.** | 12,478 | 1,037 | 4,221 | -4.24 | -8.74 | -4.57 | 4.50 | 0.33 | -4.17 |
| | **StDv** | 10,392 | 1,617 | 950 | 1.71 | 2.88 | 0.39 | 2.35 | 1.55 | 2.493 |
| **HPN** | Rep.1 | 148,315 | 10,413 | 4,335 | 0.08 | -3.56 | -4.48 | 3.65 | 4.56 | 0.917 |
| | Rep.2 | 171,935 | 9,123 | 4,240 | 1.03 | -3.85 | -4.46 | 4.88 | 5.49 | 0.611 |
| | Rep.3 | 266,748 | 9,548 | 9,841 | -0.32 | -3.65 | -3.43 | 3.33 | 3.11 | -0.22 |
| | **Avr.** | 195,666 | 9,695 | 6,139 | 0.26 | -3.69 | -4.13 | 3.95 | 4.39 | 0.436 |
| | **StDv** | 62,681 | 657 | 3,207 | 0.69 | 0.15 | 0.60 | 0.82 | 1.20 | 0.589 |
| **HSBP1** | Rep.1 | 739,041 | 741,668 | 736,840 | 2.40 | 2.59 | 2.93 | -0.19 | -0.53 | -0.34 |
| | Rep.2 | 310,328 | 857,558 | 664,962 | 1.88 | 2.70 | 2.83 | -0.83 | -0.95 | -0.13 |
| | Rep.3 | 1,413,987 | 743,511 | 811,331 | 2.08 | 2.63 | 2.93 | -0.54 | -0.85 | -0.3 |
| | **Avr**. | 821,119 | 780,912 | 737,711 | 2.12 | 2.64 | 2.90 | -0.52 | -0.78 | -0.26 |
| | **StDv** | 556,389 | 66,383 | 73,188 | 0.26 | 0.06 | 0.06 | 0.32 | 0.22 | 0.113 |
| **IGFBP1** | Rep.1 | 391 | 18 | 33 | -8.49 | -12.74 | -11.52 | 4.26 | 3.03 | -1.22 |
| | Rep.2 | 5 | 4 | 3 | -14.04 | -15.01 | -14.93 | 0.96 | 0.88 | -0.08 |
| | Rep.3 | 1,724 | 4 | 6 | -7.59 | -14.87 | -14.11 | 7.28 | 6.52 | -0.76 |
| | Avr. | 707 | 9 | 14 | -10.04 | -14.21 | -13.52 | 4.17 | 3.48 | -0.69 |
| | StDv | 902 | 8 | 17 | 3.49 | 1.27 | 1.78 | 3.16 | 2.84 | 0.575 |
| **KLK3.470** | Rep.1 | 371,338 | 339,916 | 49,813 | 1.41 | 1.46 | -0.96 | -0.06 | 2.37 | 2.423 |
| | Rep.2 | 123,291 | 234,580 | 77,137 | 0.55 | 0.83 | -0.28 | -0.29 | 0.82 | 1.11 |
| | Rep.3 | 673,083 | 288,995 | 47,031 | 1.01 | 1.27 | -1.18 | -0.25 | 2.19 | 2.443 |
| | **Avr.** | 389,237 | 287,830 | 57,994 | 0.99 | 1.19 | -0.80 | -0.20 | 1.79 | 1.992 |
| | **StDv** | 275,333 | 52,678 | 16,637 | 0.43 | 0.32 | 0.47 | 0.12 | 0.84 | 0.764 |
| **LRRN1** | Rep.1 | 2,400 | 1,967 | 3,990 | -5.87 | -5.97 | -4.60 | 0.10 | -1.27 | -1.37 |
| | Rep.2 | 1,538 | 4,512 | 3,130 | -5.78 | -4.87 | -4.90 | -0.91 | -0.88 | 0.033 |
| | Rep.3 | 4,314 | 2,719 | 3,327 | -6.27 | -5.47 | -5.00 | -0.81 | -1.27 | -0.47 |
| | **Avr.** | 2,751 | 3,066 | 3,482 | -5.97 | -5.43 | -4.83 | -0.54 | -1.14 | -0.6 |
| | **StDv** | 1,421 | 1,308 | 451 | 0.26 | 0.55 | 0.21 | 0.56 | 0.23 | 0.71 |
| **MAP3K7** | Rep.1 | 285,317 | 268,102 | 197,273 | 1.03 | 1.12 | 1.03 | -0.10 | 0.00 | 0.095 |
| | Rep.2 | 159,676 | 327,841 | 224,968 | 0.92 | 1.32 | 1.27 | -0.40 | -0.35 | 0.049 |
| | Rep.3 | 736,305 | 343,367 | 243,733 | 1.14 | 1.51 | 1.20 | -0.37 | -0.05 | 0.318 |
| | **Avr**. | 393,766 | 313,103 | 221,991 | 1.03 | 1.32 | 1.16 | -0.29 | -0.13 | 0.154 |
| | **StDv** | 303,226 | 39,738 | 23,373 | 0.11 | 0.20 | 0.12 | 0.17 | 0.19 | 0.144 |
| **MYEF2** | Rep.1 | 46,838 | 35,016 | 26,471 | -1.58 | -1.82 | -1.87 | 0.23 | 0.29 | 0.056 |
| | Rep.2 | 37,413 | 47,082 | 29,873 | -1.17 | -1.48 | -1.65 | 0.31 | 0.47 | 0.162 |
| | Rep.3 | 107,994 | 36,896 | 29,425 | -1.63 | -1.70 | -1.85 | 0.08 | 0.23 | 0.15 |
| | **Avr**. | 64,082 | 39,665 | 28,590 | -1.46 | -1.67 | -1.79 | 0.21 | 0.33 | 0.123 |
| | **StDv** | 38,320 | 6,492 | 1,848 | 0.25 | 0.17 | 0.13 | 0.12 | 0.13 | 0.058 |
| **OPRK1** | Rep.1 | 5,217 | 2,718 | 36 | -4.75 | -5.50 | -11.39 | 0.76 | 6.65 | 5.891 |
| | Rep.2 | 1,995 | 1,118 | 792 | -5.40 | -6.88 | -6.88 | 1.48 | 1.48 | 0.003 |
| | Rep.3 | 3,156 | 2,030 | 25 | -6.72 | -5.89 | -12.05 | -0.84 | 5.33 | 6.167 |
| | **Avr**. | 3,456 | 1,955 | 284 | -5.62 | -6.09 | -10.11 | 0.47 | 4.49 | 4.02 |
| | **StDv** | 1,632 | 803 | 440 | 1.01 | 0.71 | 2.81 | 1.18 | 2.68 | 3.482 |
| **PCAT14** | Rep.1 | 21,748 | 32,046 | 33,751 | -2.69 | -1.94 | -1.52 | -0.74 | -1.17 | -0.42 |
| | Rep.2 | 7,029 | 32,465 | 23,679 | -3.59 | -2.02 | -1.98 | -1.57 | -1.61 | -0.04 |
| | Rep.3 | 51,291 | 28,036 | 24,567 | -2.70 | -2.10 | -2.11 | -0.60 | -0.59 | 0.014 |
| | **Avr**. | 26,689 | 30,849 | 27,332 | -2.99 | -2.02 | -1.87 | -0.97 | -1.12 | -0.15 |
| | **StDv** | 22,541 | 2,445 | 5,576 | 0.52 | 0.08 | 0.31 | 0.52 | 0.51 | 0.238 |
| **PFKP** | Rep.1 | 128,373 | 126,959 | 148,613 | -0.13 | 0.04 | 0.62 | -0.17 | -0.74 | -0.57 |
| | Rep.2 | 79,892 | 161,519 | 164,803 | -0.08 | 0.29 | 0.82 | -0.37 | -0.90 | -0.52 |
| | Rep.3 | 337,725 | 109,308 | 143,071 | 0.02 | -0.14 | 0.43 | 0.16 | -0.41 | -0.57 |
| | **Avr**. | 181,997 | 132,595 | 152,162 | -0.06 | 0.07 | 0.62 | -0.13 | -0.68 | -0.55 |
| | **StDv** | 137,026 | 26,558 | 11,292 | 0.07 | 0.22 | 0.19 | 0.27 | 0.25 | 0.027 |
| **PFKL** | Rep.1 | 84,518 | 86,343 | 53,852 | -0.73 | -0.51 | -0.85 | -0.22 | 0.12 | 0.334 |
| | Rep.2 | 57,137 | 116,264 | 56,622 | -0.56 | -0.18 | -0.72 | -0.38 | 0.16 | 0.544 |
| | Rep.3 | 177,580 | 71,945 | 53,309 | -0.91 | -0.74 | -1.00 | -0.17 | 0.09 | 0.256 |
| | **Avr**. | 106,412 | 91,517 | 54,594 | -0.73 | -0.48 | -0.86 | -0.26 | 0.12 | 0.378 |
| | **StDv** | 63,136 | 22,608 | 1,777 | 0.17 | 0.28 | 0.14 | 0.11 | 0.04 | 0.149 |
| **PLA2G7** | Rep.1 | 35,242 | 9,098 | 2,481 | -1.99 | -3.76 | -5.29 | 1.77 | 3.30 | 1.527 |
| | Rep.2 | 18,511 | 17,773 | 2,808 | -2.19 | -2.89 | -5.06 | 0.70 | 2.87 | 2.168 |
| | Rep.3 | 26,899 | 7,983 | 3,493 | -3.63 | -3.91 | -4.93 | 0.28 | 1.30 | 1.016 |
| | **Avr.** | 26,884 | 11,618 | 2,927 | -2.60 | -3.52 | -5.09 | 0.92 | 2.49 | 1.57 |
| | **StDv** | 8,366 | 5,359 | 516 | 0.90 | 0.55 | 0.18 | 0.77 | 1.05 | 0.577 |
| **PSMA** | Rep.1 | 325,305 | 29,181 | 3,040 | 1.22 | -2.08 | -4.99 | 3.29 | 6.21 | 2.915 |
| | Rep.2 | 291,538 | 31,302 | 4,664 | 1.79 | -2.07 | -4.32 | 3.86 | 6.11 | 2.252 |
| | Rep.3 | 267,804 | 13,383 | 3,813 | -0.32 | -3.17 | -4.80 | 2.85 | 4.49 | 1.635 |
| | **Avr.** | 294,882 | 24,622 | 3,839 | 0.90 | -2.44 | -4.71 | 3.33 | 5.60 | 2.267 |
| | **StDv** | 28,896 | 9,791 | 812 | 1.09 | 0.63 | 0.34 | 0.51 | 0.97 | 0.641 |
| **SAA2** | Rep.1 | 18,550 | 47,657 | 453 | -2.92 | -1.37 | -7.74 | -1.55 | 4.82 | 6.37 |
| | Rep.2 | 3,824 | 38,395 | 27 | -4.46 | -1.78 | -11.76 | -2.69 | 7.29 | 9.979 |
| | Rep.3 | 38,531 | 49,483 | 787 | -3.11 | -1.28 | -7.08 | -1.83 | 3.96 | 5.798 |
| | **Avr.** | 20,302 | 45,178 | 422 | -3.50 | -1.48 | -8.86 | -2.02 | 5.36 | 7.382 |
| | **StDv** | 17,420 | 5,945 | 381 | 0.84 | 0.27 | 2.53 | 0.59 | 1.73 | 2.267 |
| **SERPINA1** | Rep.1 | 71,980 | 74,165 | 22,531 | -0.96 | -0.73 | -2.10 | -0.23 | 1.14 | 1.371 |
| | Rep.2 | 25,468 | 46,560 | 7,792 | -1.73 | -1.50 | -3.58 | -0.23 | 1.86 | 2.085 |
| | Rep.3 | 128,858 | 61,216 | 17,040 | -1.37 | -0.97 | -2.64 | -0.40 | 1.27 | 1.668 |
| | **Avr.** | 75,435 | 60,647 | 15,788 | -1.35 | -1.07 | -2.78 | -0.29 | 1.42 | 1.708 |
| | **StDv** | 51,782 | 13,811 | 7,449 | 0.38 | 0.39 | 0.75 | 0.10 | 0.38 | 0.358 |
| **SLC10A7** | Rep.1 | 40,424 | 11,602 | 8,678 | -1.79 | -3.41 | -3.48 | 1.62 | 1.69 | 0.071 |
| | Rep.2 | 3,727 | 2,626 | 2,051 | -4.50 | -5.65 | -5.51 | 1.15 | 1.01 | -0.14 |
| | Rep.3 | 45,902 | 6,739 | 4,999 | -2.86 | -4.16 | -4.41 | 1.30 | 1.55 | 0.254 |
| | **Avr.** | 30,018 | 6,989 | 5,243 | -3.05 | -4.40 | -4.47 | 1.35 | 1.42 | 0.063 |
| | **StDv** | 22,933 | 4,493 | 3,320 | 1.36 | 1.14 | 1.02 | 0.24 | 0.36 | 0.196 |
| **SMAD5** | Rep.1 | 284,815 | 312,813 | 262,701 | 1.02 | 1.34 | 1.44 | -0.32 | -0.42 | -0.1 |
| | Rep.2 | 131,876 | 336,415 | 220,795 | 0.64 | 1.35 | 1.24 | -0.71 | -0.60 | 0.113 |
| | Rep.3 | 589,034 | 310,738 | 276,986 | 0.82 | 1.37 | 1.38 | -0.55 | -0.56 | -0.01 |
| | **Avr.** | 335,242 | 319,989 | 253,494 | 0.83 | 1.36 | 1.35 | -0.53 | -0.52 | 0.002 |
| | **StDv** | 232,713 | 14,263 | 29,205 | 0.19 | 0.01 | 0.10 | 0.20 | 0.10 | 0.105 |
| **SPON2** | Rep.1 | 213,150 | 368,410 | 72,098 | 0.61 | 1.58 | -0.43 | -0.98 | 1.03 | 2.006 |
| | Rep.2 | 123,703 | 514,190 | 67,857 | 0.55 | 1.97 | -0.46 | -1.41 | 1.01 | 2.427 |
| | Rep.3 | 373,228 | 376,107 | 57,551 | 0.16 | 1.65 | -0.89 | -1.48 | 1.05 | 2.531 |
| | **Avr.** | 236,694 | 419,569 | 65,835 | 0.44 | 1.73 | -0.59 | -1.29 | 1.03 | 2.322 |
| | **StDv** | 126,418 | 82,035 | 7,481 | 0.24 | 0.21 | 0.26 | 0.28 | 0.02 | 0.278 |
| **SRC** | Rep.1 | 27,107 | 46,057 | 35,875 | -2.37 | -1.42 | -1.43 | -0.95 | -0.94 | 0.013 |
| | Rep.2 | 25,281 | 63,357 | 33,209 | -1.74 | -1.06 | -1.49 | -0.68 | -0.25 | 0.438 |
| | Rep.3 | 57,395 | 50,210 | 21,905 | -2.54 | -1.26 | -2.28 | -1.28 | -0.26 | 1.02 |
| | **Avr.** | 36,594 | 53,208 | 30,330 | -2.22 | -1.24 | -1.73 | -0.97 | -0.48 | 0.49 |
| | **StDv** | 18,037 | 9,031 | 7,417 | 0.42 | 0.18 | 0.47 | 0.30 | 0.40 | 0.506 |
| **SYNPO2** | Rep.1 | 702,319 | 1,004,740 | 976,835 | 2.33 | 3.03 | 3.33 | -0.70 | -1.01 | -0.31 |
| | Rep.2 | 261,029 | 1,022,928 | 1,169,889 | 1.63 | 2.96 | 3.65 | -1.33 | -2.02 | -0.69 |
| | Rep.3 | 1,912,903 | 984,079 | 1,293,086 | 2.52 | 3.03 | 3.60 | -0.51 | -1.08 | -0.57 |
| | **Avr.** | 958,750 | 1,003,916 | 1,146,603 | 2.16 | 3.01 | 3.53 | -0.85 | -1.37 | -0.52 |
| | **StDv** | 855,272 | 19,438 | 159,406 | 0.47 | 0.04 | 0.17 | 0.43 | 0.56 | 0.195 |
| **TDRD1** | Rep.1 | 415 | 153 | 1,634 | -8.40 | -9.65 | -5.89 | 1.25 | -2.51 | -3.76 |
| | Rep.2 | 3 | 4 | 39 | -14.78 | -15.01 | -11.23 | 0.23 | -3.55 | -3.78 |
| | Rep.3 | 1,886 | 5 | 24 | -7.47 | -14.55 | -12.11 | 7.09 | 4.65 | -2.44 |
| | Avr. | 768 | 54 | 566 | -10.22 | -13.07 | -9.74 | 2.86 | -0.47 | -3.33 |
| | StDv | 990 | 86 | 925 | 3.98 | 2.97 | 3.37 | 3.70 | 4.46 | 0.769 |
| **TRIB1** | Rep.1 | 221,374 | 165,506 | 56,123 | 0.66 | 0.43 | -0.79 | 0.23 | 1.45 | 1.213 |
| | Rep.2 | 134,990 | 182,298 | 54,023 | 0.68 | 0.47 | -0.79 | 0.21 | 1.47 | 1.26 |
| | Rep.3 | 321,378 | 153,222 | 57,415 | -0.05 | 0.35 | -0.89 | -0.40 | 0.84 | 1.239 |
| | Avr. | 225,914 | 167,009 | 55,854 | 0.43 | 0.42 | -0.82 | 0.01 | 1.25 | 1.237 |
| | StDv | 93,277 | 14,596 | 1,712 | 0.42 | 0.06 | 0.06 | 0.36 | 0.36 | 0.024 |
| **TSPAN13** | Rep.1 | 157,778 | 49,173 | 13,875 | 0.17 | -1.33 | -2.80 | 1.50 | 2.97 | 1.478 |
| | Rep.2 | 84,561 | 53,576 | 15,083 | 0.00 | -1.30 | -2.63 | 1.30 | 2.63 | 1.334 |
| | Rep.3 | 221,110 | 47,740 | 19,395 | -0.59 | -1.33 | -2.45 | 0.74 | 1.86 | 1.123 |
| | Avr. | 154,483 | 50,163 | 16,118 | -0.14 | -1.32 | -2.63 | 1.18 | 2.49 | 1.312 |
| | StDv | 68,334 | 3,041 | 2,902 | 0.40 | 0.02 | 0.17 | 0.39 | 0.57 | 0.179 |

**Table 11: Subject 1 - RNA biomarkers with differential expression (Log2 FC>2) in Tumor and adjacent tissues**

| | **T/Adj.G** | | **T/Adj.M** | | **Adj.G/Adj.M** | |
|---|---|---|---|---|---|---|
| **Marker** | **Avr.** | **StDv** | **Avr.** | **StDv** | **Avr.** | **StDv** |
| **ETV1** | 3.38 | 0.20 | 3.25 | 0.08 | -0.13 | 0.13 |
| **HPN** | 3.95 | 0.82 | 4.39 | 1.20 | 0.44 | 0.59 |
| **F5** | 5.16 | 0.52 | 7.30 | 3.04 | 2.14 | 2.54 |
| **PSMA** | 3.33 | 0.51 | 5.60 | 0.97 | 2.27 | 0.64 |
| **UGT2B15** | 3.11 | 4.27 | 5.14 | 0.76 | 2.04 | 3.61 |
| **CRISP3** | 6.40 | 4.02 | 7.83 | 2.67 | 1.42 | 1.42 |
| **TMC5** | 2.46 | 0.26 | 5.78 | 1.68 | 3.32 | 1.79 |
| **PDZK1IP1** | 2.45 | 0.44 | 6.88 | 1.57 | 4.44 | 2.00 |
| **MSMB** | 1.06 | 3.88 | 4.71 | 1.16 | 3.65 | 3.52 |
| **PSCA** | 0.39 | 2.10 | 4.81 | 2.10 | 4.42 | 2.19 |
| **TFAP2** | -0.42 | 0.37 | 5.30 | 4.07 | 5.71 | 3.77 |
| **KLK3 438** | -0.93 | 0.22 | 3.44 | 0.40 | 4.36 | 0.32 |
| **KLK2** | -1.40 | 0.25 | 3.50 | 0.67 | 4.91 | 0.76 |
| **OPRK1** | 0.47 | 1.18 | 4.49 | 2.68 | 4.02 | 3.48 |
| **PEX10** | 0.66 | 0.40 | 5.35 | 3.57 | 4.70 | 3.47 |
| **C15orf48** | -0.11 | 1.00 | 2.34 | 1.24 | 2.45 | 0.27 |
| **AGR2** | 1.31 | 0.38 | 4.90 | 0.85 | 3.58 | 0.75 |
| **ADM** | 1.16 | 0.74 | 3.21 | 1.96 | 2.05 | 2.20 |
| **KLK3 470** | -0.20 | 0.12 | 1.79 | 0.84 | 1.99 | 0.76 |
| **PLA2G7** | 0.92 | 0.77 | 2.49 | 1.05 | 1.57 | 0.58 |
| **SPON2** | -1.29 | 0.28 | 1.03 | 0.02 | 2.32 | 0.28 |
| **HN1** | -0.70 | 0.86 | 1.42 | 2.74 | 2.12 | 1.91 |
| **ACPP** | -2.49 | 0.70 | 2.60 | 0.44 | 5.09 | 0.41 |
| **AZGP1** | -2.68 | 1.06 | 2.28 | 0.37 | 4.96 | 0.81 |
| **SAA2** | -2.02 | 0.59 | 5.36 | 1.73 | 7.38 | 2.27 |

A number of biomarkers are found to be differentially expressed in either the tumor samples or the adjacent glandular or muscular tissues and these have been grouped in Table 12 below.

**Table 12: Subject 1 - Comparison of the tumor, adjacent glandular and adjacent muscule tissue expression of select RNA biomarkers**

| **Tumor vs adjacent glandular and muscle tissue differential expression with log2FC >2** | **RNA biomarkers** |
|---|---|
| Up regulated in tumor compared with adjacent glandular and muscle tissues and no difference between the adjacent glandular and muscle tissues. | ETV1, HPN, F5, PMSA, UGT2BI5, CRISP3 |
| Up regulated in the tumor and the glandular adjacent tissue compared with the adjacent muscle tissue, with higher up regulation in the tumor than in the glandular adjacent tissue. | TMC5, PDZK1IP1, MSMB, PSCA |
| No difference between the tumor and the adjacent glandular tissus and up regulated compared with adjacent muscule tissue. | TFAP2, KLK3 438, KLK2, OPRK1, PEX10, C15orf48, AGR2, KLK3 470, PLA2G7, SPON2, |
| Higher in the glandular tissue compared with the tumor tissue compared with the adjacent muscle tissue. | ACPP, AZGP1, SAA2 |

It is common practice in this area of cancer research, particularly when using archival FFPE blocks as the source of tumor tissue, to use tissue adjacent to the tumor as control healthy tissue when studying differential expression. However, studies that have compared gene expression profiles or the chromatin status of prostate tumor tissue with adjacent tissue and benign prostate tissue from brain dead organ donors with no evidence of prostate cancer have suggested that the adjacent tissue has a genome and transcriptome that is more similar to the tumor than to the donor control tissues, suggesting that field effects exist (Chandran et al. 2005, Aryee et al. 2013).

The RBAS analysis using Subject 1 tissue shows that the glandular adjacent tissue has an RNA expression profile more similar to the tumor which is very likely due to field effects as described for prostate cancer tissues by Chandran et al (2005), Rizzi et al. (PLoS One 3(10):e3617, 2008) and reviewed in Trujillo et al. (Prostate Cancer, 2012).

### Subject 2 RNA Biomarker Analysis

The analysis of Subject 2 used prostatectomy tissue and the data compares the relative expression of the RNA biomarkers between three tumor tissues with different Gleason scores (termed T1, T2, and T3) to the adjacent glandular tissue only. The raw counts of triplicate samples from T1, T2 and T3 tumor tissues and adjacent glandular tissue is given followed by the log₂ normalised counts. Finally the log₂ FC expression of each RNA biomarker from the tumor region of the prostatectomy tissue RNA samples is given relative to the adjacent glandular tissue RNA.

The raw counts acquired for each amplicon from Subject 2 samples is presented in Table 13 with the calculation of the normalized count and FC.

**Table 13: Subject 2 - Raw read counts, Log₂ normalization and relative quantification (Log₂ FC) of RNA biomarker specific amplicons**

| | | **Raw read counts** (**Rc**) | | | **Log₂ Normalised Rc** | | | **Differential Expression (Log₂ FC)** | |
|---|---|---|---|---|---|---|---|---|---|
| | | **T1** | **T2** | **Adj.G** | **T1** | **T2** | **Adj.G** | **T1/T2** | **T1/ Adj.G** |
| **ACPP** | Rep.1 | 1,115,466 | 578,078 | 212,966 | 4.43 | 4.28 | 2.92 | 0.16 | 1.51 |
| | Rep.2 | 4 | 381,347 | 138,256 | 1.74 | 3.79 | 2.26 | -2.06 | -0.53 |
| | Rep.3 | 478,421 | 707,704 | 171,359 | 3.87 | 3.51 | 2.43 | 0.36 | 1.44 |
| | Avr. | 531,297 | 555,710 | 174,194 | 3.35 | 3.86 | 2.54 | -0.51 | 0.81 |
| | StDv | 559,608 | 164,324 | 37,436 | 1.42 | 0.39 | 0.34 | 1.34 | 1.16 |
| **AGR2** | Rep.1 | 967,584 | 227,247 | 305,013 | 4.23 | 2.93 | 3.44 | 1.30 | 0.79 |
| | Rep.2 | 4 | 285,242 | 416,971 | 1.74 | 3.37 | 3.86 | -1.64 | -2.12 |
| | Rep.3 | 551,975 | 408,212 | 508,593 | 4.08 | 2.71 | 4.00 | 1.36 | 0.08 |
| | Avr. | 506,521 | 306,900 | 410,192 | 3.35 | 3.01 | 3.77 | 0.34 | -0.42 |
| | StDv | 485,389 | 92,406 | 101,959 | 1.40 | 0.34 | 0.29 | 1.71 | 1.51 |
| **AKR1C3** | Rep.1 | 29,847 | 6,708 | 18,883 | -0.79 | -2.15 | -0.57 | 1.36 | -0.22 |
| | Rep.2 | 1 | 12,909 | 25,412 | -0.26 | -1.09 | -0.18 | 0.83 | -0.08 |
| | Rep.3 | 15,224 | 15,973 | 4,578 | -1.10 | -1.96 | -2.80 | 0.86 | 1.69 |
| | Avr. | 15,024 | 11,863 | 16,291 | -0.72 | -1.74 | -1.18 | 1.02 | 0.46 |
| | StDv | 14,924 | 4,720 | 10,656 | 0.42 | 0.57 | 1.41 | 0.30 | 1.07 |
| **ADM** | Rep.1 | 10 | 454 | 3 | -12.33 | -6.04 | -13.19 | -6.30 | 0.86 |
| | Rep.2 | 1 | 4 | 1,210 | -0.26 | -12.75 | -4.57 | 12.48 | 4.31 |
| | Rep.3 | 1,165 | 1,647 | 6 | -4.81 | -5.24 | -12.37 | 0.43 | 7.56 |
| | Avr. | 392 | 702 | 406 | -5.80 | -8.01 | -10.05 | 2.21 | 4.24 |
| | StDv | 669 | 849 | 696 | 6.10 | 4.12 | 4.76 | 9.52 | 3.35 |
| **AR(532)** | Rep.1 | 156,637 | 62,951 | 26,553 | 1.60 | 1.08 | -0.08 | 0.52 | 1.68 |
| | Rep.2 | 2 | 55,735 | 76,486 | 0.74 | 1.02 | 1.41 | -0.28 | -0.67 |
| | Rep.3 | 69,267 | 101,758 | 90,656 | 1.08 | 0.71 | 1.51 | 0.37 | -0.43 |
| | Avr. | 75,302 | 73,481 | 64,565 | 1.14 | 0.94 | 0.95 | 0.21 | 0.19 |
| | StDv | 78,492 | 24,753 | 33,673 | 0.43 | 0.20 | 0.89 | 0.43 | 1.29 |
| **AR(460)** | Rep.1 | 90,088 | 37,428 | 54,162 | 0.80 | 0.33 | 0.95 | 0.48 | -0.14 |
| | Rep.2 | 2 | 28,087 | 20,226 | 0.74 | 0.03 | -0.51 | 0.71 | 1.25 |
| | Rep.3 | 33,627 | 62,350 | 42,563 | 0.04 | 0.00 | 0.42 | 0.04 | -0.38 |
| | Avr. | 41,239 | 42,622 | 38,984 | 0.53 | 0.12 | 0.29 | 0.41 | 0.24 |
| | StDv | 45,523 | 17,712 | 17,249 | 0.42 | 0.18 | 0.74 | 0.34 | 0.88 |
| **AZGP1** | Rep.1 | 1,205,621 | 257,386 | 176,572 | 4.55 | 3.11 | 2.65 | 1.44 | 1.89 |
| | Rep.2 | 4 | 488,064 | 484,084 | 1.74 | 4.15 | 4.07 | -2.41 | -2.33 |
| | Rep.3 | 577,755 | 953,508 | 474,743 | 4.14 | 3.94 | 3.90 | 0.21 | 0.24 |
| | Avr. | 594,460 | 566,319 | 378,466 | 3.48 | 3.73 | 3.54 | -0.26 | -0.07 |
| | StDv | 602,982 | 354,597 | 174,908 | 1.52 | 0.55 | 0.77 | 1.97 | 2.13 |
| **CLU** | Rep.1 | 31,199 | 29,463 | 27,065 | -0.73 | -0.02 | -0.05 | -0.71 | -0.67 |
| | Rep.2 | 1 | 25,901 | 45,362 | -0.26 | -0.09 | 0.66 | -0.18 | -0.92 |
| | Rep.3 | 19,033 | 65,755 | 59,009 | -0.78 | 0.08 | 0.89 | -0.86 | -1.67 |
| | Avr. | 16,744 | 40,373 | 43,812 | -0.59 | -0.01 | 0.50 | -0.58 | -1.09 |
| | StDv | 15,724 | 22,053 | 16,028 | 0.28 | 0.08 | 0.49 | 0.36 | 0.52 |
| **CRISP3** | Rep.1 | 49 | 16 | 4 | -10.04 | -10.86 | -12.78 | 0.82 | 2.74 |
| | Rep.2 | 1 | 6 | 7 | -0.26 | -12.16 | -12.01 | 11.90 | 11.74 |
| | Rep.3 | 8 | 10 | 12 | -12.00 | -12.60 | -11.37 | 0.61 | -0.62 |
| | Avr. | 19 | 11 | 8 | -7.43 | -11.88 | -12.05 | 4.44 | 4.62 |
| | StDv | 26 | 5 | 4 | 6.29 | 0.90 | 0.70 | 6.46 | 6.40 |
| **DDC** | Rep.1 | 2 | 1,199 | 1 | -14.66 | -4.64 | -14.78 | -10.02 | 0.12 |
| | Rep.2 | 1 | 1 | 2 | -0.26 | -14.75 | -13.81 | 14.48 | 13.55 |
| | Rep.3 | 1 | 1 | 2 | -15.00 | -15.93 | -13.96 | 0.93 | -1.04 |
| | Avr. | 1 | 400 | 2 | -9.97 | -11.77 | -14.18 | 1.80 | 4.21 |
| | StDv | 1 | 692 | 1 | 8.41 | 6.21 | 0.52 | 12.27 | 8.11 |
| **ETV1** | Rep.1 | 55,213 | 19,124 | 17,021 | 0.10 | -0.64 | -0.72 | 0.74 | 0.82 |
| | Rep.2 | 1 | 7,861 | 12,058 | -0.26 | -1.81 | -1.26 | 1.54 | 0.99 |
| | Rep.3 | 19,210 | 23,675 | 21,091 | -0.77 | -1.39 | -0.59 | 0.63 | -0.17 |
| | Avr. | 24,808 | 16,887 | 16,723 | -0.31 | -1.28 | -0.86 | 0.97 | 0.55 |
| | StDv | 28,028 | 8,141 | 4,524 | 0.43 | 0.59 | 0.35 | 0.50 | 0.63 |
| **ETV4** | Rep.1 | 1,075 | 1 | 4 | -5.59 | -14.86 | -12.78 | 9.28 | 7.19 |
| | Rep.2 | 1 | 2 | 3 | -0.26 | -13.75 | -13.23 | 13.48 | 12.97 |
| | Rep.3 | 1 | 1,466 | 148 | -15.00 | -5.41 | -7.75 | -9.59 | -7.25 |
| | Avr. | 359 | 490 | 52 | -6.95 | -11.34 | -11.25 | 4.39 | 4.30 |
| | StDv | 620 | 846 | 83 | 7.46 | 5.17 | 3.04 | 12.29 | 10.41 |
| **FLNA** | Rep.1 | 642,702 | 419,884 | 592,030 | 3.64 | 3.81 | 4.40 | -0.18 | -0.76 |
| | Rep.2 | 10 | 350,713 | 643,645 | 3.06 | 3.67 | 4.48 | -0.61 | -1.42 |
| | Rep.3 | 288,460 | 679,776 | 656,776 | 3.14 | 3.45 | 4.37 | -0.31 | -1.23 |
| | Avr. | 310,391 | 483,458 | 630,817 | 3.28 | 3.65 | 4.42 | -0.37 | -1.14 |
| | StDv | 321,907 | 173,499 | 34,226 | 0.31 | 0.18 | 0.06 | 0.22 | 0.34 |
| **GLO1** | Rep.1 | 66,877 | 106,272 | 53,755 | -1.21 | -0.54 | -1.33 | -0.067 | 0.12 |
| | Rep.2 | 80,576 | 105,012 | 56,706 | -1.14 | -0.36 | -1.00 | -0.78 | -0.14 |
| | Rep.3 | 66160 | 119,919 | 99018 | -0.29 | -0.21 | -0.65 | -0.08 | 0.36 |
| | Avr. | 71,204 | 110,401 | 6,9826 | -0.88 | -0.37 | -0.99 | -0.31 | 0.11 |
| | StDv | 8,124 | 8,267 | 2,5324 | 0.51 | 0.17 | 0.34 | 0.41 | 0.25 |
| **HN1** | Rep.1 | 5,906 | 3,391 | 610 | -3.13 | -3.14 | -5.52 | 0.01 | 2.40 |
| | Rep.2 | 1 | 1,965 | 1,360 | -0.26 | -3.81 | -4.40 | 3.54 | 4.14 |
| | Rep.3 | 1,485 | 2,475 | 123 | -4.46 | -4.65 | -8.01 | 0.19 | 3.55 |
| | Avr. | 2,464 | 2,610 | 698 | -2.62 | -3.87 | -5.98 | 1.25 | 3.36 |
| | StDv | 3,072 | 723 | 623 | 2.14 | 0.76 | 1.85 | 1.99 | 0.89 |
| **HPGD** | Rep.1 | 51,645 | 15,143 | 24,758 | 0.00 | -0.98 | -0.18 | 0.98 | 0.18 |
| | Rep.2 | 1 | 42,608 | 21,512 | -0.26 | 0.63 | -0.42 | -0.89 | 0.16 |
| | Rep.3 | 36,518 | 45,268 | 33,203 | 0.16 | -0.46 | 0.06 | 0.62 | 0.10 |
| | Avr. | 29,388 | 34,340 | 26,491 | -0.03 | -0.27 | -0.18 | 0.23 | 0.15 |
| | StDv | 26,550 | 16,678 | 6,035 | 0.21 | 0.82 | 0.24 | 0.99 | 0.04 |
| **KLK2** | Rep.1 | 821,034 | 397,634 | 319,495 | 3.99 | 3.74 | 3.51 | 0.26 | 0.48 |
| | Rep.2 | 5 | 327,028 | 295,541 | 2.06 | 3.57 | 3.36 | -1.51 | -1.30 |
| | Rep.3 | 282,724 | 504,677 | 269,503 | 3.11 | 3.02 | 3.08 | 0.09 | 0.03 |
| | Avr. | 367,921 | 409,780 | 294,846 | 3.05 | 3.44 | 3.32 | -0.39 | -0.26 |
| | StDv | 417,092 | 89,445 | 25,003 | 0.97 | 0.38 | 0.22 | 0.98 | 0.93 |
| **KLK3 438** | Rep.1 | 3,461,933 | 1,020,587 | 715,738 | 6.07 | 5.10 | 4.67 | 0.97 | 1.40 |
| | Rep.2 | 6 | 1,013,939 | 821,767 | 2.32 | 5.20 | 4.83 | -2.88 | -2.51 |
| | Rep.3 | 726,379 | 1,380,170 | 888,446 | 4.47 | 4.47 | 4.80 | 0.00 | -0.33 |
| | Avr. | 1,396,106 | 1,138,232 | 808,650 | 4.29 | 4.92 | 4.77 | -0.64 | -0.48 |
| | StDv | 1,825,551 | 209,551 | 87,098 | 1.88 | 0.40 | 0.09 | 2.00 | 1.96 |
| **LAMA1** | Rep.1 | 1 | 3 | 1 | -15.66 | -13.28 | -14.78 | -2.38 | -0.88 |
| | Rep.2 | 1 | 1 | 1 | -0.26 | -14.75 | -14.81 | 14.48 | 14.55 |
| | Rep.3 | 1 | 1 | 1 | -15.00 | -15.93 | -14.96 | 0.93 | -0.04 |
| | Avr. | 1 | 2 | 1 | -10.30 | -14.65 | -14.85 | 4.35 | 4.54 |
| | StDv | 0 | 1 | 0 | 8.70 | 1.33 | 0.10 | 8.93 | 8.68 |
| **MSMB** | Rep.1 | 2,227,552 | 502,180 | 575,321 | 5.43 | 4.07 | 4.36 | 1.36 | 1.07 |
| | Rep.2 | 14 | 521,847 | 606,686 | 3.54 | 4.25 | 4.40 | -0.70 | -0.85 |
| | Rep.3 | 829,160 | 1,035,285 | 539,522 | 4.67 | 4.06 | 4.08 | 0.61 | 0.58 |
| | Avr. | 1,018,909 | 686,437 | 573,843 | 4.55 | 4.13 | 4.28 | 0.42 | 0.27 |
| | StDv | 1,125,826 | 302,271 | 33,606 | 0.95 | 0.10 | 0.17 | 1.04 | 1.00 |
| **MUC1A** | **Rep.1** | 1 | 1 | 1 | -15.66 | -14.86 | -14.78 | -0.79 | -0.88 |
| | **Rep.2** | 1 | 2 | 1 | -0.26 | -13.75 | -14.81 | 13.48 | 14.55 |
| | **Rep.3** | 1 | 1 | 1 | -15.00 | -15.93 | -14.96 | 0.93 | -0.04 |
| | **Avr**. | 1 | 1 | 1 | -10.30 | -14.85 | -14.85 | 4.54 | 4.54 |
| | **StDv** | 0 | 1 | 0 | 8.70 | 1.09 | 0.10 | 7.79 | 8.68 |
| **MYLK** | Rep.1 | 1,530,334 | 910,551 | 908,063 | 4.89 | 4.93 | 5.02 | -0.04 | -0.13 |
| | Rep.2 | 4 | 690,874 | 1,217,163 | 1.74 | 4.65 | 5.40 | -2.91 | -3.66 |
| | Rep.3 | 584,868 | 1.1 10⁶ | 1.4 | 4.16 | 4.22 | 5.44 | -0.05 | -1.28 |
| | Avr. | 705,069 | 919,376 | 1,169,326 | 3.60 | 4.60 | 5.29 | -1.00 | -1.69 |
| | StDv | 772,213 | 233,040 | 240,933 | 1.65 | 0.36 | 0.24 | 1.65 | 1.80 |
| **PCAT1** | Rep.1 | 176,018 | 51,153 | 60,175 | 1.77 | 0.78 | 1.10 | 0.99 | 0.67 |
| | Rep.2 | 1 | 23,697 | 37,342 | -0.26 | -0.22 | 0.37 | -0.05 | -0.64 |
| | Rep.3 | 56,838 | 50,071 | 47,687 | 0.80 | -0.31 | 0.58 | 1.11 | 0.21 |
| | Avr. | 77,619 | 41,640 | 48,401 | 0.77 | 0.08 | 0.69 | 0.69 | 0.08 |
| | StDv | 89,830 | 15,549 | 11,433 | 1.02 | 0.60 | 0.37 | 0.64 | 0.66 |
| **PDZK1 IP1** | Rep.1 | 8,995 | 2,067 | 1,865 | -2.52 | -3.85 | -3.91 | 1.33 | 1.39 |
| | Rep.2 | 1 | 3,536 | 4,238 | -0.26 | -2.96 | -2.76 | 2.70 | 2.50 |
| | Rep.3 | 7,861 | 17,707 | 2,509 | -2.06 | -1.81 | -3.66 | -0.24 | 1.61 |
| | Avr. | 5,619 | 7,770 | 2,871 | -1.61 | -2.87 | -3.45 | 1.26 | 1.83 |
| | StDv | 4,898 | 8,637 | 1,227 | 1.19 | 1.02 | 0.60 | 1.47 | 0.59 |
| **PEX10** | Rep.1 | 7,719 | 9 | 1,944 | -2.74 | -11.70 | -3.85 | 8.95 | 1.11 |
| | Rep.2 | 1 | 784 | 1,078 | -0.26 | -5.13 | -4.74 | 4.87 | 4.48 |
| | Rep.3 | 8,785 | 3,000 | 3,908 | -1.90 | -4.37 | -3.02 | 2.48 | 1.13 |
| | Avr. | 5,502 | 1,264 | 2,310 | -1.63 | -7.07 | -3.87 | 5.43 | 2.24 |
| | StDv | 4,793 | 1,552 | 1,450 | 1.26 | 4.03 | 0.86 | 3.27 | 1.94 |
| **PIP** | Rep.1 | 2,284 | 1 | 1 | -4.50 | -14.86 | -14.78 | 10.37 | 10.28 |
| | Rep.2 | 1 | 1 | 1 | -0.26 | -14.75 | -14.81 | 14.48 | 14.55 |
| | Rep.3 | 2 | 898 | 1 | -14.00 | -6.11 | -14.96 | -7.88 | 0.96 |
| | Avr. | 762 | 300 | 1 | -6.25 | -11.91 | -14.85 | 5.66 | 8.60 |
| | StDv | 1,318 | 518 | 0 | 7.03 | 5.02 | 0.10 | 11.90 | 6.95 |
| **PSCA** | Rep.1 | 12,535 | 8,670 | 61,407 | -2.04 | -1.78 | 1.13 | -0.26 | -3.17 |
| | Rep.2 | 1 | 3,413 | 52,009 | -0.26 | -3.01 | 0.85 | 2.75 | -1.12 |
| | Rep.3 | 8,366 | 2,537 | 68,425 | -1.97 | -4.62 | 1.11 | 2.65 | -3.07 |
| | Avr. | 6,967 | 4,873 | 60,614 | -1.42 | -3.14 | 1.03 | 1.71 | -2.45 |
| | StDv | 6,383 | 3,317 | 8,237 | 1.01 | 1.42 | 0.15 | 1.71 | 1.16 |
| **RARR ES1** | Rep.1 | 170,826 | 64,937 | 19,653 | 1.73 | 1.12 | -0.51 | 0.60 | 2.24 |
| | Rep.2 | 1 | 66,464 | 22,545 | -0.26 | 1.27 | -0.35 | -1.54 | 0.09 |
| | Rep.3 | 63,506 | 84,074 | 23,140 | 0.96 | 0.43 | -0.46 | 0.52 | 1.42 |
| | Avr. | 78,111 | 71,825 | 21,779 | 0.81 | 0.94 | -0.44 | -0.14 | 1.25 |
| | StDv | 86,344 | 10,635 | 1,865 | 1.00 | 0.45 | 0.08 | 1.21 | 1.08 |
| **SELM 1** | Rep.1 | 168,631 | 61,687 | 37,098 | 1.71 | 1.05 | 0.40 | 0.66 | 1.31 |
| | Rep.2 | 2 | 64,482 | 80,173 | 0.74 | 1.23 | 1.48 | -0.49 | -0.74 |
| | Rep.3 | 69,773 | 84,097 | 59,539 | 1.09 | 0.43 | 0.90 | 0.66 | 0.19 |
| | Avr. | 79,469 | 70,089 | 58,937 | 1.18 | 0.90 | 0.93 | 0.28 | 0.25 |
| | StDv | 84,732 | 12,212 | 21,544 | 0.49 | 0.42 | 0.54 | 0.67 | 1.02 |
| **SFRP1** | Rep.1 | 54,883 | 43,772 | 8,160 | 0.09 | 0.55 | -1.78 | -0.46 | 1.87 |
| | Rep.2 | 1 | 46,965 | 28,240 | -0.26 | 0.77 | -0.03 | -1.03 | -0.23 |
| | Rep.3 | 44,799 | 57,534 | 37,830 | 0.46 | -0.11 | 0.25 | 0.57 | 0.20 |
| | Avr. | 33,228 | 49,424 | 24,743 | 0.09 | 0.40 | -0.52 | -0.31 | 0.61 |
| | StDv | 29,214 | 7,203 | 15,141 | 0.36 | 0.46 | 1.10 | 0.81 | 1.11 |
| **SPP1** | Rep.1 | 88,187 | 20,998 | 5,469 | 0.77 | -0.51 | -2.36 | 1.28 | 3.13 |
| | Rep.2 | 1 | 23,950 | 6,577 | -0.26 | -0.20 | -2.13 | -0.06 | 1.87 |
| | Rep.3 | 42,213 | 27,737 | 4,804 | 0.37 | -1.17 | -2.73 | 1.54 | 3.10 |
| | Avr. | 43,467 | 24,228 | 5,617 | 0.29 | -0.62 | -2.41 | 0.92 | 2.70 |
| | StDv | 44,106 | 3,378 | 896 | 0.52 | 0.49 | 0.30 | 0.86 | 0.72 |
| **SYNM** | Rep.1 | 113,741 | 48,343 | 49,560 | 1.14 | 0.70 | 0.82 | 0.44 | 0.32 |
| | Rep.2 | 1 | 50,482 | 69,718 | -0.26 | 0.88 | 1.28 | -1.14 | -1.54 |
| | Rep.3 | 32,666 | 84,942 | 104,171 | 0.00 | 0.45 | 1.71 | -0.45 | -1.71 |
| | Avr. | 48,803 | 61,256 | 74,483 | 0.29 | 0.67 | 1.27 | -0.38 | -0.98 |
| | StDv | 58,562 | 20,541 | 27,616 | 0.75 | 0.21 | 0.45 | 0.79 | 1.13 |
| **TFAP2 A** | Rep.1 | 4,633 | 4,198 | 7,283 | -3.48 | -2.83 | -1.95 | -0.65 | -1.53 |
| | Rep.2 | 1 | 4,808 | 2,263 | -0.26 | -2.52 | -3.67 | 2.25 | 3.41 |
| | Rep.3 | 2,925 | 6,336 | 2,544 | -3.48 | -3.30 | -3.64 | -0.19 | 0.16 |
| | Avr. | 2,520 | 5,114 | 4,030 | -2.41 | -2.88 | -3.09 | 0.47 | 0.68 |
| | StDv | 2,342 | 1,101 | 2,821 | 1.86 | 0.39 | 0.99 | 1.56 | 2.51 |
| **TMC5** | Rep.1 | 99,782 | 31,783 | 10,280 | 0.95 | 0.09 | -1.45 | 0.86 | 2.40 |
| | Rep.2 | 1 | 46,113 | 18,809 | -0.26 | 0.75 | -0.61 | -1.01 | 0.35 |
| | Rep.3 | 129,750 | 78,656 | 17,485 | 1.99 | 0.34 | -0.86 | 1.65 | 2.85 |
| | Avr. | 76,511 | 52,184 | 15,525 | 0.89 | 0.39 | -0.98 | 0.50 | 1.87 |
| | StDv | 67,933 | 24,019 | 4,590 | 1.13 | 0.33 | 0.43 | 1.37 | 1.33 |
| **TPM2** | Rep.1 | 533,651 | 370,786 | 430,778 | 3.37 | 3.64 | 3.94 | -0.27 | -0.57 |
| | Rep.2 | 2 | 286,949 | 595,345 | 0.74 | 3.38 | 4.37 | -2.65 | -3.63 |
| | Rep.3 | 266,214 | 529,695 | 678,024 | 3.03 | 3.09 | 4.41 | -0.06 | -1.39 |
| | Avr. | 266,622 | 395,810 | 568,049 | 2.38 | 3.37 | 4.24 | -0.99 | -1.86 |
| | StDv | 266,825 | 123,293 | 125,863 | 1.43 | 0.27 | 0.26 | 1.44 | 1.59 |
| **TPX2** | Rep.1 | 2,010 | 5 | 2 | -4.68 | -12.54 | -13.78 | 7.86 | 9.09 |
| | Rep.2 | 1 | 2 | 3 | -0.26 | -13.75 | -13.23 | 13.48 | 12.97 |
| | Rep.3 | 1,433 | 2 | 3 | -4.51 | -14.93 | -13.37 | 10.41 | 8.86 |
| | Avr. | 1,148 | 3 | 3 | -3.15 | -13.74 | -13.46 | 10.59 | 10.31 |
| | StDv | 1,034 | 2 | 1 | 2.50 | 1.19 | 0.28 | 2.82 | 2.31 |
| **UGT2 B15** | Rep.1 | 13 | 786 | 1,209 | -11.96 | -5.25 | -4.54 | -6.71 | -7.42 |
| | Rep.2 | 1 | 137 | 148 | -0.26 | -7.65 | -7.60 | 7.39 | 7.34 |
| | Rep.3 | 3,199 | 6 | 4,002 | -3.35 | -13.34 | -2.99 | 9.99 | -0.36 |
| | Avr. | 1,071 | 310 | 1,786 | -5.19 | -8.75 | -5.04 | 3.56 | -0.15 |
| | StDv | 1,843 | 418 | 1,991 | 6.06 | 4.16 | 2.35 | 8.98 | 7.38 |

| | | **Raw read counts (Rc)** | | | **Log₂ Normalised Rc** | | | **Differential Expression (Log₂ FC)** | |
|---|---|---|---|---|---|---|---|---|---|
| | | **T1** | **T3** | **Adj**.**G** | **T1** | **T3** | **Adj**.**G** | **T1/T3** | **T1/ Adj.G** |
| **ApoC1** | Rep.1 | 98,101 | 68,822 | 23,748 | -0.66 | -1.17 | -2.51 | 0.51 | 1.85 |
| | Rep.2 | 134,903 | 52,205 | 17,831 | -0.40 | -1.37 | -2.67 | 0.97 | 2.27 |
| | Rep.3 | 50,743 | 49,348 | 5,790 | -0.67 | -1.49 | -4.75 | 0.82 | 4.07 |
| | Avr. | 94,582 | 56,792 | 15,790 | -0.58 | -1.34 | -3.31 | 0.76 | 2.73 |
| | StDv | 42,190 | 10,516 | 9,151 | 0.16 | 0.16 | 1.25 | 0.24 | 1.18 |
| **ApoE** | Rep.1 | 113,238 | 92,674 | 50,929 | -0.45 | -0.74 | -1.41 | 0.28 | 0.96 |
| | Rep.2 | 120,951 | 97,766 | 26,427 | -0.56 | -0.46 | -2.10 | -0.10 | 1.55 |
| | Rep.3 | 53,870 | 80,438 | 36,240 | -0.59 | -0.79 | -2.10 | 0.20 | 1.51 |
| | Avr. | 96,020 | 90,293 | 37,865 | -0.53 | -0.66 | -1.87 | 0.13 | 1.34 |
| | StDv | 36,706 | 8,906 | 12,332 | 0.07 | 0.18 | 0.40 | 0.20 | 0.33 |
| **C15orf48** | Rep.1 | 462,524 | 760,825 | 23,822 | 1.58 | 2.30 | -2.51 | -0.72 | 4.08 |
| | Rep.2 | 635,716 | 641,300 | 22,420 | 1.84 | 2.25 | -2.34 | -0.41 | 4.18 |
| | Rep.3 | 321,882 | 563,978 | 7,408 | 1.99 | 2.02 | -4.39 | -0.03 | 6.38 |
| | Avr. | 473,374 | 655,368 | 17,883 | 1.80 | 2.19 | -3.08 | -0.39 | 4.88 |
| | StDv | 157,198 | 99,175 | 9,099 | 0.21 | 0.15 | 1.14 | 0.35 | 1.30 |
| **CSRP1.5 83** | Rep.1 | 921,105 | 514,866 | 939,933 | 2.57 | 1.74 | 2.79 | 0.83 | -0.22 |
| | Rep.2 | 1,361,542 | 570,555 | 989,617 | 2.94 | 2.08 | 3.12 | 0.85 | -0.19 |
| | Rep.3 | 390,734 | 242,180 | 690,001 | 2.27 | 0.80 | 2.15 | 1.47 | 0.12 |
| | Avr. | 891,127 | 442,534 | 873,184 | 2.59 | 1.54 | 2.69 | 1.05 | -0.10 |
| | StDv | 486,098 | 175,731 | 160,574 | 0.33 | 0.66 | 0.50 | 0.36 | 0.19 |
| **CSRP1.6 90** | Rep.1 | 610,121 | 317,158 | 490,682 | 1.97 | 1.04 | 1.86 | 0.94 | 0.12 |
| | Rep.2 | 789,293 | 344,428 | 517,589 | 2.15 | 1.36 | 2.19 | 0.79 | -0.04 |
| | Rep.3 | 404,039 | 122,907 | 423,777 | 2.32 | -0.18 | 1.45 | 2.50 | 0.87 |
| | Avr. | 601,151 | 261,498 | 477,349 | 2.15 | 0.74 | 1.83 | 1.41 | 0.32 |
| | StDv | 192,784 | 120,795 | 48,306 | 0.17 | 0.81 | 0.37 | 0.94 | 0.49 |
| **EBF3** | Rep.1 | 11,409 | 6,191 | 8,760 | -3.77 | -4.64 | -3.95 | 0.88 | 0.19 |
| | Rep.2 | 12,494 | 583 | 8,772 | -3.83 | -7.85 | -3.69 | 4.02 | -0.14 |
| | Rep.3 | 2,412 | 750 | 294 | -5.07 | -7.53 | -9.05 | 2.47 | 3.98 |
| | Avr. | 8,772 | 2,508 | 5,942 | -4.22 | -6.68 | -5.56 | 2.45 | 1.34 |
| | StDv | 5,534 | 3,191 | 4,891 | 0.73 | 1.77 | 3.02 | 1.57 | 2.29 |
| **F5** | Rep.1 | 19,321 | 17,161 | 6,991 | -3.01 | -3.17 | -4.28 | 0.17 | 1.27 |
| | Rep.2 | 21,147 | 13,841 | 90 | -3.07 | -3.28 | -10.30 | 0.21 | 7.23 |
| | Rep.3 | 2,486 | 20,749 | 9,499 | -5.02 | -2.74 | -4.03 | -2.28 | -0.99 |
| | Avr. | 14,318 | 17,250 | 5,527 | -3.70 | -3.07 | -6.20 | -0.64 | 2.50 |
| | StDv | 10,287 | 3,455 | 4,872 | 1.15 | 0.28 | 3.55 | 1.42 | 4.25 |
| **FGG** | Rep.1 | 5 | 2 | 2 | -14.92 | -16.24 | -16.05 | 1.32 | 1.13 |
| | Rep.2 | 4,110 | 1 | 1 | -5.44 | -17.04 | -16.79 | 11.60 | 11.36 |
| | Rep.3 | 1 | 2 | 1 | -16.30 | -16.09 | -17.25 | -0.22 | 0.94 |
| | Avr. | 1,372 | 2 | 1 | -12.22 | -16.45 | -16.70 | 4.23 | 4.47 |
| | StDv | 2,371 | 1 | 1 | 5.92 | 0.51 | 0.60 | 6.43 | 5.96 |
| **FHL2** | Rep.1 | 102,579 | 47,546 | 62,719 | -0.60 | -1.70 | -1.11 | 1.10 | 0.51 |
| | Rep.2 | 109,719 | 57,142 | 51,134 | -0.70 | -1.24 | -1.15 | 0.54 | 0.45 |
| | Rep.3 | 41,940 | 14,593 | 24,991 | -0.95 | -3.25 | -2.64 | 2.30 | 1.69 |
| | Avr. | 84,746 | 39,760 | 46,281 | -0.75 | -2.06 | -1.63 | 1.32 | 0.89 |
| | StDv | 37,243 | 22,317 | 19,326 | 0.18 | 1.06 | 0.87 | 0.90 | 0.70 |
| **GRAMD 4** | Rep.1 | 31,350 | 25,907 | 28,223 | -2.31 | -2.58 | -2.26 | 0.27 | -0.04 |
| | Rep.2 | 37,363 | 24,238 | 29,679 | -2.25 | -2.47 | -1.93 | 0.22 | -0.32 |
| | Rep.3 | 20,118 | 35,834 | 16,514 | -2.01 | -1.96 | -3.23 | -0.05 | 1.23 |
| | Avr. | 29,610 | 28,660 | 24,805 | -2.19 | -2.34 | -2.48 | 0.15 | 0.29 |
| | StDv | 8,753 | 6,269 | 7,217 | 0.16 | 0.33 | 0.68 | 0.17 | 0.82 |
| **HIF1A** | Rep.1 | 398,064 | 419,595 | 340,458 | 1.36 | 1.44 | 1.33 | -0.08 | 0.03 |
| | Rep.2 | 771,120 | 404,282 | 369,458 | 2.12 | 1.59 | 1.70 | 0.53 | 0.41 |
| | Rep.3 | 297,843 | 557,606 | 438,692 | 1.88 | 2.00 | 1.50 | -0.12 | 0.38 |
| | Avr. | 489,009 | 460,494 | 382,869 | 1.78 | 1.68 | 1.51 | 0.11 | 0.28 |
| | StDv | 249,401 | 84,449 | 50,472 | 0.39 | 0.29 | 0.19 | 0.37 | 0.21 |
| **HIPK2** | Rep.1 | 109,550 | 170,523 | 42,729 | -0.50 | 0.14 | -1.67 | -0.64 | 1.16 |
| | Rep.2 | 149,913 | 143,176 | 70,970 | -0.25 | 0.09 | -0.68 | -0.34 | 0.43 |
| | Rep.3 | 75,965 | 201,996 | 72,517 | -0.09 | 0.54 | -1.10 | -0.63 | 1.01 |
| | Avr. | 111,809 | 171,898 | 62,072 | -0.28 | 0.26 | -1.15 | -0.54 | 0.87 |
| | StDv | 37,026 | 29,434 | 16,769 | 0.21 | 0.25 | 0.50 | 0.17 | 0.39 |
| **HOXC4** | Rep.1 | 1,626 | 4,220 | 22 | -6.58 | -5.19 | -12.59 | -1.38 | 6.01 |
| | Rep.2 | 25 | 10,154 | 13 | -12.80 | -3.73 | -13.09 | -9.07 | 0.29 |
| | Rep.3 | 6,815 | 14,781 | 12 | -3.57 | -3.23 | -13.66 | -0.34 | 10.09 |
| | Avr. | 2,822 | 9,718 | 16 | -7.65 | -4.05 | -13.11 | -3.60 | 5.47 |
| | StDv | 3,549 | 5,294 | 6 | 4.71 | 1.02 | 0.54 | 4.77 | 4.92 |
| **HPN** | Rep.1 | 27,181 | 61,191 | 2,616 | -2.51 | -1.34 | -5.70 | -1.18 | 3.18 |
| | Rep.2 | 45,014 | 56,079 | 2,152 | -1.98 | -1.26 | -5.72 | -0.72 | 3.74 |
| | Rep.3 | 24,434 | 44,764 | 1,615 | -1.73 | -1.64 | -6.59 | -0.09 | 4.86 |
| | Avr. | 32,210 | 54,011 | 2,128 | -2.07 | -1.41 | -6.00 | -0.66 | 3.93 |
| | StDv | 11,174 | 8,406 | 501 | 0.40 | 0.20 | 0.51 | 0.54 | 0.85 |
| **HSBP1** | Rep.1 | 715,949 | 515,099 | 585,263 | 2.21 | 1.74 | 2.11 | 0.47 | 0.10 |
| | Rep.2 | 936,366 | 390,235 | 488,172 | 2.40 | 1.54 | 2.11 | 0.86 | 0.29 |
| | Rep.3 | 434,201 | 353,606 | 747,508 | 2.42 | 1.35 | 2.27 | 1.08 | 0.16 |
| | Avr. | 695,505 | 419,647 | 606,981 | 2.34 | 1.54 | 2.16 | 0.80 | 0.18 |
| | StDv | 251,706 | 84,669 | 131,025 | 0.12 | 0.19 | 0.09 | 0.31 | 0.10 |
| **IGFBP1** | Rep.1 | 4,956 | 3 | 3,852 | -4.97 | -15.65 | -5.14 | 10.68 | 0.17 |
| | Rep.2 | 2,768 | 3 | 9,424 | -6.01 | -15.45 | -3.59 | 9.45 | -2.42 |
| | Rep.3 | 3 | 3 | 5 | -14.72 | -15.50 | -14.92 | 0.78 | 0.20 |
| | Avr. | 2,576 | 3 | 4,427 | -8.56 | -15.54 | -7.88 | 6.97 | -0.68 |
| | StDv | 2,482 | 0 | 4,736 | 5.36 | 0.10 | 6.15 | 5.40 | 1.50 |
| **KLK3.47 0** | Rep.1 | 152,238 | 296,395 | 38,574 | -0.03 | 0.94 | -1.81 | -0.97 | 1.79 |
| | Rep.2 | 118,440 | 150,567 | 19,551 | -0.59 | 0.16 | -2.54 | -0.75 | 1.95 |
| | Rep.3 | 92,387 | 178,823 | 40,080 | 0.19 | 0.36 | -1.96 | -0.17 | 2.15 |
| | Avr. | 121,022 | 208,595 | 32,735 | -0.14 | 0.49 | -2.10 | -0.63 | 1.96 |
| | StDv | 30,009 | 77,338 | 11,442 | 0.40 | 0.40 | 0.38 | 0.41 | 0.18 |
| **LRRN1** | Rep.1 | 370 | 78 | 7,572 | -8.71 | -10.95 | -4.16 | 2.24 | -4.55 |
| | Rep.2 | 4,313 | 397 | 5 | -5.37 | -8.41 | -14.47 | 3.04 | 9.10 |
| | Rep.3 | 1,651 | 843 | 1,282 | -5.62 | -7.37 | -6.92 | 1.75 | 1.31 |
| | Avr. | 2,111 | 439 | 2,953 | -6.56 | -8.91 | -8.52 | 2.34 | 1.95 |
| | StDv | 2,011 | 384 | 4,051 | 1.86 | 1.85 | 5.34 | 0.65 | 6.85 |
| **MAP3K7** | Rep.1 | 313,649 | 286,012 | 327,741 | 1.01 | 0.89 | 1.27 | 0.13 | -0.26 |
| | Rep.2 | 481,330 | 323,184 | 393,629 | 1.44 | 1.26 | 1.79 | 0.17 | -0.36 |
| | Rep.3 | 305,428 | 340,706 | 532,702 | 1.92 | 1.29 | 1.78 | 0.62 | 0.14 |
| | Avr. | 366,802 | 316,634 | 418,024 | 1.46 | 1.15 | 1.61 | 0.31 | -0.16 |
| | StDv | 99,269 | 27,929 | 104,636 | 0.45 | 0.23 | 0.30 | 0.27 | 0.26 |
| **MYEF2** | Rep.1 | 22,256 | 26,221 | 17,459 | -2.80 | -2.56 | -2.96 | -0.24 | 0.16 |
| | Rep.2 | 43,512 | 50,275 | 11,295 | -2.03 | -1.42 | -3.33 | -0.61 | 1.30 |
| | Rep.3 | 18,439 | 33,731 | 24,686 | -2.13 | -2.04 | -2.65 | -0.09 | 0.52 |
| | Avr. | 28,069 | 36,742 | 17,813 | -2.32 | -2.01 | -2.98 | -0.31 | 0.66 |
| | StDv | 13,510 | 12,306 | 6,703 | 0.42 | 0.57 | 0.34 | 0.27 | 0.58 |
| **OPRK1** | Rep.1 | 17 | 7 | 2,208 | -13.16 | -14.43 | -5.94 | 1.27 | -7.22 |
| | Rep.2 | 2,902 | 248 | 3,210 | -5.94 | -9.08 | -5.14 | 3.15 | -0.79 |
| | Rep.3 | 71 | 3 | 4,485 | -10.15 | -15.50 | -5.11 | 5.35 | -5.04 |
| | Avr. | 997 | 86 | 3,301 | -9.75 | -13.01 | -5.40 | 3.26 | -4.35 |
| | StDv | 1,650 | 140 | 1,141 | 3.63 | 3.44 | 0.47 | 2.04 | 3.27 |
| **PCAT14** | Rep.1 | 9,159 | 11,924 | 19,837 | -4.08 | -3.70 | -2.77 | -0.39 | -1.31 |
| | Rep.2 | 16,009 | 8,041 | 9,785 | -3.47 | -4.07 | -3.54 | 0.59 | 0.06 |
| | Rep.3 | 7,083 | 5,460 | 24,145 | -3.51 | -4.67 | -2.69 | 1.16 | -0.83 |
| | Avr. | 10,750 | 8,475 | 17,922 | -3.69 | -4.14 | -3.00 | 0.45 | -0.69 |
| | StDv | 4,671 | 3,254 | 7,369 | 0.34 | 0.49 | 0.47 | 0.78 | 0.70 |
| **PFKP** | Rep.1 | 144,614 | 98,784 | 122,550 | -0.10 | -0.65 | -0.15 | 0.54 | 0.04 |
| | Rep.2 | 171,077 | 139,353 | 117,508 | -0.06 | 0.05 | 0.05 | -0.11 | -0.11 |
| | Rep.3 | 99,055 | 83,294 | 108,599 | 0.29 | -0.74 | -0.52 | 1.03 | 0.81 |
| | Avr. | 138,249 | 107,144 | 116,219 | 0.04 | -0.45 | -0.20 | 0.49 | 0.25 |
| | StDv | 36,430 | 28,949 | 7,064 | 0.22 | 0.43 | 0.29 | 0.57 | 0.49 |
| **PFKL** | Rep.1 | 43,313 | 33,493 | 41,348 | -1.84 | -2.21 | -1.71 | 0.37 | -0.13 |
| | Rep.2 | 65,474 | 71,324 | 55,748 | -1.44 | -0.92 | -1.03 | -0.53 | -0.42 |
| | Rep.3 | 44,011 | 41,829 | 66,882 | -0.88 | -1.73 | -1.22 | 0.85 | 0.34 |
| | Avr. | 50,933 | 48,882 | 54,659 | -1.39 | -1.62 | -1.32 | 0.23 | -0.07 |
| | StDv | 12,598 | 19,877 | 12,802 | 0.48 | 0.65 | 0.36 | 0.70 | 0.38 |
| **PLA2G7** | Rep.1 | 2,638 | 7,777 | 698 | -5.88 | -4.31 | -7.60 | -1.57 | 1.72 |
| | Rep.2 | 15,312 | 7,533 | 28 | -3.54 | -4.16 | -11.98 | 0.62 | 8.45 |
| | Rep.3 | 1,237 | 9,543 | 2,435 | -6.03 | -3.86 | -6.00 | -2.17 | -0.04 |
| | Avr. | 6,396 | 8,284 | 1,054 | -5.15 | -4.11 | -8.53 | -1.04 | 3.38 |
| | StDv | 7,753 | 1,097 | 1,242 | 1.40 | 0.23 | 3.10 | 1.47 | 4.48 |
| **PSMA** | Rep.1 | 48,780 | 219,535 | 13,959 | -1.67 | 0.51 | -3.28 | -2.18 | 1.61 |
| | Rep.2 | 39,582 | 266,004 | 162 | -2.17 | 0.98 | -9.45 | -3.15 | 7.28 |
| | Rep.3 | 12,045 | 155,230 | 3,076 | -2.75 | 0.16 | -5.66 | -2.91 | 2.91 |
| | Avr. | 33,469 | 213,590 | 5,732 | -2.20 | 0.55 | -6.13 | -2.74 | 3.94 |
| | StDv | 19,115 | 55,626 | 7,272 | 0.54 | 0.41 | 3.11 | 0.51 | 2.97 |
| **SAA2** | Rep.1 | 32,915 | 23,385 | 5,206 | -2.24 | -2.72 | -4.70 | 0.49 | 2.47 |
| | Rep.2 | 16,951 | 10,526 | 334 | -3.39 | -3.68 | -8.41 | 0.29 | 5.02 |
| | Rep.3 | 11,263 | 12,714 | 3,183 | -2.85 | -3.45 | -5.61 | 0.61 | 2.76 |
| | Avr. | 20,376 | 15,542 | 2,908 | -2.82 | -3.28 | -6.24 | 0.46 | 3.42 |
| | StDv | 11,225 | 6,880 | 2,448 | 0.58 | 0.50 | 1.93 | 0.16 | 1.39 |
| **SERPIN A1** | Rep.1 | 123,407 | 96,522 | 39,550 | -0.33 | -0.68 | -1.78 | 0.35 | 1.45 |
| | Rep.2 | 94,620 | 28,318 | 12,562 | -0.91 | -2.25 | -3.18 | 1.34 | 2.26 |
| | Rep.3 | 48,679 | 53,221 | 41,185 | -0.73 | -1.39 | -1.92 | 0.65 | 1.18 |
| | Avr. | 88,902 | 59,354 | 31,099 | -0.66 | -1.44 | -2.29 | 0.78 | 1.63 |
| | StDv | 37,691 | 34,513 | 16,074 | 0.30 | 0.79 | 0.77 | 0.51 | 0.56 |
| **SLC10A7** | Rep.1 | 16,866 | 34,875 | 7,675 | -3.20 | -2.15 | -4.14 | -1.05 | 0.94 |
| | Rep.2 | 3,660 | 5,356 | 3,205 | -5.60 | -4.65 | -5.15 | -0.95 | -0.46 |
| | Rep.3 | 7,367 | 13,761 | 1,632 | -3.46 | -3.34 | -6.57 | -0.12 | 3.12 |
| | Avr. | 9,298 | 17,997 | 4,171 | -4.09 | -3.38 | -5.29 | -0.71 | 1.20 |
| | StDv | 6,811 | 15,209 | 3,135 | 1.32 | 1.25 | 1.22 | 0.51 | 1.80 |
| **SMAD5** | Rep.1 | 369,739 | 350,017 | 407,427 | 1.25 | 1.18 | 1.59 | 0.07 | -0.33 |
| | Rep.2 | 290,176 | 196,854 | 221,405 | 0.71 | 0.55 | 0.96 | 0.16 | -0.26 |
| | Rep.3 | 196,008 | 163,982 | 204,033 | 1.28 | 0.24 | 0.39 | 1.04 | 0.88 |
| | Avr. | 285,308 | 236,951 | 277,622 | 1.08 | 0.66 | 0.98 | 0.42 | 0.10 |
| | StDv | 86,968 | 99,288 | 112,750 | 0.32 | 0.48 | 0.60 | 0.53 | 0.68 |
| **SPON2** | Rep.1 | 120,585 | 152,859 | 71,489 | -0.36 | -0.02 | -0.92 | -0.35 | 0.56 |
| | Rep.2 | 177,482 | 137,573 | 49,919 | 0.00 | 0.03 | -1.18 | -0.03 | 1.18 |
| | Rep.3 | 87,791 | 85,642 | 68,463 | 0.12 | -0.70 | -1.18 | 0.82 | 1.30 |
| | Avr. | 128,619 | 125,358 | 63,290 | -0.08 | -0.23 | -1.10 | 0.14 | 1.01 |
| | StDv | 45,382 | 35,234 | 11,678 | 0.25 | 0.41 | 0.15 | 0.60 | 0.40 |
| **SRC** | Rep.1 | 22,920 | 29,855 | 12,967 | -2.76 | -2.37 | -3.39 | -0.39 | 0.63 |
| | Rep.2 | 20,332 | 26,195 | 16,253 | -3.13 | -2.36 | -2.80 | -0.77 | -0.33 |
| | Rep.3 | 13,691 | 17,857 | 33,398 | -2.56 | -2.96 | -2.22 | 0.40 | -0.35 |
| | Avr. | 18,981 | 24,636 | 20,873 | -2.82 | -2.56 | -2.80 | -0.25 | -0.01 |
| | StDv | 4,761 | 6,149 | 10,971 | 0.29 | 0.34 | 0.58 | 0.59 | 0.56 |
| **SYNPO2** | Rep.1 | 1,269,282 | 764,162 | 1,271,402 | 3.03 | 2.31 | 3.23 | 0.73 | -0.20 |
| | Rep.2 | 1,854,291 | 663,642 | 1,094,823 | 3.38 | 2.30 | 3.27 | 1.08 | 0.11 |
| | Rep.3 | 1,054,005 | 725,221 | 1,560,688 | 3.70 | 2.38 | 3.33 | 1.32 | 0.37 |
| | Avr. | 1,392,526 | 717,675 | 1,308,971 | 3.37 | 2.33 | 3.28 | 1.04 | 0.10 |
| | StDv | 414,133 | 50,683 | 235,194 | 0.34 | 0.05 | 0.05 | 0.30 | 0.29 |
| **TDRD1** | Rep.1 | 9,108 | 2,685 | 847 | -4.09 | -5.85 | -7.32 | 1.76 | 3.23 |
| | Rep.2 | 3,369 | 1,050 | 1,123 | -5.72 | -7.00 | -6.66 | 1.28 | 0.94 |
| | Rep.3 | 1,790 | 176 | 5 | -5.50 | -9.63 | -14.92 | 4.13 | 9.43 |
| | Avr. | 4,756 | 1,304 | 658 | -5.10 | -7.49 | -9.64 | 2.39 | 4.53 |
| | StDv | 3,851 | 1,274 | 582 | 0.88 | 1.94 | 4.59 | 1.52 | 4.39 |
| **TRIB1** | Rep.1 | 41,926 | 46,385 | 34,225 | -1.89 | -1.74 | -1.99 | -0.15 | 0.10 |
| | Rep.2 | 47,764 | 35,288 | 23,641 | -1.90 | -1.93 | -2.26 | 0.03 | 0.37 |
| | Rep.3 | 22,768 | 15,896 | 12,646 | -1.83 | -3.13 | -3.62 | 1.30 | 1.79 |
| | Avr. | 37,486 | 32,523 | 23,504 | -1.87 | -2.27 | -2.62 | 0.39 | 0.75 |
| | StDv | 13,076 | 15,431 | 10,790 | 0.04 | 0.75 | 0.87 | 0.79 | 0.91 |
| **TSPAN13** | Rep.1 | 126,805 | 135,413 | 60,500 | -0.29 | -0.19 | -1.16 | -0.10 | 0.87 |
| | Rep.2 | 127,130 | 153,934 | 66,513 | -0.48 | 0.19 | -0.77 | -0.68 | 0.29 |
| | Rep.3 | 99,522 | 52,802 | 42,203 | 0.30 | -1.40 | -1.88 | 1.70 | 2.18 |
| | Avr. | 117,819 | 114,050 | 56,405 | -0.16 | -0.46 | -1.27 | 0.31 | 1.11 |
| | StDv | 15,847 | 53,844 | 12,662 | 0.41 | 0.83 | 0.56 | 1.24 | 0.97 |

In Table 14, the data represents those RNA biomarkers with a Log₂ FC >2 in the differential expression in the tumour compare to the adjacent gland. Most of these RNA biomarkers are up regulated in the tumor compared with the adjacent glandular tissue. Only two biomarkers were detected in a higher amount in the adjacent glandular tissue compared with all tumors. Some distinctions between the different grades of tumors can be made, for example with the OPRK1 and PSMA RNA biomarkers.

**Table 14: RNA biomarker with differential expression (Log₂ FC) in Tumor and adjacent tissues of Subject 2**

| **Differential expression (>2Log₂FC) in Subject 2 tumors* compared with adjacent glandular tissue** | | **RNA Biomarkers** |
|---|---|---|
| Up regulated in: | T1 | TPX2, SPP1, PIP |
| | T2 | HOXC4, HPN, KLK3.470, C15orf48, PSMA, PLA2G7, SAA2, HN1 |
| | T3 | HPN, C15orf48, KLK3.470, ApoC1, SAA2 |
| Down regulated in: | T1 | PSCA |
| | T2 | PSCA, OPRK1, IGFBP1 |
| | T3 | OPRK1 |

| | | |
|---|---|---|
| *T1(Gleason score 4+5), T2 (3+4), and T3 (3+3)) | | |

### Comments on RNA biomarker expression in subject 1 and subject 2

Before proceeding with the amplicon production for RBAS analysis, the efficiency of all the RNA specific primers was tested by real time PCR or by visualization of the produced amplicon of the expected size. Therefore, the lower sequence counts observed for certain amplicons produced from prostatectomy tissues RNA cannot be attributed to the inefficiency of the amplicon production. As seen in Example 1, raw sequence counts of 900 and 13,000 were obtained from the MUC1 amplicon produced from LNCaP and A549 cell RNA respectively (Table 6).

The process used to select RNA biomarkers disclosed herein is by selecting those that are up-regulated or down-regulated in a small number of prostate tumors, rather than in all prostate tumors. For this reason it is not expected that differential expression of all the RNA biomarkers would be seen in all prostate tumors or their adjacent tissues. The data indicate that tumors examined from Subjects 1 and 2 are likely not to have some of the RNA dysregulated within their tissue. The analysis of tumors from a range of subjects will will likely reveal differences in the expression of these and other RNA biomarkers. That is the major reason why, for diagnostic and prognostic use, RNA biomarker panels are selected from a large RNA biomarker pool. RBAS methodology has been developed to allow rapid screening of tumor samples for a large number of RNA biomarkers simultaneously.

In conclusion, these observations highlight the issue with staging prostate cancers and illustrate reasons for developing multi-RNA biomarker diagnostics, as it is unlikely that a single RNA biomarker can diagnose and stage prostate cancers, or distinguish prostate cancer from begnin prostate hyperplasia or prostatitis.

SEQ ID NO: 1-326 are set out in the attached Sequence Listing. The codes for nucleotide sequences used in the attached Sequence Listing, including the symbol "n," conform to WIPO Standard ST.25 (1998), Appendix 2, Table 1.

## Claims

1. A method for detecting the presence of a disorder and/or monitoring the progression of the disorder in a subject, comprising:
(a) determining the relative frequency of expression of a plurality of RNA biomarkers simultaneously in a biological sample obtained from the subject, wherein the relative frequency of expression is determined using next generation sequencing of an amplicon cDNA library prepared using a plurality oligonucleotide primers specific for the plurality of RNA biomarkers; and
(b) comparing the relative frequency of expression of the plurality of RNA biomarkers in the biological sample with a predetermined threshold value, wherein increased or decreased relative frequency of expression of the plurality of RNA biomarkers in the biological sample relative to the predetermined threshold value indicates the presence of the disorder and/or progression of the disorder in the subject.

2. The method of claim 1, wherein the amplicon cDNA library is prepared by:
(a) isolating total RNA from the biological sample;
(b) generating first strand cDNA from the total RNA using a plurality of first oligonucleotide primers specific for the plurality of RNA biomarkers;
(c) synthesizing second strand cDNA to provide double-stranded cDNA;
(d) adding at least one sequencing adapter to the double-stranded cDNA; and
(e) amplifying the double-stranded cDNA to provide the amplicon cDNA library.

3. The method of claim 2, wherein the first oligonucleotide primer is selected from the group consisting of: SEQ ID NO: 76-223 and 293-326.

4. The method of claim 2, further comprising amplifying the double-stranded cDNA by polymerase chain reaction using a plurality of oligonucleotide primer pairs specific for the plurality of RNA biomarkers after step (c) and prior to step (d).

5. The method of claim 1, wherein the amplicon cDNA library is prepared by:
(a) isolating total RNA from the biological sample;
(b) preparing first strand cDNA to provide single-stranded cDNA;
(c) amplifying the single-stranded cDNA by polymerase chain reaction using a plurality of oligonucleotide primer pairs specific for the plurality of RNA biomarkers to provide amplified double-stranded cDNA;
(d) adding at least one sequencing adapter to the amplified double-stranded cDNA; and
(e) further amplifying the amplified double-stranded cDNA using primers specific for the at least one sequencing adapter to provide the amplicon cDNA library.

6. The method of any one of claims 4 and 5, wherein at least one member of the plurality of oligonucleotide primer pairs is selected from the group consisting of SEQ ID NO: 76-223 and 293-326.

7. The method of any one of claims 1-6, wherein the disorder is a cancer.

8. The method of any one of claims 1-7, wherein the disorder is prostate cancer and the plurality of RNA biomarkers comprises a RNA sequence corresponding to a DNA sequence selected from the group consisting of: SEQ ID NO: 1-71 and 235-287.

9. The method of any one of claims 1-8, wherein the biological sample is selected from the group consisting of: urine, blood, serum, cell lines, PBMCs, biopsy tissue, and prostatectomy tissue.

10. The method of any one of claims 1-9, wherein the plurality of RNA biomarkers comprises at least ten RNA biomarkers.

11. The method of claim 1, wherein the frequency of expression of the plurality of RNA biomarkers is normalized to at least one expression standard selected from the reporters listed in Table 3.

12. The method of any one of claims 1-11, wherein amplicons in the amplicon cDNA library are 60-300 bp in size.

## Patentansprüche

1. Verfahren zum Nachweisen des Vorliegens einer Störung und/oder Überwachen des Fortschreitens der Störung bei einem Subjekt, umfassend:
(a) gleichzeitiges Bestimmen der relativen Häufigkeit der Expression einer Vielzahl von RNS-Biomarkern in einer von dem Subjekt erhaltenen biologischen Probe, wobei die relative Häufigkeit der Expression unter Verwendung von Sequenzierung der nächsten Generation einer Amplicon-cDNS-Bibliothek bestimmt wird, die unter Verwendung einer Vielzahl von Oligonukleotid-Primern hergestellt wurde, die für die Vielzahl von RNS-Biomarkern spezifisch ist; und
(b) Vergleichen der relativen Häufigkeit der Expression der Vielzahl von RNS-Biomarkern in der biologischen Probe mit einem vorbestimmten Schwellenwert, wobei eine erhöhte oder verminderte relative Häufigkeit der Expression der Vielzahl von RNS-Biomarkern in der biologischen Probe relativ zu dem vorbestimmten Schwellenwert das Vorliegen der Störung und/oder das Fortschreiten der Störung bei dem Subjekt anzeigt.

2. Verfahren nach Anspruch 1, wobei die Amplicon-cDNS-Bibliothek durch Folgendes hergestellt wird:
(a) Isolieren von Gesamt-RNS aus der biologischen Probe;
(b) Erzeugen von Erststrang-cDNS aus der Gesamt-RNS unter Verwendung einer Vielzahl von ersten Oligonukleotid-Primern, die für die Vielzahl von RNS-Biomarkern spezifisch sind;
(c) Synthetisieren von Zweitstrang-cDNS, um doppelsträngige cDNS bereitzustellen;
(d) Zusetzen mindestens eines Sequenzierungsadapters zu der doppelsträngigen cDNS; und
(e) Amplifizieren der doppelsträngigen cDNS, um die Amplicon-cDNS-Bibliothek bereitzustellen.

3. Verfahren nach Anspruch 2, wobei der erste Oligonukleotid-Primer aus der Gruppe bestehend aus SEQ ID NO: 76-223 und 293-326 ausgewählt ist.

4. Verfahren nach Anspruch 2, ferner umfassend Amplifizieren der doppelsträngigen cDNS durch Polymerasekettenreaktion unter Verwendung einer Vielzahl von Oligonukleotid-Primerpaaren, die für die Vielzahl von RNS-Biomarkern spezifisch sind, nach Schritt (c) und vor Schritt (d).

5. Verfahren nach Anspruch 1, wobei die Amplicon-cDNS-Bibliothek durch Folgendes hergestellt wird:
(a) Isolieren von Gesamt-RNS aus der biologischen Probe;
(b) Herstellen von Erststrang-cDNS, um einzelsträngige cDNS bereitzustellen;
(c) Amplifizieren der einzelsträngigen cDNS durch Polymerasekettenreaktion unter Verwendung einer Vielzahl von Oligonukleotid-Primerpaaren, die für die Vielzahl von RNS-Biomarkern spezifisch sind, um amplifizierte doppelsträngige cDNS bereitzustellen;
(d) Zusetzen mindestens eines Sequenzierungsadapters zu der amplifizierten doppelsträngigen cDNS; und
(e) weiteres Amplifizieren der amplifizierten doppelsträngigen cDNS, unter Verwendung von Primern, die den mindestens einen Sequenzierungsadapter spezifisch sind, um die Amplicon-cDNS-Bibliothek bereitzustellen.

6. Verfahren nach einem der Ansprüche 4 und 5, wobei mindestens ein Mitglied der Vielzahl von Oligonukleotid-Primerpaaren aus der Gruppe bestehend aus SEQ ID NO: 76-223 und 293-326 ausgewählt ist.

7. Verfahren nach einem der Ansprüche 1 bis 6, wobei die Störung ein Krebs ist.

8. Verfahren nach einem der Ansprüche 1 bis 7, wobei die Störung Prostatakrebs ist und die Vielzahl von RNS-Biomarkern eine RNS-Sequenz umfasst, die einer DNS-Sequenz entspricht, die aus der Gruppe bestehend aus SEQ ID NO: 1-71 und 235-287 ausgewählt ist.

9. Verfahren nach einem der Ansprüche 1-8, wobei die biologische Probe aus der Gruppe bestehend aus Urin, Blut, Serum, Zelllinien, PBMCs, Biopsiegewebe und Prostatektomiegewebe ausgewählt ist.

10. Verfahren nach einem der Ansprüche 1-9, wobei die Vielzahl von RNS-Biomarkern mindestens zehn RNS-Biomarker umfasst.

11. Verfahren nach Anspruch 1, wobei die Häufigkeit der Expression der Vielzahl von RNS-Biomarkern auf mindestens einen Expressionsstandard normalisiert wird, der aus den in Tabelle 3 aufgeführten Reportern ausgewählt wird.

12. Verfahren nach einem der Ansprüche 1-11, wobei Amplicons in der Amplicon-cDNS-Bibliothek 60-300 Bp groß sind.

## Revendications

1. Méthode pour la détection de la présence d'un trouble et/ou la surveillance de la progression du trouble chez un sujet, comprenant :
(a) la détermination de la fréquence relative de l'expression d'une pluralité de biomarqueurs ARN simultanément dans un échantillon biologique provenant du sujet, dans laquelle la fréquence relative de l'expression est déterminée à l'aide du séquençage de prochaine génération d'une banque d'ADNc d'amplicon préparée en utilisant une pluralité d'amorces oligonucléotides spécifique à la pluralité des biomarqueurs ARN ; et
(b) la comparaison de la fréquence relative de l'expression de la pluralité des biomarqueurs ARN dans l'échantillon biologique à une valeur seuil prédéterminée, dans laquelle une augmentation ou une diminution de la fréquence relative de l'expression de la pluralité des biomarqueurs ARN dans l'échantillon biologique par rapport à la valeur seuil prédéterminée indique la présence du trouble et/ou de la progression du trouble chez le sujet.

2. Méthode de la revendication 1, dans laquelle la banque d'ADNc d'amplicon est préparée en :
(a) isolant l'ARN total à partir de l'échantillon biologique ;
(b) générant un premier brin d'ADNc à partir de l'ARN total en utilisant une pluralité des premières amorces oligonucléotides spécifiques à la pluralité des biomarqueurs ARN ;
(c) synthétisant un second brin d'ADNc pour produire un ADNc bicaténaire ;
(d) ajoutant au moins un adaptateur de séquençage à l'ADNc bicaténaire ; et
(e) amplifiant l'ADNc bicaténaire pour donner la banque d'ADNc d'amplicon.

3. Méthode de la revendication 2, dans laquelle le polypeptide hétérologue est choisi dans le groupe composé de la : SEQ ID NO: 76-223 et 293-326.

4. Méthode de la revendication 2, comprenant également l'amplification de l'ADNc bicaténaire par une réaction en chaîne par polymérase utilisant une pluralité de paires d'amorces oligonucléotides spécifique à la pluralité des biomarqueurs ARN après l'étape (c) et avant l'étape (d).

5. Méthode de la revendication 1, dans laquelle la banque d'ADNc d'amplicon est préparée en :
(a) isolant l'ARN total à partir de l'échantillon biologique ;
(b) préparant un premier brin d'ADNc pour produire un ADNc monocaténaire ;
(c) amplifiant l'ADNc monocaténaire par réaction en chaîne par polymérase utilisant une pluralité de paires d'amorces oligonucléotides spécifique à la pluralité des biomarqueurs ARN pour donner un ADNc bicaténaire amplifié ;
(d) ajoutant au moins un adaptateur de séquençage à l'ADNc bicaténaire amplifié ; et
(e) ré-amplifiant l'ADNc bicaténaire amplifié à l'aide des amorces spécifiques pour l'au moins un adaptateur de séquençage pour donner la banque d'ADNc d'amplicon.

6. Méthode de l'une quelconque des revendications 4 et 5, dans laquelle l'au moins un élément de la pluralité des paires d'amorces oligonucléotides est choisi dans le groupe composé de la SEQ ID NO: 76-223 et 293-326.

7. Méthode de l'une quelconque des revendications 1 à 6, dans laquelle le trouble est un cancer.

8. Méthode de l'une quelconque des revendications 1 à 7, dans laquelle le trouble est le cancer de la prostate et la pluralité des biomarqueurs ARN comprend une séquence d'ARN correspondant à la séquence d'ADN choisie dans le groupe composé de la : SEQ ID NO: 1-71 et 235-287.

9. Méthode de l'une quelconque des revendications 1 à 8, dans laquelle l'échantillon biologique est choisi dans le groupe composé : d'urine, de sang, de sérum, de lignées cellulaires, de PBMC, de tissu de biopsie et de tissu de prostatectomie.

10. Méthode de l'une quelconque des revendications 1 à 9, dans laquelle la pluralité des biomarqueurs ARN comprend au moins dix biomarqueurs ARN.

11. Méthode de la revendication 1, dans laquelle la fréquence de l'expression de la pluralité des biomarqueurs ARN est normalisée à au moins une expression standard choisie parmi les rapporteurs énumérés dans le Tableau 3.

12. Méthode de l'une quelconque des revendications 1 à 11, dans laquelle les amplicons dans la banque d'ADNc d'amplicon ont une taille de 60 à 300 pb.
